(19) **Europäisches Patentamt**

**European Patent Office**

**Office européen des brevets**

(11) **EP 1 065 213 A2**

(12) **EUROPEAN PATENT APPLICATION**

(43) Date of publication:
**03.01.2001 Bulletin 2001/01**

(21) Application number: **00113955.9**

(22) Date of filing: **30.06.2000**

(51) Int. Cl.⁷: **C07K 14/18**, C12N 9/12,
C12N 15/54, C12N 15/10,
C12Q 1/25, A61K 38/00,
A61P 31/14

(84) Designated Contracting States:
**AT BE CH CY DE DK ES FI FR GB GR IE IT LI LU MC NL PT SE**
Designated Extension States:
**AL LT LV MK RO SI**

(30) Priority: **02.07.1999 JP 18863099**
**07.07.1999 JP 19248899**

(71) Applicant: **Japan Tobacco Inc.**
**Tokyo 105-8422 (JP)**

(72) Inventors:
• **Ago, Hideo,**
**Central Pharmaceutical Research Inst.**
**Takatsuki-shi, Osaka 569-1125 (JP)**
• **Miyano, Masashi**
**Machida-shi, Tokyo 194-0045 (JP)**
• **Adachi, Tsuyoshi,**
**Central Pharmaceutical Research**
**Takatsuki-shi, Osaka 569-1125 (JP)**

(74) Representative:
**VOSSIUS & PARTNER**
**Siebertstrasse 4**
**81675 München (DE)**

(54) **HCV polymerase suitable for crystal structure analysis and method for using the enzyme**

(57) An HCV polymerase suitable for crystal structural analysis and a method for using the enzyme are provided. The HCV polymerase suitable for crystal structural analysis and/or comprising high HCV polymerase activity can be used for the three-dimensional structural analysis, and for rational identification of HCV polymerase inhibitors by computers. The enzyme can also be used for efficiently evaluating the HCV polymerase-inhibitory activity. The evaluation can be more efficiently performed by combining identification by computers.

EP 1 065 213 A2

Printed by Xerox (UK) Business Services
2.16.7 (HRS)/3.6

SEQUENCE LISTING

<110> Japan Tobacco, Inc.

<120> HCV Polymerase Suitable For Crystal Structure Analysis
      And Method For Using The Enzyme

<130> J00-0086

<140>
<141>

<150> JP P1999-188630
<151> 1999-07-02

<150> JP P1999-192488
<151> 1999-07-07

<160> 24

<170> PatentIn Ver. 2.1

<210> 1
<211> 591
<212> PRT
<213> Hepatitis C virus

<400> 1
Ser Met Ser Tyr Thr Trp Thr Gly Ala Leu Ile Thr Pro Cys Ala Ala

```
        1                   5                   10                  15

Glu Glu Ser Lys Leu Pro Ile Asn Ala Leu Ser Asn Ser Leu Leu Arg
                20                  25                  30

His His Asn Met Val Tyr Ala Thr Thr Ser Arg Ser Ala Gly Leu Arg
                35                  40                  45

Gln Lys Lys Val Thr Phe Asp Arg Leu Gln Val Leu Asp Asp His Tyr
                50                  55                  60

Arg Asp Val Leu Lys Glu Met Lys Ala Lys Ala Ser Thr Val Lys Ala
 65                  70                  75                  80

Lys Leu Leu Ser Val Glu Glu Ala Cys Lys Leu Thr Pro Pro His Ser
                85                  90                  95

Ala Lys Ser Lys Phe Gly Tyr Gly Ala Lys Asp Val Arg Asn Leu Ser
                100                 105                 110

Ser Lys Ala Val Asn His Ile His Ser Val Trp Lys Asp Leu Leu Glu
                115                 120                 125

Asp Thr Val Thr Pro Ile Asp Thr Thr Ile Met Ala Lys Asn Glu Val
                130                 135                 140

Phe Cys Val Gln Pro Glu Lys Gly Gly Arg Lys Pro Ala Arg Leu Ile
 145                 150                 155                 160
```

Val Phe Pro Asp Leu Gly Val Arg Val Cys Glu Lys Met Ala Leu Tyr
165 170 175

Asp Val Val Ser Thr Leu Pro Gln Val Val Met Gly Ser Ser Tyr Gly
180 185 190

Phe Gln Tyr Ser Pro Gly Gln Arg Val Glu Phe Leu Val Asn Thr Trp
195 200 205

Lys Ser Lys Lys Asn Pro Met Gly Phe Ser Tyr Asp Thr Arg Cys Phe
210 215 220

Asp Ser Thr Val Thr Glu Asn Asp Ile Arg Val Glu Glu Ser Ile Tyr
225 230 235 240

Gln Cys Cys Asp Leu Ala Pro Glu Ala Arg Gln Ala Ile Lys Ser Leu
245 250 255

Thr Glu Arg Leu Tyr Ile Gly Gly Pro Leu Thr Asn Ser Lys Gly Gln
260 265 270

Asn Cys Gly Tyr Arg Arg Cys Arg Ala Ser Gly Val Leu Thr Thr Ser
275 280 285

Cys Gly Asn Thr Leu Thr Cys Tyr Leu Lys Ala Ser Ala Ala Cys Arg
290 295 300

Ala Ala Lys Leu Gln Asp Cys Thr Met Leu Val Asn Gly Asp Asp Leu
305 310 315 320

Val Val Ile Cys Glu Ser Ala Gly Thr Gln Glu Asp Ala Ala Ser Leu
                325                 330                 335

Arg Val Phe Thr Glu Ala Met Thr Arg Tyr Ser Ala Pro Pro Gly Asp
                340                 345                 350

Pro Pro Gln Pro Glu Tyr Asp Leu Glu Leu Ile Thr Ser Cys Ser Ser
                355                 360                 365

Asn Val Ser Val Ala His Asp Ala Ser Gly Lys Arg Val Tyr Tyr Leu
            370                 375                 380

Thr Arg Asp Pro Thr Thr Pro Leu Ala Arg Ala Ala Trp Glu Thr Ala
385                 390                 395                 400

Arg His Thr Pro Val Asn Ser Trp Leu Gly Asn Ile Ile Met Tyr Ala
                405                 410                 415

Pro Thr Leu Trp Ala Arg Met Ile Leu Met Thr His Phe Phe Ser Ile
                420                 425                 430

Leu Leu Ala Gln Glu Gln Leu Glu Lys Ala Leu Asp Cys Gln Ile Tyr
            435                 440                 445

Gly Ala Cys Tyr Ser Ile Glu Pro Leu Asp Leu Pro Gln Ile Ile Glu
        450                 455                 460

Arg Leu His Gly Leu Ser Ala Phe Ser Leu His Ser Tyr Ser Pro Gly

    465      470      475      480

Glu Ile Asn Arg Val Ala Ser Cys Leu Arg Lys Leu Gly Val Pro Pro
      485      490      495

Leu Arg Val Trp Arg His Arg Ala Arg Ser Val Arg Ala Arg Leu Leu
    500      505      510

Ser Gln Gly Gly Arg Ala Ala Thr Cys Gly Lys Tyr Leu Phe Asn Trp
    515      520      525

Ala Val Lys Thr Lys Leu Lys Leu Thr Pro Ile Pro Ala Ala Ser Gln
   530      535      540

Leu Asp Leu Ser Gly Trp Phe Val Ala Gly Tyr Ser Gly Gly Asp Ile
545      550      555      560

Tyr His Ser Leu Ser Arg Ala Arg Pro Arg Trp Phe Met Leu Cys Leu
    565      570      575

Leu Leu Leu Ser Val Gly Val Gly Ile Tyr Leu Leu Pro Asn Arg
    580      585      590

<210> 2

<211> 1743

<212> DNA

<213> Artificial Sequence

<220>

<223> Description of Artificial Sequence:DNA encoding
fusion protein consisting of a portion of HCV
polymerase and histidine tag at the C-terminus

<220>

<221> CDS

<222> (1)..(1743)

<400> 2

```
atg tca atg tcc tac aca tgg aca ggc gcc ttg atc acg cca tgc gct   48
Met Ser Met Ser Tyr Thr Trp Thr Gly Ala Leu Ile Thr Pro Cys Ala
1               5                   10                  15


gcg gag gaa agc aag ctg ccc atc aac gcg ttg agc aac tct ttg ctg   96
Ala Glu Glu Ser Lys Leu Pro Ile Asn Ala Leu Ser Asn Ser Leu Leu
                20                  25                  30


cgc cac cat aac atg gtt tat gcc aca aca tct cgc agc gca ggc ctg   144
Arg His His Asn Met Val Tyr Ala Thr Thr Ser Arg Ser Ala Gly Leu
                35                  40                  45


cgg cag aag aag gtc acc ttt gac aga ctg caa gtc ctg gac gac cac   192
Arg Gln Lys Lys Val Thr Phe Asp Arg Leu Gln Val Leu Asp Asp His
        50                  55                  60


tac cgg gac gtg ctc aag gag atg aag gcg aag gcg tcc aca gtt aag   240
Tyr Arg Asp Val Leu Lys Glu Met Lys Ala Lys Ala Ser Thr Val Lys
65                  70                  75                  80
```

```
gct aaa ctc cta tcc gta gag gaa gcc tgc aag ctg acg ccc cca cat    288
Ala Lys Leu Leu Ser Val Glu Glu Ala Cys Lys Leu Thr Pro Pro His
                85                  90                  95


tcg gcc aaa tcc aag ttt ggc tat ggg gca aag gac gtc cgg aac cta    336
Ser Ala Lys Ser Lys Phe Gly Tyr Gly Ala Lys Asp Val Arg Asn Leu
                100                 105                 110


tcc agc aag gcc gtt aac cac atc cac tcc gtg tgg aag gac ttg ctg    384
Ser Ser Lys Ala Val Asn His Ile His Ser Val Trp Lys Asp Leu Leu
                115                 120                 125


gaa gac act gtg aca cca att gac acc acc atc atg gca aaa aat gag    432
Glu Asp Thr Val Thr Pro Ile Asp Thr Thr Ile Met Ala Lys Asn Glu
                130                 135                 140


gtt ttc tgt gtc caa cca gag aaa gga ggc cgt aag cca gcc cgc ctt    480
Val Phe Cys Val Gln Pro Glu Lys Gly Gly Arg Lys Pro Ala Arg Leu
145                 150                 155                 160


atc gta ttc cca gat ctg gga gtc cgt gta tgc gag aag atg gcc ctc    528
Ile Val Phe Pro Asp Leu Gly Val Arg Val Cys Glu Lys Met Ala Leu
                165                 170                 175


tat gat gtg gtc tcc acc ctt cct cag gtc gtg atg ggc tcc tca tac    576
Tyr Asp Val Val Ser Thr Leu Pro Gln Val Val Met Gly Ser Ser Tyr
                180                 185                 190
```

```
gga ttc cag tac tct cct ggg cag cga gtc gag ttc ctg gtg aat acc    624
Gly Phe Gln Tyr Ser Pro Gly Gln Arg Val Glu Phe Leu Val Asn Thr
        195                 200                 205
```

```
tgg aaa tca aag aaa aac ccc atg ggc ttt tca tat gac act cgc tgt    672
Trp Lys Ser Lys Lys Asn Pro Met Gly Phe Ser Tyr Asp Thr Arg Cys
    210                 215                 220
```

```
ttc gac tca acg gtc acc gag aac gac atc cgt gtt gag gag tca att    720
Phe Asp Ser Thr Val Thr Glu Asn Asp Ile Arg Val Glu Glu Ser Ile
225                 230                 235                 240
```

```
tac caa tgt tgt gac ttg gcc ccc gaa gcc aga cag gcc ata aaa tcg    768
Tyr Gln Cys Cys Asp Leu Ala Pro Glu Ala Arg Gln Ala Ile Lys Ser
        245                 250                 255
```

```
ctc aca gag cgg ctt tat atc ggg ggt cct ctg act aat tca aaa ggg    816
Leu Thr Glu Arg Leu Tyr Ile Gly Gly Pro Leu Thr Asn Ser Lys Gly
        260                 265                 270
```

```
cag aac tgc ggt tat cgc cgg tgc cgc gcg agc ggc gtg ctg acg act    864
Gln Asn Cys Gly Tyr Arg Arg Cys Arg Ala Ser Gly Val Leu Thr Thr
        275                 280                 285
```

```
agc tgc ggt aac acc ctc aca tgt tac ttg aag gcc tct gca gcc tgt    912
Ser Cys Gly Asn Thr Leu Thr Cys Tyr Leu Lys Ala Ser Ala Ala Cys
    290                 295                 300
```

```
cga gct gcg aag ctc cag gac tgc acg atg ctc gtg aac gga gac gac    960
```

276

```
Arg Ala Ala Lys Leu Gln Asp Cys Thr Met Leu Val Asn Gly Asp Asp
305                 310             315                 320


ctc gtc gtt atc tgt gaa agc gcg gga acc caa gag gac gcg gcg agc    1008
Leu Val Val Ile Cys Glu Ser Ala Gly Thr Gln Glu Asp Ala Ala Ser
                325                 330                 335


cta cga gtc ttc acg gag gct atg act agg tac tcc gcc ccc ccc ggg    1056
Leu Arg Val Phe Thr Glu Ala Met Thr Arg Tyr Ser Ala Pro Pro Gly
                340                 345                 350


gac ccg ccc caa cca gaa tac gac ttg gag ctg ata aca tca tgt tcc    1104
Asp Pro Pro Gln Pro Glu Tyr Asp Leu Glu Leu Ile Thr Ser Cys Ser
                355                 360                 365


tcc aat gtg tcg gtc gcc cac gat gca tca ggc aaa agg gtg tac tac    1152
Ser Asn Val Ser Val Ala His Asp Ala Ser Gly Lys Arg Val Tyr Tyr
                370                 375                 380


ctc acc cgt gat ccc acc acc ccc ctc gca cgg gct gcg tgg gag aca    1200
Leu Thr Arg Asp Pro Thr Thr Pro Leu Ala Arg Ala Ala Trp Glu Thr
385                 390                 395                 400


gct aga cac act cca gtt aac tcc tgg cta ggc aac att att atg tat    1248
Ala Arg His Thr Pro Val Asn Ser Trp Leu Gly Asn Ile Ile Met Tyr
                405                 410                 415


gcg ccc act ttg tgg gca agg atg att ctg atg act cac ttc ttc tcc    1296
Ala Pro Thr Leu Trp Ala Arg Met Ile Leu Met Thr His Phe Phe Ser
```

                    420                425                430

atc ctt cta gcg cag gag caa ctt gaa aaa gcc ctg gac tgc cag atc    1344
Ile Leu Leu Ala Gln Glu Gln Leu Glu Lys Ala Leu Asp Cys Gln Ile
        435                440                445


tac ggg gcc tgt tac tcc att gag cca ctt gac cta cct cag atc att    1392
Tyr Gly Ala Cys Tyr Ser Ile Glu Pro Leu Asp Leu Pro Gln Ile Ile
        450                455                460


gaa cga ctc cat ggc ctt agc gca ttt tca ctc cat agt tac tct cca    1440
Glu Arg Leu His Gly Leu Ser Ala Phe Ser Leu His Ser Tyr Ser Pro
465                470                475                480


ggt gag atc aat agg gtg gct tca tgc ctc agg aaa ctt ggg gta cca    1488
Gly Glu Ile Asn Arg Val Ala Ser Cys Leu Arg Lys Leu Gly Val Pro
                485                490                495


ccc ttg cga gtc tgg aga cat cgg gcc agg agc gtc cgc gct agg cta    1536
Pro Leu Arg Val Trp Arg His Arg Ala Arg Ser Val Arg Ala Arg Leu
                500                505                510


ctg tcc cag ggg ggg agg gcc gcc act tgt ggc aag tac ctc ttc aac    1584
Leu Ser Gln Gly Gly Arg Ala Ala Thr Cys Gly Lys Tyr Leu Phe Asn
        515                520                525


tgg gca gtg aag acc aaa ctc aaa ctc act cca atc ccg gct gcg tcc    1632
Trp Ala Val Lys Thr Lys Leu Lys Leu Thr Pro Ile Pro Ala Ala Ser
        530                535                540

```
cag ctg gac ttg tcc ggc tgg ttc gtt gct ggt tac agc ggg gga gac    1680
Gln Leu Asp Leu Ser Gly Trp Phe Val Ala Gly Tyr Ser Gly Gly Asp
545             550             555             560


ata tat cac agc ctg tct cgt gcc cga ccc cgc gga tcc cat cac cat    1728
Ile Tyr His Ser Leu Ser Arg Ala Arg Pro Arg Gly Ser His His His
            565             570             575


cac cat cac taa taa                                                1743
His His His
        580
```

<210> 3

<211> 579

<212> PRT

<213> Artificial Sequence

<223> Description of Artificial Sequence:DNA encoding
        fusion protein consisting of a portion of HCV
        polymerase and  histidine tag at the C-terminus

<400> 3

```
Met Ser Met Ser Tyr Thr Trp Thr Gly Ala Leu Ile Thr Pro Cys Ala
1               5               10              15
Ala Glu Glu Ser Lys Leu Pro Ile Asn Ala Leu Ser Asn Ser Leu Leu
            20              25              30
Arg His His Asn Met Val Tyr Ala Thr Thr Ser Arg Ser Ala Gly Leu
        35              40              45
```

Arg Gln Lys Lys Val Thr Phe Asp Arg Leu Gln Val Leu Asp Asp His
        50              55              60

Tyr Arg Asp Val Leu Lys Glu Met Lys Ala Lys Ala Ser Thr Val Lys
    65              70              75              80

Ala Lys Leu Leu Ser Val Glu Glu Ala Cys Lys Leu Thr Pro Pro His
                85              90              95

Ser Ala Lys Ser Lys Phe Gly Tyr Gly Ala Lys Asp Val Arg Asn Leu
            100             105             110

Ser Ser Lys Ala Val Asn His Ile His Ser Val Trp Lys Asp Leu Leu
        115             120             125

Glu Asp Thr Val Thr Pro Ile Asp Thr Thr Ile Met Ala Lys Asn Glu
    130             135             140

Val Phe Cys Val Gln Pro Glu Lys Gly Gly Arg Lys Pro Ala Arg Leu
145             150             155             160

Ile Val Phe Pro Asp Leu Gly Val Arg Val Cys Glu Lys Met Ala Leu
            165             170             175

Tyr Asp Val Val Ser Thr Leu Pro Gln Val Val Met Gly Ser Ser Tyr
            180             185             190

Gly Phe Gln Tyr Ser Pro Gly Gln Arg Val Glu Phe Leu Val Asn Thr
            195             200             205

Trp Lys Ser Lys Lys Asn Pro Met Gly Phe Ser Tyr Asp Thr Arg Cys
    210             215             220

Phe Asp Ser Thr Val Thr Glu Asn Asp Ile Arg Val Glu Glu Ser Ile
225             230             235             240

Tyr Gln Cys Cys Asp Leu Ala Pro Glu Ala Arg Gln Ala Ile Lys Ser
            245             250             255

Leu Thr Glu Arg Leu Tyr Ile Gly Gly Pro Leu Thr Asn Ser Lys Gly
            260             265             270

Gln Asn Cys Gly Tyr Arg Arg Cys Arg Ala Ser Gly Val Leu Thr Thr

275 280 285
Ser Cys Gly Asn Thr Leu Thr Cys Tyr Leu Lys Ala Ser Ala Ala Cys
290 295 300
Arg Ala Ala Lys Leu Gln Asp Cys Thr Met Leu Val Asn Gly Asp Asp
305 310 315 320
Leu Val Val Ile Cys Glu Ser Ala Gly Thr Gln Glu Asp Ala Ala Ser
325 330 335
Leu Arg Val Phe Thr Glu Ala Met Thr Arg Tyr Ser Ala Pro Pro Gly
340 345 350
Asp Pro Pro Gln Pro Glu Tyr Asp Leu Glu Leu Ile Thr Ser Cys Ser
355 360 365
Ser Asn Val Ser Val Ala His Asp Ala Ser Gly Lys Arg Val Tyr Tyr
370 375 380
Leu Thr Arg Asp Pro Thr Thr Pro Leu Ala Arg Ala Ala Trp Glu Thr
385 390 395 400
Ala Arg His Thr Pro Val Asn Ser Trp Leu Gly Asn Ile Ile Met Tyr
405 410 415
Ala Pro Thr Leu Trp Ala Arg Met Ile Leu Met Thr His Phe Phe Ser
420 425 430
Ile Leu Leu Ala Gln Glu Gln Leu Glu Lys Ala Leu Asp Cys Gln Ile
435 440 445
Tyr Gly Ala Cys Tyr Ser Ile Glu Pro Leu Asp Leu Pro Gln Ile Ile
450 455 460
Glu Arg Leu His Gly Leu Ser Ala Phe Ser Leu His Ser Tyr Ser Pro
465 470 475 480
Gly Glu Ile Asn Arg Val Ala Ser Cys Leu Arg Lys Leu Gly Val Pro
485 490 495
Pro Leu Arg Val Trp Arg His Arg Ala Arg Ser Val Arg Ala Arg Leu
500 505 510

281

Leu Ser Gln Gly Gly Arg Ala Ala Thr Cys Gly Lys Tyr Leu Phe Asn
            515                 520                 525

Trp Ala Val Lys Thr Lys Leu Lys Leu Thr Pro Ile Pro Ala Ala Ser
            530                 535                 540

Gln Leu Asp Leu Ser Gly Trp Phe Val Ala Gly Tyr Ser Gly Gly Asp
545                 550                 555                 560

Ile Tyr His Ser Leu Ser Arg Ala Arg Pro Arg Gly Ser His His His
                565                 570                 575

His His His


<210> 4

<211> 30

<212> DNA

<213> Artificial Sequence


<220>

<223> Description of Artificial Sequence:Artificially
      synthesized primer sequence, 5BNde1FW.


<220>

<221> primer_bind

<222> (1)..(30)


<400> 4

catatgtcaa tgtcctacac atggacagcc                                    30

<210> 5

<211> 57

<212> DNA

<213> Artificial Sequence


<220>

<223> Description of Artificial Sequence:Artificially
      synthesized primer sequence, 5B570HRV.


<220>

<221> primer_bind

<222> (1)..(57)


<400> 5

ttattagtga tggtgatggt gatgggatcc gcggggtcgg gcacgagaca ggctgtg       57


<210> 6

<211> 57

<212> DNA

<213> Artificial Sequence


<220>

<223> Description of Artificial Sequence:Artificially
      synthesized primer sequence, 5B552HRV.


<220>

<221> primer_bind

<222> (1)..(57)

<400> 6

ttattagtga tggtgatggt gatgggatcc aacgaaccag ccggacaagt ccagctg    57

<210> 7

<211> 57

<212> DNA

<213> Artificial Sequence

<220>

<223> Description of Artificial Sequence:Artificially
    synthesized primer sequence, 5B544HRV.

<220>

<221> primer_bind

<222> (1)..(57)

<400> 7

ttattagtga tggtgatggt gatgggatcc ctgggacgca gccgggattg gagtgag    57

<210> 8

<211> 67

<212> DNA

<213> Artificial Sequence

<220>

<223> Description of Artificial Sequence:Artificially

synthesized primer sequence, 5B536HRV.

<220>

<221> primer_bind

<222> (1)..(67)


<400> 8

ttattagtga tggtgatggt gatgggatcc gagtttgagt ttggtcttca ctgcccagtt 60

gaagagg                                                              67


<210> 9

<211> 60

<212> DNA

<213> Artificial Sequence


<220>

<223> Description of Artificial Sequence:Artificially
    synthesized primer sequence, 5B531HRV.


<220>

<221> primer_bind

<222> (1)..(60)


<400> 9

ttattagtga tggtgatggt gatgggatcc cttcactgcc cagttgaaga ggtacttgcc 60


<210> 10

<211> 52

<212> DNA

<213> Artificial Sequence


<220>

<223> Description of Artificial Sequence:Artificially
      synthesized primer sequence, 5B591HRV.


<220>

<221> primer_bind

<222> (1)..(52)


<400> 10

ttattaatgg tgatggtgat ggtgtccgga tcgattgggg agcaggtaga tg            52


<210> 11

<211> 8

<212> PRT

<213> Hepatitis C virus


<400> 11

Xaa Xaa Xaa Leu Xaa Xaa Trp Phe Xaa
1                   5


<210> 12

<211> 8

<212> PRT

<213> Hepatitis C virus

<400> 12
Lys Asp Leu Ser Gly Trp Phe Lys
1               5

<210> 13
<211> 9
<212> PRT
<213> Hepatitis C virus

<400> 13
Lys Lys Asp Leu Ser Gly Trp Phe Lys
1               5

<210> 14
<211> 8
<212> PRT
<213> Hepatitis C virus

<400> 14
Lys Asp Leu Ser Gly Trp Phe Val
1               5

<210> 15
<211> 8

<212> PRT

<213> Hepatitis C virus


<400> 15

Leu Asp Leu Ser Gly Trp Phe Lys

1              5



<210> 16

<211> 8

<212> PRT

<213> Hepatitis C virus


<400> 16

Leu Asp Leu Ser Gly Trp Phe Val

1              5



<210> 17

<211> 7

<212> PRT

<213> Hepatitis C virus


<400> 17

Asp Leu Ser Gly Trp Phe Val

1              5



<210> 18

<211> 6

<212> PRT

<213> Hepatitis C virus

<400> 18

Asp Leu Ser Gly Trp Phe

1                           5

<210> 19

<211> 6

<212> PRT

<213> Hepatitis C virus

<400> 19

Leu Ser Gly Trp Phe Val

1                           5

<210> 20

<211> 5

<212> PRT

<213> Hepatitis C virus

<400> 20

Leu Ser Gly Trp Phe

1                           5

<210> 21

<211> 6

<212> PRT

<213> Hepatitis C virus


<400> 21

Leu Ser Gly Trp Phe Lys

1                          5


<210> 22

<211> 6

<212> PRT

<213> Hepatitis C virus


<400> 22

Lys Leu Ser Gly Trp Phe

1                          5


<210> 23

<211> 5

<212> PRT

<213> Hepatitis C virus


<400> 23

Leu Gly Gly Trp Phe

1                          5

<210> 24

<211> 5

<212> PRT

<213> Hepatitis C virus


<400> 24

Leu Ser Asp Trp Phe

1               5


**Description**

FIELD OF THE INVENTION

The present invention relates to a polypeptide having the hepatitis C virus (HCV) polymerase activity, suitable for crystal structure analysis, and effective for evaluating the HCV polymerase activity and the use of the polypeptide.

More specifically, the present invention relates to a polypeptide having the HCV polymerase activity, which is obtained in the form of crystals suitable for crystal structure analysis and its crystals, as well as a DNA encoding the polypeptide. The present invention also relates to: (a) a method for determining structural coordinates for a cocomplex or a variant of the polypeptide (NS5B), (b) a method for identifying HCV polymerase inhibitors from the complementarity of a test compound with an active site and/or RNA binding cleft of the polypeptide, and (c) an HCV polymerase inhibitor obtained by the methods.

Moreover, the present invention relates to a method for identifying HCV polymerase inhibitors using the polypeptide that shows the polymerase activity higher than the wild-type HCV polymerase.

BACKGROUND OF THE INVENTION

Hepatitis C is a grave problem as it infects by blood transfusion and so on, and more than half of the cases become chronic with the high probability of progressing into cirrhosis and hepatoma. A cause of hepatitis C is known to be hepatitis C virus (HCV) and its gene was cloned in 1989 by the immunoscreening method using plasma of the chimpanzee infected with the human plasma (Science, 244, 359-362, 1989).

Hepatitis C virus is a plus strand RNA virus with an envelope and comprises the RNA encoding a protein consisting of 3010 amino acids. A precursor protein biosynthesized from the RNA in a host is processed into a structural protein forming viral particles (a core protein and two envelope proteins) and a non-structural protein (NS2, NS3, NS4A, NS4B, NS5A, NS5B) by a cellular signalase and a protease encoded by the RNA of the virus itself. It has been considered that NS2 and NS3 retain the protease activity and are necessary enzymes for processing a precursor protein, and the helicase of NS3 and RNA-dependent RNA polymerase of NS5 are essential for viral replication.

At present, interferon $\alpha$ and interferon $\beta$ are used for treating hepatitis C, however, they are less or no effective for many patients. A more effective drug for the treatment is thus needed. Developing novel HCV inhibitors is underway, and attention is focused on studies on the inhibitors by targeting proteins specific for HCV, such as protease, helicase, RNA-dependent RNA polymerase.

An inhibitor for viral proliferation is generally screened by measuring activity of inhibiting viral proliferation *in vitro* or *in vivo*. As to HCV, however, techniques for effecting the viral proliferation *in vitro* has not been established yet. Moreover, the screening of the viral proliferation of HCV in hosts is difficult because the virus only infects cells of human and chimpanzee.

Therefore, in development of anti-HCV drugs, developing an inhibitor by targeting specific coding proteins essential for the HCV proliferation is of great significance and the efficient assay method is desired.

In developing inhibitors for enzyme activity, molecular designing of inhibitors has been attempted by computers based on three-dimensional structure of enzymes to enhance the screening efficiency. In this methodology, various candidate compounds are designed and identified by tentatively evaluating the inhibitory activity against the enzyme activity of the candidate compounds by computer, considering the three-dimensional structures of various candidate compounds and physical properties of the molecule. The inhibitors can be identified more efficiently by combining designing and evaluation of the inhibitors using the three-dimensional structures of these enzymes, and evaluation of the enzyme activity in actually synthesized compounds.

In order to design molecules of inhibitors by computers, three-dimensional structure of an enzyme must be revealed. Three-dimensional structure of the enzyme can be clarified by X-ray crystal structure analysis. For example, the crystal structures of HIV reverse transcriptase (Nature Structural Biology, 2, 293-302, 1995; Structure, 3, 365-379, 1995), interleukin-1$\beta$ transformation enzyme (WO95/35367), protease of cytomegalovirus (WO97/42311), HCV helicase (WO99/09148), etc., have been analyzed.

An enzyme is a macromolecular compound, and its structure is complicated. X-ray structure analysis requires crystallization of the enzyme to strengthen diffraction intensity obtained by X-ray radiation. The structure cannot be fully analyzed unless the enzyme is stably crystallized and the crystals are produced in a large amount. Thanks to the development of the recombinant technique, a large amount of enzymes can be homogeneously and highly purified. However, it is difficult to obtain enzyme crystals suitable for X-ray analysis, and if at all, the structure cannot be completely analyzed in many cases. For example, the reported crystal structure of poliovirus RNA-dependent RNA polymerase (Structure 5, 1109-1122, 1997) is not complete, and only some parts have been analyzed, presumably because partial structures in the crystallized enzyme are disordered, and the protein has no stable structure.

Based on such a background, the results of the studies on crystal structure analysis of the HCV polymerase have been recently published. In Nature Structural Biology, 6 (10), 937-43, (Oct., 1999), the three-dimensional structure of NS5B was analyzed by adding hexahistidine tag to the N-terminus of NS5B consisting of 570 amino acids and analyzing it with X-ray. In Proc. Natl. Acad. Sci. USA, 96 (23), 13034-39 (Nov. 1999), the sequence of 531 amino acids of NS5B was disclosed and the three-dimensional structure of NS5B was analyzed in the same manner. However, both of these references were published after the priority date of the present application. Moreover, the references did not describe that the HCV polymerase obtained showed activity higher than the activity of the wild type; the references neither disclose nor suggest usefulness of the HCV polymerase for the actual enzyme activity evaluation.

WO99/43792 discloses a novel HCV polymerase useful for evaluating enzyme activity, which is NS5B consisting of 570 amino acids and its variant having the glutathione S-transferase tag sequence at the N-terminus.

However, the publication did not disclose or suggest that the polymerase was useful for X-ray structure analysis or suitable for crystallization. There was no description or suggestion about a method for identifying HCV polymerase inhibitors using the three-dimensional structure.

It has been implied that the C-terminal structure of the HCV polymerase may effect self-inhibition against the replication of RNA (Structure, 7 (11), 1417-26, (Nov. 1999)). In fact, the C-terminus-deficient variant has reportedly high RNA-dependent RNA polymerase activity (Journal of Virology, 73, 1649-54, Feb. 1999, and Journal of General Virology, 81, 759-767, 2000). In the former reference, however, $NS5B_{536}$ (NS5B consisting of 536 amino acids in which the C-terminus of the full length NS5B is truncated, same hereafter) and $NS5B_{528}$, showed only slightly higher activity compared with $NS5B_{570}$ (about 1.3 to 1.4 times). In the latter, activity of $NS5B_{591}$ (the full length NS5B) was compared with only that of $NS5B_{570}$. These references demonstrate that the truncated NS5B retained the polymerase activity, but do not propose any methods for evaluating inhibition for the HCV polymerase activity more efficiently by exploiting the high polymerase activity. Moreover, these references do not suggest any method for identifying compounds having the HCV polymerase-inhibitory activity more efficiently in combination with a method for identifying inhibitors based on the three-dimensional structure of the HCV polymerase.

SUMMARY OF THE INVENTION

An objective of the present invention is to provide a polypeptide having HCV polymerase activity, which is obtained in the form of crystals suitable for crystal structure analysis, the crystals, and a DNA encoding the polypeptide. Another objective of the present invention is to provide, (a) a method for determining structural coordinates of a cocomplex and a variant of the polypeptide, (b) a method for identifying HCV polymerase inhibitors based on the complementarity of a test compound with the active site and/or the RNA binding cleft of the polypeptide, and using the structural coordinate, and (c) HCV polymerase inhibitors obtained by the above methods.

Still another objective of the present invention is to provide a polypeptide having polymerase activity higher than that of the wild-type HCV polymerase, a method for inhibiting the HCV polymerase using the polypeptide, and a method for identifying HCV polymerase inhibitors using the methods.

The present inventors extensively studied to find polypeptides having the HCV polymerase activity, which can be obtained in the form of crystals suitable for crystal structure analysis. The inventors discovered a desired polypeptide and successfully clarified its crystal structure to complete the present invention.

Specifically, the present invention is described in (1) to (16) below.

(1) A polypeptide derived from HCV polymerase NS5B having an HCV polymerase activity and consisting of an amino acid sequence X-Y, wherein X is a consecutive amino acid sequence which is a portion of the NS5B, an N-terminal amino acid of X is the amino acid residue 1 (Ser) of the NS5B, and a C-terminal amino acid residue of X is any one of amino acid residues 531 (Lys) to 570 (Arg) of the NS5B; and wherein Y is a carboxyl group or an amino acid sequence which is not derived from NS5B; and one or more amino acids in the amino acid sequence of X may be modified, and methionine residues in the amino acid sequence of X may be replaced by selenomethionoine residues.

(2) The polypeptide of (1), wherein the C-terminal amino acid residue of X is any one of amino acid residues 536 (Leu) to 552 (Val) of the NS5B.

(3) The polypeptide of (2), wherein the C-terminal amino acid residue of X is any one of amino acid residues 536 (Leu) to 544 (Gln) of the NS5B.

(4) The polypeptide of (2), wherein the C-terminal amino acid residue of X is any one of amino acid residues 531 (Lys) to 544 (Gln) of the NS5B.

(5) The polypeptides of any one of (1) to (4), wherein methionine residues in the amino acid sequence of X are replaced by selenomethionine residues.

(6) The polypeptides of any one of (1) to (5), wherein Y is an amino acid sequence not derived from NS5B, and said amino acid sequence is suitable for a column purification.

(7) The polypeptides of any one of (1) to (6), wherein the NS5B comprises an amino acid sequence of SEQ ID NO: 1.

(8) The polypeptide of (1), wherein said polypeptide is identified by an three-dimensional structural coordinates shown in Tables 2 or 3.

(9) A crystal comprising the polypeptide of any one of (1) to (8).

(10) A DNA encoding the polypeptide of any one of (1) to (8).

(11) A method for determining a three-dimensional structural coordinates of a cocomplex or a variant of HCV polymerase NS5B by the molecular replacement method using a three-dimensional structure coordinate of said NS5B.

(12) A method for designing or identifying HCV polymerase inhibitors, which comprises determining the complementarity of a test compound with an active site and/or RNA binding deft of a polypeptide using the three-dimensional structural coordinate of said polypeptide or its part and the three dimensional structural coordinate of the test compound, wherein said polypeptide is derived from the HCV polymerase NS5B having an HCV polymerase activity and consisting of an amino acid sequence X-Y, wherein X is a consecutive amino acid sequence which is a portion of the NS5B, an N-terminal amino acid of X is amino acid residue 1 (Ser) of the NS5B, a C-terminal amino acid residue of X is any one of amino acid residues 531 (Lys) to 570 (Arg) of the NS5B: and wherein Y is a carboxyl group or another amino acid sequence which is not derived from NS5B; and one or more amino acids in X may be modified, and methionine residues in the amino acid sequence of X may be replaced by selenomethionine residues.

(13) A method for designing or identifying HCV polymerase inhibitors, which comprises the steps of:

(a) determining the complementarity of a test compound with an active site and/or RNA binding cleft of the a polypeptide using a three-dimensional structural coordinate of said polypeptide or its part and a three-dimensional structural coordinate of said test compound, wherein said polypeptide is derived from the HCV polymerase NS5B having an HCV polymerase activity and consisting of an amino acid sequence X-Y, wherein X is a consecutive amino acid sequence which is a portion of the NS5B, an N-terminal amino acid of X is the amino acid residue 1 (Ser) of the NS5B, a C-terminal amino acid residue of X is any one of amino acid residues 531 (Lys) to 570 (Arg) of the NS5B; and wherein Y is a carboxyl group of another amino acid sequence which is not derived from NS5B; and one or more amino acids in X may be modified, and methionine residues in the amino acid sequence of X may be replaced by selenomethionin residues.

(b) determining HCV polymerase-inhibitory activity of said test compound, and

(c) designing or determining HCV polymerase inhibitors using the complementarity data of said test compound determined in the above (a), and the inhibitory activity data obtained in the above (b).

(14) The method of any one of (11) to (13), wherein the three-dimensional structural coordinate of the polypeptide is any one of the three-dimensional structural coordinates shown in Table 2 or 3.

(15) A method for identifying HCV polymerase inhibitors, which comprises the steps of:

(a) obtaining a polypeptide, which is derived from the HCV polymerase NS5B has an HCV polymerase activity, and consisting of an amino acid sequence X'-Y, wherein X' is a consecutive amino acid sequence which is a portion of the NS5B, an N-terminal amino acid of X' is the amino acid residue 1 (Ser) of the NS5B, a C-terminal amino acid residue of X' is any one of amino acid residues 531 (Lys) to 544 (Gln) of the NS5B; and wherein Y is a carboxyl group or another amino acid sequence which is not derived from NS5B; and one or more amino acids in X' may be modified, and methionine residues in the amino acid sequence of X' may be replaced by selenomethionin residues;

(b) determining the HCV polymerase activity of said polypeptide by reacting said polypeptide obtained in the above (a) with a template RNA and substrates in the presence of a test compound;

(c) determining the HCV polymerase activity of said polypeptide by reacting the polypeptide obtained in the above (a) with a template RNA and substrates in the absence of said test compound; and

(d) comparing the HCV polymerase activity of the above (b) with the HCV polymerase activity of the above (c).

(16) An HCV polymerase inhibitor, identified by the method in any one of (12) to (15).

(17) An HCV polymerase inhibitor that inhibits the HCV polymerase activity of HCV polymerase NS5B by acting the boundary between the Thumb and Palm domains of NS5B.

(18) The HCV polymerase inhibitor of (17), wherein said inhibitor is a polypeptide represented by the formula (I) or a pharmaceutically acceptable salf thereof:

$$Z^1\text{-}Z^2\text{-}Z^3\text{-Leu-}Z^4\text{-}Z^5\text{-Trp-Phe-}Z^6 \tag{I}$$

wherein $Z^1$ and $Z^6$ each represent a hydrophilic group or an amino acid residue; $Z^2$ and $Z^3$ each represent a single bond or an amino acid residue; and $Z^4$ and $Z^5$ each represent an amino acid residue.

The terms used herein have the following meanings.

"HCV" is an abbreviation for Hepatitis C Virus.

"HCV polymerase" means RNA-dependent RNA polymerase encoded by RNA of HCV. It means not only a specific sequence but also a polypeptide derived from HCV polymerase NS5B, including any specific HCV types, any genotypes, and any mutants having the polymerase activity.

The HCV polymerase includes a polypeptide derived from the HCV polymerase NS5B, which has a polymerase activity and consisting of an amino acid sequence X - Y, wherein X is a consecutive amino acid sequence which is a portion of the NS5B, an N-terminal amino acid of X is the amino acid residue 1 (Ser) of the NS5B, and a C-terminal amino acid residue of X is any one of the amino acid residues from 531 (Lys) to 570 (Arg) of the NS5B; and wherein Y is a carboxyl group or an amino acid sequence which is not derived from NS5B; and one or more amino acids in the amino acid sequence of X may be modified, and methionine residues in the amino acid sequence of X may be replaced by selenomethionoine residues.

Except as otherwise set forth herein, specific amino acid position(s) indicated in the present specification means the position(s) in NS5B amino acid sequence.

The amino acid sequence of SEQ ID NO: 1 is the HCV polymerase of HCV-BK strain belonging to HCV1b genotype, commonly observed worldwide, especially in Asia and Europe. Therefore it is a useful HCV polymerase in this invention.

"HCV polymerase NS5B" and "NS5B" mean nonstructural proteins encoded by the nonstructual 5B (NS5B) region of the HCV RNA, which is located at the C-terminus of the viral protein (open reading frame) and possess RNA-dependent RNA polymerase activity. Herein, "HCV polymerase NS5B" and "NS5B" are synonyms with each other, and are not restricted to a specific wild-type NS5B.

NS5B is digested with a protease of the HCV itself. For example, in the case of the HCV-BK strain, NS5B is a polypeptide consisting of 591 amino acids from amino acid 2420 to the C-terminus in the viral protein (open reading frame).

A consecutive amino acid sequence 1 - 531 in NS5B is also designated as $NS5B_{531}$, and other consecutive amino acids are designated in the same manner.

"HCV polymerase activity" means the function of the above HCV polymerase to amplify RNA.

"HCV polymerase-inhibitory activity" means the function to inhibit the above HCV polymerase activity.

"HCV polymerase inhibitor" means an agent having the activity of inhibiting the above HCV polymerase activity.

"Wild-type HCV polymerase" means full length RNA-dependent RNA polymerase encoded by the RNA of hepatitis C virus existing in nature.

"Native HCV polymerase" means the above HCV polymerase in which methionine is not replaced by selenomethionine.

"Substrate" means adenine, guanine, cytosine, uridine, and their ribonucleosides, preferably ribonucleoside triphosphates, ATP, GTP, CTP, and UTP.

"X is a consecutive amino acid sequence which is a portion of the NS5B, and an N-terminal amino acid of X is an amino acid residue 1 (Ser) of the NS5B, and a C-terminal amino acid residue of X is any one among amino acid residues 531 (Lys) to 570 (Arg)" specifically means all amino acid residues designated by consecutive amino acid residues 1 to 531, consecutive amino acid residues 1 to 532, consecutive amino acid residues 1 to 533, a consecutive amino acid residues 1 to 570, and so on.

To obtain crystals suitable for crystal structure analysis, a C-terminal amino acid residue of X is preferably any one among amino acid residues 531 to 570 of NS5B. As for specific polypeptide, $NS5B_{531}$, $NS5B_{536}$, $NS5B_{544}$, or $NS5B_{570}$ are preferable, $NS5B_{536}$, $NS5B_{544}$, or $NS5B_{570}$ are more preferable, and $NS5B_{544}$ and $NS5B_{570}$ are still more preferable. $NS5B_{544}$ is the most preferable because its crystals are of high quality.

In order to obtain NS5B having activity higher than the wild-type HCV polymerase, suitable for the enzyme assay, a C-terminal amino acid residue of X is preferably any amino acid residue among amino acid residues 531 to 554. Specifically, $NS5B_{531}$, $NS5B_{536}$, or $NS5B_{544}$ are preferable, and $NS5B_{544}$ is more preferable.

X' means the same amino acid residue as X except that a C-terminal amino acid residue is any amino acid residue among amino acid residues 531 to 554 of NS5B.

"One or more amino acids in the amino acid sequence of X may be modified" means that 1 to 20, preferably 1 to 10, and more preferably 1 to 5 amino acids may be modified in the amino acid sequence of X. The modification includes natural or artificial deletion, replacement, and addition, and modification of the N- and/or C-terminal amino acid sequences.

"Selenomethionine" means a substituted methionine in which a sulfur atom is replaced by a selenium atom.

"Amino acid sequence suitable for column purification" means any amino acid sequence that can specifically bind to a carrier used for an affinity column used for purification of the polypeptide. Such a sequence is preferably a sequence which can be easily cleaved after the purification with the affinity column and/or does not prevent crystallization of the polypeptide, and includes, for example, the histidine tag sequence, and the glutathione S-trasferase tag sequence. The histidine tag needs four or more histidines, and specifically includes - Gly - Ser - His - His - His - His - His - His, - Gly - Ser - His - His - Asp -His - His - His, etc.

In the formula (I), $Z^2$ is preferably a single bond, and $Z^1$, $Z^3$, $Z^4$, $Z^5$, and $Z^6$ are preferably any of amino acid residues.

Amino acid residues for $Z^2$, $Z^3$, $Z^4$, and $Z^5$, are preferably hydrophilic amino acid residues, including Gly, Ser, Thr, Cys, Tyr, Asn, Gln, Lys, His, Arg, Asp, and Glu. $Z^3$ is preferably Asp, $Z^4$ is preferably Ser, and $Z^5$ is preferably Gly.

An amino acid residue for $Z^1$ is preferably Leu or a hydrophilic amino acid residue, which includes the same examples as for $Z^2$, $Z^3$, $Z^4$, and $Z^5$. $Z^1$ is more preferably Leu or Lys, and still more preferably Lys.

An amino acid residue for $Z^6$ is preferably Val or a hydrophilic amino acid group, which includes the same examples as for $Z^2$, $Z^3$, $Z^4$, and $Z^5$. $Z^6$ is more preferably Val, Thr, Arg or Lys, and still more preferably Lys.

"Hydrophilic group" includes a hydroxyl group, a carboxyl group, an amino group, a dimethylamino group, a mercapto group, and so on, and also include a carboxyl group contained in a phenylalanine residue at the C-terminus of the polypeptide represented by the formula (I), and an amino group contained in the N-terminal residue of said polypeptide. A hydrophilic group for $Z^1$ is preferably an amino group, and that for $Z^6$ is preferably a carboxyl group.

Examples of the polypeptide represented by the formula (I) include:

Lys-Asp-Leu-Ser-Gly-Trp-Phe-Lys;

Lys-Lys-Asp-Leu-Ser-Gly-Trp-Phe-Lys;

Lys-Asp-Leu-Ser-Gly-Trp-Phe-Val;

Leu-Asp-Leu-Ser-Gly-Trp-Phe-Lys;

Leu-Asp-Leu-Ser-Gly-Trp-Phe-Val;

Asp-Leu-Ser-Gly-Trp-Phe-Val;

Asp-Leu-Ser-Gly-Trp-Phe;

Leu-Ser-Gly-Trp-Phe-Val;

Leu-Ser-Gly-Trp-Phe;

Leu-Ser-Gly-Trp-Phe-Lys;

Lys-Leu-Ser-Gly-Trp-Phe;

Leu-Gly-Gly-Trp-Phe;

Leu-Ser-Asp-Trp-Phe; etc.

The polypeptide is preferably

Lys-Asp-Leu-Ser-Gly-Trp-Phe-Lys and
Leu-Asp-Leu-Ser-Gly-Trp-Phe-Val,

and more preferably

Lys-Asp-Leu-Ser-Gly-Trp-Phe-Lys.

"Three-dimensional structural coordinate" and "structural coordinate" of the HCV polymerase NS5B are synonymously used, and the structural coordinate includes "structural coordinate substantially equivalent" to "structural coordinate" of the NS5B.

"Structural coordinate" is a mathematical coordinate obtained by converting diffraction intensity at each diffraction point obtained by X-ray diffraction by electrons contained in atoms of the NS5B in the crystal form, into a numerical value, and analyzing the result. It presents locations of atoms in the NS5B expressed as a three-dimensional coordinate. Specifically, examples are the structural coordinates shown in Tables 2 and 3 of Example 2.

"Substantially equivalent structural coordinate" means a derivative structural coordinate generated as a result of artificially processing the structural coordinate of the NS5B or its part by computers or the like means. When the derivative structural coordinate of the NS5B is overlapped on the structural coordinate shown in Tables 2 or 3 so as to fit the locations of the corresponding atoms, residual mean square deviation is preferably within ± 0.5 Å or less, and more preferably, ± 0.2 Å or less from an original atom. A structural coordinate is preferably that of NS5B having the polymerase activity. Two structural coordinates show the identical three-dimensional structure when the locations of the corresponding atoms included in the structural coordinates can be overlapped, even if the numerical values of the coordinates indicating the locations of the atoms are different.

"To identify" means not only determining one, but selecting the less from the more.

"Molecular replacement method" is a method for determining the crystal structure of a protein whose structure is unknown based on the structure of a known protein with the same function as an initial model. Specific procedures are described in Experimental Chemistry Course 10, Diffraction, Japanese Society of Chemistry, 260-263 (1992), or Methods in Enzymology 115, 55-77 (1985), edited by M. G. Rossman.

"Cocomplex" means a complex formed by HCV polymerase and a compound having or expectedly having the HCV polymerase-inhibitory activity, and includes a complex comprising RNA strands, substrates or a metal essential for expression of the HCV polymerase activity, for example, manganese, magnesium, etc. Cocomplexes include are those formed by cocrystals, and those formed by soaking the HCV polymerase crystals in a solution containing the compound having or expectedly having the HCV polymerase-inhibitory activity.

"Active site" means (1) the region of the HCV polymerase in which a template RNA is replicated, formed by Asp at positions 220, 318 and 319, Lys 144, and Arg 158 in the amino acid sequence of the HCV polymerase, and/or (2) a hydrophilic shallow hollow formed by Ser 282, Thr 287, and Asn 291.

"RNA binding cleft" means a portion of the HCV polymerase, including an active site, and the inner space formed by the following Fingers, Palm and Thumb domains. It is a site that can be a target for identifying HCV polymerase inhibitors, including a space where a template RNA is incorporated when RNA is replicated. "RNA binding cleft" used herein differs from an active site.

Specific examples of the "RNA binding cleft" includes "inner space of the Palm domain" and "boundary site between the Thumb and Palm domains", other than the above "active site."

"Inner space of the Palm domain" is not an RNA replication site, but a space generated between the HCV polymerase and a template RNA when RNA is replicated using RNA of HCV as a template, and formed by the regions of amino acid residues 197 to 223, 310 to 325, and 348 to 366. These regions may be shifted 1 to 20, preferably 1 to 10, and more preferably 1 to 5 amino acids, to the N- or C-terminal side.

"Boundary site between the Thumb and Palm domains" is not an RNA replication site, but a site to which the C-terminus of $NS5B_{570}$ binds, and which can be a target for identifying HCV polymerase inhibitors, comprising the hydrophobic surface existing at the boundary between the Thumb and Palm domains described below. Specifically, the site is formed by amino acid Ser 196, Pro 197, Ile 413, Met 414, Ile 447, Tyr 448, Tyr 452, Ile 454, Ile 462, and Leu 466 in the amino acid sequence of the HCV polymerase or a part of it.

A site involved in "RNA binding cleft" includes Lys 90, 98, 106, and 172 and Arg 168 in the Holder domain, and Arg 465 in the Thumb domain, which have an important role in binding of RNA strands.

A preferable site that can be a target for identifying HCV polymerase inhibitors includes "active site" and "boundary site between Thumb and Palm domains."

"A part of a structural coordinate" means the structural coordinate including all or some of structures shown in the above "active site" and "RNA binding cleft" among the structural coordinates of NS5B.

"Complementarity of a test compound with an active site and/or RNA binding cleft of the polypeptide" is determined by calculating the condition under which the test compound conformationally or physically interacts with an active site and/or RNA binding cleft, and converting binding stability of the test compound to the site into a numerical value or visualizing the binding stability. Especially, it is preferable to obtain the complementarity of the surface structure of the test compound with the surface structure of the polypeptide by calculating conformational or static complementarity.

Complementarity can be compared between unknown compounds A and B, between a known compound A with a known enzyme-inhibitory activity and an unknown compound A, between known compounds A and B, and between any compounds.

"Acting on the boundary site between the Thumb and Palm domains" means that an HCV polymerase inhibitor binds to said site of HCV polymerase NS5B physically, chemically, statically, or in a similar manner, but is not limited to these modes of action.

Any patents, patent applications, and publications cited herein are incorporated by reference.

BRIEF DESCRIPTION OF THE DRAWINGS

Figure 1 shows the ribbon model of the three-dimensional structure obtained by visualizing the structural coordinate of the HCV polymerase ($NS5B_{570}$) using the program software RasMol.

Figure 2 schematically shows the three-dimensional structure of the HCV polymerase ($NS5B_{570}$). The $\alpha$ helices and $\beta$ sheets are indicated sequentially in alphabetical and numerical order, respectively.

Figure 3 compares amino acid sequences of HCV polymerase, poliovirus polymerase, and HIV reverse transcriptase.

Figure 4 shows the three-dimensional structure of the HCV polymerase $NS5B_{570}$, emphasizing the polypeptide region at positions 547 to 556. The right figure corresponds to the left figure rotated 90 degree upward.

DETAILED DESCRIPTION OF THE INVENTION

The present invention is illustrated in detail below.

i) HCV polymerase suitable for crystal structure analysis and its gene

The HCV polymerase suitable for crystal structure analysis of the present invention can be prepared by the standard methods of recombinant techniques.

For example, a DNA encoding a polypeptide derived from the HCV polymerase NS5B is inserted into a vector. The polypeptide has the HCV polymerase activity and comprises an amino acid sequence X-Y, in which X is a consecutive amino acid sequence which is a part of NS5B, an N-terminal amino acid of X is the amino acid residue 1 (Ser) of NS5B, and a C terminal amino acid residue of X is any one of the amino acid, residues from 531 (Lys) to 570 (Arg) of NS5B; Y is a carboxyl group or an amino acid sequence which is not derived from NS5B; and one or more amino acids in the amino acid sequence of X may be modified, and methionine of the amino acid sequence of X may be replaced by selenomethionoine. The vector is used to transform *E. coli*, and the resulting transformants axe cultured to isolate the polypeptide.

The HCV polymerase "suitable for crystal structure analysis" should be easily purified.

One embodiment of the HCV polymerase is a variant of the HCV polymerase in which a part of the amino acids are replaced by amino acids suitable for column purification. The replaced sequence makes the purification of the polypeptide easy and its large quantity production possible. An amino acid sequence of the HCV polymerase, suitable for crystallization, is preferably a sequence in which the amino acids suitable for column purification can be readily cleaved after column purification and/or which does not prevent crystallization of the HCV polymerase. Purification without using a surfactant is very preferable in crystallization after the purification.

Furthermore, the HCV polymerase "suitable for crystal structure analysis" should be easily crystallized, made into crystals with high quality, which are hardly degraded. "Being easily crystallized" means that manipulation for crystallization is easy, conditions for crystallization are simple, and that crystals can be obtained with no or little influence by a small difference of conditions for crystallization, for example, temperature and kinds, concentration, pH of a solvent, and so on. "Crystals with high quality" means less lattice fault and larger crystals. "Being hardly degraded" means, for example, that the crystals are not easily dissolved according to the change of, for example, temperature and kinds, concentration, and pH of a solvent.

The crystals should have smaller fluctuation in a whole molecule or a part of the molecule of the crystallized polymerase.

The crystals of the obtained HCV polymerase can be grown, for example, by vapor diffusion, to be used for crystal structure analysis.

A cocrystal comprising the polymerase and a compound having the HCV polymerase-inhibitory activity can be used for crystal structure analysis of the cocomplex. Crystals prepared by soaking the polymerase crystal in a solution of the compound can also be used for crystal structure analysis of the cocomplex.

It is generally known that when an amino acid sequence of a physiologically active protein is slightly modified, for example, by deletion or substitution of one or more amino acids in the amino acid sequence, or addition of one or more amino acids to the amino acid sequence, the physiological activity of the protein may be retained. Not only artificial manipulation but also spontaneous mutation may retain the activity.

A polypeptide modified by deletion, substitution, or addition of amino acid(s), can be prepared by, for example, subjecting the gene encoding the polypeptide to the site-directed mutagenesis that is known in the art (for example, Nucl. Acid Research, 10 (20), 6487-6500, 1992). For example, the mutation can be performed by using synthetic oligonucleotide primers complementary to the DNA at a site where the corresponding polypeptide is to be modified.

In addition to the site-directed mutagenesis, known modification methods include a method in which a gene is treated with mutagen, or a method in which a gene is cleaved with a restriction enzyme, and a selected gene fragment is removed, added or replaced, and ligated.

A variant may include a conservatively substituted sequence. This indicates that a specific amino acid residue may be substituted by a residue with similar physiochemical properties. Unrestricted examples of the conservative substitution include the substitution among amino acid residues with an aliphatic chain, such as the substitution among Ile, Val, Leu, or Ala, or the substitution between Lys and Arg which are basic amino acids having a polar group.

The present invention provides a DNA encoding the HCV polymerase, which is a DNA encoding the polypeptide derived from the HCV polymerase NS5B, having the HCV polymerase activity and comprising the amino acid sequence X-Y, in which X is a consecutive amino acid sequence which is a part of NS5B, an N terminal amino acid of X is the amino acid residue 1 (Ser) of NS5B, and a C terminal amino acid residue of X is any one of the amino acid residues from 531 (Lys) to 570 (Arg) of NS5B; Y is a carboxyl group or an amino acid sequence which is not derived from NS5B; and one or more amino acids in the amino acid sequence of X may be modified, and methionine in the amino acid sequence of X may be replaced by selenomethionoine.

Since there are more than one codons which encode one amino acid, any DNA having a nucleotide sequence can be used as long as a desired amino acid sequence can be obtained. Therefore, the DNA of the present invention includes not only a DNA encoding an amino acid sequence of, for example, SEQ ID NO: 1, but also DNAs comprising any combinations of codons, which encode desired amino acid sequences.

ii) Crystal structure analysis of the HCV polymerase

An enzyme such as the HCV polymerase, has the complicated molecular structure, thus three-dimensional structures of its crystals and the molecule can be identified by analyzing the crystals with X-ray. The crystal structure analysis can be performed by, for example, the molecular replacement method, the multiple wavelength anomalous dispersion method, the multiple heavy atom isomorphous replacement method, and so on.

Crystal structure analysis by the multiple heavy atom isomorphous replacement method

The multiple heavy atom isomorphous replacement method is a method for analyzing the three-dimensional structure of a protein, comprising measuring X-ray diffraction intensity of native protein crystals to which a heavy atom/s is/are attached, and the native protein crystals to which a heavy atom is not introduced (a native protein), comparing the diffraction intensity data, and determining a phase angle for each diffraction for the crystals (Experimental Chemistry Course 10, Diffraction, Japanese Society of Chemistry, 253-260, 1992).

To analyze the crystal structure of the HCV polymerase by the multiple heavy atom isomorphous replacement method, a desired HCV polymerase is produced and purified by the above method.

The HCV polymerase in which a sulfur of Met was replaced with selenium was isolated and purified by producing the HCV polymerase by the above method in the medium in which selenomethionine was added in place of methionine (hereafter referred to as the selenomethionine HCV polymerase or the selenomethionine heavy atom substitution product).

Herein, the HCV polymerase obtained by the addition of Met may be expressed as a native HCV polymerase to distinguish from the selenomethionine HCV polymerase.

The crystals of the native HCV polymerase and the selenomethionine HCV polymerase can be obtained by vapor diffusion. A heavy atom substitution products of the native HCV polymerase can be prepared by soaking the crystals of the native and the native HCV polymerase in solutions containing platinum, uranium, and osmium. A structural coordinate can be determined by measuring diffraction intensity for the crystals of the obtained heavy atom substitution products and calculating the phase angle by the multiple heavy atom isomorphous replacement method.

According to the principle of the multiple heavy atom isomorphous replacement method, there is the relationship for each reflection of a structural factor from the crystals with a heavy atom (FPH), a structural factor of the native crystal data (FP), and contribution of the introduced heavy atom (FH):

$$FPH = FP + FH.$$

| FPH | and | FP | are the numerical values obtained from the experiment. From these data, the location of the heavy atom can be determined, and | FH | can be caluculated to determine the phase angle of each reflection. Subsequently, the structure of the HCV polymerase can be determined by obtaining the electron density.

These calculation can be performed using program software DENZO, Shelx, MLPHARE, SHARP, DM, O, etc.

It is known that in general even if a structural coordinate for the location of each atom is changed to some extent on a computer, the structure does not largely change and the protein activity is not inactivated. Therefore, the structure coordinate essentially equivalent to that for the HCV polymerase of the present invention includes derivative coordinates prepared by artificially processing the coordinate of the HCV polymerase. Such derivative coordinates preferably show residual mean square deviation within the range of $\pm$ 0.5 Å or less, and more preferably within the range of $\pm$ 0.2 Å or less, when the derivative coordinate is overlapped on the original structural coordinate so as to fit the locations of the atoms.

iii) Determination of "active site" and "RNA binding cleft" of the HCV polymerase

The active site of the HCV polymerase can be identified or deduced from the three-dimensional structure obtained by the crystal structure analysis of the HCV polymerase and the amino acid sequence. For identification and deduction, the amino acid sequence and the three-dimensional structure of a known polypeptide with the similar function can be referred.

From the obtained three-dimensional structure, a site which can be a target for inhibition of the HCV polymerase activity can be estimated in addition to the active site. The coordinate for the HCV polymerase obtained as a result of the structure analysis can be used, for example, for the following purposes:

(a) analysis of the crystal structure of the HCV polymerase variant;
(b) analysis of the crystal structure of the cocomplex composed of the HCV polymerase and the inhibitor; and,
(c) evaluation of the complementarity of a test compound with the active site and/or the RNA binding cleft of the HCV polymerase.

iv) Crystal structure analysis of a variant or a cocomplex of the HCV polymerase

Based on the structural coordinates for the HCV polymerase shown in Tables 2 and 3, the crystal structure of a variant or a cocomplex of HCV polymerase can be determined. The structural coordinate for the cocomplex can be important information for improving the quality of designing/evaluating a compound having the complementarity with the active site and/or the RNA binding cleft of the HCV polymerase.

In the molecular replacement method, rotational function is calculated from the crystal diffraction intensity data of the variant or the cocomplex of the HCV polymerase to determine the orientation of the molecule, and the location of the molecule is determined by calculating the translational function (Acta Crystallogr., 23, 544, 1967).

This method can be performed by using program software Amore of CCP4, Almn of CCP4 (Council for the Central Laboratory of the Research Councils), etc.

v) Designing of HCV polymerase inhibitors and evaluation of the HCV polymerase-inhibitory activity.

Since the active site of the HCV polymerase is the RNA replication site, a compound having the structural complementarity with the active site would inhibit the polymerase activity.

A compound having the complementarity with a site which can be a target for the inhibition of the HCV polymerase activity, for example, the RNA binding cleft, in addition to the active site, is presumed to indirectly inhibit the polymerase activity.

The inner space of the Palm domain is not involved in RNA replication, but presumably is the gap generated when the RNA is replicated. Therefore a compound having the structural complementarity with the inner space of the Palm domain is estimated to indirectly inhibit the polymerase activity.

Such three-dimensional structural information on the active site and the RNA binding cleft is important for designing/identifying HCV polymerase inhibitors by computers and such. Specifically, the complementarity of HCV polymerase inhibitors with the active site and/or RNA binding cleft, for example, the binding stability, can be compared by computers and the like means. A leading compound having the complementarity with the active site and/or the RNA binding cleft, and the derivative peripheral compounds can be rationally designed. Furthermore, in synthesis experiments, useless syntheses can be obviated, and actual evaluation of enzymes can be efficiently performed.

The complementarity with the active site and/or the RNA binding cleft can be determined, for example, by inputting the structural coordinates of the HCV polymerase and of a test compound to virtual screening programs, such as DOCK4 (UCSF), etc., using computers, and obtaining the state in which the test compound is incorporated into the active site and/or the RNA binding cleft of the HCV polymerase, as a numerical value stable in terms of conformation and energy, or as the visual model. Moreover, the complementarity of the test compound can be obtained using a part of the structural coordinate for the HCV polymerase in the same manner.

As a virtual screening program, FLEXY DOCK (Tripos) can be used in addition to DOCK4.

The structural coordinate for the test compound is available on a database for the three-dimensional structure of the chemical compounds. Alternatively the coordinate can be obtained by calculating the conformation using program software such as Quanta (MSI), Sybyl (Tripos), Insight II (MSI), etc.

The HCV polymerase-inhibitory activity can be evaluated by comparing the thus-obtained complementarity of the test compound with the active site and/or the RNA biding cleft of the HCV polymerase.

The molecule of an inhibitor can be designed so as to have the complementarity with the active site and/or the RNA binding cleft, based on the structure of the test compound. The molecules can be designed using the above program software Quanta, Sybyl, Insight II, DOCK4, FLEXY, DOCK, etc.

vi) Actual evaluation of the HCV polymerase-inhibitory activity.

The HCV polymerase-inhibitory activity can be measured by obtaining a compound having the complementarity with the active site and/or the RNA binding site of the HCV polymerase, evaluated by the above virtual screening, and contacting the obtained compound with the HCV polymerase in the presence of a template RNA and a substrate ribonucleoside triphosphate (rNTP).

The present invention enables designing or identifying HCV polymerase inhibitors by computers and such, and thus provides methodologies of rational designing of compounds and their analogues. Moreover, this invention enables efficiently evaluating the HCV polymerase-inhibitory activity, and thus provides efficient evaluation method of the HCV polymerase-inhibitory activity by the combination use of designing or identification by computers and the like means.

The present invention is illustrated in detail below with reference to the Examples, but is not construed being limited thereto.

## EXAMPLE 1

### Expression and purification of the native HCV polymerase (NC5B$_{570}$)

The DNA fragment comprising the histidine tag consisting of the amino acid sequence of GSHHHHHH at the C-terminus (SEQ ID NO: 2) of NS5B was prepared by PCR using pDM22 into which cDNA of HCV-BK type virus was introduced, purchased from Research Foundation for Microbial Diseases of Osaka University, as a template, and a set of primers 5BNde1FW (SEQ ID NO: 4) and 5B570HRV (SEQ ID NO: 5). The resulting fragment was inserted into pCR2.1 vector (INVITROGEN), the sequence was confirmed, and about 1.8 kDa fragment was obtained by partial digestion with *Nde*1 and *Eco*R1.

The thus-obtained fragment was inserted into the *Nde*1 and *Eco*R1 sites in pET17b vector comprising T7 promoter (NOVAGEN), and the vector was used to transform *E*. *coli* BL21 (DE3) (NOVAGEN).

The transformants were cultured in 2xYT medium at 30°C. When OD620 reached 0.8 to 1.0, IPTG (Nacalai Tesque) was added thereto to a final concentration of 0.5 mM, and the transformants were further incubated at 30°C for 3 hours to induce production of the target protein.

The harvested cells were disrupted with a microfluidizer and the soluble fraction was isolated and purified by subsequently performing Ni-NTA agarose (QIAGEN) column chromatography, Mono-S5/5 (PHARMACIA) column chromatography, and gel filtration with Sephacryl S-200 (PHARMACIA).

In the amino acid sequence of the obtained native HCV polymerase, methionine at the N-terminus was missing. The amino acid sequence of the obtained NS5B$_{570}$ was the amino acids 1 - 570 of the amino acid sequence shown in SEQ ID NO: 1, to which the histidine tag was added. In the same manner, NS5B$_{552}$, NS5B$_{544}$, NS5B$_{536}$, NS5B$_{531}$, and NS5B$_{591}$ were obtained using primers 5B552HRV (SEQ ID NO: 6), 5B544HRV (SEQ ID NO: 7), 5B536HRV (SEQ ID NO: 8), 5B531HRV (SEQ ID NO: 9), and 5B591HRV (SEQ ID NO: 10), respectively. The amino acid sequence of the histidine tag in the obtained NS5B$_{544}$ was GSHHDHHH.

NS5B$_{591}$ comprising the full length wild-type NS5B was purified by adding a detergent (CHAPS) and glycerol, and using the poly(U)-sepharose 4B (PHARMACIA) column chromatography in addition to the above column chromatographies.

### Expression and purification of selenomethionine heavy atom substitution product

In the same manner as in the expression and purification of the native HCV polymerase, the 1.8 kDa fragment obtained from pDM22 was inserted into *Nde*1 and *Eco*R1 sites of pET17b (NOVAGEN), which was used to transform *E*. *coli* B834 (DE3) (NOVAGEN).

The transformants were cultured in the medium for selenomethionine substitution mentioned below at 30°C. When OD620 reached 0.8 to 1.0, IPTG was added thereto to a final concentration of 0.5 mM, and the transformants were further cultured at 30 °C for 3 hours to induce the production of the target protein. The soluble fraction was purified in the same manner as the native HCV polymerase.

Composition of the medium for selenomethionine substitution

1. Amino acids (g/ml)

| Ala | 1.50 |
|---|---|
| Arg | 1.75 |
| Asp | 1.20 |
| Cys · HCl/$H_2O$ | 0.10 |
| Glu | 2.00 |
| Gln | 1.00 |
| Gly | 1.63 |
| His | 0.18 |
| Ile | 0.70 |
| Leu | 0.70 |
| Lys · HCl | 1.26 |
| Phe | 0.40 |
| Pro | 0.30 |
| Ser | 6.25 |
| Thr | 0.70 |
| Tyr | 0.50 |
| Val | 0.70 |

2. Salts (g/ml)

| Adenosine | 1.00 |
|---|---|
| Guanosine | 1.33 |
| Thymine | 0.33 |
| Uracil | 1.00 |
| Succinic acid | 3.00 |
| Na Acetate · $3H_2O$ | 1.50 |
| Ammonium-Cl | 1.50 |
| NaOH | 0.85 |
| $K_2HPO_4$ | 10.50 |

3. Metal, selenomethionine and others (g/ml)

| Mg $SO_4$ · $7H_2O$ | 0.25 |
|---|---|
| $FeSO_4$ · $7H_2O$ | 0.0042 |
| Glucose | 20.00 |
| Selenomethionine | 0.75 |

4. Vitamins

| KAO and MICHAYLUK BASAL VITAMIN solution | 10.00 ml/l |
|---|---|

Methionine at the N-terminus in the selenomethionine heavy atom substitution product was cleaved like in the native HCV polymerase. As a result of LC-MS, all 12 methionines were replaced by selenomethionines.

EXAMPLE 2

Crystallization of the HCV polymerase

The crystals of the native HCV polymerase obtained in Example 1 (NS5B$_{570}$) was prepared by vapor diffusion. Specifically, a mixture of the protein solution and a precipitation reagent solution (1:1 by volume), and a precipitation reagent solution were placed into a container that can be sealed so as not to contact the solutions with each other. The container was kept at the constant temperature for 2 to 4 weeks to obtain the crystals by vapor equilibration. The conditions for crystallization with good reproducibility are described below. To improve the rate of crystal growth in the solution, a detergent, such as n-dodecyl- β -D-maltoside, n-octanoylsucrose, and such, may be added to 0.1 to 0.3 CMC.

Conditions for crystallization of NS5B$_{570}$

Protein solution:
NS5B$_{570}$ dissolved in 5 mM dithiothreitol (DTT) solution to $10 \pm 5$ mg/ml.
Precipitation reagent solution:
containing 21 to 28% (w/v) polyethylene glycol 4000, 0.2 to 0.35 M ammonium acetate, 0.1 M sodium acetate, 0.02 M TES buffer (N-tris(hydroxymethyl)methyl-2-aminoethanesulfonic acid, pH 6.0 to 7.5).
Crystallization temperature:
$22 \pm 2$ °C

Conditions for crystallization of NS5B$_{544}$

Protein solution:
NS5B$_{544}$ dissolved in 5 mM dithiothreitol (DTT) solution to $10 \pm 3$ mg/ml.
Precipitation reagent solution:
the solution containing 2.0 to 5.0% (w/v) polyethylene glycol 8000, 5% (v/v) isopropanol, 0.1 M sodium citrate buffer (pH 5.5 to 6.5).

Crystallization temperature:
4 ± 2 °C

The crystals of NS5B$_{544}$, NS5B$_{536}$, and NS5B$_{531}$ were obtained in the same manner.

Table 1 shows the crystallographical parameters for NS5B$_{570}$ and NS5B$_{544}$. The crystallographical parameters for NS5B$_{536}$ and NS5B$_{531}$ were similar to those for NS5B$_{544}$.

Table 1

| Crystallographic parameters | | | |
|---|---|---|---|
| Crystal | Space group | Lattice constants | Number of independent molecule in asymmetric unit |
| NS5B$_{570}$ | P4$_3$2$_1$2 | a=b=63.7(±0.7) Å<br><br>c=262.9(±3.0) Å<br><br>$\alpha=\beta=\gamma=90°$ | 1 |
| NS5B$_{544}$ | P2$_1$2$_1$2 | a=67.6(±0.7) Å<br><br>b=95.9(±1.0) Å<br><br>c=97.6(±1.0) Å<br><br>$\alpha=\beta=\gamma=90°$ | 1 |

## EXAMPLE 3

### Crystal structure analysis of the HCV polymerase

The crystals of the native HCV polymerase (NS5B$_{570}$) were soaked in the precipitation reagent solution used in the crystallization of NS5B$_{570}$ described above containing heavy atoms, such as platinum, uranium, or osmium, to obtain the heavy atom substitution products.

The selenomethionine HCV polymerase was crystallized by vapor diffusion in the same manner described above.

Diffraction intensity of the obtained platinum heavy atom substitution product, uranium heavy atom substitution product, and osmium heavy atom substitution product of the native HCV polymerase crystal, the native polymerase crystal, as well as the selenomethionine HCV polymerase crystal were measured using Raxis IIc (Rigaku), and BL6B of synchrotron facility KEK-PF, and BL45XU of SPring-8.

The X-ray diffraction data for the platinum heavy atom substitution product, the uranium heavy atom substitution product, and the osmium heavy atom substitution product were processed with DENSO (HKL) and SCALA, FHSCAL, SCALEIT of CCP4 program (Council for the Central Laboratory of the Research Councils). The scale of data was adjusted to that of the diffraction intensity of the native HCV polymerase (NS5B$_{570}$) crystal so that the diffraction intensity of the products could be compared with each other. The first locations of the heavy atoms were determined processing the data of the uranium heavy atom substitution product, the osmium heavy atom substitution product, and the native HCV polymerase (NS5B$_{570}$) crystal with the program software Shelx (Professor Sheldrick; Crystallographic Computing 3, Clarendon Press, Oxford 184 - 189 (1985)). Subsequently, the accurate locations of each heavy atom were determined using the program software MLPHARE in CCP4 and SHARP (Laboratory of Molecular Biology) to calculate the initial phase angles. The improvement of the initial phase and expansion of the phase within 2.5 Å were calculated using the program software DM in CCP4 to prepare the Fourier map.

The selenomethionine HCV polymerase (NS5B$_{570}$) corresponds to the amino acid sequence of SEQ ID NO: 1 in which 12 methionine residues are replaced with selenomethionines. The differential Fourier map was prepared for this selenomethionine HCV polymerase using the phase information described above. The differential Fourier map of the diffraction data measured at the X-ray wave length $\lambda = 1.0400$Å, and the diffraction intensity data measured at $\lambda = 0.9797$ Å, in which 11 peaks corresponding to a selenium atom were confirmed, was used as a guide for the structure determination.

The structure of the HCV polymerase was determined based on the obtained Fourier map using the program software O (DatOno AB).

Refinement was performed using torsion angle or maximum likelihood refinement of the program software X-PLOR98 (MSI). Ramachandran plot obtained by using the program software PROCHECK (J. Appl. Cryst. 26, 283-290, 1993) confirmed that there was no amino acid residue with unacceptable structure. The structural coordinates were shown in Table 2. Each symbol in Tables means as follows.

(Atom type: from the left, the serial numbers of atoms contained in the coordinates, types of atoms and location of the atoms in amino acids, numbers: amino acid residue number of amino acids comprising the atoms, X, Y, and Z: coordinates of the atoms, Occ: occupancy, B: temperature factor)

The structural coordinate of $NS5B_{544}$, in $NS5B_{544}$, $NS5B_{536}$ and $NS5B_{531}$ obtained by the molecular replacement method was shown in Table 3. The structural coordinates of $NS5B_{536}$ and $NS5B_{531}$ were similar to that shown in Table 3.

Table 2

| | | atom type | | numbers | X | Y | Z | Occ | B |
|---|---|---|---|---|---|---|---|---|---|
| atom | 1 | CB | SER | 1 | 24.595 | 6.355 | -7.700 | 1.00 | 14.36 |
| atom | 2 | OG | SER | 1 | 24.403 | 6.849 | -6.395 | 1.00 | 33.57 |
| atom | 3 | C | SER | 1 | 24.205 | 3.980 | -7.261 | 1.00 | 18.80 |
| atom | 4 | O | SER | 1 | 23.777 | 3.593 | -6.173 | 1.00 | 22.06 |
| atom | 5 | N | SER | 1 | 22.311 | 5.540 | -7.354 | 1.00 | 17.96 |
| atom | 6 | CA | SER | 1 | 23.641 | 5.209 | -7.924 | 1.00 | 16.04 |
| atom | 7 | N | MET | 2 | 25.181 | 3.368 | -7.917 | 1.00 | 19.98 |
| atom | 8 | CA | MET | 2 | 25.812 | 2.175 | -7.382 | 1.00 | 17.12 |
| atom | 9 | CB | MET | 2 | 26.317 | 1.276 | -8.522 | 1.00 | 14.48 |
| atom | 10 | CG | MET | 2 | 25.215 | 0.731 | -9.392 | 1.00 | 12.07 |
| atom | 11 | SD | MET | 2 | 24.201 | -0.549 | -8.622 | 1.00 | 14.36 |
| atom | 12 | CE | MET | 2 | 25.522 | -1.728 | -8.316 | 1.00 | 15.13 |
| atom | 13 | C | MET | 2 | 26.976 | 2.612 | -6.501 | 1.00 | 16.76 |
| atom | 14 | O | MET | 2 | 27.731 | 3.510 | -6.858 | 1.00 | 19.33 |
| atom | 15 | N | SER | 3 | 27.092 | 1.988 | -5.341 | 1.00 | 16.24 |
| atom | 16 | CA | SER | 3 | 28.172 | 2.277 | -4.411 | 1.00 | 15.30 |
| atom | 17 | CB | SER | 3 | 28.103 | 1.275 | -3.252 | 1.00 | 10.19 |
| atom | 18 | OG | SER | 3 | 28.201 | -0.061 | -3.723 | 1.00 | 12.95 |
| atom | 19 | C | SER | 3 | 29.522 | 2.145 | -5.150 | 1.00 | 14.45 |
| atom | 20 | O | SER | 3 | 30.470 | 2.880 | -4.885 | 1.00 | 13.89 |
| atom | 21 | N | TYR | 4 | 29.593 | 1.196 | -6.075 | 1.00 | 14.55 |
| atom | 22 | CA | TYR | 4 | 30.808 | 0.968 | -6.849 | 1.00 | 19.90 |
| atom | 23 | CB | TYR | 4 | 31.730 | -0.024 | -6.161 | 1.00 | 23.29 |
| atom | 24 | CG | TYR | 4 | 32.040 | 0.242 | -4.734 | 1.00 | 25.80 |
| atom | 25 | CD1 | TYR | 4 | 31.257 | -0.317 | -3.714 | 1.00 | 23.72 |
| atom | 26 | CE1 | TYR | 4 | 31.615 | -0.171 | -2.388 | 1.00 | 23.05 |
| atom | 27 | CD2 | TYR | 4 | 33.180 | 0.958 | -4.387 | 1.00 | 23.13 |
| atom | 28 | CE2 | TYR | 4 | 33.542 | 1.108 | -3.067 | 1.00 | 26.87 |
| atom | 29 | CZ | TYR | 4 | 32.760 | 0.533 | -2.075 | 1.00 | 23.63 |
| atom | 30 | OH | TYR | 4 | 33.171 | 0.637 | -0.772 | 1.00 | 29.59 |
| atom | 31 | C | TYR | 4 | 30.620 | 0.399 | -8.248 | 1.00 | 16.78 |
| atom | 32 | O | TYR | 4 | 29.610 | -0.230 | -8.578 | 1.00 | 12.24 |

| | | | | | | | | | |
|------|----|-----|-----|---|--------|--------|---------|------|-------|
| atom | 33 | N   | THR | 5 | 31.653 |  0.599 |  -9.048 | 1.00 | 16.02 |
| atom | 34 | CA  | THR | 5 | 31.695 |  0.049 | -10.381 | 1.00 | 18.47 |
| atom | 35 | CB  | THR | 5 | 31.527 |  1.110 | -11.463 | 1.00 | 18.54 |
| atom | 36 | OG1 | THR | 5 | 30.136 |  1.399 | -11.615 | 1.00 | 14.33 |
| atom | 37 | CG2 | THR | 5 | 32.067 |  0.600 | -12.796 | 1.00 | 21.94 |
| atom | 38 | C   | THR | 5 | 33.076 | -0.534 | -10.449 | 1.00 | 16.89 |
| atom | 39 | O   | THR | 5 | 34.049 |  0.146 | -10.159 | 1.00 | 21.07 |
| atom | 40 | N   | TRP | 6 | 33.168 | -1.803 | -10.811 | 1.00 | 17.19 |
| atom | 41 | CA  | TRP | 6 | 34.469 | -2.441 | -10.892 | 1.00 | 15.52 |
| atom | 42 | CB  | TRP | 6 | 34.434 | -3.740 | -10.096 | 1.00 | 13.26 |
| atom | 43 | CG  | TRP | 6 | 33.924 | -3.575 |  -8.682 | 1.00 | 14.65 |
| atom | 44 | CD2 | TRP | 6 | 34.619 | -2.966 |  -7.586 | 1.00 |  7.71 |
| atom | 45 | CE2 | TRP | 6 | 33.795 | -3.085 |  -6.448 | 1.00 | 13.81 |
| atom | 46 | CE3 | TRP | 6 | 35.860 | -2.328 |  -7.459 | 1.00 | 12.05 |
| atom | 47 | CD1 | TRP | 6 | 32.734 | -4.026 |  -8.178 | 1.00 | 14.49 |
| atom | 48 | NE1 | TRP | 6 | 32.653 | -3.737 |  -6.831 | 1.00 | 18.73 |
| atom | 49 | CZ2 | TRP | 6 | 34.171 | -2.597 |  -5.202 | 1.00 | 14.72 |
| atom | 50 | CZ3 | TRP | 6 | 36.238 | -1.839 |  -6.220 | 1.00 | 13.25 |
| atom | 51 | CH2 | TRP | 6 | 35.392 | -1.976 |  -5.105 | 1.00 | 15.70 |
| atom | 52 | C   | TRP | 6 | 34.900 | -2.736 | -12.330 | 1.00 | 17.14 |
| atom | 53 | O   | TRP | 6 | 34.073 | -3.074 | -13.171 | 1.00 | 12.10 |
| atom | 54 | N   | THR | 7 | 36.190 | -2.577 | -12.616 | 1.00 | 19.22 |
| atom | 55 | CA  | THR | 7 | 36.697 | -2.923 | -13.936 | 1.00 | 19.75 |
| atom | 56 | CB  | THR | 7 | 38.062 | -2.269 | -14.251 | 1.00 | 18.95 |
| atom | 57 | OG1 | THR | 7 | 39.045 | -2.708 | -13.301 | 1.00 | 11.09 |
| atom | 58 | CG2 | THR | 7 | 37.945 | -0.729 | -14.230 | 1.00 |  7.76 |
| atom | 59 | C   | THR | 7 | 36.890 | -4.426 | -13.763 | 1.00 | 27.76 |
| atom | 60 | O   | THR | 7 | 35.956 | -5.142 | -13.383 | 1.00 | 30.65 |
| atom | 61 | N   | GLY | 8 | 38.092 | -4.919 | -14.005 | 1.00 | 32.30 |
| atom | 62 | CA  | GLY | 8 | 38.318 | -6.348 | -13.830 | 1.00 | 30.63 |
| atom | 63 | C   | GLY | 8 | 39.469 | -6.601 | -12.880 | 1.00 | 25.34 |
| atom | 64 | O   | GLY | 8 | 39.624 | -7.688 | -12.327 | 1.00 | 26.59 |
| atom | 65 | N   | ALA | 9 | 40.259 | -5.555 | -12.692 | 1.00 | 18.42 |
| atom | 66 | CA  | ALA | 9 | 41.429 | -5.567 | -11.852 | 1.00 | 20.57 |
| atom | 67 | CB  | ALA | 9 | 41.998 | -4.159 | -11.757 | 1.00 | 21.71 |
| atom | 68 | C   | ALA | 9 | 41.237 | -6.140 | -10.454 | 1.00 | 25.44 |

| atom | 69 | O | ALA | 9 | 40.258 | -5.864 | -9.739 | 1.00 | 28.28 |
|------|-----|-----|-----|-----|--------|---------|---------|------|-------|
| atom | 70 | N | LEU | 10 | 42.212 | -6.942 | -10.074 | 1.00 | 23.65 |
| atom | 71 | CA | LEU | 10 | 42.230 | -7.567 | -8.784 | 1.00 | 24.29 |
| atom | 72 | CB | LEU | 10 | 43.171 | -8.770 | -8.827 | 1.00 | 23.79 |
| atom | 73 | CG | LEU | 10 | 42.615 | -10.071 | -9.422 | 1.00 | 21.37 |
| atom | 74 | CD1 | LEU | 10 | 42.033 | -10.895 | -8.285 | 1.00 | 22.12 |
| atom | 75 | CD2 | LEU | 10 | 41.549 | -9.790 | -10.488 | 1.00 | 20.05 |
| atom | 76 | C | LEU | 10 | 42.778 | -6.525 | -7.840 | 1.00 | 26.02 |
| atom | 77 | O | LEU | 10 | 43.620 | -5.716 | -8.243 | 1.00 | 26.63 |
| atom | 78 | N | ILE | 11 | 42.290 | -6.503 | -6.601 | 1.00 | 26.01 |
| atom | 79 | CA | ILE | 11 | 42.839 | -5.557 | -5.647 | 1.00 | 24.74 |
| atom | 80 | CB | ILE | 11 | 41.995 | -5.462 | -4.382 | 1.00 | 25.59 |
| atom | 81 | CG2 | ILE | 11 | 42.515 | -4.345 | -3.515 | 1.00 | 26.49 |
| atom | 82 | CG1 | ILE | 11 | 40.528 | -5.217 | -4.745 | 1.00 | 30.67 |
| atom | 83 | CD1 | ILE | 11 | 40.185 | -3.786 | -4.986 | 1.00 | 26.57 |
| atom | 84 | C | ILE | 11 | 44.172 | -6.226 | -5.341 | 1.00 | 23.05 |
| atom | 85 | O | ILE | 11 | 44.216 | -7.424 | -5.107 | 1.00 | 21.67 |
| atom | 86 | N | THR | 12 | 45.262 | -5.475 | -5.368 | 1.00 | 24.58 |
| atom | 87 | CA | THR | 12 | 46.565 | -6.085 | -5.145 | 1.00 | 25.76 |
| atom | 88 | CB | THR | 12 | 47.490 | -5.739 | -6.291 | 1.00 | 22.73 |
| atom | 89 | OG1 | THR | 12 | 47.462 | -4.324 | -6.502 | 1.00 | 22.66 |
| atom | 90 | CG2 | THR | 12 | 47.039 | -6.450 | -7.548 | 1.00 | 19.62 |
| atom | 91 | C | THR | 12 | 47.300 | -5.754 | -3.857 | 1.00 | 26.55 |
| atom | 92 | O | THR | 12 | 47.194 | -4.652 | -3.335 | 1.00 | 27.29 |
| atom | 93 | N | PRO | 13 | 48.059 | -6.726 | -3.330 | 1.00 | 31.02 |
| atom | 94 | CD | PRO | 13 | 48.217 | -8.089 | -3.879 | 1.00 | 32.32 |
| atom | 95 | CA | PRO | 13 | 48.829 | -6.543 | -2.096 | 1.00 | 30.95 |
| atom | 96 | CB | PRO | 13 | 49.042 | -7.965 | -1.601 | 1.00 | 27.26 |
| atom | 97 | CG | PRO | 13 | 49.111 | -8.774 | -2.864 | 1.00 | 31.67 |
| atom | 98 | C | PRO | 13 | 50.152 | -5.855 | -2.417 | 1.00 | 34.84 |
| atom | 99 | O | PRO | 13 | 50.739 | -6.101 | -3.471 | 1.00 | 29.50 |
| atom | 100 | N | CYS | 14 | 50.613 | -4.986 | -1.520 | 1.00 | 39.99 |
| atom | 101 | CA | CYS | 14 | 51.891 | -4.302 | -1.727 | 1.00 | 42.73 |
| atom | 102 | CB | CYS | 14 | 51.794 | -2.816 | -1.347 | 1.00 | 44.03 |
| atom | 103 | SG | CYS | 14 | 50.790 | -2.471 | 0.117 | 1.00 | 45.82 |
| atom | 104 | C | CYS | 14 | 52.956 | -4.973 | -0.868 | 1.00 | 44.38 |

| atom | 105 | O   | CYS | 14 | 54.141 | -5.004  | -1.220 | 1.00 | 46.34 |
|------|-----|-----|-----|----|--------|---------|--------|------|-------|
| atom | 106 | N   | ALA | 15 | 52.518 | -5.514  | 0.264  | 1.00 | 45.12 |
| atom | 107 | CA  | ALA | 15 | 53.422 | -6.178  | 1.198  | 1.00 | 44.03 |
| atom | 108 | CB  | ALA | 15 | 53.231 | -5.608  | 2.608  | 1.00 | 40.66 |
| atom | 109 | C   | ALA | 15 | 53.183 | -7.677  | 1.216  | 1.00 | 42.16 |
| atom | 110 | O   | ALA | 15 | 52.165 | -8.170  | 0.713  | 1.00 | 40.93 |
| atom | 111 | N   | ALA | 16 | 54.144 | -8.410  | 1.765  | 1.00 | 36.85 |
| atom | 112 | CA  | ALA | 16 | 53.971 | -9.837  | 1.884  | 1.00 | 32.86 |
| atom | 113 | CB  | ALA | 16 | 55.234 | -10.469 | 2.415  | 1.00 | 26.99 |
| atom | 114 | C   | ALA | 16 | 52.868 | -9.874  | 2.934  | 1.00 | 33.29 |
| atom | 115 | O   | ALA | 16 | 52.869 | -9.064  | 3.862  | 1.00 | 37.11 |
| atom | 116 | N   | GLU | 17 | 51.898 | -10.760 | 2.785  | 1.00 | 32.39 |
| atom | 117 | CA  | GLU | 17 | 50.851 | -10.833 | 3.789  | 1.00 | 26.32 |
| atom | 118 | CB  | GLU | 17 | 49.513 | -10.306 | 3.265  | 1.00 | 30.86 |
| atom | 119 | CG  | GLU | 17 | 49.528 | -9.677  | 1.895  | 1.00 | 29.77 |
| atom | 120 | CD  | GLU | 17 | 48.157 | -9.707  | 1.254  | 1.00 | 33.50 |
| atom | 121 | OE1 | GLU | 17 | 47.415 | -8.711  | 1.424  | 1.00 | 32.53 |
| atom | 122 | OE2 | GLU | 17 | 47.819 | -10.723 | 0.595  | 1.00 | 32.84 |
| atom | 123 | C   | GLU | 17 | 50.679 | -12.255 | 4.276  | 1.00 | 24.51 |
| atom | 124 | O   | GLU | 17 | 50.514 | -13.188 | 3.485  | 1.00 | 16.59 |
| atom | 125 | N   | GLU | 18 | 50.739 | -12.385 | 5.600  | 1.00 | 26.82 |
| atom | 126 | CA  | GLU | 18 | 50.604 | -13.643 | 6.320  | 1.00 | 27.85 |
| atom | 127 | CB  | GLU | 18 | 51.444 | -13.571 | 7.589  | 1.00 | 34.84 |
| atom | 128 | CG  | GLU | 18 | 52.921 | -13.830 | 7.394  | 1.00 | 42.40 |
| atom | 129 | CD  | GLU | 18 | 53.516 | -14.582 | 8.566  | 1.00 | 45.99 |
| atom | 130 | OE1 | GLU | 18 | 54.476 | -14.061 | 9.171  | 1.00 | 45.15 |
| atom | 131 | OE2 | GLU | 18 | 53.013 | -15.692 | 8.880  | 1.00 | 44.59 |
| atom | 132 | C   | GLU | 18 | 49.151 | -13.868 | 6.708  | 1.00 | 29.21 |
| atom | 133 | O   | GLU | 18 | 48.451 | -12.931 | 7.085  | 1.00 | 28.56 |
| atom | 134 | N   | SER | 19 | 48.694 | -15.111 | 6.668  | 1.00 | 31.66 |
| atom | 135 | CA  | SER | 19 | 47.304 | -15.374 | 7.022  | 1.00 | 33.52 |
| atom | 136 | CB  | SER | 19 | 46.517 | -15.684 | 5.751  | 1.00 | 31.74 |
| atom | 137 | OG  | SER | 19 | 47.196 | -16.652 | 4.981  | 1.00 | 28.18 |
| atom | 138 | C   | SER | 19 | 47.120 | -16.510 | 8.036  | 1.00 | 36.81 |
| atom | 139 | O   | SER | 19 | 46.005 | -16.787 | 8.491  | 1.00 | 37.53 |
| atom | 140 | N   | LYS | 20 | 48.215 | -17.175 | 8.379  | 1.00 | 37.96 |

| atom | 141 | CA | LYS | 20 | 48.176 | -18.280 | 9.330 | 1.00 | 34.72 |
| atom | 142 | CB | LYS | 20 | 48.764 | -19.534 | 8.677 | 1.00 | 34.98 |
| atom | 143 | CG | LYS | 20 | 47.728 | -20.526 | 8.181 | 1.00 | 37.63 |
| atom | 144 | CD | LYS | 20 | 48.233 | -21.968 | 8.303 | 1.00 | 45.68 |
| atom | 145 | CE | LYS | 20 | 49.654 | -22.140 | 7.741 | 1.00 | 50.03 |
| atom | 146 | NZ | LYS | 20 | 49.751 | -23.135 | 6.616 | 1.00 | 51.95 |
| atom | 147 | C | LYS | 20 | 49.004 | -17.877 | 10.550 | 1.00 | 34.35 |
| atom | 148 | 0 | LYS | 20 | 50.126 | -17.384 | 10.398 | 1.00 | 34.45 |
| atom | 149 | N | LEU | 21 | 48.463 | -18.070 | 11.751 | 1.00 | 33.05 |
| atom | 150 | CA | LEU | 21 | 49.199 | -17.693 | 12.962 | 1.00 | 32.36 |
| atom | 151 | CB | LEU | 21 | 48.537 | -18.280 | 14.226 | 1.00 | 35.12 |
| atom | 152 | CG | LEU | 21 | 49.026 | -17.899 | 15.644 | 1.00 | 28.57 |
| atom | 153 | CD1 | LEU | 21 | 50.301 | -17.098 | 15.601 | 1.00 | 29.37 |
| atom | 154 | CD2 | LEU | 21 | 47.943 | -17.094 | 16.349 | 1.00 | 27.17 |
| atom | 155 | C | LEU | 21 | 50.638 | -18.181 | 12.883 | 1.00 | 29.79 |
| atom | 156 | 0 | LEU | 21 | 50.882 | -19.388 | 12.784 | 1.00 | 26.13 |
| atom | 157 | N | PRO | 22 | 51.600 | -17.237 | 12.889 | 1.00 | 30.54 |
| atom | 158 | CD | PRO | 22 | 51.282 | -15.800 | 12.861 | 1.00 | 31.66 |
| atom | 159 | CA | PRO | 22 | 53.052 | -17.454 | 12.832 | 1.00 | 32.01 |
| atom | 160 | CB | PRO | 22 | 53.629 | -16.041 | 12.738 | 1.00 | 33.70 |
| atom | 161 | CG | PRO | 22 | 52.499 | -15.210 | 12.205 | 1.00 | 30.75 |
| atom | 162 | C | PRO | 22 | 53.593 | -18.209 | 14.041 | 1.00 | 35.09 |
| atom | 163 | 0 | PRO | 22 | 54.678 | -17.897 | 14.540 | 1.00 | 39.45 |
| atom | 164 | N | ILE | 23 | 52.802 | -19.184 | 14.496 | 1.00 | 33.92 |
| atom | 165 | CA | ILE | 23 | 53.081 | -20.079 | 15.616 | 1.00 | 29.63 |
| atom | 166 | CB | ILE | 23 | 52.899 | -21.539 | 15.135 | 1.00 | 32.30 |
| atom | 167 | CG2 | ILE | 23 | 54.222 | -22.295 | 15.156 | 1.00 | 33.90 |
| atom | 168 | CG1 | ILE | 23 | 51.835 | -22.229 | 15.979 | 1.00 | 31.35 |
| atom | 169 | CD1 | ILE | 23 | 51.895 | -23.740 | 15.884 | 1.00 | 35.04 |
| atom | 170 | C | ILE | 23 | 54.440 | -19.889 | 16.298 | 1.00 | 32.72 |
| atom | 171 | 0 | ILE | 23 | 55.500 | -20.012 | 15.682 | 1.00 | 29.52 |
| atom | 172 | N | ASN | 24 | 54.393 | -19.605 | 17.595 | 1.00 | 37.99 |
| atom | 173 | CA | ASN | 24 | 55.606 | -19.343 | 18.359 | 1.00 | 39.02 |
| atom | 174 | CB | ASN | 24 | 55.800 | -17.845 | 18.534 | 1.00 | 37.86 |
| atom | 175 | CG | ASN | 24 | 57.203 | -17.430 | 18.268 | 1.00 | 41.03 |
| atom | 176 | OD1 | ASN | 24 | 58.004 | -18.220 | 17.745 | 1.00 | 37.66 |

| | | | | | | | | |
|------|-----|-----|-----|----|-----------------|--------|--------|-------|
| atom | 177 | ND2 | ASN | 24 | 57.532 | -16.187 | 18.620 | 1.00 38.75 |
| atom | 178 | C   | ASN | 24 | 55.628 | -19.937 | 19.735 | 1.00 39.37 |
| atom | 179 | O   | ASN | 24 | 54.584 | -20.238 | 20.304 | 1.00 41.52 |
| atom | 180 | N   | ALA | 25 | 56.838 | -20.069 | 20.273 | 1.00 39.67 |
| atom | 181 | CA  | ALA | 25 | 57.032 | -20.577 | 21.622 | 1.00 37.68 |
| atom | 182 | CB  | ALA | 25 | 58.497 | -20.452 | 22.028 | 1.00 42.61 |
| atom | 183 | C   | ALA | 25 | 56.180 | -19.693 | 22.506 | 1.00 33.96 |
| atom | 184 | O   | ALA | 25 | 55.320 | -20.172 | 23.241 | 1.00 35.81 |
| atom | 185 | N   | LEU | 26 | 56.426 | -18.394 | 22.410 | 1.00 30.24 |
| atom | 186 | CA  | LEU | 26 | 55.694 | -17.405 | 23.185 | 1.00 29.54 |
| atom | 187 | CB  | LEU | 26 | 56.256 | -16.005 | 22.926 | 1.00 26.07 |
| atom | 188 | CG  | LEU | 26 | 57.616 | -15.608 | 23.523 | 1.00 25.12 |
| atom | 189 | CD1 | LEU | 26 | 57.466 | -14.286 | 24.238 | 1.00 26.71 |
| atom | 190 | CD2 | LEU | 26 | 58.140 | -16.676 | 24.475 | 1.00 25.70 |
| atom | 191 | C   | LEU | 26 | 54.216 | -17.412 | 22.854 | 1.00 32.07 |
| atom | 192 | O   | LEU | 26 | 53.381 | -17.282 | 23.747 | 1.00 39.46 |
| atom | 193 | N   | SER | 27 | 53.886 | -17.573 | 21.576 | 1.00 33.97 |
| atom | 194 | CA  | SER | 27 | 52.490 | -17.565 | 21.148 | 1.00 34.97 |
| atom | 195 | CB  | SER | 27 | 52.411 | -17.627 | 19.619 | 1.00 38.54 |
| atom | 196 | OG  | SER | 27 | 51.508 | -18.642 | 19.187 | 1.00 42.65 |
| atom | 197 | C   | SER | 27 | 51.726 | -18.735 | 21.752 | 1.00 36.47 |
| atom | 198 | O   | SER | 27 | 50.501 | -18.707 | 21.868 | 1.00 34.83 |
| atom | 199 | N   | ASN | 28 | 52.460 | -19.764 | 22.150 | 1.00 37.51 |
| atom | 200 | CA  | ASN | 28 | 51.835 | -20.944 | 22.713 | 1.00 38.99 |
| atom | 201 | CB  | ASN | 28 | 52.682 | -22.177 | 22.421 | 1.00 46.35 |
| atom | 202 | CG  | ASN | 28 | 51.850 | -23.328 | 21.928 | 1.00 51.03 |
| atom | 203 | OD1 | ASN | 28 | 50.703 | -23.134 | 21.530 | 1.00 53.49 |
| atom | 204 | ND2 | ASN | 28 | 52.409 | -24.535 | 21.956 | 1.00 55.66 |
| atom | 205 | C   | ASN | 28 | 51.639 | -20.788 | 24.201 | 1.00 37.33 |
| atom | 206 | O   | ASN | 28 | 50.889 | -21.533 | 24.834 | 1.00 31.98 |
| atom | 207 | N   | SER | 29 | 52.333 | -19.818 | 24.771 | 1.00 37.14 |
| atom | 208 | CA  | SER | 29 | 52.176 | -19.579 | 26.188 | 1.00 35.88 |
| atom | 209 | CB  | SER | 29 | 53.123 | -18.461 | 26.638 | 1.00 33.82 |
| atom | 210 | OG  | SER | 29 | 52.498 | -17.580 | 27.564 | 1.00 34.09 |
| atom | 211 | C   | SER | 29 | 50.712 | -19.158 | 26.376 | 1.00 33.85 |
| atom | 212 | O   | SER | 29 | 50.021 | -19.629 | 27.282 | 1.00 36.33 |

| atom | 213 | N   | LEU | 30 | 50.239 | -18.319 | 25.460 | 1.00 | 28.11 |
|------|-----|-----|-----|----|--------|---------|--------|------|-------|
| atom | 214 | CA  | LEU | 30 | 48.890 | -17.763 | 25.505 | 1.00 | 23.92 |
| atom | 215 | CB  | LEU | 30 | 48.927 | -16.376 | 24.859 | 1.00 | 14.38 |
| atom | 216 | CG  | LEU | 30 | 47.593 | -15.690 | 24.700 | 1.00 | 11.04 |
| atom | 217 | CD1 | LEU | 30 | 46.961 | -15.499 | 26.069 | 1.00 | 12.59 |
| atom | 218 | CD2 | LEU | 30 | 47.795 | -14.352 | 23.974 | 1.00 | 13.47 |
| atom | 219 | C   | LEU | 30 | 47.731 | -18.557 | 24.900 | 1.00 | 24.67 |
| atom | 220 | O   | LEU | 30 | 46.758 | -18.876 | 25.586 | 1.00 | 20.27 |
| atom | 221 | N   | LEU | 31 | 47.830 | -18.840 | 23.604 | 1.00 | 25.98 |
| atom | 222 | CA  | LEU | 31 | 46.793 | -19.557 | 22.864 | 1.00 | 26.72 |
| atom | 223 | CB  | LEU | 31 | 46.387 | -18.738 | 21.643 | 1.00 | 28.09 |
| atom | 224 | CG  | LEU | 31 | 44.953 | -18.895 | 21.141 | 1.00 | 31.75 |
| atom | 225 | CD1 | LEU | 31 | 44.349 | -17.513 | 20.889 | 1.00 | 31.47 |
| atom | 226 | CD2 | LEU | 31 | 44.944 | -19.712 | 19.870 | 1.00 | 30.70 |
| atom | 227 | C   | LEU | 31 | 47.341 | -20.886 | 22.406 | 1.00 | 27.45 |
| atom | 228 | O   | LEU | 31 | 48.538 | -21.007 | 22.198 | 1.00 | 37.06 |
| atom | 229 | N   | ARG | 32 | 46.485 | -21.880 | 22.222 | 1.00 | 27.60 |
| atom | 230 | CA  | ARG | 32 | 46.963 | -23.188 | 21.796 | 1.00 | 31.35 |
| atom | 231 | CB  | ARG | 32 | 46.750 | -24.208 | 22.917 | 1.00 | 33.85 |
| atom | 232 | CG  | ARG | 32 | 46.668 | -25.659 | 22.465 | 1.00 | 44.85 |
| atom | 233 | CD  | ARG | 32 | 46.596 | -26.609 | 23.663 | 1.00 | 51.74 |
| atom | 234 | NE  | ARG | 32 | 47.614 | -26.290 | 24.667 | 1.00 | 56.45 |
| atom | 235 | CZ  | ARG | 32 | 48.617 | -27.098 | 25.014 | 1.00 | 59.99 |
| atom | 236 | NH1 | ARG | 32 | 48.751 | -28.290 | 24.443 | 1.00 | 60.21 |
| atom | 237 | NH2 | ARG | 32 | 49.498 | -26.711 | 25.932 | 1.00 | 61.46 |
| atom | 238 | C   | ARG | 32 | 46.330 | -23.700 | 20.502 | 1.00 | 33.99 |
| atom | 239 | O   | ARG | 32 | 46.925 | -24.524 | 19.809 | 1.00 | 38.04 |
| atom | 240 | N   | HIS | 33 | 45.139 | -23.213 | 20.173 | 1.00 | 30.07 |
| atom | 241 | CA  | HIS | 33 | 44.442 | -23.641 | 18.967 | 1.00 | 29.00 |
| atom | 242 | CB  | HIS | 33 | 42.953 | -23.794 | 19.268 | 1.00 | 23.73 |
| atom | 243 | CG  | HIS | 33 | 42.637 | -24.892 | 20.233 | 1.00 | 27.12 |
| atom | 244 | CD2 | HIS | 33 | 43.436 | -25.784 | 20.868 | 1.00 | 25.66 |
| atom | 245 | ND1 | HIS | 33 | 41.346 | -25.199 | 20.613 | 1.00 | 22.82 |
| atom | 246 | CE1 | HIS | 33 | 41.365 | -26.233 | 21.434 | 1.00 | 23.60 |
| atom | 247 | NE2 | HIS | 33 | 42.621 | -26.608 | 21.604 | 1.00 | 24.43 |
| atom | 248 | C   | HIS | 33 | 44.637 | -22.637 | 17.821 | 1.00 | 34.47 |

| atom | 249 | O | HIS | 33 | 43.676 | -22.232 | 17.172 | 1.00 | 35.26 |
|------|-----|-----|-----|----|--------|---------|--------|------|-------|
| atom | 250 | N | HIS | 34 | 45.886 | -22.255 | 17.572 | 1.00 | 37.29 |
| atom | 251 | CA | HIS | 34 | 46.250 | -21.284 | 16.533 | 1.00 | 40.45 |
| atom | 252 | CB | HIS | 34 | 47.761 | -21.260 | 16.385 | 1.00 | 43.17 |
| atom | 253 | CG | HIS | 34 | 48.333 | -22.600 | 16.057 | 1.00 | 48.92 |
| atom | 254 | CD2 | HIS | 34 | 48.895 | -23.078 | 14.922 | 1.00 | 49.02 |
| atom | 255 | ND1 | HIS | 34 | 48.309 | -23.654 | 16.945 | 1.00 | 53.07 |
| atom | 256 | CE1 | HIS | 34 | 48.830 | -24.724 | 16.373 | 1.00 | 50.31 |
| atom | 257 | NE2 | HIS | 34 | 49.194 | -24.399 | 15.145 | 1.00 | 53.13 |
| atom | 258 | C | HIS | 34 | 45.649 | -21.494 | 15.141 | 1.00 | 40.69 |
| atom | 259 | O | HIS | 34 | 45.682 | -20.591 | 14.310 | 1.00 | 42.64 |
| atom | 260 | N | ASN | 35 | 45.130 | -22.681 | 14.866 | 1.00 | 40.62 |
| atom | 261 | CA | ASN | 35 | 44.558 | -22.921 | 13.556 | 1.00 | 41.61 |
| atom | 262 | CB | ASN | 35 | 44.420 | -24.420 | 13.298 | 1.00 | 39.60 |
| atom | 263 | CG | ASN | 35 | 45.736 | -25.055 | 12.878 | 1.00 | 42.24 |
| atom | 264 | OD1 | ASN | 35 | 46.077 | -26.152 | 13.323 | 1.00 | 45.01 |
| atom | 265 | ND2 | ASN | 35 | 46.488 | -24.362 | 12.024 | 1.00 | 39.61 |
| atom | 266 | C | ASN | 35 | 43.217 | -22.224 | 13.382 | 1.00 | 41.88 |
| atom | 267 | O | ASN | 35 | 42.741 | -22.079 | 12.261 | 1.00 | 48.58 |
| atom | 268 | N | MET | 36 | 42.617 | -21.790 | 14.487 | 1.00 | 39.02 |
| atom | 269 | CA | MET | 36 | 41.331 | -21.085 | 14.466 | 1.00 | 34.47 |
| atom | 270 | CB | MET | 36 | 40.600 | -21.250 | 15.789 | 1.00 | 38.00 |
| atom | 271 | CG | MET | 36 | 40.788 | -22.577 | 16.439 | 1.00 | 38.32 |
| atom | 272 | SD | MET | 36 | 39.272 | -23.491 | 16.413 | 1.00 | 43.98 |
| atom | 273 | CE | MET | 36 | 38.050 | -22.269 | 15.969 | 1.00 | 37.20 |
| atom | 274 | C | MET | 36 | 41.542 | -19.599 | 14.266 | 1.00 | 33.18 |
| atom | 275 | O | MET | 36 | 40.592 | -18.848 | 14.059 | 1.00 | 33.83 |
| atom | 276 | N | VAL | 37 | 42.795 | -19.180 | 14.357 | 1.00 | 28.33 |
| atom | 277 | CA | VAL | 37 | 43.134 | -17.785 | 14.222 | 1.00 | 30.04 |
| atom | 278 | CB | VAL | 37 | 44.152 | -17.391 | 15.307 | 1.00 | 30.45 |
| atom | 279 | CG1 | VAL | 37 | 44.675 | -15.980 | 15.061 | 1.00 | 33.10 |
| atom | 280 | CG2 | VAL | 37 | 43.487 | -17.485 | 16.676 | 1.00 | 27.52 |
| atom | 281 | C | VAL | 37 | 43.686 | -17.529 | 12.834 | 1.00 | 32.08 |
| atom | 282 | O | VAL | 37 | 44.552 | -18.254 | 12.351 | 1.00 | 34.38 |
| atom | 283 | N | TYR | 38 | 43.171 | -16.498 | 12.182 | 1.00 | 33.37 |
| atom | 284 | CA | TYR | 38 | 43.610 | -16.178 | 10.831 | 1.00 | 32.41 |

| atom | 285 | CB | TYR | 38 | 42.694 | -16.839 | 9.805 | 1.00 | 31.04 |
|------|-----|-----|-----|----|--------|---------|-------|------|-------|
| atom | 286 | CG | TYR | 38 | 41.321 | -16.218 | 9.757 | 1.00 | 28.60 |
| atom | 287 | CD1 | TYR | 38 | 41.058 | -15.127 | 8.934 | 1.00 | 28.40 |
| atom | 288 | CE1 | TYR | 38 | 39.777 | -14.582 | 8.838 | 1.00 | 27.52 |
| atom | 289 | CD2 | TYR | 38 | 40.272 | -16.746 | 10.498 | 1.00 | 27.69 |
| atom | 290 | CE2 | TYR | 38 | 38.988 | -16.207 | 10.408 | 1.00 | 26.54 |
| atom | 291 | CZ | TYR | 38 | 38.753 | -15.129 | 9.574 | 1.00 | 28.08 |
| atom | 292 | OH | TYR | 38 | 37.487 | -14.617 | 9.456 | 1.00 | 33.47 |
| atom | 293 | C | TYR | 38 | 43.611 | -14.691 | 10.571 | 1.00 | 30.86 |
| atom | 294 | O | TYR | 38 | 43.168 | -13.895 | 11.404 | 1.00 | 29.91 |
| atom | 295 | N | ALA | 39 | 44.085 | -14.331 | 9.384 | 1.00 | 26.58 |
| atom | 296 | CA | ALA | 39 | 44.150 | -12.939 | 8.993 | 1.00 | 26.36 |
| atom | 297 | CB | ALA | 39 | 45.583 | -12.441 | 9.077 | 1.00 | 21.23 |
| atom | 298 | C | ALA | 39 | 43.596 | -12.759 | 7.582 | 1.00 | 30.17 |
| atom | 299 | O | ALA | 39 | 43.787 | -13.615 | 6.708 | 1.00 | 29.35 |
| atom | 300 | N | THR | 40 | 42.886 | -11.651 | 7.384 | 1.00 | 30.81 |
| atom | 301 | CA | THR | 40 | 42.303 | -11.328 | 6.101 | 1.00 | 31.04 |
| atom | 302 | CB | THR | 40 | 41.198 | -10.268 | 6.229 | 1.00 | 30.42 |
| atom | 303 | OG1 | THR | 40 | 41.743 | -9.070 | 6.790 | 1.00 | 29.82 |
| atom | 304 | CG2 | THR | 40 | 40.065 | -10.778 | 7.099 | 1.00 | 20.31 |
| atom | 305 | C | THR | 40 | 43.402 | -10.782 | 5.201 | 1.00 | 33.39 |
| atom | 306 | O | THR | 40 | 44.283 | -10.055 | 5.659 | 1.00 | 36.59 |
| atom | 307 | N | THR | 41 | 43.330 | -11.144 | 3.923 | 1.00 | 33.19 |
| atom | 308 | CA | THR | 41 | 44.296 | -10.735 | 2.915 | 1.00 | 31.53 |
| atom | 309 | CB | THR | 41 | 45.318 | -11.858 | 2.642 | 1.00 | 34.00 |
| atom | 310 | OG1 | THR | 41 | 44.629 | -13.042 | 2.214 | 1.00 | 33.52 |
| atom | 311 | CG2 | THR | 41 | 46.105 | -12.178 | 3.904 | 1.00 | 37.37 |
| atom | 312 | C | THR | 41 | 43.586 | -10.420 | 1.598 | 1.00 | 32.18 |
| atom | 313 | O | THR | 41 | 42.345 | -10.474 | 1.518 | 1.00 | 28.34 |
| atom | 314 | N | SER | 42 | 44.395 | -10.097 | 0.584 | 1.00 | 30.60 |
| atom | 315 | CA | SER | 42 | 43.950 | -9.755 | -0.776 | 1.00 | 28.87 |
| atom | 316 | CB | SER | 42 | 45.160 | -9.587 | -1.682 | 1.00 | 30.94 |
| atom | 317 | OG | SER | 42 | 45.386 | -8.232 | -1.985 | 1.00 | 41.09 |
| atom | 318 | C | SER | 42 | 43.078 | -10.838 | -1.386 | 1.00 | 25.21 |
| atom | 319 | O | SER | 42 | 41.975 | -10.598 | -1.859 | 1.00 | 21.53 |
| atom | 320 | N | ARG | 43 | 43.598 | -12.049 | -1.366 | 1.00 | 25.12 |

| atom | 321 | CA | ARG | 43 | 42.898 | -13.187 | -1.922 | 1.00 | 24.36 |
|------|-----|-----|-----|-----|--------|---------|--------|------|-------|
| atom | 322 | CB | ARG | 43 | 43.474 | -14.464 | -1.307 | 1.00 | 22.07 |
| atom | 323 | CG | ARG | 43 | 44.756 | -14.920 | -1.980 | 1.00 | 35.37 |
| atom | 324 | CD | ARG | 43 | 45.876 | -15.237 | -0.980 | 1.00 | 43.28 |
| atom | 325 | NE | ARG | 43 | 46.643 | -16.436 | -1.347 | 1.00 | 45.15 |
| atom | 326 | CZ | ARG | 43 | 47.970 | -16.484 | -1.484 | 1.00 | 47.79 |
| atom | 327 | NH1 | ARG | 43 | 48.717 | -15.403 | -1.289 | 1.00 | 48.79 |
| atom | 328 | NH2 | ARG | 43 | 48.557 | -17.623 | -1.826 | 1.00 | 49.62 |
| atom | 329 | C | ARG | 43 | 41.372 | -13.147 | -1.756 | 1.00 | 21.27 |
| atom | 330 | O | ARG | 43 | 40.642 | -13.709 | -2.575 | 1.00 | 21.29 |
| atom | 331 | N | SER | 44 | 40.892 | -12.478 | -0.707 | 1.00 | 21.05 |
| atom | 332 | CA | SER | 44 | 39.455 | -12.409 | -0.431 | 1.00 | 17.85 |
| atom | 333 | CB | SER | 44 | 39.181 | -12.682 | 1.068 | 1.00 | 18.70 |
| atom | 334 | OG | SER | 44 | 39.495 | -11.568 | 1.885 | 1.00 | 24.38 |
| atom | 335 | C | SER | 44 | 38.798 | -11.105 | -0.868 | 1.00 | 9.35 |
| atom | 336 | O | SER | 44 | 37.582 | -11.024 | -0.926 | 1.00 | 8.69 |
| atom | 337 | N | ALA | 45 | 39.612 | -10.101 | -1.170 | 1.00 | 6.77 |
| atom | 338 | CA | ALA | 45 | 39.136 | -8.794 | -1.635 | 1.00 | 12.00 |
| atom | 339 | CB | ALA | 45 | 40.271 | -8.062 | -2.347 | 1.00 | 10.65 |
| atom | 340 | C | ALA | 45 | 37.948 | -8.906 | -2.595 | 1.00 | 16.23 |
| atom | 341 | O | ALA | 45 | 37.045 | -8.069 | -2.600 | 1.00 | 24.10 |
| atom | 342 | N | GLY | 46 | 37.978 | -9.928 | -3.437 | 1.00 | 19.30 |
| atom | 343 | CA | GLY | 46 | 36.913 | -10.115 | -4.391 | 1.00 | 19.54 |
| atom | 344 | C | GLY | 46 | 35.587 | -10.419 | -3.738 | 1.00 | 20.90 |
| atom | 345 | O | GLY | 46 | 34.567 | -9.860 | -4.135 | 1.00 | 23.71 |
| atom | 346 | N | LEU | 47 | 35.592 | -11.319 | -2.755 | 1.00 | 22.91 |
| atom | 347 | CA | LEU | 47 | 34.369 | -11.688 | -2.058 | 1.00 | 19.97 |
| atom | 348 | CB | LEU | 47 | 34.678 | -12.655 | -0.931 | 1.00 | 23.98 |
| atom | 349 | CG | LEU | 47 | 34.891 | -14.108 | -1.336 | 1.00 | 27.07 |
| atom | 350 | CD1 | LEU | 47 | 36.076 | -14.203 | -2.281 | 1.00 | 26.74 |
| atom | 351 | CD2 | LEU | 47 | 35.114 | -14.946 | -0.092 | 1.00 | 23.16 |
| atom | 352 | C | LEU | 47 | 33.739 | -10.428 | -1.484 | 1.00 | 22.24 |
| atom | 353 | O | LEU | 47 | 32.527 | -10.206 | -1.604 | 1.00 | 25.16 |
| atom | 354 | N | ARG | 48 | 34.577 | -9.589 | -0.888 | 1.00 | 17.98 |
| atom | 355 | CA | ARG | 48 | 34.102 | -8.359 | -0.289 | 1.00 | 19.39 |
| atom | 356 | CB | ARG | 48 | 35.257 | -7.600 | 0.361 | 1.00 | 22.15 |

| atom | 357 | CG | ARG | 48 | 34.848 | -6.275 | 0.991 | 1.00 | 25.34 |
|---|---|---|---|---|---|---|---|---|---|
| atom | 358 | CD | ARG | 48 | 33.665 | -6.451 | 1.924 | 1.00 | 31.73 |
| atom | 359 | NE | ARG | 48 | 33.968 | -7.332 | 3.052 | 1.00 | 41.05 |
| atom | 360 | CZ | ARG | 48 | 33.971 | -6.954 | 4.332 | 1.00 | 44.95 |
| atom | 361 | NH1 | ARG | 48 | 33.684 | -5.697 | 4.672 | 1.00 | 44.28 |
| atom | 362 | NH2 | ARG | 48 | 34.265 | -7.840 | 5.279 | 1.00 | 45.69 |
| atom | 363 | C | ARG | 48 | 33.426 | -7.448 | -1.280 | 1.00 | 18.00 |
| atom | 364 | O | ARG | 48 | 32.389 | -6.861 | -0.978 | 1.00 | 13.22 |
| atom | 365 | N | GLN | 49 | 34.033 | -7.336 | -2.459 | 1.00 | 19.75 |
| atom | 366 | CA | GLN | 49 | 33.551 | -6.474 | -3.531 | 1.00 | 17.34 |
| atom | 367 | CB | GLN | 49 | 34.394 | -6.691 | -4.778 | 1.00 | 23.31 |
| atom | 368 | CG | GLN | 49 | 35.392 | -5.599 | -5.057 | 1.00 | 25.70 |
| atom | 369 | CD | GLN | 49 | 36.457 | -6.046 | -6.035 | 1.00 | 32.79 |
| atom | 370 | OE1 | GLN | 49 | 36.875 | -5.290 | -6.915 | 1.00 | 32.01 |
| atom | 371 | NE2 | GLN | 49 | 36.903 | -7.287 | -5.887 | 1.00 | 35.96 |
| atom | 372 | C | GLN | 49 | 32.097 | -6.705 | -3.869 | 1.00 | 20.93 |
| atom | 373 | O | GLN | 49 | 31.370 | -5.769 | -4.200 | 1.00 | 25.69 |
| atom | 374 | N | LYS | 50 | 31.658 | -7.951 | -3.782 | 1.00 | 20.84 |
| atom | 375 | CA | LYS | 50 | 30.276 | -8.271 | -4.084 | 1.00 | 20.89 |
| atom | 376 | CB | LYS | 50 | 30.129 | -9.775 | -4.339 | 1.00 | 22.67 |
| atom | 377 | CG | LYS | 50 | 31.351 | -10.432 | -4.957 | 1.00 | 25.57 |
| atom | 378 | CD | LYS | 50 | 31.347 | -10.361 | -6.464 | 1.00 | 28.52 |
| atom | 379 | CE | LYS | 50 | 32.689 | -9.833 | -7.005 | 1.00 | 33.85 |
| atom | 380 | NZ | LYS | 50 | 32.553 | -8.461 | -7.590 | 1.00 | 29.19 |
| atom | 381 | C | LYS | 50 | 29.288 | -7.848 | -3.008 | 1.00 | 20.77 |
| atom | 382 | O | LYS | 50 | 28.196 | -7.349 | -3.304 | 1.00 | 20.61 |
| atom | 383 | N | LYS | 51 | 29.667 | -8.052 | -1.753 | 1.00 | 28.30 |
| atom | 384 | CA | LYS | 51 | 28.786 | -7.733 | -0.627 | 1.00 | 27.64 |
| atom | 385 | CB | LYS | 51 | 29.338 | -8.347 | 0.666 | 1.00 | 28.63 |
| atom | 386 | CG | LYS | 51 | 29.772 | -9.809 | 0.570 | 1.00 | 34.26 |
| atom | 387 | CD | LYS | 51 | 31.099 | -10.036 | 1.324 | 1.00 | 39.78 |
| atom | 388 | CE | LYS | 51 | 30.917 | -10.860 | 2.597 | 1.00 | 41.29 |
| atom | 389 | NZ | LYS | 51 | 29.487 | -11.221 | 2.851 | 1.00 | 42.28 |
| atom | 390 | C | LYS | 51 | 28.637 | -6.239 | -0.440 | 1.00 | 25.06 |
| atom | 391 | O | LYS | 51 | 27.693 | -5.757 | 0.189 | 1.00 | 26.11 |
| atom | 392 | N | VAL | 52 | 29.574 | -5.505 | -1.014 | 1.00 | 23.63 |

| atom | 393 | CA  | VAL | 52 | 29.605 | -4.063 | -0.869 | 1.00 22.61 |
| atom | 394 | CB  | VAL | 52 | 31.070 | -3.649 | -0.616 | 1.00 19.17 |
| atom | 395 | CG1 | VAL | 52 | 31.729 | -3.212 | -1.894 | 1.00 17.90 |
| atom | 396 | CG2 | VAL | 52 | 31.131 | -2.603 |  0.428 | 1.00 24.32 |
| atom | 397 | C   | VAL | 52 | 29.024 | -3.279 | -2.054 | 1.00 19.94 |
| atom | 398 | 0   | VAL | 52 | 28.875 | -2.063 | -1.995 | 1.00 19.73 |
| atom | 399 | N   | THR | 53 | 28.671 | -3.996 | -3.111 | 1.00 20.39 |
| atom | 400 | CA  | THR | 53 | 28.170 | -3.397 | -4.339 | 1.00 18.89 |
| atom | 401 | CB  | THR | 53 | 28.843 | -4.061 | -5.557 | 1.00 18.51 |
| atom | 402 | OG1 | THR | 53 | 30.258 | -3.831 | -5.506 | 1.00 23.13 |
| atom | 403 | CG2 | THR | 53 | 28.285 | -3.519 | -6.855 | 1.00 15.71 |
| atom | 404 | C   | THR | 53 | 26.672 | -3.478 | -4.536 | 1.00 21.86 |
| atom | 405 | 0   | THR | 53 | 26.113 | -4.555 | -4.677 | 1.00 22.43 |
| atom | 406 | N   | PHE | 54 | 26.015 | -2.330 | -4.585 | 1.00 22.23 |
| atom | 407 | CA  | PHE | 54 | 24.577 | -2.329 | -4.806 | 1.00 20.95 |
| atom | 408 | CB  | PHE | 54 | 23.857 | -2.852 | -3.568 | 1.00 17.15 |
| atom | 409 | CG  | PHE | 54 | 24.485 | -2.432 | -2.299 | 1.00 15.48 |
| atom | 410 | CD1 | PHE | 54 | 24.290 | -1.156 | -1.804 | 1.00 21.00 |
| atom | 411 | CD2 | PHE | 54 | 25.313 | -3.299 | -1.607 | 1.00 28.94 |
| atom | 412 | CE1 | PHE | 54 | 24.914 | -0.738 | -0.628 | 1.00 24.14 |
| atom | 413 | CE2 | PHE | 54 | 25.949 | -2.898 | -0.428 | 1.00 30.32 |
| atom | 414 | CZ  | PHE | 54 | 25.745 | -1.608 |  0.059 | 1.00 29.70 |
| atom | 415 | C   | PHE | 54 | 24.134 | -0.911 | -5.120 | 1.00 21.37 |
| atom | 416 | 0   | PHE | 54 | 24.959 |  0.001 | -5.171 | 1.00 21.13 |
| atom | 417 | N   | ASP | 55 | 22.833 | -0.738 | -5.319 | 1.00 20.70 |
| atom | 418 | CA  | ASP | 55 | 22.251 |  0.557 | -5.628 | 1.00 20.89 |
| atom | 419 | CB  | ASP | 55 | 21.155 |  0.361 | -6.695 | 1.00 19.59 |
| atom | 420 | CG  | ASP | 55 | 20.435 |  1.663 | -7.079 | 1.00 20.07 |
| atom | 421 | OD1 | ASP | 55 | 21.080 |  2.589 | -7.622 | 1.00 14.28 |
| atom | 422 | OD2 | ASP | 55 | 19.206 |  1.743 | -6.835 | 1.00 24.20 |
| atom | 423 | C   | ASP | 55 | 21.675 |  1.253 | -4.372 | 1.00 23.84 |
| atom | 424 | 0   | ASP | 55 | 21.070 |  0.624 | -3.493 | 1.00 26.41 |
| atom | 425 | N   | ARG | 56 | 21.871 |  2.558 | -4.289 | 1.00 17.80 |
| atom | 426 | CA  | ARG | 56 | 21.344 |  3.297 | -3.177 | 1.00 16.51 |
| atom | 427 | CB  | ARG | 56 | 22.467 |  4.039 | -2.436 | 1.00 16.87 |
| atom | 428 | CG  | ARG | 56 | 23.525 |  3.130 | -1.871 | 1.00 10.07 |

| atom | 429 | CD | ARG | 56 | 24.710 | 3.082 | -2.808 | 1.00 | 15.13 |
|------|-----|-----|-----|----|--------|--------|--------|------|-------|
| atom | 430 | NE | ARG | 56 | 25.652 | 4.167 | -2.523 | 1.00 | 17.01 |
| atom | 431 | CZ | ARG | 56 | 25.953 | 5.128 | -3.389 | 1.00 | 28.89 |
| atom | 432 | NH1 | ARG | 56 | 25.388 | 5.152 | -4.598 | 1.00 | 27.60 |
| atom | 433 | NH2 | ARG | 56 | 26.838 | 6.053 | -3.064 | 1.00 | 31.98 |
| atom | 434 | C | ARG | 56 | 20.323 | 4.297 | -3.659 | 1.00 | 15.89 |
| atom | 435 | O | ARG | 56 | 20.638 | 5.136 | -4.477 | 1.00 | 18.04 |
| atom | 436 | N | LEU | 57 | 19.095 | 4.190 | -3.152 | 1.00 | 21.41 |
| atom | 437 | CA | LEU | 57 | 18.010 | 5.127 | -3.466 | 1.00 | 19.39 |
| atom | 438 | CB | LEU | 57 | 16.760 | 4.398 | -3.960 | 1.00 | 21.90 |
| atom | 439 | CG | LEU | 57 | 16.814 | 3.732 | -5.333 | 1.00 | 30.04 |
| atom | 440 | CD1 | LEU | 57 | 15.394 | 3.306 | -5.717 | 1.00 | 23.84 |
| atom | 441 | CD2 | LEU | 57 | 17.411 | 4.701 | -6.382 | 1.00 | 24.33 |
| atom | 442 | C | LEU | 57 | 17.697 | 5.801 | -2.125 | 1.00 | 22.57 |
| atom | 443 | O | LEU | 57 | 17.744 | 5.154 | -1.063 | 1.00 | 21.40 |
| atom | 444 | N | GLN | 58 | 17.360 | 7.083 | -2.176 | 1.00 | 20.39 |
| atom | 445 | CA | GLN | 58 | 17.082 | 7.850 | -0.983 | 1.00 | 17.61 |
| atom | 446 | CB | GLN | 58 | 18.271 | 8.781 | -0.695 | 1.00 | 19.01 |
| atom | 447 | CG | GLN | 58 | 19.212 | 8.352 | 0.413 | 1.00 | 18.78 |
| atom | 448 | CD | GLN | 58 | 20.304 | 9.377 | 0.649 | 1.00 | 19.26 |
| atom | 449 | OE1 | GLN | 58 | 20.684 | 10.114 | -0.265 | 1.00 | 25.14 |
| atom | 450 | NE2 | GLN | 58 | 20.813 | 9.436 | 1.870 | 1.00 | 7.96 |
| atom | 451 | C | GLN | 58 | 15.836 | 8.717 | -1.136 | 1.00 | 18.94 |
| atom | 452 | O | GLN | 58 | 15.714 | 9.465 | -2.113 | 1.00 | 18.41 |
| atom | 453 | N | VAL | 59 | 14.928 | 8.631 | -0.164 | 1.00 | 15.86 |
| atom | 454 | CA | VAL | 59 | 13.726 | 9.464 | -0.143 | 1.00 | 13.64 |
| atom | 455 | CB | VAL | 59 | 12.432 | 8.635 | -0.050 | 1.00 | 18.32 |
| atom | 456 | CG1 | VAL | 59 | 11.345 | 9.308 | -0.842 | 1.00 | 12.43 |
| atom | 457 | CG2 | VAL | 59 | 12.665 | 7.228 | -0.560 | 1.00 | 23.22 |
| atom | 458 | C | VAL | 59 | 13.872 | 10.308 | 1.120 | 1.00 | 11.72 |
| atom | 459 | O | VAL | 59 | 13.692 | 9.831 | 2.226 | 1.00 | 10.53 |
| atom | 460 | N | LEU | 60 | 14.231 | 11.566 | 0.947 | 1.00 | 14.06 |
| atom | 461 | CA | LEU | 60 | 14.457 | 12.418 | 2.090 | 1.00 | 14.87 |
| atom | 462 | CB | LEU | 60 | 15.545 | 13.433 | 1.767 | 1.00 | 12.50 |
| atom | 463 | CG | LEU | 60 | 16.894 | 12.787 | 1.394 | 1.00 | 17.55 |
| atom | 464 | CD1 | LEU | 60 | 17.892 | 13.910 | 1.096 | 1.00 | 11.66 |

| atom | 465 | CD2 | LEU | 60 | 17.414 | 11.848 | 2.527 | 1.00 | 2.00 |
|------|-----|-----|-----|----|--------|--------|-------|------|------|
| atom | 466 | C | LEU | 60 | 13.206 | 13.103 | 2.595 | 1.00 | 18.51 |
| atom | 467 | 0 | LEU | 60 | 12.423 | 13.669 | 1.825 | 1.00 | 16.16 |
| atom | 468 | N | ASP | 61 | 13.067 | 13.057 | 3.917 | 1.00 | 18.47 |
| atom | 469 | CA | ASP | 61 | 11.933 | 13.587 | 4.656 | 1.00 | 19.17 |
| atom | 470 | CB | ASP | 61 | 11.621 | 12.612 | 5.784 | 1.00 | 26.43 |
| atom | 471 | CG | ASP | 61 | 10.307 | 11.985 | 5.617 | 1.00 | 34.40 |
| atom | 472 | OD1 | ASP | 61 | 9.719 | 12.202 | 4.530 | 1.00 | 35.57 |
| atom | 473 | OD2 | ASP | 61 | 9.871 | 11.295 | 6.560 | 1.00 | 37.74 |
| atom | 474 | C | ASP | 61 | 12.000 | 14.976 | 5.287 | 1.00 | 9.45 |
| atom | 475 | 0 | ASP | 61 | 13.036 | 15.610 | 5.330 | 1.00 | 11.81 |
| atom | 476 | N | ASP | 62 | 10.856 | 15.400 | 5.808 | 1.00 | 8.67 |
| atom | 477 | CA | ASP | 62 | 10.717 | 16.659 | 6.541 | 1.00 | 13.85 |
| atom | 478 | CB | ASP | 62 | 9.235 | 16.962 | 6.773 | 1.00 | 19.68 |
| atom | 479 | CG | ASP | 62 | 8.622 | 17.785 | 5.653 | 1.00 | 24.90 |
| atom | 480 | OD1 | ASP | 62 | 9.193 | 18.828 | 5.278 | 1.00 | 29.35 |
| atom | 481 | OD2 | ASP | 62 | 7.557 | 17.388 | 5.146 | 1.00 | 36.92 |
| atom | 482 | C | ASP | 62 | 11.430 | 16.417 | 7.901 | 1.00 | 11.46 |
| atom | 483 | 0 | ASP | 62 | 12.156 | 17.265 | 8.419 | 1.00 | 14.07 |
| atom | 484 | N | HIS | 63 | 11.220 | 15.234 | 8.459 | 1.00 | 6.43 |
| atom | 485 | CA | HIS | 63 | 11.879 | 14.842 | 9.683 | 1.00 | 7.74 |
| atom | 486 | CB | HIS | 63 | 11.485 | 13.424 | 10.038 | 1.00 | 8.47 |
| atom | 487 | CG | HIS | 63 | 10.112 | 13.317 | 10.598 | 1.00 | 8.99 |
| atom | 488 | CD2 | HIS | 63 | 9.179 | 12.339 | 10.498 | 1.00 | 8.53 |
| atom | 489 | ND1 | HIS | 63 | 9.564 | 14.297 | 11.399 | 1.00 | 11.03 |
| atom | 490 | CE1 | HIS | 63 | 8.352 | 13.925 | 11.772 | 1.00 | 10.35 |
| atom | 491 | NE2 | HIS | 63 | 8.095 | 12.741 | 11.237 | 1.00 | 12.66 |
| atom | 492 | C | HIS | 63 | 13.383 | 14.920 | 9.472 | 1.00 | 10.38 |
| atom | 493 | 0 | HIS | 63 | 14.125 | 15.294 | 10.370 | 1.00 | 16.98 |
| atom | 494 | N | TYR | 64 | 13.830 | 14.574 | 8.269 | 1.00 | 11.90 |
| atom | 495 | CA | TYR | 64 | 15.246 | 14.633 | 7.927 | 1.00 | 7.03 |
| atom | 496 | CB | TYR | 64 | 15.477 | 13.834 | 6.642 | 1.00 | 9.53 |
| atom | 497 | CG | TYR | 64 | 16.891 | 13.867 | 6.083 | 1.00 | 6.78 |
| atom | 498 | CD1 | TYR | 64 | 17.815 | 12.885 | 6.415 | 1.00 | 5.13 |
| atom | 499 | CE1 | TYR | 64 | 19.105 | 12.925 | 5.916 | 1.00 | 14.54 |
| atom | 500 | CD2 | TYR | 64 | 17.294 | 14.884 | 5.250 | 1.00 | 2.00 |

| atom | 501 | CE2 | TYR | 64 | 18.555 | 14.937 | 4.749 | 1.00 | 2.00 |
|------|-----|-----|-----|----|--------|--------|-------|------|-------|
| atom | 502 | CZ | TYR | 64 | 19.475 | 13.966 | 5.077 | 1.00 | 11.69 |
| atom | 503 | OH | TYR | 64 | 20.755 | 14.046 | 4.555 | 1.00 | 11.33 |
| atom | 504 | C | TYR | 64 | 15.742 | 16.086 | 7.755 | 1.00 | 9.99 |
| atom | 505 | 0 | TYR | 64 | 16.838 | 16.414 | 8.174 | 1.00 | 10.97 |
| atom | 506 | N | ARG | 65 | 14.951 | 16.958 | 7.128 | 1.00 | 12.39 |
| atom | 507 | CA | ARG | 65 | 15.390 | 18.341 | 6.923 | 1.00 | 11.20 |
| atom | 508 | CB | ARG | 65 | 14.527 | 19.033 | 5.848 | 1.00 | 12.74 |
| atom | 509 | CG | ARG | 65 | 14.980 | 18.796 | 4.390 | 1.00 | 17.83 |
| atom | 510 | CD | ARG | 65 | 13.838 | 18.997 | 3.373 | 1.00 | 11.66 |
| atom | 511 | NE | ARG | 65 | 13.515 | 17.724 | 2.745 | 1.00 | 12.38 |
| atom | 512 | CZ | ARG | 65 | 12.293 | 17.229 | 2.669 | 1.00 | 12.36 |
| atom | 513 | NH1 | ARG | 65 | 11.272 | 17.904 | 3.181 | 1.00 | 21.59 |
| atom | 514 | NH2 | ARG | 65 | 12.090 | 16.048 | 2.107 | 1.00 | 22.71 |
| atom | 515 | C | ARG | 65 | 15.327 | 19.118 | 8.230 | 1.00 | 10.20 |
| atom | 516 | 0 | ARG | 65 | 16.164 | 19.973 | 8.494 | 1.00 | 10.28 |
| atom | 517 | N | ASP | 66 | 14.315 | 18.823 | 9.040 | 1.00 | 11.92 |
| atom | 518 | CA | ASP | 66 | 14.143 | 19.463 | 10.344 | 1.00 | 10.53 |
| atom | 519 | CB | ASP | 66 | 12.920 | 18.882 | 11.050 | 1.00 | 11.57 |
| atom | 520 | CG | ASP | 66 | 11.601 | 19.280 | 10.379 | 1.00 | 13.23 |
| atom | 521 | OD1 | ASP | 66 | 11.562 | 20.246 | 9.586 | 1.00 | 23.22 |
| atom | 522 | OD2 | ASP | 66 | 10.590 | 18.617 | 10.649 | 1.00 | 18.03 |
| atom | 523 | C | ASP | 66 | 15.375 | 19.216 | 11.196 | 1.00 | 10.59 |
| atom | 524 | 0 | ASP | 66 | 15.927 | 20.137 | 11.785 | 1.00 | 17.43 |
| atom | 525 | N | VAL | 67 | 15.805 | 17.959 | 11.254 | 1.00 | 12.05 |
| atom | 526 | CA | VAL | 67 | 16.975 | 17.582 | 12.024 | 1.00 | 14.16 |
| atom | 527 | CB | VAL | 67 | 17.135 | 16.050 | 12.080 | 1.00 | 14.68 |
| atom | 528 | CG1 | VAL | 67 | 18.492 | 15.683 | 12.680 | 1.00 | 12.91 |
| atom | 529 | CG2 | VAL | 67 | 16.019 | 15.452 | 12.901 | 1.00 | 15.06 |
| atom | 530 | C | VAL | 67 | 18.231 | 18.196 | 11.439 | 1.00 | 16.75 |
| atom | 531 | 0 | VAL | 67 | 19.103 | 18.650 | 12.168 | 1.00 | 20.85 |
| atom | 532 | N | LEU | 68 | 18.331 | 18.205 | 10.117 | 1.00 | 19.38 |
| atom | 533 | CA | LEU | 68 | 19.493 | 18.788 | 9.469 | 1.00 | 17.25 |
| atom | 534 | CB | LEU | 68 | 19.348 | 18.689 | 7.962 | 1.00 | 16.80 |
| atom | 535 | CG | LEU | 68 | 20.566 | 18.561 | 7.064 | 1.00 | 13.39 |
| atom | 536 | CD1 | LEU | 68 | 20.257 | 19.311 | 5.814 | 1.00 | 11.90 |

| atom | 537 | CD2 | LEU | 68 | 21.824 | 19.079 | 7.713 | 1.00 | 12.32 |
|------|-----|-----|-----|----|--------|--------|-------|------|-------|
| atom | 538 | C | LEU | 68 | 19.613 | 20.246 | 9.855 | 1.00 | 18.34 |
| atom | 539 | O | LEU | 68 | 20.681 | 20.691 | 10.259 | 1.00 | 18.28 |
| atom | 540 | N | LYS | 69 | 18.513 | 20.991 | 9.722 | 1.00 | 18.88 |
| atom | 541 | CA | LYS | 69 | 18.519 | 22.421 | 10.050 | 1.00 | 21.95 |
| atom | 542 | CB | LYS | 69 | 17.152 | 23.064 | 9.750 | 1.00 | 19.48 |
| atom | 543 | CG | LYS | 69 | 17.056 | 24.548 | 10.085 | 1.00 | 27.21 |
| atom | 544 | CD | LYS | 69 | 17.951 | 25.421 | 9.167 | 1.00 | 38.86 |
| atom | 545 | CE | LYS | 69 | 18.436 | 26.718 | 9.847 | 1.00 | 41.07 |
| atom | 546 | NZ | LYS | 69 | 19.792 | 26.593 | 10.505 | 1.00 | 38.52 |
| atom | 547 | C | LYS | 69 | 18.909 | 22.668 | 11.512 | 1.00 | 21.81 |
| atom | 548 | O | LYS | 69 | 19.668 | 23.597 | 11.806 | 1.00 | 20.33 |
| atom | 549 | N | GLU | 70 | 18.394 | 21.852 | 12.428 | 1.00 | 19.09 |
| atom | 550 | CA | GLU | 70 | 18.749 | 22.029 | 13.832 | 1.00 | 15.47 |
| atom | 551 | CB | GLU | 70 | 17.995 | 21.016 | 14.715 | 1.00 | 17.65 |
| atom | 552 | CG | GLU | 70 | 16.613 | 21.489 | 15.235 | 1.00 | 10.96 |
| atom | 553 | CD | GLU | 70 | 15.720 | 20.320 | 15.683 | 1.00 | 17.00 |
| atom | 554 | OE1 | GLU | 70 | 14.488 | 20.351 | 15.464 | 1.00 | 16.61 |
| atom | 555 | OE2 | GLU | 70 | 16.254 | 19.354 | 16.259 | 1.00 | 20.20 |
| atom | 556 | C | GLU | 70 | 20.275 | 21.840 | 13.950 | 1.00 | 14.28 |
| atom | 557 | O | GLU | 70 | 20.964 | 22.612 | 14.629 | 1.00 | 11.43 |
| atom | 558 | N | MET | 71 | 20.806 | 20.842 | 13.248 | 1.00 | 11.88 |
| atom | 559 | CA | MET | 71 | 22.244 | 20.563 | 13.289 | 1.00 | 14.32 |
| atom | 560 | CB | MET | 71 | 22.584 | 19.235 | 12.588 | 1.00 | 14.16 |
| atom | 561 | CG | MET | 71 | 22.224 | 17.968 | 13.368 | 1.00 | 9.37 |
| atom | 562 | SD | MET | 71 | 22.245 | 16.550 | 12.260 | 1.00 | 15.49 |
| atom | 563 | CE | MET | 71 | 23.964 | 16.104 | 12.333 | 1.00 | 8.73 |
| atom | 564 | C | MET | 71 | 23.075 | 21.669 | 12.657 | 1.00 | 15.37 |
| atom | 565 | O | MET | 71 | 24.211 | 21.893 | 13.049 | 1.00 | 21.07 |
| atom | 566 | N | LYS | 72 | 22.530 | 22.361 | 11.669 | 1.00 | 19.18 |
| atom | 567 | CA | LYS | 72 | 23.305 | 23.431 | 11.037 | 1.00 | 22.11 |
| atom | 568 | CB | LYS | 72 | 22.665 | 23.842 | 9.696 | 1.00 | 17.90 |
| atom | 569 | CG | LYS | 72 | 22.668 | 22.698 | 8.663 | 1.00 | 24.56 |
| atom | 570 | CD | LYS | 72 | 22.275 | 23.141 | 7.254 | 1.00 | 20.71 |
| atom | 571 | CE | LYS | 72 | 22.862 | 22.187 | 6.219 | 1.00 | 28.30 |
| atom | 572 | NZ | LYS | 72 | 22.248 | 22.346 | 4.857 | 1.00 | 23.88 |

| atom | 573 | C   | LYS | 72 | 23.380 | 24.619 | 12.001 | 1.00 | 22.65 |
| atom | 574 | O   | LYS | 72 | 24.397 | 25.302 | 12.108 | 1.00 | 21.19 |
| atom | 575 | N   | ALA | 73 | 22.302 | 24.835 | 12.739 | 1.00 | 19.95 |
| atom | 576 | CA  | ALA | 73 | 22.279 | 25.940 | 13.667 | 1.00 | 22.76 |
| atom | 577 | CB  | ALA | 73 | 20.881 | 26.080 | 14.257 | 1.00 | 17.61 |
| atom | 578 | C   | ALA | 73 | 23.327 | 25.765 | 14.770 | 1.00 | 25.80 |
| atom | 579 | O   | ALA | 73 | 23.894 | 26.740 | 15.250 | 1.00 | 27.15 |
| atom | 580 | N   | LYS | 74 | 23.584 | 24.524 | 15.174 | 1.00 | 28.16 |
| atom | 581 | CA  | LYS | 74 | 24.567 | 24.286 | 16.216 | 1.00 | 25.53 |
| atom | 582 | CB  | LYS | 74 | 24.415 | 22.877 | 16.808 | 1.00 | 26.08 |
| atom | 583 | CG  | LYS | 74 | 23.611 | 22.800 | 18.116 | 1.00 | 29.43 |
| atom | 584 | CD  | LYS | 74 | 24.208 | 23.672 | 19.232 | 1.00 | 31.87 |
| atom | 585 | CE  | LYS | 74 | 24.525 | 22.850 | 20.490 | 1.00 | 36.37 |
| atom | 586 | NZ  | LYS | 74 | 23.999 | 23.451 | 21.763 | 1.00 | 37.56 |
| atom | 587 | C   | LYS | 74 | 25.948 | 24.434 | 15.599 | 1.00 | 27.65 |
| atom | 588 | O   | LYS | 74 | 26.835 | 25.098 | 16.164 | 1.00 | 28.65 |
| atom | 589 | N   | ALA | 75 | 26.127 | 23.827 | 14.428 | 1.00 | 22.91 |
| atom | 590 | CA  | ALA | 75 | 27.414 | 23.873 | 13.754 | 1.00 | 20.17 |
| atom | 591 | CB  | ALA | 75 | 27.379 | 23.020 | 12.538 | 1.00 | 21.44 |
| atom | 592 | C   | ALA | 75 | 27.834 | 25.276 | 13.371 | 1.00 | 21.91 |
| atom | 593 | O   | ALA | 75 | 29.024 | 25.569 | 13.279 | 1.00 | 22.39 |
| atom | 594 | N   | SER | 76 | 26.847 | 26.133 | 13.144 | 1.00 | 24.75 |
| atom | 595 | CA  | SER | 76 | 27.095 | 27.505 | 12.751 | 1.00 | 26.04 |
| atom | 596 | CB  | SER | 76 | 25.829 | 28.114 | 12.164 | 1.00 | 27.50 |
| atom | 597 | OG  | SER | 76 | 25.166 | 28.921 | 13.119 | 1.00 | 27.03 |
| atom | 598 | C   | SER | 76 | 27.534 | 28.277 | 13.975 | 1.00 | 29.40 |
| atom | 599 | O   | SER | 76 | 27.652 | 29.502 | 13.950 | 1.00 | 30.52 |
| atom | 600 | N   | THR | 77 | 27.796 | 27.534 | 15.041 | 1.00 | 29.85 |
| atom | 601 | CA  | THR | 77 | 28.225 | 28.105 | 16.304 | 1.00 | 29.93 |
| atom | 602 | CB  | THR | 77 | 27.400 | 27.476 | 17.436 | 1.00 | 28.64 |
| atom | 603 | OG1 | THR | 77 | 26.580 | 28.490 | 18.021 | 1.00 | 28.24 |
| atom | 604 | CG2 | THR | 77 | 28.276 | 26.812 | 18.471 | 1.00 | 22.34 |
| atom | 605 | C   | THR | 77 | 29.718 | 27.836 | 16.486 | 1.00 | 29.10 |
| atom | 606 | O   | THR | 77 | 30.334 | 28.257 | 17.462 | 1.00 | 23.31 |
| atom | 607 | N   | VAL | 78 | 30.292 | 27.159 | 15.500 | 1.00 | 28.73 |
| atom | 608 | CA  | VAL | 78 | 31.698 | 26.785 | 15.522 | 1.00 | 28.31 |

| atom | 609 | CB  | VAL | 78 | 31.869 | 25.319 | 15.059 | 1.00 22.98 |
|------|-----|-----|-----|----|--------|--------|--------|------------|
| atom | 610 | CG1 | VAL | 78 | 33.326 | 24.954 | 14.999 | 1.00 24.21 |
| atom | 611 | CG2 | VAL | 78 | 31.130 | 24.389 | 16.008 | 1.00 27.29 |
| atom | 612 | C   | VAL | 78 | 32.623 | 27.664 | 14.682 | 1.00 30.31 |
| atom | 613 | O   | VAL | 78 | 32.320 | 28.012 | 13.547 | 1.00 31.32 |
| atom | 614 | N   | LYS | 79 | 33.742 | 28.053 | 15.276 | 1.00 32.75 |
| atom | 615 | CA  | LYS | 79 | 34.746 | 28.820 | 14.567 | 1.00 34.72 |
| atom | 616 | CB  | LYS | 79 | 35.109 | 30.116 | 15.296 | 1.00 33.13 |
| atom | 617 | CG  | LYS | 79 | 36.261 | 30.890 | 14.628 | 1.00 35.72 |
| atom | 618 | CD  | LYS | 79 | 36.018 | 32.411 | 14.590 | 1.00 35.69 |
| atom | 619 | CE  | LYS | 79 | 37.339 | 33.186 | 14.505 | 1.00 33.64 |
| atom | 620 | NZ  | LYS | 79 | 37.140 | 34.666 | 14.643 | 1.00 29.37 |
| atom | 621 | C   | LYS | 79 | 35.933 | 27.862 | 14.567 | 1.00 34.91 |
| atom | 622 | O   | LYS | 79 | 36.405 | 27.444 | 15.617 | 1.00 39.77 |
| atom | 623 | N   | ALA | 80 | 36.367 | 27.466 | 13.383 | 1.00 32.01 |
| atom | 624 | CA  | ALA | 80 | 37.488 | 26.564 | 13.247 | 1.00 31.60 |
| atom | 625 | CB  | ALA | 80 | 37.036 | 25.260 | 12.609 | 1.00 33.03 |
| atom | 626 | C   | ALA | 80 | 38.512 | 27.270 | 12.370 | 1.00 31.62 |
| atom | 627 | O   | ALA | 80 | 38.201 | 28.228 | 11.672 | 1.00 30.86 |
| atom | 628 | N   | LYS | 81 | 39.745 | 26.810 | 12.403 | 1.00 32.87 |
| atom | 629 | CA  | LYS | 81 | 40.744 | 27.473 | 11.605 | 1.00 39.04 |
| atom | 630 | CB  | LYS | 81 | 41.551 | 28.417 | 12.508 | 1.00 44.98 |
| atom | 631 | CG  | LYS | 81 | 40.696 | 29.521 | 13.164 | 1.00 48.22 |
| atom | 632 | CD  | LYS | 81 | 41.090 | 30.929 | 12.696 | 1.00 53.93 |
| atom | 633 | CE  | LYS | 81 | 39.866 | 31.788 | 12.344 | 1.00 60.46 |
| atom | 634 | NZ  | LYS | 81 | 40.061 | 33.259 | 12.607 | 1.00 55.43 |
| atom | 635 | C   | LYS | 81 | 41.637 | 26.476 | 10.884 | 1.00 36.02 |
| atom | 636 | O   | LYS | 81 | 41.836 | 25.363 | 11.348 | 1.00 36.54 |
| atom | 637 | N   | LEU | 82 | 42.157 | 26.871 | 9.732  | 1.00 38.69 |
| atom | 638 | CA  | LEU | 82 | 43.019 | 25.977 | 8.971  | 1.00 41.74 |
| atom | 639 | CB  | LEU | 82 | 43.277 | 26.530 | 7.571  | 1.00 38.50 |
| atom | 640 | CG  | LEU | 82 | 43.827 | 27.943 | 7.506  | 1.00 42.05 |
| atom | 641 | CD1 | LEU | 82 | 45.345 | 27.892 | 7.518  | 1.00 43.62 |
| atom | 642 | CD2 | LEU | 82 | 43.318 | 28.622 | 6.236  | 1.00 44.00 |
| atom | 643 | C   | LEU | 82 | 44.320 | 25.815 | 9.713  | 1.00 40.84 |
| atom | 644 | O   | LEU | 82 | 44.833 | 26.772 | 10.275 | 1.00 45.86 |

| atom | 645 | N   | LEU | 83 | 44.846 | 24.600 | 9.725  | 1.00 | 42.05 |
|------|-----|-----|-----|----|--------|--------|--------|------|-------|
| atom | 646 | CA  | LEU | 83 | 46.093 | 24.318 | 10.412 | 1.00 | 42.03 |
| atom | 647 | CB  | LEU | 83 | 46.077 | 22.891 | 10.955 | 1.00 | 45.18 |
| atom | 648 | CG  | LEU | 83 | 45.682 | 22.763 | 12.428 | 1.00 | 44.22 |
| atom | 649 | CD1 | LEU | 83 | 44.252 | 23.218 | 12.603 | 1.00 | 40.69 |
| atom | 650 | CD2 | LEU | 83 | 45.866 | 21.324 | 12.894 | 1.00 | 48.10 |
| atom | 651 | C   | LEU | 83 | 47.296 | 24.492 | 9.503  | 1.00 | 42.56 |
| atom | 652 | O   | LEU | 83 | 47.434 | 23.788 | 8.500  | 1.00 | 45.28 |
| atom | 653 | N   | SER | 84 | 48.173 | 25.423 | 9.862  | 1.00 | 41.78 |
| atom | 654 | CA  | SER | 84 | 49.377 | 25.676 | 9.076  | 1.00 | 43.36 |
| atom | 655 | CB  | SER | 84 | 50.366 | 26.533 | 9.858  | 1.00 | 41.89 |
| atom | 656 | OG  | SER | 84 | 51.163 | 25.696 | 10.675 | 1.00 | 46.93 |
| atom | 657 | C   | SER | 84 | 50.049 | 24.355 | 8.742  | 1.00 | 43.47 |
| atom | 658 | O   | SER | 84 | 49.926 | 23.387 | 9.487  | 1.00 | 45.37 |
| atom | 659 | N   | VAL | 85 | 50.757 | 24.329 | 7.615  | 1.00 | 42.65 |
| atom | 660 | CA  | VAL | 85 | 51.465 | 23.139 | 7.159  | 1.00 | 40.02 |
| atom | 661 | CB  | VAL | 85 | 52.556 | 23.492 | 6.143  | 1.00 | 40.79 |
| atom | 662 | CG1 | VAL | 85 | 52.603 | 22.432 | 5.039  | 1.00 | 31.56 |
| atom | 663 | CG2 | VAL | 85 | 52.326 | 24.898 | 5.623  | 1.00 | 37.48 |
| atom | 664 | C   | VAL | 85 | 52.158 | 22.399 | 8.283  | 1.00 | 38.35 |
| atom | 665 | O   | VAL | 85 | 51.895 | 21.223 | 8.515  | 1.00 | 35.15 |
| atom | 666 | N   | GLU | 86 | 53.068 | 23.105 | 8.945  | 1.00 | 41.71 |
| atom | 667 | CA  | GLU | 86 | 53.849 | 22.565 | 10.053 | 1.00 | 46.68 |
| atom | 668 | CB  | GLU | 86 | 54.530 | 23.718 | 10.804 | 1.00 | 49.13 |
| atom | 669 | CG  | GLU | 86 | 55.543 | 23.273 | 11.850 | 1.00 | 53.84 |
| atom | 670 | CD  | GLU | 86 | 56.202 | 24.449 | 12.557 | 1.00 | 57.61 |
| atom | 671 | OE1 | GLU | 86 | 56.415 | 25.499 | 11.906 | 1.00 | 57.48 |
| atom | 672 | OE2 | GLU | 86 | 56.509 | 24.322 | 13.763 | 1.00 | 55.26 |
| atom | 673 | C   | GLU | 86 | 53.003 | 21.730 | 11.024 | 1.00 | 45.28 |
| atom | 674 | O   | GLU | 86 | 53.314 | 20.558 | 11.286 | 1.00 | 45.42 |
| atom | 675 | N   | GLU | 87 | 51.938 | 22.336 | 11.550 | 1.00 | 41.57 |
| atom | 676 | CA  | GLU | 87 | 51.050 | 21.648 | 12.480 | 1.00 | 40.92 |
| atom | 677 | CB  | GLU | 87 | 49.897 | 22.558 | 12.911 | 1.00 | 43.41 |
| atom | 678 | CG  | GLU | 87 | 50.218 | 24.031 | 13.033 | 1.00 | 43.17 |
| atom | 679 | CD  | GLU | 87 | 49.103 | 24.781 | 13.736 | 1.00 | 48.23 |
| atom | 680 | OE1 | GLU | 87 | 48.904 | 24.535 | 14.947 | 1.00 | 51.88 |

| atom | 681 | OE2 | GLU | 87 | 48.418 | 25.605 | 13.085 | 1.00 | 45.74 |
|------|-----|-----|-----|----|--------|--------|--------|------|-------|
| atom | 682 | C | GLU | 87 | 50.462 | 20.413 | 11.815 | 1.00 | 40.01 |
| atom | 683 | O | GLU | 87 | 50.352 | 19.343 | 12.424 | 1.00 | 41.18 |
| atom | 684 | N | ALA | 88 | 50.069 | 20.582 | 10.557 | 1.00 | 37.93 |
| atom | 685 | CA | ALA | 88 | 49.484 | 19.509 | 9.787 | 1.00 | 32.05 |
| atom | 686 | CB | ALA | 88 | 49.147 | 20.011 | 8.399 | 1.00 | 38.95 |
| atom | 687 | C | ALA | 88 | 50.455 | 18.345 | 9.719 | 1.00 | 30.85 |
| atom | 688 | O | ALA | 88 | 50.087 | 17.202 | 10.001 | 1.00 | 30.25 |
| atom | 689 | N | CYS | 89 | 51.700 | 18.637 | 9.353 | 1.00 | 33.20 |
| atom | 690 | CA | CYS | 89 | 52.734 | 17.598 | 9.253 | 1.00 | 35.92 |
| atom | 691 | CB | CYS | 89 | 54.068 | 18.195 | 8.781 | 1.00 | 37.45 |
| atom | 692 | SG | CYS | 89 | 54.037 | 19.133 | 7.232 | 1.00 | 39.47 |
| atom | 693 | C | CYS | 89 | 52.935 | 16.940 | 10.618 | 1.00 | 34.78 |
| atom | 694 | O | CYS | 89 | 53.176 | 15.735 | 10.710 | 1.00 | 28.20 |
| atom | 695 | N | LYS | 90 | 52.832 | 17.752 | 11.670 | 1.00 | 36.61 |
| atom | 696 | CA | LYS | 90 | 52.989 | 17.281 | 13.042 | 1.00 | 35.73 |
| atom | 697 | CB | LYS | 90 | 52.757 | 18.446 | 14.004 | 1.00 | 40.25 |
| atom | 698 | CG | LYS | 90 | 53.894 | 19.470 | 14.056 | 1.00 | 39.78 |
| atom | 699 | CD | LYS | 90 | 53.742 | 20.377 | 15.285 | 1.00 | 40.47 |
| atom | 700 | CE | LYS | 90 | 53.888 | 21.857 | 14.931 | 1.00 | 40.65 |
| atom | 701 | NZ | LYS | 90 | 54.520 | 22.655 | 16.026 | 1.00 | 37.75 |
| atom | 702 | C | LYS | 90 | 52.009 | 16.141 | 13.349 | 1.00 | 34.09 |
| atom | 703 | O | LYS | 90 | 52.411 | 15.076 | 13.823 | 1.00 | 33.58 |
| atom | 704 | N | LEU | 91 | 50.727 | 16.361 | 13.067 | 1.00 | 30.64 |
| atom | 705 | CA | LEU | 91 | 49.706 | 15.336 | 13.317 | 1.00 | 31.32 |
| atom | 706 | CB | LEU | 91 | 48.299 | 15.909 | 13.041 | 1.00 | 29.65 |
| atom | 707 | CG | LEU | 91 | 47.937 | 17.261 | 13.661 | 1.00 | 29.77 |
| atom | 708 | CD1 | LEU | 91 | 46.491 | 17.609 | 13.371 | 1.00 | 27.02 |
| atom | 709 | CD2 | LEU | 91 | 48.149 | 17.208 | 15.150 | 1.00 | 28.96 |
| atom | 710 | C | LEU | 91 | 49.894 | 14.045 | 12.495 | 1.00 | 29.18 |
| atom | 711 | O | LEU | 91 | 49.156 | 13.060 | 12.669 | 1.00 | 24.10 |
| atom | 712 | N | THR | 92 | 50.889 | 14.031 | 11.616 | 1.00 | 28.58 |
| atom | 713 | CA | THR | 92 | 51.079 | 12.858 | 10.777 | 1.00 | 28.44 |
| atom | 714 | CB | THR | 92 | 51.677 | 13.241 | 9.420 | 1.00 | 28.48 |
| atom | 715 | OG1 | THR | 92 | 50.954 | 14.357 | 8.884 | 1.00 | 22.03 |
| atom | 716 | CG2 | THR | 92 | 51.604 | 12.057 | 8.458 | 1.00 | 18.87 |

| atom | 717 | C | THR | 92 | 51.904 | 11.743 | 11.397 | 1.00 | 30.83 |
|------|-----|-----|-----|-----|--------|--------|--------|------|-------|
| atom | 718 | O | THR | 92 | 53.055 | 11.937 | 11.790 | 1.00 | 27.27 |
| atom | 719 | N | PRO | 93 | 51.315 | 10.541 | 11.470 | 1.00 | 30.23 |
| atom | 720 | CD | PRO | 93 | 49.962 | 10.222 | 10.983 | 1.00 | 26.81 |
| atom | 721 | CA | PRO | 93 | 51.984 | 9.382 | 12.047 | 1.00 | 31.46 |
| atom | 722 | CB | PRO | 93 | 50.877 | 8.338 | 12.157 | 1.00 | 27.95 |
| atom | 723 | CG | PRO | 93 | 49.875 | 8.737 | 11.148 | 1.00 | 27.44 |
| atom | 724 | C | PRO | 93 | 53.137 | 8.920 | 11.195 | 1.00 | 38.55 |
| atom | 725 | O | PRO | 93 | 53.051 | 8.926 | 9.968 | 1.00 | 42.88 |
| atom | 726 | N | PRO | 94 | 54.247 | 8.526 | 11.839 | 1.00 | 44.22 |
| atom | 727 | CD | PRO | 94 | 54.416 | 8.532 | 13.302 | 1.00 | 42.29 |
| atom | 728 | CA | PRO | 94 | 55.455 | 8.046 | 11.158 | 1.00 | 45.63 |
| atom | 729 | CB | PRO | 94 | 56.553 | 8.167 | 12.220 | 1.00 | 44.07 |
| atom | 730 | CG | PRO | 94 | 55.882 | 8.755 | 13.454 | 1.00 | 41.67 |
| atom | 731 | C | PRO | 94 | 55.256 | 6.610 | 10.710 | 1.00 | 50.09 |
| atom | 732 | O | PRO | 94 | 56.194 | 5.814 | 10.691 | 1.00 | 54.82 |
| atom | 733 | N | HIS | 95 | 54.017 | 6.286 | 10.364 | 1.00 | 53.67 |
| atom | 734 | CA | HIS | 95 | 53.654 | 4.946 | 9.913 | 1.00 | 57.80 |
| atom | 735 | CB | HIS | 95 | 52.855 | 4.220 | 11.006 | 1.00 | 63.80 |
| atom | 736 | CG | HIS | 95 | 53.629 | 3.970 | 12.260 | 1.00 | 69.07 |
| atom | 737 | CD2 | HIS | 95 | 53.856 | 4.756 | 13.340 | 1.00 | 72.38 |
| atom | 738 | ND1 | HIS | 95 | 54.292 | 2.785 | 12.501 | 1.00 | 71.73 |
| atom | 739 | CE1 | HIS | 95 | 54.895 | 2.851 | 13.675 | 1.00 | 73.76 |
| atom | 740 | NE2 | HIS | 95 | 54.647 | 4.036 | 14.205 | 1.00 | 75.05 |
| atom | 741 | C | HIS | 95 | 52.799 | 5.030 | 8.645 | 1.00 | 55.40 |
| atom | 742 | O | HIS | 95 | 52.527 | 4.016 | 7.999 | 1.00 | 55.81 |
| atom | 743 | N | SER | 96 | 52.372 | 6.244 | 8.305 | 1.00 | 53.91 |
| atom | 744 | CA | SER | 96 | 51.531 | 6.472 | 7.133 | 1.00 | 52.92 |
| atom | 745 | CB | SER | 96 | 51.193 | 7.963 | 7.003 | 1.00 | 50.23 |
| atom | 746 | OG | SER | 96 | 49.841 | 8.210 | 7.345 | 1.00 | 52.35 |
| atom | 747 | C | SER | 96 | 52.163 | 5.979 | 5.835 | 1.00 | 51.06 |
| atom | 748 | O | SER | 96 | 53.388 | 5.939 | 5.698 | 1.00 | 49.44 |
| atom | 749 | N | ALA | 97 | 51.307 | 5.593 | 4.890 | 1.00 | 49.46 |
| atom | 750 | CA | ALA | 97 | 51.756 | 5.121 | 3.591 | 1.00 | 45.65 |
| atom | 751 | CB | ALA | 97 | 50.561 | 4.674 | 2.764 | 1.00 | 44.44 |
| atom | 752 | C | ALA | 97 | 52.468 | 6.294 | 2.925 | 1.00 | 42.76 |

| atom | 753 | O | ALA | 97 | 51.954 | 7.416 | 2.924 | 1.00 | 40.92 |
|------|-----|-----|-----|-----|--------|-------|-------|------|-------|
| atom | 754 | N | LYS | 98 | 53.649 | 6.043 | 2.370 | 1.00 | 41.52 |
| atom | 755 | CA | LYS | 98 | 54.407 | 7.113 | 1.740 | 1.00 | 42.05 |
| atom | 756 | CB | LYS | 98 | 55.803 | 6.637 | 1.325 | 1.00 | 45.33 |
| atom | 757 | CG | LYS | 98 | 55.893 | 5.176 | 0.910 | 1.00 | 52.15 |
| atom | 758 | CD | LYS | 98 | 57.225 | 4.882 | 0.200 | 1.00 | 55.56 |
| atom | 759 | CE | LYS | 98 | 57.951 | 3.666 | 0.803 | 1.00 | 60.84 |
| atom | 760 | NZ | LYS | 98 | 59.091 | 4.017 | 1.720 | 1.00 | 57.80 |
| atom | 761 | C | LYS | 98 | 53.682 | 7.669 | 0.540 | 1.00 | 40.45 |
| atom | 762 | O | LYS | 98 | 52.762 | 7.048 | 0.009 | 1.00 | 38.78 |
| atom | 763 | N | SER | 99 | 54.094 | 8.860 | 0.131 | 1.00 | 39.53 |
| atom | 764 | CA | SER | 99 | 53.502 | 9.535 | -1.019 | 1.00 | 38.92 |
| atom | 765 | CB | SER | 99 | 53.896 | 11.017 | -1.007 | 1.00 | 37.52 |
| atom | 766 | OG | SER | 99 | 53.532 | 11.664 | -2.210 | 1.00 | 39.62 |
| atom | 767 | C | SER | 99 | 54.015 | 8.874 | -2.290 | 1.00 | 38.92 |
| atom | 768 | O | SER | 99 | 55.129 | 8.339 | -2.320 | 1.00 | 37.85 |
| atom | 769 | N | LYS | 100 | 53.199 | 8.895 | -3.335 | 1.00 | 38.61 |
| atom | 770 | CA | LYS | 100 | 53.610 | 8.314 | -4.603 | 1.00 | 38.93 |
| atom | 771 | CB | LYS | 100 | 52.384 | 7.997 | -5.472 | 1.00 | 39.20 |
| atom | 772 | CG | LYS | 100 | 51.381 | 7.066 | -4.797 | 1.00 | 41.56 |
| atom | 773 | CD | LYS | 100 | 50.672 | 6.138 | -5.782 | 1.00 | 42.55 |
| atom | 774 | CE | LYS | 100 | 49.883 | 6.929 | -6.824 | 1.00 | 46.92 |
| atom | 775 | NZ | LYS | 100 | 48.395 | 6.748 | -6.711 | 1.00 | 45.76 |
| atom | 776 | C | LYS | 100 | 54.470 | 9.372 | -5.272 | 1.00 | 36.95 |
| atom | 777 | O | LYS | 100 | 54.808 | 9.277 | -6.444 | 1.00 | 41.42 |
| atom | 778 | N | PHE | 101 | 54.825 | 10.388 | -4.503 | 1.00 | 36.94 |
| atom | 779 | CA | PHE | 101 | 55.625 | 11.482 | -5.018 | 1.00 | 36.82 |
| atom | 780 | CB | PHE | 101 | 54.877 | 12.805 | -4.833 | 1.00 | 38.05 |
| atom | 781 | CG | PHE | 101 | 53.559 | 12.877 | -5.566 | 1.00 | 41.28 |
| atom | 782 | CD1 | PHE | 101 | 52.392 | 12.400 | -4.977 | 1.00 | 43.57 |
| atom | 783 | CD2 | PHE | 101 | 53.480 | 13.455 | -6.827 | 1.00 | 44.60 |
| atom | 784 | CE1 | PHE | 101 | 51.164 | 12.499 | -5.630 | 1.00 | 41.45 |
| atom | 785 | CE2 | PHE | 101 | 52.254 | 13.560 | -7.489 | 1.00 | 46.72 |
| atom | 786 | CZ | PHE | 101 | 51.095 | 13.079 | -6.883 | 1.00 | 43.73 |
| atom | 787 | C | PHE | 101 | 57.015 | 11.600 | -4.397 | 1.00 | 35.89 |
| atom | 788 | O | PHE | 101 | 57.520 | 12.708 | -4.234 | 1.00 | 36.78 |

| atom | 789 | N   | GLY | 102 | 57.625 | 10.479 | -4.029 | 1.00 | 32.70 |
| atom | 790 | CA  | GLY | 102 | 58.973 | 10.550 | -3.491 | 1.00 | 37.31 |
| atom | 791 | C   | GLY | 102 | 59.210 | 10.698 | -1.998 | 1.00 | 40.11 |
| atom | 792 | O   | GLY | 102 | 60.090 | 10.035 | -1.452 | 1.00 | 43.81 |
| atom | 793 | N   | TYR | 103 | 58.446 | 11.559 | -1.334 | 1.00 | 39.26 |
| atom | 794 | CA  | TYR | 103 | 58.609 | 11.784 | 0.096  | 1.00 | 35.00 |
| atom | 795 | CB  | TYR | 103 | 58.399 | 13.270 | 0.409  | 1.00 | 38.56 |
| atom | 796 | CG  | TYR | 103 | 56.984 | 13.746 | 0.170  | 1.00 | 43.82 |
| atom | 797 | CD1 | TYR | 103 | 56.087 | 13.896 | 1.231  | 1.00 | 44.08 |
| atom | 798 | CE1 | TYR | 103 | 54.774 | 14.281 | 1.009  | 1.00 | 39.21 |
| atom | 799 | CD2 | TYR | 103 | 56.522 | 14.003 | -1.123 | 1.00 | 41.55 |
| atom | 800 | CE2 | TYR | 103 | 55.208 | 14.389 | -1.350 | 1.00 | 38.72 |
| atom | 801 | CZ  | TYR | 103 | 54.343 | 14.518 | -0.281 | 1.00 | 39.56 |
| atom | 802 | OH  | TYR | 103 | 53.031 | 14.835 | -0.515 | 1.00 | 37.93 |
| atom | 803 | C   | TYR | 103 | 57.665 | 10.942 | 0.951  | 1.00 | 32.59 |
| atom | 804 | O   | TYR | 103 | 56.492 | 10.797 | 0.632  | 1.00 | 29.69 |
| atom | 805 | N   | GLY | 104 | 58.191 | 10.408 | 2.052  | 1.00 | 32.00 |
| atom | 806 | CA  | GLY | 104 | 57.394 | 9.589  | 2.952  | 1.00 | 25.84 |
| atom | 807 | C   | GLY | 104 | 56.983 | 10.318 | 4.223  | 1.00 | 25.38 |
| atom | 808 | O   | GLY | 104 | 57.114 | 11.548 | 4.312  | 1.00 | 24.18 |
| atom | 809 | N   | ALA | 105 | 56.508 | 9.550  | 5.209  | 1.00 | 24.20 |
| atom | 810 | CA  | ALA | 105 | 56.037 | 10.080 | 6.494  | 1.00 | 23.76 |
| atom | 811 | CB  | ALA | 105 | 55.611 | 8.922  | 7.407  | 1.00 | 26.92 |
| atom | 812 | C   | ALA | 105 | 57.011 | 10.984 | 7.234  | 1.00 | 24.90 |
| atom | 813 | O   | ALA | 105 | 56.660 | 12.095 | 7.592  | 1.00 | 25.76 |
| atom | 814 | N   | LYS | 106 | 58.233 | 10.516 | 7.461  | 1.00 | 30.07 |
| atom | 815 | CA  | LYS | 106 | 59.236 | 11.306 | 8.177  | 1.00 | 33.58 |
| atom | 816 | CB  | LYS | 106 | 60.552 | 10.538 | 8.272  | 1.00 | 35.25 |
| atom | 817 | CG  | LYS | 106 | 60.434 | 9.081  | 8.660  | 1.00 | 37.37 |
| atom | 818 | CD  | LYS | 106 | 61.755 | 8.348  | 8.415  | 1.00 | 39.21 |
| atom | 819 | CE  | LYS | 106 | 62.944 | 9.043  | 9.098  | 1.00 | 42.05 |
| atom | 820 | NZ  | LYS | 106 | 63.712 | 9.962  | 8.187  | 1.00 | 45.24 |
| atom | 821 | C   | LYS | 106 | 59.510 | 12.683 | 7.554  | 1.00 | 38.58 |
| atom | 822 | O   | LYS | 106 | 59.422 | 13.715 | 8.236  | 1.00 | 41.15 |
| atom | 823 | N   | ASP | 107 | 59.853 | 12.704 | 6.268  | 1.00 | 38.63 |
| atom | 824 | CA  | ASP | 107 | 60.118 | 13.969 | 5.594  | 1.00 | 38.65 |

| | | | | | | | | | |
|------|-----|-----|-----|-----|--------|--------|--------|------|-------|
| atom | 825 | CB  | ASP | 107 | 60.298 | 13.752 | 4.096  | 1.00 | 40.04 |
| atom | 826 | CG  | ASP | 107 | 61.279 | 12.671 | 3.798  | 1.00 | 40.25 |
| atom | 827 | OD1 | ASP | 107 | 60.843 | 11.515 | 3.609  | 1.00 | 44.96 |
| atom | 828 | OD2 | ASP | 107 | 62.486 | 12.982 | 3.772  | 1.00 | 40.23 |
| atom | 829 | C   | ASP | 107 | 58.950 | 14.903 | 5.832  | 1.00 | 38.71 |
| atom | 830 | O   | ASP | 107 | 59.133 | 16.118 | 5.913  | 1.00 | 40.03 |
| atom | 831 | N   | VAL | 108 | 57.754 | 14.323 | 5.938  | 1.00 | 38.49 |
| atom | 832 | CA  | VAL | 108 | 56.544 | 15.093 | 6.190  | 1.00 | 39.25 |
| atom | 833 | CB  | VAL | 108 | 55.254 | 14.240 | 6.177  | 1.00 | 38.78 |
| atom | 834 | CG1 | VAL | 108 | 54.088 | 15.088 | 6.680  | 1.00 | 36.21 |
| atom | 835 | CG2 | VAL | 108 | 54.968 | 13.715 | 4.776  | 1.00 | 38.69 |
| atom | 836 | C   | VAL | 108 | 56.639 | 15.698 | 7.570  | 1.00 | 40.88 |
| atom | 837 | O   | VAL | 108 | 56.140 | 16.798 | 7.798  | 1.00 | 42.42 |
| atom | 838 | N   | ARG | 109 | 57.259 | 14.982 | 8.501  | 1.00 | 39.54 |
| atom | 839 | CA  | ARG | 109 | 57.375 | 15.533 | 9.839  | 1.00 | 44.71 |
| atom | 840 | CB  | ARG | 109 | 57.485 | 14.429 | 10.897 | 1.00 | 44.04 |
| atom | 841 | CG  | ARG | 109 | 57.175 | 14.978 | 12.297 | 1.00 | 46.40 |
| atom | 842 | CD  | ARG | 109 | 57.340 | 13.959 | 13.411 | 1.00 | 46.18 |
| atom | 843 | NE  | ARG | 109 | 56.074 | 13.345 | 13.797 | 1.00 | 40.34 |
| atom | 844 | CZ  | ARG | 109 | 55.647 | 12.189 | 13.307 | 1.00 | 43.34 |
| atom | 845 | NH1 | ARG | 109 | 56.390 | 11.536 | 12.418 | 1.00 | 44.24 |
| atom | 846 | NH2 | ARG | 109 | 54.488 | 11.682 | 13.701 | 1.00 | 39.95 |
| atom | 847 | C   | ARG | 109 | 58.557 | 16.492 | 9.979  | 1.00 | 44.27 |
| atom | 848 | O   | ARG | 109 | 58.488 | 17.464 | 10.733 | 1.00 | 45.55 |
| atom | 849 | N   | ASN | 110 | 59.628 | 16.237 | 9.235  | 1.00 | 42.68 |
| atom | 850 | CA  | ASN | 110 | 60.813 | 17.080 | 9.318  | 1.00 | 41.58 |
| atom | 851 | CB  | ASN | 110 | 62.028 | 16.327 | 8.776  | 1.00 | 42.25 |
| atom | 852 | CG  | ASN | 110 | 62.223 | 14.976 | 9.444  | 1.00 | 43.61 |
| atom | 853 | OD1 | ASN | 110 | 61.431 | 14.562 | 10.297 | 1.00 | 43.54 |
| atom | 854 | ND2 | ASN | 110 | 63.282 | 14.277 | 9.055  | 1.00 | 49.56 |
| atom | 855 | C   | ASN | 110 | 60.675 | 18.411 | 8.594  | 1.00 | 40.58 |
| atom | 856 | O   | ASN | 110 | 61.528 | 19.289 | 8.737  | 1.00 | 40.24 |
| atom | 857 | N   | LEU | 111 | 59.600 | 18.560 | 7.828  | 1.00 | 39.98 |
| atom | 858 | CA  | LEU | 111 | 59.350 | 19.787 | 7.061  | 1.00 | 40.96 |
| atom | 859 | CB  | LEU | 111 | 59.470 | 21.027 | 7.981  | 1.00 | 43.73 |
| atom | 860 | CG  | LEU | 111 | 58.245 | 21.493 | 8.806  | 1.00 | 44.77 |

| atom | 861 | CD1 | LEU | 111 | 58.270 | 23.014 | 8.942 | 1.00 | 43.32 |
|------|-----|-----|-----|-----|--------|--------|-------|------|-------|
| atom | 862 | CD2 | LEU | 111 | 56.932 | 21.055 | 8.136 | 1.00 | 43.76 |
| atom | 863 | C | LEU | 111 | 60.313 | 19.903 | 5.861 | 1.00 | 36.53 |
| atom | 864 | O | LEU | 111 | 60.836 | 20.974 | 5.581 | 1.00 | 33.61 |
| atom | 865 | N | SER | 112 | 60.521 | 18.784 | 5.161 | 1.00 | 37.08 |
| atom | 866 | CA | SER | 112 | 61.408 | 18.702 | 3.993 | 1.00 | 37.95 |
| atom | 867 | CB | SER | 112 | 61.636 | 17.239 | 3.620 | 1.00 | 35.33 |
| atom | 868 | OG | SER | 112 | 61.881 | 16.443 | 4.772 | 1.00 | 40.02 |
| atom | 869 | C | SER | 112 | 60.896 | 19.451 | 2.758 | 1.00 | 42.04 |
| atom | 870 | O | SER | 112 | 59.685 | 19.503 | 2.507 | 1.00 | 43.96 |
| atom | 871 | N | SER | 113 | 61.821 | 20.017 | 1.979 | 1.00 | 42.58 |
| atom | 872 | CA | SER | 113 | 61.442 | 20.762 | 0.777 | 1.00 | 42.22 |
| atom | 873 | CB | SER | 113 | 62.692 | 21.302 | 0.038 | 1.00 | 39.75 |
| atom | 874 | OG | SER | 113 | 63.534 | 20.272 | -0.463 | 1.00 | 37.28 |
| atom | 875 | C | SER | 113 | 60.580 | 19.920 | -0.174 | 1.00 | 41.06 |
| atom | 876 | O | SER | 113 | 59.622 | 20.429 | -0.761 | 1.00 | 36.23 |
| atom | 877 | N | LYS | 114 | 60.899 | 18.636 | -0.320 | 1.00 | 37.37 |
| atom | 878 | CA | LYS | 114 | 60.102 | 17.811 | -1.219 | 1.00 | 37.95 |
| atom | 879 | CB | LYS | 114 | 60.759 | 16.434 | -1.449 | 1.00 | 39.06 |
| atom | 880 | CG | LYS | 114 | 61.015 | 15.611 | -0.194 | 1.00 | 43.80 |
| atom | 881 | CD | LYS | 114 | 62.256 | 14.721 | -0.361 | 1.00 | 44.68 |
| atom | 882 | CE | LYS | 114 | 61.916 | 13.233 | -0.326 | 1.00 | 45.40 |
| atom | 883 | NZ | LYS | 114 | 61.253 | 12.772 | -1.592 | 1.00 | 45.38 |
| atom | 884 | C | LYS | 114 | 58.708 | 17.650 | -0.630 | 1.00 | 33.14 |
| atom | 885 | O | LYS | 114 | 57.705 | 17.668 | -1.348 | 1.00 | 29.12 |
| atom | 886 | N | ALA | 115 | 58.645 | 17.515 | 0.688 | 1.00 | 30.70 |
| atom | 887 | CA | ALA | 115 | 57.363 | 17.346 | 1.351 | 1.00 | 27.33 |
| atom | 888 | CB | ALA | 115 | 57.576 | 16.838 | 2.757 | 1.00 | 29.72 |
| atom | 889 | C | ALA | 115 | 56.540 | 18.623 | 1.370 | 1.00 | 25.15 |
| atom | 890 | O | ALA | 115 | 55.506 | 18.710 | 0.720 | 1.00 | 25.23 |
| atom | 891 | N | VAL | 116 | 56.997 | 19.633 | 2.091 | 1.00 | 25.45 |
| atom | 892 | CA | VAL | 116 | 56.208 | 20.850 | 2.160 | 1.00 | 29.58 |
| atom | 893 | CB | VAL | 116 | 56.807 | 21.875 | 3.170 | 1.00 | 31.94 |
| atom | 894 | CG1 | VAL | 116 | 57.757 | 21.178 | 4.135 | 1.00 | 30.62 |
| atom | 895 | CG2 | VAL | 116 | 57.486 | 23.011 | 2.432 | 1.00 | 32.05 |
| atom | 896 | C | VAL | 116 | 55.982 | 21.526 | 0.814 | 1.00 | 30.56 |

| atom | 897 | O | VAL | 116 | 55.136 | 22.424 | 0.691 | 1.00 | 31.18 |
|------|-----|-----|-----|-----|--------|--------|--------|------|-------|
| atom | 898 | N | ASN | 117 | 56.741 | 21.107 | -0.197 | 1.00 | 34.10 |
| atom | 899 | CA | ASN | 117 | 56.585 | 21.673 | -1.534 | 1.00 | 28.81 |
| atom | 900 | CB | ASN | 117 | 57.845 | 21.486 | -2.352 | 1.00 | 30.56 |
| atom | 901 | CG | ASN | 117 | 58.738 | 22.691 | -2.281 | 1.00 | 34.70 |
| atom | 902 | OD1 | ASN | 117 | 58.260 | 23.812 | -2.089 | 1.00 | 33.31 |
| atom | 903 | ND2 | ASN | 117 | 60.044 | 22.477 | -2.421 | 1.00 | 34.40 |
| atom | 904 | C | ASN | 117 | 55.416 | 20.997 | -2.214 | 1.00 | 30.73 |
| atom | 905 | O | ASN | 117 | 54.545 | 21.674 | -2.774 | 1.00 | 32.39 |
| atom | 906 | N | HIS | 118 | 55.383 | 19.665 | -2.168 | 1.00 | 28.42 |
| atom | 907 | CA | HIS | 118 | 54.260 | 18.959 | -2.757 | 1.00 | 29.70 |
| atom | 908 | CB | HIS | 118 | 54.415 | 17.447 | -2.645 | 1.00 | 30.32 |
| atom | 909 | CG | HIS | 118 | 53.374 | 16.692 | -3.412 | 1.00 | 33.54 |
| atom | 910 | CD2 | HIS | 118 | 53.242 | 16.450 | -4.740 | 1.00 | 35.37 |
| atom | 911 | ND1 | HIS | 118 | 52.283 | 16.106 | -2.809 | 1.00 | 36.29 |
| atom | 912 | CE1 | HIS | 118 | 51.524 | 15.536 | -3.729 | 1.00 | 34.25 |
| atom | 913 | NE2 | HIS | 118 | 52.083 | 15.732 | -4.910 | 1.00 | 34.76 |
| atom | 914 | C | HIS | 118 | 52.985 | 19.387 | -2.032 | 1.00 | 29.99 |
| atom | 915 | O | HIS | 118 | 51.999 | 19.755 | -2.677 | 1.00 | 29.65 |
| atom | 916 | N | ILE | 119 | 53.007 | 19.358 | -0.696 | 1.00 | 27.46 |
| atom | 917 | CA | ILE | 119 | 51.830 | 19.758 | 0.078 | 1.00 | 24.42 |
| atom | 918 | CB | ILE | 119 | 52.118 | 19.945 | 1.620 | 1.00 | 24.65 |
| atom | 919 | CG2 | ILE | 119 | 50.793 | 20.085 | 2.361 | 1.00 | 20.54 |
| atom | 920 | CG1 | ILE | 119 | 52.950 | 18.796 | 2.211 | 1.00 | 17.58 |
| atom | 921 | CD1 | ILE | 119 | 52.759 | 17.472 | 1.551 | 1.00 | 15.70 |
| atom | 922 | C | ILE | 119 | 51.294 | 21.100 | -0.437 | 1.00 | 25.51 |
| atom | 923 | O | ILE | 119 | 50.091 | 21.243 | -0.673 | 1.00 | 29.09 |
| atom | 924 | N | HIS | 120 | 52.178 | 22.079 | -0.617 | 1.00 | 23.27 |
| atom | 925 | CA | HIS | 120 | 51.754 | 23.406 | -1.080 | 1.00 | 28.30 |
| atom | 926 | CB | HIS | 120 | 52.927 | 24.401 | -1.069 | 1.00 | 30.07 |
| atom | 927 | CG | HIS | 120 | 53.039 | 25.200 | 0.197 | 1.00 | 33.49 |
| atom | 928 | CD2 | HIS | 120 | 52.237 | 26.162 | 0.719 | 1.00 | 35.05 |
| atom | 929 | ND1 | HIS | 120 | 54.108 | 25.086 | 1.063 | 1.00 | 30.46 |
| atom | 930 | CE1 | HIS | 120 | 53.961 | 25.944 | 2.057 | 1.00 | 34.16 |
| atom | 931 | NE2 | HIS | 120 | 52.834 | 26.610 | 1.872 | 1.00 | 30.63 |
| atom | 932 | C | HIS | 120 | 51.190 | 23.321 | -2.483 | 1.00 | 27.89 |

| atom | 933 | O | HIS | 120 | 50.467 | 24.209 | -2.934 | 1.00 | 28.53 |
| atom | 934 | N | SER | 121 | 51.524 | 22.236 | -3.163 | 1.00 | 27.55 |
| atom | 935 | CA | SER | 121 | 51.071 | 22.030 | -4.517 | 1.00 | 28.43 |
| atom | 936 | CB | SER | 121 | 52.001 | 21.055 | -5.219 | 1.00 | 29.05 |
| atom | 937 | OG | SER | 121 | 51.649 | 19.736 | -4.876 | 1.00 | 39.03 |
| atom | 938 | C | SER | 121 | 49.642 | 21.512 | -4.531 | 1.00 | 29.23 |
| atom | 939 | O | SER | 121 | 48.781 | 22.064 | -5.234 | 1.00 | 27.16 |
| atom | 940 | N | VAL | 122 | 49.397 | 20.448 | -3.765 | 1.00 | 24.86 |
| atom | 941 | CA | VAL | 122 | 48.061 | 19.871 | -3.671 | 1.00 | 19.27 |
| atom | 942 | CB | VAL | 122 | 47.981 | 18.827 | -2.557 | 1.00 | 18.10 |
| atom | 943 | CG1 | VAL | 122 | 46.710 | 18.056 | -2.670 | 1.00 | 19.73 |
| atom | 944 | CG2 | VAL | 122 | 49.175 | 17.901 | -2.612 | 1.00 | 14.09 |
| atom | 945 | C | VAL | 122 | 47.122 | 21.014 | -3.306 | 1.00 | 24.73 |
| atom | 946 | O | VAL | 122 | 46.080 | 21.214 | -3.937 | 1.00 | 29.01 |
| atom | 947 | N | TRP | 123 | 47.518 | 21.778 | -2.294 | 1.00 | 24.11 |
| atom | 948 | CA | TRP | 123 | 46.736 | 22.905 | -1.817 | 1.00 | 23.68 |
| atom | 949 | CB | TRP | 123 | 47.474 | 23.584 | -0.658 | 1.00 | 21.51 |
| atom | 950 | CG | TRP | 123 | 46.631 | 24.562 | 0.083 | 1.00 | 24.63 |
| atom | 951 | CD2 | TRP | 123 | 45.661 | 24.270 | 1.100 | 1.00 | 24.83 |
| atom | 952 | CE2 | TRP | 123 | 45.100 | 25.507 | 1.512 | 1.00 | 26.60 |
| atom | 953 | CE3 | TRP | 123 | 45.205 | 23.086 | 1.703 | 1.00 | 21.62 |
| atom | 954 | CD1 | TRP | 123 | 46.620 | 25.922 | -0.076 | 1.00 | 25.58 |
| atom | 955 | NE1 | TRP | 123 | 45.705 | 26.497 | 0.779 | 1.00 | 26.32 |
| atom | 956 | CZ2 | TRP | 123 | 44.103 | 25.589 | 2.505 | 1.00 | 23.81 |
| atom | 957 | CZ3 | TRP | 123 | 44.208 | 23.173 | 2.697 | 1.00 | 17.40 |
| atom | 958 | CH2 | TRP | 123 | 43.674 | 24.411 | 3.080 | 1.00 | 19.50 |
| atom | 959 | C | TRP | 123 | 46.437 | 23.932 | -2.908 | 1.00 | 27.15 |
| atom | 960 | O | TRP | 123 | 45.278 | 24.340 | -3.116 | 1.00 | 26.13 |
| atom | 961 | N | LYS | 124 | 47.489 | 24.368 | -3.592 | 1.00 | 27.03 |
| atom | 962 | CA | LYS | 124 | 47.331 | 25.343 | -4.652 | 1.00 | 27.60 |
| atom | 963 | CB | LYS | 124 | 48.684 | 25.610 | -5.310 | 1.00 | 33.72 |
| atom | 964 | CG | LYS | 124 | 49.053 | 27.077 | -5.428 | 1.00 | 40.99 |
| atom | 965 | CD | LYS | 124 | 49.540 | 27.423 | -6.833 | 1.00 | 41.84 |
| atom | 966 | CE | LYS | 124 | 48.672 | 28.500 | -7.485 | 1.00 | 47.34 |
| atom | 967 | NZ | LYS | 124 | 47.546 | 28.970 | -6.607 | 1.00 | 50.55 |
| atom | 968 | C | LYS | 124 | 46.347 | 24.749 | -5.669 | 1.00 | 30.14 |

| atom | 969 | O | LYS | 124 | 45.470 | 25.442 | -6.194 | 1.00 | 32.78 |
|------|-----|----|-----|-----|--------|--------|--------|------|-------|
| atom | 970 | N | ASP | 125 | 46.490 | 23.454 | -5.928 | 1.00 | 24.17 |
| atom | 971 | CA | ASP | 125 | 45.624 | 22.772 | -6.865 | 1.00 | 26.07 |
| atom | 972 | CB | ASP | 125 | 46.210 | 21.410 | -7.197 | 1.00 | 28.24 |
| atom | 973 | CG | ASP | 125 | 45.393 | 20.679 | -8.211 | 1.00 | 30.03 |
| atom | 974 | OD1 | ASP | 125 | 44.765 | 19.662 | -7.849 | 1.00 | 31.48 |
| atom | 975 | OD2 | ASP | 125 | 45.375 | 21.131 | -9.373 | 1.00 | 34.25 |
| atom | 976 | C | ASP | 125 | 44.198 | 22.602 | -6.332 | 1.00 | 27.25 |
| atom | 977 | O | ASP | 125 | 43.231 | 22.810 | -7.057 | 1.00 | 23.54 |
| atom | 978 | N | LEU | 126 | 44.078 | 22.214 | -5.061 | 1.00 | 29.03 |
| atom | 979 | CA | LEU | 126 | 42.777 | 22.032 | -4.417 | 1.00 | 26.92 |
| atom | 980 | CB | LEU | 126 | 42.953 | 21.695 | -2.932 | 1.00 | 31.95 |
| atom | 981 | CG | LEU | 126 | 42.677 | 20.298 | -2.347 | 1.00 | 31.46 |
| atom | 982 | CD1 | LEU | 126 | 42.691 | 19.259 | -3.428 | 1.00 | 31.01 |
| atom | 983 | CD2 | LEU | 126 | 43.732 | 19.964 | -1.291 | 1.00 | 27.21 |
| atom | 984 | C | LEU | 126 | 42.000 | 23.325 | -4.544 | 1.00 | 28.21 |
| atom | 985 | O | LEU | 126 | 40.787 | 23.314 | -4.721 | 1.00 | 31.19 |
| atom | 986 | N | LEU | 127 | 42.695 | 24.453 | -4.450 | 1.00 | 32.51 |
| atom | 987 | CA | LEU | 127 | 42.021 | 25.749 | -4.582 | 1.00 | 33.82 |
| atom | 988 | CB | LEU | 127 | 42.862 | 26.874 | -3.959 | 1.00 | 33.73 |
| atom | 989 | CG | LEU | 127 | 42.570 | 27.333 | -2.520 | 1.00 | 32.63 |
| atom | 990 | CD1 | LEU | 127 | 43.875 | 27.614 | -1.812 | 1.00 | 32.67 |
| atom | 991 | CD2 | LEU | 127 | 41.709 | 28.582 | -2.521 | 1.00 | 32.52 |
| atom | 992 | C | LEU | 127 | 41.747 | 26.071 | -6.051 | 1.00 | 34.67 |
| atom | 993 | O | LEU | 127 | 40.684 | 26.581 | -6.389 | 1.00 | 35.01 |
| atom | 994 | N | GLU | 128 | 42.697 | 25.750 | -6.925 | 1.00 | 37.52 |
| atom | 995 | CA | GLU | 128 | 42.554 | 26.037 | -8.357 | 1.00 | 42.22 |
| atom | 996 | CB | GLU | 128 | 43.903 | 25.864 | -9.064 | 1.00 | 44.81 |
| atom | 997 | CG | GLU | 128 | 44.962 | 26.886 | -8.659 | 1.00 | 48.63 |
| atom | 998 | CD | GLU | 128 | 46.323 | 26.622 | -9.300 | 1.00 | 49.49 |
| atom | 999 | OE1 | GLU | 128 | 46.840 | 25.485 | -9.203 | 1.00 | 41.93 |
| atom | 1000 | OE2 | GLU | 128 | 46.878 | 27.566 | -9.904 | 1.00 | 55.45 |
| atom | 1001 | C | GLU | 128 | 41.507 | 25.192 | -9.090 | 1.00 | 43.41 |
| atom | 1002 | O | GLU | 128 | 40.710 | 25.717 | -9.882 | 1.00 | 43.52 |
| atom | 1003 | N | ASP | 129 | 41.517 | 23.886 | -8.829 | 1.00 | 41.66 |
| atom | 1004 | CA | ASP | 129 | 40.594 | 22.958 | -9.477 | 1.00 | 40.83 |

| atom | 1005 | CB  | ASP | 129 | 41.415 | 21.847 | -10.155 | 1.00 | 36.59 |
|------|------|-----|-----|-----|--------|--------|---------|------|-------|
| atom | 1006 | CG  | ASP | 129 | 40.556 | 20.706 | -10.689 | 1.00 | 37.69 |
| atom | 1007 | OD1 | ASP | 129 | 41.134 | 19.702 | -11.161 | 1.00 | 33.70 |
| atom | 1008 | OD2 | ASP | 129 | 39.315 | 20.808 | -10.635 | 1.00 | 33.52 |
| atom | 1009 | C   | ASP | 129 | 39.590 | 22.371 | -8.475  | 1.00 | 42.15 |
| atom | 1010 | O   | ASP | 129 | 39.976 | 21.865 | -7.417  | 1.00 | 46.87 |
| atom | 1011 | N   | THR | 130 | 38.304 | 22.433 | -8.811  | 1.00 | 37.86 |
| atom | 1012 | CA  | THR | 130 | 37.261 | 21.914 | -7.930  | 1.00 | 34.06 |
| atom | 1013 | CB  | THR | 130 | 36.360 | 23.037 | -7.449  | 1.00 | 33.25 |
| atom | 1014 | OG1 | THR | 130 | 37.053 | 24.282 | -7.581  | 1.00 | 40.23 |
| atom | 1015 | CG2 | THR | 130 | 35.996 | 22.816 | -5.998  | 1.00 | 40.96 |
| atom | 1016 | C   | THR | 130 | 36.367 | 20.840 | -8.543  | 1.00 | 28.72 |
| atom | 1017 | O   | THR | 130 | 35.238 | 20.625 | -8.092  | 1.00 | 26.45 |
| atom | 1018 | N   | VAL | 131 | 36.869 | 20.158 | -9.560  | 1.00 | 24.00 |
| atom | 1019 | CA  | VAL | 131 | 36.084 | 19.127 | -10.212 | 1.00 | 22.33 |
| atom | 1020 | CB  | VAL | 131 | 35.417 | 19.654 | -11.529 | 1.00 | 22.42 |
| atom | 1021 | CG1 | VAL | 131 | 34.437 | 20.761 | -11.221 | 1.00 | 16.48 |
| atom | 1022 | CG2 | VAL | 131 | 36.482 | 20.152 | -12.502 | 1.00 | 20.73 |
| atom | 1023 | C   | VAL | 131 | 36.869 | 17.876 | -10.575 | 1.00 | 23.48 |
| atom | 1024 | O   | VAL | 131 | 36.292 | 16.799 | -10.689 | 1.00 | 29.07 |
| atom | 1025 | N   | THR | 132 | 38.174 | 17.981 | -10.748 | 1.00 | 21.43 |
| atom | 1026 | CA  | THR | 132 | 38.876 | 16.783 | -11.167 | 1.00 | 26.51 |
| atom | 1027 | CB  | THR | 132 | 40.326 | 17.069 | -11.656 | 1.00 | 28.03 |
| atom | 1028 | OG1 | THR | 132 | 40.295 | 18.039 | -12.706 | 1.00 | 29.16 |
| atom | 1029 | CG2 | THR | 132 | 40.979 | 15.776 | -12.191 | 1.00 | 20.81 |
| atom | 1030 | C   | THR | 132 | 38.944 | 15.695 | -10.121 | 1.00 | 25.49 |
| atom | 1031 | O   | THR | 132 | 39.544 | 15.864 | -9.075  | 1.00 | 21.33 |
| atom | 1032 | N   | PRO | 133 | 38.324 | 14.547 | -10.401 | 1.00 | 24.92 |
| atom | 1033 | CD  | PRO | 133 | 37.538 | 14.190 | -11.591 | 1.00 | 24.51 |
| atom | 1034 | CA  | PRO | 133 | 38.373 | 13.459 | -9.424  | 1.00 | 24.23 |
| atom | 1035 | CB  | PRO | 133 | 37.913 | 12.255 | -10.222 | 1.00 | 20.97 |
| atom | 1036 | CG  | PRO | 133 | 36.987 | 12.831 | -11.232 | 1.00 | 23.00 |
| atom | 1037 | C   | PRO | 133 | 39.803 | 13.290 | -8.921  | 1.00 | 23.05 |
| atom | 1038 | O   | PRO | 133 | 40.754 | 13.507 | -9.666  | 1.00 | 18.59 |
| atom | 1039 | N   | ILE | 134 | 39.971 | 12.950 | -7.647  | 1.00 | 21.63 |
| atom | 1040 | CA  | ILE | 134 | 41.324 | 12.746 | -7.172  | 1.00 | 18.39 |

| atom | 1041 | CB | ILE | 134 | 41.639 | 13.507 | -5.916 | 1.00 | 16.31 |
|------|------|-----|-----|-----|--------|--------|--------|------|-------|
| atom | 1042 | CG2 | ILE | 134 | 43.124 | 13.434 | -5.668 | 1.00 | 19.82 |
| atom | 1043 | CG1 | ILE | 134 | 41.243 | 14.972 | -6.097 | 1.00 | 19.78 |
| atom | 1044 | CD1 | ILE | 134 | 41.493 | 15.852 | -4.891 | 1.00 | 25.49 |
| atom | 1045 | C | ILE | 134 | 41.535 | 11.287 | -6.945 | 1.00 | 18.04 |
| atom | 1046 | 0 | ILE | 134 | 40.664 | 10.588 | -6.434 | 1.00 | 20.16 |
| atom | 1047 | N | ASP | 135 | 42.700 | 10.814 | -7.361 | 1.00 | 20.29 |
| atom | 1048 | CA | ASP | 135 | 43.002 | 9.406 | -7.226 | 1.00 | 18.18 |
| atom | 1049 | CB | ASP | 135 | 44.367 | 9.106 | -7.848 | 1.00 | 14.84 |
| atom | 1050 | CG | ASP | 135 | 44.607 | 7.619 | -8.020 | 1.00 | 20.47 |
| atom | 1051 | OD1 | ASP | 135 | 45.680 | 7.130 | -7.608 | 1.00 | 22.89 |
| atom | 1052 | OD2 | ASP | 135 | 43.718 | 6.931 | -8.567 | 1.00 | 22.60 |
| atom | 1053 | C | ASP | 135 | 42.981 | 8.934 | -5.768 | 1.00 | 19.45 |
| atom | 1054 | 0 | ASP | 135 | 43.234 | 9.704 | -4.825 | 1.00 | 12.97 |
| atom | 1055 | N | THR | 136 | 42.650 | 7.663 | -5.604 | 1.00 | 17.34 |
| atom | 1056 | CA | THR | 136 | 42.660 | 7.030 | -4.304 | 1.00 | 18.09 |
| atom | 1057 | CB | THR | 136 | 41.245 | 6.860 | -3.722 | 1.00 | 15.30 |
| atom | 1058 | OG1 | THR | 136 | 40.495 | 5.971 | -4.551 | 1.00 | 8.12 |
| atom | 1059 | CG2 | THR | 136 | 40.532 | 8.200 | -3.614 | 1.00 | 14.26 |
| atom | 1060 | C | THR | 136 | 43.253 | 5.644 | -4.580 | 1.00 | 23.39 |
| atom | 1061 | 0 | THR | 136 | 43.281 | 5.183 | -5.731 | 1.00 | 21.41 |
| atom | 1062 | N | THR | 137 | 43.752 | 4.995 | -3.533 | 1.00 | 21.68 |
| atom | 1063 | CA | THR | 137 | 44.305 | 3.665 | -3.669 | 1.00 | 21.26 |
| atom | 1064 | CB | THR | 137 | 45.685 | 3.564 | -3.016 | 1.00 | 26.31 |
| atom | 1065 | OG1 | THR | 137 | 46.610 | 4.402 | -3.726 | 1.00 | 28.90 |
| atom | 1066 | CG2 | THR | 137 | 46.163 | 2.103 | -3.014 | 1.00 | 16.36 |
| atom | 1067 | C | THR | 137 | 43.340 | 2.775 | -2.922 | 1.00 | 24.37 |
| atom | 1068 | 0 | THR | 137 | 42.654 | 3.238 | -2.014 | 1.00 | 28.21 |
| atom | 1069 | N | ILE | 138 | 43.268 | 1.507 | -3.289 | 1.00 | 24.64 |
| atom | 1070 | CA | ILE | 138 | 42.360 | 0.601 | -2.603 | 1.00 | 25.62 |
| atom | 1071 | CB | ILE | 138 | 41.184 | 0.248 | -3.527 | 1.00 | 25.74 |
| atom | 1072 | CG2 | ILE | 138 | 41.709 | -0.457 | -4.785 | 1.00 | 30.99 |
| atom | 1073 | CG1 | ILE | 138 | 40.196 | -0.663 | -2.806 | 1.00 | 24.67 |
| atom | 1074 | CD1 | ILE | 138 | 39.051 | -1.123 | -3.679 | 1.00 | 17.63 |
| atom | 1075 | C | ILE | 138 | 43.112 | -0.675 | -2.211 | 1.00 | 28.00 |
| atom | 1076 | 0 | ILE | 138 | 43.899 | -1.196 | -2.997 | 1.00 | 28.97 |

| atom | 1077 | N | MET | 139 | 42.881 | -1.174 | -0.998 | 1.00 | 30.48 |
|------|------|------|------|------|--------|--------|--------|------|-------|
| atom | 1078 | CA | MET | 139 | 43.550 | -2.398 | -0.537 | 1.00 | 26.65 |
| atom | 1079 | CB | MET | 139 | 44.801 | -2.089 | 0.293 | 1.00 | 27.97 |
| atom | 1080 | CG | MET | 139 | 45.626 | -0.917 | -0.152 | 1.00 | 29.19 |
| atom | 1081 | SD | MET | 139 | 47.011 | -1.427 | -1.169 | 1.00 | 34.62 |
| atom | 1082 | CE | MET | 139 | 47.603 | -2.919 | -0.310 | 1.00 | 32.02 |
| atom | 1083 | C | MET | 139 | 42.668 | -3.273 | 0.328 | 1.00 | 26.27 |
| atom | 1084 | O | MET | 139 | 41.647 | -2.834 | 0.852 | 1.00 | 26.36 |
| atom | 1085 | N | ALA | 140 | 43.094 | -4.520 | 0.480 | 1.00 | 26.30 |
| atom | 1086 | CA | ALA | 140 | 42.408 | -5.484 | 1.319 | 1.00 | 29.53 |
| atom | 1087 | CB | ALA | 140 | 42.590 | -6.870 | 0.763 | 1.00 | 34.69 |
| atom | 1088 | C | ALA | 140 | 43.087 | -5.374 | 2.677 | 1.00 | 33.12 |
| atom | 1089 | O | ALA | 140 | 44.294 | -5.593 | 2.779 | 1.00 | 34.39 |
| atom | 1090 | N | LYS | 141 | 42.331 | -5.018 | 3.716 | 1.00 | 35.05 |
| atom | 1091 | CA | LYS | 141 | 42.923 | -4.883 | 5.045 | 1.00 | 32.82 |
| atom | 1092 | CB | LYS | 141 | 41.940 | -4.202 | 6.011 | 1.00 | 34.61 |
| atom | 1093 | CG | LYS | 141 | 42.343 | -2.775 | 6.410 | 1.00 | 36.26 |
| atom | 1094 | CD | LYS | 141 | 41.601 | -2.310 | 7.678 | 1.00 | 46.84 |
| atom | 1095 | CE | LYS | 141 | 41.236 | -0.805 | 7.655 | 1.00 | 47.24 |
| atom | 1096 | NZ | LYS | 141 | 42.184 | 0.072 | 8.439 | 1.00 | 40.93 |
| atom | 1097 | C | LYS | 141 | 43.345 | -6.242 | 5.589 | 1.00 | 29.67 |
| atom | 1098 | O | LYS | 141 | 42.719 | -7.260 | 5.303 | 1.00 | 29.80 |
| atom | 1099 | N | ASN | 142 | 44.425 | -6.256 | 6.360 | 1.00 | 31.23 |
| atom | 1100 | CA | ASN | 142 | 44.923 | -7.496 | 6.949 | 1.00 | 30.24 |
| atom | 1101 | CB | ASN | 142 | 46.396 | -7.706 | 6.591 | 1.00 | 33.52 |
| atom | 1102 | CG | ASN | 142 | 46.609 | -8.065 | 5.126 | 1.00 | 34.71 |
| atom | 1103 | OD1 | ASN | 142 | 47.347 | -8.991 | 4.817 | 1.00 | 38.81 |
| atom | 1104 | ND2 | ASN | 142 | 45.977 | -7.330 | 4.225 | 1.00 | 37.08 |
| atom | 1105 | C | ASN | 142 | 44.796 | -7.412 | 8.470 | 1.00 | 29.20 |
| atom | 1106 | O | ASN | 142 | 45.648 | -6.803 | 9.126 | 1.00 | 29.53 |
| atom | 1107 | N | GLU | 143 | 43.734 | -7.990 | 9.031 | 1.00 | 23.30 |
| atom | 1108 | CA | GLU | 143 | 43.556 | -7.979 | 10.483 | 1.00 | 21.14 |
| atom | 1109 | CB | GLU | 143 | 42.520 | -6.945 | 10.924 | 1.00 | 17.55 |
| atom | 1110 | CG | GLU | 143 | 41.199 | -7.006 | 10.249 | 1.00 | 17.18 |
| atom | 1111 | CD | GLU | 143 | 40.522 | -5.652 | 10.216 | 1.00 | 23.57 |
| atom | 1112 | OE1 | GLU | 143 | 41.225 | -4.620 | 10.318 | 1.00 | 27.69 |

| atom | 1113 | OE2 | GLU | 143 | 39.284 | -5.607 | 10.087 | 1.00 | 30.88 |
|------|------|-----|-----|-----|--------|--------|--------|------|-------|
| atom | 1114 | C   | GLU | 143 | 43.142 | -9.368 | 10.890 | 1.00 | 17.20 |
| atom | 1115 | O   | GLU | 143 | 42.574 | -10.093 | 10.097 | 1.00 | 18.93 |
| atom | 1116 | N   | VAL | 144 | 43.418 | -9.760 | 12.121 | 1.00 | 17.94 |
| atom | 1117 | CA  | VAL | 144 | 43.092 | -11.126 | 12.486 | 1.00 | 20.65 |
| atom | 1118 | CB  | VAL | 144 | 44.297 | -11.807 | 13.201 | 1.00 | 20.49 |
| atom | 1119 | CG1 | VAL | 144 | 45.518 | -10.910 | 13.115 | 1.00 | 16.50 |
| atom | 1120 | CG2 | VAL | 144 | 43.962 | -12.141 | 14.635 | 1.00 | 18.24 |
| atom | 1121 | C   | VAL | 144 | 41.812 | -11.342 | 13.263 | 1.00 | 20.00 |
| atom | 1122 | O   | VAL | 144 | 41.355 | -10.476 | 13.985 | 1.00 | 24.75 |
| atom | 1123 | N   | PHE | 145 | 41.250 | -12.528 | 13.078 | 1.00 | 19.07 |
| atom | 1124 | CA  | PHE | 145 | 40.002 | -12.946 | 13.694 | 1.00 | 16.80 |
| atom | 1125 | CB  | PHE | 145 | 38.850 | -12.777 | 12.687 | 1.00 | 12.26 |
| atom | 1126 | CG  | PHE | 145 | 38.573 | -11.358 | 12.318 | 1.00 | 15.43 |
| atom | 1127 | CD1 | PHE | 145 | 38.838 | -10.891 | 11.022 | 1.00 | 16.74 |
| atom | 1128 | CD2 | PHE | 145 | 38.076 | -10.470 | 13.261 | 1.00 | 12.81 |
| atom | 1129 | CE1 | PHE | 145 | 38.614 | -9.551 | 10.673 | 1.00 | 10.56 |
| atom | 1130 | CE2 | PHE | 145 | 37.848 | -9.117 | 12.924 | 1.00 | 14.36 |
| atom | 1131 | CZ  | PHE | 145 | 38.120 | -8.662 | 11.624 | 1.00 | 12.18 |
| atom | 1132 | C   | PHE | 145 | 40.091 | -14.421 | 14.082 | 1.00 | 16.98 |
| atom | 1133 | O   | PHE | 145 | 41.146 | -15.058 | 13.975 | 1.00 | 19.51 |
| atom | 1134 | N   | CYS | 146 | 38.960 | -14.959 | 14.513 | 1.00 | 15.55 |
| atom | 1135 | CA  | CYS | 146 | 38.861 | -16.353 | 14.886 | 1.00 | 19.61 |
| atom | 1136 | CB  | CYS | 146 | 38.352 | -16.479 | 16.325 | 1.00 | 20.46 |
| atom | 1137 | SG  | CYS | 146 | 38.349 | -18.152 | 16.978 | 1.00 | 29.33 |
| atom | 1138 | C   | CYS | 146 | 37.826 | -16.887 | 13.918 | 1.00 | 22.54 |
| atom | 1139 | O   | CYS | 146 | 36.852 | -16.197 | 13.631 | 1.00 | 24.05 |
| atom | 1140 | N   | VAL | 147 | 38.025 | -18.096 | 13.408 | 1.00 | 27.04 |
| atom | 1141 | CA  | VAL | 147 | 37.070 | -18.648 | 12.462 | 1.00 | 38.78 |
| atom | 1142 | CB  | VAL | 147 | 37.428 | -20.088 | 12.078 | 1.00 | 40.45 |
| atom | 1143 | CG1 | VAL | 147 | 38.929 | -20.190 | 11.844 | 1.00 | 37.73 |
| atom | 1144 | CG2 | VAL | 147 | 36.962 | -21.050 | 13.167 | 1.00 | 40.03 |
| atom | 1145 | C   | VAL | 147 | 35.668 | -18.609 | 13.046 | 1.00 | 42.25 |
| atom | 1146 | O   | VAL | 147 | 35.485 | -18.844 | 14.239 | 1.00 | 43.36 |
| atom | 1147 | N   | GLN | 148 | 34.695 | -18.293 | 12.192 | 1.00 | 52.45 |
| atom | 1148 | CA  | GLN | 148 | 33.294 | -18.181 | 12.585 | 1.00 | 58.94 |

| | | | | | | | | | |
|---|---|---|---|---|---|---|---|---|---|
| atom | 1149 | CB | GLN | 148 | 32.375 | -18.223 | 11.360 | 1.00 | 62.76 |
| atom | 1150 | CG | GLN | 148 | 31.141 | -17.310 | 11.473 | 1.00 | 69.47 |
| atom | 1151 | CD | GLN | 148 | 30.518 | -16.967 | 10.120 | 1.00 | 72.76 |
| atom | 1152 | OE1 | GLN | 148 | 30.620 | -15.834 | 9.644 | 1.00 | 72.30 |
| atom | 1153 | NE2 | GLN | 148 | 29.866 | -17.949 | 9.499 | 1.00 | 73.42 |
| atom | 1154 | C | GLN | 148 | 32.922 | -19.294 | 13.537 | 1.00 | 61.80 |
| atom | 1155 | O | GLN | 148 | 33.311 | -20.447 | 13.338 | 1.00 | 61.76 |
| atom | 1156 | N | PRO | 149 | 32.157 | -18.956 | 14.591 | 1.00 | 63.58 |
| atom | 1157 | CD | PRO | 149 | 31.641 | -17.602 | 14.865 | 1.00 | 63.75 |
| atom | 1158 | CA | PRO | 149 | 31.717 | -19.920 | 15.606 | 1.00 | 64.07 |
| atom | 1159 | CB | PRO | 149 | 30.373 | -19.359 | 16.072 | 1.00 | 64.66 |
| atom | 1160 | CG | PRO | 149 | 30.410 | -17.863 | 15.711 | 1.00 | 65.09 |
| atom | 1161 | C | PRO | 149 | 31.577 | -21.294 | 14.983 | 1.00 | 64.02 |
| atom | 1162 | O | PRO | 149 | 32.248 | -22.251 | 15.381 | 1.00 | 60.51 |
| atom | 1163 | N | GLU | 150 | 30.702 | -21.356 | 13.983 | 1.00 | 64.24 |
| atom | 1164 | CA | GLU | 150 | 30.440 | -22.576 | 13.243 | 1.00 | 65.65 |
| atom | 1165 | CB | GLU | 150 | 29.245 | -22.375 | 12.308 | 1.00 | 64.12 |
| atom | 1166 | CG | GLU | 150 | 28.904 | -20.909 | 12.036 | 1.00 | 65.02 |
| atom | 1167 | CD | GLU | 150 | 27.507 | -20.534 | 12.510 | 1.00 | 65.10 |
| atom | 1168 | OE1 | GLU | 150 | 26.913 | -21.330 | 13.273 | 1.00 | 61.66 |
| atom | 1169 | OE2 | GLU | 150 | 27.007 | -19.449 | 12.123 | 1.00 | 62.67 |
| atom | 1170 | C | GLU | 150 | 31.688 | -22.911 | 12.430 | 1.00 | 67.55 |
| atom | 1171 | O | GLU | 150 | 32.228 | -22.046 | 11.726 | 1.00 | 68.49 |
| atom | 1172 | N | LYS | 151 | 32.146 | -24.159 | 12.543 | 1.00 | 66.38 |
| atom | 1173 | CA | LYS | 151 | 33.328 | -24.620 | 11.818 | 1.00 | 63.34 |
| atom | 1174 | CB | LYS | 151 | 33.522 | -26.135 | 12.006 | 1.00 | 62.68 |
| atom | 1175 | CG | LYS | 151 | 34.936 | -26.532 | 12.446 | 1.00 | 61.87 |
| atom | 1176 | CD | LYS | 151 | 35.330 | -27.903 | 11.918 | 1.00 | 62.09 |
| atom | 1177 | CE | LYS | 151 | 36.406 | -27.813 | 10.847 | 1.00 | 59.59 |
| atom | 1178 | NZ | LYS | 151 | 36.715 | -29.162 | 10.279 | 1.00 | 57.99 |
| atom | 1179 | C | LYS | 151 | 33.213 | -24.282 | 10.330 | 1.00 | 60.40 |
| atom | 1180 | O | LYS | 151 | 32.533 | -24.966 | 9.559 | 1.00 | 59.04 |
| atom | 1181 | N | GLY | 152 | 33.889 | -23.208 | 9.943 | 1.00 | 57.56 |
| atom | 1182 | CA | GLY | 152 | 33.862 | -22.772 | 8.565 | 1.00 | 51.32 |
| atom | 1183 | C | GLY | 152 | 33.977 | -21.269 | 8.492 | 1.00 | 45.79 |
| atom | 1184 | O | GLY | 152 | 35.070 | -20.725 | 8.638 | 1.00 | 45.11 |

| | | | | | | | | | | |
|---|---|---|---|---|---|---|---|---|---|---|
| atom | 1185 | N | GLY | 153 | 32.841 | -20.611 | 8.280 | 1.00 | 42.64 |
| atom | 1186 | CA | GLY | 153 | 32.798 | -19.163 | 8.173 | 1.00 | 40.81 |
| atom | 1187 | C | GLY | 153 | 34.094 | -18.404 | 8.402 | 1.00 | 38.58 |
| atom | 1188 | O | GLY | 153 | 34.712 | -18.507 | 9.461 | 1.00 | 38.72 |
| atom | 1189 | N | ARG | 154 | 34.499 | -17.632 | 7.399 | 1.00 | 35.69 |
| atom | 1190 | CA | ARG | 154 | 35.709 | -16.820 | 7.472 | 1.00 | 33.89 |
| atom | 1191 | CB | ARG | 154 | 36.839 | -17.486 | 6.677 | 1.00 | 37.54 |
| atom | 1192 | CG | ARG | 154 | 38.090 | -17.857 | 7.489 | 1.00 | 34.13 |
| atom | 1193 | CD | ARG | 154 | 39.211 | -18.305 | 6.554 | 1.00 | 32.60 |
| atom | 1194 | NE | ARG | 154 | 40.342 | -18.930 | 7.236 | 1.00 | 28.80 |
| atom | 1195 | CZ | ARG | 154 | 40.262 | -20.030 | 7.974 | 1.00 | 28.75 |
| atom | 1196 | NH1 | ARG | 154 | 39.091 | -20.644 | 8.138 | 1.00 | 25.45 |
| atom | 1197 | NH2 | ARG | 154 | 41.365 | -20.523 | 8.532 | 1.00 | 22.79 |
| atom | 1198 | C | ARG | 154 | 35.349 | -15.472 | 6.853 | 1.00 | 32.85 |
| atom | 1199 | O | ARG | 154 | 34.777 | -15.417 | 5.765 | 1.00 | 36.37 |
| atom | 1200 | N | LYS | 155 | 35.666 | -14.386 | 7.539 | 1.00 | 27.50 |
| atom | 1201 | CA | LYS | 155 | 35.334 | -13.073 | 7.026 | 1.00 | 28.54 |
| atom | 1202 | CB | LYS | 155 | 35.253 | -12.071 | 8.178 | 1.00 | 31.26 |
| atom | 1203 | CG | LYS | 155 | 33.956 | -12.101 | 8.960 | 1.00 | 40.17 |
| atom | 1204 | CD | LYS | 155 | 34.057 | -12.968 | 10.230 | 1.00 | 43.63 |
| atom | 1205 | CE | LYS | 155 | 33.901 | -12.123 | 11.498 | 1.00 | 43.66 |
| atom | 1206 | NZ | LYS | 155 | 34.164 | -10.671 | 11.242 | 1.00 | 44.91 |
| atom | 1207 | C | LYS | 155 | 36.328 | -12.560 | 5.984 | 1.00 | 32.85 |
| atom | 1208 | O | LYS | 155 | 37.554 | -12.615 | 6.177 | 1.00 | 33.24 |
| atom | 1209 | N | PRO | 156 | 35.816 | -12.093 | 4.839 | 1.00 | 29.41 |
| atom | 1210 | CD | PRO | 156 | 34.400 | -12.109 | 4.434 | 1.00 | 31.41 |
| atom | 1211 | CA | PRO | 156 | 36.694 | -11.569 | 3.794 | 1.00 | 26.76 |
| atom | 1212 | CB | PRO | 156 | 35.775 | -11.421 | 2.582 | 1.00 | 32.64 |
| atom | 1213 | CG | PRO | 156 | 34.395 | -11.332 | 3.151 | 1.00 | 33.94 |
| atom | 1214 | C | PRO | 156 | 37.264 | -10.228 | 4.222 | 1.00 | 21.47 |
| atom | 1215 | O | PRO | 156 | 36.611 | -9.493 | 4.941 | 1.00 | 15.73 |
| atom | 1216 | N | ALA | 157 | 38.465 | -9.908 | 3.752 | 1.00 | 19.11 |
| atom | 1217 | CA | ALA | 157 | 39.114 | -8.658 | 4.095 | 1.00 | 23.05 |
| atom | 1218 | CB | ALA | 157 | 40.338 | -8.452 | 3.232 | 1.00 | 20.61 |
| atom | 1219 | C | ALA | 157 | 38.213 | -7.442 | 3.988 | 1.00 | 27.93 |
| atom | 1220 | O | ALA | 157 | 37.155 | -7.481 | 3.380 | 1.00 | 31.73 |

| atom | 1221 | N   | ARG | 158 | 38.661 | -6.359 | 4.608  | 1.00 | 31.17 |
|------|------|-----|-----|-----|--------|--------|--------|------|-------|
| atom | 1222 | CA  | ARG | 158 | 37.966 | -5.090 | 4.596  | 1.00 | 30.61 |
| atom | 1223 | CB  | ARG | 158 | 38.108 | -4.402 | 5.953  | 1.00 | 39.25 |
| atom | 1224 | CG  | ARG | 158 | 36.921 | -4.522 | 6.895  | 1.00 | 46.67 |
| atom | 1225 | CD  | ARG | 158 | 36.243 | -3.165 | 7.074  | 1.00 | 56.19 |
| atom | 1226 | NE  | ARG | 158 | 36.734 | -2.328 | 8.184  | 1.00 | 62.33 |
| atom | 1227 | CZ  | ARG | 158 | 37.968 | -2.314 | 8.699  | 1.00 | 65.03 |
| atom | 1228 | NH1 | ARG | 158 | 38.921 | -3.111 | 8.238  | 1.00 | 62.19 |
| atom | 1229 | NH2 | ARG | 158 | 38.252 | -1.462 | 9.683  | 1.00 | 66.14 |
| atom | 1230 | C   | ARG | 158 | 38.729 | -4.291 | 3.555  | 1.00 | 30.37 |
| atom | 1231 | O   | ARG | 158 | 39.953 | -4.405 | 3.468  | 1.00 | 30.08 |
| atom | 1232 | N   | LEU | 159 | 38.033 | -3.478 | 2.771  | 1.00 | 28.48 |
| atom | 1233 | CA  | LEU | 159 | 38.720 | -2.704 | 1.758  | 1.00 | 26.77 |
| atom | 1234 | CB  | LEU | 159 | 37.950 | -2.748 | 0.440  | 1.00 | 25.42 |
| atom | 1235 | CG  | LEU | 159 | 37.459 | -4.091 | -0.085 | 1.00 | 28.81 |
| atom | 1236 | CD1 | LEU | 159 | 37.558 | -4.057 | -1.589 | 1.00 | 26.63 |
| atom | 1237 | CD2 | LEU | 159 | 38.260 | -5.249 | 0.496  | 1.00 | 25.80 |
| atom | 1238 | C   | LEU | 159 | 38.895 | -1.259 | 2.160  | 1.00 | 25.74 |
| atom | 1239 | O   | LEU | 159 | 37.904 | -0.526 | 2.245  | 1.00 | 28.64 |
| atom | 1240 | N   | ILE | 160 | 40.132 | -0.839 | 2.419  | 1.00 | 22.24 |
| atom | 1241 | CA  | ILE | 160 | 40.351 | 0.559  | 2.759  | 1.00 | 19.95 |
| atom | 1242 | CB  | ILE | 160 | 41.538 | 0.755  | 3.759  | 1.00 | 24.51 |
| atom | 1243 | CG2 | ILE | 160 | 42.848 | 0.394  | 3.120  | 1.00 | 24.22 |
| atom | 1244 | CG1 | ILE | 160 | 41.594 | 2.222  | 4.228  | 1.00 | 33.48 |
| atom | 1245 | CD1 | ILE | 160 | 41.084 | 2.491  | 5.672  | 1.00 | 30.14 |
| atom | 1246 | C   | ILE | 160 | 40.566 | 1.354  | 1.465  | 1.00 | 20.39 |
| atom | 1247 | O   | ILE | 160 | 40.938 | 0.813  | 0.417  | 1.00 | 23.89 |
| atom | 1248 | N   | VAL | 161 | 40.270 | 2.640  | 1.530  | 1.00 | 20.34 |
| atom | 1249 | CA  | VAL | 161 | 40.392 | 3.513  | 0.384  | 1.00 | 13.51 |
| atom | 1250 | CB  | VAL | 161 | 39.002 | 3.776  | -0.226 | 1.00 | 17.37 |
| atom | 1251 | CG1 | VAL | 161 | 39.122 | 4.782  | -1.383 | 1.00 | 15.61 |
| atom | 1252 | CG2 | VAL | 161 | 38.377 | 2.453  | -0.686 | 1.00 | 11.77 |
| atom | 1253 | C   | VAL | 161 | 40.958 | 4.821  | 0.903  | 1.00 | 16.32 |
| atom | 1254 | O   | VAL | 161 | 40.330 | 5.469  | 1.725  | 1.00 | 13.98 |
| atom | 1255 | N   | PHE | 162 | 42.135 | 5.215  | 0.432  | 1.00 | 18.09 |
| atom | 1256 | CA  | PHE | 162 | 42.738 | 6.456  | 0.913  | 1.00 | 19.73 |

| atom | 1257 | CB | PHE | 162 | 43.746 | 6.135 | 2.000 | 1.00 | 19.06 |
|------|------|-----|-----|-----|--------|-------|-------|------|-------|
| atom | 1258 | CG | PHE | 162 | 44.793 | 5.155 | 1.569 | 1.00 | 23.11 |
| atom | 1259 | CD1 | PHE | 162 | 46.062 | 5.595 | 1.188 | 1.00 | 24.58 |
| atom | 1260 | CD2 | PHE | 162 | 44.524 | 3.792 | 1.557 | 1.00 | 19.16 |
| atom | 1261 | CE1 | PHE | 162 | 47.036 | 4.691 | 0.813 | 1.00 | 19.74 |
| atom | 1262 | CE2 | PHE | 162 | 45.498 | 2.878 | 1.182 | 1.00 | 18.01 |
| atom | 1263 | CZ | PHE | 162 | 46.759 | 3.330 | 0.810 | 1.00 | 18.62 |
| atom | 1264 | C | PHE | 162 | 43.450 | 7.247 | -0.177 | 1.00 | 18.75 |
| atom | 1265 | O | PHE | 162 | 44.021 | 6.665 | -1.092 | 1.00 | 13.27 |
| atom | 1266 | N | PRO | 163 | 43.438 | 8.591 | -0.072 | 1.00 | 19.79 |
| atom | 1267 | CD | PRO | 163 | 42.825 | 9.385 | 1.011 | 1.00 | 20.08 |
| atom | 1268 | CA | PRO | 163 | 44.099 | 9.451 | -1.062 | 1.00 | 18.01 |
| atom | 1269 | CB | PRO | 163 | 43.549 | 10.845 | -0.754 | 1.00 | 14.92 |
| atom | 1270 | CG | PRO | 163 | 43.317 | 10.820 | 0.718 | 1.00 | 13.92 |
| atom | 1271 | C | PRO | 163 | 45.617 | 9.368 | -0.851 | 1.00 | 22.20 |
| atom | 1272 | O | PRO | 163 | 46.119 | 8.424 | -0.228 | 1.00 | 24.81 |
| atom | 1273 | N | ASP | 164 | 46.344 | 10.352 | -1.364 | 1.00 | 22.65 |
| atom | 1274 | CA | ASP | 164 | 47.798 | 10.363 | -1.231 | 1.00 | 22.44 |
| atom | 1275 | CB | ASP | 164 | 48.437 | 10.919 | -2.528 | 1.00 | 21.02 |
| atom | 1276 | CG | ASP | 164 | 49.979 | 10.885 | -2.515 | 1.00 | 25.45 |
| atom | 1277 | OD1 | ASP | 164 | 50.596 | 11.960 | -2.340 | 1.00 | 26.87 |
| atom | 1278 | OD2 | ASP | 164 | 50.578 | 9.796 | -2.698 | 1.00 | 23.16 |
| atom | 1279 | C | ASP | 164 | 48.189 | 11.214 | -0.020 | 1.00 | 21.82 |
| atom | 1280 | O | ASP | 164 | 47.461 | 12.134 | 0.369 | 1.00 | 11.22 |
| atom | 1281 | N | LEU | 165 | 49.339 | 10.878 | 0.561 | 1.00 | 23.17 |
| atom | 1282 | CA | LEU | 165 | 49.901 | 11.567 | 1.714 | 1.00 | 24.29 |
| atom | 1283 | CB | LEU | 165 | 51.404 | 11.245 | 1.797 | 1.00 | 27.89 |
| atom | 1284 | CG | LEU | 165 | 52.245 | 11.796 | 2.960 | 1.00 | 27.55 |
| atom | 1285 | CD1 | LEU | 165 | 51.470 | 11.627 | 4.263 | 1.00 | 29.58 |
| atom | 1286 | CD2 | LEU | 165 | 53.579 | 11.080 | 3.035 | 1.00 | 23.53 |
| atom | 1287 | C | LEU | 165 | 49.700 | 13.085 | 1.641 | 1.00 | 27.92 |
| atom | 1288 | O | LEU | 165 | 49.280 | 13.739 | 2.618 | 1.00 | 26.93 |
| atom | 1289 | N | GLY | 166 | 50.015 | 13.650 | 0.484 | 1.00 | 25.18 |
| atom | 1290 | CA | GLY | 166 | 49.863 | 15.083 | 0.324 | 1.00 | 24.97 |
| atom | 1291 | C | GLY | 166 | 48.428 | 15.546 | 0.471 | 1.00 | 22.31 |
| atom | 1292 | O | GLY | 166 | 48.161 | 16.659 | 0.938 | 1.00 | 23.62 |

| atom | 1293 | N   | VAL | 167 | 47.495 | 14.710 | 0.043  | 1.00 | 18.72 |
|------|------|-----|-----|-----|--------|--------|--------|------|-------|
| atom | 1294 | CA  | VAL | 167 | 46.099 | 15.076 | 0.183  | 1.00 | 22.92 |
| atom | 1295 | CB  | VAL | 167 | 45.155 | 14.096 | -0.579 | 1.00 | 23.81 |
| atom | 1296 | CG1 | VAL | 167 | 43.712 | 14.344 | -0.172 | 1.00 | 17.63 |
| atom | 1297 | CG2 | VAL | 167 | 45.303 | 14.306 | -2.082 | 1.00 | 17.07 |
| atom | 1298 | C   | VAL | 167 | 45.834 | 15.030 | 1.679  | 1.00 | 20.19 |
| atom | 1299 | O   | VAL | 167 | 45.316 | 15.988 | 2.251  | 1.00 | 25.41 |
| atom | 1300 | N   | ARG | 168 | 46.226 | 13.926 | 2.306  | 1.00 | 17.47 |
| atom | 1301 | CA  | ARG | 168 | 46.071 | 13.747 | 3.748  | 1.00 | 20.66 |
| atom | 1302 | CB  | ARG | 168 | 46.768 | 12.442 | 4.184  | 1.00 | 12.74 |
| atom | 1303 | CG  | ARG | 168 | 45.786 | 11.303 | 4.520  | 1.00 | 14.91 |
| atom | 1304 | CD  | ARG | 168 | 46.288 | 9.924  | 4.153  | 1.00 | 5.34  |
| atom | 1305 | NE  | ARG | 168 | 47.737 | 9.836  | 4.279  | 1.00 | 20.08 |
| atom | 1306 | CZ  | ARG | 168 | 48.510 | 8.981  | 3.616  | 1.00 | 19.67 |
| atom | 1307 | NH1 | ARG | 168 | 49.826 | 8.993  | 3.812  | 1.00 | 21.17 |
| atom | 1308 | NH2 | ARG | 168 | 47.974 | 8.112  | 2.771  | 1.00 | 12.86 |
| atom | 1309 | C   | ARG | 168 | 46.599 | 14.957 | 4.564  | 1.00 | 21.32 |
| atom | 1310 | O   | ARG | 168 | 45.901 | 15.482 | 5.429  | 1.00 | 25.92 |
| atom | 1311 | N   | VAL | 169 | 47.814 | 15.417 | 4.297  | 1.00 | 20.48 |
| atom | 1312 | CA  | VAL | 169 | 48.307 | 16.562 | 5.048  | 1.00 | 20.77 |
| atom | 1313 | CB  | VAL | 169 | 49.737 | 17.006 | 4.587  | 1.00 | 17.98 |
| atom | 1314 | CG1 | VAL | 169 | 50.085 | 18.381 | 5.158  | 1.00 | 16.60 |
| atom | 1315 | CG2 | VAL | 169 | 50.753 | 15.998 | 5.043  | 1.00 | 18.11 |
| atom | 1316 | C   | VAL | 169 | 47.335 | 17.708 | 4.813  | 1.00 | 21.18 |
| atom | 1317 | O   | VAL | 169 | 47.006 | 18.459 | 5.732  | 1.00 | 24.32 |
| atom | 1318 | N   | CYS | 170 | 46.865 | 17.843 | 3.578  | 1.00 | 25.10 |
| atom | 1319 | CA  | CYS | 170 | 45.930 | 18.919 | 3.249  | 1.00 | 24.67 |
| atom | 1320 | CB  | CYS | 170 | 45.744 | 19.002 | 1.730  | 1.00 | 27.71 |
| atom | 1321 | SG  | CYS | 170 | 47.076 | 19.909 | 0.869  | 1.00 | 27.54 |
| atom | 1322 | C   | CYS | 170 | 44.584 | 18.731 | 3.955  | 1.00 | 22.25 |
| atom | 1323 | O   | CYS | 170 | 43.966 | 19.705 | 4.416  | 1.00 | 21.66 |
| atom | 1324 | N   | GLU | 171 | 44.137 | 17.481 | 4.057  | 1.00 | 18.43 |
| atom | 1325 | CA  | GLU | 171 | 42.875 | 17.216 | 4.736  | 1.00 | 18.04 |
| atom | 1326 | CB  | GLU | 171 | 42.587 | 15.699 | 4.835  | 1.00 | 8.34  |
| atom | 1327 | CG  | GLU | 171 | 42.052 | 15.108 | 3.509  | 1.00 | 2.27  |
| atom | 1328 | CD  | GLU | 171 | 41.409 | 13.717 | 3.626  | 1.00 | 8.05  |

| atom | 1329 | OE1 | GLU | 171 | 42.129 | 12.702 | 3.652 | 1.00 | 5.15 |
|---|---|---|---|---|---|---|---|---|---|
| atom | 1330 | OE2 | GLU | 171 | 40.166 | 13.633 | 3.672 | 1.00 | 9.40 |
| atom | 1331 | C | GLU | 171 | 43.049 | 17.836 | 6.106 | 1.00 | 19.14 |
| atom | 1332 | O | GLU | 171 | 42.212 | 18.637 | 6.532 | 1.00 | 14.20 |
| atom | 1333 | N | LYS | 172 | 44.170 | 17.514 | 6.764 | 1.00 | 18.46 |
| atom | 1334 | CA | LYS | 172 | 44.446 | 18.034 | 8.107 | 1.00 | 16.81 |
| atom | 1335 | CB | LYS | 172 | 45.820 | 17.586 | 8.604 | 1.00 | 18.96 |
| atom | 1336 | CG | LYS | 172 | 45.843 | 16.234 | 9.300 | 1.00 | 17.24 |
| atom | 1337 | CD | LYS | 172 | 46.653 | 15.237 | 8.517 | 1.00 | 17.70 |
| atom | 1338 | CE | LYS | 172 | 47.112 | 14.111 | 9.391 | 1.00 | 20.07 |
| atom | 1339 | NZ | LYS | 172 | 46.802 | 12.772 | 8.798 | 1.00 | 21.38 |
| atom | 1340 | C | LYS | 172 | 44.391 | 19.550 | 8.146 | 1.00 | 17.84 |
| atom | 1341 | O | LYS | 172 | 43.719 | 20.131 | 8.989 | 1.00 | 19.49 |
| atom | 1342 | N | MET | 173 | 45.094 | 20.200 | 7.236 | 1.00 | 18.73 |
| atom | 1343 | CA | MET | 173 | 45.092 | 21.649 | 7.252 | 1.00 | 25.13 |
| atom | 1344 | CB | MET | 173 | 45.825 | 22.200 | 6.025 | 1.00 | 24.15 |
| atom | 1345 | CG | MET | 173 | 47.353 | 22.055 | 6.141 | 1.00 | 32.87 |
| atom | 1346 | SD | MET | 173 | 48.276 | 22.321 | 4.610 | 1.00 | 32.61 |
| atom | 1347 | CE | MET | 173 | 48.568 | 24.104 | 4.677 | 1.00 | 29.59 |
| atom | 1348 | C | MET | 173 | 43.682 | 22.218 | 7.330 | 1.00 | 26.29 |
| atom | 1349 | O | MET | 173 | 43.358 | 22.984 | 8.239 | 1.00 | 25.83 |
| atom | 1350 | N | ALA | 174 | 42.826 | 21.814 | 6.404 | 1.00 | 25.93 |
| atom | 1351 | CA | ALA | 174 | 41.475 | 22.348 | 6.378 | 1.00 | 22.87 |
| atom | 1352 | CB | ALA | 174 | 41.007 | 22.419 | 4.946 | 1.00 | 28.71 |
| atom | 1353 | C | ALA | 174 | 40.400 | 21.648 | 7.208 | 1.00 | 23.81 |
| atom | 1354 | O | ALA | 174 | 39.341 | 22.212 | 7.426 | 1.00 | 28.91 |
| atom | 1355 | N | LEU | 175 | 40.637 | 20.447 | 7.699 | 1.00 | 20.65 |
| atom | 1356 | CA | LEU | 175 | 39.548 | 19.799 | 8.398 | 1.00 | 20.65 |
| atom | 1357 | CB | LEU | 175 | 39.014 | 18.672 | 7.509 | 1.00 | 16.61 |
| atom | 1358 | CG | LEU | 175 | 38.022 | 19.194 | 6.474 | 1.00 | 18.52 |
| atom | 1359 | CD1 | LEU | 175 | 37.617 | 18.080 | 5.514 | 1.00 | 25.09 |
| atom | 1360 | CD2 | LEU | 175 | 36.826 | 19.748 | 7.185 | 1.00 | 12.35 |
| atom | 1361 | C | LEU | 175 | 39.764 | 19.279 | 9.809 | 1.00 | 21.36 |
| atom | 1362 | O | LEU | 175 | 38.785 | 19.044 | 10.536 | 1.00 | 21.56 |
| atom | 1363 | N | TYR | 176 | 41.024 | 19.106 | 10.199 | 1.00 | 21.79 |
| atom | 1364 | CA | TYR | 176 | 41.330 | 18.590 | 11.521 | 1.00 | 24.37 |

| atom | 1365 | CB | TYR | 176 | 42.823 | 18.702 | 11.801 | 1.00 | 27.38 |
|------|------|-----|-----|-----|--------|--------|--------|------|-------|
| atom | 1366 | CG | TYR | 176 | 43.200 | 18.138 | 13.149 | 1.00 | 26.58 |
| atom | 1367 | CD1 | TYR | 176 | 43.467 | 16.780 | 13.304 | 1.00 | 18.36 |
| atom | 1368 | CE1 | TYR | 176 | 43.778 | 16.245 | 14.541 | 1.00 | 15.60 |
| atom | 1369 | CD2 | TYR | 176 | 43.255 | 18.959 | 14.276 | 1.00 | 22.63 |
| atom | 1370 | CE2 | TYR | 176 | 43.563 | 18.438 | 15.520 | 1.00 | 22.17 |
| atom | 1371 | CZ | TYR | 176 | 43.825 | 17.081 | 15.652 | 1.00 | 21.81 |
| atom | 1372 | OH | TYR | 176 | 44.135 | 16.572 | 16.897 | 1.00 | 24.09 |
| atom | 1373 | C | TYR | 176 | 40.544 | 19.281 | 12.639 | 1.00 | 23.42 |
| atom | 1374 | O | TYR | 176 | 39.834 | 18.617 | 13.401 | 1.00 | 24.86 |
| atom | 1375 | N | ASP | 177 | 40.667 | 20.602 | 12.725 | 1.00 | 19.86 |
| atom | 1376 | CA | ASP | 177 | 39.973 | 21.381 | 13.751 | 1.00 | 24.18 |
| atom | 1377 | CB | ASP | 177 | 40.235 | 22.895 | 13.584 | 1.00 | 24.04 |
| atom | 1378 | CG | ASP | 177 | 39.836 | 23.720 | 14.830 | 1.00 | 28.43 |
| atom | 1379 | OD1 | ASP | 177 | 39.646 | 24.954 | 14.698 | 1.00 | 25.72 |
| atom | 1380 | OD2 | ASP | 177 | 39.723 | 23.142 | 15.940 | 1.00 | 30.12 |
| atom | 1381 | C | ASP | 177 | 38.480 | 21.121 | 13.689 | 1.00 | 23.39 |
| atom | 1382 | O | ASP | 177 | 37.858 | 20.791 | 14.701 | 1.00 | 18.65 |
| atom | 1383 | N | VAL | 178 | 37.925 | 21.278 | 12.490 | 1.00 | 26.00 |
| atom | 1384 | CA | VAL | 178 | 36.503 | 21.068 | 12.241 | 1.00 | 22.90 |
| atom | 1385 | CB | VAL | 178 | 36.187 | 21.206 | 10.734 | 1.00 | 24.09 |
| atom | 1386 | CG1 | VAL | 178 | 34.742 | 20.761 | 10.447 | 1.00 | 21.97 |
| atom | 1387 | CG2 | VAL | 178 | 36.420 | 22.642 | 10.287 | 1.00 | 18.09 |
| atom | 1388 | C | VAL | 178 | 36.090 | 19.673 | 12.666 | 1.00 | 22.07 |
| atom | 1389 | O | VAL | 178 | 35.087 | 19.485 | 13.325 | 1.00 | 22.18 |
| atom | 1390 | N | VAL | 179 | 36.896 | 18.699 | 12.278 | 1.00 | 21.01 |
| atom | 1391 | CA | VAL | 179 | 36.624 | 17.296 | 12.538 | 1.00 | 19.30 |
| atom | 1392 | CB | VAL | 179 | 37.459 | 16.452 | 11.506 | 1.00 | 17.27 |
| atom | 1393 | CG1 | VAL | 179 | 37.942 | 15.161 | 12.075 | 1.00 | 15.59 |
| atom | 1394 | CG2 | VAL | 179 | 36.626 | 16.198 | 10.280 | 1.00 | 5.32 |
| atom | 1395 | C | VAL | 179 | 36.830 | 16.836 | 13.993 | 1.00 | 23.09 |
| atom | 1396 | O | VAL | 179 | 36.416 | 15.729 | 14.372 | 1.00 | 24.82 |
| atom | 1397 | N | SER | 180 | 37.438 | 17.676 | 14.821 | 1.00 | 20.55 |
| atom | 1398 | CA | SER | 180 | 37.638 | 17.306 | 16.217 | 1.00 | 19.50 |
| atom | 1399 | CB | SER | 180 | 39.123 | 17.268 | 16.562 | 1.00 | 17.45 |
| atom | 1400 | OG | SER | 180 | 39.768 | 18.460 | 16.176 | 1.00 | 18.00 |

| atom | 1401 | C | SER | 180 | 36.939 | 18.278 | 17.147 | 1.00 | 22.13 |
|------|------|------|-----|-----|--------|--------|--------|------|-------|
| atom | 1402 | O | SER | 180 | 37.040 | 18.166 | 18.366 | 1.00 | 23.67 |
| atom | 1403 | N | THR | 181 | 36.211 | 19.225 | 16.570 | 1.00 | 22.08 |
| atom | 1404 | CA | THR | 181 | 35.522 | 20.224 | 17.361 | 1.00 | 24.72 |
| atom | 1405 | CB | THR | 181 | 36.063 | 21.648 | 17.058 | 1.00 | 29.04 |
| atom | 1406 | OG1 | THR | 181 | 37.417 | 21.764 | 17.521 | 1.00 | 31.21 |
| atom | 1407 | CG2 | THR | 181 | 35.222 | 22.697 | 17.754 | 1.00 | 34.02 |
| atom | 1408 | C | THR | 181 | 34.024 | 20.230 | 17.141 | 1.00 | 23.96 |
| atom | 1409 | O | THR | 181 | 33.257 | 20.222 | 18.101 | 1.00 | 25.10 |
| atom | 1410 | N | LEU | 182 | 33.614 | 20.211 | 15.878 | 1.00 | 22.24 |
| atom | 1411 | CA | LEU | 182 | 32.194 | 20.278 | 15.517 | 1.00 | 20.03 |
| atom | 1412 | CB | LEU | 182 | 32.080 | 20.533 | 14.006 | 1.00 | 16.49 |
| atom | 1413 | CG | LEU | 182 | 30.764 | 20.247 | 13.288 | 1.00 | 13.61 |
| atom | 1414 | CD1 | LEU | 182 | 30.473 | 21.285 | 12.231 | 1.00 | 10.62 |
| atom | 1415 | CD2 | LEU | 182 | 30.871 | 18.883 | 12.672 | 1.00 | 18.21 |
| atom | 1416 | C | LEU | 182 | 31.239 | 19.156 | 15.932 | 1.00 | 18.70 |
| atom | 1417 | O | LEU | 182 | 30.076 | 19.411 | 16.246 | 1.00 | 17.38 |
| atom | 1418 | N | PRO | 183 | 31.705 | 17.902 | 15.949 | 1.00 | 24.20 |
| atom | 1419 | CD | PRO | 183 | 33.041 | 17.389 | 15.603 | 1.00 | 21.52 |
| atom | 1420 | CA | PRO | 183 | 30.778 | 16.824 | 16.345 | 1.00 | 25.01 |
| atom | 1421 | CB | PRO | 183 | 31.625 | 15.555 | 16.259 | 1.00 | 24.26 |
| atom | 1422 | CG | PRO | 183 | 32.766 | 15.923 | 15.337 | 1.00 | 27.42 |
| atom | 1423 | C | PRO | 183 | 30.150 | 16.988 | 17.727 | 1.00 | 27.49 |
| atom | 1424 | O | PRO | 183 | 28.938 | 16.860 | 17.890 | 1.00 | 25.95 |
| atom | 1425 | N | GLN | 184 | 30.975 | 17.274 | 18.722 | 1.00 | 28.58 |
| atom | 1426 | CA | GLN | 184 | 30.464 | 17.428 | 20.075 | 1.00 | 29.48 |
| atom | 1427 | CB | GLN | 184 | 31.605 | 17.736 | 21.046 | 1.00 | 32.11 |
| atom | 1428 | CG | GLN | 184 | 31.533 | 16.935 | 22.326 | 1.00 | 43.59 |
| atom | 1429 | CD | GLN | 184 | 32.132 | 17.669 | 23.513 | 1.00 | 52.97 |
| atom | 1430 | OE1 | GLN | 184 | 33.004 | 17.143 | 24.207 | 1.00 | 54.15 |
| atom | 1431 | NE2 | GLN | 184 | 31.668 | 18.893 | 23.750 | 1.00 | 56.44 |
| atom | 1432 | C | GLN | 184 | 29.413 | 18.517 | 20.162 | 1.00 | 26.07 |
| atom | 1433 | O | GLN | 184 | 28.370 | 18.341 | 20.794 | 1.00 | 26.07 |
| atom | 1434 | N | VAL | 185 | 29.682 | 19.643 | 19.517 | 1.00 | 22.55 |
| atom | 1435 | CA | VAL | 185 | 28.755 | 20.756 | 19.561 | 1.00 | 17.75 |
| atom | 1436 | CB | VAL | 185 | 29.354 | 22.045 | 18.958 | 1.00 | 14.40 |

| atom | 1437 | CG1 | VAL | 185 | 28.388 | 23.213 | 19.202 | 1.00 | 9.26 |
|------|------|-----|-----|-----|--------|--------|--------|------|-------|
| atom | 1438 | CG2 | VAL | 185 | 30.709 | 22.336 | 19.568 | 1.00 | 2.00 |
| atom | 1439 | C | VAL | 185 | 27.466 | 20.447 | 18.828 | 1.00 | 19.34 |
| atom | 1440 | 0 | VAL | 185 | 26.400 | 20.866 | 19.260 | 1.00 | 25.62 |
| atom | 1441 | N | VAL | 186 | 27.548 | 19.715 | 17.730 | 1.00 | 15.24 |
| atom | 1442 | CA | VAL | 186 | 26.333 | 19.398 | 16.993 | 1.00 | 14.51 |
| atom | 1443 | CB | VAL | 186 | 26.635 | 18.907 | 15.525 | 1.00 | 16.42 |
| atom | 1444 | CG1 | VAL | 186 | 25.339 | 18.383 | 14.877 | 1.00 | 4.47 |
| atom | 1445 | CG2 | VAL | 186 | 27.261 | 20.044 | 14.680 | 1.00 | 8.35 |
| atom | 1446 | C | VAL | 186 | 25.513 | 18.309 | 17.680 | 1.00 | 15.94 |
| atom | 1447 | 0 | VAL | 186 | 24.304 | 18.431 | 17.851 | 1.00 | 17.63 |
| atom | 1448 | N | MET | 187 | 26.176 | 17.238 | 18.087 | 1.00 | 18.70 |
| atom | 1449 | CA | MET | 187 | 25.467 | 16.117 | 18.674 | 1.00 | 17.25 |
| atom | 1450 | CB | MET | 187 | 26.079 | 14.814 | 18.119 | 1.00 | 18.33 |
| atom | 1451 | CG | MET | 187 | 25.890 | 14.700 | 16.581 | 1.00 | 13.46 |
| atom | 1452 | SD | MET | 187 | 26.973 | 13.526 | 15.676 | 1.00 | 20.52 |
| atom | 1453 | CE | MET | 187 | 26.373 | 12.004 | 16.291 | 1.00 | 20.96 |
| atom | 1454 | C | MET | 187 | 25.333 | 16.074 | 20.191 | 1.00 | 17.46 |
| atom | 1455 | 0 | MET | 187 | 24.592 | 15.251 | 20.718 | 1.00 | 20.33 |
| atom | 1456 | N | GLY | 188 | 26.029 | 16.956 | 20.895 | 1.00 | 17.70 |
| atom | 1457 | CA | GLY | 188 | 25.928 | 16.966 | 22.347 | 1.00 | 18.64 |
| atom | 1458 | C | GLY | 188 | 26.339 | 15.677 | 23.049 | 1.00 | 19.86 |
| atom | 1459 | 0 | GLY | 188 | 27.277 | 14.994 | 22.613 | 1.00 | 18.30 |
| atom | 1460 | N | SER | 189 | 25.624 | 15.327 | 24.118 | 1.00 | 13.87 |
| atom | 1461 | CA | SER | 189 | 25.952 | 14.128 | 24.893 | 1.00 | 17.42 |
| atom | 1462 | CB | SER | 189 | 25.061 | 13.992 | 26.145 | 1.00 | 12.03 |
| atom | 1463 | OG | SER | 189 | 23.696 | 14.207 | 25.843 | 1.00 | 18.25 |
| atom | 1464 | C | SER | 189 | 25.862 | 12.863 | 24.089 | 1.00 | 16.25 |
| atom | 1465 | 0 | SER | 189 | 26.330 | 11.825 | 24.524 | 1.00 | 15.99 |
| atom | 1466 | N | SER | 190 | 25.252 | 12.957 | 22.911 | 1.00 | 17.69 |
| atom | 1467 | CA | SER | 190 | 25.117 | 11.812 | 22.026 | 1.00 | 13.15 |
| atom | 1468 | CB | SER | 190 | 24.036 | 12.104 | 20.984 | 1.00 | 14.83 |
| atom | 1469 | OG | SER | 190 | 22.736 | 11.995 | 21.536 | 1.00 | 17.43 |
| atom | 1470 | C | SER | 190 | 26.445 | 11.462 | 21.310 | 1.00 | 15.44 |
| atom | 1471 | 0 | SER | 190 | 26.555 | 10.387 | 20.703 | 1.00 | 19.87 |
| atom | 1472 | N | TYR | 191 | 27.441 | 12.349 | 21.365 | 1.00 | 7.89 |

| atom | 1473 | CA  | TYR | 191 | 28.720 | 12.080 | 20.699 | 1.00 | 11.45 |
|------|------|-----|-----|-----|--------|--------|--------|------|-------|
| atom | 1474 | CB  | TYR | 191 | 29.500 | 13.371 | 20.479 | 1.00 | 4.60  |
| atom | 1475 | CG  | TYR | 191 | 30.682 | 13.211 | 19.570 | 1.00 | 8.04  |
| atom | 1476 | CD1 | TYR | 191 | 30.591 | 12.470 | 18.392 | 1.00 | 17.37 |
| atom | 1477 | CE1 | TYR | 191 | 31.698 | 12.339 | 17.525 | 1.00 | 15.08 |
| atom | 1478 | CD2 | TYR | 191 | 31.905 | 13.815 | 19.868 | 1.00 | 11.56 |
| atom | 1479 | CE2 | TYR | 191 | 33.006 | 13.693 | 19.016 | 1.00 | 10.55 |
| atom | 1480 | CZ  | TYR | 191 | 32.899 | 12.960 | 17.846 | 1.00 | 17.72 |
| atom | 1481 | OH  | TYR | 191 | 33.980 | 12.878 | 16.993 | 1.00 | 17.02 |
| atom | 1482 | C   | TYR | 191 | 29.614 | 11.086 | 21.438 | 1.00 | 13.99 |
| atom | 1483 | O   | TYR | 191 | 30.417 | 11.468 | 22.281 | 1.00 | 15.45 |
| atom | 1484 | N   | GLY | 192 | 29.486 | 9.810  | 21.091 | 1.00 | 16.18 |
| atom | 1485 | CA  | GLY | 192 | 30.271 | 8.772  | 21.728 | 1.00 | 11.25 |
| atom | 1486 | C   | GLY | 192 | 31.754 | 9.007  | 21.985 | 1.00 | 16.97 |
| atom | 1487 | O   | GLY | 192 | 32.189 | 8.813  | 23.105 | 1.00 | 18.14 |
| atom | 1488 | N   | PHE | 193 | 32.538 | 9.431  | 20.990 | 1.00 | 17.05 |
| atom | 1489 | CA  | PHE | 193 | 33.985 | 9.596  | 21.202 | 1.00 | 15.36 |
| atom | 1490 | CB  | PHE | 193 | 34.702 | 9.845  | 19.863 | 1.00 | 9.63  |
| atom | 1491 | CG  | PHE | 193 | 34.471 | 8.749  | 18.841 | 1.00 | 12.30 |
| atom | 1492 | CD1 | PHE | 193 | 33.799 | 9.024  | 17.636 | 1.00 | 9.68  |
| atom | 1493 | CD2 | PHE | 193 | 34.826 | 7.423  | 19.129 | 1.00 | 6.80  |
| atom | 1494 | CE1 | PHE | 193 | 33.472 | 7.995  | 16.736 | 1.00 | 6.25  |
| atom | 1495 | CE2 | PHE | 193 | 34.510 | 6.377  | 18.248 | 1.00 | 12.36 |
| atom | 1496 | CZ  | PHE | 193 | 33.827 | 6.655  | 17.045 | 1.00 | 6.66  |
| atom | 1497 | C   | PHE | 193 | 34.438 | 10.622 | 22.245 | 1.00 | 16.34 |
| atom | 1498 | O   | PHE | 193 | 35.632 | 10.764 | 22.508 | 1.00 | 17.70 |
| atom | 1499 | N   | GLN | 194 | 33.505 | 11.338 | 22.844 | 1.00 | 13.56 |
| atom | 1500 | CA  | GLN | 194 | 33.881 | 12.299 | 23.871 | 1.00 | 15.20 |
| atom | 1501 | CB  | GLN | 194 | 32.825 | 13.400 | 23.988 | 1.00 | 12.23 |
| atom | 1502 | CG  | GLN | 194 | 31.573 | 12.947 | 24.708 | 1.00 | 6.84  |
| atom | 1503 | CD  | GLN | 194 | 30.473 | 13.965 | 24.645 | 1.00 | 8.12  |
| atom | 1504 | OE1 | GLN | 194 | 30.645 | 15.117 | 25.055 | 1.00 | 15.59 |
| atom | 1505 | NE2 | GLN | 194 | 29.331 | 13.555 | 24.129 | 1.00 | 8.58  |
| atom | 1506 | C   | GLN | 194 | 33.973 | 11.547 | 25.209 | 1.00 | 18.89 |
| atom | 1507 | O   | GLN | 194 | 34.481 | 12.086 | 26.203 | 1.00 | 15.22 |
| atom | 1508 | N   | TYR | 195 | 33.492 | 10.298 | 25.210 | 1.00 | 14.89 |

| atom | 1509 | CA | TYR | 195 | 33.467 | 9.479 | 26.407 | 1.00 | 11.78 |
|------|------|-----|-----|-----|--------|--------|--------|------|-------|
| atom | 1510 | CB | TYR | 195 | 32.137 | 8.731 | 26.484 | 1.00 | 10.12 |
| atom | 1511 | CG | TYR | 195 | 30.923 | 9.639 | 26.603 | 1.00 | 7.64 |
| atom | 1512 | CD1 | TYR | 195 | 29.791 | 9.409 | 25.835 | 1.00 | 6.64 |
| atom | 1513 | CE1 | TYR | 195 | 28.684 | 10.240 | 25.913 | 1.00 | 9.04 |
| atom | 1514 | CD2 | TYR | 195 | 30.914 | 10.744 | 27.475 | 1.00 | 10.79 |
| atom | 1515 | CE2 | TYR | 195 | 29.802 | 11.587 | 27.562 | 1.00 | 3.29 |
| atom | 1516 | CZ | TYR | 195 | 28.691 | 11.324 | 26.774 | 1.00 | 8.40 |
| atom | 1517 | OH | TYR | 195 | 27.584 | 12.144 | 26.797 | 1.00 | 10.79 |
| atom | 1518 | C | TYR | 195 | 34.603 | 8.494 | 26.562 | 1.00 | 15.50 |
| atom | 1519 | O | TYR | 195 | 35.006 | 7.854 | 25.600 | 1.00 | 17.27 |
| atom | 1520 | N | SER | 196 | 35.139 | 8.395 | 27.778 | 1.00 | 17.84 |
| atom | 1521 | CA | SER | 196 | 36.213 | 7.441 | 28.071 | 1.00 | 17.44 |
| atom | 1522 | CB | SER | 196 | 36.937 | 7.792 | 29.383 | 1.00 | 22.27 |
| atom | 1523 | OG | SER | 196 | 36.088 | 7.619 | 30.511 | 1.00 | 24.61 |
| atom | 1524 | C | SER | 196 | 35.376 | 6.196 | 28.266 | 1.00 | 17.04 |
| atom | 1525 | O | SER | 196 | 34.159 | 6.304 | 28.424 | 1.00 | 19.71 |
| atom | 1526 | N | PRO | 197 | 35.980 | 5.003 | 28.222 | 1.00 | 15.92 |
| atom | 1527 | CD | PRO | 197 | 37.379 | 4.613 | 27.980 | 1.00 | 13.27 |
| atom | 1528 | CA | PRO | 197 | 35.072 | 3.861 | 28.422 | 1.00 | 18.00 |
| atom | 1529 | CB | PRO | 197 | 35.987 | 2.627 | 28.421 | 1.00 | 14.58 |
| atom | 1530 | CG | PRO | 197 | 37.391 | 3.129 | 28.250 | 1.00 | 12.92 |
| atom | 1531 | C | PRO | 197 | 34.245 | 3.988 | 29.707 | 1.00 | 21.73 |
| atom | 1532 | O | PRO | 197 | 33.088 | 3.557 | 29.760 | 1.00 | 24.27 |
| atom | 1533 | N | GLY | 198 | 34.829 | 4.609 | 30.732 | 1.00 | 25.79 |
| atom | 1534 | CA | GLY | 198 | 34.119 | 4.779 | 31.995 | 1.00 | 21.47 |
| atom | 1535 | C | GLY | 198 | 32.938 | 5.731 | 31.928 | 1.00 | 18.39 |
| atom | 1536 | O | GLY | 198 | 31.867 | 5.480 | 32.503 | 1.00 | 15.12 |
| atom | 1537 | N | GLN | 199 | 33.123 | 6.842 | 31.235 | 1.00 | 17.90 |
| atom | 1538 | CA | GLN | 199 | 32.033 | 7.798 | 31.104 | 1.00 | 23.69 |
| atom | 1539 | CB | GLN | 199 | 32.587 | 9.130 | 30.616 | 1.00 | 26.63 |
| atom | 1540 | CG | GLN | 199 | 33.592 | 9.732 | 31.595 | 1.00 | 24.77 |
| atom | 1541 | CD | GLN | 199 | 34.400 | 10.856 | 30.977 | 1.00 | 29.54 |
| atom | 1542 | OE1 | GLN | 199 | 34.958 | 10.715 | 29.889 | 1.00 | 23.75 |
| atom | 1543 | NE2 | GLN | 199 | 34.470 | 11.982 | 31.675 | 1.00 | 29.26 |
| atom | 1544 | C | GLN | 199 | 30.928 | 7.279 | 30.172 | 1.00 | 24.56 |

| atom | 1545 | O | GLN | 199 | 29.765 | 7.678 | 30.288 | 1.00 | 28.87 |
|------|------|------|------|-----|--------|--------|--------|------|-------|
| atom | 1546 | N | ARG | 200 | 31.287 | 6.373 | 29.264 | 1.00 | 21.97 |
| atom | 1547 | CA | ARG | 200 | 30.306 | 5.803 | 28.351 | 1.00 | 21.41 |
| atom | 1548 | CB | ARG | 200 | 30.965 | 4.906 | 27.307 | 1.00 | 22.44 |
| atom | 1549 | CG | ARG | 200 | 30.330 | 5.021 | 25.941 | 1.00 | 24.72 |
| atom | 1550 | CD | ARG | 200 | 30.120 | 3.666 | 25.239 | 1.00 | 29.31 |
| atom | 1551 | NE | ARG | 200 | 29.402 | 3.853 | 23.975 | 1.00 | 27.95 |
| atom | 1552 | CZ | ARG | 200 | 28.971 | 2.872 | 23.202 | 1.00 | 28.38 |
| atom | 1553 | NH1 | ARG | 200 | 29.177 | 1.603 | 23.542 | 1.00 | 39.86 |
| atom | 1554 | NH2 | ARG | 200 | 28.298 | 3.161 | 22.107 | 1.00 | 33.61 |
| atom | 1555 | C | ARG | 200 | 29.326 | 4.976 | 29.138 | 1.00 | 20.61 |
| atom | 1556 | O | ARG | 200 | 28.120 | 4.974 | 28.858 | 1.00 | 18.42 |
| atom | 1557 | N | VAL | 201 | 29.845 | 4.258 | 30.130 | 1.00 | 20.11 |
| atom | 1558 | CA | VAL | 201 | 28.981 | 3.426 | 30.951 | 1.00 | 16.47 |
| atom | 1559 | CB | VAL | 201 | 29.818 | 2.407 | 31.768 | 1.00 | 23.27 |
| atom | 1560 | CG1 | VAL | 201 | 30.856 | 3.125 | 32.639 | 1.00 | 23.85 |
| atom | 1561 | CG2 | VAL | 201 | 28.891 | 1.532 | 32.598 | 1.00 | 26.32 |
| atom | 1562 | C | VAL | 201 | 28.118 | 4.307 | 31.850 | 1.00 | 13.87 |
| atom | 1563 | O | VAL | 201 | 26.908 | 4.103 | 31.975 | 1.00 | 8.32 |
| atom | 1564 | N | GLU | 202 | 28.735 | 5.321 | 32.441 | 1.00 | 17.54 |
| atom | 1565 | CA | GLU | 202 | 28.018 | 6.232 | 33.326 | 1.00 | 16.90 |
| atom | 1566 | CB | GLU | 202 | 28.951 | 7.378 | 33.752 | 1.00 | 19.88 |
| atom | 1567 | CG | GLU | 202 | 28.444 | 8.294 | 34.881 | 1.00 | 22.45 |
| atom | 1568 | CD | GLU | 202 | 29.154 | 9.672 | 34.925 | 1.00 | 27.15 |
| atom | 1569 | OE1 | GLU | 202 | 30.263 | 9.831 | 34.348 | 1.00 | 26.78 |
| atom | 1570 | OE2 | GLU | 202 | 28.598 | 10.606 | 35.548 | 1.00 | 29.71 |
| atom | 1571 | C | GLU | 202 | 26.794 | 6.785 | 32.592 | 1.00 | 22.44 |
| atom | 1572 | O | GLU | 202 | 25.698 | 6.861 | 33.162 | 1.00 | 22.88 |
| atom | 1573 | N | PHE | 203 | 26.979 | 7.124 | 31.314 | 1.00 | 19.14 |
| atom | 1574 | CA | PHE | 203 | 25.915 | 7.693 | 30.506 | 1.00 | 15.22 |
| atom | 1575 | CB | PHE | 203 | 26.542 | 8.408 | 29.300 | 1.00 | 17.64 |
| atom | 1576 | CG | PHE | 203 | 25.544 | 9.051 | 28.376 | 1.00 | 21.29 |
| atom | 1577 | CD1 | PHE | 203 | 25.510 | 8.705 | 27.024 | 1.00 | 18.86 |
| atom | 1578 | CD2 | PHE | 203 | 24.622 | 9.989 | 28.857 | 1.00 | 19.90 |
| atom | 1579 | CE1 | PHE | 203 | 24.562 | 9.279 | 26.150 | 1.00 | 19.52 |
| atom | 1580 | CE2 | PHE | 203 | 23.671 | 10.576 | 28.002 | 1.00 | 19.32 |

| atom | 1581 | CZ | PHE | 203 | 23.639 | 10.218 | 26.643 | 1.00 | 21.32 |
|------|------|-----|-----|-----|--------|--------|--------|------|-------|
| atom | 1582 | C | PHE | 203 | 24.847 | 6.677 | 30.071 | 1.00 | 18.08 |
| atom | 1583 | O | PHE | 203 | 23.674 | 7.017 | 29.943 | 1.00 | 21.36 |
| atom | 1584 | N | LEU | 204 | 25.222 | 5.429 | 29.843 | 1.00 | 15.58 |
| atom | 1585 | CA | LEU | 204 | 24.208 | 4.460 | 29.435 | 1.00 | 16.10 |
| atom | 1586 | CB | LEU | 204 | 24.862 | 3.191 | 28.864 | 1.00 | 12.24 |
| atom | 1587 | CG | LEU | 204 | 25.732 | 3.460 | 27.630 | 1.00 | 20.18 |
| atom | 1588 | CD1 | LEU | 204 | 26.698 | 2.271 | 27.360 | 1.00 | 11.68 |
| atom | 1589 | CD2 | LEU | 204 | 24.794 | 3.757 | 26.412 | 1.00 | 15.33 |
| atom | 1590 | C | LEU | 204 | 23.333 | 4.116 | 30.641 | 1.00 | 17.00 |
| atom | 1591 | O | LEU | 204 | 22.107 | 4.178 | 30.561 | 1.00 | 14.29 |
| atom | 1592 | N | VAL | 205 | 23.976 | 3.754 | 31.754 | 1.00 | 18.18 |
| atom | 1593 | CA | VAL | 205 | 23.277 | 3.406 | 32.989 | 1.00 | 17.32 |
| atom | 1594 | CB | VAL | 205 | 24.299 | 3.141 | 34.149 | 1.00 | 21.22 |
| atom | 1595 | CG1 | VAL | 205 | 23.589 | 2.601 | 35.354 | 1.00 | 21.39 |
| atom | 1596 | CG2 | VAL | 205 | 25.374 | 2.141 | 33.718 | 1.00 | 13.01 |
| atom | 1597 | C | VAL | 205 | 22.343 | 4.572 | 33.378 | 1.00 | 20.78 |
| atom | 1598 | O | VAL | 205 | 21.143 | 4.385 | 33.624 | 1.00 | 20.38 |
| atom | 1599 | N | ASN | 206 | 22.890 | 5.780 | 33.408 | 1.00 | 22.91 |
| atom | 1600 | CA | ASN | 206 | 22.107 | 6.966 | 33.763 | 1.00 | 25.38 |
| atom | 1601 | CB | ASN | 206 | 22.974 | 8.216 | 33.745 | 1.00 | 26.40 |
| atom | 1602 | CG | ASN | 206 | 23.744 | 8.389 | 35.005 | 1.00 | 19.55 |
| atom | 1603 | OD1 | ASN | 206 | 24.444 | 9.382 | 35.184 | 1.00 | 21.80 |
| atom | 1604 | ND2 | ASN | 206 | 23.630 | 7.416 | 35.898 | 1.00 | 23.29 |
| atom | 1605 | C | ASN | 206 | 20.961 | 7.192 | 32.815 | 1.00 | 26.44 |
| atom | 1606 | O | ASN | 206 | 19.839 | 7.462 | 33.242 | 1.00 | 28.16 |
| atom | 1607 | N | THR | 207 | 21.256 | 7.127 | 31.523 | 1.00 | 24.98 |
| atom | 1608 | CA | THR | 207 | 20.219 | 7.313 | 30.530 | 1.00 | 21.70 |
| atom | 1609 | CB | THR | 207 | 20.753 | 7.043 | 29.129 | 1.00 | 17.74 |
| atom | 1610 | OG1 | THR | 207 | 21.645 | 8.099 | 28.752 | 1.00 | 16.88 |
| atom | 1611 | CG2 | THR | 207 | 19.609 | 6.968 | 28.142 | 1.00 | 22.24 |
| atom | 1612 | C | THR | 207 | 19.108 | 6.326 | 30.861 | 1.00 | 21.12 |
| atom | 1613 | O | THR | 207 | 17.939 | 6.694 | 30.952 | 1.00 | 20.10 |
| atom | 1614 | N | TRP | 208 | 19.505 | 5.073 | 31.052 | 1.00 | 23.41 |
| atom | 1615 | CA | TRP | 208 | 18.605 | 3.973 | 31.400 | 1.00 | 28.02 |
| atom | 1616 | CB | TRP | 208 | 19.424 | 2.689 | 31.533 | 1.00 | 27.05 |

| atom | 1617 | CG  | TRP | 208 | 18.613 | 1.453  | 31.511 | 1.00 | 31.65 |
|------|------|-----|-----|-----|--------|--------|--------|------|-------|
| atom | 1618 | CD2 | TRP | 208 | 18.175 | 0.738  | 30.352 | 1.00 | 33.96 |
| atom | 1619 | CE2 | TRP | 208 | 17.439 | -0.377 | 30.803 | 1.00 | 34.68 |
| atom | 1620 | CE3 | TRP | 208 | 18.324 | 0.937  | 28.975 | 1.00 | 33.40 |
| atom | 1621 | CD1 | TRP | 208 | 18.146 | 0.763  | 32.586 | 1.00 | 31.92 |
| atom | 1622 | NE1 | TRP | 208 | 17.440 | -0.339 | 32.171 | 1.00 | 35.78 |
| atom | 1623 | CZ2 | TRP | 208 | 16.863 | -1.299 | 29.929 | 1.00 | 36.13 |
| atom | 1624 | CZ3 | TRP | 208 | 17.749 | 0.019  | 28.103 | 1.00 | 31.62 |
| atom | 1625 | CH2 | TRP | 208 | 17.024 | -1.082 | 28.586 | 1.00 | 32.37 |
| atom | 1626 | C   | TRP | 208 | 17.836 | 4.214  | 32.711 | 1.00 | 32.31 |
| atom | 1627 | O   | TRP | 208 | 16.647 | 3.906  | 32.821 | 1.00 | 34.09 |
| atom | 1628 | N   | LYS | 209 | 18.520 | 4.751  | 33.716 | 1.00 | 33.04 |
| atom | 1629 | CA  | LYS | 209 | 17.884 | 5.016  | 35.001 | 1.00 | 33.68 |
| atom | 1630 | CB  | LYS | 209 | 18.943 | 5.467  | 36.027 | 1.00 | 35.81 |
| atom | 1631 | CG  | LYS | 209 | 19.240 | 4.451  | 37.153 | 1.00 | 34.82 |
| atom | 1632 | CD  | LYS | 209 | 19.556 | 3.046  | 36.626 | 1.00 | 30.83 |
| atom | 1633 | CE  | LYS | 209 | 20.925 | 2.582  | 37.117 | 1.00 | 33.81 |
| atom | 1634 | NZ  | LYS | 209 | 20.971 | 1.146  | 37.574 | 1.00 | 30.23 |
| atom | 1635 | C   | LYS | 209 | 16.785 | 6.077  | 34.881 | 1.00 | 33.73 |
| atom | 1636 | O   | LYS | 209 | 15.681 | 5.911  | 35.407 | 1.00 | 35.70 |
| atom | 1637 | N   | SER | 210 | 17.102 | 7.163  | 34.185 | 1.00 | 34.05 |
| atom | 1638 | CA  | SER | 210 | 16.188 | 8.279  | 33.979 | 1.00 | 33.11 |
| atom | 1639 | CB  | SER | 210 | 16.905 | 9.384  | 33.211 | 1.00 | 31.63 |
| atom | 1640 | OG  | SER | 210 | 17.028 | 9.031  | 31.838 | 1.00 | 26.49 |
| atom | 1641 | C   | SER | 210 | 14.932 | 7.896  | 33.208 | 1.00 | 36.12 |
| atom | 1642 | O   | SER | 210 | 14.069 | 8.733  | 32.955 | 1.00 | 38.49 |
| atom | 1643 | N   | LYS | 211 | 14.836 | 6.638  | 32.812 | 1.00 | 37.66 |
| atom | 1644 | CA  | LYS | 211 | 13.683 | 6.188  | 32.062 | 1.00 | 38.76 |
| atom | 1645 | CB  | LYS | 211 | 14.154 | 5.357  | 30.865 | 1.00 | 39.23 |
| atom | 1646 | CG  | LYS | 211 | 14.039 | 6.076  | 29.532 | 1.00 | 43.26 |
| atom | 1647 | CD  | LYS | 211 | 14.542 | 7.523  | 29.589 | 1.00 | 36.45 |
| atom | 1648 | CE  | LYS | 211 | 15.434 | 7.817  | 28.384 | 1.00 | 35.75 |
| atom | 1649 | NZ  | LYS | 211 | 15.475 | 9.271  | 28.025 | 1.00 | 36.57 |
| atom | 1650 | C   | LYS | 211 | 12.756 | 5.360  | 32.953 | 1.00 | 41.48 |
| atom | 1651 | O   | LYS | 211 | 13.161 | 4.326  | 33.493 | 1.00 | 40.77 |
| atom | 1652 | N   | LYS | 212 | 11.514 | 5.815  | 33.108 | 1.00 | 41.02 |

| atom | 1653 | CA | LYS | 212 | 10.539 | 5.100 | 33.922 | 1.00 | 39.98 |
|------|------|-----|-----|-----|--------|--------|--------|------|-------|
| atom | 1654 | CB | LYS | 212 | 9.167 | 5.736 | 33.779 | 1.00 | 38.68 |
| atom | 1655 | CG | LYS | 212 | 8.418 | 5.845 | 35.095 | 1.00 | 38.52 |
| atom | 1656 | CD | LYS | 212 | 7.007 | 6.349 | 34.886 | 1.00 | 40.92 |
| atom | 1657 | CE | LYS | 212 | 6.969 | 7.864 | 34.750 | 1.00 | 39.61 |
| atom | 1658 | NZ | LYS | 212 | 8.305 | 8.448 | 34.458 | 1.00 | 39.84 |
| atom | 1659 | C | LYS | 212 | 10.472 | 3.650 | 33.477 | 1.00 | 42.66 |
| atom | 1660 | O | LYS | 212 | 10.675 | 2.720 | 34.272 | 1.00 | 43.97 |
| atom | 1661 | N | ASN | 213 | 10.176 | 3.460 | 32.198 | 1.00 | 43.21 |
| atom | 1662 | CA | ASN | 213 | 10.111 | 2.123 | 31.632 | 1.00 | 44.99 |
| atom | 1663 | CB | ASN | 213 | 8.687 | 1.812 | 31.218 | 1.00 | 44.32 |
| atom | 1664 | CG | ASN | 213 | 7.848 | 1.389 | 32.394 | 1.00 | 47.00 |
| atom | 1665 | OD1 | ASN | 213 | 7.978 | 0.264 | 32.885 | 1.00 | 47.23 |
| atom | 1666 | ND2 | ASN | 213 | 6.994 | 2.291 | 32.871 | 1.00 | 44.11 |
| atom | 1667 | C | ASN | 213 | 11.077 | 2.061 | 30.457 | 1.00 | 45.25 |
| atom | 1668 | O | ASN | 213 | 10.706 | 2.296 | 29.302 | 1.00 | 48.85 |
| atom | 1669 | N | PRO | 214 | 12.347 | 1.740 | 30.754 | 1.00 | 42.66 |
| atom | 1670 | CD | PRO | 214 | 12.779 | 1.421 | 32.126 | 1.00 | 41.11 |
| atom | 1671 | CA | PRO | 214 | 13.465 | 1.629 | 29.815 | 1.00 | 38.85 |
| atom | 1672 | CB | PRO | 214 | 14.679 | 1.443 | 30.730 | 1.00 | 39.58 |
| atom | 1673 | CG | PRO | 214 | 14.122 | 0.774 | 31.913 | 1.00 | 40.23 |
| atom | 1674 | C | PRO | 214 | 13.414 | 0.552 | 28.745 | 1.00 | 33.30 |
| atom | 1675 | O | PRO | 214 | 13.110 | -0.612 | 29.022 | 1.00 | 29.69 |
| atom | 1676 | N | MET | 215 | 13.727 | 0.972 | 27.520 | 1.00 | 29.02 |
| atom | 1677 | CA | MET | 215 | 13.797 | 0.076 | 26.372 | 1.00 | 28.18 |
| atom | 1678 | CB | MET | 215 | 12.484 | 0.020 | 25.590 | 1.00 | 31.27 |
| atom | 1679 | CG | MET | 215 | 12.454 | -1.102 | 24.548 | 1.00 | 32.65 |
| atom | 1680 | SD | MET | 215 | 13.237 | -0.662 | 22.962 | 1.00 | 44.77 |
| atom | 1681 | CE | MET | 215 | 12.148 | 0.671 | 22.382 | 1.00 | 31.33 |
| atom | 1682 | C | MET | 215 | 14.869 | 0.639 | 25.481 | 1.00 | 25.30 |
| atom | 1683 | O | MET | 215 | 14.883 | 1.830 | 25.208 | 1.00 | 23.84 |
| atom | 1684 | N | GLY | 216 | 15.772 | -0.224 | 25.045 | 1.00 | 22.88 |
| atom | 1685 | CA | GLY | 216 | 16.840 | 0.215 | 24.184 | 1.00 | 26.69 |
| atom | 1686 | C | GLY | 216 | 17.160 | -0.813 | 23.126 | 1.00 | 28.78 |
| atom | 1687 | O | GLY | 216 | 16.853 | -1.996 | 23.283 | 1.00 | 24.75 |
| atom | 1688 | N | PHE | 217 | 17.778 | -0.344 | 22.046 | 1.00 | 27.24 |

| atom | 1689 | CA | PHE | 217 | 18.162 | -1.197 | 20.938 | 1.00 | 22.26 |
|------|------|-----|-----|-----|--------|--------|--------|------|-------|
| atom | 1690 | CB | PHE | 217 | 17.000 | -1.345 | 19.931 | 1.00 | 21.51 |
| atom | 1691 | CG | PHE | 217 | 16.623 | -0.057 | 19.212 | 1.00 | 24.23 |
| atom | 1692 | CD1 | PHE | 217 | 15.596 | 0.761 | 19.701 | 1.00 | 18.03 |
| atom | 1693 | CD2 | PHE | 217 | 17.322 | 0.359 | 18.066 | 1.00 | 24.84 |
| atom | 1694 | CE1 | PHE | 217 | 15.277 | 1.974 | 19.070 | 1.00 | 14.77 |
| atom | 1695 | CE2 | PHE | 217 | 17.007 | 1.582 | 17.426 | 1.00 | 17.90 |
| atom | 1696 | CZ | PHE | 217 | 15.986 | 2.384 | 17.933 | 1.00 | 18.19 |
| atom | 1697 | C | PHE | 217 | 19.380 | -0.619 | 20.230 | 1.00 | 23.61 |
| atom | 1698 | O | PHE | 217 | 19.649 | 0.595 | 20.273 | 1.00 | 18.23 |
| atom | 1699 | N | SER | 218 | 20.130 | -1.504 | 19.588 | 1.00 | 21.59 |
| atom | 1700 | CA | SER | 218 | 21.273 | -1.075 | 18.837 | 1.00 | 20.66 |
| atom | 1701 | CB | SER | 218 | 22.447 | -2.013 | 19.053 | 1.00 | 18.24 |
| atom | 1702 | OG | SER | 218 | 22.156 | -3.312 | 18.588 | 1.00 | 31.33 |
| atom | 1703 | C | SER | 218 | 20.752 | -1.166 | 17.425 | 1.00 | 18.59 |
| atom | 1704 | O | SER | 218 | 19.708 | -1.733 | 17.192 | 1.00 | 22.08 |
| atom | 1705 | N | TYR | 219 | 21.453 | -0.570 | 16.487 | 1.00 | 20.71 |
| atom | 1706 | CA | TYR | 219 | 21.031 | -0.629 | 15.115 | 1.00 | 24.24 |
| atom | 1707 | CB | TYR | 219 | 20.477 | 0.718 | 14.653 | 1.00 | 25.03 |
| atom | 1708 | CG | TYR | 219 | 19.842 | 0.621 | 13.290 | 1.00 | 30.08 |
| atom | 1709 | CD1 | TYR | 219 | 18.504 | 0.242 | 13.155 | 1.00 | 28.21 |
| atom | 1710 | CE1 | TYR | 219 | 17.921 | 0.080 | 11.902 | 1.00 | 28.31 |
| atom | 1711 | CD2 | TYR | 219 | 20.582 | 0.849 | 12.132 | 1.00 | 23.75 |
| atom | 1712 | CE2 | TYR | 219 | 20.003 | 0.688 | 10.871 | 1.00 | 26.41 |
| atom | 1713 | CZ | TYR | 219 | 18.677 | 0.298 | 10.765 | 1.00 | 25.69 |
| atom | 1714 | OH | TYR | 219 | 18.123 | 0.059 | 9.531 | 1.00 | 24.99 |
| atom | 1715 | C | TYR | 219 | 22.281 | -0.961 | 14.343 | 1.00 | 25.43 |
| atom | 1716 | O | TYR | 219 | 23.206 | -0.153 | 14.297 | 1.00 | 26.39 |
| atom | 1717 | N | ASP | 220 | 22.329 | -2.156 | 13.769 | 1.00 | 28.31 |
| atom | 1718 | CA | ASP | 220 | 23.496 | -2.558 | 12.996 | 1.00 | 31.56 |
| atom | 1719 | CB | ASP | 220 | 23.830 | -4.030 | 13.226 | 1.00 | 33.93 |
| atom | 1720 | CG | ASP | 220 | 25.261 | -4.361 | 12.827 | 1.00 | 42.92 |
| atom | 1721 | OD1 | ASP | 220 | 26.170 | -3.557 | 13.165 | 1.00 | 40.05 |
| atom | 1722 | OD2 | ASP | 220 | 25.476 | -5.410 | 12.170 | 1.00 | 44.65 |
| atom | 1723 | C | ASP | 220 | 23.227 | -2.320 | 11.520 | 1.00 | 33.04 |
| atom | 1724 | O | ASP | 220 | 22.497 | -3.073 | 10.877 | 1.00 | 31.00 |

| atom | 1725 | N   | THR | 221 | 23.808 | -1.263 | 10.980 | 1.00 | 32.02 |
|------|------|-----|-----|-----|--------|--------|--------|------|-------|
| atom | 1726 | CA  | THR | 221 | 23.591 | -0.958 | 9.581  | 1.00 | 38.44 |
| atom | 1727 | CB  | THR | 221 | 23.564 | 0.584  | 9.406  | 1.00 | 41.81 |
| atom | 1728 | OG1 | THR | 221 | 24.010 | 0.941  | 8.092  | 1.00 | 50.19 |
| atom | 1729 | CG2 | THR | 221 | 24.430 | 1.251  | 10.476 | 1.00 | 41.82 |
| atom | 1730 | C   | THR | 221 | 24.656 | -1.663 | 8.704  | 1.00 | 38.27 |
| atom | 1731 | O   | THR | 221 | 25.861 | -1.558 | 8.961  | 1.00 | 37.56 |
| atom | 1732 | N   | ARG | 222 | 24.204 | -2.402 | 7.689  | 1.00 | 36.38 |
| atom | 1733 | CA  | ARG | 222 | 25.105 | -3.153 | 6.805  | 1.00 | 38.82 |
| atom | 1734 | CB  | ARG | 222 | 24.277 | -4.055 | 5.887  | 1.00 | 44.88 |
| atom | 1735 | CG  | ARG | 222 | 24.392 | -5.532 | 6.237  | 1.00 | 55.39 |
| atom | 1736 | CD  | ARG | 222 | 24.585 | -6.394 | 5.000  | 1.00 | 60.84 |
| atom | 1737 | NE  | ARG | 222 | 25.888 | -6.166 | 4.375  | 1.00 | 65.59 |
| atom | 1738 | CZ  | ARG | 222 | 26.415 | -6.937 | 3.424  | 1.00 | 66.51 |
| atom | 1739 | NH1 | ARG | 222 | 25.751 | -7.997 | 2.976  | 1.00 | 65.57 |
| atom | 1740 | NH2 | ARG | 222 | 27.611 | -6.649 | 2.921  | 1.00 | 67.20 |
| atom | 1741 | C   | ARG | 222 | 26.087 | -2.312 | 5.964  | 1.00 | 35.30 |
| atom | 1742 | O   | ARG | 222 | 25.664 | -1.489 | 5.144  | 1.00 | 34.68 |
| atom | 1743 | N   | CYS | 223 | 27.391 | -2.552 | 6.140  | 1.00 | 28.37 |
| atom | 1744 | CA  | CYS | 223 | 28.425 | -1.786 | 5.431  | 1.00 | 25.64 |
| atom | 1745 | CB  | CYS | 223 | 28.693 | -2.330 | 4.026  | 1.00 | 28.82 |
| atom | 1746 | SG  | CYS | 223 | 27.878 | -3.882 | 3.597  | 1.00 | 38.53 |
| atom | 1747 | C   | CYS | 223 | 27.953 | -0.345 | 5.334  | 1.00 | 24.16 |
| atom | 1748 | O   | CYS | 223 | 27.476 | 0.127  | 4.296  | 1.00 | 26.66 |
| atom | 1749 | N   | PHE | 224 | 28.069 | 0.355  | 6.445  | 1.00 | 19.76 |
| atom | 1750 | CA  | PHE | 224 | 27.617 | 1.720  | 6.495  | 1.00 | 17.21 |
| atom | 1751 | CB  | PHE | 224 | 27.856 | 2.311  | 7.877  | 1.00 | 11.71 |
| atom | 1752 | CG  | PHE | 224 | 27.251 | 3.641  | 8.038  | 1.00 | 6.54  |
| atom | 1753 | CD1 | PHE | 224 | 28.009 | 4.766  | 7.904  | 1.00 | 3.35  |
| atom | 1754 | CD2 | PHE | 224 | 25.892 | 3.765  | 8.248  | 1.00 | 12.53 |
| atom | 1755 | CE1 | PHE | 224 | 27.436 | 6.004  | 7.966  | 1.00 | 12.14 |
| atom | 1756 | CE2 | PHE | 224 | 25.299 | 5.013  | 8.317  | 1.00 | 17.37 |
| atom | 1757 | CZ  | PHE | 224 | 26.080 | 6.136  | 8.172  | 1.00 | 12.23 |
| atom | 1758 | C   | PHE | 224 | 28.303 | 2.578  | 5.449  | 1.00 | 20.24 |
| atom | 1759 | O   | PHE | 224 | 27.700 | 3.501  | 4.899  | 1.00 | 18.35 |
| atom | 1760 | N   | ASP | 225 | 29.573 | 2.269  | 5.197  | 1.00 | 19.31 |

| atom | 1761 | CA  | ASP | 225 | 30.362 | 3.005  | 4.227  | 1.00 | 16.43 |
|------|------|-----|-----|-----|--------|--------|--------|------|-------|
| atom | 1762 | CB  | ASP | 225 | 31.798 | 2.482  | 4.217  | 1.00 | 18.29 |
| atom | 1763 | CG  | ASP | 225 | 32.601 | 2.966  | 5.417  | 1.00 | 19.09 |
| atom | 1764 | OD1 | ASP | 225 | 31.977 | 3.479  | 6.366  | 1.00 | 17.99 |
| atom | 1765 | OD2 | ASP | 225 | 33.845 | 2.837  | 5.415  | 1.00 | 17.38 |
| atom | 1766 | C   | ASP | 225 | 29.760 | 2.899  | 2.842  | 1.00 | 17.37 |
| atom | 1767 | O   | ASP | 225 | 29.723 | 3.883  | 2.110  | 1.00 | 20.94 |
| atom | 1768 | N   | SER | 226 | 29.261 | 1.717  | 2.492  | 1.00 | 15.11 |
| atom | 1769 | CA  | SER | 226 | 28.673 | 1.514  | 1.176  | 1.00 | 14.56 |
| atom | 1770 | CB  | SER | 226 | 28.655 | 0.021  | 0.851  | 1.00 | 17.21 |
| atom | 1771 | OG  | SER | 226 | 29.953 | -0.366 | 0.410  | 1.00 | 21.67 |
| atom | 1772 | C   | SER | 226 | 27.276 | 2.113  | 0.982  | 1.00 | 14.33 |
| atom | 1773 | O   | SER | 226 | 26.799 | 2.244  | -0.147 | 1.00 | 10.11 |
| atom | 1774 | N   | THR | 227 | 26.623 | 2.490  | 2.073  | 1.00 | 9.91  |
| atom | 1775 | CA  | THR | 227 | 25.294 | 3.071  | 1.971  | 1.00 | 8.56  |
| atom | 1776 | CB  | THR | 227 | 24.369 | 2.649  | 3.150  | 1.00 | 10.08 |
| atom | 1777 | OG1 | THR | 227 | 24.792 | 3.302  | 4.359  | 1.00 | 12.01 |
| atom | 1778 | CG2 | THR | 227 | 24.418 | 1.143  | 3.355  | 1.00 | 10.18 |
| atom | 1779 | C   | THR | 227 | 25.350 | 4.574  | 1.969  | 1.00 | 6.14  |
| atom | 1780 | O   | THR | 227 | 24.344 | 5.214  | 1.758  | 1.00 | 11.13 |
| atom | 1781 | N   | VAL | 228 | 26.513 | 5.153  | 2.230  | 1.00 | 5.87  |
| atom | 1782 | CA  | VAL | 228 | 26.582 | 6.601  | 2.228  | 1.00 | 9.08  |
| atom | 1783 | CB  | VAL | 228 | 27.858 | 7.094  | 2.921  | 1.00 | 7.34  |
| atom | 1784 | CG1 | VAL | 228 | 27.948 | 8.626  | 2.854  | 1.00 | 6.44  |
| atom | 1785 | CG2 | VAL | 228 | 27.844 | 6.631  | 4.371  | 1.00 | 9.87  |
| atom | 1786 | C   | VAL | 228 | 26.515 | 7.106  | 0.781  | 1.00 | 12.58 |
| atom | 1787 | O   | VAL | 228 | 27.266 | 6.663  | -0.092 | 1.00 | 14.27 |
| atom | 1788 | N   | THR | 229 | 25.599 | 8.031  | 0.532  | 1.00 | 10.63 |
| atom | 1789 | CA  | THR | 229 | 25.429 | 8.566  | -0.799 | 1.00 | 8.72  |
| atom | 1790 | CB  | THR | 229 | 23.944 | 8.879  | -1.091 | 1.00 | 7.09  |
| atom | 1791 | OG1 | THR | 229 | 23.484 | 9.923  | -0.228 | 1.00 | 5.58  |
| atom | 1792 | CG2 | THR | 229 | 23.100 | 7.649  | -0.881 | 1.00 | 5.09  |
| atom | 1793 | C   | THR | 229 | 26.245 | 9.821  | -1.001 | 1.00 | 15.73 |
| atom | 1794 | O   | THR | 229 | 26.903 | 10.327 | -0.070 | 1.00 | 17.77 |
| atom | 1795 | N   | GLU | 230 | 26.209 | 10.321 | -2.229 | 1.00 | 15.28 |
| atom | 1796 | CA  | GLU | 230 | 26.933 | 11.522 | -2.568 | 1.00 | 16.64 |

| atom | 1797 | CB | GLU | 230 | 26.949 | 11.705 | -4.082 | 1.00 | 26.00 |
|------|------|-----|-----|-----|--------|--------|--------|------|-------|
| atom | 1798 | CG | GLU | 230 | 27.757 | 10.621 | -4.804 | 1.00 | 34.06 |
| atom | 1799 | CD | GLU | 230 | 28.196 | 11.032 | -6.212 | 1.00 | 38.31 |
| atom | 1800 | OE1 | GLU | 230 | 27.683 | 12.059 | -6.730 | 1.00 | 37.25 |
| atom | 1801 | OE2 | GLU | 230 | 29.042 | 10.315 | -6.799 | 1.00 | 39.10 |
| atom | 1802 | C | GLU | 230 | 26.170 | 12.632 | -1.885 | 1.00 | 17.08 |
| atom | 1803 | O | GLU | 230 | 26.756 | 13.625 | -1.413 | 1.00 | 16.22 |
| atom | 1804 | N | ASN | 231 | 24.853 | 12.447 | -1.827 | 1.00 | 11.51 |
| atom | 1805 | CA | ASN | 231 | 23.985 | 13.406 | -1.165 | 1.00 | 13.50 |
| atom | 1806 | CB | ASN | 231 | 22.547 | 12.925 | -1.153 | 1.00 | 19.01 |
| atom | 1807 | CG | ASN | 231 | 21.666 | 13.780 | -0.266 | 1.00 | 22.76 |
| atom | 1808 | OD1 | ASN | 231 | 21.399 | 13.441 | 0.909 | 1.00 | 20.68 |
| atom | 1809 | ND2 | ASN | 231 | 21.202 | 14.897 | -0.820 | 1.00 | 17.27 |
| atom | 1810 | C | ASN | 231 | 24.424 | 13.570 | 0.285 | 1.00 | 14.00 |
| atom | 1811 | O | ASN | 231 | 24.589 | 14.691 | 0.765 | 1.00 | 13.52 |
| atom | 1812 | N | ASP | 232 | 24.593 | 12.437 | 0.974 | 1.00 | 8.91 |
| atom | 1813 | CA | ASP | 232 | 25.013 | 12.407 | 2.372 | 1.00 | 2.20 |
| atom | 1814 | CB | ASP | 232 | 25.279 | 10.970 | 2.794 | 1.00 | 2.00 |
| atom | 1815 | CG | ASP | 232 | 24.033 | 10.129 | 2.822 | 1.00 | 3.69 |
| atom | 1816 | OD1 | ASP | 232 | 24.182 | 8.903 | 2.689 | 1.00 | 10.01 |
| atom | 1817 | OD2 | ASP | 232 | 22.918 | 10.669 | 2.991 | 1.00 | 2.00 |
| atom | 1818 | C | ASP | 232 | 26.290 | 13.211 | 2.589 | 1.00 | 7.59 |
| atom | 1819 | O | ASP | 232 | 26.374 | 14.055 | 3.490 | 1.00 | 6.64 |
| atom | 1820 | N | ILE | 233 | 27.295 | 12.907 | 1.765 | 1.00 | 8.41 |
| atom | 1821 | CA | ILE | 233 | 28.588 | 13.565 | 1.820 | 1.00 | 11.32 |
| atom | 1822 | CB | ILE | 233 | 29.612 | 12.848 | 0.900 | 1.00 | 15.31 |
| atom | 1823 | CG2 | ILE | 233 | 30.929 | 13.593 | 0.898 | 1.00 | 9.82 |
| atom | 1824 | CG1 | ILE | 233 | 29.813 | 11.416 | 1.384 | 1.00 | 14.26 |
| atom | 1825 | CD1 | ILE | 233 | 29.979 | 10.419 | 0.281 | 1.00 | 19.91 |
| atom | 1826 | C | ILE | 233 | 28.481 | 15.035 | 1.420 | 1.00 | 13.30 |
| atom | 1827 | O | ILE | 233 | 29.285 | 15.857 | 1.880 | 1.00 | 10.15 |
| atom | 1828 | N | ARG | 234 | 27.498 | 15.368 | 0.574 | 1.00 | 7.69 |
| atom | 1829 | CA | ARG | 234 | 27.331 | 16.768 | 0.184 | 1.00 | 10.10 |
| atom | 1830 | CB | ARG | 234 | 26.530 | 16.900 | -1.103 | 1.00 | 6.01 |
| atom | 1831 | CG | ARG | 234 | 27.417 | 17.138 | -2.298 | 1.00 | 17.01 |
| atom | 1832 | CD | ARG | 234 | 26.619 | 17.293 | -3.600 | 1.00 | 21.96 |

| | | | | | | | | | |
|---|---|---|---|---|---|---|---|---|---|
| atom | 1833 | NE | ARG | 234 | 27.082 | 16.371 | -4.630 | 1.00 | 24.65 |
| atom | 1834 | CZ | ARG | 234 | 26.349 | 15.365 | -5.111 | 1.00 | 34.89 |
| atom | 1835 | NH1 | ARG | 234 | 25.113 | 15.153 | -4.656 | 1.00 | 34.61 |
| atom | 1836 | NH2 | ARG | 234 | 26.851 | 14.553 | -6.037 | 1.00 | 31.42 |
| atom | 1837 | C | ARG | 234 | 26.625 | 17.475 | 1.322 | 1.00 | 8.60 |
| atom | 1838 | O | ARG | 234 | 26.956 | 18.600 | 1.665 | 1.00 | 14.56 |
| atom | 1839 | N | VAL | 235 | 25.656 | 16.787 | 1.905 | 1.00 | 9.72 |
| atom | 1840 | CA | VAL | 235 | 24.902 | 17.288 | 3.039 | 1.00 | 13.66 |
| atom | 1841 | CB | VAL | 235 | 23.873 | 16.229 | 3.508 | 1.00 | 19.56 |
| atom | 1842 | CG1 | VAL | 235 | 23.341 | 16.596 | 4.897 | 1.00 | 20.84 |
| atom | 1843 | CG2 | VAL | 235 | 22.725 | 16.119 | 2.487 | 1.00 | 8.84 |
| atom | 1844 | C | VAL | 235 | 25.946 | 17.544 | 4.137 | 1.00 | 18.09 |
| atom | 1845 | O | VAL | 235 | 25.908 | 18.550 | 4.853 | 1.00 | 15.25 |
| atom | 1846 | N | GLU | 236 | 26.893 | 16.622 | 4.250 | 1.00 | 16.55 |
| atom | 1847 | CA | GLU | 236 | 27.974 | 16.776 | 5.209 | 1.00 | 21.32 |
| atom | 1848 | CB | GLU | 236 | 29.018 | 15.693 | 4.987 | 1.00 | 25.28 |
| atom | 1849 | CG | GLU | 236 | 29.445 | 14.939 | 6.205 | 1.00 | 26.53 |
| atom | 1850 | CD | GLU | 236 | 30.090 | 13.622 | 5.831 | 1.00 | 30.94 |
| atom | 1851 | OE1 | GLU | 236 | 29.355 | 12.613 | 5.779 | 1.00 | 30.71 |
| atom | 1852 | OE2 | GLU | 236 | 31.321 | 13.604 | 5.585 | 1.00 | 29.82 |
| atom | 1853 | C | GLU | 236 | 28.647 | 18.136 | 5.009 | 1.00 | 23.02 |
| atom | 1854 | O | GLU | 236 | 28.558 | 19.013 | 5.854 | 1.00 | 27.89 |
| atom | 1855 | N | GLU | 237 | 29.329 | 18.303 | 3.882 | 1.00 | 25.21 |
| atom | 1856 | CA | GLU | 237 | 30.035 | 19.547 | 3.581 | 1.00 | 25.51 |
| atom | 1857 | CB | GLU | 237 | 30.327 | 19.612 | 2.087 | 1.00 | 28.35 |
| atom | 1858 | CG | GLU | 237 | 31.306 | 20.686 | 1.692 | 1.00 | 28.67 |
| atom | 1859 | CD | GLU | 237 | 30.690 | 21.709 | 0.744 | 1.00 | 30.11 |
| atom | 1860 | OE1 | GLU | 237 | 29.455 | 21.703 | 0.543 | 1.00 | 32.75 |
| atom | 1861 | OE2 | GLU | 237 | 31.443 | 22.529 | 0.196 | 1.00 | 28.55 |
| atom | 1862 | C | GLU | 237 | 29.312 | 20.829 | 4.021 | 1.00 | 27.38 |
| atom | 1863 | O | GLU | 237 | 29.936 | 21.737 | 4.603 | 1.00 | 25.26 |
| atom | 1864 | N | SER | 238 | 28.004 | 20.900 | 3.758 | 1.00 | 25.92 |
| atom | 1865 | CA | SER | 238 | 27.221 | 22.089 | 4.113 | 1.00 | 22.66 |
| atom | 1866 | CB | SER | 238 | 25.820 | 22.024 | 3.481 | 1.00 | 16.27 |
| atom | 1867 | OG | SER | 238 | 24.918 | 21.202 | 4.201 | 1.00 | 20.41 |
| atom | 1868 | C | SER | 238 | 27.130 | 22.344 | 5.619 | 1.00 | 22.53 |

| atom | 1869 | O | SER | 238 | 26.926 | 23.481 | 6.055 | 1.00 | 23.80 |
| atom | 1870 | N | ILE | 239 | 27.278 | 21.286 | 6.408 | 1.00 | 19.38 |
| atom | 1871 | CA | ILE | 239 | 27.271 | 21.416 | 7.863 | 1.00 | 18.62 |
| atom | 1872 | CB | ILE | 239 | 27.138 | 20.044 | 8.548 | 1.00 | 12.66 |
| atom | 1873 | CG2 | ILE | 239 | 27.433 | 20.172 | 10.031 | 1.00 | 13.29 |
| atom | 1874 | CG1 | ILE | 239 | 25.719 | 19.513 | 8.320 | 1.00 | 9.59 |
| atom | 1875 | CD1 | ILE | 239 | 25.482 | 18.160 | 8.914 | 1.00 | 8.77 |
| atom | 1876 | C | ILE | 239 | 28.620 | 22.050 | 8.240 | 1.00 | 19.33 |
| atom | 1877 | O | ILE | 239 | 28.675 | 23.007 | 9.002 | 1.00 | 22.17 |
| atom | 1878 | N | TYR | 240 | 29.706 | 21.512 | 7.692 | 1.00 | 18.63 |
| atom | 1879 | CA | TYR | 240 | 31.033 | 22.060 | 7.937 | 1.00 | 15.25 |
| atom | 1880 | CB | TYR | 240 | 32.101 | 21.358 | 7.099 | 1.00 | 12.94 |
| atom | 1881 | CG | TYR | 240 | 32.232 | 19.879 | 7.303 | 1.00 | 15.61 |
| atom | 1882 | CD1 | TYR | 240 | 31.810 | 19.268 | 8.494 | 1.00 | 7.96 |
| atom | 1883 | CE1 | TYR | 240 | 31.900 | 17.894 | 8.658 | 1.00 | 14.82 |
| atom | 1884 | CD2 | TYR | 240 | 32.751 | 19.070 | 6.279 | 1.00 | 12.69 |
| atom | 1885 | CE2 | TYR | 240 | 32.845 | 17.684 | 6.428 | 1.00 | 13.70 |
| atom | 1886 | CZ | TYR | 240 | 32.412 | 17.100 | 7.622 | 1.00 | 19.88 |
| atom | 1887 | OH | TYR | 240 | 32.459 | 15.729 | 7.766 | 1.00 | 22.17 |
| atom | 1888 | C | TYR | 240 | 31.060 | 23.524 | 7.527 | 1.00 | 17.48 |
| atom | 1889 | O | TYR | 240 | 31.664 | 24.344 | 8.210 | 1.00 | 18.27 |
| atom | 1890 | N | GLN | 241 | 30.417 | 23.846 | 6.401 | 1.00 | 16.83 |
| atom | 1891 | CA | GLN | 241 | 30.428 | 25.219 | 5.900 | 1.00 | 16.08 |
| atom | 1892 | CB | GLN | 241 | 29.932 | 25.296 | 4.439 | 1.00 | 16.21 |
| atom | 1893 | CG | GLN | 241 | 30.778 | 24.585 | 3.374 | 1.00 | 9.37 |
| atom | 1894 | CD | GLN | 241 | 32.164 | 25.184 | 3.171 | 1.00 | 17.64 |
| atom | 1895 | OE1 | GLN | 241 | 32.458 | 26.302 | 3.620 | 1.00 | 14.54 |
| atom | 1896 | NE2 | GLN | 241 | 33.032 | 24.432 | 2.484 | 1.00 | 16.20 |
| atom | 1897 | C | GLN | 241 | 29.578 | 26.137 | 6.768 | 1.00 | 18.69 |
| atom | 1898 | O | GLN | 241 | 29.616 | 27.355 | 6.628 | 1.00 | 18.09 |
| atom | 1899 | N | CYS | 242 | 28.786 | 25.560 | 7.655 | 1.00 | 19.26 |
| atom | 1900 | CA | CYS | 242 | 27.983 | 26.385 | 8.519 | 1.00 | 17.13 |
| atom | 1901 | CB | CYS | 242 | 26.889 | 25.548 | 9.145 | 1.00 | 16.03 |
| atom | 1902 | SG | CYS | 242 | 25.549 | 25.325 | 7.971 | 1.00 | 24.62 |
| atom | 1903 | C | CYS | 242 | 28.922 | 26.979 | 9.568 | 1.00 | 21.09 |
| atom | 1904 | O | CYS | 242 | 28.552 | 27.911 | 10.295 | 1.00 | 19.82 |

| atom | 1905 | N   | CYS | 243 | 30.141 | 26.436 | 9.617  | 1.00 | 21.37 |
|------|------|-----|-----|-----|--------|--------|--------|------|-------|
| atom | 1906 | CA  | CYS | 243 | 31.188 | 26.907 | 10.534 | 1.00 | 24.50 |
| atom | 1907 | CB  | CYS | 243 | 32.382 | 25.951 | 10.579 | 1.00 | 15.34 |
| atom | 1908 | SG  | CYS | 243 | 32.092 | 24.409 | 11.389 | 1.00 | 29.68 |
| atom | 1909 | C   | CYS | 243 | 31.730 | 28.235 | 10.034 | 1.00 | 26.31 |
| atom | 1910 | O   | CYS | 243 | 31.511 | 28.621 | 8.883  | 1.00 | 21.13 |
| atom | 1911 | N   | ASP | 244 | 32.445 | 28.920 | 10.919 | 1.00 | 28.04 |
| atom | 1912 | CA  | ASP | 244 | 33.085 | 30.174 | 10.580 | 1.00 | 27.16 |
| atom | 1913 | CB  | ASP | 244 | 33.130 | 31.100 | 11.784 | 1.00 | 29.16 |
| atom | 1914 | CG  | ASP | 244 | 33.905 | 32.357 | 11.514 | 1.00 | 31.43 |
| atom | 1915 | OD1 | ASP | 244 | 35.117 | 32.266 | 11.220 | 1.00 | 37.16 |
| atom | 1916 | OD2 | ASP | 244 | 33.294 | 33.441 | 11.590 | 1.00 | 36.68 |
| atom | 1917 | C   | ASP | 244 | 34.487 | 29.727 | 10.237 | 1.00 | 25.91 |
| atom | 1918 | O   | ASP | 244 | 35.279 | 29.447 | 11.129 | 1.00 | 26.07 |
| atom | 1919 | N   | LEU | 245 | 34.776 | 29.634 | 8.944  | 1.00 | 26.85 |
| atom | 1920 | CA  | LEU | 245 | 36.088 | 29.191 | 8.467  | 1.00 | 25.33 |
| atom | 1921 | CB  | LEU | 245 | 35.944 | 27.889 | 7.661  | 1.00 | 23.00 |
| atom | 1922 | CG  | LEU | 245 | 35.291 | 26.675 | 8.334  | 1.00 | 23.70 |
| atom | 1923 | CD1 | LEU | 245 | 34.274 | 26.094 | 7.391  | 1.00 | 24.92 |
| atom | 1924 | CD2 | LEU | 245 | 36.349 | 25.633 | 8.710  | 1.00 | 23.20 |
| atom | 1925 | C   | LEU | 245 | 36.846 | 30.213 | 7.609  | 1.00 | 25.10 |
| atom | 1926 | O   | LEU | 245 | 36.262 | 31.073 | 6.931  | 1.00 | 19.47 |
| atom | 1927 | N   | ALA | 246 | 38.165 | 30.127 | 7.661  | 1.00 | 22.80 |
| atom | 1928 | CA  | ALA | 246 | 38.968 | 31.003 | 6.841  | 1.00 | 24.53 |
| atom | 1929 | CB  | ALA | 246 | 40.432 | 30.626 | 6.981  | 1.00 | 18.27 |
| atom | 1930 | C   | ALA | 246 | 38.488 | 30.761 | 5.391  | 1.00 | 25.10 |
| atom | 1931 | O   | ALA | 246 | 38.089 | 29.647 | 5.030  | 1.00 | 21.52 |
| atom | 1932 | N   | PRO | 247 | 38.515 | 31.802 | 4.549  | 1.00 | 25.86 |
| atom | 1933 | CD  | PRO | 247 | 38.964 | 33.168 | 4.880  | 1.00 | 26.97 |
| atom | 1934 | CA  | PRO | 247 | 38.086 | 31.683 | 3.146  | 1.00 | 24.74 |
| atom | 1935 | CB  | PRO | 247 | 38.381 | 33.070 | 2.561  | 1.00 | 24.27 |
| atom | 1936 | CG  | PRO | 247 | 38.414 | 33.994 | 3.744  | 1.00 | 23.86 |
| atom | 1937 | C   | PRO | 247 | 38.806 | 30.564 | 2.363  | 1.00 | 25.32 |
| atom | 1938 | O   | PRO | 247 | 38.227 | 29.934 | 1.483  | 1.00 | 27.92 |
| atom | 1939 | N   | GLU | 248 | 40.070 | 30.335 | 2.693  | 1.00 | 26.43 |
| atom | 1940 | CA  | GLU | 248 | 40.895 | 29.310 | 2.064  | 1.00 | 29.15 |

| atom | 1941 | CB | GLU | 248 | 42.361 | 29.577 | 2.371 | 1.00 35.18 |
|------|------|-----|-----|-----|--------|--------|--------|------------|
| atom | 1942 | CG | GLU | 248 | 42.988 | 30.634 | 1.512 | 1.00 40.16 |
| atom | 1943 | CD | GLU | 248 | 44.358 | 30.220 | 1.075 | 1.00 42.56 |
| atom | 1944 | OE1 | GLU | 248 | 44.555 | 29.000 | 0.850 | 1.00 42.61 |
| atom | 1945 | OE2 | GLU | 248 | 45.232 | 31.109 | 0.966 | 1.00 46.82 |
| atom | 1946 | C | GLU | 248 | 40.565 | 27.908 | 2.561 | 1.00 32.21 |
| atom | 1947 | O | GLU | 248 | 40.986 | 26.914 | 1.967 | 1.00 35.04 |
| atom | 1948 | N | ALA | 249 | 39.866 | 27.829 | 3.684 | 1.00 26.94 |
| atom | 1949 | CA | ALA | 249 | 39.477 | 26.542 | 4.232 | 1.00 27.37 |
| atom | 1950 | CB | ALA | 249 | 39.255 | 26.651 | 5.769 | 1.00 26.86 |
| atom | 1951 | C | ALA | 249 | 38.178 | 26.153 | 3.524 | 1.00 24.28 |
| atom | 1952 | O | ALA | 249 | 37.995 | 25.002 | 3.128 | 1.00 21.70 |
| atom | 1953 | N | ARG | 250 | 37.293 | 27.136 | 3.362 | 1.00 20.43 |
| atom | 1954 | CA | ARG | 250 | 36.021 | 26.932 | 2.700 | 1.00 23.31 |
| atom | 1955 | CB | ARG | 250 | 35.296 | 28.256 | 2.585 | 1.00 23.72 |
| atom | 1956 | CG | ARG | 250 | 34.488 | 28.629 | 3.788 | 1.00 28.13 |
| atom | 1957 | CD | ARG | 250 | 33.625 | 29.837 | 3.472 | 1.00 24.38 |
| atom | 1958 | NE | ARG | 250 | 33.181 | 30.546 | 4.674 | 1.00 31.26 |
| atom | 1959 | CZ | ARG | 250 | 32.473 | 30.001 | 5.659 | 1.00 28.67 |
| atom | 1960 | NH1 | ARG | 250 | 32.115 | 28.727 | 5.600 | 1.00 28.56 |
| atom | 1961 | NH2 | ARG | 250 | 32.115 | 30.736 | 6.702 | 1.00 34.72 |
| atom | 1962 | C | ARG | 250 | 36.224 | 26.353 | 1.308 | 1.00 23.99 |
| atom | 1963 | O | ARG | 250 | 35.535 | 25.426 | 0.898 | 1.00 24.35 |
| atom | 1964 | N | GLN | 251 | 37.178 | 26.914 | 0.580 | 1.00 29.27 |
| atom | 1965 | CA | GLN | 251 | 37.475 | 26.468 | -0.779 | 1.00 34.81 |
| atom | 1966 | CB | GLN | 251 | 38.363 | 27.506 | -1.477 | 1.00 36.88 |
| atom | 1967 | CG | GLN | 251 | 38.883 | 27.078 | -2.832 | 1.00 42.09 |
| atom | 1968 | CD | GLN | 251 | 37.819 | 27.097 | -3.916 | 1.00 45.34 |
| atom | 1969 | OE1 | GLN | 251 | 36.724 | 27.641 | -3.742 | 1.00 43.73 |
| atom | 1970 | NE2 | GLN | 251 | 38.142 | 26.494 | -5.052 | 1.00 49.17 |
| atom | 1971 | C | GLN | 251 | 38.169 | 25.103 | -0.749 | 1.00 33.48 |
| atom | 1972 | O | GLN | 251 | 38.177 | 24.362 | -1.738 | 1.00 34.28 |
| atom | 1973 | N | ALA | 252 | 38.743 | 24.782 | 0.404 | 1.00 31.45 |
| atom | 1974 | CA | ALA | 252 | 39.446 | 23.519 | 0.609 | 1.00 26.65 |
| atom | 1975 | CB | ALA | 252 | 40.392 | 23.635 | 1.821 | 1.00 26.35 |
| atom | 1976 | C | ALA | 252 | 38.437 | 22.422 | 0.856 | 1.00 23.26 |

| atom | 1977 | O   | ALA | 252 | 38.566 | 21.308 | 0.353  | 1.00 | 26.20 |
|------|------|-----|-----|-----|--------|--------|--------|------|-------|
| atom | 1978 | N   | ILE | 253 | 37.426 | 22.762 | 1.636  | 1.00 | 20.70 |
| atom | 1979 | CA  | ILE | 253 | 36.392 | 21.826 | 1.998  | 1.00 | 25.42 |
| atom | 1980 | CB  | ILE | 253 | 35.603 | 22.364 | 3.221  | 1.00 | 26.48 |
| atom | 1981 | CG2 | ILE | 253 | 34.194 | 21.777 | 3.258  | 1.00 | 25.77 |
| atom | 1982 | CG1 | ILE | 253 | 36.364 | 22.012 | 4.501  | 1.00 | 23.51 |
| atom | 1983 | CD1 | ILE | 253 | 36.576 | 23.185 | 5.419  | 1.00 | 19.45 |
| atom | 1984 | C   | ILE | 253 | 35.452 | 21.548 | 0.829  | 1.00 | 28.11 |
| atom | 1985 | O   | ILE | 253 | 34.935 | 20.433 | 0.690  | 1.00 | 28.35 |
| atom | 1986 | N   | LYS | 254 | 35.221 | 22.555 | -0.010 | 1.00 | 27.94 |
| atom | 1987 | CA  | LYS | 254 | 34.344 | 22.362 | -1.164 | 1.00 | 27.29 |
| atom | 1988 | CB  | LYS | 254 | 34.041 | 23.692 | -1.859 | 1.00 | 31.22 |
| atom | 1989 | CG  | LYS | 254 | 32.903 | 23.619 | -2.849 | 1.00 | 32.19 |
| atom | 1990 | CD  | LYS | 254 | 33.313 | 24.099 | -4.223 | 1.00 | 36.74 |
| atom | 1991 | CE  | LYS | 254 | 32.348 | 25.160 | -4.726 | 1.00 | 41.08 |
| atom | 1992 | NZ  | LYS | 254 | 31.308 | 24.585 | -5.610 | 1.00 | 43.09 |
| atom | 1993 | C   | LYS | 254 | 35.079 | 21.448 | -2.117 | 1.00 | 26.57 |
| atom | 1994 | O   | LYS | 254 | 34.535 | 20.441 | -2.600 | 1.00 | 26.31 |
| atom | 1995 | N   | SER | 255 | 36.338 | 21.794 | -2.353 | 1.00 | 23.15 |
| atom | 1996 | CA  | SER | 255 | 37.185 | 21.018 | -3.236 | 1.00 | 25.37 |
| atom | 1997 | CB  | SER | 255 | 38.569 | 21.643 | -3.327 | 1.00 | 22.33 |
| atom | 1998 | OG  | SER | 255 | 39.368 | 20.833 | -4.169 | 1.00 | 32.54 |
| atom | 1999 | C   | SER | 255 | 37.334 | 19.539 | -2.858 | 1.00 | 24.84 |
| atom | 2000 | O   | SER | 255 | 37.027 | 18.666 | -3.672 | 1.00 | 27.25 |
| atom | 2001 | N   | LEU | 256 | 37.802 | 19.243 | -1.645 | 1.00 | 17.04 |
| atom | 2002 | CA  | LEU | 256 | 37.973 | 17.840 | -1.269 | 1.00 | 15.83 |
| atom | 2003 | CB  | LEU | 256 | 38.584 | 17.739 | 0.127  | 1.00 | 18.84 |
| atom | 2004 | CG  | LEU | 256 | 40.082 | 18.046 | 0.258  | 1.00 | 16.70 |
| atom | 2005 | CD1 | LEU | 256 | 40.300 | 18.977 | 1.445  | 1.00 | 16.41 |
| atom | 2006 | CD2 | LEU | 256 | 40.855 | 16.770 | 0.457  | 1.00 | 10.37 |
| atom | 2007 | C   | LEU | 256 | 36.635 | 17.090 | -1.328 | 1.00 | 13.95 |
| atom | 2008 | O   | LEU | 256 | 36.570 | 15.906 | -1.656 | 1.00 | 14.47 |
| atom | 2009 | N   | THR | 257 | 35.552 | 17.788 | -1.023 | 1.00 | 12.60 |
| atom | 2010 | CA  | THR | 257 | 34.250 | 17.151 | -1.069 | 1.00 | 11.14 |
| atom | 2011 | CB  | THR | 257 | 33.190 | 18.104 | -0.623 | 1.00 | 8.15  |
| atom | 2012 | OG1 | THR | 257 | 33.436 | 18.441 | 0.737  | 1.00 | 16.01 |

| atom | 2013 | CG2 | THR | 257 | 31.814 | 17.490 | -0.794 | 1.00 | 2.00 |
|------|------|-----|-----|-----|--------|--------|--------|------|------|
| atom | 2014 | C | THR | 257 | 33.892 | 16.639 | -2.466 | 1.00 | 12.38 |
| atom | 2015 | O | THR | 257 | 33.528 | 15.480 | -2.603 | 1.00 | 13.28 |
| atom | 2016 | N | GLU | 258 | 33.992 | 17.501 | -3.488 | 1.00 | 15.08 |
| atom | 2017 | CA | GLU | 258 | 33.660 | 17.129 | -4.881 | 1.00 | 16.55 |
| atom | 2018 | CB | GLU | 258 | 33.467 | 18.368 | -5.777 | 1.00 | 18.49 |
| atom | 2019 | CG | GLU | 258 | 32.347 | 19.334 | -5.396 | 1.00 | 12.86 |
| atom | 2020 | CD | GLU | 258 | 30.980 | 18.902 | -5.897 | 1.00 | 19.96 |
| atom | 2021 | OE1 | GLU | 258 | 29.970 | 19.540 | -5.533 | 1.00 | 28.35 |
| atom | 2022 | OE2 | GLU | 258 | 30.892 | 17.926 | -6.658 | 1.00 | 18.70 |
| atom | 2023 | C | GLU | 258 | 34.733 | 16.270 | -5.535 | 1.00 | 16.06 |
| atom | 2024 | O | GLU | 258 | 34.445 | 15.518 | -6.463 | 1.00 | 24.28 |
| atom | 2025 | N | ARG | 259 | 35.961 | 16.355 | -5.044 | 1.00 | 13.40 |
| atom | 2026 | CA | ARG | 259 | 37.055 | 15.608 | -5.652 | 1.00 | 15.24 |
| atom | 2027 | CB | ARG | 259 | 38.331 | 16.434 | -5.616 | 1.00 | 15.45 |
| atom | 2028 | CG | ARG | 259 | 38.341 | 17.685 | -6.499 | 1.00 | 18.00 |
| atom | 2029 | CD | ARG | 259 | 39.610 | 17.646 | -7.330 | 1.00 | 14.70 |
| atom | 2030 | NE | ARG | 259 | 40.440 | 18.823 | -7.238 | 1.00 | 19.54 |
| atom | 2031 | CZ | ARG | 259 | 41.722 | 18.841 | -7.588 | 1.00 | 23.21 |
| atom | 2032 | NH1 | ARG | 259 | 42.296 | 17.739 | -8.052 | 1.00 | 18.68 |
| atom | 2033 | NH2 | ARG | 259 | 42.424 | 19.965 | -7.500 | 1.00 | 26.81 |
| atom | 2034 | C | ARG | 259 | 37.375 | 14.253 | -5.062 | 1.00 | 15.76 |
| atom | 2035 | O | ARG | 259 | 37.733 | 13.317 | -5.774 | 1.00 | 16.41 |
| atom | 2036 | N | LEU | 260 | 37.279 | 14.148 | -3.749 | 1.00 | 18.48 |
| atom | 2037 | CA | LEU | 260 | 37.620 | 12.905 | -3.104 | 1.00 | 14.79 |
| atom | 2038 | CB | LEU | 260 | 38.926 | 13.123 | -2.358 | 1.00 | 14.19 |
| atom | 2039 | CG | LEU | 260 | 39.383 | 12.280 | -1.181 | 1.00 | 13.57 |
| atom | 2040 | CD1 | LEU | 260 | 40.401 | 11.272 | -1.632 | 1.00 | 9.18 |
| atom | 2041 | CD2 | LEU | 260 | 39.978 | 13.222 | -0.155 | 1.00 | 10.22 |
| atom | 2042 | C | LEU | 260 | 36.532 | 12.315 | -2.210 | 1.00 | 13.96 |
| atom | 2043 | O | LEU | 260 | 36.221 | 11.140 | -2.337 | 1.00 | 9.01 |
| atom | 2044 | N | TYR | 261 | 35.933 | 13.120 | -1.340 | 1.00 | 12.89 |
| atom | 2045 | CA | TYR | 261 | 34.901 | 12.597 | -0.436 | 1.00 | 15.89 |
| atom | 2046 | CB | TYR | 261 | 34.425 | 13.698 | 0.526 | 1.00 | 13.43 |
| atom | 2047 | CG | TYR | 261 | 35.528 | 14.180 | 1.445 | 1.00 | 11.40 |
| atom | 2048 | CD1 | TYR | 261 | 36.600 | 13.367 | 1.761 | 1.00 | 7.86 |

| atom | 2049 | CE1 | TYR | 261 | 37.627 | 13.832 | 2.555 | 1.00 | 13.56 |
|------|------|-----|-----|-----|--------|--------|-------|------|-------|
| atom | 2050 | CD2 | TYR | 261 | 35.517 | 15.467 | 1.947 | 1.00 | 13.43 |
| atom | 2051 | CE2 | TYR | 261 | 36.531 | 15.934 | 2.727 | 1.00 | 13.28 |
| atom | 2052 | CZ | TYR | 261 | 37.583 | 15.121 | 3.032 | 1.00 | 14.20 |
| atom | 2053 | OH | TYR | 261 | 38.567 | 15.619 | 3.845 | 1.00 | 18.77 |
| atom | 2054 | C | TYR | 261 | 33.702 | 11.987 | -1.139 | 1.00 | 14.37 |
| atom | 2055 | O | TYR | 261 | 33.258 | 10.903 | -0.791 | 1.00 | 13.68 |
| atom | 2056 | N | ILE | 262 | 33.189 | 12.698 | -2.134 | 1.00 | 17.32 |
| atom | 2057 | CA | ILE | 262 | 32.035 | 12.262 | -2.906 | 1.00 | 18.38 |
| atom | 2058 | CB | ILE | 262 | 31.620 | 13.392 | -3.850 | 1.00 | 24.13 |
| atom | 2059 | CG2 | ILE | 262 | 31.733 | 12.967 | -5.299 | 1.00 | 23.73 |
| atom | 2060 | CG1 | ILE | 262 | 30.228 | 13.869 | -3.464 | 1.00 | 24.22 |
| atom | 2061 | CD1 | ILE | 262 | 30.270 | 15.040 | -2.530 | 1.00 | 22.83 |
| atom | 2062 | C | ILE | 262 | 32.279 | 10.981 | -3.698 | 1.00 | 17.72 |
| atom | 2063 | O | ILE | 262 | 31.361 | 10.208 | -3.945 | 1.00 | 23.83 |
| atom | 2064 | N | GLY | 263 | 33.522 | 10.750 | -4.089 | 1.00 | 14.02 |
| atom | 2065 | CA | GLY | 263 | 33.822 | 9.567 | -4.860 | 1.00 | 13.07 |
| atom | 2066 | C | GLY | 263 | 35.048 | 9.801 | -5.722 | 1.00 | 15.51 |
| atom | 2067 | O | GLY | 263 | 35.675 | 10.874 | -5.649 | 1.00 | 12.52 |
| atom | 2068 | N | GLY | 264 | 35.380 | 8.808 | -6.548 | 1.00 | 11.84 |
| atom | 2069 | CA | GLY | 264 | 36.543 | 8.912 | -7.408 | 1.00 | 10.59 |
| atom | 2070 | C | GLY | 264 | 37.030 | 7.554 | -7.880 | 1.00 | 15.77 |
| atom | 2071 | O | GLY | 264 | 36.468 | 6.526 | -7.501 | 1.00 | 18.26 |
| atom | 2072 | N | PRO | 265 | 38.062 | 7.522 | -8.739 | 1.00 | 16.69 |
| atom | 2073 | CD | PRO | 265 | 38.743 | 8.716 | -9.275 | 1.00 | 21.57 |
| atom | 2074 | CA | PRO | 265 | 38.642 | 6.295 | -9.283 | 1.00 | 15.40 |
| atom | 2075 | CB | PRO | 265 | 39.563 | 6.796 | -10.403 | 1.00 | 15.76 |
| atom | 2076 | CG | PRO | 265 | 39.950 | 8.134 | -10.015 | 1.00 | 15.43 |
| atom | 2077 | C | PRO | 265 | 39.414 | 5.482 | -8.253 | 1.00 | 15.52 |
| atom | 2078 | O | PRO | 265 | 40.034 | 6.044 | -7.363 | 1.00 | 11.46 |
| atom | 2079 | N | LEU | 266 | 39.404 | 4.161 | -8.425 | 1.00 | 19.24 |
| atom | 2080 | CA | LEU | 266 | 40.088 | 3.242 | -7.522 | 1.00 | 19.03 |
| atom | 2081 | CB | LEU | 266 | 39.097 | 2.180 | -7.029 | 1.00 | 19.47 |
| atom | 2082 | CG | LEU | 266 | 37.951 | 2.784 | -6.208 | 1.00 | 23.78 |
| atom | 2083 | CD1 | LEU | 266 | 36.662 | 2.020 | -6.470 | 1.00 | 16.22 |
| atom | 2084 | CD2 | LEU | 266 | 38.329 | 2.771 | -4.716 | 1.00 | 22.20 |

| atom | 2085 | C | LEU | 266 | 41.324 | 2.555 | -8.118 | 1.00 18.52 |
|------|------|------|------|------|--------|--------|--------|-----------|
| atom | 2086 | O | LEU | 266 | 41.220 | 1.656 | -8.939 | 1.00 11.92 |
| atom | 2087 | N | THR | 267 | 42.502 | 2.964 | -7.662 | 1.00 23.15 |
| atom | 2088 | CA | THR | 267 | 43.746 | 2.385 | -8.146 | 1.00 26.03 |
| atom | 2089 | CB | THR | 267 | 44.731 | 3.515 | -8.442 | 1.00 26.23 |
| atom | 2090 | OG1 | THR | 267 | 44.023 | 4.584 | -9.085 | 1.00 22.47 |
| atom | 2091 | CG2 | THR | 267 | 45.871 | 3.025 | -9.330 | 1.00 22.46 |
| atom | 2092 | C | THR | 267 | 44.341 | 1.402 | -7.111 | 1.00 28.57 |
| atom | 2093 | O | THR | 267 | 44.317 | 1.685 | -5.907 | 1.00 34.94 |
| atom | 2094 | N | ASN | 268 | 44.854 | 0.257 | -7.574 | 1.00 24.49 |
| atom | 2095 | CA | ASN | 268 | 45.447 | -0.741 | -6.682 | 1.00 25.47 |
| atom | 2096 | CB | ASN | 268 | 45.309 | -2.144 | -7.298 | 1.00 26.74 |
| atom | 2097 | CG | ASN | 268 | 46.118 | -2.317 | -8.567 | 1.00 26.59 |
| atom | 2098 | OD1 | ASN | 268 | 47.108 | -1.631 | -8.789 | 1.00 31.14 |
| atom | 2099 | ND2 | ASN | 268 | 45.697 | -3.244 | -9.403 | 1.00 23.96 |
| atom | 2100 | C | ASN | 268 | 46.916 | -0.448 | -6.321 | 1.00 24.45 |
| atom | 2101 | O | ASN | 268 | 47.415 | 0.641 | -6.576 | 1.00 25.01 |
| atom | 2102 | N | SER | 269 | 47.609 | -1.402 | -5.709 | 1.00 25.69 |
| atom | 2103 | CA | SER | 269 | 49.019 | -1.182 | -5.344 | 1.00 26.70 |
| atom | 2104 | CB | SER | 269 | 49.531 | -2.304 | -4.423 | 1.00 23.84 |
| atom | 2105 | OG | SER | 269 | 49.137 | -3.591 | -4.876 | 1.00 24.85 |
| atom | 2106 | C | SER | 269 | 49.941 | -1.080 | -6.570 | 1.00 27.41 |
| atom | 2107 | O | SER | 269 | 50.966 | -0.383 | -6.544 | 1.00 24.61 |
| atom | 2108 | N | LYS | 270 | 49.560 | -1.759 | -7.646 | 1.00 27.65 |
| atom | 2109 | CA | LYS | 270 | 50.350 | -1.756 | -8.877 | 1.00 34.24 |
| atom | 2110 | CB | LYS | 270 | 50.439 | -3.172 | -9.427 | 1.00 38.14 |
| atom | 2111 | CG | LYS | 270 | 49.904 | -4.202 | -8.453 | 1.00 41.52 |
| atom | 2112 | CD | LYS | 270 | 50.995 | -5.139 | -7.996 | 1.00 39.96 |
| atom | 2113 | CE | LYS | 270 | 50.755 | -6.511 | -8.584 | 1.00 41.32 |
| atom | 2114 | NZ | LYS | 270 | 49.453 | -6.575 | -9.308 | 1.00 43.81 |
| atom | 2115 | C | LYS | 270 | 49.826 | -0.827 | -9.965 | 1.00 32.73 |
| atom | 2116 | O | LYS | 270 | 49.728 | -1.225 | -11.122 | 1.00 32.11 |
| atom | 2117 | N | GLY | 271 | 49.483 | 0.400 | -9.569 | 1.00 32.07 |
| atom | 2118 | CA | GLY | 271 | 48.991 | 1.414 | -10.486 | 1.00 27.34 |
| atom | 2119 | C | GLY | 271 | 47.811 | 1.148 | -11.414 | 1.00 26.74 |
| atom | 2120 | O | GLY | 271 | 47.401 | 2.070 | -12.131 | 1.00 29.62 |

| atom | 2121 | N   | GLN | 272 | 47.266 | -0.065 | -11.434 | 1.00 | 16.78 |
|------|------|-----|-----|-----|--------|--------|---------|------|-------|
| atom | 2122 | CA  | GLN | 272 | 46.130 | -0.367 | -12.298 | 1.00 | 22.50 |
| atom | 2123 | CB  | GLN | 272 | 46.006 | -1.879 | -12.461 | 1.00 | 25.20 |
| atom | 2124 | CG  | GLN | 272 | 45.118 | -2.350 | -13.607 | 1.00 | 28.05 |
| atom | 2125 | CD  | GLN | 272 | 45.018 | -3.871 | -13.633 | 1.00 | 32.35 |
| atom | 2126 | OE1 | GLN | 272 | 44.301 | -4.474 | -14.450 | 1.00 | 33.79 |
| atom | 2127 | NE2 | GLN | 272 | 45.741 | -4.500 | -12.724 | 1.00 | 31.21 |
| atom | 2128 | C   | GLN | 272 | 44.817 | 0.220  | -11.742 | 1.00 | 27.23 |
| atom | 2129 | O   | GLN | 272 | 44.737 | 0.578  | -10.560 | 1.00 | 29.76 |
| atom | 2130 | N   | ASN | 273 | 43.786 | 0.316  | -12.586 | 1.00 | 26.20 |
| atom | 2131 | CA  | ASN | 273 | 42.499 | 0.884  | -12.162 | 1.00 | 23.35 |
| atom | 2132 | CB  | ASN | 273 | 42.012 | 1.888  | -13.211 | 1.00 | 30.32 |
| atom | 2133 | CG  | ASN | 273 | 40.898 | 2.782  | -12.692 | 1.00 | 38.52 |
| atom | 2134 | OD1 | ASN | 273 | 39.724 | 2.603  | -13.043 | 1.00 | 38.61 |
| atom | 2135 | ND2 | ASN | 273 | 41.258 | 3.748  | -11.842 | 1.00 | 35.47 |
| atom | 2136 | C   | ASN | 273 | 41.450 | -0.196 | -11.913 | 1.00 | 23.09 |
| atom | 2137 | O   | ASN | 273 | 41.161 | -1.012 | -12.788 | 1.00 | 25.78 |
| atom | 2138 | N   | CYS | 274 | 40.859 | -0.186 | -10.724 | 1.00 | 23.19 |
| atom | 2139 | CA  | CYS | 274 | 39.897 | -1.224 | -10.330 | 1.00 | 25.74 |
| atom | 2140 | CB  | CYS | 274 | 40.167 | -1.645 | -8.878  | 1.00 | 24.20 |
| atom | 2141 | SG  | CYS | 274 | 41.736 | -2.465 | -8.623  | 1.00 | 32.90 |
| atom | 2142 | C   | CYS | 274 | 38.408 | -0.941 | -10.453 | 1.00 | 26.08 |
| atom | 2143 | O   | CYS | 274 | 37.586 | -1.876 | -10.466 | 1.00 | 28.31 |
| atom | 2144 | N   | GLY | 275 | 38.050 | 0.335  | -10.517 | 1.00 | 23.19 |
| atom | 2145 | CA  | GLY | 275 | 36.646 | 0.672  | -10.599 | 1.00 | 16.16 |
| atom | 2146 | C   | GLY | 275 | 36.427 | 2.072  | -10.091 | 1.00 | 17.69 |
| atom | 2147 | O   | GLY | 275 | 37.376 | 2.835  | -9.931  | 1.00 | 19.74 |
| atom | 2148 | N   | TYR | 276 | 35.173 | 2.416  | -9.835  | 1.00 | 19.21 |
| atom | 2149 | CA  | TYR | 276 | 34.836 | 3.739  | -9.355  | 1.00 | 16.79 |
| atom | 2150 | CB  | TYR | 276 | 34.070 | 4.493  | -10.431 | 1.00 | 17.22 |
| atom | 2151 | CG  | TYR | 276 | 34.243 | 5.978  | -10.351 | 1.00 | 19.00 |
| atom | 2152 | CD1 | TYR | 276 | 35.434 | 6.571  | -10.743 | 1.00 | 22.14 |
| atom | 2153 | CE1 | TYR | 276 | 35.615 | 7.941  | -10.648 | 1.00 | 19.36 |
| atom | 2154 | CD2 | TYR | 276 | 33.226 | 6.798  | -9.852  | 1.00 | 14.77 |
| atom | 2155 | CE2 | TYR | 276 | 33.397 | 8.170  | -9.754  | 1.00 | 10.07 |
| atom | 2156 | CZ  | TYR | 276 | 34.597 | 8.732  | -10.155 | 1.00 | 16.49 |

| atom | 2157 | OH  | TYR | 276 | 34.787 | 10.097 | -10.115 | 1.00 | 22.80 |
|------|------|-----|-----|-----|--------|--------|---------|------|-------|
| atom | 2158 | C   | TYR | 276 | 34.011 | 3.693  | -8.076  | 1.00 | 22.07 |
| atom | 2159 | O   | TYR | 276 | 33.140 | 2.816  | -7.904  | 1.00 | 16.49 |
| atom | 2160 | N   | ARG | 277 | 34.286 | 4.661  | -7.193  | 1.00 | 22.20 |
| atom | 2161 | CA  | ARG | 277 | 33.605 | 4.779  | -5.896  | 1.00 | 19.05 |
| atom | 2162 | CB  | ARG | 277 | 34.646 | 4.965  | -4.796  | 1.00 | 16.38 |
| atom | 2163 | CG  | ARG | 277 | 34.065 | 5.125  | -3.422  | 1.00 | 13.13 |
| atom | 2164 | CD  | ARG | 277 | 35.184 | 5.349  | -2.433  | 1.00 | 12.73 |
| atom | 2165 | NE  | ARG | 277 | 35.316 | 6.761  | -2.171  | 1.00 | 10.46 |
| atom | 2166 | CZ  | ARG | 277 | 36.252 | 7.522  | -2.699  | 1.00 | 13.10 |
| atom | 2167 | NH1 | ARG | 277 | 37.155 | 6.999  | -3.524  | 1.00 | 19.62 |
| atom | 2168 | NH2 | ARG | 277 | 36.262 | 8.811  | -2.428  | 1.00 | 11.60 |
| atom | 2169 | C   | ARG | 277 | 32.587 | 5.927  | -5.812  | 1.00 | 16.53 |
| atom | 2170 | O   | ARG | 277 | 32.871 | 7.046  | -6.231  | 1.00 | 13.19 |
| atom | 2171 | N   | ARG | 278 | 31.412 | 5.643  | -5.259  | 1.00 | 11.39 |
| atom | 2172 | CA  | ARG | 278 | 30.382 | 6.666  | -5.109  | 1.00 | 17.01 |
| atom | 2173 | CB  | ARG | 278 | 29.261 | 6.436  | -6.126  | 1.00 | 15.88 |
| atom | 2174 | CG  | ARG | 278 | 29.617 | 6.850  | -7.545  | 1.00 | 25.88 |
| atom | 2175 | CD  | ARG | 278 | 28.637 | 6.259  | -8.557  | 1.00 | 31.56 |
| atom | 2176 | NE  | ARG | 278 | 28.891 | 6.737  | -9.914  | 1.00 | 38.90 |
| atom | 2177 | CZ  | ARG | 278 | 29.297 | 5.959  | -10.918 | 1.00 | 46.20 |
| atom | 2178 | NH1 | ARG | 278 | 29.498 | 4.657  | -10.721 | 1.00 | 40.83 |
| atom | 2179 | NH2 | ARG | 278 | 29.501 | 6.485  | -12.124 | 1.00 | 44.92 |
| atom | 2180 | C   | ARG | 278 | 29.802 | 6.709  | -3.678  | 1.00 | 18.33 |
| atom | 2181 | O   | ARG | 278 | 28.791 | 7.363  | -3.413  | 1.00 | 14.86 |
| atom | 2182 | N   | CYS | 279 | 30.443 | 5.988  | -2.763  | 1.00 | 18.74 |
| atom | 2183 | CA  | CYS | 279 | 30.013 | 5.951  | -1.372  | 1.00 | 20.34 |
| atom | 2184 | CB  | CYS | 279 | 29.734 | 4.521  | -0.910  | 1.00 | 13.54 |
| atom | 2185 | SG  | CYS | 279 | 31.032 | 3.386  | -1.400  | 1.00 | 11.81 |
| atom | 2186 | C   | CYS | 279 | 31.158 | 6.530  | -0.580  | 1.00 | 19.14 |
| atom | 2187 | O   | CYS | 279 | 31.995 | 7.229  | -1.131  | 1.00 | 18.85 |
| atom | 2188 | N   | ARG | 280 | 31.204 | 6.230  | 0.708   | 1.00 | 20.55 |
| atom | 2189 | CA  | ARG | 280 | 32.266 | 6.765  | 1.551   | 1.00 | 19.72 |
| atom | 2190 | CB  | ARG | 280 | 31.760 | 6.890  | 2.994   | 1.00 | 17.16 |
| atom | 2191 | CG  | ARG | 280 | 32.765 | 6.504  | 4.033   | 1.00 | 18.71 |
| atom | 2192 | CD  | ARG | 280 | 32.721 | 7.441  | 5.217   | 1.00 | 21.86 |

| atom | 2193 | NE | ARG | 280 | 32.610 | 8.844 | 4.846 | 1.00 | 12.51 |
|------|------|-----|-----|-----|--------|--------|-------|------|-------|
| atom | 2194 | CZ | ARG | 280 | 31.542 | 9.602 | 5.089 | 1.00 | 16.04 |
| atom | 2195 | NH1 | ARG | 280 | 30.483 | 9.096 | 5.697 | 1.00 | 10.02 |
| atom | 2196 | NH2 | ARG | 280 | 31.543 | 10.884 | 4.738 | 1.00 | 16.85 |
| atom | 2197 | C | ARG | 280 | 33.534 | 5.924 | 1.507 | 1.00 | 17.65 |
| atom | 2198 | O | ARG | 280 | 33.477 | 4.698 | 1.508 | 1.00 | 16.85 |
| atom | 2199 | N | ALA | 281 | 34.673 | 6.602 | 1.461 | 1.00 | 19.88 |
| atom | 2200 | CA | ALA | 281 | 35.978 | 5.943 | 1.465 | 1.00 | 20.82 |
| atom | 2201 | CB | ALA | 281 | 37.025 | 6.809 | 0.748 | 1.00 | 17.70 |
| atom | 2202 | C | ALA | 281 | 36.359 | 5.788 | 2.933 | 1.00 | 20.37 |
| atom | 2203 | O | ALA | 281 | 36.121 | 6.686 | 3.730 | 1.00 | 17.90 |
| atom | 2204 | N | SER | 282 | 36.964 | 4.658 | 3.275 | 1.00 | 23.63 |
| atom | 2205 | CA | SER | 282 | 37.369 | 4.377 | 4.643 | 1.00 | 24.68 |
| atom | 2206 | CB | SER | 282 | 37.569 | 2.883 | 4.808 | 1.00 | 28.10 |
| atom | 2207 | OG | SER | 282 | 38.795 | 2.513 | 4.200 | 1.00 | 34.10 |
| atom | 2208 | C | SER | 282 | 38.640 | 5.071 | 5.121 | 1.00 | 22.98 |
| atom | 2209 | O | SER | 282 | 38.854 | 5.217 | 6.322 | 1.00 | 24.18 |
| atom | 2210 | N | GLY | 283 | 39.491 | 5.492 | 4.200 | 1.00 | 22.59 |
| atom | 2211 | CA | GLY | 283 | 40.740 | 6.108 | 4.625 | 1.00 | 15.51 |
| atom | 2212 | C | GLY | 283 | 40.877 | 7.601 | 4.545 | 1.00 | 14.39 |
| atom | 2213 | O | GLY | 283 | 41.999 | 8.113 | 4.428 | 1.00 | 19.94 |
| atom | 2214 | N | VAL | 284 | 39.763 | 8.316 | 4.625 | 1.00 | 12.87 |
| atom | 2215 | CA | VAL | 284 | 39.817 | 9.774 | 4.547 | 1.00 | 16.46 |
| atom | 2216 | CB | VAL | 284 | 38.707 | 10.334 | 3.625 | 1.00 | 18.23 |
| atom | 2217 | CG1 | VAL | 284 | 39.306 | 10.638 | 2.256 | 1.00 | 15.09 |
| atom | 2218 | CG2 | VAL | 284 | 37.548 | 9.344 | 3.526 | 1.00 | 15.13 |
| atom | 2219 | C | VAL | 284 | 39.698 | 10.370 | 5.935 | 1.00 | 16.16 |
| atom | 2220 | O | VAL | 284 | 39.365 | 9.654 | 6.873 | 1.00 | 21.25 |
| atom | 2221 | N | LEU | 285 | 39.976 | 11.662 | 6.085 | 1.00 | 14.54 |
| atom | 2222 | CA | LEU | 285 | 39.925 | 12.283 | 7.419 | 1.00 | 18.17 |
| atom | 2223 | CB | LEU | 285 | 40.591 | 13.671 | 7.397 | 1.00 | 11.77 |
| atom | 2224 | CG | LEU | 285 | 40.737 | 14.212 | 8.834 | 1.00 | 14.87 |
| atom | 2225 | CD1 | LEU | 285 | 41.712 | 13.349 | 9.575 | 1.00 | 11.35 |
| atom | 2226 | CD2 | LEU | 285 | 41.217 | 15.638 | 8.873 | 1.00 | 11.98 |
| atom | 2227 | C | LEU | 285 | 38.514 | 12.438 | 8.003 | 1.00 | 18.78 |
| atom | 2228 | O | LEU | 285 | 38.289 | 12.278 | 9.209 | 1.00 | 19.14 |

| atom | 2229 | N | THR | 286 | 37.579 | 12.769 | 7.122 | 1.00 | 16.92 |
|------|------|------|------|------|--------|--------|--------|------|-------|
| atom | 2230 | CA | THR | 286 | 36.196 | 13.021 | 7.463 | 1.00 | 8.41 |
| atom | 2231 | CB | THR | 286 | 35.523 | 13.887 | 6.360 | 1.00 | 11.85 |
| atom | 2232 | OG1 | THR | 286 | 35.864 | 13.329 | 5.089 | 1.00 | 11.25 |
| atom | 2233 | CG2 | THR | 286 | 35.980 | 15.366 | 6.408 | 1.00 | 3.47 |
| atom | 2234 | C | THR | 286 | 35.373 | 11.752 | 7.600 | 1.00 | 8.58 |
| atom | 2235 | O | THR | 286 | 34.179 | 11.830 | 7.885 | 1.00 | 6.30 |
| atom | 2236 | N | THR | 287 | 35.974 | 10.578 | 7.437 | 1.00 | 9.98 |
| atom | 2237 | CA | THR | 287 | 35.132 | 9.384 | 7.502 | 1.00 | 14.51 |
| atom | 2238 | CB | THR | 287 | 35.869 | 8.126 | 6.964 | 1.00 | 11.97 |
| atom | 2239 | OG1 | THR | 287 | 35.300 | 6.948 | 7.537 | 1.00 | 16.98 |
| atom | 2240 | CG2 | THR | 287 | 37.317 | 8.174 | 7.251 | 1.00 | 16.02 |
| atom | 2241 | C | THR | 287 | 34.472 | 9.109 | 8.858 | 1.00 | 14.94 |
| atom | 2242 | O | THR | 287 | 33.288 | 8.733 | 8.922 | 1.00 | 17.04 |
| atom | 2243 | N | SER | 288 | 35.214 | 9.323 | 9.937 | 1.00 | 15.88 |
| atom | 2244 | CA | SER | 288 | 34.672 | 9.123 | 11.291 | 1.00 | 15.93 |
| atom | 2245 | CB | SER | 288 | 35.797 | 9.242 | 12.319 | 1.00 | 14.91 |
| atom | 2246 | OG | SER | 288 | 35.262 | 9.346 | 13.595 | 1.00 | 17.41 |
| atom | 2247 | C | SER | 288 | 33.593 | 10.172 | 11.602 | 1.00 | 13.04 |
| atom | 2248 | O | SER | 288 | 32.439 | 9.844 | 11.871 | 1.00 | 12.73 |
| atom | 2249 | N | CYS | 289 | 33.983 | 11.438 | 11.559 | 1.00 | 7.29 |
| atom | 2250 | CA | CYS | 289 | 33.046 | 12.511 | 11.836 | 1.00 | 10.66 |
| atom | 2251 | CB | CYS | 289 | 33.793 | 13.851 | 11.834 | 1.00 | 5.96 |
| atom | 2252 | SG | CYS | 289 | 32.783 | 15.279 | 11.493 | 1.00 | 22.59 |
| atom | 2253 | C | CYS | 289 | 31.863 | 12.527 | 10.846 | 1.00 | 12.55 |
| atom | 2254 | O | CYS | 289 | 30.734 | 12.875 | 11.212 | 1.00 | 14.62 |
| atom | 2255 | N | GLY | 290 | 32.127 | 12.168 | 9.591 | 1.00 | 14.52 |
| atom | 2256 | CA | GLY | 290 | 31.074 | 12.120 | 8.590 | 1.00 | 9.51 |
| atom | 2257 | C | GLY | 290 | 30.057 | 11.040 | 8.936 | 1.00 | 14.36 |
| atom | 2258 | O | GLY | 290 | 28.852 | 11.313 | 9.001 | 1.00 | 13.85 |
| atom | 2259 | N | ASN | 291 | 30.524 | 9.813 | 9.168 | 1.00 | 10.21 |
| atom | 2260 | CA | ASN | 291 | 29.611 | 8.721 | 9.508 | 1.00 | 13.14 |
| atom | 2261 | CB | ASN | 291 | 30.372 | 7.399 | 9.663 | 1.00 | 12.39 |
| atom | 2262 | CG | ASN | 291 | 30.742 | 6.787 | 8.343 | 1.00 | 16.70 |
| atom | 2263 | OD1 | ASN | 291 | 30.444 | 7.334 | 7.291 | 1.00 | 19.76 |
| atom | 2264 | ND2 | ASN | 291 | 31.398 | 5.645 | 8.388 | 1.00 | 14.60 |

| atom | 2265 | C   | ASN | 291 | 28.840 | 9.007  | 10.803 | 1.00 | 13.64 |
|------|------|-----|-----|-----|--------|--------|--------|------|-------|
| atom | 2266 | O   | ASN | 291 | 27.672 | 8.669  | 10.912 | 1.00 | 16.48 |
| atom | 2267 | N   | THR | 292 | 29.498 | 9.615  | 11.787 | 1.00 | 13.64 |
| atom | 2268 | CA  | THR | 292 | 28.829 | 9.926  | 13.033 | 1.00 | 11.88 |
| atom | 2269 | CB  | THR | 292 | 29.814 | 10.420 | 14.092 | 1.00 | 11.39 |
| atom | 2270 | OG1 | THR | 292 | 30.878 | 9.460  | 14.247 | 1.00 | 9.50  |
| atom | 2271 | CG2 | THR | 292 | 29.099 | 10.572 | 15.406 | 1.00 | 2.48  |
| atom | 2272 | C   | THR | 292 | 27.731 | 10.956 | 12.834 | 1.00 | 12.72 |
| atom | 2273 | O   | THR | 292 | 26.619 | 10.785 | 13.354 | 1.00 | 9.10  |
| atom | 2274 | N   | LEU | 293 | 28.030 | 11.997 | 12.060 | 1.00 | 7.16  |
| atom | 2275 | CA  | LEU | 293 | 27.045 | 13.036 | 11.767 | 1.00 | 10.97 |
| atom | 2276 | CB  | LEU | 293 | 27.704 | 14.195 | 11.018 | 1.00 | 8.15  |
| atom | 2277 | CG  | LEU | 293 | 28.403 | 15.187 | 11.956 | 1.00 | 18.61 |
| atom | 2278 | CD1 | LEU | 293 | 29.564 | 15.857 | 11.252 | 1.00 | 14.67 |
| atom | 2279 | CD2 | LEU | 293 | 27.400 | 16.228 | 12.448 | 1.00 | 18.24 |
| atom | 2280 | C   | LEU | 293 | 25.879 | 12.498 | 10.927 | 1.00 | 11.98 |
| atom | 2281 | O   | LEU | 293 | 24.693 | 12.837 | 11.143 | 1.00 | 10.31 |
| atom | 2282 | N   | THR | 294 | 26.218 | 11.644 | 9.973  | 1.00 | 9.34  |
| atom | 2283 | CA  | THR | 294 | 25.218 | 11.088 | 9.078  | 1.00 | 13.14 |
| atom | 2284 | CB  | THR | 294 | 25.932 | 10.501 | 7.830  | 1.00 | 17.76 |
| atom | 2285 | OG1 | THR | 294 | 26.584 | 11.576 | 7.136  | 1.00 | 9.54  |
| atom | 2286 | CG2 | THR | 294 | 24.952 | 9.770  | 6.888  | 1.00 | 13.10 |
| atom | 2287 | C   | THR | 294 | 24.325 | 10.051 | 9.775  | 1.00 | 13.78 |
| atom | 2288 | O   | THR | 294 | 23.103 | 10.068 | 9.612  | 1.00 | 12.50 |
| atom | 2289 | N   | CYS | 295 | 24.927 | 9.183  | 10.586 | 1.00 | 12.96 |
| atom | 2290 | CA  | CYS | 295 | 24.158 | 8.164  | 11.297 | 1.00 | 15.53 |
| atom | 2291 | CB  | CYS | 295 | 25.083 | 7.216  | 12.054 | 1.00 | 14.80 |
| atom | 2292 | SG  | CYS | 295 | 24.228 | 5.909  | 12.939 | 1.00 | 21.83 |
| atom | 2293 | C   | CYS | 295 | 23.237 | 8.852  | 12.279 | 1.00 | 15.64 |
| atom | 2294 | O   | CYS | 295 | 22.123 | 8.424  | 12.506 | 1.00 | 19.96 |
| atom | 2295 | N   | TYR | 296 | 23.714 | 9.945  | 12.846 | 1.00 | 16.53 |
| atom | 2296 | CA  | TYR | 296 | 22.944 | 10.688 | 13.808 | 1.00 | 13.09 |
| atom | 2297 | CB  | TYR | 296 | 23.831 | 11.751 | 14.460 | 1.00 | 19.53 |
| atom | 2298 | CG  | TYR | 296 | 23.101 | 12.750 | 15.335 | 1.00 | 23.83 |
| atom | 2299 | CD1 | TYR | 296 | 22.977 | 12.549 | 16.705 | 1.00 | 20.52 |
| atom | 2300 | CE1 | TYR | 296 | 22.251 | 13.432 | 17.495 | 1.00 | 23.16 |

| | | | | | | | | | |
|---|---|---|---|---|---|---|---|---|---|
| atom | 2301 | CD2 | TYR | 296 | 22.485 | 13.865 | 14.774 | 1.00 | 22.40 |
| atom | 2302 | CE2 | TYR | 296 | 21.763 | 14.742 | 15.543 | 1.00 | 25.16 |
| atom | 2303 | CZ | TYR | 296 | 21.639 | 14.525 | 16.901 | 1.00 | 26.73 |
| atom | 2304 | OH | TYR | 296 | 20.878 | 15.398 | 17.644 | 1.00 | 29.21 |
| atom | 2305 | C | TYR | 296 | 21.727 | 11.326 | 13.175 | 1.00 | 13.13 |
| atom | 2306 | O | TYR | 296 | 20.627 | 11.241 | 13.705 | 1.00 | 19.26 |
| atom | 2307 | N | LEU | 297 | 21.930 | 11.965 | 12.037 | 1.00 | 15.33 |
| atom | 2308 | CA | LEU | 297 | 20.861 | 12.651 | 11.329 | 1.00 | 8.06 |
| atom | 2309 | CB | LEU | 297 | 21.478 | 13.414 | 10.167 | 1.00 | 4.00 |
| atom | 2310 | CG | LEU | 297 | 20.657 | 13.832 | 8.973 | 1.00 | 5.36 |
| atom | 2311 | CD1 | LEU | 297 | 19.567 | 14.751 | 9.449 | 1.00 | 2.30 |
| atom | 2312 | CD2 | LEU | 297 | 21.600 | 14.508 | 7.935 | 1.00 | 2.00 |
| atom | 2313 | C | LEU | 297 | 19.795 | 11.679 | 10.854 | 1.00 | 10.56 |
| atom | 2314 | O | LEU | 297 | 18.602 | 11.927 | 10.989 | 1.00 | 8.08 |
| atom | 2315 | N | LYS | 298 | 20.224 | 10.554 | 10.306 | 1.00 | 9.28 |
| atom | 2316 | CA | LYS | 298 | 19.259 | 9.584 | 9.843 | 1.00 | 14.54 |
| atom | 2317 | CB | LYS | 298 | 19.959 | 8.524 | 8.975 | 1.00 | 14.39 |
| atom | 2318 | CG | LYS | 298 | 20.284 | 9.041 | 7.564 | 1.00 | 14.80 |
| atom | 2319 | CD | LYS | 298 | 21.047 | 8.018 | 6.741 | 1.00 | 9.15 |
| atom | 2320 | CE | LYS | 298 | 21.993 | 8.691 | 5.772 | 1.00 | 6.39 |
| atom | 2321 | NZ | LYS | 298 | 22.398 | 7.721 | 4.710 | 1.00 | 12.57 |
| atom | 2322 | C | LYS | 298 | 18.526 | 8.926 | 11.022 | 1.00 | 17.93 |
| atom | 2323 | O | LYS | 298 | 17.302 | 8.706 | 10.976 | 1.00 | 14.55 |
| atom | 2324 | N | ALA | 299 | 19.275 | 8.622 | 12.081 | 1.00 | 13.25 |
| atom | 2325 | CA | ALA | 299 | 18.682 | 7.971 | 13.224 | 1.00 | 15.80 |
| atom | 2326 | CB | ALA | 299 | 19.760 | 7.440 | 14.140 | 1.00 | 11.84 |
| atom | 2327 | C | ALA | 299 | 17.746 | 8.923 | 13.968 | 1.00 | 18.33 |
| atom | 2328 | O | ALA | 299 | 16.701 | 8.507 | 14.476 | 1.00 | 17.79 |
| atom | 2329 | N | SER | 300 | 18.098 | 10.203 | 14.005 | 1.00 | 18.37 |
| atom | 2330 | CA | SER | 300 | 17.250 | 11.173 | 14.693 | 1.00 | 19.72 |
| atom | 2331 | CB | SER | 300 | 17.917 | 12.537 | 14.736 | 1.00 | 15.36 |
| atom | 2332 | OG | SER | 300 | 18.855 | 12.587 | 15.777 | 1.00 | 20.47 |
| atom | 2333 | C | SER | 300 | 15.917 | 11.296 | 13.973 | 1.00 | 17.03 |
| atom | 2334 | O | SER | 300 | 14.853 | 11.152 | 14.579 | 1.00 | 15.98 |
| atom | 2335 | N | ALA | 301 | 15.997 | 11.565 | 12.673 | 1.00 | 16.11 |
| atom | 2336 | CA | ALA | 301 | 14.818 | 11.703 | 11.831 | 1.00 | 12.11 |

| | | | | | | | | | |
|---|---|---|---|---|---|---|---|---|---|
| atom | 2337 | CB | ALA | 301 | 15.234 | 12.062 | 10.399 | 1.00 | 14.67 |
| atom | 2338 | C | ALA | 301 | 14.048 | 10.394 | 11.859 | 1.00 | 5.18 |
| atom | 2339 | O | ALA | 301 | 12.829 | 10.375 | 11.892 | 1.00 | 7.90 |
| atom | 2340 | N | ALA | 302 | 14.762 | 9.285 | 11.869 | 1.00 | 8.95 |
| atom | 2341 | CA | ALA | 302 | 14.083 | 7.997 | 11.923 | 1.00 | 10.58 |
| atom | 2342 | CB | ALA | 302 | 15.068 | 6.887 | 11.656 | 1.00 | 2.00 |
| atom | 2343 | C | ALA | 302 | 13.351 | 7.764 | 13.276 | 1.00 | 14.11 |
| atom | 2344 | O | ALA | 302 | 12.315 | 7.102 | 13.310 | 1.00 | 9.62 |
| atom | 2345 | N | CYS | 303 | 13.885 | 8.308 | 14.373 | 1.00 | 18.06 |
| atom | 2346 | CA | CYS | 303 | 13.263 | 8.163 | 15.705 | 1.00 | 23.37 |
| atom | 2347 | CB | CYS | 303 | 14.190 | 8.725 | 16.803 | 1.00 | 21.74 |
| atom | 2348 | SG | CYS | 303 | 15.531 | 7.631 | 17.351 | 1.00 | 27.25 |
| atom | 2349 | C | CYS | 303 | 11.917 | 8.925 | 15.742 | 1.00 | 26.18 |
| atom | 2350 | O | CYS | 303 | 10.903 | 8.446 | 16.285 | 1.00 | 23.74 |
| atom | 2351 | N | ARG | 304 | 11.929 | 10.122 | 15.161 | 1.00 | 22.67 |
| atom | 2352 | CA | ARG | 304 | 10.747 | 10.940 | 15.097 | 1.00 | 22.19 |
| atom | 2353 | CB | ARG | 304 | 11.099 | 12.306 | 14.503 | 1.00 | 25.32 |
| atom | 2354 | CG | ARG | 304 | 12.197 | 13.063 | 15.254 | 1.00 | 16.91 |
| atom | 2355 | CD | ARG | 304 | 12.192 | 14.548 | 14.889 | 1.00 | 15.19 |
| atom | 2356 | NE | ARG | 304 | 13.299 | 15.295 | 15.499 | 1.00 | 19.62 |
| atom | 2357 | CZ | ARG | 304 | 13.467 | 16.616 | 15.392 | 1.00 | 22.51 |
| atom | 2358 | NH1 | ARG | 304 | 12.595 | 17.339 | 14.690 | 1.00 | 23.10 |
| atom | 2359 | NH2 | ARG | 304 | 14.483 | 17.228 | 16.012 | 1.00 | 11.90 |
| atom | 2360 | C | ARG | 304 | 9.677 | 10.226 | 14.256 | 1.00 | 26.83 |
| atom | 2361 | O | ARG | 304 | 8.476 | 10.394 | 14.489 | 1.00 | 25.97 |
| atom | 2362 | N | ALA | 305 | 10.106 | 9.419 | 13.290 | 1.00 | 30.84 |
| atom | 2363 | CA | ALA | 305 | 9.158 | 8.683 | 12.449 | 1.00 | 34.55 |
| atom | 2364 | CB | ALA | 305 | 9.898 | 7.809 | 11.444 | 1.00 | 35.65 |
| atom | 2365 | C | ALA | 305 | 8.225 | 7.821 | 13.299 | 1.00 | 37.13 |
| atom | 2366 | O | ALA | 305 | 7.190 | 7.358 | 12.815 | 1.00 | 37.73 |
| atom | 2367 | N | ALA | 306 | 8.592 | 7.610 | 14.562 | 1.00 | 38.22 |
| atom | 2368 | CA | ALA | 306 | 7.762 | 6.824 | 15.474 | 1.00 | 36.67 |
| atom | 2369 | CB | ALA | 306 | 8.310 | 5.422 | 15.583 | 1.00 | 35.11 |
| atom | 2370 | C | ALA | 306 | 7.692 | 7.468 | 16.860 | 1.00 | 38.80 |
| atom | 2371 | O | ALA | 306 | 6.844 | 8.320 | 17.132 | 1.00 | 38.28 |
| atom | 2372 | N | LYS | 307 | 8.608 | 7.040 | 17.720 | 1.00 | 41.45 |

| atom | 2373 | CA | LYS | 307 | 8.740 | 7.491 | 19.096 | 1.00 | 42.81 |
|------|------|----|-----|-----|-------|-------|--------|------|-------|
| atom | 2374 | CB | LYS | 307 | 10.214 | 7.458 | 19.503 | 1.00 | 40.40 |
| atom | 2375 | CG | LYS | 307 | 10.729 | 6.087 | 19.916 | 1.00 | 37.86 |
| atom | 2376 | CD | LYS | 307 | 10.575 | 5.048 | 18.831 | 1.00 | 37.54 |
| atom | 2377 | CE | LYS | 307 | 11.918 | 4.432 | 18.481 | 1.00 | 37.85 |
| atom | 2378 | NZ | LYS | 307 | 12.924 | 5.507 | 18.258 | 1.00 | 35.80 |
| atom | 2379 | C | LYS | 307 | 8.167 | 8.865 | 19.449 | 1.00 | 49.60 |
| atom | 2380 | O | LYS | 307 | 8.788 | 9.907 | 19.188 | 1.00 | 46.97 |
| atom | 2381 | N | LEU | 308 | 6.982 | 8.842 | 20.066 | 1.00 | 51.53 |
| atom | 2382 | CA | LEU | 308 | 6.279 | 10.039 | 20.524 | 1.00 | 51.53 |
| atom | 2383 | CB | LEU | 308 | 4.758 | 9.842 | 20.389 | 1.00 | 52.02 |
| atom | 2384 | CG | LEU | 308 | 4.157 | 8.437 | 20.605 | 1.00 | 51.76 |
| atom | 2385 | CD1 | LEU | 308 | 3.708 | 8.288 | 22.068 | 1.00 | 47.91 |
| atom | 2386 | CD2 | LEU | 308 | 2.983 | 8.206 | 19.643 | 1.00 | 43.18 |
| atom | 2387 | C | LEU | 308 | 6.663 | 10.183 | 21.998 | 1.00 | 54.77 |
| atom | 2388 | O | LEU | 308 | 5.997 | 10.874 | 22.786 | 1.00 | 57.44 |
| atom | 2389 | N | GLN | 309 | 7.766 | 9.517 | 22.338 | 1.00 | 56.72 |
| atom | 2390 | CA | GLN | 309 | 8.312 | 9.459 | 23.693 | 1.00 | 53.19 |
| atom | 2391 | CB | GLN | 309 | 8.499 | 8.002 | 24.077 | 1.00 | 52.08 |
| atom | 2392 | CG | GLN | 309 | 9.172 | 7.229 | 22.970 | 1.00 | 47.86 |
| atom | 2393 | CD | GLN | 309 | 8.527 | 5.897 | 22.742 | 1.00 | 48.89 |
| atom | 2394 | OE1 | GLN | 309 | 8.830 | 5.208 | 21.775 | 1.00 | 51.59 |
| atom | 2395 | NE2 | GLN | 309 | 7.627 | 5.513 | 23.642 | 1.00 | 55.53 |
| atom | 2396 | C | GLN | 309 | 9.645 | 10.190 | 23.888 | 1.00 | 52.39 |
| atom | 2397 | O | GLN | 309 | 9.840 | 11.303 | 23.383 | 1.00 | 53.77 |
| atom | 2398 | N | ASP | 310 | 10.573 | 9.545 | 24.599 | 1.00 | 46.73 |
| atom | 2399 | CA | ASP | 310 | 11.850 | 10.182 | 24.914 | 1.00 | 46.17 |
| atom | 2400 | CB | ASP | 310 | 11.877 | 10.525 | 26.404 | 1.00 | 50.62 |
| atom | 2401 | CG | ASP | 310 | 10.471 | 10.620 | 27.000 | 1.00 | 54.61 |
| atom | 2402 | OD1 | ASP | 310 | 9.975 | 9.590 | 27.507 | 1.00 | 57.26 |
| atom | 2403 | OD2 | ASP | 310 | 9.857 | 11.716 | 26.954 | 1.00 | 52.46 |
| atom | 2404 | C | ASP | 310 | 13.081 | 9.382 | 24.538 | 1.00 | 43.56 |
| atom | 2405 | O | ASP | 310 | 13.580 | 8.537 | 25.297 | 1.00 | 34.86 |
| atom | 2406 | N | CYS | 311 | 13.571 | 9.683 | 23.342 | 1.00 | 41.82 |
| atom | 2407 | CA | CYS | 311 | 14.726 | 9.016 | 22.783 | 1.00 | 36.02 |
| atom | 2408 | CB | CYS | 311 | 14.703 | 9.126 | 21.269 | 1.00 | 35.10 |

| atom | 2409 | SG | CYS | 311 | 13.643 | 7.931 | 20.531 | 1.00 | 41.50 |
| atom | 2410 | C | CYS | 311 | 16.001 | 9.612 | 23.292 | 1.00 | 32.40 |
| atom | 2411 | O | CYS | 311 | 16.050 | 10.780 | 23.655 | 1.00 | 32.96 |
| atom | 2412 | N | THR | 312 | 17.032 | 8.783 | 23.335 | 1.00 | 29.68 |
| atom | 2413 | CA | THR | 312 | 18.348 | 9.221 | 23.757 | 1.00 | 24.81 |
| atom | 2414 | CB | THR | 312 | 18.551 | 9.120 | 25.297 | 1.00 | 23.09 |
| atom | 2415 | OG1 | THR | 312 | 17.974 | 10.268 | 25.917 | 1.00 | 20.96 |
| atom | 2416 | CG2 | THR | 312 | 20.039 | 9.103 | 25.647 | 1.00 | 7.30 |
| atom | 2417 | C | THR | 312 | 19.286 | 8.291 | 23.048 | 1.00 | 19.93 |
| atom | 2418 | O | THR | 312 | 19.215 | 7.071 | 23.208 | 1.00 | 21.00 |
| atom | 2419 | N | MET | 313 | 20.146 | 8.863 | 22.225 | 1.00 | 21.26 |
| atom | 2420 | CA | MET | 313 | 21.087 | 8.039 | 21.502 | 1.00 | 19.10 |
| atom | 2421 | CB | MET | 313 | 20.845 | 8.108 | 20.003 | 1.00 | 27.05 |
| atom | 2422 | CG | MET | 313 | 20.425 | 9.437 | 19.460 | 1.00 | 32.49 |
| atom | 2423 | SD | MET | 313 | 19.755 | 9.147 | 17.816 | 1.00 | 41.09 |
| atom | 2424 | CE | MET | 313 | 18.946 | 7.570 | 18.055 | 1.00 | 41.67 |
| atom | 2425 | C | MET | 313 | 22.522 | 8.371 | 21.775 | 1.00 | 15.03 |
| atom | 2426 | O | MET | 313 | 22.862 | 9.456 | 22.237 | 1.00 | 9.15 |
| atom | 2427 | N | LEU | 314 | 23.360 | 7.394 | 21.477 | 1.00 | 14.59 |
| atom | 2428 | CA | LEU | 314 | 24.786 | 7.512 | 21.649 | 1.00 | 13.18 |
| atom | 2429 | CB | LEU | 314 | 25.237 | 6.561 | 22.753 | 1.00 | 16.64 |
| atom | 2430 | CG | LEU | 314 | 26.661 | 6.691 | 23.270 | 1.00 | 12.33 |
| atom | 2431 | CD1 | LEU | 314 | 26.848 | 8.047 | 23.960 | 1.00 | 17.77 |
| atom | 2432 | CD2 | LEU | 314 | 26.894 | 5.591 | 24.223 | 1.00 | 12.90 |
| atom | 2433 | C | LEU | 314 | 25.239 | 7.028 | 20.299 | 1.00 | 14.13 |
| atom | 2434 | O | LEU | 314 | 24.850 | 5.949 | 19.880 | 1.00 | 14.97 |
| atom | 2435 | N | VAL | 315 | 26.035 | 7.834 | 19.611 | 1.00 | 14.46 |
| atom | 2436 | CA | VAL | 315 | 26.490 | 7.507 | 18.260 | 1.00 | 8.47 |
| atom | 2437 | CB | VAL | 315 | 25.917 | 8.539 | 17.235 | 1.00 | 9.94 |
| atom | 2438 | CG1 | VAL | 315 | 26.126 | 8.042 | 15.792 | 1.00 | 6.93 |
| atom | 2439 | CG2 | VAL | 315 | 24.448 | 8.787 | 17.529 | 1.00 | 7.37 |
| atom | 2440 | C | VAL | 315 | 28.000 | 7.504 | 18.113 | 1.00 | 8.16 |
| atom | 2441 | O | VAL | 315 | 28.666 | 8.471 | 18.449 | 1.00 | 11.71 |
| atom | 2442 | N | ASN | 316 | 28.534 | 6.416 | 17.597 | 1.00 | 13.18 |
| atom | 2443 | CA | ASN | 316 | 29.976 | 6.296 | 17.363 | 1.00 | 16.47 |
| atom | 2444 | CB | ASN | 316 | 30.589 | 5.250 | 18.304 | 1.00 | 20.03 |

| atom | 2445 | CG  | ASN | 316 | 30.712 | 5.740  | 19.732 | 1.00 | 18.37 |
|------|------|-----|-----|-----|--------|--------|--------|------|-------|
| atom | 2446 | OD1 | ASN | 316 | 31.819 | 5.855  | 20.260 | 1.00 | 24.84 |
| atom | 2447 | ND2 | ASN | 316 | 29.581 | 6.024  | 20.364 | 1.00 | 11.13 |
| atom | 2448 | C   | ASN | 316 | 30.130 | 5.846  | 15.898 | 1.00 | 15.78 |
| atom | 2449 | O   | ASN | 316 | 30.014 | 4.652  | 15.596 | 1.00 | 12.76 |
| atom | 2450 | N   | GLY | 317 | 30.383 | 6.805  | 15.002 | 1.00 | 15.43 |
| atom | 2451 | CA  | GLY | 317 | 30.492 | 6.486  | 13.587 | 1.00 | 16.69 |
| atom | 2452 | C   | GLY | 317 | 29.186 | 5.824  | 13.155 | 1.00 | 19.29 |
| atom | 2453 | O   | GLY | 317 | 28.119 | 6.441  | 13.232 | 1.00 | 13.28 |
| atom | 2454 | N   | ASP | 318 | 29.262 | 4.558  | 12.736 | 1.00 | 20.60 |
| atom | 2455 | CA  | ASP | 318 | 28.080 | 3.798  | 12.313 | 1.00 | 22.17 |
| atom | 2456 | CB  | ASP | 318 | 28.486 | 2.767  | 11.281 | 1.00 | 23.30 |
| atom | 2457 | CG  | ASP | 318 | 29.724 | 2.027  | 11.684 | 1.00 | 25.41 |
| atom | 2458 | OD1 | ASP | 318 | 30.744 | 2.692  | 11.980 | 1.00 | 27.38 |
| atom | 2459 | OD2 | ASP | 318 | 29.676 | 0.788  | 11.710 | 1.00 | 25.90 |
| atom | 2460 | C   | ASP | 318 | 27.403 | 3.067  | 13.479 | 1.00 | 24.22 |
| atom | 2461 | O   | ASP | 318 | 26.245 | 2.626  | 13.380 | 1.00 | 20.11 |
| atom | 2462 | N   | ASP | 319 | 28.135 | 2.899  | 14.574 | 1.00 | 25.94 |
| atom | 2463 | CA  | ASP | 319 | 27.559 | 2.217  | 15.730 | 1.00 | 26.01 |
| atom | 2464 | CB  | ASP | 319 | 28.654 | 1.768  | 16.680 | 1.00 | 26.27 |
| atom | 2465 | CG  | ASP | 319 | 28.826 | 0.275  | 16.677 | 1.00 | 29.65 |
| atom | 2466 | OD1 | ASP | 319 | 29.973 | -0.184 | 16.521 | 1.00 | 35.30 |
| atom | 2467 | OD2 | ASP | 319 | 27.813 | -0.439 | 16.828 | 1.00 | 30.47 |
| atom | 2468 | C   | ASP | 319 | 26.552 | 3.110  | 16.452 | 1.00 | 20.07 |
| atom | 2469 | O   | ASP | 319 | 26.826 | 4.269  | 16.766 | 1.00 | 15.33 |
| atom | 2470 | N   | LEU | 320 | 25.376 | 2.567  | 16.697 | 1.00 | 16.66 |
| atom | 2471 | CA  | LEU | 320 | 24.341 | 3.341  | 17.350 | 1.00 | 17.93 |
| atom | 2472 | CB  | LEU | 320 | 23.428 | 3.994  | 16.294 | 1.00 | 14.80 |
| atom | 2473 | CG  | LEU | 320 | 22.126 | 4.657  | 16.786 | 1.00 | 12.07 |
| atom | 2474 | CD1 | LEU | 320 | 22.214 | 6.175  | 16.732 | 1.00 | 12.14 |
| atom | 2475 | CD2 | LEU | 320 | 20.993 | 4.180  | 15.980 | 1.00 | 14.40 |
| atom | 2476 | C   | LEU | 320 | 23.484 | 2.538  | 18.314 | 1.00 | 18.43 |
| atom | 2477 | O   | LEU | 320 | 23.097 | 1.410  | 18.042 | 1.00 | 23.21 |
| atom | 2478 | N   | VAL | 321 | 23.211 | 3.135  | 19.460 | 1.00 | 18.50 |
| atom | 2479 | CA  | VAL | 321 | 22.327 | 2.539  | 20.441 | 1.00 | 21.88 |
| atom | 2480 | CB  | VAL | 321 | 23.097 | 1.966  | 21.662 | 1.00 | 20.42 |

| atom | 2481 | CG1 | VAL | 321 | 24.196 | 2.895 | 22.062 | 1.00 | 17.51 |
|------|------|-----|-----|-----|--------|-------|--------|------|-------|
| atom | 2482 | CG2 | VAL | 321 | 22.136 | 1.735 | 22.817 | 1.00 | 22.27 |
| atom | 2483 | C   | VAL | 321 | 21.413 | 3.683 | 20.878 | 1.00 | 20.73 |
| atom | 2484 | O   | VAL | 321 | 21.826 | 4.844 | 20.897 | 1.00 | 21.92 |
| atom | 2485 | N   | VAL | 322 | 20.162 | 3.371 | 21.181 | 1.00 | 22.82 |
| atom | 2486 | CA  | VAL | 322 | 19.230 | 4.400 | 21.623 | 1.00 | 20.84 |
| atom | 2487 | CB  | VAL | 322 | 18.343 | 4.957 | 20.448 | 1.00 | 18.24 |
| atom | 2488 | CG1 | VAL | 322 | 18.628 | 4.215 | 19.149 | 1.00 | 22.10 |
| atom | 2489 | CG2 | VAL | 322 | 16.880 | 4.885 | 20.810 | 1.00 | 6.40 |
| atom | 2490 | C   | VAL | 322 | 18.353 | 3.819 | 22.714 | 1.00 | 23.90 |
| atom | 2491 | O   | VAL | 322 | 17.873 | 2.685 | 22.608 | 1.00 | 22.28 |
| atom | 2492 | N   | ILE | 323 | 18.176 | 4.585 | 23.786 | 1.00 | 24.77 |
| atom | 2493 | CA  | ILE | 323 | 17.349 | 4.134 | 24.893 | 1.00 | 25.24 |
| atom | 2494 | CB  | ILE | 323 | 18.158 | 4.137 | 26.190 | 1.00 | 22.50 |
| atom | 2495 | CG2 | ILE | 323 | 17.259 | 3.829 | 27.384 | 1.00 | 23.14 |
| atom | 2496 | CG1 | ILE | 323 | 19.261 | 3.095 | 26.066 | 1.00 | 16.23 |
| atom | 2497 | CD1 | ILE | 323 | 20.397 | 3.297 | 27.008 | 1.00 | 15.90 |
| atom | 2498 | C   | ILE | 323 | 16.131 | 5.042 | 24.994 | 1.00 | 25.29 |
| atom | 2499 | O   | ILE | 323 | 16.254 | 6.266 | 25.033 | 1.00 | 25.71 |
| atom | 2500 | N   | CYS | 324 | 14.955 | 4.434 | 25.021 | 1.00 | 23.65 |
| atom | 2501 | CA  | CYS | 324 | 13.719 | 5.201 | 25.074 | 1.00 | 28.54 |
| atom | 2502 | CB  | CYS | 324 | 13.120 | 5.298 | 23.668 | 1.00 | 22.91 |
| atom | 2503 | SG  | CYS | 324 | 12.651 | 3.689 | 23.036 | 1.00 | 24.99 |
| atom | 2504 | C   | CYS | 324 | 12.698 | 4.560 | 26.004 | 1.00 | 32.49 |
| atom | 2505 | O   | CYS | 324 | 12.954 | 3.502 | 26.611 | 1.00 | 36.12 |
| atom | 2506 | N   | GLU | 325 | 11.541 | 5.212 | 26.117 | 1.00 | 34.49 |
| atom | 2507 | CA  | GLU | 325 | 10.454 | 4.697 | 26.942 | 1.00 | 34.97 |
| atom | 2508 | CB  | GLU | 325 | 9.367  | 5.759 | 27.101 | 1.00 | 33.51 |
| atom | 2509 | CG  | GLU | 325 | 9.577  | 6.705 | 28.273 | 1.00 | 39.83 |
| atom | 2510 | CD  | GLU | 325 | 10.005 | 6.005 | 29.565 | 1.00 | 40.86 |
| atom | 2511 | OE1 | GLU | 325 | 9.637  | 4.828 | 29.786 | 1.00 | 41.53 |
| atom | 2512 | OE2 | GLU | 325 | 10.716 | 6.644 | 30.367 | 1.00 | 41.72 |
| atom | 2513 | C   | GLU | 325 | 9.863  | 3.470 | 26.243 | 1.00 | 33.00 |
| atom | 2514 | O   | GLU | 325 | 10.045 | 3.291 | 25.038 | 1.00 | 33.16 |
| atom | 2515 | N   | SER | 326 | 9.177  | 2.608 | 26.981 | 1.00 | 29.74 |
| atom | 2516 | CA  | SER | 326 | 8.566  | 1.462 | 26.332 | 1.00 | 27.88 |

| atom | 2517 | CB | SER | 326 | 8.895 | 0.154 | 27.045 | 1.00 | 29.60 |
| atom | 2518 | OG | SER | 326 | 8.592 | -0.968 | 26.221 | 1.00 | 27.55 |
| atom | 2519 | C | SER | 326 | 7.082 | 1.687 | 26.359 | 1.00 | 29.75 |
| atom | 2520 | O | SER | 326 | 6.580 | 2.500 | 27.130 | 1.00 | 26.24 |
| atom | 2521 | N | ALA | 327 | 6.382 | 0.983 | 25.483 | 1.00 | 33.65 |
| atom | 2522 | CA | ALA | 327 | 4.937 | 1.093 | 25.404 | 1.00 | 32.64 |
| atom | 2523 | CB | ALA | 327 | 4.533 | 1.949 | 24.224 | 1.00 | 35.74 |
| atom | 2524 | C | ALA | 327 | 4.455 | -0.314 | 25.212 | 1.00 | 35.26 |
| atom | 2525 | O | ALA | 327 | 3.405 | -0.541 | 24.613 | 1.00 | 36.42 |
| atom | 2526 | N | GLY | 328 | 5.250 | -1.259 | 25.715 | 1.00 | 35.74 |
| atom | 2527 | CA | GLY | 328 | 4.906 | -2.658 | 25.604 | 1.00 | 35.12 |
| atom | 2528 | C | GLY | 328 | 5.739 | -3.370 | 24.563 | 1.00 | 37.03 |
| atom | 2529 | O | GLY | 328 | 6.267 | -2.751 | 23.648 | 1.00 | 39.73 |
| atom | 2530 | N | THR | 329 | 5.828 | -4.686 | 24.705 | 1.00 | 35.68 |
| atom | 2531 | CA | THR | 329 | 6.592 | -5.553 | 23.816 | 1.00 | 35.67 |
| atom | 2532 | CB | THR | 329 | 6.517 | -6.994 | 24.334 | 1.00 | 31.01 |
| atom | 2533 | OG1 | THR | 329 | 6.992 | -7.020 | 25.679 | 1.00 | 30.45 |
| atom | 2534 | CG2 | THR | 329 | 7.348 | -7.935 | 23.486 | 1.00 | 35.07 |
| atom | 2535 | C | THR | 329 | 6.188 | -5.555 | 22.338 | 1.00 | 41.30 |
| atom | 2536 | O | THR | 329 | 6.985 | -5.217 | 21.459 | 1.00 | 42.33 |
| atom | 2537 | N | GLN | 330 | 4.954 | -5.964 | 22.068 | 1.00 | 45.00 |
| atom | 2538 | CA | GLN | 330 | 4.464 | -6.047 | 20.705 | 1.00 | 45.98 |
| atom | 2539 | CB | GLN | 330 | 3.072 | -6.675 | 20.703 | 1.00 | 50.57 |
| atom | 2540 | CG | GLN | 330 | 2.859 | -7.708 | 19.613 | 1.00 | 53.88 |
| atom | 2541 | CD | GLN | 330 | 1.764 | -8.697 | 19.954 | 1.00 | 55.97 |
| atom | 2542 | OE1 | GLN | 330 | 2.015 | -9.713 | 20.598 | 1.00 | 58.15 |
| atom | 2543 | NE2 | GLN | 330 | 0.541 | -8.404 | 19.524 | 1.00 | 58.64 |
| atom | 2544 | C | GLN | 330 | 4.417 | -4.690 | 20.017 | 1.00 | 46.57 |
| atom | 2545 | O | GLN | 330 | 4.625 | -4.588 | 18.809 | 1.00 | 45.26 |
| atom | 2546 | N | GLU | 331 | 4.138 | -3.648 | 20.785 | 1.00 | 42.44 |
| atom | 2547 | CA | GLU | 331 | 4.054 | -2.318 | 20.216 | 1.00 | 42.19 |
| atom | 2548 | CB | GLU | 331 | 3.307 | -1.414 | 21.193 | 1.00 | 45.19 |
| atom | 2549 | CG | GLU | 331 | 2.738 | -2.173 | 22.420 | 1.00 | 53.99 |
| atom | 2550 | CD | GLU | 331 | 1.499 | -3.035 | 22.106 | 1.00 | 61.39 |
| atom | 2551 | OE1 | GLU | 331 | 1.620 | -4.022 | 21.340 | 1.00 | 62.11 |
| atom | 2552 | OE2 | GLU | 331 | 0.401 | -2.733 | 22.631 | 1.00 | 60.82 |

| atom | 2553 | C   | GLU | 331 | 5.468  | -1.807 | 19.926 | 1.00 | 42.42 |
|------|------|-----|-----|-----|--------|--------|--------|------|-------|
| atom | 2554 | O   | GLU | 331 | 5.746  | -1.304 | 18.838 | 1.00 | 41.63 |
| atom | 2555 | N   | ASP | 332 | 6.361  | -1.970 | 20.899 | 1.00 | 40.40 |
| atom | 2556 | CA  | ASP | 332 | 7.759  | -1.554 | 20.779 | 1.00 | 39.72 |
| atom | 2557 | CB  | ASP | 332 | 8.522  | -1.903 | 22.066 | 1.00 | 43.46 |
| atom | 2558 | CG  | ASP | 332 | 8.576  | -0.750 | 23.070 | 1.00 | 44.40 |
| atom | 2559 | OD1 | ASP | 332 | 9.624  | -0.613 | 23.737 | 1.00 | 50.21 |
| atom | 2560 | OD2 | ASP | 332 | 7.587  | 0.005  | 23.214 | 1.00 | 42.70 |
| atom | 2561 | C   | ASP | 332 | 8.455  | -2.238 | 19.587 | 1.00 | 38.03 |
| atom | 2562 | O   | ASP | 332 | 9.360  | -1.669 | 18.973 | 1.00 | 35.13 |
| atom | 2563 | N   | ALA | 333 | 8.036  | -3.463 | 19.274 | 1.00 | 35.59 |
| atom | 2564 | CA  | ALA | 333 | 8.606  | -4.223 | 18.157 | 1.00 | 34.66 |
| atom | 2565 | CB  | ALA | 333 | 8.080  | -5.660 | 18.188 | 1.00 | 28.59 |
| atom | 2566 | C   | ALA | 333 | 8.250  | -3.559 | 16.818 | 1.00 | 35.39 |
| atom | 2567 | O   | ALA | 333 | 9.096  | -3.397 | 15.918 | 1.00 | 32.47 |
| atom | 2568 | N   | ALA | 334 | 6.980  | -3.194 | 16.701 | 1.00 | 32.90 |
| atom | 2569 | CA  | ALA | 334 | 6.475  | -2.528 | 15.522 | 1.00 | 35.01 |
| atom | 2570 | CB  | ALA | 334 | 4.958  | -2.387 | 15.614 | 1.00 | 30.49 |
| atom | 2571 | C   | ALA | 334 | 7.132  | -1.151 | 15.441 | 1.00 | 36.42 |
| atom | 2572 | O   | ALA | 334 | 7.257  | -0.580 | 14.360 | 1.00 | 38.09 |
| atom | 2573 | N   | SER | 335 | 7.554  | -0.622 | 16.588 | 1.00 | 35.49 |
| atom | 2574 | CA  | SER | 335 | 8.197  | 0.685  | 16.624 | 1.00 | 33.47 |
| atom | 2575 | CB  | SER | 335 | 8.376  | 1.156  | 18.066 | 1.00 | 35.80 |
| atom | 2576 | OG  | SER | 335 | 7.166  | 1.715  | 18.558 | 1.00 | 44.77 |
| atom | 2577 | C   | SER | 335 | 9.549  | 0.611  | 15.937 | 1.00 | 33.00 |
| atom | 2578 | O   | SER | 335 | 9.923  | 1.509  | 15.186 | 1.00 | 31.79 |
| atom | 2579 | N   | LEU | 336 | 10.278 | -0.468 | 16.193 | 1.00 | 30.58 |
| atom | 2580 | CA  | LEU | 336 | 11.580 | -0.643 | 15.580 | 1.00 | 32.25 |
| atom | 2581 | CB  | LEU | 336 | 12.355 | -1.740 | 16.300 | 1.00 | 33.78 |
| atom | 2582 | CG  | LEU | 336 | 13.103 | -1.268 | 17.549 | 1.00 | 32.01 |
| atom | 2583 | CD1 | LEU | 336 | 12.219 | -0.378 | 18.402 | 1.00 | 19.15 |
| atom | 2584 | CD2 | LEU | 336 | 13.556 | -2.491 | 18.319 | 1.00 | 32.33 |
| atom | 2585 | C   | LEU | 336 | 11.397 | -0.989 | 14.111 | 1.00 | 33.05 |
| atom | 2586 | O   | LEU | 336 | 12.301 | -0.798 | 13.298 | 1.00 | 32.54 |
| atom | 2587 | N   | ARG | 337 | 10.212 | -1.500 | 13.785 | 1.00 | 34.57 |
| atom | 2588 | CA  | ARG | 337 | 9.861  | -1.840 | 12.415 | 1.00 | 33.94 |

| atom | 2589 | CB  | ARG | 337 | 8.481  | -2.502 | 12.376 | 1.00 | 40.01 |
|------|------|-----|-----|-----|--------|--------|--------|------|-------|
| atom | 2590 | CG  | ARG | 337 | 8.468  | -3.887 | 11.748 | 1.00 | 50.31 |
| atom | 2591 | CD  | ARG | 337 | 7.152  | -4.156 | 11.006 | 1.00 | 60.06 |
| atom | 2592 | NE  | ARG | 337 | 7.349  | -4.414 | 9.573  | 1.00 | 64.64 |
| atom | 2593 | CZ  | ARG | 337 | 6.370  | -4.443 | 8.666  | 1.00 | 66.06 |
| atom | 2594 | NH1 | ARG | 337 | 5.108  | -4.228 | 9.027  | 1.00 | 65.93 |
| atom | 2595 | NH2 | ARG | 337 | 6.653  | -4.693 | 7.392  | 1.00 | 63.83 |
| atom | 2596 | C   | ARG | 337 | 9.833  | -0.524 | 11.628 | 1.00 | 31.50 |
| atom | 2597 | O   | ARG | 337 | 10.527 | -0.367 | 10.614 | 1.00 | 30.22 |
| atom | 2598 | N   | VAL | 338 | 9.034  | 0.426  | 12.104 | 1.00 | 25.96 |
| atom | 2599 | CA  | VAL | 338 | 8.942  | 1.725  | 11.446 | 1.00 | 23.74 |
| atom | 2600 | CB  | VAL | 338 | 8.008  | 2.682  | 12.212 | 1.00 | 20.53 |
| atom | 2601 | CG1 | VAL | 338 | 7.954  | 4.005  | 11.504 | 1.00 | 16.58 |
| atom | 2602 | CG2 | VAL | 338 | 6.586  | 2.080  | 12.323 | 1.00 | 23.01 |
| atom | 2603 | C   | VAL | 338 | 10.321 | 2.375  | 11.393 | 1.00 | 25.39 |
| atom | 2604 | O   | VAL | 338 | 10.760 | 2.853  | 10.348 | 1.00 | 25.50 |
| atom | 2605 | N   | PHE | 339 | 11.008 | 2.372  | 12.534 | 1.00 | 24.65 |
| atom | 2606 | CA  | PHE | 339 | 12.323 | 2.985  | 12.643 | 1.00 | 19.60 |
| atom | 2607 | CB  | PHE | 339 | 12.934 | 2.698  | 14.018 | 1.00 | 15.60 |
| atom | 2608 | CG  | PHE | 339 | 14.325 | 3.191  | 14.164 | 1.00 | 3.47  |
| atom | 2609 | CD1 | PHE | 339 | 14.569 | 4.487  | 14.582 | 1.00 | 9.45  |
| atom | 2610 | CD2 | PHE | 339 | 15.390 | 2.374  | 13.849 | 1.00 | 4.97  |
| atom | 2611 | CE1 | PHE | 339 | 15.859 | 4.965  | 14.685 | 1.00 | 5.74  |
| atom | 2612 | CE2 | PHE | 339 | 16.685 | 2.830  | 13.941 | 1.00 | 3.08  |
| atom | 2613 | CZ  | PHE | 339 | 16.927 | 4.126  | 14.360 | 1.00 | 5.97  |
| atom | 2614 | C   | PHE | 339 | 13.232 | 2.480  | 11.534 | 1.00 | 18.70 |
| atom | 2615 | O   | PHE | 339 | 13.866 | 3.267  | 10.845 | 1.00 | 12.55 |
| atom | 2616 | N   | THR | 340 | 13.282 | 1.164  | 11.381 | 1.00 | 20.16 |
| atom | 2617 | CA  | THR | 340 | 14.077 | 0.510  | 10.341 | 1.00 | 23.32 |
| atom | 2618 | CB  | THR | 340 | 13.934 | -1.028 | 10.463 | 1.00 | 23.80 |
| atom | 2619 | OG1 | THR | 340 | 14.663 | -1.472 | 11.616 | 1.00 | 26.76 |
| atom | 2620 | CG2 | THR | 340 | 14.441 | -1.740 | 9.182  | 1.00 | 14.19 |
| atom | 2621 | C   | THR | 340 | 13.618 | 0.947  | 8.925  | 1.00 | 21.79 |
| atom | 2622 | O   | THR | 340 | 14.432 | 1.172  | 8.031  | 1.00 | 21.76 |
| atom | 2623 | N   | GLU | 341 | 12.308 | 1.053  | 8.731  | 1.00 | 18.62 |
| atom | 2624 | CA  | GLU | 341 | 11.766 | 1.482  | 7.446  | 1.00 | 20.97 |

| atom | 2625 | CB  | GLU | 341 | 10.242 | 1.518  | 7.477  | 1.00 | 18.92 |
| atom | 2626 | CG  | GLU | 341 | 9.568  | 0.369  | 6.799  | 1.00 | 27.50 |
| atom | 2627 | CD  | GLU | 341 | 8.219  | 0.080  | 7.416  | 1.00 | 32.36 |
| atom | 2628 | OE1 | GLU | 341 | 8.195  | -0.569 | 8.481  | 1.00 | 33.70 |
| atom | 2629 | OE2 | GLU | 341 | 7.187  | 0.507  | 6.846  | 1.00 | 36.79 |
| atom | 2630 | C   | GLU | 341 | 12.259 | 2.878  | 7.081  | 1.00 | 18.52 |
| atom | 2631 | O   | GLU | 341 | 12.616 | 3.117  | 5.943  | 1.00 | 16.55 |
| atom | 2632 | N   | ALA | 342 | 12.248 | 3.801  | 8.042  | 1.00 | 15.66 |
| atom | 2633 | CA  | ALA | 342 | 12.703 | 5.147  | 7.760  | 1.00 | 11.85 |
| atom | 2634 | CB  | ALA | 342 | 12.420 | 6.075  | 8.949  | 1.00 | 5.98  |
| atom | 2635 | C   | ALA | 342 | 14.192 | 5.089  | 7.451  | 1.00 | 13.06 |
| atom | 2636 | O   | ALA | 342 | 14.630 | 5.655  | 6.466  | 1.00 | 17.39 |
| atom | 2637 | N   | MET | 343 | 14.958 | 4.378  | 8.279  | 1.00 | 14.83 |
| atom | 2638 | CA  | MET | 343 | 16.401 | 4.240  | 8.096  | 1.00 | 15.43 |
| atom | 2639 | CB  | MET | 343 | 17.005 | 3.288  | 9.152  | 1.00 | 17.16 |
| atom | 2640 | CG  | MET | 343 | 17.219 | 3.903  | 10.544 | 1.00 | 14.16 |
| atom | 2641 | SD  | MET | 343 | 18.366 | 5.309  | 10.607 | 1.00 | 15.78 |
| atom | 2642 | CE  | MET | 343 | 19.905 | 4.462  | 10.694 | 1.00 | 8.77  |
| atom | 2643 | C   | MET | 343 | 16.729 | 3.717  | 6.686  | 1.00 | 20.04 |
| atom | 2644 | O   | MET | 343 | 17.685 | 4.188  | 6.066  | 1.00 | 22.60 |
| atom | 2645 | N   | THR | 344 | 15.952 | 2.748  | 6.196  | 1.00 | 15.69 |
| atom | 2646 | CA  | THR | 344 | 16.158 | 2.191  | 4.859  | 1.00 | 17.93 |
| atom | 2647 | CB  | THR | 344 | 15.265 | 0.945  | 4.622  | 1.00 | 19.23 |
| atom | 2648 | OG1 | THR | 344 | 15.461 | 0.013  | 5.680  | 1.00 | 16.90 |
| atom | 2649 | CG2 | THR | 344 | 15.626 | 0.265  | 3.313  | 1.00 | 23.01 |
| atom | 2650 | C   | THR | 344 | 15.841 | 3.233  | 3.767  | 1.00 | 16.50 |
| atom | 2651 | O   | THR | 344 | 16.473 | 3.258  | 2.708  | 1.00 | 14.88 |
| atom | 2652 | N   | ARG | 345 | 14.857 | 4.086  | 4.028  | 1.00 | 12.81 |
| atom | 2653 | CA  | ARG | 345 | 14.483 | 5.130  | 3.075  | 1.00 | 12.96 |
| atom | 2654 | CB  | ARG | 345 | 13.256 | 5.890  | 3.558  | 1.00 | 12.13 |
| atom | 2655 | CG  | ARG | 345 | 12.127 | 5.933  | 2.572  | 1.00 | 24.12 |
| atom | 2656 | CD  | ARG | 345 | 10.831 | 5.426  | 3.174  | 1.00 | 19.46 |
| atom | 2657 | NE  | ARG | 345 | 10.495 | 6.121  | 4.416  | 1.00 | 22.42 |
| atom | 2658 | CZ  | ARG | 345 | 9.921  | 5.523  | 5.455  | 1.00 | 23.15 |
| atom | 2659 | NH1 | ARG | 345 | 9.615  | 4.230  | 5.387  | 1.00 | 22.49 |
| atom | 2660 | NH2 | ARG | 345 | 9.642  | 6.211  | 6.553  | 1.00 | 22.68 |

| atom | 2661 | C   | ARG | 345 | 15.628 | 6.110  | 2.972  | 1.00 | 11.64 |
|------|------|-----|-----|-----|--------|--------|--------|------|-------|
| atom | 2662 | O   | ARG | 345 | 15.861 | 6.699  | 1.939  | 1.00 | 15.57 |
| atom | 2663 | N   | TYR | 346 | 16.344 | 6.285  | 4.073  | 1.00 | 16.09 |
| atom | 2664 | CA  | TYR | 346 | 17.452 | 7.219  | 4.117  | 1.00 | 10.12 |
| atom | 2665 | CB  | TYR | 346 | 17.673 | 7.718  | 5.537  | 1.00 | 10.24 |
| atom | 2666 | CG  | TYR | 346 | 16.491 | 8.434  | 6.161  | 1.00 | 8.55  |
| atom | 2667 | CD1 | TYR | 346 | 16.356 | 8.477  | 7.546  | 1.00 | 2.00  |
| atom | 2668 | CE1 | TYR | 346 | 15.341 | 9.215  | 8.159  | 1.00 | 7.85  |
| atom | 2669 | CD2 | TYR | 346 | 15.566 | 9.149  | 5.378  | 1.00 | 3.32  |
| atom | 2670 | CE2 | TYR | 346 | 14.532 | 9.900  | 5.980  | 1.00 | 4.00  |
| atom | 2671 | CZ  | TYR | 346 | 14.434 | 9.921  | 7.377  | 1.00 | 11.88 |
| atom | 2672 | OH  | TYR | 346 | 13.415 | 10.589 | 8.011  | 1.00 | 16.58 |
| atom | 2673 | C   | TYR | 346 | 18.688 | 6.530  | 3.649  | 1.00 | 10.73 |
| atom | 2674 | O   | TYR | 346 | 19.759 | 7.131  | 3.648  | 1.00 | 14.56 |
| atom | 2675 | N   | SER | 347 | 18.544 | 5.262  | 3.263  | 1.00 | 8.88  |
| atom | 2676 | CA  | SER | 347 | 19.682 | 4.485  | 2.779  | 1.00 | 11.02 |
| atom | 2677 | CB  | SER | 347 | 20.436 | 5.257  | 1.719  | 1.00 | 7.10  |
| atom | 2678 | OG  | SER | 347 | 21.677 | 4.649  | 1.524  | 1.00 | 15.91 |
| atom | 2679 | C   | SER | 347 | 20.655 | 4.127  | 3.891  | 1.00 | 17.57 |
| atom | 2680 | O   | SER | 347 | 21.824 | 4.535  | 3.894  | 1.00 | 16.67 |
| atom | 2681 | N   | ALA | 348 | 20.139 | 3.374  | 4.850  | 1.00 | 21.53 |
| atom | 2682 | CA  | ALA | 348 | 20.901 | 2.902  | 5.984  | 1.00 | 16.82 |
| atom | 2683 | CB  | ALA | 348 | 20.931 | 3.942  | 7.063  | 1.00 | 19.61 |
| atom | 2684 | C   | ALA | 348 | 20.129 | 1.676  | 6.436  | 1.00 | 17.95 |
| atom | 2685 | O   | ALA | 348 | 19.612 | 1.636  | 7.541  | 1.00 | 19.35 |
| atom | 2686 | N   | PRO | 349 | 20.019 | 0.666  | 5.558  | 1.00 | 17.48 |
| atom | 2687 | CD  | PRO | 349 | 20.619 | 0.634  | 4.218  | 1.00 | 17.13 |
| atom | 2688 | CA  | PRO | 349 | 19.309 | -0.585 | 5.845  | 1.00 | 22.29 |
| atom | 2689 | CB  | PRO | 349 | 19.249 | -1.270 | 4.485  | 1.00 | 18.68 |
| atom | 2690 | CG  | PRO | 349 | 20.508 | -0.822 | 3.840  | 1.00 | 18.33 |
| atom | 2691 | C   | PRO | 349 | 20.107 | -1.390 | 6.883  | 1.00 | 22.65 |
| atom | 2692 | O   | PRO | 349 | 21.327 | -1.266 | 6.976  | 1.00 | 22.99 |
| atom | 2693 | N   | PRO | 350 | 19.433 | -2.257 | 7.640  | 1.00 | 25.69 |
| atom | 2694 | CD  | PRO | 350 | 17.999 | -2.613 | 7.620  | 1.00 | 28.35 |
| atom | 2695 | CA  | PRO | 350 | 20.147 | -3.026 | 8.656  | 1.00 | 29.11 |
| atom | 2696 | CB  | PRO | 350 | 19.093 | -3.186 | 9.747  | 1.00 | 23.51 |

| atom | 2697 | CG | PRO | 350 | 17.818 | -3.395 | 8.940 | 1.00 | 21.77 |
|------|------|------|------|------|--------|--------|--------|------|-------|
| atom | 2698 | C | PRO | 350 | 20.721 | -4.376 | 8.241 | 1.00 | 33.76 |
| atom | 2699 | O | PRO | 350 | 20.133 | -5.090 | 7.431 | 1.00 | 35.09 |
| atom | 2700 | N | GLY | 351 | 21.874 | -4.718 | 8.815 | 1.00 | 38.72 |
| atom | 2701 | CA | GLY | 351 | 22.476 | -6.010 | 8.549 | 1.00 | 42.12 |
| atom | 2702 | C | GLY | 351 | 21.610 | -7.000 | 9.315 | 1.00 | 45.61 |
| atom | 2703 | O | GLY | 351 | 21.067 | -7.954 | 8.743 | 1.00 | 47.44 |
| atom | 2704 | N | ASP | 352 | 21.483 | -6.752 | 10.621 | 1.00 | 44.23 |
| atom | 2705 | CA | ASP | 352 | 20.660 | -7.564 | 11.519 | 1.00 | 41.08 |
| atom | 2706 | CB | ASP | 352 | 21.451 | -7.984 | 12.764 | 1.00 | 43.20 |
| atom | 2707 | CG | ASP | 352 | 22.665 | -8.844 | 12.434 | 1.00 | 47.05 |
| atom | 2708 | OD1 | ASP | 352 | 22.475 | -10.011 | 12.015 | 1.00 | 49.57 |
| atom | 2709 | OD2 | ASP | 352 | 23.809 | -8.358 | 12.599 | 1.00 | 43.57 |
| atom | 2710 | C | ASP | 352 | 19.544 | -6.618 | 11.929 | 1.00 | 35.01 |
| atom | 2711 | O | ASP | 352 | 19.787 | -5.445 | 12.194 | 1.00 | 36.47 |
| atom | 2712 | N | PRO | 353 | 18.304 | -7.100 | 11.969 | 1.00 | 31.96 |
| atom | 2713 | CD | PRO | 353 | 17.786 | -8.437 | 11.653 | 1.00 | 33.23 |
| atom | 2714 | CA | PRO | 353 | 17.238 | -6.177 | 12.363 | 1.00 | 32.72 |
| atom | 2715 | CB | PRO | 353 | 15.961 | -7.010 | 12.218 | 1.00 | 30.87 |
| atom | 2716 | CG | PRO | 353 | 16.352 | -8.150 | 11.317 | 1.00 | 33.49 |
| atom | 2717 | C | PRO | 353 | 17.426 | -5.659 | 13.778 | 1.00 | 29.30 |
| atom | 2718 | O | PRO | 353 | 18.168 | -6.231 | 14.563 | 1.00 | 31.98 |
| atom | 2719 | N | PRO | 354 | 16.808 | -4.526 | 14.103 | 1.00 | 26.36 |
| atom | 2720 | CD | PRO | 354 | 15.998 | -3.614 | 13.280 | 1.00 | 25.22 |
| atom | 2721 | CA | PRO | 354 | 16.977 | -4.037 | 15.472 | 1.00 | 25.21 |
| atom | 2722 | CB | PRO | 354 | 16.557 | -2.581 | 15.380 | 1.00 | 23.85 |
| atom | 2723 | CG | PRO | 354 | 15.528 | -2.571 | 14.271 | 1.00 | 23.54 |
| atom | 2724 | C | PRO | 354 | 16.042 | -4.838 | 16.383 | 1.00 | 27.24 |
| atom | 2725 | O | PRO | 354 | 14.889 | -5.090 | 16.034 | 1.00 | 22.85 |
| atom | 2726 | N | GLN | 355 | 16.532 | -5.260 | 17.540 | 1.00 | 29.74 |
| atom | 2727 | CA | GLN | 355 | 15.672 | -6.012 | 18.452 | 1.00 | 32.54 |
| atom | 2728 | CB | GLN | 355 | 16.264 | -7.391 | 18.752 | 1.00 | 33.31 |
| atom | 2729 | CG | GLN | 355 | 15.215 | -8.408 | 19.199 | 1.00 | 42.98 |
| atom | 2730 | CD | GLN | 355 | 15.701 | -9.856 | 19.102 | 1.00 | 47.82 |
| atom | 2731 | OE1 | GLN | 355 | 14.915 | -10.774 | 18.818 | 1.00 | 47.88 |
| atom | 2732 | NE2 | GLN | 355 | 17.000 | -10.066 | 19.337 | 1.00 | 47.91 |

| atom | 2733 | C   | GLN | 355 | 15.504 | -5.227 | 19.744 | 1.00 | 29.39 |
|------|------|-----|-----|-----|--------|--------|--------|------|-------|
| atom | 2734 | O   | GLN | 355 | 16.490 | -4.764 | 20.316 | 1.00 | 28.43 |
| atom | 2735 | N   | PRO | 356 | 14.255 | -5.056 | 20.217 | 1.00 | 28.59 |
| atom | 2736 | CD  | PRO | 356 | 12.960 | -5.521 | 19.690 | 1.00 | 28.05 |
| atom | 2737 | CA  | PRO | 356 | 14.118 | -4.298 | 21.463 | 1.00 | 29.05 |
| atom | 2738 | CB  | PRO | 356 | 12.621 | -4.007 | 21.568 | 1.00 | 23.89 |
| atom | 2739 | CG  | PRO | 356 | 11.954 | -4.958 | 20.661 | 1.00 | 27.97 |
| atom | 2740 | C   | PRO | 356 | 14.659 | -5.097 | 22.649 | 1.00 | 33.81 |
| atom | 2741 | O   | PRO | 356 | 14.409 | -6.307 | 22.776 | 1.00 | 33.07 |
| atom | 2742 | N   | GLU | 357 | 15.426 | -4.404 | 23.487 | 1.00 | 32.99 |
| atom | 2743 | CA  | GLU | 357 | 16.037 | -4.979 | 24.667 | 1.00 | 34.32 |
| atom | 2744 | CB  | GLU | 357 | 17.539 | -4.694 | 24.656 | 1.00 | 34.64 |
| atom | 2745 | CG  | GLU | 357 | 18.438 | -5.917 | 24.654 | 1.00 | 41.61 |
| atom | 2746 | CD  | GLU | 357 | 17.794 | -7.141 | 24.028 | 1.00 | 46.37 |
| atom | 2747 | OE1 | GLU | 357 | 16.749 | -7.006 | 23.347 | 1.00 | 49.58 |
| atom | 2748 | OE2 | GLU | 357 | 18.342 | -8.247 | 24.215 | 1.00 | 47.15 |
| atom | 2749 | C   | GLU | 357 | 15.413 | -4.379 | 25.916 | 1.00 | 36.99 |
| atom | 2750 | O   | GLU | 357 | 14.964 | -3.227 | 25.908 | 1.00 | 40.64 |
| atom | 2751 | N   | TYR | 358 | 15.401 | -5.161 | 26.990 | 1.00 | 37.60 |
| atom | 2752 | CA  | TYR | 358 | 14.841 | -4.724 | 28.257 | 1.00 | 35.53 |
| atom | 2753 | CB  | TYR | 358 | 13.519 | -5.463 | 28.480 | 1.00 | 32.72 |
| atom | 2754 | CG  | TYR | 358 | 12.515 | -5.183 | 27.373 | 1.00 | 31.24 |
| atom | 2755 | CD1 | TYR | 358 | 12.387 | -6.055 | 26.278 | 1.00 | 29.89 |
| atom | 2756 | CE1 | TYR | 358 | 11.507 | -5.776 | 25.234 | 1.00 | 30.39 |
| atom | 2757 | CD2 | TYR | 358 | 11.724 | -4.023 | 27.393 | 1.00 | 21.47 |
| atom | 2758 | CE2 | TYR | 358 | 10.844 | -3.736 | 26.364 | 1.00 | 21.96 |
| atom | 2759 | CZ  | TYR | 358 | 10.740 | -4.616 | 25.279 | 1.00 | 27.72 |
| atom | 2760 | OH  | TYR | 358 | 9.900  | -4.319 | 24.229 | 1.00 | 21.73 |
| atom | 2761 | C   | TYR | 358 | 15.862 | -5.014 | 29.369 | 1.00 | 37.22 |
| atom | 2762 | O   | TYR | 358 | 15.656 | -4.689 | 30.541 | 1.00 | 35.67 |
| atom | 2763 | N   | ASP | 359 | 16.974 | -5.620 | 28.962 | 1.00 | 36.82 |
| atom | 2764 | CA  | ASP | 359 | 18.074 | -5.977 | 29.851 | 1.00 | 39.59 |
| atom | 2765 | CB  | ASP | 359 | 18.331 | -7.487 | 29.776 | 1.00 | 45.28 |
| atom | 2766 | CG  | ASP | 359 | 18.558 | -8.120 | 31.136 | 1.00 | 48.29 |
| atom | 2767 | OD1 | ASP | 359 | 18.876 | -7.395 | 32.103 | 1.00 | 55.92 |
| atom | 2768 | OD2 | ASP | 359 | 18.426 | -9.355 | 31.239 | 1.00 | 49.53 |

| atom | 2769 | C   | ASP | 359 | 19.324 | -5.228 | 29.385 | 1.00 | 38.72 |
|------|------|-----|-----|-----|--------|--------|--------|------|-------|
| atom | 2770 | 0   | ASP | 359 | 20.122 | -5.763 | 28.607 | 1.00 | 39.87 |
| atom | 2771 | N   | LEU | 360 | 19.476 | -3.994 | 29.857 | 1.00 | 36.52 |
| atom | 2772 | CA  | LEU | 360 | 20.603 | -3.129 | 29.505 | 1.00 | 36.77 |
| atom | 2773 | CB  | LEU | 360 | 20.901 | -2.161 | 30.651 | 1.00 | 34.29 |
| atom | 2774 | CG  | LEU | 360 | 22.143 | -1.272 | 30.465 | 1.00 | 37.55 |
| atom | 2775 | CD1 | LEU | 360 | 21.973 | -0.372 | 29.248 | 1.00 | 29.93 |
| atom | 2776 | CD2 | LEU | 360 | 22.365 | -0.420 | 31.727 | 1.00 | 36.42 |
| atom | 2777 | C   | LEU | 360 | 21.875 | -3.882 | 29.146 | 1.00 | 36.90 |
| atom | 2778 | 0   | LEU | 360 | 22.551 | -3.552 | 28.177 | 1.00 | 38.17 |
| atom | 2779 | N   | GLU | 361 | 22.194 | -4.893 | 29.942 | 1.00 | 37.83 |
| atom | 2780 | CA  | GLU | 361 | 23.377 | -5.717 | 29.744 | 1.00 | 35.87 |
| atom | 2781 | CB  | GLU | 361 | 23.416 | -6.785 | 30.849 | 1.00 | 34.29 |
| atom | 2782 | CG  | GLU | 361 | 24.650 | -7.694 | 30.868 | 1.00 | 32.02 |
| atom | 2783 | CD  | GLU | 361 | 24.779 | -8.475 | 32.178 | 1.00 | 36.91 |
| atom | 2784 | OE1 | GLU | 361 | 24.869 | -9.725 | 32.113 | 1.00 | 36.54 |
| atom | 2785 | OE2 | GLU | 361 | 24.789 | -7.842 | 33.271 | 1.00 | 34.86 |
| atom | 2786 | C   | GLU | 361 | 23.415 | -6.384 | 28.355 | 1.00 | 37.17 |
| atom | 2787 | 0   | GLU | 361 | 24.481 | -6.547 | 27.769 | 1.00 | 37.31 |
| atom | 2788 | N   | LEU | 362 | 22.254 | -6.756 | 27.826 | 1.00 | 39.56 |
| atom | 2789 | CA  | LEU | 362 | 22.195 | -7.429 | 26.529 | 1.00 | 40.91 |
| atom | 2790 | CB  | LEU | 362 | 20.932 | -8.298 | 26.442 | 1.00 | 42.13 |
| atom | 2791 | CG  | LEU | 362 | 21.081 | -9.614 | 27.229 | 1.00 | 43.71 |
| atom | 2792 | CD1 | LEU | 362 | 20.850 | -9.356 | 28.708 | 1.00 | 43.86 |
| atom | 2793 | CD2 | LEU | 362 | 20.106 | -10.654 | 26.729 | 1.00 | 42.44 |
| atom | 2794 | C   | LEU | 362 | 22.279 | -6.497 | 25.329 | 1.00 | 38.74 |
| atom | 2795 | 0   | LEU | 362 | 22.270 | -6.945 | 24.189 | 1.00 | 34.53 |
| atom | 2796 | N   | ILE | 363 | 22.375 | -5.199 | 25.588 | 1.00 | 35.68 |
| atom | 2797 | CA  | ILE | 363 | 22.493 | -4.246 | 24.507 | 1.00 | 33.38 |
| atom | 2798 | CB  | ILE | 363 | 21.907 | -2.870 | 24.889 | 1.00 | 31.46 |
| atom | 2799 | CG2 | ILE | 363 | 22.167 | -1.849 | 23.766 | 1.00 | 25.00 |
| atom | 2800 | CG1 | ILE | 363 | 20.406 | -3.015 | 25.153 | 1.00 | 33.04 |
| atom | 2801 | CD1 | ILE | 363 | 19.783 | -1.853 | 25.910 | 1.00 | 32.87 |
| atom | 2802 | C   | ILE | 363 | 23.966 | -4.082 | 24.202 | 1.00 | 33.97 |
| atom | 2803 | 0   | ILE | 363 | 24.668 | -3.379 | 24.922 | 1.00 | 35.19 |
| atom | 2804 | N   | THR | 364 | 24.453 | -4.739 | 23.156 | 1.00 | 36.06 |

| atom | 2805 | CA | THR | 364 | 25.860 | -4.580 | 22.825 | 1.00 | 38.67 |
|------|------|-----|-----|-----|--------|--------|--------|------|-------|
| atom | 2806 | CB | THR | 364 | 26.496 | -5.893 | 22.330 | 1.00 | 38.78 |
| atom | 2807 | OG1 | THR | 364 | 27.191 | -5.659 | 21.100 | 1.00 | 39.96 |
| atom | 2808 | CG2 | THR | 364 | 25.436 | -6.964 | 22.146 | 1.00 | 42.76 |
| atom | 2809 | C | THR | 364 | 25.946 | -3.501 | 21.760 | 1.00 | 41.16 |
| atom | 2810 | O | THR | 364 | 25.108 | -3.444 | 20.865 | 1.00 | 42.45 |
| atom | 2811 | N | SER | 365 | 26.940 | -2.624 | 21.889 | 1.00 | 43.05 |
| atom | 2812 | CA | SER | 365 | 27.134 | -1.515 | 20.963 | 1.00 | 43.64 |
| atom | 2813 | CB | SER | 365 | 26.338 | -0.298 | 21.435 | 1.00 | 43.29 |
| atom | 2814 | OG | SER | 365 | 27.194 | 0.807 | 21.650 | 1.00 | 47.26 |
| atom | 2815 | C | SER | 365 | 28.616 | -1.176 | 20.899 | 1.00 | 44.44 |
| atom | 2816 | O | SER | 365 | 29.278 | -1.050 | 21.939 | 1.00 | 42.95 |
| atom | 2817 | N | CYS | 366 | 29.121 | -1.014 | 19.676 | 1.00 | 44.33 |
| atom | 2818 | CA | CYS | 366 | 30.535 | -0.734 | 19.444 | 1.00 | 41.74 |
| atom | 2819 | CB | CYS | 366 | 31.041 | 0.377 | 20.363 | 1.00 | 42.48 |
| atom | 2820 | SG | CYS | 366 | 30.515 | 2.037 | 19.903 | 1.00 | 37.51 |
| atom | 2821 | C | CYS | 366 | 31.250 | -2.036 | 19.773 | 1.00 | 42.11 |
| atom | 2822 | O | CYS | 366 | 32.321 | -2.052 | 20.392 | 1.00 | 38.89 |
| atom | 2823 | N | SER | 367 | 30.617 | -3.128 | 19.361 | 1.00 | 41.96 |
| atom | 2824 | CA | SER | 367 | 31.134 | -4.466 | 19.589 | 1.00 | 45.83 |
| atom | 2825 | CB | SER | 367 | 32.423 | -4.656 | 18.791 | 1.00 | 47.19 |
| atom | 2826 | OG | SER | 367 | 32.141 | -5.257 | 17.537 | 1.00 | 53.00 |
| atom | 2827 | C | SER | 367 | 31.375 | -4.789 | 21.071 | 1.00 | 45.17 |
| atom | 2828 | O | SER | 367 | 32.167 | -5.686 | 21.381 | 1.00 | 41.42 |
| atom | 2829 | N | SER | 368 | 30.687 | -4.070 | 21.967 | 1.00 | 42.35 |
| atom | 2830 | CA | SER | 368 | 30.829 | -4.268 | 23.418 | 1.00 | 41.58 |
| atom | 2831 | CB | SER | 368 | 31.988 | -3.428 | 23.952 | 1.00 | 38.01 |
| atom | 2832 | OG | SER | 368 | 31.614 | -2.077 | 24.035 | 1.00 | 35.59 |
| atom | 2833 | C | SER | 368 | 29.554 | -3.930 | 24.193 | 1.00 | 39.93 |
| atom | 2834 | O | SER | 368 | 28.644 | -3.323 | 23.648 | 1.00 | 45.35 |
| atom | 2835 | N | ASN | 369 | 29.489 | -4.315 | 25.465 | 1.00 | 37.24 |
| atom | 2836 | CA | ASN | 369 | 28.292 | -4.064 | 26.275 | 1.00 | 36.60 |
| atom | 2837 | CB | ASN | 369 | 27.423 | -5.307 | 26.268 | 1.00 | 31.85 |
| atom | 2838 | CG | ASN | 369 | 28.114 | -6.459 | 26.917 | 1.00 | 35.41 |
| atom | 2839 | OD1 | ASN | 369 | 29.050 | -7.021 | 26.344 | 1.00 | 29.53 |
| atom | 2840 | ND2 | ASN | 369 | 27.692 | -6.807 | 28.136 | 1.00 | 31.65 |

| atom | 2841 | C   | ASN | 369 | 28.537 | -3.650 | 27.737 | 1.00 | 30.94 |
| atom | 2842 | O   | ASN | 369 | 29.665 | -3.589 | 28.203 | 1.00 | 34.12 |
| atom | 2843 | N   | VAL | 370 | 27.453 | -3.381 | 28.453 | 1.00 | 30.55 |
| atom | 2844 | CA  | VAL | 370 | 27.508 | -2.948 | 29.850 | 1.00 | 24.47 |
| atom | 2845 | CB  | VAL | 370 | 26.425 | -1.882 | 30.166 | 1.00 | 27.62 |
| atom | 2846 | CG1 | VAL | 370 | 26.311 | -1.705 | 31.672 | 1.00 | 30.91 |
| atom | 2847 | CG2 | VAL | 370 | 26.758 | -0.549 | 29.495 | 1.00 | 24.11 |
| atom | 2848 | C   | VAL | 370 | 27.248 | -4.109 | 30.776 | 1.00 | 23.63 |
| atom | 2849 | O   | VAL | 370 | 26.241 | -4.799 | 30.640 | 1.00 | 22.44 |
| atom | 2850 | N   | SER | 371 | 28.136 | -4.313 | 31.739 | 1.00 | 22.16 |
| atom | 2851 | CA  | SER | 371 | 27.956 | -5.410 | 32.676 | 1.00 | 22.77 |
| atom | 2852 | CB  | SER | 371 | 28.886 | -6.575 | 32.309 | 1.00 | 24.91 |
| atom | 2853 | OG  | SER | 371 | 28.557 | -7.746 | 33.040 | 1.00 | 22.94 |
| atom | 2854 | C   | SER | 371 | 28.214 | -4.976 | 34.121 | 1.00 | 22.34 |
| atom | 2855 | O   | SER | 371 | 28.838 | -3.932 | 34.384 | 1.00 | 20.05 |
| atom | 2856 | N   | VAL | 372 | 27.745 | -5.800 | 35.056 | 1.00 | 21.56 |
| atom | 2857 | CA  | VAL | 372 | 27.898 | -5.509 | 36.473 | 1.00 | 15.57 |
| atom | 2858 | CB  | VAL | 372 | 26.561 | -5.566 | 37.207 | 1.00 | 19.67 |
| atom | 2859 | CG1 | VAL | 372 | 26.720 | -4.920 | 38.592 | 1.00 | 26.20 |
| atom | 2860 | CG2 | VAL | 372 | 25.471 | -4.875 | 36.402 | 1.00 | 10.49 |
| atom | 2861 | C   | VAL | 372 | 28.832 | -6.431 | 37.221 | 1.00 | 15.58 |
| atom | 2862 | O   | VAL | 372 | 28.930 | -7.627 | 36.922 | 1.00 | 14.13 |
| atom | 2863 | N   | ALA | 373 | 29.520 | -5.845 | 38.196 | 1.00 | 16.39 |
| atom | 2864 | CA  | ALA | 373 | 30.438 | -6.563 | 39.074 | 1.00 | 18.31 |
| atom | 2865 | CB  | ALA | 373 | 31.826 | -6.545 | 38.495 | 1.00 | 16.15 |
| atom | 2866 | C   | ALA | 373 | 30.416 | -5.854 | 40.438 | 1.00 | 20.53 |
| atom | 2867 | O   | ALA | 373 | 29.603 | -4.963 | 40.667 | 1.00 | 26.41 |
| atom | 2868 | N   | HIS | 374 | 31.302 | -6.233 | 41.347 | 1.00 | 23.28 |
| atom | 2869 | CA  | HIS | 374 | 31.333 | -5.589 | 42.654 | 1.00 | 21.68 |
| atom | 2870 | CB  | HIS | 374 | 30.696 | -6.495 | 43.685 | 1.00 | 23.76 |
| atom | 2871 | CG  | HIS | 374 | 29.232 | -6.653 | 43.487 | 1.00 | 23.91 |
| atom | 2872 | CD2 | HIS | 374 | 28.196 | -5.868 | 43.863 | 1.00 | 25.69 |
| atom | 2873 | ND1 | HIS | 374 | 28.694 | -7.672 | 42.732 | 1.00 | 26.95 |
| atom | 2874 | CE1 | HIS | 374 | 27.386 | -7.507 | 42.650 | 1.00 | 29.89 |
| atom | 2875 | NE2 | HIS | 374 | 27.058 | -6.420 | 43.327 | 1.00 | 29.47 |
| atom | 2876 | C   | HIS | 374 | 32.732 | -5.231 | 43.070 | 1.00 | 21.80 |

| atom | 2877 | O   | HIS | 374 | 33.660 | -6.002 | 42.859 | 1.00 | 21.70 |
| atom | 2878 | N   | ASP | 375 | 32.893 | -4.064 | 43.675 | 1.00 | 24.44 |
| atom | 2879 | CA  | ASP | 375 | 34.228 | -3.644 | 44.070 | 1.00 | 28.87 |
| atom | 2880 | CB  | ASP | 375 | 34.365 | -2.128 | 43.963 | 1.00 | 26.72 |
| atom | 2881 | CG  | ASP | 375 | 34.061 | -1.422 | 45.274 | 1.00 | 35.28 |
| atom | 2882 | OD1 | ASP | 375 | 32.918 | -1.530 | 45.763 | 1.00 | 34.88 |
| atom | 2883 | OD2 | ASP | 375 | 34.971 | -0.762 | 45.820 | 1.00 | 41.21 |
| atom | 2884 | C   | ASP | 375 | 34.624 | -4.112 | 45.471 | 1.00 | 32.70 |
| atom | 2885 | O   | ASP | 375 | 33.977 | -4.977 | 46.062 | 1.00 | 31.75 |
| atom | 2886 | N   | ALA | 376 | 35.724 | -3.549 | 45.961 | 1.00 | 37.02 |
| atom | 2887 | CA  | ALA | 376 | 36.280 | -3.854 | 47.268 | 1.00 | 36.04 |
| atom | 2888 | CB  | ALA | 376 | 37.426 | -2.890 | 47.558 | 1.00 | 35.93 |
| atom | 2889 | C   | ALA | 376 | 35.227 | -3.765 | 48.368 | 1.00 | 38.47 |
| atom | 2890 | O   | ALA | 376 | 35.063 | -4.700 | 49.158 | 1.00 | 40.65 |
| atom | 2891 | N   | SER | 377 | 34.522 | -2.636 | 48.418 | 1.00 | 36.68 |
| atom | 2892 | CA  | SER | 377 | 33.486 | -2.408 | 49.421 | 1.00 | 36.49 |
| atom | 2893 | CB  | SER | 377 | 33.243 | -0.906 | 49.597 | 1.00 | 37.48 |
| atom | 2894 | OG  | SER | 377 | 32.235 | -0.446 | 48.709 | 1.00 | 39.42 |
| atom | 2895 | C   | SER | 377 | 32.174 | -3.088 | 49.060 | 1.00 | 35.46 |
| atom | 2896 | O   | SER | 377 | 31.138 | -2.804 | 49.655 | 1.00 | 40.61 |
| atom | 2897 | N   | GLY | 378 | 32.218 | -3.968 | 48.071 | 1.00 | 31.94 |
| atom | 2898 | CA  | GLY | 378 | 31.031 | -4.694 | 47.656 | 1.00 | 30.54 |
| atom | 2899 | C   | GLY | 378 | 29.927 | -3.897 | 46.985 | 1.00 | 29.00 |
| atom | 2900 | O   | GLY | 378 | 28.841 | -4.407 | 46.741 | 1.00 | 29.96 |
| atom | 2901 | N   | LYS | 379 | 30.181 | -2.639 | 46.686 | 1.00 | 31.48 |
| atom | 2902 | CA  | LYS | 379 | 29.157 | -1.850 | 46.036 | 1.00 | 35.92 |
| atom | 2903 | CB  | LYS | 379 | 29.458 | -0.361 | 46.194 | 1.00 | 38.88 |
| atom | 2904 | CG  | LYS | 379 | 28.219 | 0.535  | 46.092 | 1.00 | 46.72 |
| atom | 2905 | CD  | LYS | 379 | 28.395 | 1.885  | 46.812 | 1.00 | 49.32 |
| atom | 2906 | CE  | LYS | 379 | 29.863 | 2.313  | 46.891 | 1.00 | 52.18 |
| atom | 2907 | NZ  | LYS | 379 | 30.492 | 1.882  | 48.176 | 1.00 | 50.19 |
| atom | 2908 | C   | LYS | 379 | 29.128 | -2.234 | 44.555 | 1.00 | 36.66 |
| atom | 2909 | O   | LYS | 379 | 30.180 | -2.461 | 43.931 | 1.00 | 33.38 |
| atom | 2910 | N   | ARG | 380 | 27.923 | -2.320 | 44.001 | 1.00 | 33.24 |
| atom | 2911 | CA  | ARG | 380 | 27.769 | -2.663 | 42.598 | 1.00 | 31.43 |
| atom | 2912 | CB  | ARG | 380 | 26.283 | -2.688 | 42.216 | 1.00 | 28.74 |

| atom | 2913 | CG | ARG | 380 | 25.776 | -4.105 | 41.995 | 1.00 | 31.92 |
|------|------|-----|-----|-----|--------|--------|--------|------|-------|
| atom | 2914 | CD | ARG | 380 | 24.294 | -4.180 | 41.834 | 1.00 | 29.13 |
| atom | 2915 | NE | ARG | 380 | 23.707 | -2.877 | 41.580 | 1.00 | 37.71 |
| atom | 2916 | CZ | ARG | 380 | 23.446 | -2.397 | 40.370 | 1.00 | 44.95 |
| atom | 2917 | NH1 | ARG | 380 | 23.727 | -3.122 | 39.291 | 1.00 | 44.04 |
| atom | 2918 | NH2 | ARG | 380 | 22.876 | -1.198 | 40.239 | 1.00 | 46.73 |
| atom | 2919 | C | ARG | 380 | 28.514 | -1.682 | 41.700 | 1.00 | 30.44 |
| atom | 2920 | O | ARG | 380 | 28.505 | -0.480 | 41.941 | 1.00 | 30.65 |
| atom | 2921 | N | VAL | 381 | 29.185 | -2.207 | 40.680 | 1.00 | 28.65 |
| atom | 2922 | CA | VAL | 381 | 29.908 | -1.366 | 39.736 | 1.00 | 25.65 |
| atom | 2923 | CB | VAL | 381 | 31.431 | -1.334 | 40.028 | 1.00 | 24.97 |
| atom | 2924 | CG1 | VAL | 381 | 32.146 | -0.472 | 38.976 | 1.00 | 27.18 |
| atom | 2925 | CG2 | VAL | 381 | 31.683 | -0.758 | 41.417 | 1.00 | 21.96 |
| atom | 2926 | C | VAL | 381 | 29.671 | -1.827 | 38.289 | 1.00 | 25.25 |
| atom | 2927 | O | VAL | 381 | 29.862 | -3.003 | 37.927 | 1.00 | 24.76 |
| atom | 2928 | N | TYR | 382 | 29.229 | -0.878 | 37.477 | 1.00 | 20.70 |
| atom | 2929 | CA | TYR | 382 | 28.960 | -1.113 | 36.069 | 1.00 | 22.49 |
| atom | 2930 | CB | TYR | 382 | 27.831 | -0.198 | 35.603 | 1.00 | 19.31 |
| atom | 2931 | CG | TYR | 382 | 26.470 | -0.566 | 36.127 | 1.00 | 19.83 |
| atom | 2932 | CD1 | TYR | 382 | 25.954 | 0.048 | 37.275 | 1.00 | 23.95 |
| atom | 2933 | CE1 | TYR | 382 | 24.647 | -0.207 | 37.707 | 1.00 | 23.01 |
| atom | 2934 | CD2 | TYR | 382 | 25.653 | -1.453 | 35.428 | 1.00 | 15.52 |
| atom | 2935 | CE2 | TYR | 382 | 24.348 | -1.718 | 35.841 | 1.00 | 16.17 |
| atom | 2936 | CZ | TYR | 382 | 23.847 | -1.083 | 36.980 | 1.00 | 24.06 |
| atom | 2937 | OH | TYR | 382 | 22.539 | -1.289 | 37.367 | 1.00 | 27.97 |
| atom | 2938 | C | TYR | 382 | 30.222 | -0.797 | 35.268 | 1.00 | 20.28 |
| atom | 2939 | O | TYR | 382 | 30.849 | 0.228 | 35.494 | 1.00 | 17.61 |
| atom | 2940 | N | TYR | 383 | 30.577 | -1.679 | 34.333 | 1.00 | 22.04 |
| atom | 2941 | CA | TYR | 383 | 31.759 | -1.498 | 33.480 | 1.00 | 17.05 |
| atom | 2942 | CB | TYR | 383 | 32.930 | -2.279 | 34.066 | 1.00 | 14.01 |
| atom | 2943 | CG | TYR | 383 | 32.781 | -3.782 | 33.968 | 1.00 | 15.16 |
| atom | 2944 | CD1 | TYR | 383 | 31.806 | -4.463 | 34.725 | 1.00 | 12.97 |
| atom | 2945 | CE1 | TYR | 383 | 31.650 | -5.861 | 34.630 | 1.00 | 6.21 |
| atom | 2946 | CD2 | TYR | 383 | 33.608 | -4.536 | 33.111 | 1.00 | 9.83 |
| atom | 2947 | CE2 | TYR | 383 | 33.461 | -5.920 | 33.013 | 1.00 | 7.71 |
| atom | 2948 | CZ | TYR | 383 | 32.482 | -6.570 | 33.769 | 1.00 | 10.71 |

| atom | 2949 | OH | TYR | 383 | 32.324 | -7.918 | 33.628 | 1.00 | 17.89 |
| atom | 2950 | C | TYR | 383 | 31.539 | -1.936 | 32.006 | 1.00 | 18.86 |
| atom | 2951 | O | TYR | 383 | 30.604 | -2.676 | 31.682 | 1.00 | 9.75 |
| atom | 2952 | N | LEU | 384 | 32.428 | -1.468 | 31.133 | 1.00 | 20.41 |
| atom | 2953 | CA | LEU | 384 | 32.387 | -1.761 | 29.700 | 1.00 | 22.72 |
| atom | 2954 | CB | LEU | 384 | 33.089 | -0.653 | 28.906 | 1.00 | 27.28 |
| atom | 2955 | CG | LEU | 384 | 32.206 | 0.373 | 28.189 | 1.00 | 33.06 |
| atom | 2956 | CD1 | LEU | 384 | 33.065 | 1.244 | 27.233 | 1.00 | 34.76 |
| atom | 2957 | CD2 | LEU | 384 | 31.112 | -0.361 | 27.442 | 1.00 | 21.43 |
| atom | 2958 | C | LEU | 384 | 33.091 | -3.066 | 29.392 | 1.00 | 23.29 |
| atom | 2959 | O | LEU | 384 | 34.285 | -3.224 | 29.672 | 1.00 | 16.65 |
| atom | 2960 | N | THR | 385 | 32.368 | -4.002 | 28.803 | 1.00 | 20.42 |
| atom | 2961 | CA | THR | 385 | 33.006 | -5.257 | 28.488 | 1.00 | 25.24 |
| atom | 2962 | CB | THR | 385 | 32.725 | -6.312 | 29.566 | 1.00 | 24.34 |
| atom | 2963 | OG1 | THR | 385 | 33.725 | -7.341 | 29.500 | 1.00 | 25.86 |
| atom | 2964 | CG2 | THR | 385 | 31.365 | -6.920 | 29.353 | 1.00 | 25.15 |
| atom | 2965 | C | THR | 385 | 32.625 | -5.803 | 27.115 | 1.00 | 28.01 |
| atom | 2966 | O | THR | 385 | 31.850 | -5.190 | 26.371 | 1.00 | 27.17 |
| atom | 2967 | N | ARG | 386 | 33.151 | -6.976 | 26.788 | 1.00 | 29.12 |
| atom | 2968 | CA | ARG | 386 | 32.886 | -7.513 | 25.479 | 1.00 | 27.83 |
| atom | 2969 | CB | ARG | 386 | 33.539 | -6.542 | 24.501 | 1.00 | 27.61 |
| atom | 2970 | CG | ARG | 386 | 33.829 | -7.097 | 23.168 | 1.00 | 31.30 |
| atom | 2971 | CD | ARG | 386 | 35.308 | -7.124 | 22.857 | 1.00 | 23.16 |
| atom | 2972 | NE | ARG | 386 | 35.409 | -7.801 | 21.577 | 1.00 | 16.35 |
| atom | 2973 | CZ | ARG | 386 | 35.558 | -7.169 | 20.428 | 1.00 | 12.96 |
| atom | 2974 | NH1 | ARG | 386 | 35.634 | -5.839 | 20.419 | 1.00 | 12.85 |
| atom | 2975 | NH2 | ARG | 386 | 35.542 | -7.860 | 19.294 | 1.00 | 15.28 |
| atom | 2976 | C | ARG | 386 | 33.418 | -8.940 | 25.299 | 1.00 | 27.06 |
| atom | 2977 | O | ARG | 386 | 34.192 | -9.428 | 26.117 | 1.00 | 25.84 |
| atom | 2978 | N | ASP | 387 | 32.968 | -9.613 | 24.242 | 1.00 | 27.53 |
| atom | 2979 | CA | ASP | 387 | 33.450 | -10.958 | 23.924 | 1.00 | 24.59 |
| atom | 2980 | CB | ASP | 387 | 32.841 | -11.422 | 22.615 | 1.00 | 24.42 |
| atom | 2981 | CG | ASP | 387 | 33.009 | -12.888 | 22.400 | 1.00 | 22.95 |
| atom | 2982 | OD1 | ASP | 387 | 32.016 | -13.604 | 22.563 | 1.00 | 23.62 |
| atom | 2983 | OD2 | ASP | 387 | 34.134 | -13.329 | 22.072 | 1.00 | 27.28 |
| atom | 2984 | C | ASP | 387 | 34.966 | -10.785 | 23.765 | 1.00 | 23.96 |

| atom | 2985 | 0   | ASP | 387 | 35.424 | -9.879  | 23.071 | 1.00 | 22.34 |
|------|------|-----|-----|-----|--------|---------|--------|------|-------|
| atom | 2986 | N   | PRO | 388 | 35.764 | -11.665 | 24.379 | 1.00 | 19.21 |
| atom | 2987 | CD  | PRO | 388 | 35.473 | -12.838 | 25.222 | 1.00 | 20.90 |
| atom | 2988 | CA  | PRO | 388 | 37.199 | -11.474 | 24.242 | 1.00 | 20.36 |
| atom | 2989 | CB  | PRO | 388 | 37.714 | -11.909 | 25.611 | 1.00 | 15.78 |
| atom | 2990 | CG  | PRO | 388 | 36.797 | -13.039 | 25.993 | 1.00 | 10.82 |
| atom | 2991 | C   | PRO | 388 | 37.897 | -12.209 | 23.107 | 1.00 | 22.20 |
| atom | 2992 | 0   | PRO | 388 | 39.105 | -12.068 | 22.936 | 1.00 | 27.27 |
| atom | 2993 | N   | THR | 389 | 37.147 | -12.986 | 22.341 | 1.00 | 21.30 |
| atom | 2994 | CA  | THR | 389 | 37.707 | -13.756 | 21.233 | 1.00 | 20.33 |
| atom | 2995 | CB  | THR | 389 | 36.572 | -14.319 | 20.365 | 1.00 | 22.95 |
| atom | 2996 | OG1 | THR | 389 | 35.905 | -15.371 | 21.074 | 1.00 | 16.21 |
| atom | 2997 | CG2 | THR | 389 | 37.116 | -14.857 | 19.059 | 1.00 | 24.76 |
| atom | 2998 | C   | THR | 389 | 38.714 | -13.017 | 20.333 | 1.00 | 21.29 |
| atom | 2999 | 0   | THR | 389 | 39.837 | -13.472 | 20.135 | 1.00 | 25.30 |
| atom | 3000 | N   | THR | 390 | 38.314 | -11.879 | 19.790 | 1.00 | 21.06 |
| atom | 3001 | CA  | THR | 390 | 39.189 | -11.112 | 18.907 | 1.00 | 20.45 |
| atom | 3002 | CB  | THR | 390 | 38.366 | -9.966  | 18.182 | 1.00 | 22.74 |
| atom | 3003 | OG1 | THR | 390 | 37.404 | -10.571 | 17.309 | 1.00 | 15.53 |
| atom | 3004 | CG2 | THR | 390 | 39.276 | -9.031  | 17.364 | 1.00 | 9.68  |
| atom | 3005 | C   | THR | 390 | 40.424 | -10.547 | 19.625 | 1.00 | 21.65 |
| atom | 3006 | 0   | THR | 390 | 41.555 | -10.778 | 19.192 | 1.00 | 26.77 |
| atom | 3007 | N   | PRO | 391 | 40.232 | -9.781  | 20.711 | 1.00 | 20.31 |
| atom | 3008 | CD  | PRO | 391 | 38.970 | -9.345  | 21.336 | 1.00 | 24.13 |
| atom | 3009 | CA  | PRO | 391 | 41.405 | -9.241  | 21.410 | 1.00 | 18.82 |
| atom | 3010 | CB  | PRO | 391 | 40.820 | -8.613  | 22.671 | 1.00 | 19.00 |
| atom | 3011 | CG  | PRO | 391 | 39.420 | -8.283  | 22.309 | 1.00 | 22.38 |
| atom | 3012 | C   | PRO | 391 | 42.391 | -10.359 | 21.751 | 1.00 | 19.91 |
| atom | 3013 | 0   | PRO | 391 | 43.597 | -10.187 | 21.684 | 1.00 | 21.23 |
| atom | 3014 | N   | LEU | 392 | 41.856 | -11.511 | 22.127 | 1.00 | 20.70 |
| atom | 3015 | CA  | LEU | 392 | 42.692 | -12.650 | 22.458 | 1.00 | 20.82 |
| atom | 3016 | CB  | LEU | 392 | 41.841 | -13.759 | 23.073 | 1.00 | 17.55 |
| atom | 3017 | CG  | LEU | 392 | 41.345 | -13.392 | 24.478 | 1.00 | 21.46 |
| atom | 3018 | CD1 | LEU | 392 | 40.825 | -14.630 | 25.205 | 1.00 | 15.26 |
| atom | 3019 | CD2 | LEU | 392 | 42.485 | -12.737 | 25.250 | 1.00 | 15.90 |
| atom | 3020 | C   | LEU | 392 | 43.416 | -13.148 | 21.204 | 1.00 | 24.09 |

| atom | 3021 | O | LEU | 392 | 44.650 | -13.216 | 21.188 | 1.00 | 23.77 |
|------|------|------|------|------|--------|---------|--------|------|-------|
| atom | 3022 | N | ALA | 393 | 42.664 | -13.472 | 20.151 | 1.00 | 17.72 |
| atom | 3023 | CA | ALA | 393 | 43.297 | -13.951 | 18.936 | 1.00 | 16.17 |
| atom | 3024 | CB | ALA | 393 | 42.258 | -14.247 | 17.869 | 1.00 | 22.26 |
| atom | 3025 | C | ALA | 393 | 44.293 | -12.917 | 18.436 | 1.00 | 12.90 |
| atom | 3026 | O | ALA | 393 | 45.385 | -13.261 | 18.017 | 1.00 | 13.43 |
| atom | 3027 | N | ARG | 394 | 43.931 | -11.645 | 18.489 | 1.00 | 14.42 |
| atom | 3028 | CA | ARG | 394 | 44.846 | -10.624 | 18.021 | 1.00 | 17.59 |
| atom | 3029 | CB | ARG | 394 | 44.150 | -9.256 | 17.943 | 1.00 | 14.82 |
| atom | 3030 | CG | ARG | 394 | 42.984 | -9.215 | 16.962 | 1.00 | 16.07 |
| atom | 3031 | CD | ARG | 394 | 42.631 | -7.760 | 16.526 | 1.00 | 12.41 |
| atom | 3032 | NE | ARG | 394 | 41.684 | -7.773 | 15.413 | 1.00 | 14.24 |
| atom | 3033 | CZ | ARG | 394 | 40.850 | -6.787 | 15.087 | 1.00 | 17.39 |
| atom | 3034 | NH1 | ARG | 394 | 40.817 | -5.667 | 15.781 | 1.00 | 10.53 |
| atom | 3035 | NH2 | ARG | 394 | 40.019 | -6.940 | 14.066 | 1.00 | 20.76 |
| atom | 3036 | C | ARG | 394 | 46.004 | -10.594 | 19.016 | 1.00 | 22.24 |
| atom | 3037 | O | ARG | 394 | 47.155 | -10.344 | 18.655 | 1.00 | 26.32 |
| atom | 3038 | N | ALA | 395 | 45.685 | -10.866 | 20.274 | 1.00 | 24.17 |
| atom | 3039 | CA | ALA | 395 | 46.683 | -10.893 | 21.330 | 1.00 | 22.92 |
| atom | 3040 | CB | ALA | 395 | 46.023 | -11.255 | 22.648 | 1.00 | 21.94 |
| atom | 3041 | C | ALA | 395 | 47.737 | -11.929 | 20.955 | 1.00 | 21.60 |
| atom | 3042 | O | ALA | 395 | 48.914 | -11.617 | 20.853 | 1.00 | 26.01 |
| atom | 3043 | N | ALA | 396 | 47.310 | -13.161 | 20.731 | 1.00 | 17.87 |
| atom | 3044 | CA | ALA | 396 | 48.241 | -14.216 | 20.349 | 1.00 | 20.24 |
| atom | 3045 | CB | ALA | 396 | 47.456 | -15.498 | 20.012 | 1.00 | 14.04 |
| atom | 3046 | C | ALA | 396 | 49.138 | -13.809 | 19.158 | 1.00 | 21.01 |
| atom | 3047 | O | ALA | 396 | 50.365 | -13.943 | 19.201 | 1.00 | 22.33 |
| atom | 3048 | N | TRP | 397 | 48.510 | -13.311 | 18.100 | 1.00 | 23.99 |
| atom | 3049 | CA | TRP | 397 | 49.211 | -12.899 | 16.895 | 1.00 | 24.01 |
| atom | 3050 | CB | TRP | 397 | 48.224 | -12.242 | 15.910 | 1.00 | 26.48 |
| atom | 3051 | CG | TRP | 397 | 48.767 | -12.062 | 14.503 | 1.00 | 21.25 |
| atom | 3052 | CD2 | TRP | 397 | 48.534 | -12.918 | 13.380 | 1.00 | 18.44 |
| atom | 3053 | CE2 | TRP | 397 | 49.284 | -12.406 | 12.295 | 1.00 | 21.16 |
| atom | 3054 | CE3 | TRP | 397 | 47.767 | -14.068 | 13.183 | 1.00 | 20.72 |
| atom | 3055 | CD1 | TRP | 397 | 49.621 | -11.083 | 14.067 | 1.00 | 24.22 |
| atom | 3056 | NE1 | TRP | 397 | 49.936 | -11.286 | 12.739 | 1.00 | 26.31 |

| atom | 3057 | CZ2 | TRP | 397 | 49.289 | -13.010 | 11.037 | 1.00 | 20.85 |
|------|------|-----|-----|-----|--------|---------|--------|------|-------|
| atom | 3058 | CZ3 | TRP | 397 | 47.771 | -14.672 | 11.924 | 1.00 | 21.73 |
| atom | 3059 | CH2 | TRP | 397 | 48.528 | -14.140 | 10.872 | 1.00 | 22.07 |
| atom | 3060 | C | TRP | 397 | 50.347 | -11.933 | 17.192 | 1.00 | 26.70 |
| atom | 3061 | O | TRP | 397 | 51.503 | -12.193 | 16.868 | 1.00 | 28.31 |
| atom | 3062 | N | GLU | 398 | 50.017 | -10.812 | 17.811 | 1.00 | 26.37 |
| atom | 3063 | CA | GLU | 398 | 51.020 | -9.806 | 18.112 | 1.00 | 27.65 |
| atom | 3064 | CB | GLU | 398 | 50.315 | -8.560 | 18.657 | 1.00 | 28.99 |
| atom | 3065 | CG | GLU | 398 | 49.578 | -7.787 | 17.567 | 1.00 | 18.59 |
| atom | 3066 | CD | GLU | 398 | 48.272 | -7.200 | 18.038 | 1.00 | 27.32 |
| atom | 3067 | OE1 | GLU | 398 | 47.227 | -7.608 | 17.484 | 1.00 | 24.97 |
| atom | 3068 | OE2 | GLU | 398 | 48.294 | -6.338 | 18.953 | 1.00 | 22.57 |
| atom | 3069 | C | GLU | 398 | 52.159 | -10.274 | 19.032 | 1.00 | 30.21 |
| atom | 3070 | O | GLU | 398 | 53.148 | -9.560 | 19.213 | 1.00 | 31.40 |
| atom | 3071 | N | THR | 399 | 52.014 | -11.475 | 19.599 | 1.00 | 31.36 |
| atom | 3072 | CA | THR | 399 | 53.037 | -12.088 | 20.452 | 1.00 | 28.89 |
| atom | 3073 | CB | THR | 399 | 52.398 | -12.967 | 21.550 | 1.00 | 28.74 |
| atom | 3074 | OG1 | THR | 399 | 51.402 | -12.216 | 22.242 | 1.00 | 28.05 |
| atom | 3075 | CG2 | THR | 399 | 53.439 | -13.418 | 22.545 | 1.00 | 27.81 |
| atom | 3076 | C | THR | 399 | 53.919 | -12.988 | 19.548 | 1.00 | 29.90 |
| atom | 3077 | O | THR | 399 | 55.110 | -13.197 | 19.803 | 1.00 | 21.09 |
| atom | 3078 | N | ALA | 400 | 53.309 | -13.537 | 18.500 | 1.00 | 31.86 |
| atom | 3079 | CA | ALA | 400 | 54.027 | -14.377 | 17.532 | 1.00 | 29.44 |
| atom | 3080 | CB | ALA | 400 | 53.043 | -15.260 | 16.751 | 1.00 | 24.28 |
| atom | 3081 | C | ALA | 400 | 54.805 | -13.499 | 16.558 | 1.00 | 27.37 |
| atom | 3082 | O | ALA | 400 | 55.970 | -13.745 | 16.318 | 1.00 | 28.03 |
| atom | 3083 | N | ARG | 401 | 54.158 | -12.463 | 16.021 | 1.00 | 30.79 |
| atom | 3084 | CA | ARG | 401 | 54.770 | -11.549 | 15.041 | 1.00 | 33.84 |
| atom | 3085 | CB | ARG | 401 | 54.055 | -11.686 | 13.691 | 1.00 | 36.55 |
| atom | 3086 | CG | ARG | 401 | 54.832 | -11.172 | 12.471 | 1.00 | 42.71 |
| atom | 3087 | CD | ARG | 401 | 54.461 | -12.002 | 11.236 | 1.00 | 54.18 |
| atom | 3088 | NE | ARG | 401 | 55.092 | -11.549 | 9.991 | 1.00 | 62.65 |
| atom | 3089 | CZ | ARG | 401 | 54.437 | -11.329 | 8.849 | 1.00 | 63.48 |
| atom | 3090 | NH1 | ARG | 401 | 53.126 | -11.515 | 8.781 | 1.00 | 62.64 |
| atom | 3091 | NH2 | ARG | 401 | 55.093 | -10.928 | 7.768 | 1.00 | 62.06 |
| atom | 3092 | C | ARG | 401 | 54.734 | -10.078 | 15.451 | 1.00 | 35.86 |

| | | | | | | | | | |
|---|---|---|---|---|---|---|---|---|---|
| atom | 3093 | O | ARG | 401 | 53.655 | -9.508 | 15.650 | 1.00 | 36.31 |
| atom | 3094 | N | HIS | 402 | 55.908 | -9.459 | 15.550 | 1.00 | 36.73 |
| atom | 3095 | CA | HIS | 402 | 55.977 | -8.055 | 15.929 | 1.00 | 43.53 |
| atom | 3096 | CB | HIS | 402 | 57.419 | -7.533 | 15.895 | 1.00 | 47.85 |
| atom | 3097 | CG | HIS | 402 | 57.548 | -6.102 | 16.332 | 1.00 | 54.22 |
| atom | 3098 | CD2 | HIS | 402 | 58.535 | -5.456 | 17.001 | 1.00 | 57.20 |
| atom | 3099 | ND1 | HIS | 402 | 56.583 | -5.151 | 16.068 | 1.00 | 56.15 |
| atom | 3100 | CE1 | HIS | 402 | 56.969 | -3.984 | 16.552 | 1.00 | 56.20 |
| atom | 3101 | NE2 | HIS | 402 | 58.151 | -4.140 | 17.123 | 1.00 | 57.51 |
| atom | 3102 | C | HIS | 402 | 55.103 | -7.160 | 15.055 | 1.00 | 43.10 |
| atom | 3103 | O | HIS | 402 | 55.241 | -7.116 | 13.828 | 1.00 | 37.82 |
| atom | 3104 | N | THR | 403 | 54.228 | -6.426 | 15.734 | 1.00 | 44.21 |
| atom | 3105 | CA | THR | 403 | 53.286 | -5.512 | 15.114 | 1.00 | 46.78 |
| atom | 3106 | CB | THR | 403 | 51.872 | -5.967 | 15.444 | 1.00 | 47.77 |
| atom | 3107 | OG1 | THR | 403 | 51.724 | -6.042 | 16.865 | 1.00 | 53.44 |
| atom | 3108 | CG2 | THR | 403 | 51.630 | -7.356 | 14.879 | 1.00 | 45.97 |
| atom | 3109 | C | THR | 403 | 53.549 | -4.099 | 15.655 | 1.00 | 47.61 |
| atom | 3110 | O | THR | 403 | 53.456 | -3.852 | 16.855 | 1.00 | 48.66 |
| atom | 3111 | N | PRO | 404 | 53.870 | -3.149 | 14.769 | 1.00 | 46.68 |
| atom | 3112 | CD | PRO | 404 | 53.955 | -3.250 | 13.304 | 1.00 | 46.25 |
| atom | 3113 | CA | PRO | 404 | 54.147 | -1.790 | 15.232 | 1.00 | 46.27 |
| atom | 3114 | CB | PRO | 404 | 54.578 | -1.057 | 13.961 | 1.00 | 45.29 |
| atom | 3115 | CG | PRO | 404 | 53.928 | -1.812 | 12.871 | 1.00 | 42.84 |
| atom | 3116 | C | PRO | 404 | 52.976 | -1.117 | 15.923 | 1.00 | 45.84 |
| atom | 3117 | O | PRO | 404 | 53.160 | -0.411 | 16.919 | 1.00 | 50.57 |
| atom | 3118 | N | VAL | 405 | 51.776 | -1.312 | 15.384 | 1.00 | 42.54 |
| atom | 3119 | CA | VAL | 405 | 50.579 | -0.724 | 15.981 | 1.00 | 38.63 |
| atom | 3120 | CB | VAL | 405 | 49.803 | 0.128 | 14.966 | 1.00 | 37.25 |
| atom | 3121 | CG1 | VAL | 405 | 48.415 | 0.436 | 15.507 | 1.00 | 35.73 |
| atom | 3122 | CG2 | VAL | 405 | 50.569 | 1.426 | 14.696 | 1.00 | 40.20 |
| atom | 3123 | C | VAL | 405 | 49.700 | -1.852 | 16.487 | 1.00 | 35.69 |
| atom | 3124 | O | VAL | 405 | 48.826 | -2.352 | 15.771 | 1.00 | 35.97 |
| atom | 3125 | N | ASN | 406 | 49.941 | -2.248 | 17.733 | 1.00 | 32.68 |
| atom | 3126 | CA | ASN | 406 | 49.213 | -3.352 | 18.334 | 1.00 | 31.20 |
| atom | 3127 | CB | ASN | 406 | 50.015 | -3.941 | 19.506 | 1.00 | 36.47 |
| atom | 3128 | CG | ASN | 406 | 50.173 | -2.966 | 20.668 | 1.00 | 35.06 |

```
atom   3129  OD1  ASN   406     51.285  -2.715  21.130  1.00 41.22
atom   3130  ND2  ASN   406     49.065  -2.424  21.146  1.00 31.42
atom   3131  C    ASN   406     47.808  -3.032  18.804  1.00 29.41
atom   3132  O    ASN   406     47.365  -1.882  18.785  1.00 26.28
atom   3133  N    SER   407     47.121  -4.081  19.238  1.00 27.69
atom   3134  CA   SER   407     45.767  -3.967  19.737  1.00 26.15
atom   3135  CB   SER   407     44.853  -4.921  18.963  1.00 26.19
atom   3136  OG   SER   407     45.168  -6.272  19.273  1.00 23.64
atom   3137  C    SER   407     45.714  -4.314  21.226  1.00 22.95
atom   3138  O    SER   407     44.863  -3.800  21.955  1.00 20.75
atom   3139  N    TRP   408     46.620  -5.178  21.675  1.00 21.03
atom   3140  CA   TRP   408     46.619  -5.595  23.075  1.00 21.98
atom   3141  CB   TRP   408     47.656  -6.716  23.317  1.00 24.50
atom   3142  CG   TRP   408     49.082  -6.282  23.337  1.00 34.03
atom   3143  CD2  TRP   408     49.810  -5.745  24.453  1.00 38.01
atom   3144  CE2  TRP   408     51.112  -5.440  23.996  1.00 40.00
atom   3145  CE3  TRP   408     49.488  -5.484  25.793  1.00 40.43
atom   3146  CD1  TRP   408     49.947  -6.290  22.286  1.00 39.23
atom   3147  NE1  TRP   408     51.166  -5.783  22.670  1.00 41.55
atom   3148  CZ2  TRP   408     52.096  -4.890  24.829  1.00 38.74
atom   3149  CZ3  TRP   408     50.468  -4.935  26.622  1.00 39.17
atom   3150  CH2  TRP   408     51.756  -4.644  26.132  1.00 38.81
atom   3151  C    TRP   408     46.768  -4.475  24.109  1.00 17.96
atom   3152  O    TRP   408     46.078  -4.492  25.123  1.00 14.08
atom   3153  N    LEU   409     47.641  -3.496  23.867  1.00 18.71
atom   3154  CA   LEU   409     47.797  -2.409  24.821  1.00 20.06
atom   3155  CB   LEU   409     48.926  -1.444  24.406  1.00 20.61
atom   3156  CG   LEU   409     49.414  -0.424  25.474  1.00 18.35
atom   3157  CD1  LEU   409     48.825  -0.708  26.837  1.00  5.22
atom   3158  CD2  LEU   409     50.919  -0.481  25.586  1.00 23.57
atom   3159  C    LEU   409     46.474  -1.656  24.944  1.00 22.79
atom   3160  O    LEU   409     46.007  -1.364  26.053  1.00 29.34
atom   3161  N    GLY   410     45.878  -1.348  23.798  1.00 25.51
atom   3162  CA   GLY   410     44.605  -0.645  23.766  1.00 17.80
atom   3163  C    GLY   410     43.507  -1.458  24.420  1.00 17.47
atom   3164  O    GLY   410     42.665  -0.911  25.130  1.00 20.34
```

| atom | 3165 | N   | ASN | 411 | 43.495 | -2.767 | 24.201 | 1.00 | 14.98 |
|------|------|-----|-----|-----|--------|--------|--------|------|-------|
| atom | 3166 | CA  | ASN | 411 | 42.454 | -3.585 | 24.810 | 1.00 | 19.92 |
| atom | 3167 | CB  | ASN | 411 | 42.508 | -5.018 | 24.266 | 1.00 | 21.16 |
| atom | 3168 | CG  | ASN | 411 | 41.692 | -5.177 | 22.990 | 1.00 | 26.01 |
| atom | 3169 | OD1 | ASN | 411 | 40.599 | -4.618 | 22.865 | 1.00 | 21.55 |
| atom | 3170 | ND2 | ASN | 411 | 42.227 | -5.928 | 22.029 | 1.00 | 26.80 |
| atom | 3171 | C   | ASN | 411 | 42.598 | -3.557 | 26.339 | 1.00 | 23.12 |
| atom | 3172 | O   | ASN | 411 | 41.613 | -3.374 | 27.062 | 1.00 | 24.27 |
| atom | 3173 | N   | ILE | 412 | 43.828 | -3.712 | 26.828 | 1.00 | 23.33 |
| atom | 3174 | CA  | ILE | 412 | 44.063 | -3.656 | 28.259 | 1.00 | 24.19 |
| atom | 3175 | CB  | ILE | 412 | 45.569 | -3.726 | 28.603 | 1.00 | 23.03 |
| atom | 3176 | CG2 | ILE | 412 | 45.826 | -3.053 | 29.955 | 1.00 | 15.90 |
| atom | 3177 | CG1 | ILE | 412 | 46.035 | -5.182 | 28.659 | 1.00 | 18.08 |
| atom | 3178 | CD1 | ILE | 412 | 47.533 | -5.304 | 28.729 | 1.00 | 18.86 |
| atom | 3179 | C   | ILE | 412 | 43.518 | -2.306 | 28.732 | 1.00 | 27.08 |
| atom | 3180 | O   | ILE | 412 | 42.661 | -2.239 | 29.627 | 1.00 | 26.35 |
| atom | 3181 | N   | ILE | 413 | 44.013 | -1.235 | 28.113 | 1.00 | 23.29 |
| atom | 3182 | CA  | ILE | 413 | 43.589 | 0.109  | 28.474 | 1.00 | 24.70 |
| atom | 3183 | CB  | ILE | 413 | 44.176 | 1.146  | 27.508 | 1.00 | 23.52 |
| atom | 3184 | CG2 | ILE | 413 | 43.518 | 2.492  | 27.738 | 1.00 | 22.97 |
| atom | 3185 | CG1 | ILE | 413 | 45.694 | 1.227  | 27.684 | 1.00 | 18.99 |
| atom | 3186 | CD1 | ILE | 413 | 46.187 | 2.585  | 28.110 | 1.00 | 16.43 |
| atom | 3187 | C   | ILE | 413 | 42.067 | 0.270  | 28.478 | 1.00 | 28.54 |
| atom | 3188 | O   | ILE | 413 | 41.460 | 0.546  | 29.509 | 1.00 | 28.45 |
| atom | 3189 | N   | MET | 414 | 41.452 | 0.086  | 27.319 | 1.00 | 28.25 |
| atom | 3190 | CA  | MET | 414 | 40.015 | 0.253  | 27.203 | 1.00 | 29.19 |
| atom | 3191 | CB  | MET | 414 | 39.604 | 0.219  | 25.728 | 1.00 | 32.24 |
| atom | 3192 | CG  | MET | 414 | 40.255 | 1.305  | 24.859 | 1.00 | 34.56 |
| atom | 3193 | SD  | MET | 414 | 39.867 | 2.994  | 25.373 | 1.00 | 39.45 |
| atom | 3194 | CE  | MET | 414 | 38.086 | 3.098  | 24.803 | 1.00 | 32.14 |
| atom | 3195 | C   | MET | 414 | 39.187 | -0.757 | 27.972 | 1.00 | 27.51 |
| atom | 3196 | O   | MET | 414 | 38.041 | -0.487 | 28.318 | 1.00 | 23.15 |
| atom | 3197 | N   | TYR | 415 | 39.758 | -1.917 | 28.263 | 1.00 | 25.08 |
| atom | 3198 | CA  | TYR | 415 | 38.982 | -2.922 | 28.955 | 1.00 | 20.32 |
| atom | 3199 | CB  | TYR | 415 | 38.725 | -4.072 | 27.992 | 1.00 | 24.28 |
| atom | 3200 | CG  | TYR | 415 | 37.789 | -3.703 | 26.859 | 1.00 | 25.64 |

| atom | 3201 | CD1 | TYR | 415 | 38.232 | -3.672 | 25.534 | 1.00 | 29.56 |
| atom | 3202 | CE1 | TYR | 415 | 37.361 | -3.339 | 24.487 | 1.00 | 27.98 |
| atom | 3203 | CD2 | TYR | 415 | 36.462 | -3.393 | 27.111 | 1.00 | 18.42 |
| atom | 3204 | CE2 | TYR | 415 | 35.593 | -3.063 | 26.090 | 1.00 | 26.13 |
| atom | 3205 | CZ | TYR | 415 | 36.044 | -3.036 | 24.781 | 1.00 | 29.85 |
| atom | 3206 | OH | TYR | 415 | 35.174 | -2.681 | 23.781 | 1.00 | 36.03 |
| atom | 3207 | C | TYR | 415 | 39.593 | -3.430 | 30.254 | 1.00 | 21.46 |
| atom | 3208 | O | TYR | 415 | 39.341 | -4.565 | 30.658 | 1.00 | 25.89 |
| atom | 3209 | N | ALA | 416 | 40.352 | -2.571 | 30.928 | 1.00 | 15.41 |
| atom | 3210 | CA | ALA | 416 | 41.025 | -2.928 | 32.166 | 1.00 | 15.54 |
| atom | 3211 | CB | ALA | 416 | 41.809 | -1.707 | 32.723 | 1.00 | 11.41 |
| atom | 3212 | C | ALA | 416 | 40.135 | -3.493 | 33.264 | 1.00 | 15.02 |
| atom | 3213 | O | ALA | 416 | 40.489 | -4.460 | 33.928 | 1.00 | 19.20 |
| atom | 3214 | N | PRO | 417 | 38.939 | -2.947 | 33.425 | 1.00 | 15.08 |
| atom | 3215 | CD | PRO | 417 | 38.235 | -1.912 | 32.652 | 1.00 | 12.21 |
| atom | 3216 | CA | PRO | 417 | 38.135 | -3.507 | 34.508 | 1.00 | 13.68 |
| atom | 3217 | CB | PRO | 417 | 36.989 | -2.508 | 34.674 | 1.00 | 18.98 |
| atom | 3218 | CG | PRO | 417 | 37.110 | -1.512 | 33.544 | 1.00 | 14.83 |
| atom | 3219 | C | PRO | 417 | 37.627 | -4.915 | 34.303 | 1.00 | 19.04 |
| atom | 3220 | O | PRO | 417 | 37.314 | -5.597 | 35.268 | 1.00 | 18.22 |
| atom | 3221 | N | THR | 418 | 37.546 | -5.373 | 33.059 | 1.00 | 20.00 |
| atom | 3222 | CA | THR | 418 | 37.007 | -6.709 | 32.851 | 1.00 | 20.89 |
| atom | 3223 | CB | THR | 418 | 36.708 | -7.032 | 31.360 | 1.00 | 18.03 |
| atom | 3224 | OG1 | THR | 418 | 37.937 | -7.202 | 30.657 | 1.00 | 23.26 |
| atom | 3225 | CG2 | THR | 418 | 35.899 | -5.922 | 30.713 | 1.00 | 19.85 |
| atom | 3226 | C | THR | 418 | 37.886 | -7.812 | 33.389 | 1.00 | 19.47 |
| atom | 3227 | O | THR | 418 | 39.036 | -7.597 | 33.759 | 1.00 | 21.87 |
| atom | 3228 | N | LEU | 419 | 37.305 | -9.001 | 33.388 | 1.00 | 18.19 |
| atom | 3229 | CA | LEU | 419 | 37.917 | -10.204 | 33.863 | 1.00 | 17.41 |
| atom | 3230 | CB | LEU | 419 | 36.788 | -11.115 | 34.280 | 1.00 | 17.35 |
| atom | 3231 | CG | LEU | 419 | 36.701 | -12.615 | 34.162 | 1.00 | 17.06 |
| atom | 3232 | CD1 | LEU | 419 | 37.430 | -13.251 | 35.320 | 1.00 | 15.19 |
| atom | 3233 | CD2 | LEU | 419 | 35.206 | -13.000 | 34.168 | 1.00 | 2.59 |
| atom | 3234 | C | LEU | 419 | 38.863 | -10.856 | 32.854 | 1.00 | 21.73 |
| atom | 3235 | O | LEU | 419 | 39.865 | -11.452 | 33.246 | 1.00 | 25.73 |
| atom | 3236 | N | TRP | 420 | 38.586 | -10.727 | 31.559 | 1.00 | 19.46 |

| atom | 3237 | CA | TRP | 420 | 39.486 | -11.317 | 30.572 | 1.00 | 15.85 |
|------|------|-----|-----|-----|--------|---------|--------|------|-------|
| atom | 3238 | CB | TRP | 420 | 38.828 | -11.420 | 29.185 | 1.00 | 17.26 |
| atom | 3239 | CG | TRP | 420 | 38.072 | -10.210 | 28.695 | 1.00 | 16.89 |
| atom | 3240 | CD2 | TRP | 420 | 38.525 | -9.232 | 27.738 | 1.00 | 20.44 |
| atom | 3241 | CE2 | TRP | 420 | 37.459 | -8.323 | 27.530 | 1.00 | 17.05 |
| atom | 3242 | CE3 | TRP | 420 | 39.724 | -9.039 | 27.033 | 1.00 | 15.89 |
| atom | 3243 | CD1 | TRP | 420 | 36.790 | -9.859 | 29.015 | 1.00 | 18.15 |
| atom | 3244 | NE1 | TRP | 420 | 36.416 | -8.727 | 28.321 | 1.00 | 17.75 |
| atom | 3245 | CZ2 | TRP | 420 | 37.559 | -7.234 | 26.655 | 1.00 | 21.08 |
| atom | 3246 | CZ3 | TRP | 420 | 39.817 | -7.957 | 26.157 | 1.00 | 18.36 |
| atom | 3247 | CH2 | TRP | 420 | 38.741 | -7.070 | 25.975 | 1.00 | 14.83 |
| atom | 3248 | C | TRP | 420 | 40.773 | -10.517 | 30.460 | 1.00 | 20.31 |
| atom | 3249 | O | TRP | 420 | 41.873 | -11.087 | 30.443 | 1.00 | 21.36 |
| atom | 3250 | N | ALA | 421 | 40.645 | -9.192 | 30.395 | 1.00 | 18.99 |
| atom | 3251 | CA | ALA | 421 | 41.820 | -8.323 | 30.278 | 1.00 | 19.53 |
| atom | 3252 | CB | ALA | 421 | 41.381 | -6.885 | 30.005 | 1.00 | 14.34 |
| atom | 3253 | C | ALA | 421 | 42.723 | -8.365 | 31.510 | 1.00 | 22.41 |
| atom | 3254 | O | ALA | 421 | 43.950 | -8.434 | 31.397 | 1.00 | 22.08 |
| atom | 3255 | N | ARG | 422 | 42.103 | -8.320 | 32.685 | 1.00 | 25.03 |
| atom | 3256 | CA | ARG | 422 | 42.836 | -8.340 | 33.945 | 1.00 | 25.45 |
| atom | 3257 | CB | ARG | 422 | 41.871 | -8.167 | 35.132 | 1.00 | 22.24 |
| atom | 3258 | CG | ARG | 422 | 41.631 | -6.722 | 35.541 | 1.00 | 26.34 |
| atom | 3259 | CD | ARG | 422 | 40.429 | -6.583 | 36.472 | 1.00 | 25.25 |
| atom | 3260 | NE | ARG | 422 | 40.825 | -6.700 | 37.880 | 1.00 | 31.26 |
| atom | 3261 | CZ | ARG | 422 | 40.010 | -7.084 | 38.859 | 1.00 | 26.82 |
| atom | 3262 | NH1 | ARG | 422 | 38.748 | -7.390 | 38.585 | 1.00 | 25.53 |
| atom | 3263 | NH2 | ARG | 422 | 40.458 | -7.149 | 40.107 | 1.00 | 28.34 |
| atom | 3264 | C | ARG | 422 | 43.612 | -9.635 | 34.129 | 1.00 | 25.74 |
| atom | 3265 | O | ARG | 422 | 44.832 | -9.624 | 34.302 | 1.00 | 24.21 |
| atom | 3266 | N | MET | 423 | 42.893 | -10.751 | 34.070 | 1.00 | 25.90 |
| atom | 3267 | CA | MET | 423 | 43.493 | -12.046 | 34.287 | 1.00 | 23.33 |
| atom | 3268 | CB | MET | 423 | 42.416 | -13.048 | 34.692 | 1.00 | 22.80 |
| atom | 3269 | CG | MET | 423 | 41.858 | -12.787 | 36.097 | 1.00 | 17.76 |
| atom | 3270 | SD | MET | 423 | 40.368 | -13.726 | 36.473 | 1.00 | 26.13 |
| atom | 3271 | CE | MET | 423 | 41.069 | -15.281 | 36.925 | 1.00 | 29.78 |
| atom | 3272 | C | MET | 423 | 44.296 | -12.585 | 33.131 | 1.00 | 27.38 |

| atom | 3273 | O   | MET | 423 | 45.426 | -13.025 | 33.318 | 1.00 | 32.41 |
|------|------|-----|-----|-----|--------|---------|--------|------|-------|
| atom | 3274 | N   | ILE | 424 | 43.738 | -12.548 | 31.932 | 1.00 | 28.00 |
| atom | 3275 | CA  | ILE | 424 | 44.464 | -13.078 | 30.796 | 1.00 | 28.12 |
| atom | 3276 | CB  | ILE | 424 | 43.499 | -13.585 | 29.690 | 1.00 | 29.39 |
| atom | 3277 | CG2 | ILE | 424 | 44.250 | -14.508 | 28.733 | 1.00 | 32.06 |
| atom | 3278 | CG1 | ILE | 424 | 42.316 | -14.325 | 30.315 | 1.00 | 27.61 |
| atom | 3279 | CD1 | ILE | 424 | 41.834 | -15.508 | 29.508 | 1.00 | 26.72 |
| atom | 3280 | C   | ILE | 424 | 45.456 | -12.105 | 30.170 | 1.00 | 26.23 |
| atom | 3281 | O   | ILE | 424 | 46.645 | -12.374 | 30.126 | 1.00 | 21.98 |
| atom | 3282 | N   | LEU | 425 | 44.973 | -10.969 | 29.694 | 1.00 | 26.96 |
| atom | 3283 | CA  | LEU | 425 | 45.856 | -10.022 | 29.046 | 1.00 | 23.05 |
| atom | 3284 | CB  | LEU | 425 | 45.034 | -8.867  | 28.472 | 1.00 | 23.77 |
| atom | 3285 | CG  | LEU | 425 | 44.802 | -8.870  | 26.943 | 1.00 | 21.55 |
| atom | 3286 | CD1 | LEU | 425 | 44.583 | -10.282 | 26.377 | 1.00 | 21.92 |
| atom | 3287 | CD2 | LEU | 425 | 43.602 | -7.999  | 26.653 | 1.00 | 17.53 |
| atom | 3288 | C   | LEU | 425 | 46.998 | -9.519  | 29.926 | 1.00 | 26.70 |
| atom | 3289 | O   | LEU | 425 | 48.140 | -9.950  | 29.742 | 1.00 | 30.65 |
| atom | 3290 | N   | MET | 426 | 46.713 | -8.636  | 30.884 | 1.00 | 24.60 |
| atom | 3291 | CA  | MET | 426 | 47.761 | -8.101  | 31.771 | 1.00 | 19.17 |
| atom | 3292 | CB  | MET | 426 | 47.148 | -7.514  | 33.049 | 1.00 | 17.82 |
| atom | 3293 | CG  | MET | 426 | 46.479 | -6.141  | 32.881 | 1.00 | 24.33 |
| atom | 3294 | SD  | MET | 426 | 45.521 | -5.688  | 34.357 | 1.00 | 22.16 |
| atom | 3295 | CE  | MET | 426 | 44.029 | -4.983  | 33.628 | 1.00 | 23.93 |
| atom | 3296 | C   | MET | 426 | 48.777 | -9.165  | 32.188 | 1.00 | 19.10 |
| atom | 3297 | O   | MET | 426 | 49.989 | -8.979  | 32.054 | 1.00 | 12.33 |
| atom | 3298 | N   | THR | 427 | 48.267 | -10.281 | 32.700 | 1.00 | 18.78 |
| atom | 3299 | CA  | THR | 427 | 49.125 | -11.352 | 33.182 | 1.00 | 21.30 |
| atom | 3300 | CB  | THR | 427 | 48.283 | -12.569 | 33.636 | 1.00 | 20.56 |
| atom | 3301 | OG1 | THR | 427 | 47.293 | -12.125 | 34.571 | 1.00 | 21.39 |
| atom | 3302 | CG2 | THR | 427 | 49.155 | -13.620 | 34.317 | 1.00 | 18.69 |
| atom | 3303 | C   | THR | 427 | 50.149 | -11.783 | 32.149 | 1.00 | 25.61 |
| atom | 3304 | O   | THR | 427 | 51.348 | -11.796 | 32.435 | 1.00 | 23.48 |
| atom | 3305 | N   | HIS | 428 | 49.671 | -12.071 | 30.934 | 1.00 | 29.58 |
| atom | 3306 | CA  | HIS | 428 | 50.514 | -12.543 | 29.834 | 1.00 | 25.46 |
| atom | 3307 | CB  | HIS | 428 | 49.628 | -13.067 | 28.688 | 1.00 | 25.48 |
| atom | 3308 | CG  | HIS | 428 | 50.383 | -13.412 | 27.438 | 1.00 | 28.07 |

| | | | | | | | | | |
|---|---|---|---|---|---|---|---|---|---|
| atom | 3309 | CD2 | HIS | 428 | 50.706 | -14.609 | 26.893 | 1.00 | 26.29 |
| atom | 3310 | ND1 | HIS | 428 | 50.920 | -12.456 | 26.599 | 1.00 | 32.13 |
| atom | 3311 | CE1 | HIS | 428 | 51.541 | -13.049 | 25.595 | 1.00 | 28.43 |
| atom | 3312 | NE2 | HIS | 428 | 51.425 | -14.355 | 25.750 | 1.00 | 27.25 |
| atom | 3313 | C | HIS | 428 | 51.553 | -11.574 | 29.270 | 1.00 | 23.01 |
| atom | 3314 | 0 | HIS | 428 | 52.687 | -11.962 | 29.011 | 1.00 | 24.23 |
| atom | 3315 | N | PHE | 429 | 51.192 | -10.318 | 29.086 | 1.00 | 21.06 |
| atom | 3316 | CA | PHE | 429 | 52.150 | -9.404 | 28.496 | 1.00 | 22.48 |
| atom | 3317 | CB | PHE | 429 | 51.430 | -8.262 | 27.773 | 1.00 | 25.70 |
| atom | 3318 | CG | PHE | 429 | 50.958 | -8.633 | 26.394 | 1.00 | 28.92 |
| atom | 3319 | CD1 | PHE | 429 | 49.682 | -9.146 | 26.200 | 1.00 | 26.69 |
| atom | 3320 | CD2 | PHE | 429 | 51.804 | -8.501 | 25.291 | 1.00 | 27.61 |
| atom | 3321 | CE1 | PHE | 429 | 49.252 | -9.529 | 24.932 | 1.00 | 31.49 |
| atom | 3322 | CE2 | PHE | 429 | 51.380 | -8.883 | 24.017 | 1.00 | 27.65 |
| atom | 3323 | CZ | PHE | 429 | 50.105 | -9.398 | 23.835 | 1.00 | 26.84 |
| atom | 3324 | C | PHE | 429 | 53.137 | -8.872 | 29.492 | 1.00 | 20.77 |
| atom | 3325 | 0 | PHE | 429 | 54.265 | -8.563 | 29.139 | 1.00 | 21.91 |
| atom | 3326 | N | PHE | 430 | 52.733 | -8.751 | 30.745 | 1.00 | 22.30 |
| atom | 3327 | CA | PHE | 430 | 53.700 | -8.286 | 31.726 | 1.00 | 22.59 |
| atom | 3328 | CB | PHE | 430 | 53.033 | -7.977 | 33.072 | 1.00 | 24.91 |
| atom | 3329 | CG | PHE | 430 | 52.774 | -6.514 | 33.259 | 1.00 | 26.03 |
| atom | 3330 | CD1 | PHE | 430 | 51.602 | -5.939 | 32.762 | 1.00 | 30.68 |
| atom | 3331 | CD2 | PHE | 430 | 53.749 | -5.686 | 33.811 | 1.00 | 20.79 |
| atom | 3332 | CE1 | PHE | 430 | 51.415 | -4.553 | 32.802 | 1.00 | 28.98 |
| atom | 3333 | CE2 | PHE | 430 | 53.572 | -4.323 | 33.855 | 1.00 | 21.47 |
| atom | 3334 | CZ | PHE | 430 | 52.405 | -3.747 | 33.348 | 1.00 | 22.42 |
| atom | 3335 | C | PHE | 430 | 54.799 | -9.330 | 31.867 | 1.00 | 13.18 |
| atom | 3336 | 0 | PHE | 430 | 55.966 | -8.994 | 31.865 | 1.00 | 12.59 |
| atom | 3337 | N | SER | 431 | 54.435 | -10.596 | 31.928 | 1.00 | 11.96 |
| atom | 3338 | CA | SER | 431 | 55.455 | -11.637 | 32.033 | 1.00 | 20.34 |
| atom | 3339 | CB | SER | 431 | 54.813 | -13.002 | 32.226 | 1.00 | 20.23 |
| atom | 3340 | OG | SER | 431 | 55.091 | -13.821 | 31.115 | 1.00 | 30.81 |
| atom | 3341 | C | SER | 431 | 56.344 | -11.684 | 30.794 | 1.00 | 23.41 |
| atom | 3342 | 0 | SER | 431 | 57.555 | -11.847 | 30.903 | 1.00 | 22.98 |
| atom | 3343 | N | ILE | 432 | 55.732 | -11.548 | 29.617 | 1.00 | 26.02 |
| atom | 3344 | CA | ILE | 432 | 56.470 | -11.565 | 28.354 | 1.00 | 24.15 |

| atom | 3345 | CB  | ILE | 432 | 55.507 | -11.580 | 27.130 | 1.00 | 29.47 |
| atom | 3346 | CG2 | ILE | 432 | 56.290 | -11.349 | 25.834 | 1.00 | 26.68 |
| atom | 3347 | CG1 | ILE | 432 | 54.737 | -12.905 | 27.079 | 1.00 | 23.71 |
| atom | 3348 | CD1 | ILE | 432 | 55.608 | -14.126 | 26.962 | 1.00 | 28.14 |
| atom | 3349 | C   | ILE | 432 | 57.348 | -10.329 | 28.264 | 1.00 | 23.16 |
| atom | 3350 | O   | ILE | 432 | 58.558 | -10.426 | 28.106 | 1.00 | 23.35 |
| atom | 3351 | N   | LEU | 433 | 56.724 | -9.164  | 28.371 | 1.00 | 23.94 |
| atom | 3352 | CA  | LEU | 433 | 57.435 | -7.894  | 28.309 | 1.00 | 28.88 |
| atom | 3353 | CB  | LEU | 433 | 56.441 | -6.747  | 28.501 | 1.00 | 30.58 |
| atom | 3354 | CG  | LEU | 433 | 56.346 | -5.678  | 27.407 | 1.00 | 33.37 |
| atom | 3355 | CD1 | LEU | 433 | 56.205 | -6.322  | 26.028 | 1.00 | 32.23 |
| atom | 3356 | CD2 | LEU | 433 | 55.145 | -4.785  | 27.718 | 1.00 | 36.50 |
| atom | 3357 | C   | LEU | 433 | 58.553 | -7.817  | 29.363 | 1.00 | 31.43 |
| atom | 3358 | O   | LEU | 433 | 59.625 | -7.268  | 29.121 | 1.00 | 31.96 |
| atom | 3359 | N   | LEU | 434 | 58.284 | -8.367  | 30.540 | 1.00 | 34.05 |
| atom | 3360 | CA  | LEU | 434 | 59.254 | -8.402  | 31.629 | 1.00 | 32.03 |
| atom | 3361 | CB  | LEU | 434 | 58.606 | -9.059  | 32.855 | 1.00 | 33.37 |
| atom | 3362 | CG  | LEU | 434 | 59.129 | -8.941  | 34.288 | 1.00 | 31.50 |
| atom | 3363 | CD1 | LEU | 434 | 59.629 | -7.550  | 34.594 | 1.00 | 34.29 |
| atom | 3364 | CD2 | LEU | 434 | 57.989 | -9.282  | 35.220 | 1.00 | 34.33 |
| atom | 3365 | C   | LEU | 434 | 60.470 | -9.231  | 31.203 | 1.00 | 30.59 |
| atom | 3366 | O   | LEU | 434 | 61.612 | -8.795  | 31.326 | 1.00 | 30.54 |
| atom | 3367 | N   | ALA | 435 | 60.200 | -10.429 | 30.696 | 1.00 | 28.04 |
| atom | 3368 | CA  | ALA | 435 | 61.236 | -11.357 | 30.283 | 1.00 | 27.70 |
| atom | 3369 | CB  | ALA | 435 | 60.606 | -12.560 | 29.606 | 1.00 | 23.29 |
| atom | 3370 | C   | ALA | 435 | 62.303 | -10.759 | 29.389 | 1.00 | 31.48 |
| atom | 3371 | O   | ALA | 435 | 63.493 | -10.976 | 29.618 | 1.00 | 37.97 |
| atom | 3372 | N   | GLN | 436 | 61.890 | -10.007 | 28.379 | 1.00 | 33.13 |
| atom | 3373 | CA  | GLN | 436 | 62.827 | -9.398  | 27.444 | 1.00 | 34.38 |
| atom | 3374 | CB  | GLN | 436 | 62.195 | -9.358  | 26.051 | 1.00 | 33.83 |
| atom | 3375 | CG  | GLN | 436 | 61.279 | -10.536 | 25.748 | 1.00 | 36.51 |
| atom | 3376 | CD  | GLN | 436 | 60.003 | -10.132 | 25.018 | 1.00 | 42.61 |
| atom | 3377 | OE1 | GLN | 436 | 59.401 | -9.102  | 25.321 | 1.00 | 44.59 |
| atom | 3378 | NE2 | GLN | 436 | 59.585 | -10.948 | 24.051 | 1.00 | 42.60 |
| atom | 3379 | C   | GLN | 436 | 63.300 | -7.991  | 27.832 | 1.00 | 35.80 |
| atom | 3380 | O   | GLN | 436 | 63.988 | -7.329  | 27.065 | 1.00 | 34.86 |

| atom | 3381 | N   | GLU | 437 | 62.953 | -7.537 | 29.028 | 1.00 | 39.49 |
|------|------|-----|-----|-----|--------|--------|--------|------|-------|
| atom | 3382 | CA  | GLU | 437 | 63.343 | -6.191 | 29.471 | 1.00 | 45.63 |
| atom | 3383 | CB  | GLU | 437 | 64.878 | -6.048 | 29.504 | 1.00 | 47.97 |
| atom | 3384 | CG  | GLU | 437 | 65.637 | -7.268 | 30.035 | 1.00 | 51.93 |
| atom | 3385 | CD  | GLU | 437 | 65.701 | -7.312 | 31.563 | 1.00 | 56.66 |
| atom | 3386 | OE1 | GLU | 437 | 65.318 | -6.303 | 32.208 | 1.00 | 56.30 |
| atom | 3387 | OE2 | GLU | 437 | 66.128 | -8.355 | 32.117 | 1.00 | 50.71 |
| atom | 3388 | C   | GLU | 437 | 62.733 | -5.103 | 28.553 | 1.00 | 45.05 |
| atom | 3389 | O   | GLU | 437 | 63.433 | -4.196 | 28.096 | 1.00 | 40.40 |
| atom | 3390 | N   | GLN | 438 | 61.422 | -5.206 | 28.316 | 1.00 | 44.81 |
| atom | 3391 | CA  | GLN | 438 | 60.671 | -4.273 | 27.467 | 1.00 | 44.57 |
| atom | 3392 | CB  | GLN | 438 | 59.854 | -5.054 | 26.427 | 1.00 | 43.31 |
| atom | 3393 | CG  | GLN | 438 | 60.666 | -5.719 | 25.329 | 1.00 | 41.52 |
| atom | 3394 | CD  | GLN | 438 | 61.618 | -4.766 | 24.653 | 1.00 | 39.73 |
| atom | 3395 | OE1 | GLN | 438 | 61.417 | -3.560 | 24.682 | 1.00 | 39.88 |
| atom | 3396 | NE2 | GLN | 438 | 62.669 | -5.304 | 24.040 | 1.00 | 40.97 |
| atom | 3397 | C   | GLN | 438 | 59.709 | -3.302 | 28.191 | 1.00 | 44.17 |
| atom | 3398 | O   | GLN | 438 | 59.284 | -2.309 | 27.600 | 1.00 | 47.18 |
| atom | 3399 | N   | LEU | 439 | 59.358 | -3.591 | 29.443 | 1.00 | 39.41 |
| atom | 3400 | CA  | LEU | 439 | 58.437 | -2.757 | 30.234 | 1.00 | 35.63 |
| atom | 3401 | CB  | LEU | 439 | 58.619 | -3.047 | 31.725 | 1.00 | 36.65 |
| atom | 3402 | CG  | LEU | 439 | 57.675 | -3.977 | 32.484 | 1.00 | 35.38 |
| atom | 3403 | CD1 | LEU | 439 | 56.661 | -4.630 | 31.580 | 1.00 | 37.24 |
| atom | 3404 | CD2 | LEU | 439 | 58.520 | -5.014 | 33.152 | 1.00 | 35.93 |
| atom | 3405 | C   | LEU | 439 | 58.533 | -1.240 | 30.046 | 1.00 | 34.88 |
| atom | 3406 | O   | LEU | 439 | 57.513 | -0.533 | 30.050 | 1.00 | 31.04 |
| atom | 3407 | N   | GLU | 440 | 59.752 | -0.732 | 29.900 | 1.00 | 31.23 |
| atom | 3408 | CA  | GLU | 440 | 59.929 | 0.700  | 29.749 | 1.00 | 34.08 |
| atom | 3409 | CB  | GLU | 440 | 61.203 | 1.137  | 30.471 | 1.00 | 36.60 |
| atom | 3410 | CG  | GLU | 440 | 61.344 | 0.546  | 31.869 | 1.00 | 46.25 |
| atom | 3411 | CD  | GLU | 440 | 62.326 | -0.619 | 31.917 | 1.00 | 51.65 |
| atom | 3412 | OE1 | GLU | 440 | 62.004 | -1.705 | 31.381 | 1.00 | 55.45 |
| atom | 3413 | OE2 | GLU | 440 | 63.425 | -0.451 | 32.489 | 1.00 | 56.28 |
| atom | 3414 | C   | GLU | 440 | 59.933 | 1.195  | 28.297 | 1.00 | 33.24 |
| atom | 3415 | O   | GLU | 440 | 60.157 | 2.383  | 28.047 | 1.00 | 32.66 |
| atom | 3416 | N   | LYS | 441 | 59.674 | 0.288  | 27.357 | 1.00 | 29.40 |

| atom | 3417 | CA  | LYS | 441 | 59.625 | 0.618  | 25.933 | 1.00 | 32.91 |
|------|------|-----|-----|-----|--------|--------|--------|------|-------|
| atom | 3418 | CB  | LYS | 441 | 60.021 | -0.616 | 25.097 | 1.00 | 35.80 |
| atom | 3419 | CG  | LYS | 441 | 59.636 | -0.582 | 23.593 | 1.00 | 44.21 |
| atom | 3420 | CD  | LYS | 441 | 59.066 | -1.942 | 23.090 | 1.00 | 43.76 |
| atom | 3421 | CE  | LYS | 441 | 59.752 | -2.436 | 21.800 | 1.00 | 42.14 |
| atom | 3422 | NZ  | LYS | 441 | 59.679 | -3.931 | 21.557 | 1.00 | 32.91 |
| atom | 3423 | C   | LYS | 441 | 58.202 | 1.057  | 25.586 | 1.00 | 32.30 |
| atom | 3424 | O   | LYS | 441 | 57.277 | 0.247  | 25.575 | 1.00 | 34.79 |
| atom | 3425 | N   | ALA | 442 | 58.011 | 2.341  | 25.328 | 1.00 | 32.09 |
| atom | 3426 | CA  | ALA | 442 | 56.679 | 2.814  | 24.988 | 1.00 | 34.73 |
| atom | 3427 | CB  | ALA | 442 | 56.654 | 4.321  | 24.923 | 1.00 | 34.99 |
| atom | 3428 | C   | ALA | 442 | 56.272 | 2.233  | 23.646 | 1.00 | 35.06 |
| atom | 3429 | O   | ALA | 442 | 57.004 | 2.353  | 22.667 | 1.00 | 34.73 |
| atom | 3430 | N   | LEU | 443 | 55.107 | 1.597  | 23.612 | 1.00 | 34.80 |
| atom | 3431 | CA  | LEU | 443 | 54.583 | 1.006  | 22.388 | 1.00 | 32.57 |
| atom | 3432 | CB  | LEU | 443 | 54.064 | -0.403 | 22.665 | 1.00 | 32.82 |
| atom | 3433 | CG  | LEU | 443 | 54.984 | -1.291 | 23.493 | 1.00 | 31.23 |
| atom | 3434 | CD1 | LEU | 443 | 54.192 | -2.002 | 24.599 | 1.00 | 29.05 |
| atom | 3435 | CD2 | LEU | 443 | 55.669 | -2.279 | 22.557 | 1.00 | 27.43 |
| atom | 3436 | C   | LEU | 443 | 53.444 | 1.873  | 21.859 | 1.00 | 33.71 |
| atom | 3437 | O   | LEU | 443 | 52.839 | 2.644  | 22.602 | 1.00 | 32.92 |
| atom | 3438 | N   | ASP | 444 | 53.166 | 1.754  | 20.569 | 1.00 | 34.97 |
| atom | 3439 | CA  | ASP | 444 | 52.098 | 2.517  | 19.940 | 1.00 | 34.69 |
| atom | 3440 | CB  | ASP | 444 | 52.472 | 2.874  | 18.505 | 1.00 | 35.84 |
| atom | 3441 | CG  | ASP | 444 | 53.339 | 4.102  | 18.421 | 1.00 | 40.11 |
| atom | 3442 | OD1 | ASP | 444 | 53.370 | 4.882  | 19.394 | 1.00 | 47.24 |
| atom | 3443 | OD2 | ASP | 444 | 53.996 | 4.295  | 17.382 | 1.00 | 43.96 |
| atom | 3444 | C   | ASP | 444 | 50.868 | 1.635  | 19.921 | 1.00 | 36.94 |
| atom | 3445 | O   | ASP | 444 | 50.976 | 0.412  | 19.918 | 1.00 | 42.96 |
| atom | 3446 | N   | CYS | 445 | 49.700 | 2.255  | 19.911 | 1.00 | 35.62 |
| atom | 3447 | CA  | CYS | 445 | 48.446 | 1.530  | 19.876 | 1.00 | 32.06 |
| atom | 3448 | CB  | CYS | 445 | 48.165 | 0.899  | 21.244 | 1.00 | 31.49 |
| atom | 3449 | SG  | CYS | 445 | 47.942 | 2.070  | 22.618 | 1.00 | 32.37 |
| atom | 3450 | C   | CYS | 445 | 47.427 | 2.596  | 19.526 | 1.00 | 34.19 |
| atom | 3451 | O   | CYS | 445 | 47.747 | 3.783  | 19.552 | 1.00 | 36.00 |
| atom | 3452 | N   | GLN | 446 | 46.208 | 2.210  | 19.189 | 1.00 | 38.21 |

| atom | 3453 | CA  | GLN | 446 | 45.232 | 3.232 | 18.843 | 1.00 | 40.12 |
|------|------|-----|-----|-----|--------|-------|--------|------|-------|
| atom | 3454 | CB  | GLN | 446 | 44.836 | 3.088 | 17.375 | 1.00 | 43.49 |
| atom | 3455 | CG  | GLN | 446 | 44.134 | 1.781 | 17.046 | 1.00 | 52.73 |
| atom | 3456 | CD  | GLN | 446 | 43.416 | 1.812 | 15.698 | 1.00 | 53.41 |
| atom | 3457 | OE1 | GLN | 446 | 42.751 | 2.793 | 15.356 | 1.00 | 52.33 |
| atom | 3458 | NE2 | GLN | 446 | 43.547 | 0.729 | 14.931 | 1.00 | 52.81 |
| atom | 3459 | C   | GLN | 446 | 43.984 | 3.293 | 19.738 | 1.00 | 38.91 |
| atom | 3460 | O   | GLN | 446 | 43.387 | 2.275 | 20.087 | 1.00 | 37.38 |
| atom | 3461 | N   | ILE | 447 | 43.621 | 4.514 | 20.115 | 1.00 | 35.95 |
| atom | 3462 | CA  | ILE | 447 | 42.458 | 4.779 | 20.950 | 1.00 | 35.14 |
| atom | 3463 | CB  | ILE | 447 | 42.826 | 5.654 | 22.194 | 1.00 | 35.06 |
| atom | 3464 | CG2 | ILE | 447 | 41.566 | 6.057 | 22.954 | 1.00 | 28.93 |
| atom | 3465 | CG1 | ILE | 447 | 43.773 | 4.894 | 23.124 | 1.00 | 35.72 |
| atom | 3466 | CD1 | ILE | 447 | 44.576 | 5.816 | 24.017 | 1.00 | 35.52 |
| atom | 3467 | C   | ILE | 447 | 41.499 | 5.578 | 20.071 | 1.00 | 36.57 |
| atom | 3468 | O   | ILE | 447 | 41.780 | 6.736 | 19.729 | 1.00 | 37.05 |
| atom | 3469 | N   | TYR | 448 | 40.380 | 4.969 | 19.687 | 1.00 | 32.88 |
| atom | 3470 | CA  | TYR | 448 | 39.405 | 5.666 | 18.850 | 1.00 | 29.32 |
| atom | 3471 | CB  | TYR | 448 | 38.867 | 6.903 | 19.574 | 1.00 | 30.94 |
| atom | 3472 | CG  | TYR | 448 | 37.916 | 6.619 | 20.725 | 1.00 | 35.92 |
| atom | 3473 | CD1 | TYR | 448 | 37.379 | 7.668 | 21.478 | 1.00 | 34.37 |
| atom | 3474 | CE1 | TYR | 448 | 36.485 | 7.425 | 22.517 | 1.00 | 32.55 |
| atom | 3475 | CD2 | TYR | 448 | 37.529 | 5.308 | 21.049 | 1.00 | 33.84 |
| atom | 3476 | CE2 | TYR | 448 | 36.634 | 5.058 | 22.090 | 1.00 | 32.27 |
| atom | 3477 | CZ  | TYR | 448 | 36.118 | 6.124 | 22.819 | 1.00 | 33.86 |
| atom | 3478 | OH  | TYR | 448 | 35.242 | 5.899 | 23.863 | 1.00 | 34.45 |
| atom | 3479 | C   | TYR | 448 | 39.994 | 6.105 | 17.513 | 1.00 | 26.03 |
| atom | 3480 | O   | TYR | 448 | 39.660 | 7.168 | 17.007 | 1.00 | 25.23 |
| atom | 3481 | N   | GLY | 449 | 40.892 | 5.303 | 16.955 | 1.00 | 25.14 |
| atom | 3482 | CA  | GLY | 449 | 41.467 | 5.646 | 15.665 | 1.00 | 30.15 |
| atom | 3483 | C   | GLY | 449 | 42.817 | 6.336 | 15.692 | 1.00 | 29.86 |
| atom | 3484 | O   | GLY | 449 | 43.684 | 6.062 | 14.857 | 1.00 | 31.59 |
| atom | 3485 | N   | ALA | 450 | 42.993 | 7.258 | 16.626 | 1.00 | 30.00 |
| atom | 3486 | CA  | ALA | 450 | 44.265 | 7.942 | 16.740 | 1.00 | 27.32 |
| atom | 3487 | CB  | ALA | 450 | 44.132 | 9.207 | 17.585 | 1.00 | 29.76 |
| atom | 3488 | C   | ALA | 450 | 45.190 | 6.951 | 17.423 | 1.00 | 27.92 |

| atom | 3489 | O   | ALA | 450 | 44.768 | 6.122  | 18.240 | 1.00 | 21.53 |
|------|------|-----|-----|-----|--------|--------|--------|------|-------|
| atom | 3490 | N   | CYS | 451 | 46.456 | 7.022  | 17.057 | 1.00 | 29.64 |
| atom | 3491 | CA  | CYS | 451 | 47.436 | 6.141  | 17.640 | 1.00 | 33.17 |
| atom | 3492 | CB  | CYS | 451 | 48.345 | 5.611  | 16.524 | 1.00 | 27.89 |
| atom | 3493 | SG  | CYS | 451 | 50.112 | 5.858  | 16.743 | 1.00 | 35.52 |
| atom | 3494 | C   | CYS | 451 | 48.185 | 6.956  | 18.720 | 1.00 | 31.89 |
| atom | 3495 | O   | CYS | 451 | 48.393 | 8.168  | 18.586 | 1.00 | 29.04 |
| atom | 3496 | N   | TYR | 452 | 48.539 | 6.309  | 19.821 | 1.00 | 31.86 |
| atom | 3497 | CA  | TYR | 452 | 49.242 | 7.028  | 20.878 | 1.00 | 35.81 |
| atom | 3498 | CB  | TYR | 452 | 48.324 | 7.220  | 22.095 | 1.00 | 34.55 |
| atom | 3499 | CG  | TYR | 452 | 47.172 | 8.175  | 21.868 | 1.00 | 35.92 |
| atom | 3500 | CD1 | TYR | 452 | 45.941 | 7.719  | 21.381 | 1.00 | 39.87 |
| atom | 3501 | CE1 | TYR | 452 | 44.862 | 8.594  | 21.203 | 1.00 | 37.75 |
| atom | 3502 | CD2 | TYR | 452 | 47.298 | 9.526  | 22.161 | 1.00 | 35.65 |
| atom | 3503 | CE2 | TYR | 452 | 46.230 | 10.403 | 21.987 | 1.00 | 39.32 |
| atom | 3504 | CZ  | TYR | 452 | 45.021 | 9.935  | 21.509 | 1.00 | 38.29 |
| atom | 3505 | OH  | TYR | 452 | 43.981 | 10.820 | 21.357 | 1.00 | 44.55 |
| atom | 3506 | C   | TYR | 452 | 50.531 | 6.336  | 21.319 | 1.00 | 35.46 |
| atom | 3507 | O   | TYR | 452 | 50.795 | 5.173  | 20.975 | 1.00 | 34.31 |
| atom | 3508 | N   | SER | 453 | 51.339 | 7.067  | 22.074 | 1.00 | 34.30 |
| atom | 3509 | CA  | SER | 453 | 52.563 | 6.500  | 22.581 | 1.00 | 38.03 |
| atom | 3510 | CB  | SER | 453 | 53.733 | 7.413  | 22.256 | 1.00 | 41.05 |
| atom | 3511 | OG  | SER | 453 | 54.223 | 7.101  | 20.955 | 1.00 | 47.23 |
| atom | 3512 | C   | SER | 453 | 52.405 | 6.315  | 24.078 | 1.00 | 35.40 |
| atom | 3513 | O   | SER | 453 | 52.534 | 7.261  | 24.846 | 1.00 | 35.22 |
| atom | 3514 | N   | ILE | 454 | 52.109 | 5.084  | 24.476 | 1.00 | 33.48 |
| atom | 3515 | CA  | ILE | 454 | 51.903 | 4.767  | 25.877 | 1.00 | 36.42 |
| atom | 3516 | CB  | ILE | 454 | 50.510 | 4.148  | 26.088 | 1.00 | 36.41 |
| atom | 3517 | CG2 | ILE | 454 | 50.079 | 4.305  | 27.540 | 1.00 | 33.83 |
| atom | 3518 | CG1 | ILE | 454 | 49.514 | 4.827  | 25.157 | 1.00 | 35.30 |
| atom | 3519 | CD1 | ILE | 454 | 48.079 | 4.660  | 25.578 | 1.00 | 44.75 |
| atom | 3520 | C   | ILE | 454 | 52.938 | 3.818  | 26.461 | 1.00 | 37.22 |
| atom | 3521 | O   | ILE | 454 | 53.294 | 2.806  | 25.854 | 1.00 | 38.43 |
| atom | 3522 | N   | GLU | 455 | 53.417 | 4.153  | 27.654 | 1.00 | 38.85 |
| atom | 3523 | CA  | GLU | 455 | 54.386 | 3.317  | 28.343 | 1.00 | 37.89 |
| atom | 3524 | CB  | GLU | 455 | 55.300 | 4.174  | 29.215 | 1.00 | 44.07 |

| atom | 3525 | CG | GLU | 455 | 56.360 | 3.369 | 29.960 | 1.00 | 54.04 |
|------|------|-----|-----|-----|--------|-------|--------|------|-------|
| atom | 3526 | CD | GLU | 455 | 57.480 | 4.247 | 30.509 | 1.00 | 61.38 |
| atom | 3527 | OE1 | GLU | 455 | 57.521 | 5.453 | 30.145 | 1.00 | 60.82 |
| atom | 3528 | OE2 | GLU | 455 | 58.313 | 3.730 | 31.300 | 1.00 | 58.53 |
| atom | 3529 | C | GLU | 455 | 53.602 | 2.351 | 29.216 | 1.00 | 33.96 |
| atom | 3530 | O | GLU | 455 | 52.775 | 2.765 | 30.026 | 1.00 | 31.59 |
| atom | 3531 | N | PRO | 456 | 53.844 | 1.045 | 29.061 | 1.00 | 32.69 |
| atom | 3532 | CD | PRO | 456 | 54.779 | 0.431 | 28.107 | 1.00 | 31.89 |
| atom | 3533 | CA | PRO | 456 | 53.117 | 0.056 | 29.870 | 1.00 | 34.87 |
| atom | 3534 | CB | PRO | 456 | 53.615 | -1.300 | 29.346 | 1.00 | 31.61 |
| atom | 3535 | CG | PRO | 456 | 54.819 | -1.005 | 28.540 | 1.00 | 31.68 |
| atom | 3536 | C | PRO | 456 | 53.241 | 0.186 | 31.404 | 1.00 | 35.06 |
| atom | 3537 | O | PRO | 456 | 52.373 | -0.286 | 32.150 | 1.00 | 35.96 |
| atom | 3538 | N | LEU | 457 | 54.302 | 0.826 | 31.880 | 1.00 | 31.41 |
| atom | 3539 | CA | LEU | 457 | 54.464 | 0.990 | 33.318 | 1.00 | 31.83 |
| atom | 3540 | CB | LEU | 457 | 55.936 | 1.214 | 33.668 | 1.00 | 21.51 |
| atom | 3541 | CG | LEU | 457 | 56.746 | -0.081 | 33.628 | 1.00 | 22.27 |
| atom | 3542 | CD1 | LEU | 457 | 58.182 | 0.232 | 33.223 | 1.00 | 18.61 |
| atom | 3543 | CD2 | LEU | 457 | 56.697 | -0.781 | 34.997 | 1.00 | 22.63 |
| atom | 3544 | C | LEU | 457 | 53.603 | 2.147 | 33.841 | 1.00 | 33.12 |
| atom | 3545 | O | LEU | 457 | 53.574 | 2.425 | 35.046 | 1.00 | 36.08 |
| atom | 3546 | N | ASP | 458 | 52.899 | 2.812 | 32.932 | 1.00 | 33.35 |
| atom | 3547 | CA | ASP | 458 | 52.026 | 3.920 | 33.306 | 1.00 | 31.48 |
| atom | 3548 | CB | ASP | 458 | 52.074 | 5.004 | 32.219 | 1.00 | 31.92 |
| atom | 3549 | CG | ASP | 458 | 53.235 | 5.977 | 32.401 | 1.00 | 35.20 |
| atom | 3550 | OD1 | ASP | 458 | 53.988 | 5.831 | 33.385 | 1.00 | 40.06 |
| atom | 3551 | OD2 | ASP | 458 | 53.398 | 6.891 | 31.562 | 1.00 | 32.72 |
| atom | 3552 | C | ASP | 458 | 50.579 | 3.402 | 33.492 | 1.00 | 30.57 |
| atom | 3553 | O | ASP | 458 | 49.716 | 4.104 | 34.020 | 1.00 | 29.93 |
| atom | 3554 | N | LEU | 459 | 50.337 | 2.160 | 33.079 | 1.00 | 29.54 |
| atom | 3555 | CA | LEU | 459 | 49.011 | 1.552 | 33.172 | 1.00 | 32.41 |
| atom | 3556 | CB | LEU | 459 | 49.097 | 0.036 | 32.962 | 1.00 | 31.91 |
| atom | 3557 | CG | LEU | 459 | 48.435 | -0.559 | 31.712 | 1.00 | 31.59 |
| atom | 3558 | CD1 | LEU | 459 | 47.692 | 0.510 | 30.923 | 1.00 | 30.88 |
| atom | 3559 | CD2 | LEU | 459 | 49.508 | -1.226 | 30.839 | 1.00 | 33.41 |
| atom | 3560 | C | LEU | 459 | 48.187 | 1.837 | 34.422 | 1.00 | 31.36 |

| atom | 3561 | O   | LEU | 459 | 47.092 | 2.379  | 34.314 | 1.00 | 35.42 |
|------|------|-----|-----|-----|--------|--------|--------|------|-------|
| atom | 3562 | N   | PRO | 460 | 48.680 | 1.471  | 35.618 | 1.00 | 31.69 |
| atom | 3563 | CD  | PRO | 460 | 49.948 | 0.798  | 35.949 | 1.00 | 37.70 |
| atom | 3564 | CA  | PRO | 460 | 47.893 | 1.742  | 36.824 | 1.00 | 31.94 |
| atom | 3565 | CB  | PRO | 460 | 48.867 | 1.443  | 37.961 | 1.00 | 34.04 |
| atom | 3566 | CG  | PRO | 460 | 49.768 | 0.421  | 37.403 | 1.00 | 34.31 |
| atom | 3567 | C   | PRO | 460 | 47.370 | 3.172  | 36.869 | 1.00 | 31.06 |
| atom | 3568 | O   | PRO | 460 | 46.207 | 3.404  | 37.171 | 1.00 | 32.13 |
| atom | 3569 | N   | GLN | 461 | 48.240 | 4.122  | 36.559 | 1.00 | 34.11 |
| atom | 3570 | CA  | GLN | 461 | 47.877 | 5.542  | 36.539 | 1.00 | 38.17 |
| atom | 3571 | CB  | GLN | 461 | 49.088 | 6.398  | 36.121 | 1.00 | 38.18 |
| atom | 3572 | CG  | GLN | 461 | 50.311 | 6.252  | 37.023 | 1.00 | 42.90 |
| atom | 3573 | CD  | GLN | 461 | 51.124 | 5.006  | 36.725 | 1.00 | 46.75 |
| atom | 3574 | OE1 | GLN | 461 | 50.761 | 3.892  | 37.128 | 1.00 | 47.13 |
| atom | 3575 | NE2 | GLN | 461 | 52.237 | 5.186  | 36.018 | 1.00 | 48.03 |
| atom | 3576 | C   | GLN | 461 | 46.734 | 5.780  | 35.549 | 1.00 | 34.45 |
| atom | 3577 | O   | GLN | 461 | 45.742 | 6.418  | 35.873 | 1.00 | 33.29 |
| atom | 3578 | N   | ILE | 462 | 46.894 | 5.256  | 34.340 | 1.00 | 30.87 |
| atom | 3579 | CA  | ILE | 462 | 45.895 | 5.408  | 33.289 | 1.00 | 28.05 |
| atom | 3580 | CB  | ILE | 462 | 46.406 | 4.806  | 31.963 | 1.00 | 28.23 |
| atom | 3581 | CG2 | ILE | 462 | 45.305 | 4.784  | 30.928 | 1.00 | 21.36 |
| atom | 3582 | CG1 | ILE | 462 | 47.603 | 5.621  | 31.466 | 1.00 | 28.26 |
| atom | 3583 | CD1 | ILE | 462 | 48.241 | 5.073  | 30.227 | 1.00 | 30.74 |
| atom | 3584 | C   | ILE | 462 | 44.607 | 4.712  | 33.688 | 1.00 | 27.56 |
| atom | 3585 | O   | ILE | 462 | 43.534 | 5.332  | 33.726 | 1.00 | 27.06 |
| atom | 3586 | N   | ILE | 463 | 44.725 | 3.424  | 33.996 | 1.00 | 22.72 |
| atom | 3587 | CA  | ILE | 463 | 43.574 | 2.636  | 34.400 | 1.00 | 20.93 |
| atom | 3588 | CB  | ILE | 463 | 44.003 | 1.216  | 34.896 | 1.00 | 21.25 |
| atom | 3589 | CG2 | ILE | 463 | 42.817 | 0.479  | 35.517 | 1.00 | 18.27 |
| atom | 3590 | CG1 | ILE | 463 | 44.537 | 0.396  | 33.715 | 1.00 | 16.48 |
| atom | 3591 | CD1 | ILE | 463 | 45.126 | -0.924 | 34.099 | 1.00 | 9.67  |
| atom | 3592 | C   | ILE | 463 | 42.758 | 3.351  | 35.480 | 1.00 | 19.90 |
| atom | 3593 | O   | ILE | 463 | 41.537 | 3.416  | 35.375 | 1.00 | 22.61 |
| atom | 3594 | N   | GLU | 464 | 43.418 | 3.903  | 36.497 | 1.00 | 22.67 |
| atom | 3595 | CA  | GLU | 464 | 42.710 | 4.592  | 37.581 | 1.00 | 23.09 |
| atom | 3596 | CB  | GLU | 464 | 43.654 | 4.981  | 38.726 | 1.00 | 22.31 |

| atom | 3597 | CG | GLU | 464 | 42.882 | 5.206 | 40.033 | 1.00 | 24.89 |
|------|------|------|------|------|--------|--------|--------|------|-------|
| atom | 3598 | CD | GLU | 464 | 43.508 | 6.229 | 40.954 | 1.00 | 22.17 |
| atom | 3599 | OE1 | GLU | 464 | 43.109 | 7.406 | 40.905 | 1.00 | 31.72 |
| atom | 3600 | OE2 | GLU | 464 | 44.390 | 5.865 | 41.738 | 1.00 | 16.70 |
| atom | 3601 | C | GLU | 464 | 41.959 | 5.840 | 37.132 | 1.00 | 25.17 |
| atom | 3602 | O | GLU | 464 | 40.845 | 6.102 | 37.610 | 1.00 | 21.06 |
| atom | 3603 | N | ARG | 465 | 42.562 | 6.609 | 36.225 | 1.00 | 24.41 |
| atom | 3604 | CA | ARG | 465 | 41.920 | 7.818 | 35.729 | 1.00 | 25.52 |
| atom | 3605 | CB | ARG | 465 | 42.908 | 8.681 | 34.943 | 1.00 | 31.25 |
| atom | 3606 | CG | ARG | 465 | 43.219 | 10.019 | 35.642 | 1.00 | 39.07 |
| atom | 3607 | CD | ARG | 465 | 42.685 | 11.216 | 34.860 | 1.00 | 43.22 |
| atom | 3608 | NE | ARG | 465 | 43.641 | 12.326 | 34.767 | 1.00 | 47.82 |
| atom | 3609 | CZ | ARG | 465 | 44.942 | 12.198 | 34.504 | 1.00 | 50.16 |
| atom | 3610 | NH1 | ARG | 465 | 45.481 | 11.002 | 34.303 | 1.00 | 51.31 |
| atom | 3611 | NH2 | ARG | 465 | 45.711 | 13.276 | 34.421 | 1.00 | 50.41 |
| atom | 3612 | C | ARG | 465 | 40.700 | 7.524 | 34.864 | 1.00 | 22.97 |
| atom | 3613 | O | ARG | 465 | 39.681 | 8.210 | 34.986 | 1.00 | 17.94 |
| atom | 3614 | N | LEU | 466 | 40.800 | 6.486 | 34.031 | 1.00 | 19.59 |
| atom | 3615 | CA | LEU | 466 | 39.729 | 6.087 | 33.122 | 1.00 | 24.29 |
| atom | 3616 | CB | LEU | 466 | 40.314 | 5.360 | 31.904 | 1.00 | 22.68 |
| atom | 3617 | CG | LEU | 466 | 41.009 | 6.289 | 30.905 | 1.00 | 31.93 |
| atom | 3618 | CD1 | LEU | 466 | 41.374 | 5.545 | 29.624 | 1.00 | 31.87 |
| atom | 3619 | CD2 | LEU | 466 | 40.074 | 7.457 | 30.599 | 1.00 | 36.14 |
| atom | 3620 | C | LEU | 466 | 38.609 | 5.227 | 33.692 | 1.00 | 27.95 |
| atom | 3621 | O | LEU | 466 | 37.461 | 5.286 | 33.219 | 1.00 | 30.02 |
| atom | 3622 | N | HIS | 467 | 38.923 | 4.420 | 34.693 | 1.00 | 26.38 |
| atom | 3623 | CA | HIS | 467 | 37.901 | 3.554 | 35.240 | 1.00 | 28.67 |
| atom | 3624 | CB | HIS | 467 | 38.215 | 2.094 | 34.905 | 1.00 | 28.83 |
| atom | 3625 | CG | HIS | 467 | 38.546 | 1.853 | 33.465 | 1.00 | 28.42 |
| atom | 3626 | CD2 | HIS | 467 | 39.716 | 1.958 | 32.789 | 1.00 | 28.48 |
| atom | 3627 | ND1 | HIS | 467 | 37.620 | 1.385 | 32.557 | 1.00 | 29.37 |
| atom | 3628 | CE1 | HIS | 467 | 38.205 | 1.209 | 31.386 | 1.00 | 23.72 |
| atom | 3629 | NE2 | HIS | 467 | 39.478 | 1.549 | 31.501 | 1.00 | 26.25 |
| atom | 3630 | C | HIS | 467 | 37.681 | 3.673 | 36.734 | 1.00 | 28.76 |
| atom | 3631 | O | HIS | 467 | 36.669 | 3.188 | 37.246 | 1.00 | 31.17 |
| atom | 3632 | N | GLY | 468 | 38.611 | 4.313 | 37.435 | 1.00 | 27.32 |

116

| atom | 3633 | CA | GLY | 468 | 38.475 | 4.421 | 38.875 | 1.00 | 24.96 |
|------|------|-----|-----|-----|--------|--------|--------|------|-------|
| atom | 3634 | C | GLY | 468 | 39.180 | 3.265 | 39.570 | 1.00 | 26.11 |
| atom | 3635 | 0 | GLY | 468 | 39.347 | 2.187 | 38.992 | 1.00 | 20.04 |
| atom | 3636 | N | LEU | 469 | 39.604 | 3.504 | 40.810 | 1.00 | 28.32 |
| atom | 3637 | CA | LEU | 469 | 40.303 | 2.506 | 41.619 | 1.00 | 31.09 |
| atom | 3638 | CB | LEU | 469 | 40.497 | 3.027 | 43.050 | 1.00 | 34.04 |
| atom | 3639 | CG | LEU | 469 | 41.631 | 2.433 | 43.893 | 1.00 | 33.39 |
| atom | 3640 | CD1 | LEU | 469 | 42.976 | 2.827 | 43.310 | 1.00 | 29.13 |
| atom | 3641 | CD2 | LEU | 469 | 41.509 | 2.939 | 45.332 | 1.00 | 36.67 |
| atom | 3642 | C | LEU | 469 | 39.572 | 1.177 | 41.669 | 1.00 | 30.69 |
| atom | 3643 | 0 | LEU | 469 | 40.203 | 0.121 | 41.757 | 1.00 | 32.77 |
| atom | 3644 | N | SER | 470 | 38.243 | 1.240 | 41.617 | 1.00 | 28.25 |
| atom | 3645 | CA | SER | 470 | 37.399 | 0.050 | 41.658 | 1.00 | 26.30 |
| atom | 3646 | CB | SER | 470 | 35.967 | 0.416 | 41.294 | 1.00 | 29.68 |
| atom | 3647 | OG | SER | 470 | 35.871 | 0.665 | 39.903 | 1.00 | 32.61 |
| atom | 3648 | C | SER | 470 | 37.896 | -0.988 | 40.677 | 1.00 | 26.83 |
| atom | 3649 | 0 | SER | 470 | 37.786 | -2.187 | 40.908 | 1.00 | 29.66 |
| atom | 3650 | N | ALA | 471 | 38.433 | -0.501 | 39.569 | 1.00 | 27.16 |
| atom | 3651 | CA | ALA | 471 | 38.957 | -1.346 | 38.518 | 1.00 | 25.29 |
| atom | 3652 | CB | ALA | 471 | 39.607 | -0.481 | 37.436 | 1.00 | 23.68 |
| atom | 3653 | C | ALA | 471 | 39.970 | -2.327 | 39.081 | 1.00 | 24.06 |
| atom | 3654 | 0 | ALA | 471 | 40.142 | -3.416 | 38.556 | 1.00 | 21.95 |
| atom | 3655 | N | PHE | 472 | 40.648 | -1.940 | 40.151 | 1.00 | 22.90 |
| atom | 3656 | CA | PHE | 472 | 41.631 | -2.831 | 40.745 | 1.00 | 22.67 |
| atom | 3657 | CB | PHE | 472 | 42.758 | -2.010 | 41.375 | 1.00 | 21.44 |
| atom | 3658 | CG | PHE | 472 | 43.412 | -1.035 | 40.424 | 1.00 | 22.73 |
| atom | 3659 | CD1 | PHE | 472 | 43.519 | 0.321 | 40.750 | 1.00 | 25.96 |
| atom | 3660 | CD2 | PHE | 472 | 43.936 | -1.467 | 39.207 | 1.00 | 22.57 |
| atom | 3661 | CE1 | PHE | 472 | 44.145 | 1.241 | 39.863 | 1.00 | 24.45 |
| atom | 3662 | CE2 | PHE | 472 | 44.567 | -0.560 | 38.312 | 1.00 | 19.58 |
| atom | 3663 | CZ | PHE | 472 | 44.668 | 0.791 | 38.642 | 1.00 | 18.43 |
| atom | 3664 | C | PHE | 472 | 41.045 | -3.806 | 41.780 | 1.00 | 19.61 |
| atom | 3665 | 0 | PHE | 472 | 41.796 | -4.509 | 42.433 | 1.00 | 20.06 |
| atom | 3666 | N | SER | 473 | 39.717 | -3.887 | 41.889 | 1.00 | 17.33 |
| atom | 3667 | CA | SER | 473 | 39.087 | -4.771 | 42.864 | 1.00 | 19.26 |
| atom | 3668 | CB | SER | 473 | 38.894 | -4.015 | 44.182 | 1.00 | 15.31 |

| atom | 3669 | OG | SER | 473 | 39.568 | -2.768 | 44.152 | 1.00 | 22.47 |
|------|------|-----|-----|-----|--------|--------|--------|------|-------|
| atom | 3670 | C | SER | 473 | 37.747 | -5.447 | 42.501 | 1.00 | 24.97 |
| atom | 3671 | O | SER | 473 | 37.088 | -6.025 | 43.390 | 1.00 | 26.79 |
| atom | 3672 | N | LEU | 474 | 37.322 | -5.384 | 41.239 | 1.00 | 19.87 |
| atom | 3673 | CA | LEU | 474 | 36.064 | -6.032 | 40.853 | 1.00 | 17.55 |
| atom | 3674 | CB | LEU | 474 | 35.741 | -5.734 | 39.398 | 1.00 | 16.67 |
| atom | 3675 | CG | LEU | 474 | 35.419 | -4.253 | 39.139 | 1.00 | 19.81 |
| atom | 3676 | CD1 | LEU | 474 | 35.037 | -4.078 | 37.684 | 1.00 | 16.66 |
| atom | 3677 | CD2 | LEU | 474 | 34.284 | -3.777 | 40.027 | 1.00 | 19.28 |
| atom | 3678 | C | LEU | 474 | 36.195 | -7.544 | 41.097 | 1.00 | 16.76 |
| atom | 3679 | O | LEU | 474 | 37.261 | -8.115 | 40.867 | 1.00 | 14.80 |
| atom | 3680 | N | HIS | 475 | 35.122 | -8.183 | 41.577 | 1.00 | 17.09 |
| atom | 3681 | CA | HIS | 475 | 35.189 | -9.595 | 41.917 | 1.00 | 20.48 |
| atom | 3682 | CB | HIS | 475 | 35.651 | -9.737 | 43.382 | 1.00 | 23.75 |
| atom | 3683 | CG | HIS | 475 | 34.738 | -9.069 | 44.373 | 1.00 | 23.68 |
| atom | 3684 | CD2 | HIS | 475 | 34.748 | -7.811 | 44.893 | 1.00 | 23.89 |
| atom | 3685 | ND1 | HIS | 475 | 33.598 | -9.668 | 44.857 | 1.00 | 15.87 |
| atom | 3686 | CE1 | HIS | 475 | 32.939 | -8.815 | 45.623 | 1.00 | 18.64 |
| atom | 3687 | NE2 | HIS | 475 | 33.619 | -7.680 | 45.660 | 1.00 | 17.92 |
| atom | 3688 | C | HIS | 475 | 33.972 | -10.490 | 41.703 | 1.00 | 23.35 |
| atom | 3689 | O | HIS | 475 | 34.113 | -11.667 | 41.395 | 1.00 | 32.81 |
| atom | 3690 | N | SER | 476 | 32.772 | -9.986 | 41.864 | 1.00 | 23.75 |
| atom | 3691 | CA | SER | 476 | 31.647 | -10.896 | 41.683 | 1.00 | 25.31 |
| atom | 3692 | CB | SER | 476 | 30.632 | -10.674 | 42.808 | 1.00 | 25.03 |
| atom | 3693 | OG | SER | 476 | 31.256 | -10.944 | 44.047 | 1.00 | 25.05 |
| atom | 3694 | C | SER | 476 | 31.016 | -10.707 | 40.311 | 1.00 | 26.91 |
| atom | 3695 | O | SER | 476 | 29.891 | -10.203 | 40.189 | 1.00 | 21.63 |
| atom | 3696 | N | TYR | 477 | 31.774 | -11.114 | 39.287 | 1.00 | 27.12 |
| atom | 3697 | CA | TYR | 477 | 31.382 | -10.999 | 37.888 | 1.00 | 20.57 |
| atom | 3698 | CB | TYR | 477 | 32.517 | -11.476 | 36.980 | 1.00 | 19.34 |
| atom | 3699 | CG | TYR | 477 | 33.662 | -10.492 | 36.852 | 1.00 | 18.80 |
| atom | 3700 | CD1 | TYR | 477 | 34.885 | -10.722 | 37.514 | 1.00 | 19.07 |
| atom | 3701 | CE1 | TYR | 477 | 35.955 | -9.831 | 37.400 | 1.00 | 13.98 |
| atom | 3702 | CD2 | TYR | 477 | 33.541 | -9.339 | 36.067 | 1.00 | 13.86 |
| atom | 3703 | CE2 | TYR | 477 | 34.615 | -8.434 | 35.942 | 1.00 | 13.49 |
| atom | 3704 | CZ | TYR | 477 | 35.817 | -8.689 | 36.612 | 1.00 | 16.89 |

| atom | 3705 | OH | TYR | 477 | 36.878 | -7.817 | 36.501 | 1.00 | 14.53 |
|------|------|-----|-----|-----|--------|---------|--------|------|-------|
| atom | 3706 | C | TYR | 477 | 30.126 | -11.777 | 37.577 | 1.00 | 21.86 |
| atom | 3707 | O | TYR | 477 | 29.913 | -12.852 | 38.113 | 1.00 | 16.61 |
| atom | 3708 | N | SER | 478 | 29.304 | -11.219 | 36.686 | 1.00 | 25.77 |
| atom | 3709 | CA | SER | 478 | 28.030 | -11.820 | 36.285 | 1.00 | 27.29 |
| atom | 3710 | CB | SER | 478 | 27.289 | -10.875 | 35.339 | 1.00 | 28.80 |
| atom | 3711 | OG | SER | 478 | 27.483 | -9.522 | 35.731 | 1.00 | 40.31 |
| atom | 3712 | C | SER | 478 | 28.165 | -13.186 | 35.628 | 1.00 | 27.44 |
| atom | 3713 | O | SER | 478 | 29.099 | -13.423 | 34.876 | 1.00 | 31.57 |
| atom | 3714 | N | PRO | 479 | 27.229 | -14.109 | 35.910 | 1.00 | 25.94 |
| atom | 3715 | CD | PRO | 479 | 26.087 | -13.974 | 36.828 | 1.00 | 22.83 |
| atom | 3716 | CA | PRO | 479 | 27.286 | -15.450 | 35.314 | 1.00 | 21.99 |
| atom | 3717 | CB | PRO | 479 | 26.038 | -16.131 | 35.859 | 1.00 | 21.63 |
| atom | 3718 | CG | PRO | 479 | 25.779 | -15.415 | 37.166 | 1.00 | 24.95 |
| atom | 3719 | C | PRO | 479 | 27.302 | -15.373 | 33.790 | 1.00 | 22.09 |
| atom | 3720 | O | PRO | 479 | 27.898 | -16.206 | 33.104 | 1.00 | 16.28 |
| atom | 3721 | N | GLY | 480 | 26.642 | -14.362 | 33.253 | 1.00 | 25.56 |
| atom | 3722 | CA | GLY | 480 | 26.652 | -14.222 | 31.808 | 1.00 | 30.51 |
| atom | 3723 | C | GLY | 480 | 28.071 | -13.966 | 31.315 | 1.00 | 29.37 |
| atom | 3724 | O | GLY | 480 | 28.544 | -14.612 | 30.382 | 1.00 | 32.55 |
| atom | 3725 | N | GLU | 481 | 28.757 | -13.031 | 31.971 | 1.00 | 28.12 |
| atom | 3726 | CA | GLU | 481 | 30.112 | -12.654 | 31.609 | 1.00 | 23.84 |
| atom | 3727 | CB | GLU | 481 | 30.521 | -11.414 | 32.406 | 1.00 | 20.37 |
| atom | 3728 | CG | GLU | 481 | 31.903 | -10.903 | 32.070 | 1.00 | 21.27 |
| atom | 3729 | CD | GLU | 481 | 31.929 | -10.215 | 30.731 | 1.00 | 19.94 |
| atom | 3730 | OE1 | GLU | 481 | 30.873 | -10.180 | 30.074 | 1.00 | 20.91 |
| atom | 3731 | OE2 | GLU | 481 | 32.994 | -9.713 | 30.343 | 1.00 | 14.84 |
| atom | 3732 | C | GLU | 481 | 31.123 | -13.782 | 31.811 | 1.00 | 23.04 |
| atom | 3733 | O | GLU | 481 | 31.966 | -14.028 | 30.958 | 1.00 | 25.54 |
| atom | 3734 | N | ILE | 482 | 31.040 | -14.470 | 32.937 | 1.00 | 23.36 |
| atom | 3735 | CA | ILE | 482 | 31.963 | -15.565 | 33.220 | 1.00 | 22.76 |
| atom | 3736 | CB | ILE | 482 | 31.638 | -16.214 | 34.601 | 1.00 | 19.81 |
| atom | 3737 | CG2 | ILE | 482 | 32.395 | -17.523 | 34.778 | 1.00 | 15.83 |
| atom | 3738 | CG1 | ILE | 482 | 32.010 | -15.250 | 35.739 | 1.00 | 23.83 |
| atom | 3739 | CD1 | ILE | 482 | 31.125 | -15.395 | 36.991 | 1.00 | 10.56 |
| atom | 3740 | C | ILE | 482 | 31.881 | -16.636 | 32.132 | 1.00 | 22.80 |

| atom | 3741 | O | ILE | 482 | 32.896 | -17.185 | 31.692 | 1.00 | 19.29 |
|------|------|------|------|-----|--------|---------|--------|------|-------|
| atom | 3742 | N | ASN | 483 | 30.659 | -16.923 | 31.699 | 1.00 | 24.99 |
| atom | 3743 | CA | ASN | 483 | 30.435 | -17.945 | 30.698 | 1.00 | 26.00 |
| atom | 3744 | CB | ASN | 483 | 28.969 | -18.345 | 30.716 | 1.00 | 28.37 |
| atom | 3745 | CG | ASN | 483 | 28.590 | -19.073 | 32.002 | 1.00 | 31.91 |
| atom | 3746 | OD1 | ASN | 483 | 27.471 | -18.946 | 32.500 | 1.00 | 30.31 |
| atom | 3747 | ND2 | ASN | 483 | 29.530 | -19.841 | 32.542 | 1.00 | 29.92 |
| atom | 3748 | C | ASN | 483 | 30.877 | -17.563 | 29.300 | 1.00 | 26.60 |
| atom | 3749 | O | ASN | 483 | 31.177 | -18.434 | 28.497 | 1.00 | 30.66 |
| atom | 3750 | N | ARG | 484 | 30.902 | -16.271 | 28.997 | 1.00 | 27.04 |
| atom | 3751 | CA | ARG | 484 | 31.363 | -15.830 | 27.687 | 1.00 | 27.37 |
| atom | 3752 | CB | ARG | 484 | 31.201 | -14.317 | 27.520 | 1.00 | 26.13 |
| atom | 3753 | CG | ARG | 484 | 30.361 | -13.921 | 26.325 | 1.00 | 21.21 |
| atom | 3754 | CD | ARG | 484 | 30.981 | -12.821 | 25.556 | 1.00 | 17.82 |
| atom | 3755 | NE | ARG | 484 | 31.222 | -11.629 | 26.361 | 1.00 | 19.84 |
| atom | 3756 | CZ | ARG | 484 | 30.484 | -10.531 | 26.282 | 1.00 | 18.20 |
| atom | 3757 | NH1 | ARG | 484 | 29.462 | -10.472 | 25.447 | 1.00 | 22.95 |
| atom | 3758 | NH2 | ARG | 484 | 30.792 | -9.477 | 27.005 | 1.00 | 17.83 |
| atom | 3759 | C | ARG | 484 | 32.841 | -16.174 | 27.637 | 1.00 | 26.63 |
| atom | 3760 | O | ARG | 484 | 33.268 | -17.021 | 26.861 | 1.00 | 27.51 |
| atom | 3761 | N | VAL | 485 | 33.612 | -15.507 | 28.491 | 1.00 | 25.94 |
| atom | 3762 | CA | VAL | 485 | 35.048 | -15.721 | 28.581 | 1.00 | 20.15 |
| atom | 3763 | CB | VAL | 485 | 35.633 | -15.070 | 29.836 | 1.00 | 21.83 |
| atom | 3764 | CG1 | VAL | 485 | 37.121 | -15.461 | 29.970 | 1.00 | 13.97 |
| atom | 3765 | CG2 | VAL | 485 | 35.419 | -13.566 | 29.793 | 1.00 | 7.66 |
| atom | 3766 | C | VAL | 485 | 35.399 | -17.191 | 28.660 | 1.00 | 19.86 |
| atom | 3767 | O | VAL | 485 | 36.117 | -17.710 | 27.815 | 1.00 | 21.45 |
| atom | 3768 | N | ALA | 486 | 34.900 | -17.847 | 29.699 | 1.00 | 20.86 |
| atom | 3769 | CA | ALA | 486 | 35.175 | -19.265 | 29.928 | 1.00 | 23.05 |
| atom | 3770 | CB | ALA | 486 | 34.380 | -19.781 | 31.157 | 1.00 | 16.74 |
| atom | 3771 | C | ALA | 486 | 34.888 | -20.127 | 28.704 | 1.00 | 19.32 |
| atom | 3772 | O | ALA | 486 | 35.545 | -21.134 | 28.492 | 1.00 | 18.46 |
| atom | 3773 | N | SER | 487 | 33.898 | -19.746 | 27.910 | 1.00 | 23.08 |
| atom | 3774 | CA | SER | 487 | 33.594 | -20.493 | 26.699 | 1.00 | 25.52 |
| atom | 3775 | CB | SER | 487 | 32.197 | -20.141 | 26.176 | 1.00 | 26.82 |
| atom | 3776 | OG | SER | 487 | 31.572 | -21.282 | 25.597 | 1.00 | 32.23 |

| atom | 3777 | C   | SER | 487 | 34.642 | -20.132 | 25.646 | 1.00 | 23.85 |
|------|------|-----|-----|-----|--------|---------|--------|------|-------|
| atom | 3778 | O   | SER | 487 | 35.121 | -20.981 | 24.903 | 1.00 | 30.98 |
| atom | 3779 | N   | CYS | 488 | 35.004 | -18.865 | 25.584 | 1.00 | 20.37 |
| atom | 3780 | CA  | CYS | 488 | 35.991 | -18.441 | 24.617 | 1.00 | 21.95 |
| atom | 3781 | CB  | CYS | 488 | 36.079 | -16.918 | 24.589 | 1.00 | 27.12 |
| atom | 3782 | SG  | CYS | 488 | 37.671 | -16.295 | 23.964 | 1.00 | 35.96 |
| atom | 3783 | C   | CYS | 488 | 37.374 | -19.017 | 24.911 | 1.00 | 22.98 |
| atom | 3784 | O   | CYS | 488 | 38.218 | -19.080 | 24.019 | 1.00 | 16.47 |
| atom | 3785 | N   | LEU | 489 | 37.629 | -19.425 | 26.153 | 1.00 | 21.12 |
| atom | 3786 | CA  | LEU | 489 | 38.949 | -19.966 | 26.456 | 1.00 | 24.98 |
| atom | 3787 | CB  | LEU | 489 | 39.232 | -19.926 | 27.959 | 1.00 | 23.49 |
| atom | 3788 | CG  | LEU | 489 | 39.134 | -18.554 | 28.631 | 1.00 | 24.48 |
| atom | 3789 | CD1 | LEU | 489 | 39.791 | -18.618 | 30.008 | 1.00 | 27.39 |
| atom | 3790 | CD2 | LEU | 489 | 39.794 | -17.494 | 27.765 | 1.00 | 15.03 |
| atom | 3791 | C   | LEU | 489 | 39.055 | -21.382 | 25.912 | 1.00 | 28.28 |
| atom | 3792 | O   | LEU | 489 | 40.080 | -21.754 | 25.336 | 1.00 | 28.63 |
| atom | 3793 | N   | ARG | 490 | 37.987 | -22.162 | 26.077 | 1.00 | 29.70 |
| atom | 3794 | CA  | ARG | 490 | 37.972 | -23.531 | 25.559 | 1.00 | 35.67 |
| atom | 3795 | CB  | ARG | 490 | 36.630 | -24.241 | 25.837 | 1.00 | 34.47 |
| atom | 3796 | CG  | ARG | 490 | 35.916 | -23.839 | 27.102 | 1.00 | 39.26 |
| atom | 3797 | CD  | ARG | 490 | 36.649 | -24.331 | 28.330 | 1.00 | 46.84 |
| atom | 3798 | NE  | ARG | 490 | 37.425 | -25.530 | 28.036 | 1.00 | 49.75 |
| atom | 3799 | CZ  | ARG | 490 | 38.709 | -25.685 | 28.338 | 1.00 | 48.63 |
| atom | 3800 | NH1 | ARG | 490 | 39.375 | -24.709 | 28.953 | 1.00 | 50.68 |
| atom | 3801 | NH2 | ARG | 490 | 39.324 | -26.813 | 28.015 | 1.00 | 45.57 |
| atom | 3802 | C   | ARG | 490 | 38.173 | -23.455 | 24.042 | 1.00 | 33.22 |
| atom | 3803 | O   | ARG | 490 | 39.071 | -24.085 | 23.488 | 1.00 | 34.18 |
| atom | 3804 | N   | LYS | 491 | 37.331 | -22.651 | 23.396 | 1.00 | 33.18 |
| atom | 3805 | CA  | LYS | 491 | 37.350 | -22.469 | 21.949 | 1.00 | 30.62 |
| atom | 3806 | CB  | LYS | 491 | 36.399 | -21.339 | 21.551 | 1.00 | 31.61 |
| atom | 3807 | CG  | LYS | 491 | 36.178 | -21.210 | 20.053 | 1.00 | 23.96 |
| atom | 3808 | CD  | LYS | 491 | 36.306 | -19.782 | 19.606 | 1.00 | 19.54 |
| atom | 3809 | CE  | LYS | 491 | 34.973 | -19.080 | 19.677 | 1.00 | 17.94 |
| atom | 3810 | NZ  | LYS | 491 | 35.126 | -17.705 | 20.232 | 1.00 | 17.67 |
| atom | 3811 | C   | LYS | 491 | 38.722 | -22.181 | 21.384 | 1.00 | 29.09 |
| atom | 3812 | O   | LYS | 491 | 39.187 | -22.897 | 20.503 | 1.00 | 35.24 |

| atom | 3813 | N | LEU | 492 | 39.369 | -21.138 | 21.893 | 1.00 | 24.34 |
|------|------|-----|-----|-----|--------|---------|--------|------|-------|
| atom | 3814 | CA | LEU | 492 | 40.679 | -20.755 | 21.401 | 1.00 | 19.50 |
| atom | 3815 | CB | LEU | 492 | 40.993 | -19.286 | 21.743 | 1.00 | 19.76 |
| atom | 3816 | CG | LEU | 492 | 40.131 | -18.223 | 21.032 | 1.00 | 21.61 |
| atom | 3817 | CD1 | LEU | 492 | 40.670 | -16.834 | 21.255 | 1.00 | 14.82 |
| atom | 3818 | CD2 | LEU | 492 | 40.088 | -18.522 | 19.560 | 1.00 | 20.60 |
| atom | 3819 | C | LEU | 492 | 41.763 | -21.639 | 21.949 | 1.00 | 19.62 |
| atom | 3820 | O | LEU | 492 | 42.872 | -21.679 | 21.413 | 1.00 | 19.94 |
| atom | 3821 | N | GLY | 493 | 41.447 | -22.362 | 23.014 | 1.00 | 20.58 |
| atom | 3822 | CA | GLY | 493 | 42.451 | -23.220 | 23.615 | 1.00 | 15.08 |
| atom | 3823 | C | GLY | 493 | 43.358 | -22.412 | 24.528 | 1.00 | 17.40 |
| atom | 3824 | O | GLY | 493 | 44.549 | -22.733 | 24.699 | 1.00 | 12.58 |
| atom | 3825 | N | VAL | 494 | 42.811 | -21.332 | 25.085 | 1.00 | 18.23 |
| atom | 3826 | CA | VAL | 494 | 43.567 | -20.505 | 26.029 | 1.00 | 24.48 |
| atom | 3827 | CB | VAL | 494 | 42.927 | -19.093 | 26.182 | 1.00 | 25.08 |
| atom | 3828 | CG1 | VAL | 494 | 43.639 | -18.302 | 27.263 | 1.00 | 19.85 |
| atom | 3829 | CG2 | VAL | 494 | 43.032 | -18.344 | 24.869 | 1.00 | 23.44 |
| atom | 3830 | C | VAL | 494 | 43.523 | -21.267 | 27.369 | 1.00 | 21.96 |
| atom | 3831 | O | VAL | 494 | 42.544 | -21.966 | 27.650 | 1.00 | 23.77 |
| atom | 3832 | N | PRO | 495 | 44.591 | -21.193 | 28.180 | 1.00 | 19.53 |
| atom | 3833 | CD | PRO | 495 | 45.876 | -20.502 | 27.998 | 1.00 | 22.34 |
| atom | 3834 | CA | PRO | 495 | 44.532 | -21.925 | 29.452 | 1.00 | 23.72 |
| atom | 3835 | CB | PRO | 495 | 45.966 | -21.847 | 29.992 | 1.00 | 22.00 |
| atom | 3836 | CG | PRO | 495 | 46.564 | -20.672 | 29.327 | 1.00 | 22.88 |
| atom | 3837 | C | PRO | 495 | 43.495 | -21.359 | 30.428 | 1.00 | 24.18 |
| atom | 3838 | O | PRO | 495 | 43.237 | -20.166 | 30.425 | 1.00 | 26.06 |
| atom | 3839 | N | PRO | 496 | 42.867 | -22.227 | 31.253 | 1.00 | 29.06 |
| atom | 3840 | CD | PRO | 496 | 43.117 | -23.682 | 31.251 | 1.00 | 29.39 |
| atom | 3841 | CA | PRO | 496 | 41.843 | -21.870 | 32.256 | 1.00 | 26.64 |
| atom | 3842 | CB | PRO | 496 | 41.639 | -23.175 | 33.025 | 1.00 | 26.28 |
| atom | 3843 | CG | PRO | 496 | 41.942 | -24.230 | 32.014 | 1.00 | 27.86 |
| atom | 3844 | C | PRO | 496 | 42.220 | -20.709 | 33.191 | 1.00 | 24.39 |
| atom | 3845 | O | PRO | 496 | 43.382 | -20.560 | 33.560 | 1.00 | 25.02 |
| atom | 3846 | N | LEU | 497 | 41.235 | -19.901 | 33.580 | 1.00 | 22.77 |
| atom | 3847 | CA | LEU | 497 | 41.479 | -18.762 | 34.476 | 1.00 | 25.05 |
| atom | 3848 | CB | LEU | 497 | 40.174 | -18.055 | 34.832 | 1.00 | 22.12 |

| atom | 3849 | CG | LEU | 497 | 39.459 | -17.316 | 33.694 | 1.00 | 24.99 |
|------|------|-----|-----|-----|--------|---------|--------|------|-------|
| atom | 3850 | CD1 | LEU | 497 | 37.983 | -17.213 | 34.013 | 1.00 | 10.42 |
| atom | 3851 | CD2 | LEU | 497 | 40.089 | -15.948 | 33.486 | 1.00 | 22.67 |
| atom | 3852 | C | LEU | 497 | 42.171 | -19.192 | 35.767 | 1.00 | 28.83 |
| atom | 3853 | O | LEU | 497 | 43.112 | -18.534 | 36.231 | 1.00 | 30.24 |
| atom | 3854 | N | ARG | 498 | 41.709 | -20.291 | 36.354 | 1.00 | 28.96 |
| atom | 3855 | CA | ARG | 498 | 42.318 | -20.776 | 37.585 | 1.00 | 31.80 |
| atom | 3856 | CB | ARG | 498 | 41.849 | -22.192 | 37.881 | 1.00 | 33.41 |
| atom | 3857 | CG | ARG | 498 | 42.803 | -23.240 | 37.364 | 1.00 | 36.66 |
| atom | 3858 | CD | ARG | 498 | 42.101 | -24.168 | 36.443 | 1.00 | 34.50 |
| atom | 3859 | NE | ARG | 498 | 41.020 | -24.850 | 37.130 | 1.00 | 35.78 |
| atom | 3860 | CZ | ARG | 498 | 40.765 | -26.148 | 37.006 | 1.00 | 41.82 |
| atom | 3861 | NH1 | ARG | 498 | 41.523 | -26.904 | 36.215 | 1.00 | 41.86 |
| atom | 3862 | NH2 | ARG | 498 | 39.751 | -26.691 | 37.672 | 1.00 | 40.78 |
| atom | 3863 | C | ARG | 498 | 43.829 | -20.769 | 37.416 | 1.00 | 30.31 |
| atom | 3864 | O | ARG | 498 | 44.561 | -20.349 | 38.310 | 1.00 | 33.99 |
| atom | 3865 | N | VAL | 499 | 44.285 | -21.216 | 36.250 | 1.00 | 29.25 |
| atom | 3866 | CA | VAL | 499 | 45.712 | -21.278 | 35.947 | 1.00 | 31.50 |
| atom | 3867 | CB | VAL | 499 | 45.972 | -22.101 | 34.640 | 1.00 | 33.12 |
| atom | 3868 | CG1 | VAL | 499 | 47.390 | -21.877 | 34.132 | 1.00 | 28.70 |
| atom | 3869 | CG2 | VAL | 499 | 45.735 | -23.587 | 34.906 | 1.00 | 31.44 |
| atom | 3870 | C | VAL | 499 | 46.377 | -19.901 | 35.829 | 1.00 | 31.96 |
| atom | 3871 | O | VAL | 499 | 47.547 | -19.753 | 36.190 | 1.00 | 35.00 |
| atom | 3872 | N | TRP | 500 | 45.648 | -18.901 | 35.324 | 1.00 | 31.77 |
| atom | 3873 | CA | TRP | 500 | 46.198 | -17.546 | 35.181 | 1.00 | 27.36 |
| atom | 3874 | CB | TRP | 500 | 45.277 | -16.663 | 34.333 | 1.00 | 25.16 |
| atom | 3875 | CG | TRP | 500 | 45.213 | -17.041 | 32.882 | 1.00 | 26.28 |
| atom | 3876 | CD2 | TRP | 500 | 46.174 | -16.737 | 31.862 | 1.00 | 26.06 |
| atom | 3877 | CE2 | TRP | 500 | 45.698 | -17.303 | 30.658 | 1.00 | 27.61 |
| atom | 3878 | CE3 | TRP | 500 | 47.386 | -16.039 | 31.846 | 1.00 | 29.91 |
| atom | 3879 | CD1 | TRP | 500 | 44.225 | -17.760 | 32.270 | 1.00 | 25.36 |
| atom | 3880 | NE1 | TRP | 500 | 44.510 | -17.921 | 30.938 | 1.00 | 25.93 |
| atom | 3881 | CZ2 | TRP | 500 | 46.396 | -17.200 | 29.451 | 1.00 | 30.21 |
| atom | 3882 | CZ3 | TRP | 500 | 48.083 | -15.934 | 30.645 | 1.00 | 31.35 |
| atom | 3883 | CH2 | TRP | 500 | 47.583 | -16.510 | 29.461 | 1.00 | 32.07 |
| atom | 3884 | C | TRP | 500 | 46.366 | -16.926 | 36.574 | 1.00 | 28.41 |

| atom | 3885 | O   | TRP | 500 | 47.248 | -16.073 | 36.803 | 1.00 | 23.28 |
|------|------|-----|-----|-----|--------|---------|--------|------|-------|
| atom | 3886 | N   | ARG | 501 | 45.507 | -17.361 | 37.496 | 1.00 | 27.19 |
| atom | 3887 | CA  | ARG | 501 | 45.556 | -16.910 | 38.880 | 1.00 | 28.97 |
| atom | 3888 | CB  | ARG | 501 | 44.641 | -17.780 | 39.747 | 1.00 | 37.11 |
| atom | 3889 | CG  | ARG | 501 | 43.168 | -17.454 | 39.655 | 1.00 | 39.64 |
| atom | 3890 | CD  | ARG | 501 | 42.755 | -16.596 | 40.834 | 1.00 | 51.11 |
| atom | 3891 | NE  | ARG | 501 | 41.304 | -16.451 | 40.928 | 1.00 | 56.63 |
| atom | 3892 | CZ  | ARG | 501 | 40.664 | -16.140 | 42.048 | 1.00 | 59.94 |
| atom | 3893 | NH1 | ARG | 501 | 41.360 | -15.942 | 43.162 | 1.00 | 60.33 |
| atom | 3894 | NH2 | ARG | 501 | 39.334 | -16.048 | 42.059 | 1.00 | 58.22 |
| atom | 3895 | C   | ARG | 501 | 46.993 | -17.084 | 39.359 | 1.00 | 28.69 |
| atom | 3896 | O   | ARG | 501 | 47.698 | -16.119 | 39.671 | 1.00 | 26.56 |
| atom | 3897 | N   | HIS | 502 | 47.425 | -18.339 | 39.391 | 1.00 | 25.63 |
| atom | 3898 | CA  | HIS | 502 | 48.770 | -18.675 | 39.831 | 1.00 | 28.17 |
| atom | 3899 | CB  | HIS | 502 | 48.879 | -20.202 | 40.022 | 1.00 | 31.75 |
| atom | 3900 | CG  | HIS | 502 | 48.016 | -20.733 | 41.133 | 1.00 | 36.89 |
| atom | 3901 | CD2 | HIS | 502 | 46.691 | -20.583 | 41.387 | 1.00 | 40.38 |
| atom | 3902 | ND1 | HIS | 502 | 48.513 | -21.507 | 42.159 | 1.00 | 32.91 |
| atom | 3903 | CE1 | HIS | 502 | 47.535 | -21.809 | 42.995 | 1.00 | 34.77 |
| atom | 3904 | NE2 | HIS | 502 | 46.419 | -21.262 | 42.550 | 1.00 | 35.75 |
| atom | 3905 | C   | HIS | 502 | 49.858 | -18.148 | 38.888 | 1.00 | 27.48 |
| atom | 3906 | O   | HIS | 502 | 50.984 | -17.881 | 39.306 | 1.00 | 29.54 |
| atom | 3907 | N   | ARG | 503 | 49.540 | -17.997 | 37.612 | 1.00 | 30.63 |
| atom | 3908 | CA  | ARG | 503 | 50.531 | -17.470 | 36.692 | 1.00 | 29.02 |
| atom | 3909 | CB  | ARG | 503 | 49.992 | -17.498 | 35.259 | 1.00 | 29.73 |
| atom | 3910 | CG  | ARG | 503 | 50.645 | -18.533 | 34.351 | 1.00 | 29.98 |
| atom | 3911 | CD  | ARG | 503 | 49.619 | -19.560 | 33.919 | 1.00 | 30.05 |
| atom | 3912 | NE  | ARG | 503 | 50.134 | -20.487 | 32.927 | 1.00 | 30.15 |
| atom | 3913 | CZ  | ARG | 503 | 49.764 | -20.485 | 31.652 | 1.00 | 33.95 |
| atom | 3914 | NH1 | ARG | 503 | 48.878 | -19.599 | 31.222 | 1.00 | 38.59 |
| atom | 3915 | NH2 | ARG | 503 | 50.262 | -21.376 | 30.808 | 1.00 | 32.53 |
| atom | 3916 | C   | ARG | 503 | 50.807 | -16.033 | 37.134 | 1.00 | 28.79 |
| atom | 3917 | O   | ARG | 503 | 51.964 | -15.622 | 37.248 | 1.00 | 28.60 |
| atom | 3918 | N   | ALA | 504 | 49.732 | -15.284 | 37.402 | 1.00 | 30.13 |
| atom | 3919 | CA  | ALA | 504 | 49.833 | -13.880 | 37.832 | 1.00 | 29.74 |
| atom | 3920 | CB  | ALA | 504 | 48.439 | -13.224 | 37.834 | 1.00 | 28.52 |

| atom | 3921 | C | ALA | 504 | 50.513 | -13.701 | 39.200 | 1.00 | 26.24 |
|------|------|-----|-----|-----|--------|---------|--------|------|-------|
| atom | 3922 | O | ALA | 504 | 51.160 | -12.681 | 39.436 | 1.00 | 27.81 |
| atom | 3923 | N | ARG | 505 | 50.353 | -14.676 | 40.098 | 1.00 | 28.84 |
| atom | 3924 | CA | ARG | 505 | 50.995 | -14.634 | 41.419 | 1.00 | 27.37 |
| atom | 3925 | CB | ARG | 505 | 50.813 | -15.971 | 42.154 | 1.00 | 28.29 |
| atom | 3926 | CG | ARG | 505 | 50.326 | -15.869 | 43.598 | 1.00 | 30.58 |
| atom | 3927 | CD | ARG | 505 | 49.435 | -17.057 | 43.992 | 1.00 | 28.65 |
| atom | 3928 | NE | ARG | 505 | 48.048 | -16.661 | 44.262 | 1.00 | 29.11 |
| atom | 3929 | CZ | ARG | 505 | 47.034 | -17.515 | 44.419 | 1.00 | 30.61 |
| atom | 3930 | NH1 | ARG | 505 | 47.236 | -18.825 | 44.334 | 1.00 | 34.91 |
| atom | 3931 | NH2 | ARG | 505 | 45.810 | -17.065 | 44.661 | 1.00 | 32.55 |
| atom | 3932 | C | ARG | 505 | 52.475 | -14.410 | 41.148 | 1.00 | 26.98 |
| atom | 3933 | O | ARG | 505 | 53.089 | -13.495 | 41.697 | 1.00 | 29.63 |
| atom | 3934 | N | SER | 506 | 53.027 | -15.241 | 40.265 | 1.00 | 26.48 |
| atom | 3935 | CA | SER | 506 | 54.432 | -15.173 | 39.884 | 1.00 | 26.79 |
| atom | 3936 | CB | SER | 506 | 54.750 | -16.256 | 38.866 | 1.00 | 28.06 |
| atom | 3937 | OG | SER | 506 | 56.006 | -15.995 | 38.278 | 1.00 | 33.56 |
| atom | 3938 | C | SER | 506 | 54.889 | -13.831 | 39.336 | 1.00 | 28.42 |
| atom | 3939 | O | SER | 506 | 55.780 | -13.212 | 39.912 | 1.00 | 36.05 |
| atom | 3940 | N | VAL | 507 | 54.292 | -13.374 | 38.234 | 1.00 | 25.55 |
| atom | 3941 | CA | VAL | 507 | 54.675 | -12.090 | 37.639 | 1.00 | 20.31 |
| atom | 3942 | CB | VAL | 507 | 53.732 | -11.674 | 36.459 | 1.00 | 24.81 |
| atom | 3943 | CG1 | VAL | 507 | 54.470 | -10.744 | 35.515 | 1.00 | 19.95 |
| atom | 3944 | CG2 | VAL | 507 | 53.214 | -12.895 | 35.722 | 1.00 | 27.87 |
| atom | 3945 | C | VAL | 507 | 54.659 | -10.936 | 38.640 | 1.00 | 20.26 |
| atom | 3946 | O | VAL | 507 | 55.573 | -10.094 | 38.630 | 1.00 | 18.17 |
| atom | 3947 | N | ARG | 508 | 53.619 | -10.889 | 39.483 | 1.00 | 16.63 |
| atom | 3948 | CA | ARG | 508 | 53.476 | -9.826 | 40.486 | 1.00 | 20.36 |
| atom | 3949 | CB | ARG | 508 | 52.234 | -10.048 | 41.358 | 1.00 | 22.81 |
| atom | 3950 | CG | ARG | 508 | 52.235 | -9.227 | 42.625 | 1.00 | 21.60 |
| atom | 3951 | CD | ARG | 508 | 50.998 | -9.475 | 43.477 | 1.00 | 28.22 |
| atom | 3952 | NE | ARG | 508 | 51.296 | -9.361 | 44.911 | 1.00 | 37.21 |
| atom | 3953 | CZ | ARG | 508 | 50.847 | -8.379 | 45.689 | 1.00 | 38.38 |
| atom | 3954 | NH1 | ARG | 508 | 50.076 | -7.423 | 45.175 | 1.00 | 46.52 |
| atom | 3955 | NH2 | ARG | 508 | 51.169 | -8.339 | 46.972 | 1.00 | 28.71 |
| atom | 3956 | C | ARG | 508 | 54.691 | -9.723 | 41.383 | 1.00 | 22.41 |

| atom | 3957 | O | ARG | 508 | 55.154 | -8.626 | 41.671 | 1.00 | 22.27 |
|------|------|-----|-----|-----|--------|--------|--------|------|-------|
| atom | 3958 | N | ALA | 509 | 55.208 | -10.866 | 41.829 | 1.00 | 24.91 |
| atom | 3959 | CA | ALA | 509 | 56.389 | -10.855 | 42.687 | 1.00 | 27.02 |
| atom | 3960 | CB | ALA | 509 | 56.740 | -12.276 | 43.144 | 1.00 | 22.60 |
| atom | 3961 | C | ALA | 509 | 57.560 | -10.234 | 41.932 | 1.00 | 28.45 |
| atom | 3962 | O | ALA | 509 | 58.210 | -9.320 | 42.436 | 1.00 | 32.34 |
| atom | 3963 | N | ARG | 510 | 57.829 | -10.722 | 40.724 | 1.00 | 29.56 |
| atom | 3964 | CA | ARG | 510 | 58.928 | -10.180 | 39.924 | 1.00 | 30.26 |
| atom | 3965 | CB | ARG | 510 | 58.978 | -10.870 | 38.562 | 1.00 | 30.46 |
| atom | 3966 | CG | ARG | 510 | 58.777 | -12.369 | 38.607 | 1.00 | 25.33 |
| atom | 3967 | CD | ARG | 510 | 60.069 | -13.055 | 38.961 | 1.00 | 28.12 |
| atom | 3968 | NE | ARG | 510 | 59.879 | -14.419 | 39.451 | 1.00 | 35.39 |
| atom | 3969 | CZ | ARG | 510 | 60.226 | -14.836 | 40.667 | 1.00 | 34.87 |
| atom | 3970 | NH1 | ARG | 510 | 60.784 | -13.999 | 41.539 | 1.00 | 33.89 |
| atom | 3971 | NH2 | ARG | 510 | 60.048 | -16.105 | 41.001 | 1.00 | 36.28 |
| atom | 3972 | C | ARG | 510 | 58.746 | -8.670 | 39.729 | 1.00 | 32.22 |
| atom | 3973 | O | ARG | 510 | 59.712 | -7.909 | 39.697 | 1.00 | 34.67 |
| atom | 3974 | N | LEU | 511 | 57.497 | -8.234 | 39.602 | 1.00 | 33.14 |
| atom | 3975 | CA | LEU | 511 | 57.211 | -6.812 | 39.424 | 1.00 | 32.24 |
| atom | 3976 | CB | LEU | 511 | 55.755 | -6.617 | 38.985 | 1.00 | 31.16 |
| atom | 3977 | CG | LEU | 511 | 55.532 | -6.888 | 37.492 | 1.00 | 30.87 |
| atom | 3978 | CD1 | LEU | 511 | 54.132 | -7.401 | 37.266 | 1.00 | 19.45 |
| atom | 3979 | CD2 | LEU | 511 | 55.787 | -5.611 | 36.708 | 1.00 | 25.52 |
| atom | 3980 | C | LEU | 511 | 57.479 | -6.017 | 40.701 | 1.00 | 31.18 |
| atom | 3981 | O | LEU | 511 | 58.169 | -4.989 | 40.669 | 1.00 | 26.79 |
| atom | 3982 | N | LEU | 512 | 56.928 | -6.493 | 41.819 | 1.00 | 30.24 |
| atom | 3983 | CA | LEU | 512 | 57.110 | -5.819 | 43.108 | 1.00 | 32.29 |
| atom | 3984 | CB | LEU | 512 | 56.482 | -6.619 | 44.261 | 1.00 | 25.88 |
| atom | 3985 | CG | LEU | 512 | 54.984 | -6.940 | 44.299 | 1.00 | 26.51 |
| atom | 3986 | CD1 | LEU | 512 | 54.716 | -7.891 | 45.457 | 1.00 | 28.99 |
| atom | 3987 | CD2 | LEU | 512 | 54.165 | -5.677 | 44.444 | 1.00 | 24.37 |
| atom | 3988 | C | LEU | 512 | 58.594 | -5.676 | 43.402 | 1.00 | 33.82 |
| atom | 3989 | O | LEU | 512 | 59.094 | -4.580 | 43.661 | 1.00 | 33.78 |
| atom | 3990 | N | SER | 513 | 59.289 | -6.803 | 43.335 | 1.00 | 34.94 |
| atom | 3991 | CA | SER | 513 | 60.711 | -6.866 | 43.630 | 1.00 | 37.95 |
| atom | 3992 | CB | SER | 513 | 61.138 | -8.332 | 43.650 | 1.00 | 42.15 |

| atom | 3993 | OG | SER | 513 | 60.281 | -9.073 | 44.514 | 1.00 | 45.41 |
|------|------|-----|-----|-----|--------|--------|--------|------|-------|
| atom | 3994 | C | SER | 513 | 61.644 | -6.046 | 42.743 | 1.00 | 36.68 |
| atom | 3995 | O | SER | 513 | 62.864 | -6.162 | 42.849 | 1.00 | 35.08 |
| atom | 3996 | N | GLN | 514 | 61.067 | -5.206 | 41.891 | 1.00 | 37.16 |
| atom | 3997 | CA | GLN | 514 | 61.836 | -4.358 | 40.983 | 1.00 | 37.50 |
| atom | 3998 | CB | GLN | 514 | 61.248 | -4.429 | 39.569 | 1.00 | 43.16 |
| atom | 3999 | CG | GLN | 514 | 61.986 | -5.334 | 38.574 | 1.00 | 46.16 |
| atom | 4000 | CD | GLN | 514 | 61.951 | -4.784 | 37.144 | 1.00 | 48.42 |
| atom | 4001 | OE1 | GLN | 514 | 61.409 | -3.700 | 36.886 | 1.00 | 45.55 |
| atom | 4002 | NE2 | GLN | 514 | 62.535 | -5.531 | 36.211 | 1.00 | 43.64 |
| atom | 4003 | C | GLN | 514 | 61.753 | -2.922 | 41.483 | 1.00 | 37.67 |
| atom | 4004 | O | GLN | 514 | 62.463 | -2.039 | 41.007 | 1.00 | 35.72 |
| atom | 4005 | N | GLY | 515 | 60.865 | -2.697 | 42.446 | 1.00 | 38.87 |
| atom | 4006 | CA | GLY | 515 | 60.689 | -1.370 | 43.007 | 1.00 | 36.83 |
| atom | 4007 | C | GLY | 515 | 60.157 | -0.355 | 42.012 | 1.00 | 36.39 |
| atom | 4008 | O | GLY | 515 | 60.024 | -0.650 | 40.827 | 1.00 | 37.60 |
| atom | 4009 | N | GLY | 516 | 59.841 | 0.839 | 42.505 | 1.00 | 33.81 |
| atom | 4010 | CA | GLY | 516 | 59.334 | 1.897 | 41.655 | 1.00 | 33.91 |
| atom | 4011 | C | GLY | 516 | 58.143 | 1.533 | 40.794 | 1.00 | 38.28 |
| atom | 4012 | O | GLY | 516 | 57.298 | 0.722 | 41.181 | 1.00 | 38.92 |
| atom | 4013 | N | ARG | 517 | 58.091 | 2.144 | 39.614 | 1.00 | 38.05 |
| atom | 4014 | CA | ARG | 517 | 57.012 | 1.934 | 38.659 | 1.00 | 35.86 |
| atom | 4015 | CB | ARG | 517 | 57.396 | 2.537 | 37.305 | 1.00 | 40.10 |
| atom | 4016 | CG | ARG | 517 | 56.812 | 3.919 | 37.054 | 1.00 | 43.18 |
| atom | 4017 | CD | ARG | 517 | 56.079 | 3.950 | 35.735 | 1.00 | 47.23 |
| atom | 4018 | NE | ARG | 517 | 56.470 | 5.105 | 34.939 | 1.00 | 54.17 |
| atom | 4019 | CZ | ARG | 517 | 57.512 | 5.130 | 34.112 | 1.00 | 56.95 |
| atom | 4020 | NH1 | ARG | 517 | 58.279 | 4.056 | 33.965 | 1.00 | 58.23 |
| atom | 4021 | NH2 | ARG | 517 | 57.794 | 6.237 | 33.436 | 1.00 | 58.13 |
| atom | 4022 | C | ARG | 517 | 56.605 | 0.476 | 38.477 | 1.00 | 31.56 |
| atom | 4023 | O | ARG | 517 | 55.414 | 0.160 | 38.475 | 1.00 | 34.95 |
| atom | 4024 | N | ALA | 518 | 57.573 | -0.419 | 38.328 | 1.00 | 25.19 |
| atom | 4025 | CA | ALA | 518 | 57.228 | -1.825 | 38.150 | 1.00 | 21.50 |
| atom | 4026 | CB | ALA | 518 | 58.457 | -2.630 | 37.885 | 1.00 | 11.56 |
| atom | 4027 | C | ALA | 518 | 56.480 | -2.398 | 39.347 | 1.00 | 22.19 |
| atom | 4028 | O | ALA | 518 | 55.663 | -3.305 | 39.193 | 1.00 | 26.95 |

| atom | 4029 | N | ALA | 519 | 56.768 | -1.867 | 40.534 | 1.00 | 22.61 |
|------|------|------|------|-----|--------|--------|--------|------|-------|
| atom | 4030 | CA | ALA | 519 | 56.138 | -2.303 | 41.782 | 1.00 | 19.97 |
| atom | 4031 | CB | ALA | 519 | 56.867 | -1.669 | 42.975 | 1.00 | 17.81 |
| atom | 4032 | C | ALA | 519 | 54.656 | -1.938 | 41.828 | 1.00 | 20.24 |
| atom | 4033 | O | ALA | 519 | 53.810 | -2.760 | 42.207 | 1.00 | 19.71 |
| atom | 4034 | N | THR | 520 | 54.346 | -0.697 | 41.453 | 1.00 | 19.16 |
| atom | 4035 | CA | THR | 520 | 52.968 | -0.221 | 41.453 | 1.00 | 21.36 |
| atom | 4036 | CB | THR | 520 | 52.907 | 1.225 | 40.977 | 1.00 | 20.09 |
| atom | 4037 | OG1 | THR | 520 | 53.970 | 1.952 | 41.595 | 1.00 | 23.76 |
| atom | 4038 | CG2 | THR | 520 | 51.582 | 1.868 | 41.356 | 1.00 | 18.61 |
| atom | 4039 | C | THR | 520 | 52.059 | -1.092 | 40.574 | 1.00 | 24.98 |
| atom | 4040 | O | THR | 520 | 50.947 | -1.434 | 40.984 | 1.00 | 25.03 |
| atom | 4041 | N | CYS | 521 | 52.541 | -1.451 | 39.377 | 1.00 | 23.25 |
| atom | 4042 | CA | CYS | 521 | 51.785 | -2.288 | 38.431 | 1.00 | 21.68 |
| atom | 4043 | CB | CYS | 521 | 52.573 | -2.510 | 37.118 | 1.00 | 21.82 |
| atom | 4044 | SG | CYS | 521 | 52.914 | -1.044 | 36.097 | 1.00 | 17.89 |
| atom | 4045 | C | CYS | 521 | 51.504 | -3.643 | 39.054 | 1.00 | 20.51 |
| atom | 4046 | O | CYS | 521 | 50.398 | -4.168 | 38.959 | 1.00 | 19.81 |
| atom | 4047 | N | GLY | 522 | 52.529 | -4.206 | 39.683 | 1.00 | 21.73 |
| atom | 4048 | CA | GLY | 522 | 52.404 | -5.504 | 40.317 | 1.00 | 21.72 |
| atom | 4049 | C | GLY | 522 | 51.402 | -5.544 | 41.453 | 1.00 | 23.41 |
| atom | 4050 | O | GLY | 522 | 50.619 | -6.497 | 41.589 | 1.00 | 22.54 |
| atom | 4051 | N | LYS | 523 | 51.405 | -4.492 | 42.256 | 1.00 | 21.41 |
| atom | 4052 | CA | LYS | 523 | 50.527 | -4.425 | 43.406 | 1.00 | 21.80 |
| atom | 4053 | CB | LYS | 523 | 51.148 | -3.442 | 44.416 | 1.00 | 22.06 |
| atom | 4054 | CG | LYS | 523 | 50.178 | -2.735 | 45.320 | 1.00 | 22.90 |
| atom | 4055 | CD | LYS | 523 | 50.911 | -1.834 | 46.293 | 1.00 | 21.46 |
| atom | 4056 | CE | LYS | 523 | 49.933 | -0.961 | 47.083 | 1.00 | 13.21 |
| atom | 4057 | NZ | LYS | 523 | 50.606 | 0.324 | 47.409 | 1.00 | 23.93 |
| atom | 4058 | C | LYS | 523 | 49.087 | -4.048 | 43.023 | 1.00 | 22.07 |
| atom | 4059 | O | LYS | 523 | 48.112 | -4.609 | 43.533 | 1.00 | 21.31 |
| atom | 4060 | N | TYR | 524 | 48.951 | -3.117 | 42.093 | 1.00 | 23.53 |
| atom | 4061 | CA | TYR | 524 | 47.624 | -2.692 | 41.678 | 1.00 | 24.18 |
| atom | 4062 | CB | TYR | 524 | 47.707 | -1.248 | 41.170 | 1.00 | 26.96 |
| atom | 4063 | CG | TYR | 524 | 47.799 | -0.222 | 42.292 | 1.00 | 31.99 |
| atom | 4064 | CD1 | TYR | 524 | 49.028 | 0.147 | 42.835 | 1.00 | 31.39 |

| atom | 4065 | CE1 | TYR | 524 | 49.108 | 1.041 | 43.892 | 1.00 | 31.53 |
|------|------|-----|-----|-----|--------|-------|--------|------|-------|
| atom | 4066 | CD2 | TYR | 524 | 46.653 | 0.343 | 42.832 | 1.00 | 36.34 |
| atom | 4067 | CE2 | TYR | 524 | 46.720 | 1.238 | 43.891 | 1.00 | 35.33 |
| atom | 4068 | CZ | TYR | 524 | 47.943 | 1.585 | 44.417 | 1.00 | 34.86 |
| atom | 4069 | OH | TYR | 524 | 47.983 | 2.452 | 45.486 | 1.00 | 30.70 |
| atom | 4070 | C | TYR | 524 | 46.958 | -3.618 | 40.629 | 1.00 | 25.33 |
| atom | 4071 | O | TYR | 524 | 45.806 | -4.043 | 40.792 | 1.00 | 22.85 |
| atom | 4072 | N | LEU | 525 | 47.686 | -3.956 | 39.570 | 1.00 | 20.76 |
| atom | 4073 | CA | LEU | 525 | 47.109 | -4.787 | 38.539 | 1.00 | 19.46 |
| atom | 4074 | CB | LEU | 525 | 47.957 | -4.766 | 37.252 | 1.00 | 20.85 |
| atom | 4075 | CG | LEU | 525 | 48.736 | -3.549 | 36.746 | 1.00 | 18.37 |
| atom | 4076 | CD1 | LEU | 525 | 49.208 | -3.846 | 35.334 | 1.00 | 19.62 |
| atom | 4077 | CD2 | LEU | 525 | 47.869 | -2.316 | 36.731 | 1.00 | 23.39 |
| atom | 4078 | C | LEU | 525 | 46.930 | -6.227 | 38.957 | 1.00 | 19.55 |
| atom | 4079 | O | LEU | 525 | 46.037 | -6.908 | 38.443 | 1.00 | 14.61 |
| atom | 4080 | N | PHE | 526 | 47.759 | -6.710 | 39.879 | 1.00 | 21.79 |
| atom | 4081 | CA | PHE | 526 | 47.660 | -8.129 | 40.239 | 1.00 | 22.87 |
| atom | 4082 | CB | PHE | 526 | 48.976 | -8.848 | 39.882 | 1.00 | 23.31 |
| atom | 4083 | CG | PHE | 526 | 49.394 | -8.686 | 38.425 | 1.00 | 25.42 |
| atom | 4084 | CD1 | PHE | 526 | 50.444 | -7.836 | 38.070 | 1.00 | 25.95 |
| atom | 4085 | CD2 | PHE | 526 | 48.741 | -9.382 | 37.412 | 1.00 | 22.77 |
| atom | 4086 | CE1 | PHE | 526 | 50.830 | -7.690 | 36.723 | 1.00 | 25.73 |
| atom | 4087 | CE2 | PHE | 526 | 49.129 | -9.233 | 36.054 | 1.00 | 19.20 |
| atom | 4088 | CZ | PHE | 526 | 50.166 | -8.394 | 35.716 | 1.00 | 12.30 |
| atom | 4089 | C | PHE | 526 | 47.230 | -8.493 | 41.645 | 1.00 | 19.74 |
| atom | 4090 | O | PHE | 526 | 47.406 | -9.626 | 42.077 | 1.00 | 20.87 |
| atom | 4091 | N | ASN | 527 | 46.649 | -7.540 | 42.351 | 1.00 | 20.80 |
| atom | 4092 | CA | ASN | 527 | 46.168 | -7.800 | 43.697 | 1.00 | 22.67 |
| atom | 4093 | CB | ASN | 527 | 45.676 | -6.503 | 44.329 | 1.00 | 21.32 |
| atom | 4094 | CG | ASN | 527 | 45.875 | -6.480 | 45.820 | 1.00 | 27.69 |
| atom | 4095 | OD1 | ASN | 527 | 47.001 | -6.622 | 46.320 | 1.00 | 26.48 |
| atom | 4096 | ND2 | ASN | 527 | 44.786 | -6.298 | 46.550 | 1.00 | 22.52 |
| atom | 4097 | C | ASN | 527 | 45.025 | -8.824 | 43.661 | 1.00 | 26.89 |
| atom | 4098 | O | ASN | 527 | 44.758 | -9.504 | 44.654 | 1.00 | 29.14 |
| atom | 4099 | N | TRP | 528 | 44.360 | -8.933 | 42.509 | 1.00 | 26.74 |
| atom | 4100 | CA | TRP | 528 | 43.245 | -9.863 | 42.334 | 1.00 | 22.63 |

| | | | | | | | | | |
|---|---|---|---|---|---|---|---|---|---|
| atom | 4101 | CB | TRP | 528 | 42.511 | -9.588 | 40.999 | 1.00 | 23.42 |
| atom | 4102 | CG | TRP | 528 | 43.373 | -9.841 | 39.769 | 1.00 | 22.88 |
| atom | 4103 | CD2 | TRP | 528 | 43.737 | -11.119 | 39.226 | 1.00 | 16.34 |
| atom | 4104 | CE2 | TRP | 528 | 44.716 | -10.891 | 38.224 | 1.00 | 16.94 |
| atom | 4105 | CE3 | TRP | 528 | 43.341 | -12.430 | 39.493 | 1.00 | 16.31 |
| atom | 4106 | CD1 | TRP | 528 | 44.109 | -8.909 | 39.074 | 1.00 | 21.03 |
| atom | 4107 | NE1 | TRP | 528 | 44.920 | -9.533 | 38.151 | 1.00 | 20.51 |
| atom | 4108 | CZ2 | TRP | 528 | 45.307 | -11.930 | 37.491 | 1.00 | 12.66 |
| atom | 4109 | CZ3 | TRP | 528 | 43.935 | -13.478 | 38.757 | 1.00 | 22.18 |
| atom | 4110 | CH2 | TRP | 528 | 44.908 | -13.214 | 37.770 | 1.00 | 10.51 |
| atom | 4111 | C | TRP | 528 | 43.770 | -11.295 | 42.350 | 1.00 | 24.25 |
| atom | 4112 | O | TRP | 528 | 43.006 | -12.246 | 42.533 | 1.00 | 20.16 |
| atom | 4113 | N | ALA | 529 | 45.081 | -11.444 | 42.172 | 1.00 | 27.26 |
| atom | 4114 | CA | ALA | 529 | 45.700 | -12.772 | 42.137 | 1.00 | 28.52 |
| atom | 4115 | CB | ALA | 529 | 46.966 | -12.742 | 41.293 | 1.00 | 19.41 |
| atom | 4116 | C | ALA | 529 | 46.023 | -13.306 | 43.523 | 1.00 | 30.29 |
| atom | 4117 | O | ALA | 529 | 45.648 | -14.427 | 43.861 | 1.00 | 35.11 |
| atom | 4118 | N | VAL | 530 | 46.725 | -12.507 | 44.317 | 1.00 | 33.55 |
| atom | 4119 | CA | VAL | 530 | 47.103 | -12.910 | 45.670 | 1.00 | 33.91 |
| atom | 4120 | CB | VAL | 530 | 47.857 | -11.769 | 46.412 | 1.00 | 31.28 |
| atom | 4121 | CG1 | VAL | 530 | 48.789 | -11.047 | 45.464 | 1.00 | 26.39 |
| atom | 4122 | CG2 | VAL | 530 | 46.874 | -10.791 | 47.004 | 1.00 | 29.38 |
| atom | 4123 | C | VAL | 530 | 45.875 | -13.319 | 46.486 | 1.00 | 36.28 |
| atom | 4124 | O | VAL | 530 | 44.759 | -12.853 | 46.236 | 1.00 | 38.88 |
| atom | 4125 | N | LYS | 531 | 46.090 | -14.191 | 47.465 | 1.00 | 38.17 |
| atom | 4126 | CA | LYS | 531 | 45.017 | -14.687 | 48.327 | 1.00 | 38.07 |
| atom | 4127 | CB | LYS | 531 | 45.476 | -15.987 | 48.995 | 1.00 | 39.85 |
| atom | 4128 | CG | LYS | 531 | 46.671 | -16.647 | 48.300 | 1.00 | 38.60 |
| atom | 4129 | CD | LYS | 531 | 47.946 | -15.836 | 48.534 | 1.00 | 43.75 |
| atom | 4130 | CE | LYS | 531 | 49.106 | -16.300 | 47.666 | 1.00 | 42.42 |
| atom | 4131 | NZ | LYS | 531 | 49.773 | -17.500 | 48.247 | 1.00 | 43.42 |
| atom | 4132 | C | LYS | 531 | 44.593 | -13.644 | 49.378 | 1.00 | 36.13 |
| atom | 4133 | O | LYS | 531 | 43.404 | -13.304 | 49.494 | 1.00 | 36.59 |
| atom | 4134 | N | THR | 532 | 45.560 | -13.142 | 50.143 | 1.00 | 29.06 |
| atom | 4135 | CA | THR | 532 | 45.271 | -12.122 | 51.142 | 1.00 | 26.83 |
| atom | 4136 | CB | THR | 532 | 46.183 | -12.237 | 52.362 | 1.00 | 27.27 |

| atom | 4137 | OG1 | THR | 532 | 46.813 | -13.520 | 52.364 | 1.00 | 35.92 |
|------|------|-----|-----|-----|--------|---------|--------|------|-------|
| atom | 4138 | CG2 | THR | 532 | 45.371 | -12.051 | 53.638 | 1.00 | 26.98 |
| atom | 4139 | C   | THR | 532 | 45.512 | -10.779 | 50.479 | 1.00 | 23.15 |
| atom | 4140 | O   | THR | 532 | 46.638 | -10.298 | 50.392 | 1.00 | 17.89 |
| atom | 4141 | N   | LYS | 533 | 44.444 | -10.161 | 50.019 | 1.00 | 24.30 |
| atom | 4142 | CA  | LYS | 533 | 44.610 | -8.915  | 49.313 | 1.00 | 30.02 |
| atom | 4143 | CB  | LYS | 533 | 43.330 | -8.601  | 48.543 | 1.00 | 34.16 |
| atom | 4144 | CG  | LYS | 533 | 43.261 | -9.297  | 47.186 | 1.00 | 42.24 |
| atom | 4145 | CD  | LYS | 533 | 41.884 | -9.901  | 46.917 | 1.00 | 44.15 |
| atom | 4146 | CE  | LYS | 533 | 41.955 | -11.415 | 46.751 | 1.00 | 46.89 |
| atom | 4147 | NZ  | LYS | 533 | 41.927 | -11.827 | 45.328 | 1.00 | 43.79 |
| atom | 4148 | C   | LYS | 533 | 44.993 | -7.746  | 50.186 | 1.00 | 30.92 |
| atom | 4149 | O   | LYS | 533 | 44.629 | -7.697  | 51.363 | 1.00 | 34.83 |
| atom | 4150 | N   | LEU | 534 | 45.748 | -6.809  | 49.613 | 1.00 | 32.89 |
| atom | 4151 | CA  | LEU | 534 | 46.119 | -5.607  | 50.338 | 1.00 | 32.08 |
| atom | 4152 | CB  | LEU | 534 | 47.526 | -5.142  | 49.986 | 1.00 | 30.92 |
| atom | 4153 | CG  | LEU | 534 | 48.112 | -5.387  | 48.608 | 1.00 | 35.03 |
| atom | 4154 | CD1 | LEU | 534 | 47.865 | -4.160  | 47.762 | 1.00 | 33.89 |
| atom | 4155 | CD2 | LEU | 534 | 49.621 | -5.677  | 48.725 | 1.00 | 32.33 |
| atom | 4156 | C   | LEU | 534 | 45.101 | -4.549  | 49.960 | 1.00 | 34.40 |
| atom | 4157 | O   | LEU | 534 | 44.462 | -4.653  | 48.919 | 1.00 | 34.91 |
| atom | 4158 | N   | LYS | 535 | 44.925 | -3.551  | 50.824 | 1.00 | 37.40 |
| atom | 4159 | CA  | LYS | 535 | 43.961 | -2.480  | 50.580 | 1.00 | 34.10 |
| atom | 4160 | CB  | LYS | 535 | 43.499 | -1.866  | 51.911 | 1.00 | 36.92 |
| atom | 4161 | CG  | LYS | 535 | 41.986 | -1.841  | 52.114 | 1.00 | 38.59 |
| atom | 4162 | CD  | LYS | 535 | 41.402 | -0.439  | 51.877 | 1.00 | 44.81 |
| atom | 4163 | CE  | LYS | 535 | 41.745 | 0.537   | 53.041 | 1.00 | 48.73 |
| atom | 4164 | NZ  | LYS | 535 | 41.791 | 2.007   | 52.687 | 1.00 | 35.50 |
| atom | 4165 | C   | LYS | 535 | 44.569 | -1.399  | 49.695 | 1.00 | 33.67 |
| atom | 4166 | O   | LYS | 535 | 45.465 | -0.651  | 50.111 | 1.00 | 33.50 |
| atom | 4167 | N   | LEU | 536 | 44.062 | -1.302  | 48.475 | 1.00 | 31.05 |
| atom | 4168 | CA  | LEU | 536 | 44.574 | -0.317  | 47.536 | 1.00 | 28.88 |
| atom | 4169 | CB  | LEU | 536 | 44.148 | -0.686  | 46.120 | 1.00 | 20.05 |
| atom | 4170 | CG  | LEU | 536 | 44.814 | -2.001  | 45.746 | 1.00 | 17.34 |
| atom | 4171 | CD1 | LEU | 536 | 44.447 | -2.418  | 44.336 | 1.00 | 14.63 |
| atom | 4172 | CD2 | LEU | 536 | 46.316 | -1.828  | 45.895 | 1.00 | 16.21 |

| atom | 4173 | C   | LEU | 536 | 44.157 | 1.104  | 47.866 | 1.00 | 29.61 |
|------|------|-----|-----|-----|--------|--------|--------|------|-------|
| atom | 4174 | O   | LEU | 536 | 42.984 | 1.383  | 48.100 | 1.00 | 29.85 |
| atom | 4175 | N   | THR | 537 | 45.148 | 1.987  | 47.916 | 1.00 | 28.82 |
| atom | 4176 | CA  | THR | 537 | 44.927 | 3.396  | 48.186 | 1.00 | 30.68 |
| atom | 4177 | CB  | THR | 537 | 46.005 | 3.978  | 49.116 | 1.00 | 34.05 |
| atom | 4178 | OG1 | THR | 537 | 47.102 | 4.463  | 48.325 | 1.00 | 37.16 |
| atom | 4179 | CG2 | THR | 537 | 46.505 | 2.923  | 50.095 | 1.00 | 37.83 |
| atom | 4180 | C   | THR | 537 | 45.055 | 4.086  | 46.833 | 1.00 | 31.45 |
| atom | 4181 | O   | THR | 537 | 45.804 | 3.629  | 45.972 | 1.00 | 30.15 |
| atom | 4182 | N   | PRO | 538 | 44.314 | 5.176  | 46.617 | 1.00 | 29.13 |
| atom | 4183 | CD  | PRO | 538 | 43.325 | 5.821  | 47.497 | 1.00 | 30.93 |
| atom | 4184 | CA  | PRO | 538 | 44.434 | 5.844  | 45.321 | 1.00 | 29.71 |
| atom | 4185 | CB  | PRO | 538 | 43.652 | 7.135  | 45.511 | 1.00 | 31.77 |
| atom | 4186 | CG  | PRO | 538 | 42.634 | 6.788  | 46.578 | 1.00 | 32.13 |
| atom | 4187 | C   | PRO | 538 | 45.900 | 6.075  | 44.973 | 1.00 | 30.50 |
| atom | 4188 | O   | PRO | 538 | 46.692 | 6.472  | 45.831 | 1.00 | 29.48 |
| atom | 4189 | N   | ILE | 539 | 46.257 | 5.796  | 43.719 | 1.00 | 28.66 |
| atom | 4190 | CA  | ILE | 539 | 47.626 | 5.955  | 43.261 | 1.00 | 30.89 |
| atom | 4191 | CB  | ILE | 539 | 47.843 | 5.328  | 41.855 | 1.00 | 32.12 |
| atom | 4192 | CG2 | ILE | 539 | 49.236 | 5.640  | 41.357 | 1.00 | 23.73 |
| atom | 4193 | CG1 | ILE | 539 | 47.669 | 3.808  | 41.922 | 1.00 | 33.30 |
| atom | 4194 | CD1 | ILE | 539 | 47.241 | 3.163  | 40.605 | 1.00 | 32.29 |
| atom | 4195 | C   | ILE | 539 | 47.978 | 7.425  | 43.212 | 1.00 | 35.32 |
| atom | 4196 | O   | ILE | 539 | 47.301 | 8.217  | 42.561 | 1.00 | 36.86 |
| atom | 4197 | N   | PRO | 540 | 49.040 | 7.810  | 43.923 | 1.00 | 42.57 |
| atom | 4198 | CD  | PRO | 540 | 49.869 | 6.899  | 44.735 | 1.00 | 45.45 |
| atom | 4199 | CA  | PRO | 540 | 49.500 | 9.203  | 43.973 | 1.00 | 47.52 |
| atom | 4200 | CB  | PRO | 540 | 50.494 | 9.224  | 45.134 | 1.00 | 47.95 |
| atom | 4201 | CG  | PRO | 540 | 50.913 | 7.798  | 45.335 | 1.00 | 46.55 |
| atom | 4202 | C   | PRO | 540 | 50.135 | 9.671  | 42.667 | 1.00 | 51.52 |
| atom | 4203 | O   | PRO | 540 | 49.905 | 10.793 | 42.228 | 1.00 | 52.60 |
| atom | 4204 | N   | ALA | 541 | 50.937 | 8.810  | 42.053 | 1.00 | 53.79 |
| atom | 4205 | CA  | ALA | 541 | 51.592 | 9.151  | 40.797 | 1.00 | 58.44 |
| atom | 4206 | CB  | ALA | 541 | 52.733 | 8.168  | 40.535 | 1.00 | 61.73 |
| atom | 4207 | C   | ALA | 541 | 50.600 | 9.147  | 39.621 | 1.00 | 60.41 |
| atom | 4208 | O   | ALA | 541 | 50.855 | 8.529  | 38.582 | 1.00 | 58.45 |

| atom | 4209 | N   | ALA | 542 | 49.473 | 9.839  | 39.793 | 1.00 62.52 |
| atom | 4210 | CA  | ALA | 542 | 48.433 | 9.920  | 38.766 | 1.00 62.72 |
| atom | 4211 | CB  | ALA | 542 | 47.760 | 8.558  | 38.601 | 1.00 64.28 |
| atom | 4212 | C   | ALA | 542 | 47.384 | 10.967 | 39.142 | 1.00 64.21 |
| atom | 4213 | O   | ALA | 542 | 46.574 | 10.739 | 40.046 | 1.00 64.50 |
| atom | 4214 | N   | SER | 543 | 47.391 | 12.105 | 38.450 | 1.00 64.02 |
| atom | 4215 | CA  | SER | 543 | 46.428 | 13.173 | 38.725 | 1.00 61.74 |
| atom | 4216 | CB  | SER | 543 | 46.552 | 13.643 | 40.180 | 1.00 60.37 |
| atom | 4217 | OG  | SER | 543 | 45.414 | 13.287 | 40.952 | 1.00 56.95 |
| atom | 4218 | C   | SER | 543 | 46.654 | 14.352 | 37.788 | 1.00 61.94 |
| atom | 4219 | O   | SER | 543 | 47.593 | 14.261 | 36.965 | 1.00 63.12 |
| atom | 4220 | CB  | LEU | 547 | 43.716 | 10.598 | 30.109 | 1.00 46.53 |
| atom | 4221 | CG  | LEU | 547 | 44.915 | 9.938  | 30.813 | 1.00 41.86 |
| atom | 4222 | CD1 | LEU | 547 | 44.610 | 8.464  | 31.097 | 1.00 39.82 |
| atom | 4223 | CD2 | LEU | 547 | 46.162 | 10.064 | 29.939 | 1.00 40.47 |
| atom | 4224 | C   | LEU | 547 | 42.571 | 12.609 | 29.095 | 1.00 50.18 |
| atom | 4225 | O   | LEU | 547 | 41.655 | 11.854 | 28.772 | 1.00 53.79 |
| atom | 4226 | N   | LEU | 547 | 44.121 | 12.899 | 30.947 | 1.00 47.33 |
| atom | 4227 | CA  | LEU | 547 | 43.855 | 12.084 | 29.729 | 1.00 50.02 |
| atom | 4228 | N   | SER | 548 | 42.516 | 13.923 | 28.930 | 1.00 51.66 |
| atom | 4229 | CA  | SER | 548 | 41.354 | 14.582 | 28.350 | 1.00 52.11 |
| atom | 4230 | CB  | SER | 548 | 41.366 | 16.070 | 28.731 | 1.00 53.79 |
| atom | 4231 | OG  | SER | 548 | 42.546 | 16.705 | 28.255 | 1.00 56.74 |
| atom | 4232 | C   | SER | 548 | 41.320 | 14.440 | 26.823 | 1.00 50.14 |
| atom | 4233 | O   | SER | 548 | 40.273 | 14.124 | 26.250 | 1.00 47.18 |
| atom | 4234 | N   | GLY | 549 | 42.472 | 14.666 | 26.185 | 1.00 47.75 |
| atom | 4235 | CA  | GLY | 549 | 42.576 | 14.589 | 24.733 | 1.00 46.46 |
| atom | 4236 | C   | GLY | 549 | 42.238 | 13.259 | 24.083 | 1.00 43.60 |
| atom | 4237 | O   | GLY | 549 | 41.764 | 13.209 | 22.947 | 1.00 45.49 |
| atom | 4238 | N   | TRP | 550 | 42.472 | 12.183 | 24.819 | 1.00 40.58 |
| atom | 4239 | CA  | TRP | 550 | 42.230 | 10.830 | 24.349 | 1.00 37.12 |
| atom | 4240 | CB  | TRP | 550 | 42.491 | 9.850  | 25.479 | 1.00 33.40 |
| atom | 4241 | CG  | TRP | 550 | 43.931 | 9.703  | 25.814 | 1.00 39.27 |
| atom | 4242 | CD2 | TRP | 550 | 44.542 | 8.594  | 26.472 | 1.00 38.11 |
| atom | 4243 | CE2 | TRP | 550 | 45.913 | 8.884  | 26.586 | 1.00 39.99 |
| atom | 4244 | CE3 | TRP | 550 | 44.060 | 7.385  | 26.985 | 1.00 40.66 |

| atom | 4245 | CD1 | TRP | 550 | 44.928 | 10.594 | 25.557 | 1.00 | 38.74 |
|------|------|-----|-----|-----|--------|--------|--------|------|-------|
| atom | 4246 | NE1 | TRP | 550 | 46.122 | 10.111 | 26.013 | 1.00 | 39.45 |
| atom | 4247 | CZ2 | TRP | 550 | 46.814 | 8.004 | 27.177 | 1.00 | 39.82 |
| atom | 4248 | CZ3 | TRP | 550 | 44.950 | 6.513 | 27.571 | 1.00 | 41.68 |
| atom | 4249 | CH2 | TRP | 550 | 46.315 | 6.829 | 27.669 | 1.00 | 40.92 |
| atom | 4250 | C | TRP | 550 | 40.821 | 10.610 | 23.850 | 1.00 | 37.72 |
| atom | 4251 | O | TRP | 550 | 40.592 | 9.901 | 22.854 | 1.00 | 36.59 |
| atom | 4252 | N | PHE | 551 | 39.877 | 11.218 | 24.555 | 1.00 | 36.18 |
| atom | 4253 | CA | PHE | 551 | 38.480 | 11.055 | 24.225 | 1.00 | 33.96 |
| atom | 4254 | CB | PHE | 551 | 37.799 | 10.321 | 25.371 | 1.00 | 30.39 |
| atom | 4255 | CG | PHE | 551 | 38.513 | 9.055 | 25.777 | 1.00 | 25.23 |
| atom | 4256 | CD1 | PHE | 551 | 39.427 | 9.054 | 26.826 | 1.00 | 27.56 |
| atom | 4257 | CD2 | PHE | 551 | 38.266 | 7.857 | 25.115 | 1.00 | 27.89 |
| atom | 4258 | CE1 | PHE | 551 | 40.091 | 7.860 | 27.216 | 1.00 | 24.35 |
| atom | 4259 | CE2 | PHE | 551 | 38.920 | 6.656 | 25.492 | 1.00 | 19.97 |
| atom | 4260 | CZ | PHE | 551 | 39.828 | 6.664 | 26.541 | 1.00 | 22.33 |
| atom | 4261 | C | PHE | 551 | 37.791 | 12.370 | 23.892 | 1.00 | 35.69 |
| atom | 4262 | O | PHE | 551 | 36.898 | 12.843 | 24.607 | 1.00 | 35.41 |
| atom | 4263 | N | VAL | 552 | 38.232 | 12.935 | 22.770 | 1.00 | 32.09 |
| atom | 4264 | CA | VAL | 552 | 37.725 | 14.186 | 22.237 | 1.00 | 29.85 |
| atom | 4265 | CB | VAL | 552 | 38.901 | 15.090 | 21.838 | 1.00 | 31.14 |
| atom | 4266 | CG1 | VAL | 552 | 38.469 | 16.069 | 20.772 | 1.00 | 29.68 |
| atom | 4267 | CG2 | VAL | 552 | 39.442 | 15.809 | 23.065 | 1.00 | 28.42 |
| atom | 4268 | C | VAL | 552 | 36.854 | 13.902 | 20.998 | 1.00 | 27.42 |
| atom | 4269 | O | VAL | 552 | 35.723 | 14.361 | 20.902 | 1.00 | 27.21 |
| atom | 4270 | N | ALA | 553 | 37.394 | 13.130 | 20.065 | 1.00 | 24.42 |
| atom | 4271 | CA | ALA | 553 | 36.697 | 12.783 | 18.829 | 1.00 | 23.14 |
| atom | 4272 | CB | ALA | 553 | 36.902 | 13.877 | 17.785 | 1.00 | 18.35 |
| atom | 4273 | C | ALA | 553 | 37.228 | 11.468 | 18.280 | 1.00 | 20.69 |
| atom | 4274 | O | ALA | 553 | 38.277 | 10.987 | 18.709 | 1.00 | 18.72 |
| atom | 4275 | N | GLY | 554 | 36.497 | 10.897 | 17.328 | 1.00 | 18.56 |
| atom | 4276 | CA | GLY | 554 | 36.918 | 9.657 | 16.701 | 1.00 | 15.95 |
| atom | 4277 | C | GLY | 554 | 37.778 | 9.988 | 15.492 | 1.00 | 19.28 |
| atom | 4278 | O | GLY | 554 | 37.555 | 10.992 | 14.817 | 1.00 | 17.64 |
| atom | 4279 | N | TYR | 555 | 38.756 | 9.138 | 15.201 | 1.00 | 22.01 |
| atom | 4280 | CA | TYR | 555 | 39.639 | 9.399 | 14.079 | 1.00 | 24.13 |

| atom | 4281 | CB | TYR | 555 | 40.937 | 10.005 | 14.595 | 1.00 | 20.22 |
| atom | 4282 | CG | TYR | 555 | 40.748 | 11.356 | 15.221 | 1.00 | 19.94 |
| atom | 4283 | CD1 | TYR | 555 | 40.759 | 11.516 | 16.607 | 1.00 | 18.14 |
| atom | 4284 | CE1 | TYR | 555 | 40.695 | 12.784 | 17.180 | 1.00 | 20.60 |
| atom | 4285 | CD2 | TYR | 555 | 40.651 | 12.490 | 14.427 | 1.00 | 18.09 |
| atom | 4286 | CE2 | TYR | 555 | 40.587 | 13.747 | 14.984 | 1.00 | 18.43 |
| atom | 4287 | CZ | TYR | 555 | 40.622 | 13.888 | 16.351 | 1.00 | 19.06 |
| atom | 4288 | OH | TYR | 555 | 40.673 | 15.148 | 16.863 | 1.00 | 24.90 |
| atom | 4289 | C | TYR | 555 | 39.985 | 8.216 | 13.197 | 1.00 | 26.88 |
| atom | 4290 | O | TYR | 555 | 40.916 | 8.304 | 12.400 | 1.00 | 30.02 |
| atom | 4291 | N | SER | 556 | 39.272 | 7.106 | 13.315 | 1.00 | 30.32 |
| atom | 4292 | CA | SER | 556 | 39.629 | 5.978 | 12.464 | 1.00 | 37.27 |
| atom | 4293 | CB | SER | 556 | 38.942 | 4.690 | 12.933 | 1.00 | 37.51 |
| atom | 4294 | OG | SER | 556 | 37.535 | 4.827 | 12.988 | 1.00 | 51.44 |
| atom | 4295 | C | SER | 556 | 39.263 | 6.293 | 11.020 | 1.00 | 37.29 |
| atom | 4296 | O | SER | 556 | 38.051 | 6.322 | 10.719 | 1.00 | 38.86 |
| atom | 4297 | OT | SER | 556 | 40.198 | 6.526 | 10.221 | 1.00 | 34.94 |
| atom | 4298 | O | HOH | 601 | 46.597 | 10.920 | 10.721 | 1.00 | 20.61 |
| atom | 4299 | O | HOH | 602 | 22.175 | 2.399 | -11.303 | 1.00 | 39.23 |
| atom | 4300 | O | HOH | 603 | 22.555 | -6.018 | 20.759 | 1.00 | 25.24 |
| atom | 4301 | O | HOH | 604 | 10.699 | 23.284 | 3.535 | 1.00 | 11.38 |
| atom | 4302 | O | HOH | 605 | 33.779 | -15.862 | -12.840 | 1.00 | 32.40 |
| atom | 4303 | O | HOH | 606 | 23.250 | -5.255 | 39.290 | 1.00 | 19.05 |
| atom | 4304 | O | HOH | 607 | 56.607 | 18.994 | 13.165 | 1.00 | 29.80 |
| atom | 4305 | O | HOH | 608 | 32.158 | -6.088 | 14.607 | 1.00 | 26.14 |
| atom | 4306 | O | HOH | 609 | 56.341 | -19.171 | 32.102 | 1.00 | 30.79 |
| atom | 4307 | O | HOH | 610 | 42.183 | -16.676 | 46.222 | 1.00 | 43.54 |
| atom | 4308 | O | HOH | 611 | 45.029 | 9.354 | 9.054 | 1.00 | 41.58 |
| atom | 4309 | O | HOH | 612 | 47.451 | 26.726 | 6.177 | 1.00 | 47.73 |
| atom | 4310 | O | HOH | 613 | 2.470 | -1.802 | 27.736 | 1.00 | 38.21 |
| atom | 4311 | O | HOH | 614 | 47.043 | 21.787 | 16.034 | 1.00 | 23.91 |
| atom | 4312 | O | HOH | 615 | 34.075 | 17.253 | 18.692 | 1.00 | 22.58 |
| atom | 4313 | O | HOH | 616 | 50.817 | 23.460 | -12.759 | 1.00 | 40.31 |
| atom | 4314 | O | HOH | 617 | 38.747 | 2.676 | 9.078 | 1.00 | 25.13 |
| atom | 4315 | O | HOH | 618 | 35.002 | 0.690 | 23.109 | 1.00 | 15.25 |
| atom | 4316 | O | HOH | 619 | 52.799 | 16.218 | -9.314 | 1.00 | 39.47 |

| atom | 4317 | O | HOH | 620 | 57.492 | 32.252 | 19.690 | 1.00 | 51.60 |
|------|------|---|-----|-----|--------|--------|--------|------|-------|
| atom | 4318 | O | HOH | 621 | 24.538 | -2.398 | -12.767 | 1.00 | 35.98 |
| atom | 4319 | O | HOH | 622 | 45.774 | -26.860 | 17.172 | 1.00 | 47.38 |
| atom | 4320 | O | HOH | 623 | 16.918 | -0.379 | -0.855 | 1.00 | 50.73 |
| atom | 4321 | O | HOH | 624 | 35.816 | -18.589 | 0.233 | 1.00 | 41.40 |
| atom | 4322 | O | HOH | 625 | 48.368 | 29.897 | 14.750 | 1.00 | 21.51 |
| atom | 4323 | O | HOH | 626 | 36.518 | 3.819 | 41.949 | 1.00 | 11.95 |
| atom | 4324 | O | HOH | 627 | 43.658 | 6.306 | -16.389 | 1.00 | 42.56 |
| atom | 4325 | O | HOH | 628 | 48.323 | 10.990 | 7.196 | 1.00 | 23.08 |
| atom | 4326 | O | HOH | 629 | 38.139 | -19.255 | 43.774 | 1.00 | 44.50 |
| atom | 4327 | O | HOH | 630 | 34.071 | 6.076 | 12.772 | 1.00 | 24.32 |
| atom | 4328 | O | HOH | 631 | 58.322 | 10.881 | 14.337 | 1.00 | 39.25 |
| atom | 4329 | O | HOH | 632 | 26.691 | 26.778 | 4.448 | 1.00 | 28.44 |
| atom | 4330 | O | HOH | 633 | 53.979 | 24.631 | 19.675 | 1.00 | 54.93 |
| atom | 4331 | O | HOH | 634 | 2.628 | 2.180 | 11.312 | 1.00 | 36.74 |
| atom | 4332 | O | HOH | 635 | 5.149 | 14.418 | 28.631 | 1.00 | 35.56 |
| atom | 4333 | O | HOH | 636 | 32.587 | -3.817 | 12.697 | 1.00 | 63.31 |
| atom | 4334 | O | HOH | 637 | 27.170 | -9.280 | 31.802 | 1.00 | 24.21 |
| atom | 4335 | O | HOH | 638 | 43.450 | 5.359 | 10.944 | 1.00 | 36.10 |
| atom | 4336 | O | HOH | 639 | 26.062 | -12.225 | 14.751 | 1.00 | 49.87 |
| atom | 4337 | O | HOH | 640 | 14.706 | 7.497 | -5.096 | 1.00 | 18.84 |
| atom | 4338 | O | HOH | 641 | 57.477 | -14.762 | 35.981 | 1.00 | 28.21 |
| atom | 4339 | O | HOH | 642 | 51.547 | 9.783 | 48.172 | 1.00 | 53.68 |
| atom | 4340 | O | HOH | 643 | 30.933 | -6.757 | -7.733 | 1.00 | 35.43 |
| atom | 4341 | O | HOH | 644 | 25.070 | 13.151 | 5.827 | 1.00 | 2.00 |
| atom | 4342 | O | HOH | 645 | 56.672 | -10.309 | 18.187 | 1.00 | 45.96 |
| atom | 4343 | O | HOH | 646 | 11.579 | 20.415 | 6.160 | 1.00 | 28.95 |
| atom | 4344 | O | HOH | 647 | 27.916 | -15.124 | 2.287 | 1.00 | 18.06 |
| atom | 4345 | O | HOH | 648 | 35.221 | 2.326 | 34.458 | 1.00 | 25.12 |
| atom | 4346 | O | HOH | 649 | 38.312 | 9.262 | 36.937 | 1.00 | 67.07 |
| atom | 4347 | O | HOH | 650 | 46.258 | 6.938 | 4.683 | 1.00 | 24.22 |
| atom | 4348 | O | HOH | 651 | 42.606 | -3.262 | 36.649 | 1.00 | 21.46 |
| atom | 4349 | O | HOH | 652 | 40.235 | 11.518 | 20.853 | 1.00 | 27.91 |
| atom | 4350 | O | HOH | 653 | 22.794 | 25.287 | 5.447 | 1.00 | 26.04 |
| atom | 4351 | O | HOH | 654 | 38.206 | 9.504 | -13.291 | 1.00 | 30.62 |
| atom | 4352 | O | HOH | 655 | 45.226 | 13.150 | 18.803 | 1.00 | 18.63 |

| atom | 4353 | 0 | HOH | 656 | 33.031 | -18.621 | 22.852 | 1.00 | 44.32 |
|------|------|---|-----|-----|--------|---------|--------|------|-------|
| atom | 4354 | 0 | HOH | 657 | 9.650 | -6.271 | 22.144 | 1.00 | 32.02 |
| atom | 4355 | 0 | HOH | 658 | 30.463 | 24.471 | -1.145 | 1.00 | 18.42 |
| atom | 4356 | 0 | HOH | 659 | 41.452 | 8.031 | 8.619 | 1.00 | 20.10 |
| atom | 4357 | 0 | HOH | 660 | 24.327 | 5.781 | 5.531 | 1.00 | 13.22 |
| atom | 4358 | 0 | HOH | 661 | 47.983 | 6.984 | -10.411 | 1.00 | 60.77 |
| atom | 4359 | 0 | HOH | 662 | 30.966 | -22.909 | 5.590 | 1.00 | 36.24 |
| atom | 4360 | 0 | HOH | 663 | 33.405 | -1.565 | 17.123 | 1.00 | 38.51 |
| atom | 4361 | 0 | HOH | 664 | 21.076 | 7.717 | -8.594 | 1.00 | 32.16 |
| atom | 4362 | 0 | HOH | 665 | 16.982 | 24.733 | 15.974 | 1.00 | 34.58 |
| atom | 4363 | 0 | HOH | 666 | 26.953 | 23.531 | 1.354 | 1.00 | 31.58 |
| atom | 4364 | 0 | HOH | 667 | 40.572 | -13.743 | 44.640 | 1.00 | 37.35 |
| atom | 4365 | 0 | HOH | 668 | 31.441 | 0.335 | 7.724 | 1.00 | 41.72 |
| atom | 4366 | 0 | HOH | 669 | 47.678 | -7.636 | 13.712 | 1.00 | 31.80 |
| atom | 4367 | 0 | HOH | 670 | 46.557 | 22.825 | -11.411 | 1.00 | 67.81 |
| atom | 4368 | 0 | HOH | 671 | 21.234 | 10.081 | -2.885 | 1.00 | 10.38 |
| atom | 4369 | 0 | HOH | 672 | 31.211 | -1.023 | 4.408 | 1.00 | 24.09 |
| atom | 4370 | 0 | HOH | 673 | 50.141 | -6.813 | -11.682 | 1.00 | 21.54 |
| atom | 4371 | 0 | HOH | 674 | 33.244 | 15.024 | -9.889 | 1.00 | 67.86 |
| atom | 4372 | 0 | HOH | 675 | 38.979 | 11.058 | 31.410 | 1.00 | 26.12 |
| atom | 4373 | 0 | HOH | 676 | 34.118 | 11.305 | 3.467 | 1.00 | 28.25 |
| atom | 4374 | 0 | HOH | 677 | 46.763 | -27.510 | 31.664 | 1.00 | 52.83 |
| atom | 4375 | 0 | HOH | 678 | 35.099 | 2.565 | 8.047 | 1.00 | 28.30 |
| atom | 4376 | 0 | HOH | 679 | 9.176 | 25.088 | 4.798 | 1.00 | 18.72 |
| atom | 4377 | 0 | HOH | 680 | 14.704 | 13.412 | 25.182 | 1.00 | 48.26 |
| atom | 4378 | 0 | HOH | 681 | 6.438 | 4.023 | 3.328 | 1.00 | 61.72 |
| atom | 4379 | 0 | HOH | 682 | 32.219 | 15.747 | 2.622 | 1.00 | 38.25 |
| atom | 4380 | 0 | HOH | 683 | 31.014 | -13.129 | 15.368 | 1.00 | 36.02 |
| atom | 4381 | 0 | HOH | 684 | 41.423 | 22.998 | -13.412 | 1.00 | 46.45 |
| atom | 4382 | 0 | HOH | 685 | 41.073 | 14.541 | 32.544 | 1.00 | 57.67 |
| atom | 4383 | 0 | HOH | 686 | 41.923 | 30.239 | 10.109 | 1.00 | 24.51 |
| atom | 4384 | 0 | HOH | 687 | 13.043 | 1.835 | 1.524 | 1.00 | 45.08 |
| atom | 4385 | 0 | HOH | 688 | 36.384 | 1.728 | 2.325 | 1.00 | 23.11 |
| atom | 4386 | 0 | HOH | 689 | 38.926 | -12.759 | 41.527 | 1.00 | 26.09 |
| atom | 4387 | 0 | HOH | 690 | 13.533 | 4.342 | -1.256 | 1.00 | 53.49 |
| atom | 4388 | 0 | HOH | 691 | 42.859 | -30.767 | 29.292 | 1.00 | 49.63 |

| | | | | | | | | | |
|---|---|---|---|---|---|---|---|---|---|
| atom | 4389 | O | HOH | 692 | 46.981 | -5.614 | -11.265 | 1.00 | 28.30 |
| atom | 4390 | O | HOH | 693 | 34.904 | -2.672 | 2.841 | 1.00 | 25.95 |
| atom | 4391 | O | HOH | 694 | 22.975 | 23.743 | 2.224 | 1.00 | 32.47 |
| atom | 4392 | O | HOH | 695 | 45.861 | 14.150 | 31.270 | 1.00 | 37.81 |
| atom | 4393 | O | HOH | 696 | 46.016 | 15.972 | 22.828 | 1.00 | 88.18 |
| atom | 4394 | O | HOH | 697 | 13.753 | -0.702 | 38.177 | 1.00 | 52.89 |
| atom | 4395 | O | HOH | 698 | 34.502 | 16.040 | 22.414 | 1.00 | 27.95 |
| atom | 4396 | O | HOH | 699 | 22.706 | 27.973 | 10.089 | 1.00 | 35.75 |
| atom | 4397 | O | HOH | 700 | 63.426 | -24.001 | 17.375 | 1.00 | 42.25 |
| atom | 4398 | O | HOH | 701 | 34.349 | 9.690 | 1.548 | 1.00 | 9.34 |
| atom | 4399 | O | HOH | 702 | 41.163 | 2.343 | 49.912 | 1.00 | 27.48 |
| atom | 4400 | O | HOH | 703 | 9.851 | 15.333 | 21.500 | 1.00 | 58.68 |
| atom | 4401 | O | HOH | 704 | 44.019 | 27.355 | 14.119 | 1.00 | 23.41 |
| atom | 4402 | O | HOH | 705 | 47.294 | -8.487 | 10.420 | 1.00 | 28.83 |
| atom | 4403 | O | HOH | 706 | -0.567 | -15.964 | 21.058 | 1.00 | 71.82 |
| atom | 4404 | O | HOH | 707 | 45.139 | 17.223 | 28.013 | 1.00 | 36.95 |
| atom | 4405 | O | HOH | 708 | 54.938 | 29.596 | 18.826 | 1.00 | 45.27 |
| atom | 4406 | O | HOH | 709 | 56.658 | -1.774 | 11.129 | 1.00 | 59.82 |
| atom | 4407 | O | HOH | 710 | 41.378 | -2.253 | 48.336 | 1.00 | 83.26 |
| atom | 4408 | O | HOH | 711 | 34.016 | 0.844 | 32.187 | 1.00 | 19.51 |
| atom | 4409 | O | HOH | 712 | 40.079 | 23.922 | 18.790 | 1.00 | 40.86 |
| atom | 4410 | O | HOH | 713 | 23.939 | 16.548 | -6.662 | 1.00 | 73.98 |
| atom | 4411 | O | HOH | 714 | 43.805 | -5.760 | 37.997 | 1.00 | 33.15 |
| atom | 4412 | O | HOH | 715 | 43.983 | 12.763 | 7.024 | 1.00 | 49.45 |
| atom | 4413 | O | HOH | 716 | 17.467 | -6.790 | 34.490 | 1.00 | 60.85 |
| atom | 4414 | O | HOH | 717 | 57.128 | -9.673 | 7.961 | 1.00 | 47.78 |
| atom | 4415 | O | HOH | 718 | 28.310 | -13.504 | 15.674 | 1.00 | 32.19 |
| atom | 4416 | O | HOH | 719 | 48.350 | 12.483 | 24.005 | 1.00 | 59.90 |
| atom | 4417 | O | HOH | 720 | 19.085 | 8.242 | -9.951 | 1.00 | 25.97 |
| atom | 4418 | O | HOH | 721 | 55.772 | 11.621 | 19.184 | 1.00 | 35.36 |
| atom | 4419 | O | HOH | 722 | 7.019 | 15.603 | 32.528 | 1.00 | 63.48 |
| atom | 4420 | O | HOH | 723 | 36.866 | -13.652 | 15.802 | 1.00 | 27.53 |
| atom | 4421 | O | HOH | 724 | 43.517 | 24.315 | -12.119 | 1.00 | 35.85 |
| atom | 4422 | O | HOH | 725 | 37.708 | -15.785 | 39.175 | 1.00 | 54.73 |
| atom | 4423 | O | HOH | 726 | 5.217 | 0.089 | 34.330 | 1.00 | 62.89 |
| atom | 4424 | O | HOH | 727 | 22.932 | 16.773 | 24.935 | 1.00 | 39.55 |

| | | | | | | | | | |
|---|---|---|---|---|---|---|---|---|---|
| atom | 4425 | O | HOH | 728 | 57.302 | -9.957 | -7.609 | 1.00 | 30.04 |
| atom | 4426 | O | HOH | 729 | 7.732 | 5.303 | -6.078 | 1.00 | 77.38 |
| atom | 4427 | O | HOH | 730 | 20.565 | 18.229 | -3.485 | 1.00 | 34.46 |
| atom | 4428 | O | HOH | 731 | 40.313 | 14.721 | 37.485 | 1.00 | 45.60 |
| atom | 4429 | O | HOH | 732 | 45.334 | -26.610 | 3.300 | 1.00 | 52.20 |
| atom | 4430 | O | HOH | 733 | 21.456 | 12.342 | 33.566 | 1.00 | 43.56 |
| atom | 4431 | O | HOH | 734 | 53.572 | 28.112 | -7.495 | 1.00 | 34.62 |
| atom | 4432 | O | HOH | 735 | 44.392 | 3.535 | -16.228 | 1.00 | 30.67 |
| atom | 4433 | O | HOH | 736 | 21.788 | 26.910 | 17.354 | 1.00 | 29.40 |
| atom | 4434 | O | HOH | 737 | 1.982 | -17.218 | 22.679 | 1.00 | 34.59 |
| atom | 4435 | O | HOH | 738 | 49.791 | -14.945 | 51.490 | 1.00 | 29.55 |
| atom | 4436 | O | HOH | 739 | 65.383 | -10.773 | 31.724 | 1.00 | 21.33 |
| atom | 4437 | O | HOH | 740 | 48.882 | -23.983 | 38.693 | 1.00 | 27.07 |
| atom | 4438 | O | HOH | 741 | 38.324 | 18.978 | 30.698 | 1.00 | 51.87 |
| atom | 4439 | O | HOH | 742 | 18.500 | 12.871 | 19.098 | 1.00 | 37.82 |
| atom | 4440 | O | HOH | 743 | 43.444 | -24.077 | 3.285 | 1.00 | 34.56 |
| atom | 4441 | O | HOH | 744 | 19.073 | 14.997 | 20.678 | 1.00 | 31.54 |
| atom | 4442 | O | HOH | 745 | 43.718 | 14.040 | -9.258 | 1.00 | 48.86 |
| atom | 4443 | O | HOH | 746 | 19.757 | 12.108 | 21.932 | 1.00 | 16.56 |
| atom | 4444 | O | HOH | 747 | 28.539 | -1.306 | 9.121 | 1.00 | 19.91 |
| atom | 4445 | O | HOH | 748 | 33.299 | 13.289 | 28.355 | 1.00 | 31.35 |
| atom | 4446 | O | HOH | 749 | 55.796 | 5.609 | 21.850 | 1.00 | 25.26 |
| atom | 4447 | O | HOH | 750 | 71.236 | -9.462 | 30.197 | 1.00 | 16.58 |
| atom | 4448 | O | HOH | 751 | 48.317 | 16.021 | 28.186 | 1.00 | 53.92 |
| atom | 4449 | O | HOH | 752 | 37.023 | -14.017 | -12.141 | 1.00 | 60.05 |
| atom | 4450 | O | HOH | 753 | 47.056 | 10.022 | 14.240 | 1.00 | 16.37 |
| atom | 4451 | O | HOH | 754 | 41.937 | -14.514 | -12.014 | 1.00 | 28.96 |
| atom | 4452 | O | HOH | 755 | 58.191 | 18.257 | -4.318 | 1.00 | 24.23 |
| atom | 4453 | O | HOH | 756 | 34.855 | 15.167 | -13.410 | 1.00 | 26.63 |
| atom | 4454 | O | HOH | 757 | 46.083 | 7.332 | -4.348 | 1.00 | 41.74 |
| atom | 4455 | O | HOH | 758 | 34.334 | 19.475 | 20.562 | 1.00 | 36.75 |
| atom | 4456 | O | HOH | 759 | 48.439 | 12.298 | 18.766 | 1.00 | 37.20 |
| atom | 4457 | O | HOH | 760 | 26.446 | 9.654 | -7.040 | 1.00 | 24.37 |
| atom | 4458 | O | HOH | 761 | 43.303 | 7.883 | -11.435 | 1.00 | 27.78 |
| atom | 4459 | O | HOH | 762 | 20.443 | 18.574 | 16.107 | 1.00 | 41.69 |
| atom | 4460 | O | HOH | 763 | 48.286 | 15.033 | 32.831 | 1.00 | 57.35 |

| atom | 4461 | 0 | HOH | 764 | 10.893 | 18.980 | 16.387 | 1.00 | 59.57 |
|------|------|---|-----|-----|--------|--------|--------|------|-------|
| atom | 4462 | 0 | HOH | 765 | 34.601 | 4.808 | 35.497 | 1.00 | 43.50 |
| atom | 4463 | 0 | HOH | 766 | 31.888 | 17.167 | -8.403 | 1.00 | 52.16 |
| atom | 4464 | 0 | HOH | 767 | 36.373 | -3.491 | 21.106 | 1.00 | 13.51 |
| atom | 4465 | 0 | HOH | 768 | 23.246 | 19.202 | -6.517 | 1.00 | 41.10 |
| atom | 4466 | 0 | HOH | 769 | 45.743 | 17.021 | -5.597 | 1.00 | 31.22 |
| atom | 4467 | 0 | HOH | 770 | 33.244 | -13.586 | 18.326 | 1.00 | 41.46 |
| atom | 4468 | 0 | HOH | 771 | 57.928 | 30.606 | 16.929 | 1.00 | 38.33 |
| atom | 4469 | 0 | HOH | 772 | 69.124 | -11.949 | 27.989 | 1.00 | 69.32 |
| atom | 4470 | 0 | HOH | 773 | 29.727 | -22.652 | 27.281 | 1.00 | 30.26 |
| atom | 4471 | 0 | HOH | 774 | 37.839 | -4.465 | 13.760 | 1.00 | 22.09 |
| atom | 4472 | 0 | HOH | 775 | 41.248 | -0.915 | 16.241 | 1.00 | 49.75 |
| atom | 4473 | 0 | HOH | 776 | 32.612 | -23.200 | 24.163 | 1.00 | 53.24 |
| atom | 4474 | 0 | HOH | 777 | 47.550 | 17.765 | -7.284 | 1.00 | 30.72 |
| atom | 4475 | 0 | HOH | 778 | 22.460 | 12.258 | 5.644 | 1.00 | 12.35 |
| atom | 4476 | 0 | HOH | 779 | 6.843 | 19.969 | 17.253 | 1.00 | 24.63 |
| atom | 4477 | 0 | HOH | 780 | -1.037 | 0.592 | 12.041 | 1.00 | 32.13 |
| atom | 4478 | 0 | HOH | 781 | 16.879 | 16.213 | -1.036 | 1.00 | 31.03 |
| atom | 4479 | 0 | HOH | 782 | 4.952 | -10.654 | 24.420 | 1.00 | 66.04 |
| atom | 4480 | 0 | HOH | 783 | 30.976 | 21.960 | 23.463 | 1.00 | 26.66 |
| atom | 4481 | 0 | HOH | 784 | 54.549 | -16.852 | 43.639 | 1.00 | 44.86 |
| atom | 4482 | 0 | HOH | 785 | 35.858 | 0.543 | 37.145 | 1.00 | 32.91 |
| atom | 4483 | 0 | HOH | 786 | 37.629 | 0.228 | 45.081 | 1.00 | 25.52 |
| atom | 4484 | 0 | HOH | 787 | 34.090 | -24.595 | 29.357 | 1.00 | 44.67 |
| atom | 4485 | 0 | HOH | 788 | 51.277 | 31.211 | -7.019 | 1.00 | 41.33 |
| atom | 4486 | 0 | HOH | 789 | 30.912 | -2.933 | 10.609 | 1.00 | 72.82 |
| atom | 4487 | 0 | HOH | 790 | 58.746 | -7.303 | -10.029 | 1.00 | 58.21 |
| atom | 4488 | 0 | HOH | 791 | 53.940 | 14.157 | 19.431 | 1.00 | 35.15 |
| atom | 4489 | 0 | HOH | 792 | 32.011 | -0.149 | 14.251 | 1.00 | 47.16 |
| atom | 4490 | 0 | HOH | 793 | 13.553 | 23.341 | 8.528 | 1.00 | 53.03 |
| atom | 4491 | 0 | HOH | 794 | 37.675 | -7.811 | -8.912 | 1.00 | 44.94 |
| atom | 4492 | 0 | HOH | 795 | 48.715 | -0.365 | 3.142 | 1.00 | 41.74 |
| atom | 4493 | 0 | HOH | 796 | 50.589 | -16.659 | 6.378 | 1.00 | 18.40 |
| atom | 4494 | 0 | HOH | 797 | 44.199 | -19.067 | 6.859 | 1.00 | 36.05 |
| atom | 4495 | 0 | HOH | 798 | 53.441 | -26.855 | 20.517 | 1.00 | 50.57 |
| atom | 4496 | 0 | HOH | 799 | 49.408 | -26.628 | 20.724 | 1.00 | 50.72 |

| | | | | | | | | | |
|---|---|---|---|---|---|---|---|---|---|
| atom | 4497 | O | HOH | 800 | 52.640 | -19.756 | 30.198 | 1.00 | 47.50 |
| atom | 4498 | O | HOH | 801 | 18.857 | 3.501 | -9.706 | 1.00 | 21.84 |
| atom | 4499 | O | HOH | 802 | 17.896 | 1.182 | -11.952 | 1.00 | 32.00 |
| atom | 4500 | O | HOH | 803 | 13.919 | 4.000 | -10.397 | 1.00 | 46.84 |
| atom | 4501 | O | HOH | 804 | 11.557 | 5.995 | -4.803 | 1.00 | 48.55 |
| atom | 4502 | O | HOH | 805 | 11.140 | 9.279 | 7.880 | 1.00 | 25.88 |
| atom | 4503 | O | HOH | 806 | 18.676 | 16.664 | 2.033 | 1.00 | 34.39 |
| atom | 4504 | O | HOH | 807 | 27.433 | 31.963 | 16.774 | 1.00 | 20.95 |
| atom | 4505 | O | HOH | 808 | 53.253 | 29.573 | 9.882 | 1.00 | 33.76 |
| atom | 4506 | O | HOH | 809 | 54.907 | 28.009 | 11.555 | 1.00 | 52.31 |
| atom | 4507 | O | HOH | 810 | 62.641 | 11.739 | -4.252 | 1.00 | 32.53 |
| atom | 4508 | O | HOH | 811 | 65.068 | 5.329 | 5.372 | 1.00 | 54.51 |
| atom | 4509 | O | HOH | 812 | 65.803 | 8.849 | 4.108 | 1.00 | 47.37 |
| atom | 4510 | O | HOH | 813 | 43.721 | 20.002 | -11.175 | 1.00 | 30.38 |
| atom | 4511 | O | HOH | 814 | 34.406 | -17.139 | 17.170 | 1.00 | 41.80 |
| atom | 4512 | O | HOH | 815 | 34.703 | -23.625 | 4.105 | 1.00 | 61.03 |
| atom | 4513 | O | HOH | 816 | 34.197 | -17.469 | 3.287 | 1.00 | 44.37 |
| atom | 4514 | O | HOH | 817 | 50.104 | 7.478 | -2.288 | 1.00 | 25.23 |
| atom | 4515 | O | HOH | 818 | 41.677 | 18.551 | 18.796 | 1.00 | 45.33 |
| atom | 4516 | O | HOH | 819 | 38.682 | 19.681 | 20.416 | 1.00 | 45.22 |
| atom | 4517 | O | HOH | 820 | 11.768 | 4.457 | 37.706 | 1.00 | 51.55 |
| atom | 4518 | O | HOH | 821 | 8.218 | 0.919 | 37.720 | 1.00 | 44.65 |
| atom | 4519 | O | HOH | 822 | 3.397 | -1.753 | 33.199 | 1.00 | 33.07 |
| atom | 4520 | O | HOH | 823 | 34.080 | 1.178 | 3.231 | 1.00 | 31.48 |
| atom | 4521 | O | HOH | 824 | 31.348 | 10.501 | -7.664 | 1.00 | 31.78 |
| atom | 4522 | O | HOH | 825 | 38.108 | 4.850 | -12.161 | 1.00 | 31.16 |
| atom | 4523 | O | HOH | 826 | 38.982 | 10.342 | 10.406 | 1.00 | 32.25 |
| atom | 4524 | O | HOH | 827 | 5.149 | 5.654 | 13.265 | 1.00 | 31.34 |
| atom | 4525 | O | HOH | 828 | 6.416 | -8.088 | 20.222 | 1.00 | 49.34 |
| atom | 4526 | O | HOH | 829 | -1.858 | -3.132 | 18.913 | 1.00 | 33.22 |
| atom | 4527 | O | HOH | 830 | 11.873 | 9.162 | 4.510 | 1.00 | 29.62 |
| atom | 4528 | O | HOH | 831 | 19.232 | -5.660 | 17.559 | 1.00 | 26.97 |
| atom | 4529 | O | HOH | 832 | 12.983 | -8.915 | 15.306 | 1.00 | 41.69 |
| atom | 4530 | O | HOH | 833 | 18.416 | -11.585 | 21.008 | 1.00 | 35.22 |
| atom | 4531 | O | HOH | 834 | 22.507 | -3.262 | 33.451 | 1.00 | 77.42 |
| atom | 4532 | O | HOH | 835 | 26.191 | -1.017 | 24.576 | 1.00 | 26.33 |

```
atom  4533  0  HOH  836   36.425   -0.682  16.799  1.00 25.51
atom  4534  0  HOH  837   36.167    2.136  19.428  1.00 35.47
atom  4535  0  HOH  838   31.892   -9.361  18.329  1.00 38.04
atom  4536  0  HOH  839   29.958    0.247  50.234  1.00 39.65
atom  4537  0  HOH  840   25.664   -2.880  45.446  1.00 25.56
atom  4538  0  HOH  841   28.111  -11.787  23.722  1.00 20.36
atom  4539  0  HOH  842   25.802  -13.741  20.864  1.00 48.90
atom  4540  0  HOH  843   66.721  -13.408  27.755  1.00 46.58
atom  4541  0  HOH  844   64.307   -3.316  36.898  1.00 35.37
atom  4542  0  HOH  845   65.583   -0.436  36.938  1.00 37.85
atom  4543  0  HOH  846   41.048  -10.431  50.836  1.00 29.82
atom  4544  0  HOH  847   41.737   18.341  25.701  1.00 64.44
atom  4545  0  HOH  848   30.918   -2.921 -11.856  1.00 30.43
atom  4546  0  HOH  849   51.446   22.839  17.216  1.00 43.87
atom  4547  0  HOH  850   60.785    2.614  -0.874  1.00 35.10
atom  4548  0  HOH  851   56.838   -0.895   2.586  1.00 49.62
atom  4549  0  HOH  852   47.396   10.491  -8.843  1.00 52.54
atom  4550  0  HOH  853   38.574  -14.690   4.444  1.00 30.06
atom  4551  0  HOH  854   18.756   -0.542  39.542  1.00 39.05
atom  4552  0  HOH  855   29.513   29.579   6.595  1.00 36.41
atom  4553  0  HOH  856   32.813   33.195   8.357  1.00 28.63
atom  4554  0  HOH  857   40.705    7.094 -13.994  1.00 29.74
atom  4555  0  HOH  858   50.499    1.421  -2.924  1.00 31.42
atom  4556  0  HOH  859   47.470    0.293 -15.630  1.00 26.52
atom  4557  0  HOH  860   46.826   -4.091  15.608  1.00 27.08
atom  4558  0  HOH  861   43.465   -1.915  20.388  1.00 50.46
atom  4559  0  HOH  862   39.396   -8.251  42.274  1.00 25.43
atom  4560  0  HOH  863   62.025   -4.244  31.799  1.00 24.26
atom  4561  0  HOH  864   51.328   10.747  31.485  1.00 32.16
atom  4562  0  HOH  865   35.283    7.979  42.362  1.00 38.81
atom  4563  0  HOH  866   57.095  -18.565  40.803  1.00 52.30
atom  4564  0  HOH  867   58.155   -2.683  46.266  1.00 22.74
atom  4565  0  HOH  868   48.057   20.382 -12.222  1.00 50.35
atom  4566  0  HOH  869   49.547    0.925   0.838  1.00 51.45
atom  4567  0  HOH  870   46.392    4.053   4.644  1.00 50.25
atom  4568  0  HOH  871   17.601   15.375  19.352  1.00 65.08
```

| atom | 4569 | O | HOH | 872 | 4.479 | 9.329 | 31.775 | 1.00 | 36.17 |
|------|------|---|-----|-----|-------|-------|--------|------|-------|
| atom | 4570 | O | HOH | 873 | 22.319 | 18.788 | -0.495 | 1.00 | 24.87 |
| atom | 4571 | O | HOH | 874 | 35.839 | 30.245 | -0.342 | 1.00 | 48.51 |
| atom | 4572 | O | HOH | 875 | 48.453 | 2.849 | -6.547 | 1.00 | 33.73 |
| atom | 4573 | O | HOH | 876 | 0.605 | 7.424 | 25.945 | 1.00 | 39.41 |
| atom | 4574 | O | HOH | 877 | 2.396 | -3.829 | 13.234 | 1.00 | 30.89 |
| atom | 4575 | O | HOH | 878 | 14.152 | -2.287 | 5.419 | 1.00 | 18.22 |
| atom | 4576 | O | HOH | 879 | 35.704 | -8.865 | 16.297 | 1.00 | 31.23 |
| atom | 4577 | O | HOH | 880 | 47.890 | 11.218 | 33.219 | 1.00 | 51.93 |
| atom | 4578 | O | HOH | 881 | 35.361 | 8.156 | 33.971 | 1.00 | 47.66 |
| atom | 4579 | O | HOH | 882 | 40.052 | 7.610 | 41.373 | 1.00 | 36.84 |
| atom | 4580 | O | HOH | 883 | 56.929 | -15.208 | 46.351 | 1.00 | 55.62 |
| atom | 4581 | O | HOH | 884 | 52.052 | -0.414 | 49.836 | 1.00 | 55.41 |

## Table 3

|        | atom |     | type | numbers | X | Y | Z | Occ | B |
|--------|------|-----|------|---------|--------|--------|--------|------|-------|
| atom | 1 | CB | SER | 1 | -0.472 | 36.318 | 37.903 | 1.00 | 39.80 |
| atom | 2 | OG | SER | 1 | 0.927 | 36.299 | 37.658 | 1.00 | 43.45 |
| atom | 3 | C | SER | 1 | -0.592 | 34.149 | 36.651 | 1.00 | 36.40 |
| atom | 4 | O | SER | 1 | 0.320 | 33.337 | 36.680 | 1.00 | 35.78 |
| atom | 5 | N | SER | 1 | -0.777 | 34.227 | 39.141 | 1.00 | 38.06 |
| atom | 6 | CA | SER | 1 | -1.083 | 34.914 | 37.872 | 1.00 | 36.88 |
| atom | 7 | N | MET | 2 | -1.243 | 34.411 | 35.532 | 1.00 | 36.57 |
| atom | 8 | CA | MET | 2 | -0.922 | 33.825 | 34.250 | 1.00 | 36.47 |
| atom | 9 | CB | MET | 2 | -2.049 | 34.140 | 33.254 | 1.00 | 34.39 |
| atom | 10 | CG | MET | 2 | -3.375 | 33.528 | 33.693 | 1.00 | 34.97 |
| atom | 11 | SD | MET | 2 | -3.304 | 31.708 | 33.770 | 1.00 | 36.74 |
| atom | 12 | CE | MET | 2 | -3.074 | 31.335 | 32.043 | 1.00 | 32.43 |
| atom | 13 | C | MET | 2 | 0.423 | 34.384 | 33.775 | 1.00 | 37.18 |
| atom | 14 | O | MET | 2 | 0.657 | 35.595 | 33.779 | 1.00 | 36.40 |
| atom | 15 | N | SER | 3 | 1.297 | 33.489 | 33.332 | 1.00 | 36.77 |
| atom | 16 | CA | SER | 3 | 2.584 | 33.855 | 32.778 | 1.00 | 37.83 |
| atom | 17 | CB | SER | 3 | 3.395 | 32.599 | 32.438 | 1.00 | 36.10 |
| atom | 18 | OG | SER | 3 | 2.772 | 31.910 | 31.367 | 1.00 | 34.54 |
| atom | 19 | C | SER | 3 | 2.375 | 34.683 | 31.518 | 1.00 | 38.41 |
| atom | 20 | O | SER | 3 | 3.198 | 35.511 | 31.134 | 1.00 | 39.16 |
| atom | 21 | N | TYR | 4 | 1.350 | 34.380 | 30.737 | 1.00 | 38.61 |
| atom | 22 | CA | TYR | 4 | 1.031 | 35.093 | 29.512 | 1.00 | 39.70 |
| atom | 23 | CB | TYR | 4 | 1.606 | 34.437 | 28.268 | 1.00 | 40.36 |
| atom | 24 | CG | TYR | 4 | 3.109 | 34.438 | 28.099 | 1.00 | 42.35 |
| atom | 25 | CD1 | TYR | 4 | 3.896 | 33.421 | 28.635 | 1.00 | 41.85 |
| atom | 26 | CE1 | TYR | 4 | 5.267 | 33.433 | 28.488 | 1.00 | 42.19 |
| atom | 27 | CD2 | TYR | 4 | 3.747 | 35.449 | 27.382 | 1.00 | 43.13 |
| atom | 28 | CE2 | TYR | 4 | 5.120 | 35.460 | 27.218 | 1.00 | 42.63 |
| atom | 29 | CZ | TYR | 4 | 5.873 | 34.443 | 27.774 | 1.00 | 41.86 |
| atom | 30 | OH | TYR | 4 | 7.236 | 34.448 | 27.621 | 1.00 | 40.75 |
| atom | 31 | C | TYR | 4 | -0.496 | 35.212 | 29.368 | 1.00 | 39.55 |
| atom | 32 | O | TYR | 4 | -1.275 | 34.457 | 29.932 | 1.00 | 39.12 |

| atom | 33 | N | THR | 5 | -0.939 | 36.220 | 28.660 | 1.00 | 39.23 |
|------|----|------|-----|---|--------|--------|--------|------|-------|
| atom | 34 | CA | THR | 5 | -2.335 | 36.497 | 28.358 | 1.00 | 40.53 |
| atom | 35 | CB | THR | 5 | -2.938 | 37.630 | 29.184 | 1.00 | 38.69 |
| atom | 36 | OG1 | THR | 5 | -3.046 | 37.279 | 30.571 | 1.00 | 34.93 |
| atom | 37 | CG2 | THR | 5 | -4.318 | 37.999 | 28.669 | 1.00 | 38.43 |
| atom | 38 | C | THR | 5 | -2.236 | 36.839 | 26.867 | 1.00 | 41.97 |
| atom | 39 | O | THR | 5 | -1.393 | 37.692 | 26.558 | 1.00 | 42.83 |
| atom | 40 | N | TRP | 6 | -2.900 | 36.119 | 25.992 | 1.00 | 42.95 |
| atom | 41 | CA | TRP | 6 | -2.730 | 36.317 | 24.558 | 1.00 | 44.74 |
| atom | 42 | CB | TRP | 6 | -2.542 | 34.966 | 23.842 | 1.00 | 43.82 |
| atom | 43 | CG | TRP | 6 | -1.352 | 34.227 | 24.390 | 1.00 | 43.63 |
| atom | 44 | CD2 | TRP | 6 | 0.023 | 34.517 | 24.113 | 1.00 | 43.11 |
| atom | 45 | CE2 | TRP | 6 | 0.797 | 33.594 | 24.849 | 1.00 | 44.18 |
| atom | 46 | CE3 | TRP | 6 | 0.678 | 35.449 | 23.304 | 1.00 | 42.40 |
| atom | 47 | CD1 | TRP | 6 | -1.363 | 33.201 | 25.284 | 1.00 | 43.73 |
| atom | 48 | NE1 | TRP | 6 | -0.078 | 32.803 | 25.557 | 1.00 | 45.01 |
| atom | 49 | CZ2 | TRP | 6 | 2.194 | 33.578 | 24.807 | 1.00 | 41.91 |
| atom | 50 | CZ3 | TRP | 6 | 2.069 | 35.433 | 23.262 | 1.00 | 43.17 |
| atom | 51 | CH2 | TRP | 6 | 2.809 | 34.510 | 24.016 | 1.00 | 41.02 |
| atom | 52 | C | TRP | 6 | -3.875 | 37.090 | 23.926 | 1.00 | 46.32 |
| atom | 53 | O | TRP | 6 | -4.998 | 37.140 | 24.420 | 1.00 | 46.84 |
| atom | 54 | N | THR | 7 | -3.579 | 37.665 | 22.768 | 1.00 | 47.47 |
| atom | 55 | CA | THR | 7 | -4.526 | 38.536 | 22.086 | 1.00 | 48.96 |
| atom | 56 | CB | THR | 7 | -3.702 | 39.778 | 21.658 | 1.00 | 49.60 |
| atom | 57 | OG1 | THR | 7 | -4.344 | 40.976 | 22.089 | 1.00 | 50.99 |
| atom | 58 | CG2 | THR | 7 | -3.446 | 39.832 | 20.169 | 1.00 | 47.40 |
| atom | 59 | C | THR | 7 | -5.233 | 37.927 | 20.894 | 1.00 | 49.38 |
| atom | 60 | O | THR | 7 | -6.376 | 38.271 | 20.585 | 1.00 | 50.13 |
| atom | 61 | N | GLY | 8 | -4.547 | 37.056 | 20.165 | 1.00 | 49.09 |
| atom | 62 | CA | GLY | 8 | -5.109 | 36.501 | 18.931 | 1.00 | 48.71 |
| atom | 63 | C | GLY | 8 | -4.080 | 36.756 | 17.830 | 1.00 | 48.37 |
| atom | 64 | O | GLY | 8 | -3.783 | 35.854 | 17.062 | 1.00 | 49.34 |
| atom | 65 | N | ALA | 9 | -3.503 | 37.951 | 17.803 | 1.00 | 48.26 |
| atom | 66 | CA | ALA | 9 | -2.475 | 38.279 | 16.824 | 1.00 | 48.30 |
| atom | 67 | CB | ALA | 9 | -1.916 | 39.665 | 17.083 | 1.00 | 47.45 |
| atom | 68 | C | ALA | 9 | -1.376 | 37.213 | 16.879 | 1.00 | 48.57 |

| atom | 69  | O   | ALA | 9  | -0.934 | 36.800 | 17.951 | 1.00 | 48.51 |
|------|-----|-----|-----|----|--------|--------|--------|------|-------|
| atom | 70  | N   | LEU | 10 | -0.976 | 36.732 | 15.713 | 1.00 | 48.18 |
| atom | 71  | CA  | LEU | 10 | 0.035  | 35.704 | 15.606 | 1.00 | 48.82 |
| atom | 72  | CB  | LEU | 10 | -0.061 | 35.030 | 14.223 | 1.00 | 48.40 |
| atom | 73  | CG  | LEU | 10 | -1.448 | 34.465 | 13.885 | 1.00 | 47.81 |
| atom | 74  | CD1 | LEU | 10 | -1.679 | 34.402 | 12.390 | 1.00 | 46.98 |
| atom | 75  | CD2 | LEU | 10 | -1.613 | 33.082 | 14.500 | 1.00 | 48.24 |
| atom | 76  | C   | LEU | 10 | 1.434  | 36.285 | 15.775 | 1.00 | 49.54 |
| atom | 77  | O   | LEU | 10 | 1.670  | 37.491 | 15.667 | 1.00 | 49.75 |
| atom | 78  | N   | ILE | 11 | 2.370  | 35.380 | 16.025 | 1.00 | 49.50 |
| atom | 79  | CA  | ILE | 11 | 3.778  | 35.757 | 16.123 | 1.00 | 49.29 |
| atom | 80  | CB  | ILE | 11 | 4.554  | 34.893 | 17.124 | 1.00 | 45.30 |
| atom | 81  | CG2 | ILE | 11 | 6.051  | 35.167 | 17.055 | 1.00 | 42.68 |
| atom | 82  | CG1 | ILE | 11 | 3.964  | 35.199 | 18.499 | 1.00 | 42.36 |
| atom | 83  | CD1 | ILE | 11 | 4.247  | 34.188 | 19.568 | 1.00 | 41.57 |
| atom | 84  | C   | ILE | 11 | 4.322  | 35.582 | 14.708 | 1.00 | 50.19 |
| atom | 85  | O   | ILE | 11 | 4.488  | 34.465 | 14.233 | 1.00 | 50.42 |
| atom | 86  | N   | THR | 12 | 4.525  | 36.699 | 14.049 | 1.00 | 51.43 |
| atom | 87  | CA  | THR | 12 | 4.942  | 36.697 | 12.670 | 1.00 | 53.79 |
| atom | 88  | CB  | THR | 12 | 4.413  | 37.972 | 11.967 | 1.00 | 52.90 |
| atom | 89  | OG1 | THR | 12 | 5.085  | 39.115 | 12.523 | 1.00 | 51.37 |
| atom | 90  | CG2 | THR | 12 | 2.913  | 38.058 | 12.180 | 1.00 | 52.05 |
| atom | 91  | C   | THR | 12 | 6.437  | 36.686 | 12.427 | 1.00 | 56.24 |
| atom | 92  | O   | THR | 12 | 7.232  | 37.311 | 13.124 | 1.00 | 55.82 |
| atom | 93  | N   | PRO | 13 | 6.780  | 36.014 | 11.335 | 1.00 | 57.99 |
| atom | 94  | CD  | PRO | 13 | 5.897  | 35.269 | 10.411 | 1.00 | 58.37 |
| atom | 95  | CA  | PRO | 13 | 8.161  | 35.986 | 10.881 | 1.00 | 59.97 |
| atom | 96  | CB  | PRO | 13 | 8.196  | 34.742 | 10.005 | 1.00 | 59.58 |
| atom | 97  | CG  | PRO | 13 | 6.835  | 34.657 | 9.409  | 1.00 | 58.45 |
| atom | 98  | C   | PRO | 13 | 8.371  | 37.247 | 10.051 | 1.00 | 61.93 |
| atom | 99  | O   | PRO | 13 | 7.380  | 37.851 | 9.628  | 1.00 | 61.46 |
| atom | 100 | N   | CYS | 14 | 9.614  | 37.628 | 9.826  | 1.00 | 64.47 |
| atom | 101 | CA  | CYS | 14 | 9.833  | 38.792 | 8.962  | 1.00 | 68.29 |
| atom | 102 | CB  | CYS | 14 | 10.793 | 39.771 | 9.607  | 1.00 | 70.08 |
| atom | 103 | SG  | CYS | 14 | 12.293 | 38.991 | 10.257 | 1.00 | 71.95 |
| atom | 104 | C   | CYS | 14 | 10.335 | 38.261 | 7.621  | 1.00 | 70.31 |

| atom | 105 | O   | CYS | 14 | 10.042 | 38.807 | 6.560  | 1.00 | 71.30 |
|------|-----|-----|-----|----|--------|--------|--------|------|-------|
| atom | 106 | N   | ALA | 15 | 11.052 | 37.141 | 7.692  | 1.00 | 71.65 |
| atom | 107 | CA  | ALA | 15 | 11.606 | 36.497 | 6.509  | 1.00 | 72.80 |
| atom | 108 | CB  | ALA | 15 | 13.129 | 36.532 | 6.571  | 1.00 | 72.78 |
| atom | 109 | C   | ALA | 15 | 11.126 | 35.054 | 6.367  | 1.00 | 73.30 |
| atom | 110 | O   | ALA | 15 | 10.694 | 34.439 | 7.341  | 1.00 | 73.61 |
| atom | 111 | N   | ALA | 16 | 11.194 | 34.530 | 5.146  | 1.00 | 73.43 |
| atom | 112 | CA  | ALA | 16 | 10.807 | 33.145 | 4.897  | 1.00 | 73.44 |
| atom | 113 | CB  | ALA | 16 | 10.812 | 32.830 | 3.413  | 1.00 | 74.24 |
| atom | 114 | C   | ALA | 16 | 11.786 | 32.231 | 5.638  | 1.00 | 72.96 |
| atom | 115 | O   | ALA | 16 | 12.990 | 32.269 | 5.413  | 1.00 | 72.72 |
| atom | 116 | N   | GLU | 17 | 11.258 | 31.448 | 6.559  | 1.00 | 72.63 |
| atom | 117 | CA  | GLU | 17 | 12.042 | 30.526 | 7.366  | 1.00 | 72.40 |
| atom | 118 | CB  | GLU | 17 | 11.392 | 30.394 | 8.747  | 1.00 | 70.45 |
| atom | 119 | CG  | GLU | 17 | 10.858 | 31.696 | 9.331  | 1.00 | 67.97 |
| atom | 120 | CD  | GLU | 17 | 9.677  | 31.473 | 10.256 | 1.00 | 66.73 |
| atom | 121 | OE1 | GLU | 17 | 8.531  | 31.389 | 9.766  | 1.00 | 67.03 |
| atom | 122 | OE2 | GLU | 17 | 9.866  | 31.379 | 11.481 | 1.00 | 65.53 |
| atom | 123 | C   | GLU | 17 | 12.104 | 29.155 | 6.697  | 1.00 | 72.58 |
| atom | 124 | O   | GLU | 17 | 11.043 | 28.578 | 6.433  | 1.00 | 72.64 |
| atom | 125 | N   | GLU | 18 | 13.297 | 28.654 | 6.398  | 1.00 | 72.52 |
| atom | 126 | CA  | GLU | 18 | 13.399 | 27.331 | 5.775  | 1.00 | 72.92 |
| atom | 127 | CB  | GLU | 18 | 14.647 | 27.182 | 4.922  | 1.00 | 72.56 |
| atom | 128 | CG  | GLU | 18 | 14.654 | 27.982 | 3.635  | 1.00 | 72.95 |
| atom | 129 | CD  | GLU | 18 | 13.523 | 27.640 | 2.687  | 1.00 | 73.36 |
| atom | 130 | OE1 | GLU | 18 | 12.915 | 26.554 | 2.803  | 1.00 | 73.08 |
| atom | 131 | OE2 | GLU | 18 | 13.245 | 28.495 | 1.819  | 1.00 | 73.29 |
| atom | 132 | C   | GLU | 18 | 13.364 | 26.269 | 6.867  | 1.00 | 73.09 |
| atom | 133 | O   | GLU | 18 | 13.788 | 26.584 | 7.979  | 1.00 | 72.95 |
| atom | 134 | N   | SER | 19 | 12.863 | 25.073 | 6.572  | 1.00 | 73.60 |
| atom | 135 | CA  | SER | 19 | 12.776 | 24.043 | 7.601  | 1.00 | 74.00 |
| atom | 136 | CB  | SER | 19 | 11.312 | 23.900 | 8.038  | 1.00 | 73.40 |
| atom | 137 | OG  | SER | 19 | 10.474 | 23.561 | 6.952  | 1.00 | 73.41 |
| atom | 138 | C   | SER | 19 | 13.304 | 22.669 | 7.226  | 1.00 | 74.74 |
| atom | 139 | O   | SER | 19 | 13.405 | 21.802 | 8.100  | 1.00 | 74.11 |
| atom | 140 | N   | LYS | 20 | 13.659 | 22.448 | 5.969  | 1.00 | 75.90 |

| atom | 141 | CA | LYS | 20 | 14.167 | 21.136 | 5.564 | 1.00 | 77.54 |
|------|-----|-----|-----|----|--------|--------|-------|------|-------|
| atom | 142 | CB | LYS | 20 | 13.161 | 20.477 | 4.633 | 1.00 | 78.75 |
| atom | 143 | CG | LYS | 20 | 12.981 | 18.984 | 4.755 | 1.00 | 81.11 |
| atom | 144 | CD | LYS | 20 | 12.326 | 18.509 | 6.040 | 1.00 | 82.87 |
| atom | 145 | CE | LYS | 20 | 13.347 | 17.985 | 7.035 | 1.00 | 83.93 |
| atom | 146 | NZ | LYS | 20 | 12.768 | 17.017 | 8.007 | 1.00 | 83.21 |
| atom | 147 | C  | LYS | 20 | 15.564 | 21.262 | 4.973 | 1.00 | 78.02 |
| atom | 148 | O  | LYS | 20 | 15.925 | 22.262 | 4.360 | 1.00 | 77.75 |
| atom | 149 | N  | LEU | 21 | 16.406 | 20.279 | 5.263 | 1.00 | 79.33 |
| atom | 150 | CA | LEU | 21 | 17.784 | 20.247 | 4.790 | 1.00 | 81.02 |
| atom | 151 | CB | LEU | 21 | 18.519 | 19.090 | 5.434 | 1.00 | 79.55 |
| atom | 152 | CG | LEU | 21 | 19.932 | 19.216 | 5.969 | 1.00 | 78.49 |
| atom | 153 | CD1 | LEU | 21 | 20.464 | 17.819 | 6.265 | 1.00 | 77.56 |
| atom | 154 | CD2 | LEU | 21 | 20.872 | 19.994 | 5.076 | 1.00 | 78.06 |
| atom | 155 | C  | LEU | 21 | 17.749 | 20.071 | 3.274 | 1.00 | 82.89 |
| atom | 156 | O  | LEU | 21 | 17.162 | 19.110 | 2.789 | 1.00 | 82.63 |
| atom | 157 | N  | PRO | 22 | 18.365 | 20.990 | 2.551 | 1.00 | 84.77 |
| atom | 158 | CD | PRO | 22 | 19.074 | 22.168 | 3.118 | 1.00 | 85.14 |
| atom | 159 | CA | PRO | 22 | 18.403 | 20.969 | 1.106 | 1.00 | 86.53 |
| atom | 160 | CB | PRO | 22 | 19.129 | 22.266 | 0.760 | 1.00 | 86.09 |
| atom | 161 | CG | PRO | 22 | 19.912 | 22.637 | 1.961 | 1.00 | 85.74 |
| atom | 162 | C  | PRO | 22 | 19.128 | 19.792 | 0.480 | 1.00 | 88.37 |
| atom | 163 | O  | PRO | 22 | 19.907 | 19.069 | 1.103 | 1.00 | 88.95 |
| atom | 164 | N  | ILE | 23 | 18.892 | 19.596 | -0.814 | 1.00 | 90.04 |
| atom | 165 | CA | ILE | 23 | 19.504 | 18.531 | -1.601 | 1.00 | 91.20 |
| atom | 166 | CB | ILE | 23 | 18.624 | 18.197 | -2.820 | 1.00 | 91.67 |
| atom | 167 | CG2 | ILE | 23 | 19.190 | 17.015 | -3.594 | 1.00 | 92.15 |
| atom | 168 | CG1 | ILE | 23 | 17.181 | 17.929 | -2.387 | 1.00 | 91.61 |
| atom | 169 | CD1 | ILE | 23 | 16.983 | 16.798 | -1.405 | 1.00 | 91.74 |
| atom | 170 | C  | ILE | 23 | 20.905 | 18.936 | -2.043 | 1.00 | 91.77 |
| atom | 171 | O  | ILE | 23 | 21.098 | 19.513 | -3.112 | 1.00 | 91.80 |
| atom | 172 | N  | ASN | 24 | 21.889 | 18.647 | -1.196 | 1.00 | 92.21 |
| atom | 173 | CA | ASN | 24 | 23.277 | 18.997 | -1.466 | 1.00 | 92.31 |
| atom | 174 | CB | ASN | 24 | 23.737 | 20.109 | -0.524 | 1.00 | 94.23 |
| atom | 175 | CG | ASN | 24 | 25.223 | 20.382 | -0.563 | 1.00 | 95.58 |
| atom | 176 | OD1 | ASN | 24 | 25.890 | 20.202 | -1.584 | 1.00 | 96.47 |

| atom | 177 | ND2 | ASN | 24 | 25.764 | 20.818 | 0.567 | 1.00 | 96.25 |
|------|-----|-----|-----|----|--------|--------|-------|------|-------|
| atom | 178 | C | ASN | 24 | 24.191 | 17.783 | -1.348 | 1.00 | 91.74 |
| atom | 179 | 0 | ASN | 24 | 24.074 | 16.955 | -0.447 | 1.00 | 91.73 |
| atom | 180 | N | ALA | 25 | 25.150 | 17.712 | -2.268 | 1.00 | 90.74 |
| atom | 181 | CA | ALA | 25 | 26.105 | 16.617 | -2.308 | 1.00 | 89.35 |
| atom | 182 | CB | ALA | 25 | 26.779 | 16.552 | -3.672 | 1.00 | 90.14 |
| atom | 183 | C | ALA | 25 | 27.152 | 16.711 | -1.207 | 1.00 | 88.10 |
| atom | 184 | 0 | ALA | 25 | 27.636 | 15.669 | -0.755 | 1.00 | 88.08 |
| atom | 185 | N | LEU | 26 | 27.491 | 17.917 | -0.763 | 1.00 | 86.59 |
| atom | 186 | CA | LEU | 26 | 28.478 | 18.075 | 0.304 | 1.00 | 84.50 |
| atom | 187 | CB | LEU | 26 | 29.138 | 19.445 | 0.243 | 1.00 | 85.13 |
| atom | 188 | CG | LEU | 26 | 30.578 | 19.586 | 0.748 | 1.00 | 85.62 |
| atom | 189 | CD1 | LEU | 26 | 31.520 | 18.619 | 0.033 | 1.00 | 86.28 |
| atom | 190 | CD2 | LEU | 26 | 31.073 | 21.011 | 0.482 | 1.00 | 85.78 |
| atom | 191 | C | LEU | 26 | 27.849 | 17.800 | 1.667 | 1.00 | 82.81 |
| atom | 192 | 0 | LEU | 26 | 28.544 | 17.366 | 2.589 | 1.00 | 82.13 |
| atom | 193 | N | SER | 27 | 26.541 | 18.014 | 1.793 | 1.00 | 81.07 |
| atom | 194 | CA | SER | 27 | 25.827 | 17.749 | 3.031 | 1.00 | 78.89 |
| atom | 195 | CB | SER | 27 | 24.477 | 18.450 | 3.082 | 1.00 | 79.48 |
| atom | 196 | OG | SER | 27 | 23.510 | 17.798 | 2.275 | 1.00 | 80.57 |
| atom | 197 | C | SER | 27 | 25.611 | 16.249 | 3.205 | 1.00 | 77.65 |
| atom | 198 | 0 | SER | 27 | 25.787 | 15.723 | 4.308 | 1.00 | 77.29 |
| atom | 199 | N | ASN | 28 | 25.314 | 15.537 | 2.110 | 1.00 | 75.91 |
| atom | 200 | CA | ASN | 28 | 25.118 | 14.090 | 2.180 | 1.00 | 74.01 |
| atom | 201 | CB | ASN | 28 | 24.601 | 13.464 | 0.892 | 1.00 | 77.84 |
| atom | 202 | CG | ASN | 28 | 25.639 | 12.937 | -0.073 | 1.00 | 80.62 |
| atom | 203 | OD1 | ASN | 28 | 26.330 | 11.939 | 0.167 | 1.00 | 82.15 |
| atom | 204 | ND2 | ASN | 28 | 25.778 | 13.604 | -1.216 | 1.00 | 81.65 |
| atom | 205 | C | ASN | 28 | 26.410 | 13.405 | 2.617 | 1.00 | 71.58 |
| atom | 206 | 0 | ASN | 28 | 26.422 | 12.379 | 3.291 | 1.00 | 71.16 |
| atom | 207 | N | SER | 29 | 27.545 | 13.972 | 2.236 | 1.00 | 69.48 |
| atom | 208 | CA | SER | 29 | 28.874 | 13.531 | 2.610 | 1.00 | 66.84 |
| atom | 209 | CB | SER | 29 | 29.894 | 14.580 | 2.152 | 1.00 | 67.30 |
| atom | 210 | OG | SER | 29 | 31.223 | 14.100 | 2.189 | 1.00 | 67.93 |
| atom | 211 | C | SER | 29 | 28.989 | 13.314 | 4.119 | 1.00 | 64.86 |
| atom | 212 | 0 | SER | 29 | 29.578 | 12.319 | 4.553 | 1.00 | 64.88 |

| atom | 213 | N   | LEU | 30 | 28.429 | 14.214 | 4.921 | 1.00 61.99 |
|------|-----|-----|-----|----|--------|--------|-------|------------|
| atom | 214 | CA  | LEU | 30 | 28.437 | 14.096 | 6.363 | 1.00 59.28 |
| atom | 215 | CB  | LEU | 30 | 28.273 | 15.469 | 7.011 | 1.00 57.26 |
| atom | 216 | CG  | LEU | 30 | 29.093 | 15.827 | 8.247 | 1.00 55.69 |
| atom | 217 | CD1 | LEU | 30 | 28.493 | 17.089 | 8.879 | 1.00 55.27 |
| atom | 218 | CD2 | LEU | 30 | 29.189 | 14.727 | 9.280 | 1.00 53.50 |
| atom | 219 | C   | LEU | 30 | 27.358 | 13.153 | 6.882 | 1.00 57.55 |
| atom | 220 | O   | LEU | 30 | 27.716 | 12.215 | 7.592 | 1.00 57.89 |
| atom | 221 | N   | LEU | 31 | 26.077 | 13.390 | 6.639 | 1.00 56.00 |
| atom | 222 | CA  | LEU | 31 | 25.061 | 12.453 | 7.152 | 1.00 54.63 |
| atom | 223 | CB  | LEU | 31 | 24.421 | 12.925 | 8.442 | 1.00 52.22 |
| atom | 224 | CG  | LEU | 31 | 23.524 | 14.157 | 8.464 | 1.00 50.49 |
| atom | 225 | CD1 | LEU | 31 | 22.123 | 13.818 | 7.970 | 1.00 48.56 |
| atom | 226 | CD2 | LEU | 31 | 23.460 | 14.755 | 9.864 | 1.00 48.41 |
| atom | 227 | C   | LEU | 31 | 24.066 | 12.167 | 6.030 | 1.00 53.76 |
| atom | 228 | O   | LEU | 31 | 23.695 | 13.079 | 5.290 | 1.00 53.21 |
| atom | 229 | N   | ALA | 32 | 23.686 | 10.906 | 5.890 | 1.00 53.25 |
| atom | 230 | CA  | ALA | 32 | 22.805 | 10.470 | 4.817 | 1.00 52.62 |
| atom | 231 | CB  | ALA | 32 | 23.105 | 9.008  | 4.481 | 1.00 51.97 |
| atom | 232 | C   | ALA | 32 | 21.333 | 10.628 | 5.143 | 1.00 52.49 |
| atom | 233 | O   | ALA | 32 | 20.537 | 10.911 | 4.239 | 1.00 52.30 |
| atom | 234 | N   | HIS | 33 | 20.935 | 10.485 | 6.408 | 1.00 52.12 |
| atom | 235 | CA  | HIS | 33 | 19.525 | 10.623 | 6.760 | 1.00 52.10 |
| atom | 236 | CB  | HIS | 33 | 19.203 | 9.853  | 8.038 | 1.00 50.55 |
| atom | 237 | CG  | HIS | 33 | 19.707 | 8.450  | 8.102 | 1.00 49.72 |
| atom | 238 | CD2 | HIS | 33 | 19.745 | 7.574  | 9.139 | 1.00 48.19 |
| atom | 239 | ND1 | HIS | 33 | 20.228 | 7.780  | 7.014 | 1.00 49.49 |
| atom | 240 | CE1 | HIS | 33 | 20.573 | 6.560  | 7.392 | 1.00 49.32 |
| atom | 241 | NE2 | HIS | 33 | 20.290 | 6.412  | 8.677 | 1.00 47.18 |
| atom | 242 | C   | HIS | 33 | 19.099 | 12.078 | 6.906 | 1.00 52.75 |
| atom | 243 | O   | HIS | 33 | 18.701 | 12.544 | 7.978 | 1.00 52.91 |
| atom | 244 | N   | HIS | 34 | 19.087 | 12.840 | 5.824 | 1.00 53.30 |
| atom | 245 | CA  | HIS | 34 | 18.733 | 14.242 | 5.790 | 1.00 54.14 |
| atom | 246 | CB  | HIS | 34 | 18.958 | 14.814 | 4.389 | 1.00 55.06 |
| atom | 247 | CG  | HIS | 34 | 18.052 | 14.209 | 3.361 | 1.00 56.41 |
| atom | 248 | CD2 | HIS | 34 | 16.742 | 14.415 | 3.093 | 1.00 57.74 |

| atom | 249 | ND1 | HIS | 34 | 18.477 | 13.263 | 2.455 | 1.00 | 56.68 |
|------|-----|-----|-----|-----|--------|--------|-------|------|-------|
| atom | 250 | CE1 | HIS | 34 | 17.470 | 12.913 | 1.677 | 1.00 | 57.31 |
| atom | 251 | NE2 | HIS | 34 | 16.400 | 13.591 | 2.046 | 1.00 | 57.46 |
| atom | 252 | C | HIS | 34 | 17.299 | 14.514 | 6.218 | 1.00 | 54.70 |
| atom | 253 | O | HIS | 34 | 16.991 | 15.575 | 6.769 | 1.00 | 54.03 |
| atom | 254 | N | ASN | 35 | 16.401 | 13.544 | 6.042 | 1.00 | 54.88 |
| atom | 255 | CA | ASN | 35 | 15.019 | 13.695 | 6.444 | 1.00 | 55.81 |
| atom | 256 | CB | ASN | 35 | 14.130 | 12.520 | 6.023 | 1.00 | 57.87 |
| atom | 257 | CG | ASN | 35 | 13.729 | 12.545 | 4.567 | 1.00 | 59.00 |
| atom | 258 | OD1 | ASN | 35 | 13.223 | 13.553 | 4.066 | 1.00 | 59.97 |
| atom | 259 | ND2 | ASN | 35 | 13.963 | 11.422 | 3.901 | 1.00 | 59.90 |
| atom | 260 | C | ASN | 35 | 14.813 | 13.876 | 7.943 | 1.00 | 55.51 |
| atom | 261 | O | ASN | 35 | 13.693 | 14.248 | 8.318 | 1.00 | 55.52 |
| atom | 262 | N | MET | 36 | 15.792 | 13.604 | 8.797 | 1.00 | 55.29 |
| atom | 263 | CA | MET | 36 | 15.595 | 13.787 | 10.230 | 1.00 | 54.58 |
| atom | 264 | CB | MET | 36 | 16.036 | 12.540 | 10.976 | 1.00 | 53.90 |
| atom | 265 | CG | MET | 36 | 17.441 | 12.040 | 10.726 | 1.00 | 55.33 |
| atom | 266 | SD | MET | 36 | 17.657 | 10.292 | 11.131 | 1.00 | 53.50 |
| atom | 267 | CE | MET | 36 | 16.721 | 10.170 | 12.652 | 1.00 | 53.11 |
| atom | 268 | C | MET | 36 | 16.218 | 15.068 | 10.765 | 1.00 | 54.43 |
| atom | 269 | O | MET | 36 | 16.223 | 15.320 | 11.975 | 1.00 | 53.41 |
| atom | 270 | N | VAL | 37 | 16.733 | 15.917 | 9.880 | 1.00 | 54.25 |
| atom | 271 | CA | VAL | 37 | 17.304 | 17.206 | 10.286 | 1.00 | 54.04 |
| atom | 272 | CB | VAL | 37 | 18.595 | 17.558 | 9.540 | 1.00 | 52.81 |
| atom | 273 | CG1 | VAL | 37 | 19.191 | 18.894 | 9.947 | 1.00 | 51.32 |
| atom | 274 | CG2 | VAL | 37 | 19.626 | 16.459 | 9.796 | 1.00 | 52.64 |
| atom | 275 | C | VAL | 37 | 16.229 | 18.257 | 10.025 | 1.00 | 53.96 |
| atom | 276 | O | VAL | 37 | 15.707 | 18.243 | 8.908 | 1.00 | 54.73 |
| atom | 277 | N | TYR | 38 | 15.873 | 19.073 | 11.010 | 1.00 | 53.59 |
| atom | 278 | CA | TYR | 38 | 14.809 | 20.051 | 10.757 | 1.00 | 52.71 |
| atom | 279 | CB | TYR | 38 | 13.479 | 19.447 | 11.198 | 1.00 | 52.45 |
| atom | 280 | CG | TYR | 38 | 13.317 | 19.347 | 12.697 | 1.00 | 53.31 |
| atom | 281 | CD1 | TYR | 38 | 12.697 | 20.357 | 13.427 | 1.00 | 52.98 |
| atom | 282 | CE1 | TYR | 38 | 12.551 | 20.263 | 14.799 | 1.00 | 52.88 |
| atom | 283 | CD2 | TYR | 38 | 13.792 | 18.242 | 13.391 | 1.00 | 53.50 |
| atom | 284 | CE2 | TYR | 38 | 13.635 | 18.141 | 14.763 | 1.00 | 53.26 |

| atom | 285 | CZ  | TYR | 38 | 13.022 | 19.152 | 15.465 | 1.00 | 53.19 |
| atom | 286 | OH  | TYR | 38 | 12.871 | 19.052 | 16.832 | 1.00 | 52.72 |
| atom | 287 | C   | TYR | 38 | 15.069 | 21.381 | 11.446 | 1.00 | 51.93 |
| atom | 288 | O   | TYR | 38 | 15.951 | 21.468 | 12.307 | 1.00 | 52.29 |
| atom | 289 | N   | ALA | 39 | 14.279 | 22.392 | 11.097 | 1.00 | 50.25 |
| atom | 290 | CA  | ALA | 39 | 14.440 | 23.702 | 11.723 | 1.00 | 49.26 |
| atom | 291 | CB  | ALA | 39 | 15.096 | 24.669 | 10.761 | 1.00 | 45.03 |
| atom | 292 | C   | ALA | 39 | 13.111 | 24.247 | 12.241 | 1.00 | 49.15 |
| atom | 293 | O   | ALA | 39 | 12.069 | 24.102 | 11.619 | 1.00 | 49.04 |
| atom | 294 | N   | THR | 40 | 13.140 | 24.859 | 13.420 | 1.00 | 48.90 |
| atom | 295 | CA  | THR | 40 | 11.977 | 25.487 | 14.010 | 1.00 | 48.54 |
| atom | 296 | CB  | THR | 40 | 12.243 | 25.928 | 15.463 | 1.00 | 48.35 |
| atom | 297 | OG1 | THR | 40 | 13.371 | 26.808 | 15.563 | 1.00 | 48.45 |
| atom | 298 | CG2 | THR | 40 | 12.479 | 24.730 | 16.373 | 1.00 | 45.92 |
| atom | 299 | C   | THR | 40 | 11.582 | 26.698 | 13.161 | 1.00 | 48.73 |
| atom | 300 | O   | THR | 40 | 12.460 | 27.366 | 12.611 | 1.00 | 48.50 |
| atom | 301 | N   | THR | 41 | 10.292 | 26.922 | 12.948 | 1.00 | 48.44 |
| atom | 302 | CA  | THR | 41 | 9.792  | 28.058 | 12.204 | 1.00 | 48.28 |
| atom | 303 | CB  | THR | 41 | 9.183  | 27.844 | 10.806 | 1.00 | 48.31 |
| atom | 304 | OG1 | THR | 41 | 7.903  | 27.197 | 10.961 | 1.00 | 44.40 |
| atom | 305 | CG2 | THR | 41 | 10.089 | 27.066 | 9.870  | 1.00 | 49.21 |
| atom | 306 | C   | THR | 41 | 8.641  | 28.663 | 13.032 | 1.00 | 47.97 |
| atom | 307 | O   | THR | 41 | 8.184  | 28.107 | 14.021 | 1.00 | 47.57 |
| atom | 308 | N   | SER | 42 | 8.127  | 29.765 | 12.539 | 1.00 | 47.83 |
| atom | 309 | CA  | SER | 42 | 6.986  | 30.482 | 13.071 | 1.00 | 48.76 |
| atom | 310 | CB  | SER | 42 | 6.708  | 31.659 | 12.115 | 1.00 | 50.85 |
| atom | 311 | OG  | SER | 42 | 6.426  | 32.845 | 12.823 | 1.00 | 53.24 |
| atom | 312 | C   | SER | 42 | 5.723  | 29.645 | 13.189 | 1.00 | 48.46 |
| atom | 313 | O   | SER | 42 | 4.942  | 29.828 | 14.125 | 1.00 | 48.42 |
| atom | 314 | N   | ARG | 43 | 5.474  | 28.667 | 12.328 | 1.00 | 48.84 |
| atom | 315 | CA  | ARG | 43 | 4.312  | 27.807 | 12.327 | 1.00 | 48.83 |
| atom | 316 | CB  | ARG | 43 | 4.350  | 26.805 | 11.157 | 1.00 | 50.73 |
| atom | 317 | CG  | ARG | 43 | 4.431  | 27.467 | 9.791  | 1.00 | 54.10 |
| atom | 318 | CD  | ARG | 43 | 5.195  | 26.586 | 8.800  | 1.00 | 55.79 |
| atom | 319 | NE  | ARG | 43 | 6.033  | 27.464 | 7.979  | 1.00 | 58.33 |
| atom | 320 | CZ  | ARG | 43 | 7.260  | 27.187 | 7.564  | 1.00 | 59.19 |

| atom | 321 | NH1 | ARG | 43 | 7.805 | 26.025 | 7.901 | 1.00 | 60.73 |
|------|-----|-----|-----|----|-------|--------|-------|------|-------|
| atom | 322 | NH2 | ARG | 43 | 7.931 | 28.066 | 6.828 | 1.00 | 59.48 |
| atom | 323 | C | ARG | 43 | 4.104 | 27.007 | 13.602 | 1.00 | 48.20 |
| atom | 324 | O | ARG | 43 | 2.968 | 26.556 | 13.832 | 1.00 | 49.47 |
| atom | 325 | N | SER | 44 | 5.110 | 26.779 | 14.434 | 1.00 | 46.74 |
| atom | 326 | CA | SER | 44 | 4.890 | 26.045 | 15.672 | 1.00 | 45.71 |
| atom | 327 | CB | SER | 44 | 5.960 | 24.983 | 15.856 | 1.00 | 45.95 |
| atom | 328 | OG | SER | 44 | 7.231 | 25.490 | 16.180 | 1.00 | 47.64 |
| atom | 329 | C | SER | 44 | 4.809 | 26.980 | 16.871 | 1.00 | 45.68 |
| atom | 330 | O | SER | 44 | 4.743 | 26.565 | 18.038 | 1.00 | 45.69 |
| atom | 331 | N | ALA | 45 | 4.737 | 28.284 | 16.618 | 1.00 | 44.63 |
| atom | 332 | CA | ALA | 45 | 4.626 | 29.302 | 17.647 | 1.00 | 43.46 |
| atom | 333 | CB | ALA | 45 | 4.431 | 30.671 | 16.992 | 1.00 | 45.20 |
| atom | 334 | C | ALA | 45 | 3.484 | 29.026 | 18.605 | 1.00 | 42.81 |
| atom | 335 | O | ALA | 45 | 3.607 | 29.072 | 19.832 | 1.00 | 42.05 |
| atom | 336 | N | GLY | 46 | 2.316 | 28.703 | 18.035 | 1.00 | 42.83 |
| atom | 337 | CA | GLY | 46 | 1.136 | 28.394 | 18.830 | 1.00 | 41.71 |
| atom | 338 | C | GLY | 46 | 1.381 | 27.236 | 19.772 | 1.00 | 41.75 |
| atom | 339 | O | GLY | 46 | 0.927 | 27.218 | 20.921 | 1.00 | 42.40 |
| atom | 340 | N | LEU | 47 | 2.110 | 26.215 | 19.326 | 1.00 | 42.01 |
| atom | 341 | CA | LEU | 47 | 2.424 | 25.067 | 20.175 | 1.00 | 41.56 |
| atom | 342 | CB | LEU | 47 | 3.123 | 23.988 | 19.373 | 1.00 | 43.61 |
| atom | 343 | CG | LEU | 47 | 2.332 | 22.847 | 18.748 | 1.00 | 45.80 |
| atom | 344 | CD1 | LEU | 47 | 0.856 | 23.168 | 18.590 | 1.00 | 47.20 |
| atom | 345 | CD2 | LEU | 47 | 2.930 | 22.494 | 17.388 | 1.00 | 46.43 |
| atom | 346 | C | LEU | 47 | 3.281 | 25.488 | 21.357 | 1.00 | 41.27 |
| atom | 347 | O | LEU | 47 | 3.053 | 25.028 | 22.477 | 1.00 | 40.42 |
| atom | 348 | N | ARG | 48 | 4.232 | 26.389 | 21.104 | 1.00 | 41.50 |
| atom | 349 | CA | ARG | 48 | 5.112 | 26.899 | 22.149 | 1.00 | 41.92 |
| atom | 350 | CB | ARG | 48 | 6.274 | 27.674 | 21.523 | 1.00 | 44.19 |
| atom | 351 | CG | ARG | 48 | 7.347 | 28.133 | 22.505 | 1.00 | 44.56 |
| atom | 352 | CD | ARG | 48 | 8.174 | 26.939 | 22.966 | 1.00 | 45.68 |
| atom | 353 | NE | ARG | 48 | 8.853 | 27.215 | 24.210 | 1.00 | 47.69 |
| atom | 354 | CZ | ARG | 48 | 9.410 | 26.331 | 25.028 | 1.00 | 49.16 |
| atom | 355 | NH1 | ARG | 48 | 9.380 | 25.034 | 24.746 | 1.00 | 48.49 |
| atom | 356 | NH2 | ARG | 48 | 9.992 | 26.796 | 26.136 | 1.00 | 48.32 |

| atom | 357 | C | ARG | 48 | 4.343 | 27.771 | 23.131 | 1.00 | 42.13 |
|------|-----|-----|-----|-----|--------|--------|--------|------|-------|
| atom | 358 | 0 | ARG | 48 | 4.549 | 27.800 | 24.350 | 1.00 | 41.64 |
| atom | 359 | N | GLN | 49 | 3.364 | 28.505 | 22.599 | 1.00 | 42.92 |
| atom | 360 | CA | GLN | 49 | 2.523 | 29.374 | 23.418 | 1.00 | 43.03 |
| atom | 361 | CB | GLN | 49 | 1.561 | 30.183 | 22.550 | 1.00 | 43.25 |
| atom | 362 | CG | GLN | 49 | 2.244 | 31.328 | 21.818 | 1.00 | 44.34 |
| atom | 363 | CD | GLN | 49 | 1.242 | 32.087 | 20.969 | 1.00 | 45.10 |
| atom | 364 | OE1 | GLN | 49 | 0.585 | 33.009 | 21.441 | 1.00 | 44.29 |
| atom | 365 | NE2 | GLN | 49 | 1.118 | 31.697 | 19.705 | 1.00 | 46.35 |
| atom | 366 | C | GLN | 49 | 1.742 | 28.580 | 24.456 | 1.00 | 43.20 |
| atom | 367 | 0 | GLN | 49 | 1.676 | 28.974 | 25.629 | 1.00 | 41.94 |
| atom | 368 | N | LYS | 50 | 1.156 | 27.463 | 24.011 | 1.00 | 43.29 |
| atom | 369 | CA | LYS | 50 | 0.391 | 26.629 | 24.939 | 1.00 | 44.77 |
| atom | 370 | CB | LYS | 50 | -0.279 | 25.442 | 24.254 | 1.00 | 47.32 |
| atom | 371 | CG | LYS | 50 | -1.033 | 24.516 | 25.199 | 1.00 | 50.97 |
| atom | 372 | CD | LYS | 50 | -2.022 | 23.624 | 24.455 | 1.00 | 55.32 |
| atom | 373 | CE | LYS | 50 | -3.056 | 23.032 | 25.404 | 1.00 | 57.88 |
| atom | 374 | NZ | LYS | 50 | -4.351 | 22.748 | 24.715 | 1.00 | 59.50 |
| atom | 375 | C | LYS | 50 | 1.276 | 26.160 | 26.092 | 1.00 | 44.30 |
| atom | 376 | 0 | LYS | 50 | 0.921 | 26.325 | 27.258 | 1.00 | 43.80 |
| atom | 377 | N | LYS | 51 | 2.449 | 25.623 | 25.769 | 1.00 | 44.24 |
| atom | 378 | CA | LYS | 51 | 3.389 | 25.142 | 26.766 | 1.00 | 44.13 |
| atom | 379 | CB | LYS | 51 | 4.618 | 24.490 | 26.111 | 1.00 | 46.99 |
| atom | 380 | CG | LYS | 51 | 5.474 | 23.712 | 27.114 | 1.00 | 49.28 |
| atom | 381 | CD | LYS | 51 | 6.606 | 22.970 | 26.418 | 1.00 | 51.45 |
| atom | 382 | CE | LYS | 51 | 7.582 | 22.358 | 27.401 | 1.00 | 51.69 |
| atom | 383 | NZ | LYS | 51 | 8.976 | 22.274 | 26.875 | 1.00 | 52.67 |
| atom | 384 | C | LYS | 51 | 3.875 | 26.203 | 27.742 | 1.00 | 43.37 |
| atom | 385 | 0 | LYS | 51 | 3.964 | 25.892 | 28.934 | 1.00 | 42.33 |
| atom | 386 | N | VAL | 52 | 4.180 | 27.422 | 27.288 | 1.00 | 42.47 |
| atom | 387 | CA | VAL | 52 | 4.672 | 28.422 | 28.236 | 1.00 | 41.76 |
| atom | 388 | CB | VAL | 52 | 5.690 | 29.374 | 27.563 | 1.00 | 40.97 |
| atom | 389 | CG1 | VAL | 52 | 6.842 | 28.566 | 26.982 | 1.00 | 38.70 |
| atom | 390 | CG2 | VAL | 52 | 5.013 | 30.271 | 26.546 | 1.00 | 39.51 |
| atom | 391 | C | VAL | 52 | 3.632 | 29.267 | 28.953 | 1.00 | 40.69 |
| atom | 392 | 0 | VAL | 52 | 3.994 | 30.180 | 29.694 | 1.00 | 40.46 |

| atom | 393 | N | THR | 53 | 2.351 | 29.009 | 28.775 | 1.00 | 40.57 |
|------|-----|----|-----|----|-------|--------|--------|------|-------|
| atom | 394 | CA | THR | 53 | 1.281 | 29.788 | 29.365 | 1.00 | 39.05 |
| atom | 395 | CB | THR | 53 | 0.285 | 30.247 | 28.286 | 1.00 | 37.45 |
| atom | 396 | OG1 | THR | 53 | 0.986 | 31.005 | 27.294 | 1.00 | 36.00 |
| atom | 397 | CG2 | THR | 53 | -0.863 | 31.066 | 28.854 | 1.00 | 34.34 |
| atom | 398 | C | THR | 53 | 0.544 | 28.973 | 30.414 | 1.00 | 40.24 |
| atom | 399 | O | THR | 53 | -0.090 | 27.956 | 30.137 | 1.00 | 39.97 |
| atom | 400 | N | PHE | 54 | 0.668 | 29.421 | 31.661 | 1.00 | 40.35 |
| atom | 401 | CA | PHE | 54 | 0.028 | 28.730 | 32.772 | 1.00 | 40.53 |
| atom | 402 | CB | PHE | 54 | 0.741 | 27.419 | 33.082 | 1.00 | 39.02 |
| atom | 403 | CG | PHE | 54 | 2.216 | 27.589 | 33.348 | 1.00 | 37.12 |
| atom | 404 | CD1 | PHE | 54 | 2.685 | 27.847 | 34.617 | 1.00 | 36.56 |
| atom | 405 | CD2 | PHE | 54 | 3.123 | 27.462 | 32.308 | 1.00 | 36.96 |
| atom | 406 | CE1 | PHE | 54 | 4.034 | 28.017 | 34.858 | 1.00 | 37.14 |
| atom | 407 | CE2 | PHE | 54 | 4.482 | 27.620 | 32.524 | 1.00 | 37.52 |
| atom | 408 | CZ | PHE | 54 | 4.928 | 27.913 | 33.799 | 1.00 | 39.42 |
| atom | 409 | C | PHE | 54 | 0.027 | 29.644 | 33.993 | 1.00 | 41.16 |
| atom | 410 | O | PHE | 54 | 0.771 | 30.615 | 34.060 | 1.00 | 40.63 |
| atom | 411 | N | ASP | 55 | -0.829 | 29.317 | 34.946 | 1.00 | 42.21 |
| atom | 412 | CA | ASP | 55 | -0.924 | 30.114 | 36.162 | 1.00 | 43.50 |
| atom | 413 | CB | ASP | 55 | -2.286 | 29.867 | 36.806 | 1.00 | 45.47 |
| atom | 414 | CG | ASP | 55 | -2.538 | 30.897 | 37.886 | 1.00 | 48.38 |
| atom | 415 | OD1 | ASP | 55 | -2.462 | 30.536 | 39.073 | 1.00 | 50.47 |
| atom | 416 | OD2 | ASP | 55 | -2.794 | 32.058 | 37.521 | 1.00 | 52.46 |
| atom | 417 | C | ASP | 55 | 0.225 | 29.735 | 37.089 | 1.00 | 44.10 |
| atom | 418 | O | ASP | 55 | 0.533 | 28.541 | 37.199 | 1.00 | 44.96 |
| atom | 419 | N | ARG | 56 | 0.886 | 30.713 | 37.683 | 1.00 | 43.47 |
| atom | 420 | CA | ARG | 56 | 1.977 | 30.419 | 38.594 | 1.00 | 43.79 |
| atom | 421 | CB | ARG | 56 | 3.200 | 31.307 | 38.415 | 1.00 | 40.17 |
| atom | 422 | CG | ARG | 56 | 4.137 | 30.963 | 37.269 | 1.00 | 38.01 |
| atom | 423 | CD | ARG | 56 | 3.599 | 31.432 | 35.918 | 1.00 | 35.65 |
| atom | 424 | NE | ARG | 56 | 3.148 | 32.828 | 36.030 | 1.00 | 36.10 |
| atom | 425 | CZ | ARG | 56 | 3.947 | 33.893 | 36.057 | 1.00 | 34.55 |
| atom | 426 | NH1 | ARG | 56 | 5.264 | 33.729 | 35.958 | 1.00 | 31.83 |
| atom | 427 | NH2 | ARG | 56 | 3.419 | 35.108 | 36.182 | 1.00 | 33.37 |
| atom | 428 | C | ARG | 56 | 1.443 | 30.559 | 40.025 | 1.00 | 45.01 |

| atom | 429 | O | ARG | 56 | 0.811 | 31.541 | 40.411 | 1.00 | 45.26 |
|------|-----|-----|-----|----|-------|--------|--------|------|-------|
| atom | 430 | N | LEU | 57 | 1.716 | 29.537 | 40.819 | 1.00 | 45.63 |
| atom | 431 | CA | LEU | 57 | 1.332 | 29.560 | 42.231 | 1.00 | 46.04 |
| atom | 432 | CB | LEU | 57 | 0.370 | 28.435 | 42.561 | 1.00 | 48.09 |
| atom | 433 | CG | LEU | 57 | -0.926 | 28.822 | 43.270 | 1.00 | 48.40 |
| atom | 434 | CD1 | LEU | 57 | -1.702 | 29.835 | 42.446 | 1.00 | 47.65 |
| atom | 435 | CD2 | LEU | 57 | -1.763 | 27.573 | 43.528 | 1.00 | 48.93 |
| atom | 436 | C | LEU | 57 | 2.651 | 29.388 | 42.977 | 1.00 | 45.34 |
| atom | 437 | O | LEU | 57 | 3.413 | 28.495 | 42.582 | 1.00 | 47.21 |
| atom | 438 | N | GLN | 58 | 2.942 | 30.268 | 43.912 | 1.00 | 43.82 |
| atom | 439 | CA | GLN | 58 | 4.248 | 30.144 | 44.560 | 1.00 | 43.34 |
| atom | 440 | CB | GLN | 58 | 5.187 | 31.190 | 43.982 | 1.00 | 42.19 |
| atom | 441 | CG | GLN | 58 | 6.488 | 31.460 | 44.721 | 1.00 | 39.25 |
| atom | 442 | CD | GLN | 58 | 7.362 | 32.418 | 43.912 | 1.00 | 37.12 |
| atom | 443 | OE1 | GLN | 58 | 8.485 | 32.091 | 43.537 | 1.00 | 34.57 |
| atom | 444 | NE2 | GLN | 58 | 6.820 | 33.596 | 43.627 | 1.00 | 35.00 |
| atom | 445 | C | GLN | 58 | 4.061 | 30.244 | 46.059 | 1.00 | 43.03 |
| atom | 446 | O | GLN | 58 | 3.588 | 31.271 | 46.525 | 1.00 | 43.40 |
| atom | 447 | N | VAL | 59 | 4.377 | 29.151 | 46.743 | 1.00 | 41.98 |
| atom | 448 | CA | VAL | 59 | 4.274 | 29.101 | 48.183 | 1.00 | 42.02 |
| atom | 449 | CB | VAL | 59 | 3.437 | 27.903 | 48.677 | 1.00 | 42.96 |
| atom | 450 | CG1 | VAL | 59 | 3.303 | 27.942 | 50.195 | 1.00 | 41.64 |
| atom | 451 | CG2 | VAL | 59 | 2.080 | 27.902 | 47.985 | 1.00 | 38.98 |
| atom | 452 | C | VAL | 59 | 5.680 | 29.006 | 48.767 | 1.00 | 42.52 |
| atom | 453 | O | VAL | 59 | 6.373 | 28.021 | 48.533 | 1.00 | 41.65 |
| atom | 454 | N | LEU | 60 | 6.068 | 30.004 | 49.543 | 1.00 | 42.60 |
| atom | 455 | CA | LEU | 60 | 7.391 | 30.023 | 50.130 | 1.00 | 43.86 |
| atom | 456 | CB | LEU | 60 | 7.970 | 31.435 | 49.975 | 1.00 | 45.44 |
| atom | 457 | CG | LEU | 60 | 7.958 | 32.047 | 48.573 | 1.00 | 45.19 |
| atom | 458 | CD1 | LEU | 60 | 8.240 | 33.541 | 48.674 | 1.00 | 44.60 |
| atom | 459 | CD2 | LEU | 60 | 8.988 | 31.441 | 47.635 | 1.00 | 45.05 |
| atom | 460 | C | LEU | 60 | 7.436 | 29.616 | 51.592 | 1.00 | 44.33 |
| atom | 461 | O | LEU | 60 | 6.433 | 29.550 | 52.287 | 1.00 | 45.34 |
| atom | 462 | N | ASP | 61 | 8.647 | 29.329 | 52.061 | 1.00 | 44.64 |
| atom | 463 | CA | ASP | 61 | 8.856 | 28.889 | 53.436 | 1.00 | 44.80 |
| atom | 464 | CB | ASP | 61 | 9.066 | 27.376 | 53.544 | 1.00 | 45.10 |

| atom | 465 | CG  | ASP | 61 | 9.891  | 26.785 | 52.414 | 1.00 | 47.12 |
|------|-----|-----|-----|----|--------|--------|--------|------|-------|
| atom | 466 | OD1 | ASP | 61 | 9.279  | 25.969 | 51.681 | 1.00 | 49.15 |
| atom | 467 | OD2 | ASP | 61 | 11.086 | 27.118 | 52.266 | 1.00 | 43.75 |
| atom | 468 | C   | ASP | 61 | 10.077 | 29.571 | 54.025 | 1.00 | 44.62 |
| atom | 469 | O   | ASP | 61 | 10.710 | 30.383 | 53.368 | 1.00 | 44.15 |
| atom | 470 | N   | ASP | 62 | 10.418 | 29.205 | 55.255 | 1.00 | 44.61 |
| atom | 471 | CA  | ASP | 62 | 11.567 | 29.744 | 55.953 | 1.00 | 44.62 |
| atom | 472 | CB  | ASP | 62 | 11.575 | 29.238 | 57.403 | 1.00 | 48.07 |
| atom | 473 | CG  | ASP | 62 | 10.507 | 29.864 | 58.281 | 1.00 | 52.35 |
| atom | 474 | OD1 | ASP | 62 | 9.967  | 30.941 | 57.935 | 1.00 | 53.35 |
| atom | 475 | OD2 | ASP | 62 | 10.189 | 29.281 | 59.345 | 1.00 | 54.48 |
| atom | 476 | C   | ASP | 62 | 12.898 | 29.394 | 55.297 | 1.00 | 44.21 |
| atom | 477 | O   | ASP | 62 | 13.898 | 30.093 | 55.497 | 1.00 | 43.37 |
| atom | 478 | N   | HIS | 63 | 12.948 | 28.269 | 54.582 | 1.00 | 43.04 |
| atom | 479 | CA  | HIS | 63 | 14.163 | 27.816 | 53.926 | 1.00 | 42.55 |
| atom | 480 | CB  | HIS | 63 | 14.024 | 26.418 | 53.340 | 1.00 | 41.62 |
| atom | 481 | CG  | HIS | 63 | 14.144 | 25.314 | 54.345 | 1.00 | 42.36 |
| atom | 482 | CD2 | HIS | 63 | 13.461 | 24.143 | 54.473 | 1.00 | 41.51 |
| atom | 483 | ND1 | HIS | 63 | 15.065 | 25.329 | 55.372 | 1.00 | 41.21 |
| atom | 484 | CE1 | HIS | 63 | 14.956 | 24.226 | 56.085 | 1.00 | 39.97 |
| atom | 485 | NE2 | HIS | 63 | 13.988 | 23.488 | 55.568 | 1.00 | 40.36 |
| atom | 486 | C   | HIS | 63 | 14.547 | 28.830 | 52.840 | 1.00 | 41.85 |
| atom | 487 | O   | HIS | 63 | 15.687 | 29.286 | 52.775 | 1.00 | 40.79 |
| atom | 488 | N   | TYR | 64 | 13.553 | 29.190 | 52.040 | 1.00 | 40.61 |
| atom | 489 | CA  | TYR | 64 | 13.711 | 30.205 | 51.012 | 1.00 | 41.03 |
| atom | 490 | CB  | TYR | 64 | 12.373 | 30.445 | 50.344 | 1.00 | 41.17 |
| atom | 491 | CG  | TYR | 64 | 12.293 | 31.446 | 49.218 | 1.00 | 41.91 |
| atom | 492 | CD1 | TYR | 64 | 12.546 | 31.078 | 47.902 | 1.00 | 41.84 |
| atom | 493 | CE1 | TYR | 64 | 12.432 | 31.984 | 46.862 | 1.00 | 42.35 |
| atom | 494 | CD2 | TYR | 64 | 11.918 | 32.757 | 49.457 | 1.00 | 41.85 |
| atom | 495 | CE2 | TYR | 64 | 11.802 | 33.676 | 48.428 | 1.00 | 42.23 |
| atom | 496 | CZ  | TYR | 64 | 12.063 | 33.284 | 47.132 | 1.00 | 42.53 |
| atom | 497 | OH  | TYR | 64 | 11.965 | 34.216 | 46.133 | 1.00 | 41.63 |
| atom | 498 | C   | TYR | 64 | 14.222 | 31.492 | 51.674 | 1.00 | 40.73 |
| atom | 499 | O   | TYR | 64 | 15.320 | 31.973 | 51.423 | 1.00 | 38.94 |
| atom | 500 | N   | ARG | 65 | 13.433 | 31.982 | 52.625 | 1.00 | 40.55 |

| atom | 501 | CA | ARG | 65 | 13.727 | 33.172 | 53.393 | 1.00 | 40.28 |
|------|-----|-----|-----|----|--------|--------|--------|------|-------|
| atom | 502 | CB | ARG | 65 | 12.651 | 33.378 | 54.476 | 1.00 | 38.44 |
| atom | 503 | CG | ARG | 65 | 11.305 | 33.752 | 53.853 | 1.00 | 38.51 |
| atom | 504 | CD | ARG | 65 | 10.210 | 33.633 | 54.908 | 1.00 | 39.11 |
| atom | 505 | NE | ARG | 65 | 8.908 | 33.501 | 54.267 | 1.00 | 38.37 |
| atom | 506 | CZ | ARG | 65 | 7.999 | 32.601 | 54.625 | 1.00 | 37.39 |
| atom | 507 | NH1 | ARG | 65 | 8.270 | 31.746 | 55.603 | 1.00 | 36.19 |
| atom | 508 | NH2 | ARG | 65 | 6.831 | 32.542 | 54.001 | 1.00 | 36.67 |
| atom | 509 | C | ARG | 65 | 15.110 | 33.179 | 54.017 | 1.00 | 40.14 |
| atom | 510 | O | ARG | 65 | 15.751 | 34.238 | 53.963 | 1.00 | 39.16 |
| atom | 511 | N | ASP | 66 | 15.564 | 32.075 | 54.599 | 1.00 | 40.51 |
| atom | 512 | CA | ASP | 66 | 16.892 | 32.061 | 55.203 | 1.00 | 41.32 |
| atom | 513 | CB | ASP | 66 | 17.135 | 30.838 | 56.077 | 1.00 | 44.20 |
| atom | 514 | CG | ASP | 66 | 16.252 | 30.712 | 57.293 | 1.00 | 45.90 |
| atom | 515 | OD1 | ASP | 66 | 15.661 | 31.724 | 57.719 | 1.00 | 47.48 |
| atom | 516 | OD2 | ASP | 66 | 16.157 | 29.573 | 57.806 | 1.00 | 47.90 |
| atom | 517 | C | ASP | 66 | 17.982 | 32.115 | 54.130 | 1.00 | 40.88 |
| atom | 518 | O | ASP | 66 | 19.035 | 32.702 | 54.361 | 1.00 | 40.92 |
| atom | 519 | N | VAL | 67 | 17.750 | 31.516 | 52.961 | 1.00 | 39.84 |
| atom | 520 | CA | VAL | 67 | 18.729 | 31.536 | 51.882 | 1.00 | 38.05 |
| atom | 521 | CB | VAL | 67 | 18.414 | 30.468 | 50.826 | 1.00 | 37.62 |
| atom | 522 | CG1 | VAL | 67 | 19.327 | 30.630 | 49.612 | 1.00 | 37.66 |
| atom | 523 | CG2 | VAL | 67 | 18.496 | 29.060 | 51.388 | 1.00 | 33.64 |
| atom | 524 | C | VAL | 67 | 18.768 | 32.926 | 51.241 | 1.00 | 37.95 |
| atom | 525 | O | VAL | 67 | 19.834 | 33.459 | 50.909 | 1.00 | 36.06 |
| atom | 526 | N | LEU | 68 | 17.596 | 33.552 | 51.148 | 1.00 | 37.45 |
| atom | 527 | CA | LEU | 68 | 17.463 | 34.886 | 50.591 | 1.00 | 38.57 |
| atom | 528 | CB | LEU | 68 | 16.009 | 35.312 | 50.459 | 1.00 | 37.68 |
| atom | 529 | CG | LEU | 68 | 15.562 | 36.269 | 49.361 | 1.00 | 38.19 |
| atom | 530 | CD1 | LEU | 68 | 14.223 | 36.915 | 49.722 | 1.00 | 36.22 |
| atom | 531 | CD2 | LEU | 68 | 16.573 | 37.351 | 49.033 | 1.00 | 39.57 |
| atom | 532 | C | LEU | 68 | 18.176 | 35.899 | 51.487 | 1.00 | 39.41 |
| atom | 533 | O | LEU | 68 | 18.879 | 36.792 | 51.019 | 1.00 | 38.99 |
| atom | 534 | N | LYS | 69 | 18.038 | 35.727 | 52.801 | 1.00 | 39.99 |
| atom | 535 | CA | LYS | 69 | 18.680 | 36.619 | 53.765 | 1.00 | 40.29 |
| atom | 536 | CB | LYS | 69 | 18.242 | 36.283 | 55.193 | 1.00 | 44.44 |

| atom | 537 | CG | LYS | 69 | 18.492 | 37.403 | 56.187 | 1.00 | 46.87 |
| atom | 538 | CD | LYS | 69 | 18.654 | 36.869 | 57.603 | 1.00 | 50.33 |
| atom | 539 | CE | LYS | 69 | 18.959 | 38.000 | 58.583 | 1.00 | 52.00 |
| atom | 540 | NZ | LYS | 69 | 19.503 | 37.478 | 59.869 | 1.00 | 51.70 |
| atom | 541 | C | LYS | 69 | 20.196 | 36.507 | 53.623 | 1.00 | 39.36 |
| atom | 542 | 0 | LYS | 69 | 20.955 | 37.482 | 53.649 | 1.00 | 37.80 |
| atom | 543 | N | GLU | 70 | 20.654 | 35.264 | 53.437 | 1.00 | 38.54 |
| atom | 544 | CA | GLU | 70 | 22.088 | 35.043 | 53.229 | 1.00 | 37.60 |
| atom | 545 | CB | GLU | 70 | 22.394 | 33.556 | 53.273 | 1.00 | 37.89 |
| atom | 546 | CG | GLU | 70 | 22.067 | 32.854 | 54.577 | 1.00 | 40.36 |
| atom | 547 | CD | GLU | 70 | 22.090 | 31.341 | 54.415 | 1.00 | 43.66 |
| atom | 548 | OE1 | GLU | 70 | 22.539 | 30.874 | 53.336 | 1.00 | 43.89 |
| atom | 549 | OE2 | GLU | 70 | 21.662 | 30.622 | 55.350 | 1.00 | 44.51 |
| atom | 550 | C | GLU | 70 | 22.571 | 35.674 | 51.926 | 1.00 | 36.36 |
| atom | 551 | 0 | GLU | 70 | 23.690 | 36.190 | 51.909 | 1.00 | 36.24 |
| atom | 552 | N | MET | 71 | 21.775 | 35.667 | 50.864 | 1.00 | 35.24 |
| atom | 553 | CA | MET | 71 | 22.157 | 36.253 | 49.583 | 1.00 | 35.02 |
| atom | 554 | CB | MET | 71 | 21.215 | 35.808 | 48.460 | 1.00 | 31.17 |
| atom | 555 | CG | MET | 71 | 21.230 | 34.304 | 48.229 | 1.00 | 27.39 |
| atom | 556 | SD | MET | 71 | 19.954 | 33.721 | 47.123 | 1.00 | 28.75 |
| atom | 557 | CE | MET | 71 | 20.529 | 34.376 | 45.543 | 1.00 | 25.70 |
| atom | 558 | C | MET | 71 | 22.228 | 37.774 | 49.637 | 1.00 | 35.53 |
| atom | 559 | 0 | MET | 71 | 23.190 | 38.364 | 49.118 | 1.00 | 36.06 |
| atom | 560 | N | LYS | 72 | 21.282 | 38.400 | 50.335 | 1.00 | 35.34 |
| atom | 561 | CA | LYS | 72 | 21.287 | 39.850 | 50.480 | 1.00 | 35.57 |
| atom | 562 | CB | LYS | 72 | 19.926 | 40.327 | 50.982 | 1.00 | 35.91 |
| atom | 563 | CG | LYS | 72 | 18.845 | 40.008 | 49.952 | 1.00 | 38.77 |
| atom | 564 | CD | LYS | 72 | 17.684 | 40.961 | 49.925 | 1.00 | 39.60 |
| atom | 565 | CE | LYS | 72 | 16.566 | 40.684 | 50.896 | 1.00 | 41.24 |
| atom | 566 | NZ | LYS | 72 | 15.271 | 41.256 | 50.404 | 1.00 | 41.43 |
| atom | 567 | C | LYS | 72 | 22.452 | 40.333 | 51.325 | 1.00 | 35.53 |
| atom | 568 | 0 | LYS | 72 | 23.041 | 41.388 | 51.061 | 1.00 | 35.89 |
| atom | 569 | N | ALA | 73 | 22.887 | 39.548 | 52.307 | 1.00 | 35.15 |
| atom | 570 | CA | ALA | 73 | 24.029 | 39.893 | 53.139 | 1.00 | 34.59 |
| atom | 571 | CB | ALA | 73 | 24.129 | 38.997 | 54.353 | 1.00 | 32.33 |
| atom | 572 | C | ALA | 73 | 25.314 | 39.857 | 52.308 | 1.00 | 35.26 |

| atom | 573 | O   | ALA | 73 | 26.144 | 40.761 | 52.448 | 1.00 | 34.70 |
| atom | 574 | N   | LYS | 74 | 25.450 | 38.888 | 51.398 | 1.00 | 35.03 |
| atom | 575 | CA  | LYS | 74 | 26.622 | 38.885 | 50.533 | 1.00 | 36.44 |
| atom | 576 | CB  | LYS | 74 | 26.820 | 37.552 | 49.818 | 1.00 | 40.17 |
| atom | 577 | CG  | LYS | 74 | 26.601 | 36.331 | 50.689 | 1.00 | 42.89 |
| atom | 578 | CD  | LYS | 74 | 27.439 | 35.132 | 50.258 | 1.00 | 45.10 |
| atom | 579 | CE  | LYS | 74 | 26.789 | 33.818 | 50.693 | 1.00 | 46.50 |
| atom | 580 | NZ  | LYS | 74 | 25.392 | 33.634 | 50.179 | 1.00 | 44.34 |
| atom | 581 | C   | LYS | 74 | 26.535 | 40.025 | 49.514 | 1.00 | 36.36 |
| atom | 582 | O   | LYS | 74 | 27.524 | 40.695 | 49.197 | 1.00 | 35.71 |
| atom | 583 | N   | ALA | 75 | 25.314 | 40.284 | 49.033 | 1.00 | 35.85 |
| atom | 584 | CA  | ALA | 75 | 25.084 | 41.332 | 48.049 | 1.00 | 35.98 |
| atom | 585 | CB  | ALA | 75 | 23.638 | 41.339 | 47.578 | 1.00 | 30.21 |
| atom | 586 | C   | ALA | 75 | 25.504 | 42.678 | 48.602 | 1.00 | 36.82 |
| atom | 587 | O   | ALA | 75 | 26.116 | 43.503 | 47.918 | 1.00 | 37.93 |
| atom | 588 | N   | SER | 76 | 25.272 | 42.924 | 49.888 | 1.00 | 37.57 |
| atom | 589 | CA  | SER | 76 | 25.602 | 44.134 | 50.605 | 1.00 | 37.59 |
| atom | 590 | CB  | SER | 76 | 24.977 | 44.114 | 52.010 | 1.00 | 37.62 |
| atom | 591 | OG  | SER | 76 | 23.552 | 44.121 | 51.919 | 1.00 | 40.32 |
| atom | 592 | C   | SER | 76 | 27.084 | 44.445 | 50.708 | 1.00 | 37.58 |
| atom | 593 | O   | SER | 76 | 27.446 | 45.555 | 51.136 | 1.00 | 39.39 |
| atom | 594 | N   | THR | 77 | 27.965 | 43.567 | 50.288 | 1.00 | 36.91 |
| atom | 595 | CA  | THR | 77 | 29.406 | 43.791 | 50.257 | 1.00 | 36.16 |
| atom | 596 | CB  | THR | 77 | 30.193 | 42.504 | 50.599 | 1.00 | 33.95 |
| atom | 597 | OG1 | THR | 77 | 30.040 | 41.601 | 49.495 | 1.00 | 35.78 |
| atom | 598 | CG2 | THR | 77 | 29.625 | 41.864 | 51.862 | 1.00 | 32.60 |
| atom | 599 | C   | THR | 77 | 29.841 | 44.217 | 48.858 | 1.00 | 35.36 |
| atom | 600 | O   | THR | 77 | 30.974 | 44.657 | 48.651 | 1.00 | 35.65 |
| atom | 601 | N   | VAL | 78 | 28.931 | 44.110 | 47.890 | 1.00 | 34.69 |
| atom | 602 | CA  | VAL | 78 | 29.254 | 44.451 | 46.499 | 1.00 | 33.38 |
| atom | 603 | CB  | VAL | 78 | 28.442 | 43.546 | 45.556 | 1.00 | 30.05 |
| atom | 604 | CG1 | VAL | 78 | 28.744 | 43.788 | 44.101 | 1.00 | 27.71 |
| atom | 605 | CG2 | VAL | 78 | 28.716 | 42.086 | 45.935 | 1.00 | 30.64 |
| atom | 606 | C   | VAL | 78 | 29.070 | 45.919 | 46.151 | 1.00 | 33.45 |
| atom | 607 | O   | VAL | 78 | 28.065 | 46.552 | 46.493 | 1.00 | 33.19 |
| atom | 608 | N   | LYS | 79 | 30.084 | 46.465 | 45.453 | 1.00 | 32.83 |

| atom | 609 | CA  | LYS | 79 | 30.019 | 47.870 | 45.037 | 1.00 | 32.01 |
|------|-----|-----|-----|----|--------|--------|--------|------|-------|
| atom | 610 | CB  | LYS | 79 | 31.137 | 48.731 | 45.608 | 1.00 | 29.97 |
| atom | 611 | CG  | LYS | 79 | 30.777 | 50.201 | 45.532 | 1.00 | 33.49 |
| atom | 612 | CD  | LYS | 79 | 31.912 | 51.115 | 45.141 | 1.00 | 34.72 |
| atom | 613 | CE  | LYS | 79 | 31.432 | 52.568 | 45.144 | 1.00 | 37.97 |
| atom | 614 | NZ  | LYS | 79 | 31.200 | 52.956 | 46.577 | 1.00 | 42.83 |
| atom | 615 | C   | LYS | 79 | 30.047 | 47.890 | 43.514 | 1.00 | 31.34 |
| atom | 616 | O   | LYS | 79 | 30.784 | 47.069 | 42.962 | 1.00 | 32.09 |
| atom | 617 | N   | ALA | 80 | 29.209 | 48.700 | 42.862 | 1.00 | 30.61 |
| atom | 618 | CA  | ALA | 80 | 29.157 | 48.630 | 41.397 | 1.00 | 29.02 |
| atom | 619 | CB  | ALA | 80 | 28.098 | 47.645 | 40.945 | 1.00 | 27.11 |
| atom | 620 | C   | ALA | 80 | 28.927 | 50.026 | 40.855 | 1.00 | 29.80 |
| atom | 621 | O   | ALA | 80 | 28.348 | 50.845 | 41.556 | 1.00 | 29.38 |
| atom | 622 | N   | LYS | 81 | 29.433 | 50.284 | 39.661 | 1.00 | 30.05 |
| atom | 623 | CA  | LYS | 81 | 29.341 | 51.600 | 39.061 | 1.00 | 30.27 |
| atom | 624 | CB  | LYS | 81 | 30.756 | 52.090 | 38.681 | 1.00 | 31.87 |
| atom | 625 | CG  | LYS | 81 | 31.374 | 51.339 | 37.519 | 1.00 | 37.86 |
| atom | 626 | CD  | LYS | 81 | 32.467 | 52.105 | 36.789 | 1.00 | 42.99 |
| atom | 627 | CE  | LYS | 81 | 32.751 | 51.492 | 35.417 | 1.00 | 47.33 |
| atom | 628 | NZ  | LYS | 81 | 33.666 | 52.336 | 34.571 | 1.00 | 49.16 |
| atom | 629 | C   | LYS | 81 | 28.492 | 51.632 | 37.792 | 1.00 | 29.77 |
| atom | 630 | O   | LYS | 81 | 28.247 | 50.643 | 37.111 | 1.00 | 28.82 |
| atom | 631 | N   | LEU | 82 | 28.104 | 52.852 | 37.425 | 1.00 | 29.31 |
| atom | 632 | CA  | LEU | 82 | 27.374 | 53.069 | 36.199 | 1.00 | 29.67 |
| atom | 633 | CB  | LEU | 82 | 26.804 | 54.492 | 36.160 | 1.00 | 29.45 |
| atom | 634 | CG  | LEU | 82 | 25.541 | 54.704 | 36.991 | 1.00 | 31.17 |
| atom | 635 | CD1 | LEU | 82 | 25.400 | 56.191 | 37.288 | 1.00 | 30.63 |
| atom | 636 | CD2 | LEU | 82 | 24.338 | 54.144 | 36.248 | 1.00 | 27.47 |
| atom | 637 | C   | LEU | 82 | 28.324 | 52.973 | 35.004 | 1.00 | 29.02 |
| atom | 638 | O   | LEU | 82 | 29.481 | 53.380 | 35.168 | 1.00 | 30.17 |
| atom | 639 | N   | LEU | 83 | 27.828 | 52.483 | 33.871 | 1.00 | 27.76 |
| atom | 640 | CA  | LEU | 83 | 28.676 | 52.574 | 32.674 | 1.00 | 26.06 |
| atom | 641 | CB  | LEU | 83 | 28.653 | 51.355 | 31.787 | 1.00 | 25.12 |
| atom | 642 | CG  | LEU | 83 | 29.775 | 50.336 | 32.017 | 1.00 | 27.00 |
| atom | 643 | CD1 | LEU | 83 | 29.738 | 49.792 | 33.433 | 1.00 | 24.66 |
| atom | 644 | CD2 | LEU | 83 | 29.699 | 49.174 | 31.026 | 1.00 | 25.27 |

| atom | 645 | C | LEU | 83 | 28.200 | 53.823 | 31.914 | 1.00 | 25.12 |
|------|-----|-----|-----|-----|--------|--------|--------|------|-------|
| atom | 646 | O | LEU | 83 | 27.000 | 54.156 | 31.940 | 1.00 | 23.92 |
| atom | 647 | N | SER | 84 | 29.137 | 54.506 | 31.270 | 1.00 | 23.18 |
| atom | 648 | CA | SER | 84 | 28.690 | 55.674 | 30.511 | 1.00 | 24.40 |
| atom | 649 | CB | SER | 84 | 29.912 | 56.507 | 30.106 | 1.00 | 21.23 |
| atom | 650 | OG | SER | 84 | 30.753 | 55.636 | 29.360 | 1.00 | 26.21 |
| atom | 651 | C | SER | 84 | 27.976 | 55.183 | 29.254 | 1.00 | 24.25 |
| atom | 652 | O | SER | 84 | 28.195 | 54.037 | 28.834 | 1.00 | 24.11 |
| atom | 653 | N | VAL | 85 | 27.278 | 56.071 | 28.561 | 1.00 | 24.35 |
| atom | 654 | CA | VAL | 85 | 26.693 | 55.755 | 27.264 | 1.00 | 24.12 |
| atom | 655 | CB | VAL | 85 | 26.167 | 57.028 | 26.575 | 1.00 | 23.28 |
| atom | 656 | CG1 | VAL | 85 | 25.927 | 56.800 | 25.077 | 1.00 | 17.75 |
| atom | 657 | CG2 | VAL | 85 | 24.920 | 57.608 | 27.255 | 1.00 | 20.76 |
| atom | 658 | C | VAL | 85 | 27.786 | 55.152 | 26.364 | 1.00 | 26.06 |
| atom | 659 | O | VAL | 85 | 27.630 | 54.092 | 25.734 | 1.00 | 25.64 |
| atom | 660 | N | GLU | 86 | 28.944 | 55.826 | 26.316 | 1.00 | 26.18 |
| atom | 661 | CA | GLU | 86 | 30.038 | 55.358 | 25.462 | 1.00 | 27.19 |
| atom | 662 | CB | GLU | 86 | 31.186 | 56.376 | 25.451 | 1.00 | 27.26 |
| atom | 663 | CG | GLU | 86 | 30.791 | 57.667 | 24.728 | 1.00 | 29.83 |
| atom | 664 | CD | GLU | 86 | 30.198 | 58.734 | 25.606 | 1.00 | 28.64 |
| atom | 665 | OE1 | GLU | 86 | 29.920 | 59.868 | 25.161 | 1.00 | 30.79 |
| atom | 666 | OE2 | GLU | 86 | 30.020 | 58.504 | 26.814 | 1.00 | 28.74 |
| atom | 667 | C | GLU | 86 | 30.556 | 53.973 | 25.799 | 1.00 | 27.11 |
| atom | 668 | O | GLU | 86 | 30.782 | 53.190 | 24.868 | 1.00 | 28.14 |
| atom | 669 | N | GLU | 87 | 30.715 | 53.627 | 27.069 | 1.00 | 26.46 |
| atom | 670 | CA | GLU | 87 | 31.212 | 52.309 | 27.424 | 1.00 | 26.29 |
| atom | 671 | CB | GLU | 87 | 31.590 | 52.207 | 28.905 | 1.00 | 24.60 |
| atom | 672 | CG | GLU | 87 | 32.903 | 52.903 | 29.252 | 1.00 | 21.73 |
| atom | 673 | CD | GLU | 87 | 32.969 | 53.165 | 30.743 | 1.00 | 22.51 |
| atom | 674 | OE1 | GLU | 87 | 31.933 | 53.458 | 31.376 | 1.00 | 25.19 |
| atom | 675 | OE2 | GLU | 87 | 34.036 | 53.095 | 31.352 | 1.00 | 22.51 |
| atom | 676 | C | GLU | 87 | 30.187 | 51.238 | 27.068 | 1.00 | 26.24 |
| atom | 677 | O | GLU | 87 | 30.609 | 50.207 | 26.557 | 1.00 | 26.33 |
| atom | 678 | N | ALA | 88 | 28.912 | 51.509 | 27.329 | 1.00 | 25.60 |
| atom | 679 | CA | ALA | 88 | 27.830 | 50.592 | 27.006 | 1.00 | 25.57 |
| atom | 680 | CB | ALA | 88 | 26.513 | 51.155 | 27.531 | 1.00 | 22.54 |

| atom | 681 | C   | ALA | 88 | 27.743 | 50.354 | 25.493 | 1.00 | 26.44 |
|------|-----|-----|-----|----|--------|--------|--------|------|-------|
| atom | 682 | O   | ALA | 88 | 27.465 | 49.228 | 25.058 | 1.00 | 25.04 |
| atom | 683 | N   | CYS | 89 | 27.950 | 51.428 | 24.716 | 1.00 | 25.77 |
| atom | 684 | CA  | CYS | 89 | 27.982 | 51.319 | 23.266 | 1.00 | 26.77 |
| atom | 685 | CB  | CYS | 89 | 28.122 | 52.667 | 22.552 | 1.00 | 25.38 |
| atom | 686 | SG  | CYS | 89 | 26.685 | 53.789 | 22.602 | 1.00 | 19.63 |
| atom | 687 | C   | CYS | 89 | 29.114 | 50.388 | 22.829 | 1.00 | 27.53 |
| atom | 688 | O   | CYS | 89 | 28.855 | 49.422 | 22.111 | 1.00 | 26.89 |
| atom | 689 | N   | LYS | 90 | 30.344 | 50.570 | 23.315 | 1.00 | 28.08 |
| atom | 690 | CA  | LYS | 90 | 31.486 | 49.730 | 22.974 | 1.00 | 27.26 |
| atom | 691 | CB  | LYS | 90 | 32.764 | 50.306 | 23.599 | 1.00 | 26.77 |
| atom | 692 | CG  | LYS | 90 | 33.430 | 51.485 | 22.932 | 1.00 | 28.77 |
| atom | 693 | CD  | LYS | 90 | 34.004 | 52.474 | 23.926 | 1.00 | 28.79 |
| atom | 694 | CE  | LYS | 90 | 34.918 | 53.546 | 23.360 | 1.00 | 28.74 |
| atom | 695 | NZ  | LYS | 90 | 34.777 | 53.715 | 21.886 | 1.00 | 32.57 |
| atom | 696 | C   | LYS | 90 | 31.318 | 48.282 | 23.417 | 1.00 | 28.19 |
| atom | 697 | O   | LYS | 90 | 31.882 | 47.334 | 22.864 | 1.00 | 27.49 |
| atom | 698 | N   | LEU | 91 | 30.570 | 48.042 | 24.494 | 1.00 | 28.75 |
| atom | 699 | CA  | LEU | 91 | 30.244 | 46.706 | 24.961 | 1.00 | 30.16 |
| atom | 700 | CB  | LEU | 91 | 30.086 | 46.655 | 26.470 | 1.00 | 34.90 |
| atom | 701 | CG  | LEU | 91 | 31.290 | 46.352 | 27.359 | 1.00 | 36.27 |
| atom | 702 | CD1 | LEU | 91 | 32.575 | 46.904 | 26.792 | 1.00 | 35.39 |
| atom | 703 | CD2 | LEU | 91 | 31.063 | 46.937 | 28.755 | 1.00 | 36.52 |
| atom | 704 | C   | LEU | 91 | 28.989 | 46.160 | 24.284 | 1.00 | 29.92 |
| atom | 705 | O   | LEU | 91 | 28.521 | 45.087 | 24.656 | 1.00 | 30.50 |
| atom | 706 | N   | THR | 92 | 28.455 | 46.823 | 23.275 | 1.00 | 30.07 |
| atom | 707 | CA  | THR | 92 | 27.300 | 46.330 | 22.542 | 1.00 | 32.39 |
| atom | 708 | CB  | THR | 92 | 26.568 | 47.507 | 21.854 | 1.00 | 31.77 |
| atom | 709 | OG1 | THR | 92 | 25.884 | 48.248 | 22.877 | 1.00 | 28.62 |
| atom | 710 | CG2 | THR | 92 | 25.627 | 47.052 | 20.754 | 1.00 | 28.77 |
| atom | 711 | C   | THR | 92 | 27.730 | 45.309 | 21.490 | 1.00 | 33.38 |
| atom | 712 | O   | THR | 92 | 28.496 | 45.634 | 20.597 | 1.00 | 33.88 |
| atom | 713 | N   | PRO | 93 | 27.199 | 44.102 | 21.543 | 1.00 | 34.88 |
| atom | 714 | CD  | PRO | 93 | 26.225 | 43.644 | 22.568 | 1.00 | 35.43 |
| atom | 715 | CA  | PRO | 93 | 27.507 | 43.061 | 20.588 | 1.00 | 36.36 |
| atom | 716 | CB  | PRO | 93 | 26.692 | 41.858 | 21.025 | 1.00 | 36.29 |

| atom | 717 | CG  | PRO | 93 | 26.181 | 42.160 | 22.383 | 1.00 | 35.81 |
|------|-----|-----|-----|----|--------|--------|--------|------|-------|
| atom | 718 | C   | PRO | 93 | 27.197 | 43.491 | 19.162 | 1.00 | 37.78 |
| atom | 719 | O   | PRO | 93 | 26.209 | 44.152 | 18.874 | 1.00 | 36.97 |
| atom | 720 | N   | PRO | 94 | 28.064 | 43.091 | 18.239 | 1.00 | 39.47 |
| atom | 721 | CD  | PRO | 94 | 29.298 | 42.295 | 18.533 | 1.00 | 39.82 |
| atom | 722 | CA  | PRO | 94 | 27.992 | 43.416 | 16.842 | 1.00 | 40.50 |
| atom | 723 | CB  | PRO | 94 | 29.272 | 42.830 | 16.226 | 1.00 | 39.92 |
| atom | 724 | CG  | PRO | 94 | 29.594 | 41.708 | 17.168 | 1.00 | 40.67 |
| atom | 725 | C   | PRO | 94 | 26.807 | 42.904 | 16.066 | 1.00 | 41.64 |
| atom | 726 | O   | PRO | 94 | 26.539 | 43.528 | 15.017 | 1.00 | 43.65 |
| atom | 727 | N   | HIS | 95 | 26.119 | 41.824 | 16.406 | 1.00 | 41.97 |
| atom | 728 | CA  | HIS | 95 | 24.987 | 41.464 | 15.531 | 1.00 | 43.47 |
| atom | 729 | CB  | HIS | 95 | 25.139 | 40.045 | 14.971 | 1.00 | 45.58 |
| atom | 730 | CG  | HIS | 95 | 26.434 | 39.875 | 14.225 | 1.00 | 48.05 |
| atom | 731 | CD2 | HIS | 95 | 27.510 | 39.076 | 14.434 | 1.00 | 47.72 |
| atom | 732 | ND1 | HIS | 95 | 26.729 | 40.658 | 13.125 | 1.00 | 49.11 |
| atom | 733 | CE1 | HIS | 95 | 27.927 | 40.328 | 12.665 | 1.00 | 48.45 |
| atom | 734 | NE2 | HIS | 95 | 28.409 | 39.373 | 13.442 | 1.00 | 49.37 |
| atom | 735 | C   | HIS | 95 | 23.626 | 41.688 | 16.181 | 1.00 | 43.10 |
| atom | 736 | O   | HIS | 95 | 22.665 | 40.965 | 15.898 | 1.00 | 43.67 |
| atom | 737 | N   | SER | 96 | 23.495 | 42.726 | 16.993 | 1.00 | 41.70 |
| atom | 738 | CA  | SER | 96 | 22.260 | 43.075 | 17.667 | 1.00 | 39.84 |
| atom | 739 | CB  | SER | 96 | 22.575 | 44.220 | 18.645 | 1.00 | 39.09 |
| atom | 740 | OG  | SER | 96 | 22.614 | 43.786 | 19.984 | 1.00 | 37.46 |
| atom | 741 | C   | SER | 96 | 21.170 | 43.550 | 16.721 | 1.00 | 38.69 |
| atom | 742 | O   | SER | 96 | 21.438 | 44.322 | 15.796 | 1.00 | 39.13 |
| atom | 743 | N   | ALA | 97 | 19.931 | 43.173 | 16.992 | 1.00 | 37.32 |
| atom | 744 | CA  | ALA | 97 | 18.808 | 43.657 | 16.186 | 1.00 | 36.47 |
| atom | 745 | CB  | ALA | 97 | 17.505 | 43.147 | 16.794 | 1.00 | 33.90 |
| atom | 746 | C   | ALA | 97 | 18.814 | 45.186 | 16.145 | 1.00 | 36.20 |
| atom | 747 | O   | ALA | 97 | 18.901 | 45.833 | 17.198 | 1.00 | 35.49 |
| atom | 748 | N   | LYS | 98 | 18.754 | 45.767 | 14.964 | 1.00 | 36.09 |
| atom | 749 | CA  | LYS | 98 | 18.775 | 47.213 | 14.812 | 1.00 | 37.34 |
| atom | 750 | CB  | LYS | 98 | 18.741 | 47.613 | 13.346 | 1.00 | 38.64 |
| atom | 751 | CG  | LYS | 98 | 17.368 | 47.680 | 12.716 | 1.00 | 40.70 |
| atom | 752 | CD  | LYS | 98 | 17.485 | 47.339 | 11.233 | 1.00 | 45.63 |

| atom | 753 | CE  | LYS | 98  | 16.352 | 48.015 | 10.462 | 1.00 | 48.60 |
|------|-----|-----|-----|-----|--------|--------|--------|------|-------|
| atom | 754 | NZ  | LYS | 98  | 16.610 | 49.489 | 10.371 | 1.00 | 52.60 |
| atom | 755 | C   | LYS | 98  | 17.606 | 47.853 | 15.565 | 1.00 | 38.41 |
| atom | 756 | O   | LYS | 98  | 16.598 | 47.227 | 15.882 | 1.00 | 38.17 |
| atom | 757 | N   | SER | 99  | 17.770 | 49.146 | 15.867 | 1.00 | 38.38 |
| atom | 758 | CA  | SER | 99  | 16.777 | 49.921 | 16.557 | 1.00 | 37.73 |
| atom | 759 | CB  | SER | 99  | 17.383 | 51.242 | 17.036 | 1.00 | 38.40 |
| atom | 760 | OG  | SER | 99  | 16.444 | 52.082 | 17.719 | 1.00 | 37.78 |
| atom | 761 | C   | SER | 99  | 15.600 | 50.236 | 15.635 | 1.00 | 38.93 |
| atom | 762 | O   | SER | 99  | 15.691 | 50.376 | 14.411 | 1.00 | 38.97 |
| atom | 763 | N   | LYS | 100 | 14.462 | 50.484 | 16.295 | 1.00 | 38.75 |
| atom | 764 | CA  | LYS | 100 | 13.277 | 50.942 | 15.578 | 1.00 | 38.63 |
| atom | 765 | CB  | LYS | 100 | 11.980 | 50.645 | 16.304 | 1.00 | 41.47 |
| atom | 766 | CG  | LYS | 100 | 11.414 | 49.244 | 16.149 | 1.00 | 46.95 |
| atom | 767 | CD  | LYS | 100 | 10.441 | 48.906 | 17.284 | 1.00 | 50.39 |
| atom | 768 | CE  | LYS | 100 | 9.617  | 47.668 | 16.985 | 1.00 | 52.31 |
| atom | 769 | NZ  | LYS | 100 | 8.779  | 47.226 | 18.144 | 1.00 | 54.91 |
| atom | 770 | C   | LYS | 100 | 13.463 | 52.446 | 15.377 | 1.00 | 37.76 |
| atom | 771 | O   | LYS | 100 | 12.744 | 53.012 | 14.570 | 1.00 | 37.49 |
| atom | 772 | N   | PHE | 101 | 14.430 | 53.104 | 16.009 | 1.00 | 36.57 |
| atom | 773 | CA  | PHE | 101 | 14.616 | 54.547 | 15.904 | 1.00 | 35.11 |
| atom | 774 | CB  | PHE | 101 | 14.746 | 55.167 | 17.318 | 1.00 | 34.86 |
| atom | 775 | CG  | PHE | 101 | 13.545 | 54.794 | 18.160 | 1.00 | 35.15 |
| atom | 776 | CD1 | PHE | 101 | 13.599 | 53.726 | 19.036 | 1.00 | 35.56 |
| atom | 777 | CD2 | PHE | 101 | 12.344 | 55.477 | 18.028 | 1.00 | 36.84 |
| atom | 778 | CE1 | PHE | 101 | 12.504 | 53.358 | 19.799 | 1.00 | 36.02 |
| atom | 779 | CE2 | PHE | 101 | 11.230 | 55.126 | 18.774 | 1.00 | 35.71 |
| atom | 780 | CZ  | PHE | 101 | 11.315 | 54.055 | 19.655 | 1.00 | 36.60 |
| atom | 781 | C   | PHE | 101 | 15.718 | 55.002 | 14.970 | 1.00 | 33.93 |
| atom | 782 | O   | PHE | 101 | 16.276 | 56.093 | 15.040 | 1.00 | 32.19 |
| atom | 783 | N   | GLY | 102 | 16.009 | 54.182 | 13.959 | 1.00 | 34.44 |
| atom | 784 | CA  | GLY | 102 | 16.878 | 54.513 | 12.852 | 1.00 | 32.69 |
| atom | 785 | C   | GLY | 102 | 18.339 | 54.195 | 12.867 | 1.00 | 31.96 |
| atom | 786 | O   | GLY | 102 | 19.049 | 54.794 | 12.059 | 1.00 | 32.33 |
| atom | 787 | N   | TYR | 103 | 18.837 | 53.334 | 13.739 | 1.00 | 31.53 |
| atom | 788 | CA  | TYR | 103 | 20.255 | 53.016 | 13.788 | 1.00 | 31.02 |

| atom | 789 | CB  | TYR | 103 | 20.995 | 53.944 | 14.749 | 1.00 | 30.17 |
|------|-----|-----|-----|-----|--------|--------|--------|------|-------|
| atom | 790 | CG  | TYR | 103 | 20.492 | 53.849 | 16.167 | 1.00 | 31.09 |
| atom | 791 | CD1 | TYR | 103 | 21.041 | 52.954 | 17.089 | 1.00 | 30.47 |
| atom | 792 | CE1 | TYR | 103 | 20.580 | 52.899 | 18.389 | 1.00 | 28.33 |
| atom | 793 | CD2 | TYR | 103 | 19.461 | 54.687 | 16.588 | 1.00 | 30.24 |
| atom | 794 | CE2 | TYR | 103 | 18.995 | 54.619 | 17.882 | 1.00 | 29.56 |
| atom | 795 | CZ  | TYR | 103 | 19.552 | 53.718 | 18.775 | 1.00 | 28.28 |
| atom | 796 | OH  | TYR | 103 | 19.063 | 53.674 | 20.056 | 1.00 | 26.38 |
| atom | 797 | C   | TYR | 103 | 20.429 | 51.558 | 14.181 | 1.00 | 30.53 |
| atom | 798 | 0   | TYR | 103 | 19.469 | 50.962 | 14.679 | 1.00 | 31.65 |
| atom | 799 | N   | GLY | 104 | 21.609 | 51.009 | 13.954 | 1.00 | 29.78 |
| atom | 800 | CA  | GLY | 104 | 21.889 | 49.619 | 14.308 | 1.00 | 29.76 |
| atom | 801 | C   | GLY | 104 | 23.143 | 49.447 | 15.153 | 1.00 | 29.99 |
| atom | 802 | 0   | GLY | 104 | 23.791 | 50.429 | 15.556 | 1.00 | 29.56 |
| atom | 803 | N   | ALA | 105 | 23.472 | 48.183 | 15.431 | 1.00 | 30.04 |
| atom | 804 | CA  | ALA | 105 | 24.627 | 47.815 | 16.231 | 1.00 | 31.21 |
| atom | 805 | CB  | ALA | 105 | 24.871 | 46.308 | 16.238 | 1.00 | 32.66 |
| atom | 806 | C   | ALA | 105 | 25.933 | 48.477 | 15.831 | 1.00 | 31.18 |
| atom | 807 | 0   | ALA | 105 | 26.613 | 49.001 | 16.720 | 1.00 | 32.94 |
| atom | 808 | N   | LYS | 106 | 26.341 | 48.457 | 14.585 | 1.00 | 31.82 |
| atom | 809 | CA  | LYS | 106 | 27.588 | 49.096 | 14.159 | 1.00 | 33.16 |
| atom | 810 | CB  | LYS | 106 | 27.985 | 48.714 | 12.736 | 1.00 | 36.56 |
| atom | 811 | CG  | LYS | 106 | 26.892 | 48.764 | 11.701 | 1.00 | 41.69 |
| atom | 812 | CD  | LYS | 106 | 27.335 | 48.256 | 10.335 | 1.00 | 46.79 |
| atom | 813 | CE  | LYS | 106 | 26.145 | 48.016 | 9.402  | 1.00 | 48.14 |
| atom | 814 | NZ  | LYS | 106 | 26.515 | 48.303 | 7.977  | 1.00 | 51.75 |
| atom | 815 | C   | LYS | 106 | 27.528 | 50.603 | 14.383 | 1.00 | 32.10 |
| atom | 816 | 0   | LYS | 106 | 28.532 | 51.179 | 14.775 | 1.00 | 31.01 |
| atom | 817 | N   | ASP | 107 | 26.380 | 51.267 | 14.217 | 1.00 | 32.44 |
| atom | 818 | CA  | ASP | 107 | 26.304 | 52.691 | 14.525 | 1.00 | 32.34 |
| atom | 819 | CB  | ASP | 107 | 24.940 | 53.308 | 14.262 | 1.00 | 32.09 |
| atom | 820 | CG  | ASP | 107 | 24.534 | 53.145 | 12.815 | 1.00 | 31.69 |
| atom | 821 | OD1 | ASP | 107 | 25.280 | 53.650 | 11.959 | 1.00 | 34.96 |
| atom | 822 | OD2 | ASP | 107 | 23.505 | 52.523 | 12.520 | 1.00 | 32.64 |
| atom | 823 | C   | ASP | 107 | 26.630 | 52.884 | 16.011 | 1.00 | 32.49 |
| atom | 824 | 0   | ASP | 107 | 27.493 | 53.699 | 16.312 | 1.00 | 33.38 |

| | | | | | | | | | |
|---|---|---|---|---|---|---|---|---|---|
| atom | 825 | N | VAL | 108 | 25.960 | 52.120 | 16.866 | 1.00 | 30.97 |
| atom | 826 | CA | VAL | 108 | 26.165 | 52.165 | 18.294 | 1.00 | 30.58 |
| atom | 827 | CB | VAL | 108 | 25.338 | 51.132 | 19.081 | 1.00 | 29.70 |
| atom | 828 | CG1 | VAL | 108 | 25.815 | 51.023 | 20.536 | 1.00 | 27.03 |
| atom | 829 | CG2 | VAL | 108 | 23.851 | 51.473 | 19.061 | 1.00 | 27.78 |
| atom | 830 | C | VAL | 108 | 27.642 | 51.941 | 18.611 | 1.00 | 31.50 |
| atom | 831 | 0 | VAL | 108 | 28.263 | 52.779 | 19.262 | 1.00 | 30.29 |
| atom | 832 | N | ARG | 109 | 28.181 | 50.825 | 18.131 | 1.00 | 31.94 |
| atom | 833 | CA | ARG | 109 | 29.582 | 50.479 | 18.333 | 1.00 | 33.32 |
| atom | 834 | CB | ARG | 109 | 29.862 | 49.087 | 17.742 | 1.00 | 39.04 |
| atom | 835 | CG | ARG | 109 | 29.219 | 47.982 | 18.577 | 1.00 | 44.64 |
| atom | 836 | CD | ARG | 109 | 28.861 | 46.760 | 17.749 | 1.00 | 49.57 |
| atom | 837 | NE | ARG | 109 | 29.989 | 46.182 | 17.032 | 1.00 | 54.31 |
| atom | 838 | CZ | ARG | 109 | 31.089 | 45.684 | 17.585 | 1.00 | 56.41 |
| atom | 839 | NH1 | ARG | 109 | 31.224 | 45.671 | 18.907 | 1.00 | 56.29 |
| atom | 840 | NH2 | ARG | 109 | 32.068 | 45.184 | 16.836 | 1.00 | 58.08 |
| atom | 841 | C | ARG | 109 | 30.564 | 51.519 | 17.806 | 1.00 | 32.51 |
| atom | 842 | 0 | ARG | 109 | 31.650 | 51.652 | 18.389 | 1.00 | 30.97 |
| atom | 843 | N | ASN | 110 | 30.217 | 52.307 | 16.797 | 1.00 | 32.28 |
| atom | 844 | CA | ASN | 110 | 31.053 | 53.383 | 16.292 | 1.00 | 33.36 |
| atom | 845 | CB | ASN | 110 | 30.933 | 53.568 | 14.777 | 1.00 | 36.00 |
| atom | 846 | CG | ASN | 110 | 31.387 | 52.346 | 14.002 | 1.00 | 41.32 |
| atom | 847 | OD1 | ASN | 110 | 30.905 | 52.089 | 12.890 | 1.00 | 44.02 |
| atom | 848 | ND2 | ASN | 110 | 32.297 | 51.576 | 14.590 | 1.00 | 42.16 |
| atom | 849 | C | ASN | 110 | 30.714 | 54.721 | 16.949 | 1.00 | 32.78 |
| atom | 850 | 0 | ASN | 110 | 31.141 | 55.771 | 16.480 | 1.00 | 33.29 |
| atom | 851 | N | LEU | 111 | 29.895 | 54.731 | 17.985 | 1.00 | 31.98 |
| atom | 852 | CA | LEU | 111 | 29.486 | 55.939 | 18.682 | 1.00 | 31.49 |
| atom | 853 | CB | LEU | 111 | 30.616 | 56.499 | 19.517 | 1.00 | 28.30 |
| atom | 854 | CG | LEU | 111 | 31.277 | 55.633 | 20.583 | 1.00 | 27.81 |
| atom | 855 | CD1 | LEU | 111 | 32.342 | 56.428 | 21.349 | 1.00 | 26.90 |
| atom | 856 | CD2 | LEU | 111 | 30.249 | 55.097 | 21.548 | 1.00 | 29.48 |
| atom | 857 | C | LEU | 111 | 28.952 | 56.968 | 17.688 | 1.00 | 32.46 |
| atom | 858 | 0 | LEU | 111 | 29.360 | 58.137 | 17.671 | 1.00 | 32.73 |
| atom | 859 | N | SER | 112 | 27.999 | 56.536 | 16.856 | 1.00 | 32.11 |
| atom | 860 | CA | SER | 112 | 27.469 | 57.497 | 15.882 | 1.00 | 33.29 |

| atom | 861 | CB  | SER | 112 | 26.766 | 56.766 | 14.752 | 1.00 | 30.43 |
| atom | 862 | OG  | SER | 112 | 25.377 | 56.745 | 14.951 | 1.00 | 32.82 |
| atom | 863 | C   | SER | 112 | 26.624 | 58.516 | 16.627 | 1.00 | 33.57 |
| atom | 864 | O   | SER | 112 | 26.006 | 58.212 | 17.649 | 1.00 | 35.13 |
| atom | 865 | N   | SER | 113 | 26.584 | 59.755 | 16.171 | 1.00 | 33.55 |
| atom | 866 | CA  | SER | 113 | 25.812 | 60.812 | 16.819 | 1.00 | 34.53 |
| atom | 867 | CB  | SER | 113 | 26.016 | 62.154 | 16.102 | 1.00 | 31.71 |
| atom | 868 | OG  | SER | 113 | 25.665 | 62.012 | 14.735 | 1.00 | 32.40 |
| atom | 869 | C   | SER | 113 | 24.325 | 60.475 | 16.901 | 1.00 | 35.07 |
| atom | 870 | O   | SER | 113 | 23.638 | 60.741 | 17.894 | 1.00 | 34.43 |
| atom | 871 | N   | LYS | 114 | 23.803 | 59.840 | 15.857 | 1.00 | 35.84 |
| atom | 872 | CA  | LYS | 114 | 22.414 | 59.401 | 15.819 | 1.00 | 37.45 |
| atom | 873 | CB  | LYS | 114 | 22.111 | 58.804 | 14.458 | 1.00 | 42.53 |
| atom | 874 | CG  | LYS | 114 | 20.757 | 58.214 | 14.202 | 1.00 | 48.91 |
| atom | 875 | CD  | LYS | 114 | 19.887 | 59.051 | 13.279 | 1.00 | 54.77 |
| atom | 876 | CE  | LYS | 114 | 19.557 | 60.426 | 13.859 | 1.00 | 57.51 |
| atom | 877 | NZ  | LYS | 114 | 18.483 | 61.097 | 13.069 | 1.00 | 57.88 |
| atom | 878 | C   | LYS | 114 | 22.158 | 58.415 | 16.958 | 1.00 | 36.85 |
| atom | 879 | O   | LYS | 114 | 21.239 | 58.646 | 17.754 | 1.00 | 36.41 |
| atom | 880 | N   | ALA | 115 | 22.980 | 57.372 | 17.103 | 1.00 | 36.24 |
| atom | 881 | CA  | ALA | 115 | 22.775 | 56.427 | 18.206 | 1.00 | 34.92 |
| atom | 882 | CB  | ALA | 115 | 23.623 | 55.165 | 18.053 | 1.00 | 34.23 |
| atom | 883 | C   | ALA | 115 | 23.054 | 57.057 | 19.568 | 1.00 | 33.80 |
| atom | 884 | O   | ALA | 115 | 22.268 | 56.831 | 20.489 | 1.00 | 32.66 |
| atom | 885 | N   | VAL | 116 | 24.109 | 57.855 | 19.725 | 1.00 | 32.93 |
| atom | 886 | CA  | VAL | 116 | 24.380 | 58.425 | 21.045 | 1.00 | 32.73 |
| atom | 887 | CB  | VAL | 116 | 25.763 | 59.097 | 21.123 | 1.00 | 32.48 |
| atom | 888 | CG1 | VAL | 116 | 26.044 | 59.573 | 22.542 | 1.00 | 33.83 |
| atom | 889 | CG2 | VAL | 116 | 26.858 | 58.111 | 20.723 | 1.00 | 31.94 |
| atom | 890 | C   | VAL | 116 | 23.282 | 59.360 | 21.526 | 1.00 | 32.02 |
| atom | 891 | O   | VAL | 116 | 22.913 | 59.324 | 22.710 | 1.00 | 32.23 |
| atom | 892 | N   | ASN | 117 | 22.704 | 60.184 | 20.662 | 1.00 | 30.87 |
| atom | 893 | CA  | ASN | 117 | 21.655 | 61.117 | 21.060 | 1.00 | 29.79 |
| atom | 894 | CB  | ASN | 117 | 21.376 | 62.163 | 19.984 | 1.00 | 32.08 |
| atom | 895 | CG  | ASN | 117 | 22.516 | 63.131 | 19.719 | 1.00 | 38.47 |
| atom | 896 | OD1 | ASN | 117 | 23.570 | 63.130 | 20.379 | 1.00 | 40.33 |

| atom | 897 | ND2 | ASN | 117 | 22.448 | 64.027 | 18.721 | 1.00 | 37.80 |
|------|-----|-----|-----|-----|--------|--------|--------|------|-------|
| atom | 898 | C | ASN | 117 | 20.396 | 60.335 | 21.428 | 1.00 | 28.77 |
| atom | 899 | 0 | ASN | 117 | 19.751 | 60.661 | 22.417 | 1.00 | 27.56 |
| atom | 900 | N | HIS | 118 | 20.106 | 59.248 | 20.706 | 1.00 | 27.51 |
| atom | 901 | CA | HIS | 118 | 18.911 | 58.464 | 21.004 | 1.00 | 27.17 |
| atom | 902 | CB | HIS | 118 | 18.561 | 57.461 | 19.896 | 1.00 | 26.40 |
| atom | 903 | CG | HIS | 118 | 17.312 | 56.722 | 20.256 | 1.00 | 29.35 |
| atom | 904 | CD2 | HIS | 118 | 16.029 | 57.160 | 20.398 | 1.00 | 29.90 |
| atom | 905 | ND1 | HIS | 118 | 17.303 | 55.378 | 20.559 | 1.00 | 29.23 |
| atom | 906 | CE1 | HIS | 118 | 16.076 | 55.005 | 20.878 | 1.00 | 28.93 |
| atom | 907 | NE2 | HIS | 118 | 15.282 | 56.065 | 20.785 | 1.00 | 29.97 |
| atom | 908 | C | HIS | 118 | 19.029 | 57.764 | 22.350 | 1.00 | 26.40 |
| atom | 909 | 0 | HIS | 118 | 18.082 | 57.793 | 23.129 | 1.00 | 26.23 |
| atom | 910 | N | ILE | 119 | 20.176 | 57.168 | 22.608 | 1.00 | 25.54 |
| atom | 911 | CA | ILE | 119 | 20.453 | 56.443 | 23.845 | 1.00 | 25.77 |
| atom | 912 | CB | ILE | 119 | 21.853 | 55.791 | 23.704 | 1.00 | 25.09 |
| atom | 913 | CG2 | ILE | 119 | 22.440 | 55.411 | 25.034 | 1.00 | 20.78 |
| atom | 914 | CG1 | ILE | 119 | 21.712 | 54.604 | 22.740 | 1.00 | 23.62 |
| atom | 915 | CD1 | ILE | 119 | 23.043 | 54.114 | 22.215 | 1.00 | 23.78 |
| atom | 916 | C | ILE | 119 | 20.332 | 57.352 | 25.049 | 1.00 | 25.72 |
| atom | 917 | 0 | ILE | 119 | 19.716 | 57.004 | 26.046 | 1.00 | 26.34 |
| atom | 918 | N | HIS | 120 | 20.871 | 58.567 | 24.987 | 1.00 | 25.92 |
| atom | 919 | CA | HIS | 120 | 20.753 | 59.587 | 26.008 | 1.00 | 25.03 |
| atom | 920 | CB | HIS | 120 | 21.451 | 60.875 | 25.568 | 1.00 | 19.50 |
| atom | 921 | CG | HIS | 120 | 22.906 | 60.892 | 25.880 | 1.00 | 21.11 |
| atom | 922 | CD2 | HIS | 120 | 24.032 | 60.819 | 25.126 | 1.00 | 20.44 |
| atom | 923 | ND1 | HIS | 120 | 23.342 | 60.996 | 27.190 | 1.00 | 23.85 |
| atom | 924 | CE1 | HIS | 120 | 24.671 | 61.004 | 27.218 | 1.00 | 22.45 |
| atom | 925 | NE2 | HIS | 120 | 25.109 | 60.897 | 25.973 | 1.00 | 20.40 |
| atom | 926 | C | HIS | 120 | 19.293 | 59.931 | 26.316 | 1.00 | 26.49 |
| atom | 927 | 0 | HIS | 120 | 18.893 | 59.938 | 27.493 | 1.00 | 26.12 |
| atom | 928 | N | SER | 121 | 18.485 | 60.136 | 25.277 | 1.00 | 25.93 |
| atom | 929 | CA | SER | 121 | 17.074 | 60.444 | 25.480 | 1.00 | 27.56 |
| atom | 930 | CB | SER | 121 | 16.369 | 60.863 | 24.187 | 1.00 | 28.43 |
| atom | 931 | OG | SER | 121 | 15.995 | 59.717 | 23.422 | 1.00 | 34.04 |
| atom | 932 | C | SER | 121 | 16.387 | 59.245 | 26.119 | 1.00 | 27.33 |

| atom | 933 | O | SER | 121 | 15.519 | 59.459 | 26.962 | 1.00 | 28.01 |
|---|---|---|---|---|---|---|---|---|---|
| atom | 934 | N | VAL | 122 | 16.781 | 58.025 | 25.788 | 1.00 | 27.20 |
| atom | 935 | CA | VAL | 122 | 16.169 | 56.835 | 26.361 | 1.00 | 28.11 |
| atom | 936 | CB | VAL | 122 | 16.545 | 55.526 | 25.631 | 1.00 | 26.41 |
| atom | 937 | CG1 | VAL | 122 | 15.885 | 54.343 | 26.333 | 1.00 | 23.66 |
| atom | 938 | CG2 | VAL | 122 | 16.108 | 55.594 | 24.173 | 1.00 | 24.72 |
| atom | 939 | C | VAL | 122 | 16.503 | 56.714 | 27.842 | 1.00 | 29.35 |
| atom | 940 | O | VAL | 122 | 15.604 | 56.450 | 28.653 | 1.00 | 29.90 |
| atom | 941 | N | TRP | 123 | 17.762 | 56.937 | 28.197 | 1.00 | 29.56 |
| atom | 942 | CA | TRP | 123 | 18.183 | 56.921 | 29.595 | 1.00 | 30.64 |
| atom | 943 | CB | TRP | 123 | 19.692 | 57.187 | 29.654 | 1.00 | 29.90 |
| atom | 944 | CG | TRP | 123 | 20.286 | 57.238 | 31.026 | 1.00 | 28.10 |
| atom | 945 | CD2 | TRP | 123 | 20.587 | 56.114 | 31.865 | 1.00 | 26.10 |
| atom | 946 | CE2 | TRP | 123 | 21.147 | 56.628 | 33.053 | 1.00 | 27.09 |
| atom | 947 | CE3 | TRP | 123 | 20.435 | 54.731 | 31.736 | 1.00 | 25.33 |
| atom | 948 | CD1 | TRP | 123 | 20.667 | 58.368 | 31.711 | 1.00 | 26.54 |
| atom | 949 | NE1 | TRP | 123 | 21.180 | 58.004 | 32.932 | 1.00 | 27.87 |
| atom | 950 | CZ2 | TRP | 123 | 21.553 | 55.803 | 34.094 | 1.00 | 24.63 |
| atom | 951 | CZ3 | TRP | 123 | 20.835 | 53.910 | 32.781 | 1.00 | 23.50 |
| atom | 952 | CH2 | TRP | 123 | 21.386 | 54.450 | 33.944 | 1.00 | 24.32 |
| atom | 953 | C | TRP | 123 | 17.470 | 57.990 | 30.405 | 1.00 | 31.64 |
| atom | 954 | O | TRP | 123 | 17.064 | 57.746 | 31.540 | 1.00 | 32.63 |
| atom | 955 | N | LYS | 124 | 17.339 | 59.214 | 29.886 | 1.00 | 32.83 |
| atom | 956 | CA | LYS | 124 | 16.653 | 60.283 | 30.620 | 1.00 | 33.02 |
| atom | 957 | CB | LYS | 124 | 16.725 | 61.620 | 29.898 | 1.00 | 34.20 |
| atom | 958 | CG | LYS | 124 | 15.818 | 62.722 | 30.428 | 1.00 | 35.32 |
| atom | 959 | CD | LYS | 124 | 16.036 | 63.975 | 29.577 | 1.00 | 37.39 |
| atom | 960 | CE | LYS | 124 | 15.459 | 65.246 | 30.148 | 1.00 | 41.57 |
| atom | 961 | NZ | LYS | 124 | 14.008 | 65.188 | 30.536 | 1.00 | 44.15 |
| atom | 962 | C | LYS | 124 | 15.206 | 59.873 | 30.876 | 1.00 | 32.46 |
| atom | 963 | O | LYS | 124 | 14.740 | 60.038 | 31.995 | 1.00 | 32.18 |
| atom | 964 | N | ASP | 125 | 14.525 | 59.292 | 29.886 | 1.00 | 31.76 |
| atom | 965 | CA | ASP | 125 | 13.165 | 58.815 | 30.037 | 1.00 | 30.82 |
| atom | 966 | CB | ASP | 125 | 12.652 | 58.213 | 28.723 | 1.00 | 31.14 |
| atom | 967 | CG | ASP | 125 | 11.245 | 57.663 | 28.897 | 1.00 | 30.89 |
| atom | 968 | OD1 | ASP | 125 | 10.335 | 58.503 | 28.783 | 1.00 | 32.56 |

| atom | 969 | OD2 | ASP | 125 | 11.084 | 56.456 | 29.173 | 1.00 | 30.48 |
|------|-----|-----|-----|-----|--------|--------|--------|------|-------|
| atom | 970 | C | ASP | 125 | 13.055 | 57.768 | 31.144 | 1.00 | 30.92 |
| atom | 971 | O | ASP | 125 | 12.098 | 57.780 | 31.928 | 1.00 | 30.73 |
| atom | 972 | N | LEU | 126 | 14.024 | 56.872 | 31.223 | 1.00 | 30.06 |
| atom | 973 | CA | LEU | 126 | 14.064 | 55.832 | 32.236 | 1.00 | 31.19 |
| atom | 974 | CB | LEU | 126 | 15.275 | 54.925 | 32.085 | 1.00 | 30.34 |
| atom | 975 | CG | LEU | 126 | 15.310 | 53.700 | 31.205 | 1.00 | 28.80 |
| atom | 976 | CD1 | LEU | 126 | 16.611 | 52.958 | 31.528 | 1.00 | 28.14 |
| atom | 977 | CD2 | LEU | 126 | 14.093 | 52.811 | 31.440 | 1.00 | 26.25 |
| atom | 978 | C | LEU | 126 | 14.163 | 56.426 | 33.639 | 1.00 | 32.34 |
| atom | 979 | O | LEU | 126 | 13.596 | 55.858 | 34.571 | 1.00 | 33.01 |
| atom | 980 | N | LEU | 127 | 14.913 | 57.518 | 33.799 | 1.00 | 32.51 |
| atom | 981 | CA | LEU | 127 | 15.026 | 58.138 | 35.111 | 1.00 | 33.15 |
| atom | 982 | CB | LEU | 127 | 16.241 | 59.077 | 35.179 | 1.00 | 29.94 |
| atom | 983 | CG | LEU | 127 | 17.600 | 58.395 | 34.970 | 1.00 | 29.21 |
| atom | 984 | CD1 | LEU | 127 | 18.711 | 59.438 | 34.927 | 1.00 | 27.52 |
| atom | 985 | CD2 | LEU | 127 | 17.846 | 57.355 | 36.053 | 1.00 | 27.47 |
| atom | 986 | C | LEU | 127 | 13.760 | 58.907 | 35.479 | 1.00 | 33.47 |
| atom | 987 | O | LEU | 127 | 13.318 | 58.873 | 36.626 | 1.00 | 32.84 |
| atom | 988 | N | GLU | 128 | 13.177 | 59.612 | 34.525 | 1.00 | 34.41 |
| atom | 989 | CA | GLU | 128 | 11.999 | 60.438 | 34.746 | 1.00 | 35.83 |
| atom | 990 | CB | GLU | 128 | 12.004 | 61.563 | 33.696 | 1.00 | 39.47 |
| atom | 991 | CG | GLU | 128 | 13.073 | 62.598 | 34.026 | 1.00 | 44.87 |
| atom | 992 | CD | GLU | 128 | 13.218 | 63.638 | 32.945 | 1.00 | 49.70 |
| atom | 993 | OE1 | GLU | 128 | 12.362 | 63.700 | 32.024 | 1.00 | 53.87 |
| atom | 994 | OE2 | GLU | 128 | 14.200 | 64.413 | 32.978 | 1.00 | 50.76 |
| atom | 995 | C | GLU | 128 | 10.653 | 59.743 | 34.746 | 1.00 | 35.73 |
| atom | 996 | O | GLU | 128 | 9.718 | 60.263 | 35.356 | 1.00 | 35.85 |
| atom | 997 | N | ASP | 129 | 10.512 | 58.602 | 34.084 | 1.00 | 35.27 |
| atom | 998 | CA | ASP | 129 | 9.275 | 57.827 | 34.086 | 1.00 | 33.25 |
| atom | 999 | CB | ASP | 129 | 8.666 | 57.763 | 32.695 | 1.00 | 33.45 |
| atom | 1000 | CG | ASP | 129 | 7.490 | 56.831 | 32.516 | 1.00 | 32.17 |
| atom | 1001 | OD1 | ASP | 129 | 7.134 | 56.081 | 33.444 | 1.00 | 31.38 |
| atom | 1002 | OD2 | ASP | 129 | 6.877 | 56.845 | 31.422 | 1.00 | 32.33 |
| atom | 1003 | C | ASP | 129 | 9.638 | 56.438 | 34.594 | 1.00 | 33.11 |
| atom | 1004 | O | ASP | 129 | 10.533 | 55.782 | 34.058 | 1.00 | 32.38 |

| atom | 1005 | N   | THR | 130 | 8.979  | 55.986 | 35.658 | 1.00 32.93 |
|------|------|-----|-----|-----|--------|--------|--------|------------|
| atom | 1006 | CA  | THR | 130 | 9.300  | 54.670 | 36.209 | 1.00 33.32 |
| atom | 1007 | CB  | THR | 130 | 9.856  | 54.769 | 37.645 | 1.00 33.49 |
| atom | 1008 | OG1 | THR | 130 | 8.803  | 55.253 | 38.493 | 1.00 34.96 |
| atom | 1009 | CG2 | THR | 130 | 11.045 | 55.701 | 37.778 | 1.00 30.06 |
| atom | 1010 | C   | THR | 130 | 8.070  | 53.776 | 36.203 | 1.00 34.04 |
| atom | 1011 | O   | THR | 130 | 8.012  | 52.793 | 36.942 | 1.00 33.63 |
| atom | 1012 | N   | VAL | 131 | 7.086  | 54.057 | 35.336 | 1.00 34.56 |
| atom | 1013 | CA  | VAL | 131 | 5.865  | 53.280 | 35.312 | 1.00 35.87 |
| atom | 1014 | CB  | VAL | 131 | 4.662  | 54.046 | 35.934 | 1.00 38.04 |
| atom | 1015 | CG1 | VAL | 131 | 4.868  | 54.432 | 37.393 | 1.00 39.19 |
| atom | 1016 | CG2 | VAL | 131 | 4.322  | 55.323 | 35.165 | 1.00 37.80 |
| atom | 1017 | C   | VAL | 131 | 5.433  | 52.758 | 33.949 | 1.00 35.83 |
| atom | 1018 | O   | VAL | 131 | 5.009  | 51.596 | 33.897 | 1.00 36.49 |
| atom | 1019 | N   | THR | 132 | 5.487  | 53.529 | 32.884 | 1.00 35.80 |
| atom | 1020 | CA  | THR | 132 | 5.023  | 53.141 | 31.565 | 1.00 35.92 |
| atom | 1021 | CB  | THR | 132 | 5.240  | 54.305 | 30.565 | 1.00 36.71 |
| atom | 1022 | OG1 | THR | 132 | 4.834  | 55.539 | 31.165 | 1.00 36.50 |
| atom | 1023 | CG2 | THR | 132 | 4.468  | 54.082 | 29.268 | 1.00 36.00 |
| atom | 1024 | C   | THR | 132 | 5.737  | 51.921 | 31.002 | 1.00 36.28 |
| atom | 1025 | O   | THR | 132 | 6.949  | 51.929 | 30.739 | 1.00 35.29 |
| atom | 1026 | N   | PRO | 133 | 4.964  | 50.884 | 30.733 | 1.00 36.02 |
| atom | 1027 | CD  | PRO | 133 | 3.506  | 50.744 | 30.971 | 1.00 36.57 |
| atom | 1028 | CA  | PRO | 133 | 5.502  | 49.638 | 30.193 | 1.00 35.93 |
| atom | 1029 | CB  | PRO | 133 | 4.277  | 48.741 | 30.012 | 1.00 36.78 |
| atom | 1030 | CG  | PRO | 133 | 3.401  | 49.228 | 31.138 | 1.00 36.77 |
| atom | 1031 | C   | PRO | 133 | 6.258  | 49.893 | 28.911 | 1.00 34.89 |
| atom | 1032 | O   | PRO | 133 | 5.771  | 50.591 | 28.033 | 1.00 34.65 |
| atom | 1033 | N   | ILE | 134 | 7.505  | 49.413 | 28.884 | 1.00 34.47 |
| atom | 1034 | CA  | ILE | 134 | 8.332  | 49.618 | 27.681 | 1.00 32.52 |
| atom | 1035 | CB  | ILE | 134 | 9.799  | 49.544 | 28.124 | 1.00 31.09 |
| atom | 1036 | CG2 | ILE | 134 | 10.726 | 49.294 | 26.936 | 1.00 31.60 |
| atom | 1037 | CG1 | ILE | 134 | 10.199 | 50.807 | 28.897 | 1.00 26.79 |
| atom | 1038 | CD1 | ILE | 134 | 11.625 | 50.760 | 29.431 | 1.00 25.33 |
| atom | 1039 | C   | ILE | 134 | 7.920  | 48.552 | 26.685 | 1.00 31.87 |
| atom | 1040 | O   | ILE | 134 | 7.542  | 47.439 | 27.074 | 1.00 31.37 |

| atom | 1041 | N   | ASP | 135 | 7.967  | 48.826 | 25.408 | 1.00 | 32.82 |
|------|------|-----|-----|-----|--------|--------|--------|------|-------|
| atom | 1042 | CA  | ASP | 135 | 7.591  | 47.879 | 24.364 | 1.00 | 32.31 |
| atom | 1043 | CB  | ASP | 135 | 7.515  | 48.618 | 23.007 | 1.00 | 32.21 |
| atom | 1044 | CG  | ASP | 135 | 6.869  | 47.726 | 21.967 | 1.00 | 34.28 |
| atom | 1045 | OD1 | ASP | 135 | 7.181  | 47.751 | 20.768 | 1.00 | 37.79 |
| atom | 1046 | OD2 | ASP | 135 | 5.984  | 46.949 | 22.366 | 1.00 | 35.47 |
| atom | 1047 | C   | ASP | 135 | 8.612  | 46.764 | 24.210 | 1.00 | 32.38 |
| atom | 1048 | O   | ASP | 135 | 9.790  | 47.008 | 24.473 | 1.00 | 32.37 |
| atom | 1049 | N   | THR | 136 | 8.192  | 45.574 | 23.830 | 1.00 | 31.67 |
| atom | 1050 | CA  | THR | 136 | 9.080  | 44.455 | 23.554 | 1.00 | 31.75 |
| atom | 1051 | CB  | THR | 136 | 9.086  | 43.379 | 24.657 | 1.00 | 31.21 |
| atom | 1052 | OG1 | THR | 136 | 7.756  | 42.876 | 24.849 | 1.00 | 30.66 |
| atom | 1053 | CG2 | THR | 136 | 9.588  | 43.890 | 25.997 | 1.00 | 29.18 |
| atom | 1054 | C   | THR | 136 | 8.656  | 43.787 | 22.240 | 1.00 | 31.11 |
| atom | 1055 | O   | THR | 136 | 7.463  | 43.842 | 21.912 | 1.00 | 30.39 |
| atom | 1056 | N   | THR | 137 | 9.581  | 43.238 | 21.468 | 1.00 | 31.05 |
| atom | 1057 | CA  | THR | 137 | 9.258  | 42.499 | 20.244 | 1.00 | 30.25 |
| atom | 1058 | CB  | THR | 137 | 10.220 | 42.730 | 19.079 | 1.00 | 27.72 |
| atom | 1059 | OG1 | THR | 137 | 10.327 | 44.099 | 18.701 | 1.00 | 26.38 |
| atom | 1060 | CG2 | THR | 137 | 9.750  | 41.986 | 17.821 | 1.00 | 29.69 |
| atom | 1061 | C   | THR | 137 | 9.263  | 40.987 | 20.546 | 1.00 | 31.26 |
| atom | 1062 | O   | THR | 137 | 10.152 | 40.466 | 21.232 | 1.00 | 30.49 |
| atom | 1063 | N   | ILE | 138 | 8.267  | 40.252 | 20.065 | 1.00 | 32.31 |
| atom | 1064 | CA  | ILE | 138 | 8.215  | 38.807 | 20.298 | 1.00 | 33.92 |
| atom | 1065 | CB  | ILE | 138 | 6.914  | 38.350 | 20.971 | 1.00 | 33.90 |
| atom | 1066 | CG2 | ILE | 138 | 5.664  | 38.798 | 20.225 | 1.00 | 32.67 |
| atom | 1067 | CG1 | ILE | 138 | 6.935  | 36.830 | 21.134 | 1.00 | 35.41 |
| atom | 1068 | CD1 | ILE | 138 | 6.099  | 36.216 | 22.224 | 1.00 | 34.14 |
| atom | 1069 | C   | ILE | 138 | 8.524  | 38.119 | 18.978 | 1.00 | 35.72 |
| atom | 1070 | O   | ILE | 138 | 8.053  | 38.616 | 17.951 | 1.00 | 36.87 |
| atom | 1071 | N   | MET | 139 | 9.377  | 37.109 | 18.955 | 1.00 | 36.77 |
| atom | 1072 | CA  | MET | 139 | 9.761  | 36.422 | 17.735 | 1.00 | 38.76 |
| atom | 1073 | CB  | MET | 139 | 11.122 | 36.821 | 17.166 | 1.00 | 41.09 |
| atom | 1074 | CG  | MET | 139 | 11.282 | 38.177 | 16.535 | 1.00 | 44.16 |
| atom | 1075 | SD  | MET | 139 | 10.002 | 38.569 | 15.325 | 1.00 | 46.83 |
| atom | 1076 | CE  | MET | 139 | 10.869 | 38.182 | 13.810 | 1.00 | 45.10 |

| atom | 1077 | C   | MET | 139 | 9.901  | 34.914 | 17.996 | 1.00 | 39.71 |
| atom | 1078 | O   | MET | 139 | 10.089 | 34.500 | 19.144 | 1.00 | 38.98 |
| atom | 1079 | N   | ALA | 140 | 9.870  | 34.162 | 16.893 | 1.00 | 40.22 |
| atom | 1080 | CA  | ALA | 140 | 10.069 | 32.714 | 17.039 | 1.00 | 42.07 |
| atom | 1081 | CB  | ALA | 140 | 9.086  | 31.913 | 16.230 | 1.00 | 40.20 |
| atom | 1082 | C   | ALA | 140 | 11.523 | 32.443 | 16.651 | 1.00 | 43.52 |
| atom | 1083 | O   | ALA | 140 | 11.980 | 33.029 | 15.670 | 1.00 | 43.47 |
| atom | 1084 | N   | LYS | 141 | 12.249 | 31.671 | 17.442 | 1.00 | 45.45 |
| atom | 1085 | CA  | LYS | 141 | 13.645 | 31.390 | 17.086 | 1.00 | 48.14 |
| atom | 1086 | CB  | LYS | 141 | 14.396 | 30.920 | 18.315 | 1.00 | 51.73 |
| atom | 1087 | CG  | LYS | 141 | 15.832 | 31.397 | 18.498 | 1.00 | 54.11 |
| atom | 1088 | CD  | LYS | 141 | 16.264 | 30.915 | 19.878 | 1.00 | 55.56 |
| atom | 1089 | CE  | LYS | 141 | 17.552 | 31.516 | 20.391 | 1.00 | 57.47 |
| atom | 1090 | NZ  | LYS | 141 | 17.499 | 31.571 | 21.894 | 1.00 | 57.93 |
| atom | 1091 | C   | LYS | 141 | 13.669 | 30.319 | 15.999 | 1.00 | 48.57 |
| atom | 1092 | O   | LYS | 141 | 12.813 | 29.433 | 15.982 | 1.00 | 48.20 |
| atom | 1093 | N   | ASN | 142 | 14.639 | 30.404 | 15.101 | 1.00 | 49.53 |
| atom | 1094 | CA  | ASN | 142 | 14.774 | 29.409 | 14.040 | 1.00 | 50.30 |
| atom | 1095 | CB  | ASN | 142 | 14.833 | 30.027 | 12.653 | 1.00 | 49.86 |
| atom | 1096 | CG  | ASN | 142 | 13.593 | 30.786 | 12.234 | 1.00 | 49.54 |
| atom | 1097 | OD1 | ASN | 142 | 13.742 | 31.824 | 11.590 | 1.00 | 50.12 |
| atom | 1098 | ND2 | ASN | 142 | 12.422 | 30.278 | 12.592 | 1.00 | 47.83 |
| atom | 1099 | C   | ASN | 142 | 16.060 | 28.624 | 14.284 | 1.00 | 50.99 |
| atom | 1100 | O   | ASN | 142 | 17.142 | 29.155 | 14.024 | 1.00 | 52.12 |
| atom | 1101 | N   | GLU | 143 | 15.944 | 27.402 | 14.792 | 1.00 | 51.06 |
| atom | 1102 | CA  | GLU | 143 | 17.151 | 26.614 | 15.050 | 1.00 | 51.24 |
| atom | 1103 | CB  | GLU | 143 | 17.594 | 26.700 | 16.508 | 1.00 | 51.89 |
| atom | 1104 | CG  | GLU | 143 | 16.521 | 27.067 | 17.510 | 1.00 | 52.83 |
| atom | 1105 | CD  | GLU | 143 | 17.061 | 27.579 | 18.827 | 1.00 | 53.67 |
| atom | 1106 | OE1 | GLU | 143 | 16.301 | 27.593 | 19.819 | 1.00 | 53.37 |
| atom | 1107 | OE2 | GLU | 143 | 18.245 | 27.966 | 18.884 | 1.00 | 53.92 |
| atom | 1108 | C   | GLU | 143 | 16.993 | 25.180 | 14.566 | 1.00 | 50.60 |
| atom | 1109 | O   | GLU | 143 | 15.902 | 24.609 | 14.571 | 1.00 | 50.70 |
| atom | 1110 | N   | VAL | 144 | 18.117 | 24.628 | 14.125 | 1.00 | 49.54 |
| atom | 1111 | CA  | VAL | 144 | 18.218 | 23.286 | 13.577 | 1.00 | 48.44 |
| atom | 1112 | CB  | VAL | 144 | 19.408 | 23.237 | 12.583 | 1.00 | 47.66 |

| atom | 1113 | CG1 | VAL | 144 | 19.576 | 21.845 | 11.985 | 1.00 | 45.89 |
|------|------|-----|-----|-----|--------|--------|--------|------|-------|
| atom | 1114 | CG2 | VAL | 144 | 19.229 | 24.281 | 11.493 | 1.00 | 45.75 |
| atom | 1115 | C   | VAL | 144 | 18.429 | 22.194 | 14.608 | 1.00 | 48.29 |
| atom | 1116 | O   | VAL | 144 | 19.151 | 22.346 | 15.596 | 1.00 | 47.36 |
| atom | 1117 | N   | PHE | 145 | 17.681 | 21.099 | 14.492 | 1.00 | 48.67 |
| atom | 1118 | CA  | PHE | 145 | 17.752 | 19.933 | 15.353 | 1.00 | 49.51 |
| atom | 1119 | CB  | PHE | 145 | 16.731 | 19.960 | 16.491 | 1.00 | 50.47 |
| atom | 1120 | CG  | PHE | 145 | 16.740 | 21.178 | 17.368 | 1.00 | 53.11 |
| atom | 1121 | CD1 | PHE | 145 | 15.922 | 22.254 | 17.048 | 1.00 | 54.39 |
| atom | 1122 | CD2 | PHE | 145 | 17.552 | 21.276 | 18.487 | 1.00 | 52.47 |
| atom | 1123 | CE1 | PHE | 145 | 15.919 | 23.401 | 17.820 | 1.00 | 55.80 |
| atom | 1124 | CE2 | PHE | 145 | 17.536 | 22.414 | 19.268 | 1.00 | 53.25 |
| atom | 1125 | CZ  | PHE | 145 | 16.723 | 23.482 | 18.941 | 1.00 | 53.85 |
| atom | 1126 | C   | PHE | 145 | 17.548 | 18.624 | 14.569 | 1.00 | 50.20 |
| atom | 1127 | O   | PHE | 145 | 17.323 | 18.585 | 13.358 | 1.00 | 48.57 |
| atom | 1128 | N   | CYS | 146 | 17.649 | 17.513 | 15.297 | 1.00 | 51.48 |
| atom | 1129 | CA  | CYS | 146 | 17.412 | 16.186 | 14.755 | 1.00 | 53.41 |
| atom | 1130 | CB  | CYS | 146 | 18.586 | 15.233 | 14.961 | 1.00 | 49.50 |
| atom | 1131 | SG  | CYS | 146 | 18.209 | 13.479 | 14.699 | 1.00 | 43.89 |
| atom | 1132 | C   | CYS | 146 | 16.149 | 15.609 | 15.394 | 1.00 | 56.34 |
| atom | 1133 | O   | CYS | 146 | 15.948 | 15.730 | 16.598 | 1.00 | 55.75 |
| atom | 1134 | N   | VAL | 147 | 15.291 | 15.001 | 14.593 | 1.00 | 60.16 |
| atom | 1135 | CA  | VAL | 147 | 14.058 | 14.379 | 15.081 | 1.00 | 65.88 |
| atom | 1136 | CB  | VAL | 147 | 13.352 | 13.589 | 13.969 | 1.00 | 64.85 |
| atom | 1137 | CG1 | VAL | 147 | 12.067 | 12.960 | 14.481 | 1.00 | 65.06 |
| atom | 1138 | CG2 | VAL | 147 | 13.039 | 14.487 | 12.778 | 1.00 | 63.31 |
| atom | 1139 | C   | VAL | 147 | 14.394 | 13.493 | 16.275 | 1.00 | 70.14 |
| atom | 1140 | O   | VAL | 147 | 15.501 | 12.937 | 16.255 | 1.00 | 70.11 |
| atom | 1141 | N   | GLN | 148 | 13.513 | 13.346 | 17.280 | 1.00 | 75.29 |
| atom | 1142 | CA  | GLN | 148 | 13.964 | 12.611 | 18.441 | 1.00 | 81.43 |
| atom | 1143 | CB  | GLN | 148 | 14.115 | 13.614 | 19.628 | 1.00 | 83.52 |
| atom | 1144 | CG  | GLN | 148 | 15.106 | 13.089 | 20.653 | 1.00 | 86.12 |
| atom | 1145 | CD  | GLN | 148 | 15.549 | 14.033 | 21.736 | 1.00 | 87.68 |
| atom | 1146 | OE1 | GLN | 148 | 15.392 | 15.254 | 21.674 | 1.00 | 88.39 |
| atom | 1147 | NE2 | GLN | 148 | 16.147 | 13.481 | 22.793 | 1.00 | 88.48 |
| atom | 1148 | C   | GLN | 148 | 13.409 | 11.340 | 19.052 | 1.00 | 85.20 |

| atom | 1149 | O | GLN | 148 | 12.319 | 11.185 | 19.581 | 1.00 85.67 |
|------|------|------|------|------|--------|--------|--------|-----------|
| atom | 1150 | N | PRO | 149 | 14.378 | 10.407 | 19.175 | 1.00 88.26 |
| atom | 1151 | CD | PRO | 149 | 15.757 | 10.587 | 18.620 | 1.00 89.22 |
| atom | 1152 | CA | PRO | 149 | 14.290 | 9.117 | 19.817 | 1.00 90.26 |
| atom | 1153 | CB | PRO | 149 | 15.661 | 8.472 | 19.718 | 1.00 90.27 |
| atom | 1154 | CG | PRO | 149 | 16.390 | 9.238 | 18.679 | 1.00 89.82 |
| atom | 1155 | C | PRO | 149 | 13.922 | 9.287 | 21.290 | 1.00 92.03 |
| atom | 1156 | O | PRO | 149 | 14.706 | 9.563 | 22.184 | 1.00 92.21 |
| atom | 1157 | N | GLU | 150 | 12.624 | 9.089 | 21.434 | 1.00 93.97 |
| atom | 1158 | CA | GLU | 150 | 11.908 | 9.290 | 22.678 | 1.00 95.87 |
| atom | 1159 | CB | GLU | 150 | 12.607 | 10.394 | 23.458 | 1.00 98.31 |
| atom | 1160 | CG | GLU | 150 | 12.283 | 10.605 | 24.909 | 1.00100.68 |
| atom | 1161 | CD | GLU | 150 | 13.046 | 11.784 | 25.491 | 1.00101.94 |
| atom | 1162 | OE1 | GLU | 150 | 14.100 | 12.163 | 24.936 | 1.00103.08 |
| atom | 1163 | OE2 | GLU | 150 | 12.586 | 12.335 | 26.512 | 1.00102.49 |
| atom | 1164 | C | GLU | 150 | 10.551 | 9.839 | 22.214 | 1.00 96.14 |
| atom | 1165 | O | GLU | 150 | 10.269 | 9.938 | 21.018 | 1.00 96.34 |
| atom | 1166 | N | LYS | 151 | 9.798 | 10.273 | 23.210 | 1.00 96.11 |
| atom | 1167 | CA | LYS | 151 | 8.518 | 10.915 | 22.920 | 1.00 95.79 |
| atom | 1168 | CB | LYS | 151 | 7.837 | 11.311 | 24.227 | 1.00 97.99 |
| atom | 1169 | CG | LYS | 151 | 7.587 | 10.140 | 25.172 | 1.00100.01 |
| atom | 1170 | CD | LYS | 151 | 7.001 | 10.581 | 26.502 | 1.00101.48 |
| atom | 1171 | CE | LYS | 151 | 6.709 | 9.420 | 27.440 | 1.00102.17 |
| atom | 1172 | NZ | LYS | 151 | 6.204 | 9.887 | 28.765 | 1.00102.71 |
| atom | 1173 | C | LYS | 151 | 8.818 | 12.102 | 22.007 | 1.00 94.58 |
| atom | 1174 | O | LYS | 151 | 9.853 | 12.756 | 22.141 | 1.00 94.58 |
| atom | 1175 | N | GLY | 152 | 7.952 | 12.315 | 21.024 | 1.00 93.28 |
| atom | 1176 | CA | GLY | 152 | 8.065 | 13.398 | 20.078 | 1.00 90.94 |
| atom | 1177 | C | GLY | 152 | 8.896 | 13.133 | 18.835 | 1.00 89.22 |
| atom | 1178 | O | GLY | 152 | 9.680 | 12.191 | 18.725 | 1.00 89.83 |
| atom | 1179 | N | GLY | 153 | 8.705 | 14.031 | 17.874 | 1.00 86.68 |
| atom | 1180 | CA | GLY | 153 | 9.424 | 14.027 | 16.603 | 1.00 82.74 |
| atom | 1181 | C | GLY | 153 | 9.381 | 15.475 | 16.105 | 1.00 80.15 |
| atom | 1182 | O | GLY | 153 | 8.766 | 15.739 | 15.072 | 1.00 80.63 |
| atom | 1183 | N | ARG | 154 | 9.948 | 16.394 | 16.873 | 1.00 76.37 |
| atom | 1184 | CA | ARG | 154 | 10.009 | 17.805 | 16.578 | 1.00 71.79 |

| atom | 1185 | CB | ARG | 154 | 9.312 | 18.235 | 15.288 | 1.00 | 72.12 |
| atom | 1186 | CG | ARG | 154 | 10.132 | 18.196 | 14.015 | 1.00 | 72.47 |
| atom | 1187 | CD | ARG | 154 | 9.313 | 17.691 | 12.848 | 1.00 | 72.65 |
| atom | 1188 | NE | ARG | 154 | 10.077 | 17.491 | 11.629 | 1.00 | 72.61 |
| atom | 1189 | CZ | ARG | 154 | 10.332 | 18.435 | 10.730 | 1.00 | 72.95 |
| atom | 1190 | NH1 | ARG | 154 | 9.897 | 19.679 | 10.885 | 1.00 | 72.44 |
| atom | 1191 | NH2 | ARG | 154 | 11.033 | 18.166 | 9.635 | 1.00 | 73.93 |
| atom | 1192 | C | ARG | 154 | 9.446 | 18.681 | 17.702 | 1.00 | 68.33 |
| atom | 1193 | O | ARG | 154 | 8.271 | 18.596 | 18.046 | 1.00 | 68.32 |
| atom | 1194 | N | LYS | 155 | 10.310 | 19.549 | 18.221 | 1.00 | 64.59 |
| atom | 1195 | CA | LYS | 155 | 9.865 | 20.505 | 19.240 | 1.00 | 60.19 |
| atom | 1196 | CB | LYS | 155 | 10.800 | 20.618 | 20.424 | 1.00 | 60.37 |
| atom | 1197 | CG | LYS | 155 | 12.220 | 21.008 | 20.070 | 1.00 | 60.21 |
| atom | 1198 | CD | LYS | 155 | 13.191 | 20.328 | 21.039 | 1.00 | 61.19 |
| atom | 1199 | CE | LYS | 155 | 14.606 | 20.465 | 20.485 | 1.00 | 62.47 |
| atom | 1200 | NZ | LYS | 155 | 14.555 | 20.269 | 19.003 | 1.00 | 63.83 |
| atom | 1201 | C | LYS | 155 | 9.660 | 21.848 | 18.536 | 1.00 | 56.40 |
| atom | 1202 | O | LYS | 155 | 10.225 | 22.133 | 17.480 | 1.00 | 56.09 |
| atom | 1203 | N | PRO | 156 | 8.786 | 22.662 | 19.103 | 1.00 | 53.47 |
| atom | 1204 | CD | PRO | 156 | 8.018 | 22.421 | 20.337 | 1.00 | 52.46 |
| atom | 1205 | CA | PRO | 156 | 8.450 | 23.938 | 18.504 | 1.00 | 52.10 |
| atom | 1206 | CB | PRO | 156 | 7.105 | 24.296 | 19.106 | 1.00 | 51.66 |
| atom | 1207 | CG | PRO | 156 | 6.988 | 23.512 | 20.352 | 1.00 | 52.02 |
| atom | 1208 | C | PRO | 156 | 9.481 | 25.033 | 18.726 | 1.00 | 50.59 |
| atom | 1209 | O | PRO | 156 | 10.326 | 24.988 | 19.614 | 1.00 | 49.69 |
| atom | 1210 | N | ALA | 157 | 9.395 | 26.013 | 17.819 | 1.00 | 48.97 |
| atom | 1211 | CA | ALA | 157 | 10.264 | 27.173 | 17.889 | 1.00 | 47.02 |
| atom | 1212 | CB | ALA | 157 | 9.804 | 28.257 | 16.918 | 1.00 | 44.00 |
| atom | 1213 | C | ALA | 157 | 10.247 | 27.744 | 19.307 | 1.00 | 45.89 |
| atom | 1214 | O | ALA | 157 | 9.200 | 27.794 | 19.955 | 1.00 | 44.79 |
| atom | 1215 | N | ARG | 158 | 11.423 | 28.161 | 19.745 | 1.00 | 45.07 |
| atom | 1216 | CA | ARG | 158 | 11.535 | 28.855 | 21.025 | 1.00 | 45.21 |
| atom | 1217 | CB | ARG | 158 | 12.968 | 28.810 | 21.537 | 1.00 | 51.46 |
| atom | 1218 | CG | ARG | 158 | 13.385 | 29.911 | 22.500 | 1.00 | 58.97 |
| atom | 1219 | CD | ARG | 158 | 12.526 | 29.950 | 23.748 | 1.00 | 64.01 |
| atom | 1220 | NE | ARG | 158 | 12.826 | 31.007 | 24.715 | 1.00 | 67.26 |

177

| atom | 1221 | CZ  | ARG | 158 | 11.928 | 31.343 | 25.655 | 1.00 | 71.12 |
|------|------|-----|-----|-----|--------|--------|--------|------|-------|
| atom | 1222 | NH1 | ARG | 158 | 10.739 | 30.728 | 25.663 | 1.00 | 72.84 |
| atom | 1223 | NH2 | ARG | 158 | 12.170 | 32.295 | 26.554 | 1.00 | 70.70 |
| atom | 1224 | C   | ARG | 158 | 11.057 | 30.286 | 20.764 | 1.00 | 43.32 |
| atom | 1225 | O   | ARG | 158 | 11.200 | 30.832 | 19.658 | 1.00 | 44.12 |
| atom | 1226 | N   | LEU | 159 | 10.445 | 30.916 | 21.737 | 1.00 | 41.55 |
| atom | 1227 | CA  | LEU | 159 | 9.974  | 32.291 | 21.595 | 1.00 | 40.40 |
| atom | 1228 | CB  | LEU | 159 | 8.595  | 32.429 | 22.256 | 1.00 | 36.54 |
| atom | 1229 | CG  | LEU | 159 | 7.489  | 31.561 | 21.657 | 1.00 | 35.48 |
| atom | 1230 | CD1 | LEU | 159 | 6.182  | 31.777 | 22.401 | 1.00 | 34.89 |
| atom | 1231 | CD2 | LEU | 159 | 7.323  | 31.849 | 20.164 | 1.00 | 33.42 |
| atom | 1232 | C   | LEU | 159 | 10.944 | 33.251 | 22.263 | 1.00 | 39.74 |
| atom | 1233 | O   | LEU | 159 | 11.415 | 32.940 | 23.358 | 1.00 | 40.34 |
| atom | 1234 | N   | ILE | 160 | 11.292 | 34.373 | 21.643 | 1.00 | 39.04 |
| atom | 1235 | CA  | ILE | 160 | 12.170 | 35.355 | 22.261 | 1.00 | 36.86 |
| atom | 1236 | CB  | ILE | 160 | 13.505 | 35.529 | 21.530 | 1.00 | 39.67 |
| atom | 1237 | CG2 | ILE | 160 | 14.326 | 34.244 | 21.530 | 1.00 | 40.57 |
| atom | 1238 | CG1 | ILE | 160 | 13.282 | 36.013 | 20.099 | 1.00 | 40.36 |
| atom | 1239 | CD1 | ILE | 160 | 14.542 | 36.535 | 19.428 | 1.00 | 39.21 |
| atom | 1240 | C   | ILE | 160 | 11.483 | 36.716 | 22.365 | 1.00 | 35.47 |
| atom | 1241 | O   | ILE | 160 | 10.699 | 37.106 | 21.493 | 1.00 | 33.81 |
| atom | 1242 | N   | VAL | 161 | 11.696 | 37.401 | 23.505 | 1.00 | 34.23 |
| atom | 1243 | CA  | VAL | 161 | 11.102 | 38.720 | 23.688 | 1.00 | 33.16 |
| atom | 1244 | CB  | VAL | 161 | 9.836  | 38.801 | 24.546 | 1.00 | 32.23 |
| atom | 1245 | CG1 | VAL | 161 | 9.191  | 37.458 | 24.831 | 1.00 | 29.69 |
| atom | 1246 | CG2 | VAL | 161 | 9.960  | 39.632 | 25.802 | 1.00 | 32.34 |
| atom | 1247 | C   | VAL | 161 | 12.187 | 39.706 | 24.110 | 1.00 | 32.49 |
| atom | 1248 | O   | VAL | 161 | 12.910 | 39.410 | 25.068 | 1.00 | 32.88 |
| atom | 1249 | N   | PHE | 162 | 12.314 | 40.823 | 23.405 | 1.00 | 30.58 |
| atom | 1250 | CA  | PHE | 162 | 13.368 | 41.769 | 23.719 | 1.00 | 31.04 |
| atom | 1251 | CB  | PHE | 162 | 14.579 | 41.504 | 22.791 | 1.00 | 26.66 |
| atom | 1252 | CG  | PHE | 162 | 14.245 | 41.472 | 21.320 | 1.00 | 23.68 |
| atom | 1253 | CD1 | PHE | 162 | 14.367 | 42.580 | 20.517 | 1.00 | 24.34 |
| atom | 1254 | CD2 | PHE | 162 | 13.794 | 40.302 | 20.731 | 1.00 | 23.64 |
| atom | 1255 | CE1 | PHE | 162 | 14.056 | 42.541 | 19.164 | 1.00 | 25.52 |
| atom | 1256 | CE2 | PHE | 162 | 13.465 | 40.247 | 19.391 | 1.00 | 25.47 |

| atom | 1257 | CZ  | PHE | 162 | 13.603 | 41.365 | 18.587 | 1.00 | 24.73 |
| atom | 1258 | C   | PHE | 162 | 12.994 | 43.240 | 23.544 | 1.00 | 31.53 |
| atom | 1259 | O   | PHE | 162 | 12.274 | 43.575 | 22.601 | 1.00 | 30.99 |
| atom | 1260 | N   | PRO | 163 | 13.588 | 44.072 | 24.381 | 1.00 | 31.60 |
| atom | 1261 | CD  | PRO | 163 | 14.481 | 43.695 | 25.497 | 1.00 | 32.29 |
| atom | 1262 | CA  | PRO | 163 | 13.428 | 45.519 | 24.275 | 1.00 | 32.66 |
| atom | 1263 | CB  | PRO | 163 | 14.046 | 46.038 | 25.568 | 1.00 | 32.36 |
| atom | 1264 | CG  | PRO | 163 | 15.033 | 45.004 | 25.984 | 1.00 | 31.84 |
| atom | 1265 | C   | PRO | 163 | 14.156 | 45.971 | 23.014 | 1.00 | 32.16 |
| atom | 1266 | O   | PRO | 163 | 14.844 | 45.203 | 22.325 | 1.00 | 33.48 |
| atom | 1267 | N   | ASP | 164 | 14.025 | 47.229 | 22.664 | 1.00 | 31.19 |
| atom | 1268 | CA  | ASP | 164 | 14.670 | 47.819 | 21.490 | 1.00 | 30.65 |
| atom | 1269 | CB  | ASP | 164 | 13.891 | 49.090 | 21.128 | 1.00 | 30.44 |
| atom | 1270 | CG  | ASP | 164 | 14.457 | 49.871 | 19.959 | 1.00 | 32.84 |
| atom | 1271 | OD1 | ASP | 164 | 15.167 | 50.886 | 20.174 | 1.00 | 31.69 |
| atom | 1272 | OD2 | ASP | 164 | 14.225 | 49.527 | 18.770 | 1.00 | 31.65 |
| atom | 1273 | C   | ASP | 164 | 16.135 | 48.166 | 21.770 | 1.00 | 29.41 |
| atom | 1274 | O   | ASP | 164 | 16.509 | 48.413 | 22.917 | 1.00 | 27.20 |
| atom | 1275 | N   | LEU | 165 | 16.954 | 48.236 | 20.729 | 1.00 | 28.63 |
| atom | 1276 | CA  | LEU | 165 | 18.369 | 48.527 | 20.841 | 1.00 | 28.79 |
| atom | 1277 | CB  | LEU | 165 | 18.934 | 48.938 | 19.468 | 1.00 | 28.92 |
| atom | 1278 | CG  | LEU | 165 | 20.333 | 48.448 | 19.106 | 1.00 | 30.49 |
| atom | 1279 | CD1 | LEU | 165 | 20.931 | 49.284 | 17.985 | 1.00 | 27.44 |
| atom | 1280 | CD2 | LEU | 165 | 21.276 | 48.379 | 20.294 | 1.00 | 29.85 |
| atom | 1281 | C   | LEU | 165 | 18.705 | 49.616 | 21.858 | 1.00 | 28.59 |
| atom | 1282 | O   | LEU | 165 | 19.543 | 49.397 | 22.735 | 1.00 | 28.47 |
| atom | 1283 | N   | GLY | 166 | 18.099 | 50.796 | 21.765 | 1.00 | 27.36 |
| atom | 1284 | CA  | GLY | 166 | 18.369 | 51.919 | 22.621 | 1.00 | 26.57 |
| atom | 1285 | C   | GLY | 166 | 18.282 | 51.536 | 24.089 | 1.00 | 27.07 |
| atom | 1286 | O   | GLY | 166 | 19.186 | 51.846 | 24.871 | 1.00 | 27.43 |
| atom | 1287 | N   | VAL | 167 | 17.191 | 50.882 | 24.458 | 1.00 | 26.12 |
| atom | 1288 | CA  | VAL | 167 | 16.991 | 50.372 | 25.800 | 1.00 | 25.77 |
| atom | 1289 | CB  | VAL | 167 | 15.586 | 49.747 | 25.923 | 1.00 | 23.29 |
| atom | 1290 | CG1 | VAL | 167 | 15.381 | 49.080 | 27.273 | 1.00 | 18.96 |
| atom | 1291 | CG2 | VAL | 167 | 14.511 | 50.795 | 25.658 | 1.00 | 21.24 |
| atom | 1292 | C   | VAL | 167 | 18.025 | 49.311 | 26.182 | 1.00 | 26.68 |

| atom | 1293 | O | VAL | 167 | 18.403 | 49.248 | 27.360 | 1.00 26.72 |
|------|------|-----|-----|-----|--------|--------|--------|-----------|
| atom | 1294 | N | ARG | 168 | 18.489 | 48.469 | 25.269 | 1.00 26.68 |
| atom | 1295 | CA | ARG | 168 | 19.459 | 47.418 | 25.582 | 1.00 27.27 |
| atom | 1296 | CB | ARG | 168 | 19.694 | 46.417 | 24.448 | 1.00 24.53 |
| atom | 1297 | CG | ARG | 168 | 18.436 | 45.773 | 23.878 | 1.00 24.37 |
| atom | 1298 | CD | ARG | 168 | 18.678 | 44.377 | 23.265 | 1.00 22.09 |
| atom | 1299 | NE | ARG | 168 | 19.321 | 44.543 | 21.942 | 1.00 23.63 |
| atom | 1300 | CZ | ARG | 168 | 18.685 | 44.833 | 20.820 | 1.00 23.07 |
| atom | 1301 | NH1 | ARG | 168 | 17.356 | 44.984 | 20.793 | 1.00 24.10 |
| atom | 1302 | NH2 | ARG | 168 | 19.351 | 44.959 | 19.682 | 1.00 23.94 |
| atom | 1303 | C | ARG | 168 | 20.785 | 48.051 | 25.991 | 1.00 27.36 |
| atom | 1304 | O | ARG | 168 | 21.415 | 47.555 | 26.918 | 1.00 28.72 |
| atom | 1305 | N | VAL | 169 | 21.190 | 49.164 | 25.384 | 1.00 27.03 |
| atom | 1306 | CA | VAL | 169 | 22.425 | 49.838 | 25.775 | 1.00 25.89 |
| atom | 1307 | CB | VAL | 169 | 22.819 | 50.911 | 24.752 | 1.00 26.20 |
| atom | 1308 | CG1 | VAL | 169 | 24.169 | 51.525 | 25.122 | 1.00 24.47 |
| atom | 1309 | CG2 | VAL | 169 | 22.843 | 50.365 | 23.331 | 1.00 23.69 |
| atom | 1310 | C | VAL | 169 | 22.258 | 50.483 | 27.153 | 1.00 26.25 |
| atom | 1311 | O | VAL | 169 | 23.159 | 50.519 | 27.990 | 1.00 25.80 |
| atom | 1312 | N | CYS | 170 | 21.066 | 51.008 | 27.432 | 1.00 25.63 |
| atom | 1313 | CA | CYS | 170 | 20.711 | 51.618 | 28.706 | 1.00 25.33 |
| atom | 1314 | CB | CYS | 170 | 19.366 | 52.337 | 28.638 | 1.00 23.90 |
| atom | 1315 | SG | CYS | 170 | 19.372 | 53.920 | 27.742 | 1.00 21.71 |
| atom | 1316 | C | CYS | 170 | 20.744 | 50.562 | 29.811 | 1.00 25.08 |
| atom | 1317 | O | CYS | 170 | 21.176 | 50.841 | 30.931 | 1.00 25.55 |
| atom | 1318 | N | GLU | 171 | 20.373 | 49.325 | 29.470 | 1.00 23.47 |
| atom | 1319 | CA | GLU | 171 | 20.443 | 48.220 | 30.404 | 1.00 23.16 |
| atom | 1320 | CB | GLU | 171 | 19.915 | 46.927 | 29.791 | 1.00 25.03 |
| atom | 1321 | CG | GLU | 171 | 18.383 | 46.881 | 29.769 | 1.00 26.53 |
| atom | 1322 | CD | GLU | 171 | 17.964 | 45.479 | 29.333 | 1.00 28.34 |
| atom | 1323 | OE1 | GLU | 171 | 17.861 | 44.601 | 30.227 | 1.00 26.99 |
| atom | 1324 | OE2 | GLU | 171 | 17.786 | 45.359 | 28.099 | 1.00 26.26 |
| atom | 1325 | C | GLU | 171 | 21.906 | 48.035 | 30.798 | 1.00 22.69 |
| atom | 1326 | O | GLU | 171 | 22.213 | 47.945 | 31.980 | 1.00 22.79 |
| atom | 1327 | N | LYS | 172 | 22.822 | 48.079 | 29.832 | 1.00 22.81 |
| atom | 1328 | CA | LYS | 172 | 24.239 | 47.997 | 30.175 | 1.00 23.43 |

| atom | 1329 | CB | LYS | 172 | 25.134 | 47.915 | 28.931 | 1.00 | 21.94 |
|------|------|-----|-----|-----|--------|--------|--------|------|-------|
| atom | 1330 | CG | LYS | 172 | 24.938 | 46.608 | 28.184 | 1.00 | 22.99 |
| atom | 1331 | CD | LYS | 172 | 25.926 | 46.493 | 27.035 | 1.00 | 24.09 |
| atom | 1332 | CE | LYS | 172 | 25.965 | 45.052 | 26.533 | 1.00 | 24.99 |
| atom | 1333 | NZ | LYS | 172 | 24.617 | 44.633 | 26.029 | 1.00 | 23.60 |
| atom | 1334 | C | LYS | 172 | 24.687 | 49.160 | 31.060 | 1.00 | 23.07 |
| atom | 1335 | O | LYS | 172 | 25.380 | 48.926 | 32.067 | 1.00 | 23.65 |
| atom | 1336 | N | MET | 173 | 24.264 | 50.385 | 30.784 | 1.00 | 22.15 |
| atom | 1337 | CA | MET | 173 | 24.716 | 51.497 | 31.615 | 1.00 | 24.27 |
| atom | 1338 | CB | MET | 173 | 24.113 | 52.855 | 31.234 | 1.00 | 22.37 |
| atom | 1339 | CG | MET | 173 | 24.435 | 53.191 | 29.782 | 1.00 | 23.33 |
| atom | 1340 | SD | MET | 173 | 23.491 | 54.657 | 29.266 | 1.00 | 22.78 |
| atom | 1341 | CE | MET | 173 | 22.705 | 54.026 | 27.846 | 1.00 | 25.17 |
| atom | 1342 | C | MET | 173 | 24.436 | 51.264 | 33.099 | 1.00 | 23.38 |
| atom | 1343 | O | MET | 173 | 25.306 | 51.526 | 33.920 | 1.00 | 22.63 |
| atom | 1344 | N | ALA | 174 | 23.221 | 50.823 | 33.370 | 1.00 | 24.08 |
| atom | 1345 | CA | ALA | 174 | 22.776 | 50.595 | 34.734 | 1.00 | 24.36 |
| atom | 1346 | CB | ALA | 174 | 21.223 | 50.495 | 34.710 | 1.00 | 23.43 |
| atom | 1347 | C | ALA | 174 | 23.248 | 49.308 | 35.376 | 1.00 | 23.71 |
| atom | 1348 | O | ALA | 174 | 23.348 | 49.307 | 36.603 | 1.00 | 23.27 |
| atom | 1349 | N | LEU | 175 | 23.351 | 48.214 | 34.610 | 1.00 | 23.24 |
| atom | 1350 | CA | LEU | 175 | 23.558 | 46.910 | 35.243 | 1.00 | 23.43 |
| atom | 1351 | CB | LEU | 175 | 22.235 | 46.101 | 35.090 | 1.00 | 19.85 |
| atom | 1352 | CG | LEU | 175 | 21.082 | 46.571 | 35.997 | 1.00 | 21.30 |
| atom | 1353 | CD1 | LEU | 175 | 19.722 | 46.446 | 35.303 | 1.00 | 20.92 |
| atom | 1354 | CD2 | LEU | 175 | 21.062 | 45.802 | 37.315 | 1.00 | 17.62 |
| atom | 1355 | C | LEU | 175 | 24.706 | 46.086 | 34.712 | 1.00 | 23.71 |
| atom | 1356 | O | LEU | 175 | 24.918 | 44.986 | 35.242 | 1.00 | 22.87 |
| atom | 1357 | N | TYR | 176 | 25.428 | 46.587 | 33.699 | 1.00 | 23.85 |
| atom | 1358 | CA | TYR | 176 | 26.537 | 45.793 | 33.196 | 1.00 | 24.76 |
| atom | 1359 | CB | TYR | 176 | 27.396 | 46.517 | 32.164 | 1.00 | 24.24 |
| atom | 1360 | CG | TYR | 176 | 28.469 | 45.609 | 31.604 | 1.00 | 23.70 |
| atom | 1361 | CD1 | TYR | 176 | 28.192 | 44.765 | 30.542 | 1.00 | 25.39 |
| atom | 1362 | CE1 | TYR | 176 | 29.151 | 43.921 | 30.004 | 1.00 | 24.59 |
| atom | 1363 | CD2 | TYR | 176 | 29.731 | 45.570 | 32.141 | 1.00 | 24.42 |
| atom | 1364 | CE2 | TYR | 176 | 30.716 | 44.743 | 31.627 | 1.00 | 25.18 |

| atom | 1365 | CZ | TYR | 176 | 30.420 | 43.930 | 30.553 | 1.00 | 25.15 |
| atom | 1366 | OH | TYR | 176 | 31.394 | 43.109 | 30.035 | 1.00 | 23.81 |
| atom | 1367 | C | TYR | 176 | 27.467 | 45.373 | 34.333 | 1.00 | 25.17 |
| atom | 1368 | O | TYR | 176 | 27.849 | 44.222 | 34.486 | 1.00 | 26.16 |
| atom | 1369 | N | ASP | 177 | 27.872 | 46.342 | 35.124 | 1.00 | 26.14 |
| atom | 1370 | CA | ASP | 177 | 28.813 | 46.177 | 36.210 | 1.00 | 28.16 |
| atom | 1371 | CB | ASP | 177 | 29.289 | 47.582 | 36.622 | 1.00 | 26.85 |
| atom | 1372 | CG | ASP | 177 | 30.519 | 47.463 | 37.503 | 1.00 | 29.03 |
| atom | 1373 | OD1 | ASP | 177 | 31.468 | 46.776 | 37.086 | 1.00 | 27.87 |
| atom | 1374 | OD2 | ASP | 177 | 30.497 | 48.066 | 38.592 | 1.00 | 30.70 |
| atom | 1375 | C | ASP | 177 | 28.267 | 45.371 | 37.374 | 1.00 | 28.55 |
| atom | 1376 | O | ASP | 177 | 28.995 | 44.663 | 38.067 | 1.00 | 29.26 |
| atom | 1377 | N | VAL | 178 | 26.965 | 45.383 | 37.575 | 1.00 | 29.44 |
| atom | 1378 | CA | VAL | 178 | 26.290 | 44.615 | 38.615 | 1.00 | 30.24 |
| atom | 1379 | CB | VAL | 178 | 24.848 | 45.147 | 38.771 | 1.00 | 28.07 |
| atom | 1380 | CG1 | VAL | 178 | 23.930 | 44.151 | 39.465 | 1.00 | 25.12 |
| atom | 1381 | CG2 | VAL | 178 | 24.946 | 46.473 | 39.515 | 1.00 | 26.85 |
| atom | 1382 | C | VAL | 178 | 26.220 | 43.147 | 38.222 | 1.00 | 31.80 |
| atom | 1383 | O | VAL | 178 | 26.691 | 42.196 | 38.838 | 1.00 | 30.97 |
| atom | 1384 | N | VAL | 179 | 25.654 | 42.968 | 37.035 | 1.00 | 32.63 |
| atom | 1385 | CA | VAL | 179 | 25.458 | 41.663 | 36.402 | 1.00 | 34.98 |
| atom | 1386 | CB | VAL | 179 | 24.669 | 41.979 | 35.115 | 1.00 | 33.16 |
| atom | 1387 | CG1 | VAL | 179 | 25.225 | 41.365 | 33.861 | 1.00 | 27.98 |
| atom | 1388 | CG2 | VAL | 179 | 23.197 | 41.679 | 35.380 | 1.00 | 30.80 |
| atom | 1389 | C | VAL | 179 | 26.784 | 40.958 | 36.239 | 1.00 | 37.79 |
| atom | 1390 | O | VAL | 179 | 26.905 | 39.726 | 36.240 | 1.00 | 38.71 |
| atom | 1391 | N | SER | 180 | 27.853 | 41.728 | 36.082 | 1.00 | 39.59 |
| atom | 1392 | CA | SER | 180 | 29.209 | 41.301 | 35.932 | 1.00 | 42.67 |
| atom | 1393 | CB | SER | 180 | 30.041 | 42.560 | 35.605 | 1.00 | 44.28 |
| atom | 1394 | OG | SER | 180 | 31.440 | 42.346 | 35.663 | 1.00 | 43.59 |
| atom | 1395 | C | SER | 180 | 29.899 | 40.683 | 37.136 | 1.00 | 43.76 |
| atom | 1396 | O | SER | 180 | 30.746 | 39.807 | 36.923 | 1.00 | 45.63 |
| atom | 1397 | N | THR | 181 | 29.714 | 41.212 | 38.337 | 1.00 | 43.21 |
| atom | 1398 | CA | THR | 181 | 30.421 | 40.760 | 39.520 | 1.00 | 42.53 |
| atom | 1399 | CB | THR | 181 | 31.307 | 41.936 | 39.999 | 1.00 | 43.48 |
| atom | 1400 | OG1 | THR | 181 | 30.405 | 42.959 | 40.439 | 1.00 | 46.39 |

| atom | 1401 | CG2 | THR | 181 | 32.147 | 42.584 | 38.913 | 1.00 | 44.93 |
|------|------|-----|-----|-----|--------|--------|--------|------|-------|
| atom | 1402 | C | THR | 181 | 29.603 | 40.332 | 40.727 | 1.00 | 41.82 |
| atom | 1403 | O | THR | 181 | 30.134 | 39.633 | 41.595 | 1.00 | 42.60 |
| atom | 1404 | N | LEU | 182 | 28.324 | 40.646 | 40.873 | 1.00 | 40.33 |
| atom | 1405 | CA | LEU | 182 | 27.491 | 40.267 | 42.004 | 1.00 | 38.76 |
| atom | 1406 | CB | LEU | 182 | 26.196 | 41.084 | 41.944 | 1.00 | 39.19 |
| atom | 1407 | CG | LEU | 182 | 25.008 | 40.615 | 42.779 | 1.00 | 40.58 |
| atom | 1408 | CD1 | LEU | 182 | 25.205 | 40.932 | 44.251 | 1.00 | 39.89 |
| atom | 1409 | CD2 | LEU | 182 | 23.724 | 41.261 | 42.272 | 1.00 | 41.35 |
| atom | 1410 | C | LEU | 182 | 27.150 | 38.789 | 42.152 | 1.00 | 37.89 |
| atom | 1411 | O | LEU | 182 | 27.169 | 38.239 | 43.266 | 1.00 | 37.41 |
| atom | 1412 | N | PRO | 183 | 26.798 | 38.101 | 41.069 | 1.00 | 35.88 |
| atom | 1413 | CD | PRO | 183 | 26.692 | 38.654 | 39.705 | 1.00 | 35.44 |
| atom | 1414 | CA | PRO | 183 | 26.502 | 36.684 | 41.125 | 1.00 | 35.52 |
| atom | 1415 | CB | PRO | 183 | 26.340 | 36.276 | 39.671 | 1.00 | 35.52 |
| atom | 1416 | CG | PRO | 183 | 26.228 | 37.513 | 38.865 | 1.00 | 35.30 |
| atom | 1417 | C | PRO | 183 | 27.614 | 35.898 | 41.803 | 1.00 | 35.13 |
| atom | 1418 | O | PRO | 183 | 27.413 | 35.154 | 42.756 | 1.00 | 34.50 |
| atom | 1419 | N | GLN | 184 | 28.857 | 36.073 | 41.362 | 1.00 | 35.21 |
| atom | 1420 | CA | GLN | 184 | 30.005 | 35.361 | 41.913 | 1.00 | 34.77 |
| atom | 1421 | CB | GLN | 184 | 31.298 | 35.829 | 41.241 | 1.00 | 38.60 |
| atom | 1422 | CG | GLN | 184 | 32.234 | 34.739 | 40.814 | 1.00 | 43.86 |
| atom | 1423 | CD | GLN | 184 | 33.313 | 34.371 | 41.802 | 1.00 | 48.44 |
| atom | 1424 | OE1 | GLN | 184 | 34.465 | 34.294 | 41.352 | 1.00 | 48.65 |
| atom | 1425 | NE2 | GLN | 184 | 33.029 | 34.140 | 43.081 | 1.00 | 48.92 |
| atom | 1426 | C | GLN | 184 | 30.131 | 35.584 | 43.414 | 1.00 | 32.86 |
| atom | 1427 | O | GLN | 184 | 30.439 | 34.650 | 44.158 | 1.00 | 32.66 |
| atom | 1428 | N | VAL | 185 | 29.934 | 36.819 | 43.853 | 1.00 | 30.61 |
| atom | 1429 | CA | VAL | 185 | 30.054 | 37.125 | 45.278 | 1.00 | 29.54 |
| atom | 1430 | CB | VAL | 185 | 29.968 | 38.647 | 45.532 | 1.00 | 27.09 |
| atom | 1431 | CG1 | VAL | 185 | 29.927 | 38.945 | 47.011 | 1.00 | 25.22 |
| atom | 1432 | CG2 | VAL | 185 | 31.117 | 39.385 | 44.853 | 1.00 | 22.47 |
| atom | 1433 | C | VAL | 185 | 28.972 | 36.443 | 46.099 | 1.00 | 29.28 |
| atom | 1434 | O | VAL | 185 | 29.198 | 36.001 | 47.232 | 1.00 | 29.26 |
| atom | 1435 | N | VAL | 186 | 27.750 | 36.446 | 45.593 | 1.00 | 29.09 |
| atom | 1436 | CA | VAL | 186 | 26.627 | 35.899 | 46.338 | 1.00 | 30.15 |

| atom | 1437 | CB | VAL | 186 | 25.292 | 36.408 | 45.764 | 1.00 | 29.67 |
|------|------|-----|-----|-----|--------|--------|--------|------|-------|
| atom | 1438 | CG1 | VAL | 186 | 24.087 | 35.743 | 46.417 | 1.00 | 30.18 |
| atom | 1439 | CG2 | VAL | 186 | 25.172 | 37.927 | 45.896 | 1.00 | 29.60 |
| atom | 1440 | C | VAL | 186 | 26.653 | 34.372 | 46.307 | 1.00 | 31.70 |
| atom | 1441 | O | VAL | 186 | 26.306 | 33.702 | 47.289 | 1.00 | 31.41 |
| atom | 1442 | N | MET | 187 | 27.047 | 33.820 | 45.164 | 1.00 | 31.70 |
| atom | 1443 | CA | MET | 187 | 26.987 | 32.392 | 44.940 | 1.00 | 33.11 |
| atom | 1444 | CB | MET | 187 | 26.341 | 32.132 | 43.553 | 1.00 | 34.28 |
| atom | 1445 | CG | MET | 187 | 24.885 | 32.605 | 43.541 | 1.00 | 34.57 |
| atom | 1446 | SD | MET | 187 | 24.070 | 32.396 | 41.953 | 1.00 | 33.22 |
| atom | 1447 | CE | MET | 187 | 24.772 | 33.723 | 41.010 | 1.00 | 34.90 |
| atom | 1448 | C | MET | 187 | 28.294 | 31.651 | 45.076 | 1.00 | 33.52 |
| atom | 1449 | O | MET | 187 | 28.264 | 30.416 | 45.172 | 1.00 | 33.72 |
| atom | 1450 | N | GLY | 188 | 29.417 | 32.355 | 45.049 | 1.00 | 33.25 |
| atom | 1451 | CA | GLY | 188 | 30.710 | 31.704 | 45.180 | 1.00 | 33.01 |
| atom | 1452 | C | GLY | 188 | 30.957 | 30.673 | 44.100 | 1.00 | 33.89 |
| atom | 1453 | O | GLY | 188 | 30.602 | 30.842 | 42.925 | 1.00 | 34.45 |
| atom | 1454 | N | SER | 189 | 31.559 | 29.535 | 44.451 | 1.00 | 33.10 |
| atom | 1455 | CA | SER | 189 | 31.889 | 28.486 | 43.506 | 1.00 | 33.32 |
| atom | 1456 | CB | SER | 189 | 32.643 | 27.322 | 44.184 | 1.00 | 32.00 |
| atom | 1457 | OG | SER | 189 | 31.794 | 26.657 | 45.098 | 1.00 | 31.26 |
| atom | 1458 | C | SER | 189 | 30.715 | 27.915 | 42.730 | 1.00 | 32.57 |
| atom | 1459 | O | SER | 189 | 30.999 | 27.292 | 41.701 | 1.00 | 32.83 |
| atom | 1460 | N | SER | 190 | 29.471 | 28.099 | 43.135 | 1.00 | 31.96 |
| atom | 1461 | CA | SER | 190 | 28.314 | 27.601 | 42.415 | 1.00 | 31.58 |
| atom | 1462 | CB | SER | 190 | 27.036 | 27.599 | 43.273 | 1.00 | 30.10 |
| atom | 1463 | OG | SER | 190 | 27.233 | 26.799 | 44.423 | 1.00 | 33.24 |
| atom | 1464 | C | SER | 190 | 28.013 | 28.441 | 41.179 | 1.00 | 31.49 |
| atom | 1465 | O | SER | 190 | 27.230 | 27.959 | 40.356 | 1.00 | 31.89 |
| atom | 1466 | N | TYR | 191 | 28.547 | 29.656 | 41.092 | 1.00 | 31.65 |
| atom | 1467 | CA | TYR | 191 | 28.296 | 30.498 | 39.918 | 1.00 | 33.01 |
| atom | 1468 | CB | TYR | 191 | 28.775 | 31.924 | 40.174 | 1.00 | 33.45 |
| atom | 1469 | CG | TYR | 191 | 28.517 | 32.860 | 39.005 | 1.00 | 34.87 |
| atom | 1470 | CD1 | TYR | 191 | 27.229 | 33.068 | 38.514 | 1.00 | 34.40 |
| atom | 1471 | CE1 | TYR | 191 | 27.011 | 33.937 | 37.464 | 1.00 | 34.42 |
| atom | 1472 | CD2 | TYR | 191 | 29.568 | 33.552 | 38.421 | 1.00 | 35.33 |

| atom | 1473 | CE2 | TYR | 191 | 29.349 | 34.413 | 37.362 | 1.00 | 35.36 |
|---|---|---|---|---|---|---|---|---|---|
| atom | 1474 | CZ | TYR | 191 | 28.071 | 34.603 | 36.897 | 1.00 | 35.64 |
| atom | 1475 | OH | TYR | 191 | 27.879 | 35.480 | 35.849 | 1.00 | 38.03 |
| atom | 1476 | C | TYR | 191 | 29.015 | 29.936 | 38.702 | 1.00 | 33.04 |
| atom | 1477 | O | TYR | 191 | 30.230 | 30.109 | 38.583 | 1.00 | 34.05 |
| atom | 1478 | N | GLY | 192 | 28.317 | 29.215 | 37.849 | 1.00 | 33.71 |
| atom | 1479 | CA | GLY | 192 | 28.895 | 28.522 | 36.720 | 1.00 | 35.22 |
| atom | 1480 | C | GLY | 192 | 29.687 | 29.317 | 35.712 | 1.00 | 36.79 |
| atom | 1481 | O | GLY | 192 | 30.744 | 28.854 | 35.272 | 1.00 | 37.39 |
| atom | 1482 | N | PHE | 193 | 29.233 | 30.509 | 35.314 | 1.00 | 37.16 |
| atom | 1483 | CA | PHE | 193 | 29.910 | 31.329 | 34.337 | 1.00 | 37.88 |
| atom | 1484 | CB | PHE | 193 | 29.023 | 32.490 | 33.857 | 1.00 | 40.40 |
| atom | 1485 | CG | PHE | 193 | 27.755 | 32.006 | 33.203 | 1.00 | 43.20 |
| atom | 1486 | CD1 | PHE | 193 | 26.567 | 32.040 | 33.923 | 1.00 | 44.55 |
| atom | 1487 | CD2 | PHE | 193 | 27.741 | 31.552 | 31.898 | 1.00 | 42.25 |
| atom | 1488 | CE1 | PHE | 193 | 25.374 | 31.618 | 33.361 | 1.00 | 43.85 |
| atom | 1489 | CE2 | PHE | 193 | 26.562 | 31.110 | 31.336 | 1.00 | 44.52 |
| atom | 1490 | CZ | PHE | 193 | 25.382 | 31.142 | 32.068 | 1.00 | 45.39 |
| atom | 1491 | C | PHE | 193 | 31.270 | 31.876 | 34.730 | 1.00 | 37.20 |
| atom | 1492 | O | PHE | 193 | 31.959 | 32.352 | 33.824 | 1.00 | 36.82 |
| atom | 1493 | N | GLN | 194 | 31.745 | 31.791 | 35.960 | 1.00 | 37.24 |
| atom | 1494 | CA | GLN | 194 | 33.075 | 32.276 | 36.299 | 1.00 | 37.75 |
| atom | 1495 | CB | GLN | 194 | 33.277 | 32.372 | 37.821 | 1.00 | 37.47 |
| atom | 1496 | CG | GLN | 194 | 33.301 | 31.029 | 38.525 | 1.00 | 37.92 |
| atom | 1497 | CD | GLN | 194 | 33.166 | 31.129 | 40.023 | 1.00 | 37.87 |
| atom | 1498 | OE1 | GLN | 194 | 32.102 | 30.858 | 40.591 | 1.00 | 38.09 |
| atom | 1499 | NE2 | GLN | 194 | 34.251 | 31.522 | 40.682 | 1.00 | 38.15 |
| atom | 1500 | C | GLN | 194 | 34.162 | 31.365 | 35.732 | 1.00 | 38.86 |
| atom | 1501 | O | GLN | 194 | 35.292 | 31.810 | 35.545 | 1.00 | 38.81 |
| atom | 1502 | N | TYR | 195 | 33.847 | 30.104 | 35.466 | 1.00 | 38.48 |
| atom | 1503 | CA | TYR | 195 | 34.793 | 29.130 | 35.012 | 1.00 | 38.71 |
| atom | 1504 | CB | TYR | 195 | 34.303 | 27.708 | 35.416 | 1.00 | 35.34 |
| atom | 1505 | CG | TYR | 195 | 34.044 | 27.521 | 36.885 | 1.00 | 33.34 |
| atom | 1506 | CD1 | TYR | 195 | 32.775 | 27.222 | 37.374 | 1.00 | 30.48 |
| atom | 1507 | CE1 | TYR | 195 | 32.554 | 27.049 | 38.717 | 1.00 | 29.26 |
| atom | 1508 | CD2 | TYR | 195 | 35.081 | 27.627 | 37.807 | 1.00 | 32.84 |

| atom | 1509 | CE2 | TYR | 195 | 34.863 | 27.445 | 39.159 | 1.00 | 31.95 |
|------|------|-----|-----|-----|--------|--------|--------|------|-------|
| atom | 1510 | CZ | TYR | 195 | 33.590 | 27.166 | 39.610 | 1.00 | 31.67 |
| atom | 1511 | OH | TYR | 195 | 33.406 | 27.020 | 40.976 | 1.00 | 32.50 |
| atom | 1512 | C | TYR | 195 | 35.106 | 28.998 | 33.530 | 1.00 | 39.50 |
| atom | 1513 | O | TYR | 195 | 34.228 | 28.882 | 32.682 | 1.00 | 40.78 |
| atom | 1514 | N | SER | 196 | 36.390 | 28.794 | 33.235 | 1.00 | 40.30 |
| atom | 1515 | CA | SER | 196 | 36.785 | 28.421 | 31.878 | 1.00 | 42.95 |
| atom | 1516 | CB | SER | 196 | 38.289 | 28.496 | 31.722 | 1.00 | 43.28 |
| atom | 1517 | OG | SER | 196 | 38.930 | 27.683 | 32.697 | 1.00 | 42.81 |
| atom | 1518 | C | SER | 196 | 36.310 | 26.970 | 31.745 | 1.00 | 44.87 |
| atom | 1519 | O | SER | 196 | 35.903 | 26.326 | 32.721 | 1.00 | 44.98 |
| atom | 1520 | N | PRO | 197 | 36.398 | 26.395 | 30.562 | 1.00 | 46.04 |
| atom | 1521 | CD | PRO | 197 | 36.875 | 27.042 | 29.313 | 1.00 | 46.52 |
| atom | 1522 | CA | PRO | 197 | 35.950 | 25.027 | 30.348 | 1.00 | 46.02 |
| atom | 1523 | CB | PRO | 197 | 36.109 | 24.806 | 28.855 | 1.00 | 46.57 |
| atom | 1524 | CG | PRO | 197 | 37.048 | 25.861 | 28.384 | 1.00 | 46.56 |
| atom | 1525 | C | PRO | 197 | 36.776 | 24.068 | 31.189 | 1.00 | 46.02 |
| atom | 1526 | O | PRO | 197 | 36.233 | 23.187 | 31.857 | 1.00 | 45.86 |
| atom | 1527 | N | GLY | 198 | 38.098 | 24.259 | 31.185 | 1.00 | 44.59 |
| atom | 1528 | CA | GLY | 198 | 39.004 | 23.434 | 31.967 | 1.00 | 42.99 |
| atom | 1529 | C | GLY | 198 | 38.737 | 23.541 | 33.462 | 1.00 | 42.19 |
| atom | 1530 | O | GLY | 198 | 38.824 | 22.572 | 34.206 | 1.00 | 42.13 |
| atom | 1531 | N | GLN | 199 | 38.394 | 24.738 | 33.938 | 1.00 | 41.97 |
| atom | 1532 | CA | GLN | 199 | 38.064 | 25.006 | 35.327 | 1.00 | 40.16 |
| atom | 1533 | CB | GLN | 199 | 38.038 | 26.508 | 35.582 | 1.00 | 39.85 |
| atom | 1534 | CG | GLN | 199 | 39.372 | 27.225 | 35.578 | 1.00 | 39.97 |
| atom | 1535 | CD | GLN | 199 | 39.171 | 28.737 | 35.544 | 1.00 | 41.46 |
| atom | 1536 | OE1 | GLN | 199 | 38.239 | 29.292 | 34.960 | 1.00 | 41.70 |
| atom | 1537 | NE2 | GLN | 199 | 40.058 | 29.463 | 36.209 | 1.00 | 39.98 |
| atom | 1538 | C | GLN | 199 | 36.714 | 24.399 | 35.738 | 1.00 | 38.84 |
| atom | 1539 | O | GLN | 199 | 36.569 | 24.033 | 36.902 | 1.00 | 38.24 |
| atom | 1540 | N | ARG | 200 | 35.756 | 24.288 | 34.827 | 1.00 | 36.96 |
| atom | 1541 | CA | ARG | 200 | 34.477 | 23.669 | 35.129 | 1.00 | 35.75 |
| atom | 1542 | CB | ARG | 200 | 33.457 | 23.895 | 34.026 | 1.00 | 35.60 |
| atom | 1543 | CG | ARG | 200 | 32.217 | 23.019 | 34.106 | 1.00 | 38.15 |
| atom | 1544 | CD | ARG | 200 | 31.285 | 23.372 | 32.955 | 1.00 | 39.79 |

| atom | 1545 | NE | ARG | 200 | 29.891 | 23.069 | 33.221 | 1.00 | 40.42 |
| atom | 1546 | CZ | ARG | 200 | 29.065 | 23.889 | 33.862 | 1.00 | 42.22 |
| atom | 1547 | NH1 | ARG | 200 | 29.452 | 25.066 | 34.337 | 1.00 | 42.11 |
| atom | 1548 | NH2 | ARG | 200 | 27.804 | 23.525 | 34.047 | 1.00 | 42.72 |
| atom | 1549 | C | ARG | 200 | 34.707 | 22.173 | 35.343 | 1.00 | 35.09 |
| atom | 1550 | O | ARG | 200 | 34.343 | 21.624 | 36.389 | 1.00 | 34.41 |
| atom | 1551 | N | VAL | 201 | 35.397 | 21.545 | 34.387 | 1.00 | 34.15 |
| atom | 1552 | CA | VAL | 201 | 35.718 | 20.116 | 34.499 | 1.00 | 33.25 |
| atom | 1553 | CB | VAL | 201 | 36.665 | 19.710 | 33.353 | 1.00 | 33.33 |
| atom | 1554 | CG1 | VAL | 201 | 37.437 | 18.426 | 33.629 | 1.00 | 32.32 |
| atom | 1555 | CG2 | VAL | 201 | 35.844 | 19.539 | 32.076 | 1.00 | 32.58 |
| atom | 1556 | C | VAL | 201 | 36.344 | 19.825 | 35.858 | 1.00 | 32.64 |
| atom | 1557 | O | VAL | 201 | 35.928 | 18.986 | 36.635 | 1.00 | 31.44 |
| atom | 1558 | N | GLU | 202 | 37.390 | 20.574 | 36.197 | 1.00 | 32.74 |
| atom | 1559 | CA | GLU | 202 | 38.090 | 20.471 | 37.461 | 1.00 | 33.49 |
| atom | 1560 | CB | GLU | 202 | 39.183 | 21.527 | 37.526 | 1.00 | 35.52 |
| atom | 1561 | CG | GLU | 202 | 39.768 | 21.794 | 38.900 | 1.00 | 39.42 |
| atom | 1562 | CD | GLU | 202 | 40.952 | 22.738 | 38.763 | 1.00 | 43.89 |
| atom | 1563 | OE1 | GLU | 202 | 42.025 | 22.306 | 38.291 | 1.00 | 46.56 |
| atom | 1564 | OE2 | GLU | 202 | 40.808 | 23.930 | 39.081 | 1.00 | 47.09 |
| atom | 1565 | C | GLU | 202 | 37.156 | 20.635 | 38.656 | 1.00 | 33.68 |
| atom | 1566 | O | GLU | 202 | 37.220 | 19.905 | 39.657 | 1.00 | 33.03 |
| atom | 1567 | N | PHE | 203 | 36.293 | 21.659 | 38.560 | 1.00 | 33.67 |
| atom | 1568 | CA | PHE | 203 | 35.318 | 21.887 | 39.618 | 1.00 | 32.51 |
| atom | 1569 | CB | PHE | 203 | 34.482 | 23.150 | 39.411 | 1.00 | 31.28 |
| atom | 1570 | CG | PHE | 203 | 33.586 | 23.391 | 40.606 | 1.00 | 31.35 |
| atom | 1571 | CD1 | PHE | 203 | 34.129 | 23.626 | 41.857 | 1.00 | 29.80 |
| atom | 1572 | CD2 | PHE | 203 | 32.208 | 23.365 | 40.462 | 1.00 | 30.73 |
| atom | 1573 | CE1 | PHE | 203 | 33.309 | 23.830 | 42.956 | 1.00 | 29.54 |
| atom | 1574 | CE2 | PHE | 203 | 31.382 | 23.593 | 41.553 | 1.00 | 30.11 |
| atom | 1575 | CZ | PHE | 203 | 31.935 | 23.809 | 42.799 | 1.00 | 29.32 |
| atom | 1576 | C | PHE | 203 | 34.402 | 20.668 | 39.793 | 1.00 | 31.59 |
| atom | 1577 | O | PHE | 203 | 34.171 | 20.287 | 40.938 | 1.00 | 30.73 |
| atom | 1578 | N | LEU | 204 | 33.927 | 20.070 | 38.715 | 1.00 | 31.00 |
| atom | 1579 | CA | LEU | 204 | 33.023 | 18.924 | 38.781 | 1.00 | 31.28 |
| atom | 1580 | CB | LEU | 204 | 32.368 | 18.642 | 37.415 | 1.00 | 30.69 |

| atom | 1581 | CG   | LEU | 204 | 31.249 | 19.613 | 37.005 | 1.00 | 32.28 |
|------|------|------|-----|-----|--------|--------|--------|------|-------|
| atom | 1582 | CD1  | LEU | 204 | 30.638 | 19.305 | 35.647 | 1.00 | 30.99 |
| atom | 1583 | CD2  | LEU | 204 | 30.139 | 19.601 | 38.067 | 1.00 | 33.45 |
| atom | 1584 | C    | LEU | 204 | 33.737 | 17.700 | 39.346 | 1.00 | 30.48 |
| atom | 1585 | O    | LEU | 204 | 33.302 | 17.083 | 40.324 | 1.00 | 29.05 |
| atom | 1586 | N    | VAL | 205 | 34.886 | 17.388 | 38.755 | 1.00 | 30.18 |
| atom | 1587 | CA   | VAL | 205 | 35.743 | 16.295 | 39.189 | 1.00 | 30.00 |
| atom | 1588 | CB   | VAL | 205 | 37.011 | 16.186 | 38.313 | 1.00 | 28.83 |
| atom | 1589 | CG1  | VAL | 205 | 37.863 | 15.024 | 38.798 | 1.00 | 29.40 |
| atom | 1590 | CG2  | VAL | 205 | 36.628 | 15.979 | 36.855 | 1.00 | 27.25 |
| atom | 1591 | C    | VAL | 205 | 36.160 | 16.434 | 40.655 | 1.00 | 30.31 |
| atom | 1592 | O    | VAL | 205 | 36.049 | 15.424 | 41.384 | 1.00 | 30.22 |
| atom | 1593 | N    | ASN | 206 | 36.604 | 17.607 | 41.115 | 1.00 | 29.50 |
| atom | 1594 | CA   | ASN | 206 | 37.004 | 17.700 | 42.530 | 1.00 | 30.76 |
| atom | 1595 | CB   | ASN | 206 | 37.862 | 18.916 | 42.845 | 1.00 | 29.42 |
| atom | 1596 | CG   | ASN | 206 | 39.230 | 18.929 | 42.216 | 1.00 | 30.67 |
| atom | 1597 | OD1  | ASN | 206 | 39.792 | 17.884 | 41.892 | 1.00 | 30.70 |
| atom | 1598 | ND2  | ASN | 206 | 39.783 | 20.134 | 42.037 | 1.00 | 30.69 |
| atom | 1599 | C    | ASN | 206 | 35.829 | 17.654 | 43.506 | 1.00 | 30.12 |
| atom | 1600 | O    | ASN | 206 | 35.973 | 17.169 | 44.625 | 1.00 | 30.14 |
| atom | 1601 | N    | THR | 207 | 34.669 | 18.155 | 43.148 | 1.00 | 30.71 |
| atom | 1602 | CA   | THR | 207 | 33.490 | 18.093 | 43.996 | 1.00 | 31.87 |
| atom | 1603 | CB   | THR | 207 | 32.330 | 18.890 | 43.387 | 1.00 | 31.69 |
| atom | 1604 | OG1  | THR | 207 | 32.699 | 20.279 | 43.268 | 1.00 | 33.18 |
| atom | 1605 | CG2  | THR | 207 | 31.089 | 18.755 | 44.242 | 1.00 | 31.99 |
| atom | 1606 | C    | THR | 207 | 33.084 | 16.621 | 44.122 | 1.00 | 33.08 |
| atom | 1607 | O    | THR | 207 | 32.935 | 16.096 | 45.222 | 1.00 | 32.62 |
| atom | 1608 | N    | TRP | 208 | 32.993 | 15.947 | 42.974 | 1.00 | 33.41 |
| atom | 1609 | CA   | TRP | 208 | 32.631 | 14.538 | 42.920 | 1.00 | 33.63 |
| atom | 1610 | CB   | TRP | 208 | 32.650 | 14.041 | 41.467 | 1.00 | 32.15 |
| atom | 1611 | CG   | TRP | 208 | 32.081 | 12.671 | 41.303 | 1.00 | 30.93 |
| atom | 1612 | CD2  | TRP | 208 | 30.699 | 12.318 | 41.228 | 1.00 | 29.25 |
| atom | 1613 | CE2  | TRP | 208 | 30.635 | 10.916 | 41.078 | 1.00 | 31.13 |
| atom | 1614 | CE3  | TRP | 208 | 29.512 | 13.034 | 41.269 | 1.00 | 30.56 |
| atom | 1615 | CD1  | TRP | 208 | 32.787 | 11.499 | 41.181 | 1.00 | 30.68 |
| atom | 1616 | NE1  | TRP | 208 | 31.928 | 10.433 | 41.048 | 1.00 | 30.82 |

| atom | 1617 | CZ2 | TRP | 208 | 29.429 | 10.240 | 40.989 | 1.00 | 30.19 |
|------|------|-----|-----|-----|--------|--------|--------|------|-------|
| atom | 1618 | CZ3 | TRP | 208 | 28.307 | 12.360 | 41.184 | 1.00 | 30.44 |
| atom | 1619 | CH2 | TRP | 208 | 28.269 | 10.973 | 41.038 | 1.00 | 29.20 |
| atom | 1620 | C | TRP | 208 | 33.556 | 13.677 | 43.767 | 1.00 | 33.64 |
| atom | 1621 | O | TRP | 208 | 33.113 | 12.822 | 44.537 | 1.00 | 33.39 |
| atom | 1622 | N | LYS | 209 | 34.854 | 13.926 | 43.680 | 1.00 | 34.34 |
| atom | 1623 | CA | LYS | 209 | 35.842 | 13.151 | 44.419 | 1.00 | 35.55 |
| atom | 1624 | CB | LYS | 209 | 37.211 | 13.214 | 43.739 | 1.00 | 36.79 |
| atom | 1625 | CG | LYS | 209 | 37.255 | 12.334 | 42.481 | 1.00 | 37.29 |
| atom | 1626 | CD | LYS | 209 | 38.405 | 12.740 | 41.595 | 1.00 | 36.78 |
| atom | 1627 | CE | LYS | 209 | 38.723 | 11.703 | 40.528 | 1.00 | 38.01 |
| atom | 1628 | NZ | LYS | 209 | 40.121 | 11.930 | 40.024 | 1.00 | 37.45 |
| atom | 1629 | C | LYS | 209 | 35.946 | 13.494 | 45.893 | 1.00 | 36.04 |
| atom | 1630 | O | LYS | 209 | 36.487 | 12.669 | 46.616 | 1.00 | 35.22 |
| atom | 1631 | N | SER | 210 | 35.403 | 14.606 | 46.349 | 1.00 | 37.38 |
| atom | 1632 | CA | SER | 210 | 35.410 | 14.978 | 47.750 | 1.00 | 38.66 |
| atom | 1633 | CB | SER | 210 | 35.244 | 16.500 | 47.901 | 1.00 | 36.64 |
| atom | 1634 | OG | SER | 210 | 34.006 | 16.903 | 47.368 | 1.00 | 37.31 |
| atom | 1635 | C | SER | 210 | 34.332 | 14.254 | 48.544 | 1.00 | 39.97 |
| atom | 1636 | O | SER | 210 | 34.425 | 14.213 | 49.780 | 1.00 | 41.73 |
| atom | 1637 | N | LYS | 211 | 33.320 | 13.682 | 47.904 | 1.00 | 40.39 |
| atom | 1638 | CA | LYS | 211 | 32.294 | 12.966 | 48.659 | 1.00 | 40.78 |
| atom | 1639 | CB | LYS | 211 | 30.969 | 12.844 | 47.911 | 1.00 | 39.35 |
| atom | 1640 | CG | LYS | 211 | 30.406 | 14.114 | 47.297 | 1.00 | 36.33 |
| atom | 1641 | CD | LYS | 211 | 30.172 | 15.145 | 48.394 | 1.00 | 32.41 |
| atom | 1642 | CE | LYS | 211 | 30.706 | 16.494 | 47.993 | 1.00 | 33.68 |
| atom | 1643 | NZ | LYS | 211 | 29.726 | 17.578 | 47.767 | 1.00 | 30.46 |
| atom | 1644 | C | LYS | 211 | 32.804 | 11.548 | 48.900 | 1.00 | 41.51 |
| atom | 1645 | O | LYS | 211 | 33.529 | 11.042 | 48.035 | 1.00 | 42.27 |
| atom | 1646 | N | LYS | 212 | 32.415 | 10.956 | 50.018 | 1.00 | 42.09 |
| atom | 1647 | CA | LYS | 212 | 32.813 | 9.570 | 50.260 | 1.00 | 42.73 |
| atom | 1648 | CB | LYS | 212 | 32.647 | 9.121 | 51.700 | 1.00 | 47.67 |
| atom | 1649 | CG | LYS | 212 | 33.835 | 9.544 | 52.573 | 1.00 | 52.77 |
| atom | 1650 | CD | LYS | 212 | 33.410 | 10.635 | 53.557 | 1.00 | 55.96 |
| atom | 1651 | CE | LYS | 212 | 34.486 | 11.700 | 53.694 | 1.00 | 56.85 |
| atom | 1652 | NZ | LYS | 212 | 34.562 | 12.538 | 52.446 | 1.00 | 58.01 |

| atom | 1653 | C | LYS | 212 | 32.042 | 8.668 | 49.295 | 1.00 | 41.96 |
|------|------|-----|-----|-----|--------|--------|--------|------|-------|
| atom | 1654 | O | LYS | 212 | 32.661 | 7.799 | 48.693 | 1.00 | 41.86 |
| atom | 1655 | N | ASN | 213 | 30.738 | 8.855 | 49.164 | 1.00 | 40.57 |
| atom | 1656 | CA | ASN | 213 | 29.897 | 8.132 | 48.232 | 1.00 | 40.24 |
| atom | 1657 | CB | ASN | 213 | 28.908 | 7.156 | 48.845 | 1.00 | 43.93 |
| atom | 1658 | CG | ASN | 213 | 29.522 | 5.957 | 49.514 | 1.00 | 46.78 |
| atom | 1659 | OD1 | ASN | 213 | 29.491 | 5.860 | 50.743 | 1.00 | 48.49 |
| atom | 1660 | ND2 | ASN | 213 | 30.085 | 5.028 | 48.751 | 1.00 | 48.81 |
| atom | 1661 | C | ASN | 213 | 29.061 | 9.189 | 47.478 | 1.00 | 38.36 |
| atom | 1662 | O | ASN | 213 | 28.001 | 9.567 | 47.956 | 1.00 | 36.73 |
| atom | 1663 | N | PRO | 214 | 29.593 | 9.663 | 46.357 | 1.00 | 36.90 |
| atom | 1664 | CD | PRO | 214 | 30.897 | 9.244 | 45.766 | 1.00 | 36.04 |
| atom | 1665 | CA | PRO | 214 | 28.961 | 10.704 | 45.586 | 1.00 | 34.83 |
| atom | 1666 | CB | PRO | 214 | 29.999 | 11.069 | 44.528 | 1.00 | 34.47 |
| atom | 1667 | CG | PRO | 214 | 30.818 | 9.848 | 44.372 | 1.00 | 35.67 |
| atom | 1668 | C | PRO | 214 | 27.667 | 10.321 | 44.905 | 1.00 | 33.48 |
| atom | 1669 | O | PRO | 214 | 27.518 | 9.241 | 44.371 | 1.00 | 32.25 |
| atom | 1670 | N | MET | 215 | 26.719 | 11.247 | 44.901 | 1.00 | 32.89 |
| atom | 1671 | CA | MET | 215 | 25.448 | 11.115 | 44.209 | 1.00 | 31.22 |
| atom | 1672 | CB | MET | 215 | 24.242 | 10.733 | 45.053 | 1.00 | 29.27 |
| atom | 1673 | CG | MET | 215 | 22.935 | 10.582 | 44.293 | 1.00 | 25.54 |
| atom | 1674 | SD | MET | 215 | 22.060 | 12.125 | 43.986 | 1.00 | 24.21 |
| atom | 1675 | CE | MET | 215 | 21.271 | 12.381 | 45.589 | 1.00 | 26.77 |
| atom | 1676 | C | MET | 215 | 25.186 | 12.495 | 43.596 | 1.00 | 30.38 |
| atom | 1677 | O | MET | 215 | 25.512 | 13.467 | 44.278 | 1.00 | 30.08 |
| atom | 1678 | N | GLY | 216 | 24.648 | 12.506 | 42.385 | 1.00 | 30.51 |
| atom | 1679 | CA | GLY | 216 | 24.367 | 13.763 | 41.718 | 1.00 | 30.00 |
| atom | 1680 | C | GLY | 216 | 23.233 | 13.688 | 40.710 | 1.00 | 30.39 |
| atom | 1681 | O | GLY | 216 | 22.832 | 12.623 | 40.232 | 1.00 | 28.99 |
| atom | 1682 | N | PHE | 217 | 22.734 | 14.895 | 40.377 | 1.00 | 30.36 |
| atom | 1683 | CA | PHE | 217 | 21.605 | 14.973 | 39.459 | 1.00 | 29.58 |
| atom | 1684 | CB | PHE | 217 | 20.311 | 14.655 | 40.206 | 1.00 | 25.93 |
| atom | 1685 | CG | PHE | 217 | 19.855 | 15.599 | 41.274 | 1.00 | 25.74 |
| atom | 1686 | CD1 | PHE | 217 | 20.243 | 15.387 | 42.587 | 1.00 | 25.74 |
| atom | 1687 | CD2 | PHE | 217 | 19.025 | 16.690 | 41.006 | 1.00 | 24.43 |
| atom | 1688 | CE1 | PHE | 217 | 19.828 | 16.213 | 43.619 | 1.00 | 25.92 |

| atom | 1689 | CE2 | PHE | 217 | 18.611 | 17.534 | 42.020 | 1.00 | 24.72 |
|------|------|-----|-----|-----|--------|--------|--------|------|-------|
| atom | 1690 | CZ | PHE | 217 | 19.000 | 17.291 | 43.327 | 1.00 | 26.23 |
| atom | 1691 | C | PHE | 217 | 21.506 | 16.365 | 38.855 | 1.00 | 30.02 |
| atom | 1692 | O | PHE | 217 | 21.876 | 17.336 | 39.518 | 1.00 | 30.75 |
| atom | 1693 | N | SER | 218 | 20.998 | 16.409 | 37.639 | 1.00 | 29.45 |
| atom | 1694 | CA | SER | 218 | 20.743 | 17.692 | 36.996 | 1.00 | 30.72 |
| atom | 1695 | CB | SER | 218 | 21.100 | 17.649 | 35.513 | 1.00 | 28.55 |
| atom | 1696 | OG | SER | 218 | 20.526 | 16.471 | 34.951 | 1.00 | 31.16 |
| atom | 1697 | C | SER | 218 | 19.243 | 17.920 | 37.218 | 1.00 | 32.13 |
| atom | 1698 | O | SER | 218 | 18.495 | 16.935 | 37.362 | 1.00 | 29.92 |
| atom | 1699 | N | TYR | 219 | 18.856 | 19.199 | 37.306 | 1.00 | 33.57 |
| atom | 1700 | CA | TYR | 219 | 17.433 | 19.485 | 37.502 | 1.00 | 34.36 |
| atom | 1701 | CB | TYR | 219 | 17.185 | 20.246 | 38.813 | 1.00 | 34.43 |
| atom | 1702 | CG | TYR | 219 | 15.699 | 20.396 | 39.089 | 1.00 | 34.58 |
| atom | 1703 | CD1 | TYR | 219 | 14.991 | 19.339 | 39.664 | 1.00 | 34.12 |
| atom | 1704 | CE1 | TYR | 219 | 13.640 | 19.446 | 39.927 | 1.00 | 33.31 |
| atom | 1705 | CD2 | TYR | 219 | 15.010 | 21.556 | 38.772 | 1.00 | 34.04 |
| atom | 1706 | CE2 | TYR | 219 | 13.656 | 21.675 | 39.035 | 1.00 | 33.64 |
| atom | 1707 | CZ | TYR | 219 | 12.979 | 20.616 | 39.609 | 1.00 | 33.80 |
| atom | 1708 | OH | TYR | 219 | 11.627 | 20.743 | 39.855 | 1.00 | 33.27 |
| atom | 1709 | C | TYR | 219 | 16.913 | 20.281 | 36.318 | 1.00 | 34.60 |
| atom | 1710 | O | TYR | 219 | 17.396 | 21.387 | 36.092 | 1.00 | 34.60 |
| atom | 1711 | N | ASP | 220 | 15.964 | 19.741 | 35.584 | 1.00 | 35.98 |
| atom | 1712 | CA | ASP | 220 | 15.389 | 20.432 | 34.427 | 1.00 | 37.31 |
| atom | 1713 | CB | ASP | 220 | 15.172 | 19.412 | 33.306 | 1.00 | 41.24 |
| atom | 1714 | CG | ASP | 220 | 14.571 | 19.997 | 32.050 | 1.00 | 45.42 |
| atom | 1715 | OD1 | ASP | 220 | 14.383 | 19.204 | 31.091 | 1.00 | 48.98 |
| atom | 1716 | OD2 | ASP | 220 | 14.275 | 21.210 | 31.991 | 1.00 | 46.14 |
| atom | 1717 | C | ASP | 220 | 14.059 | 21.097 | 34.761 | 1.00 | 36.86 |
| atom | 1718 | O | ASP | 220 | 13.007 | 20.457 | 34.856 | 1.00 | 37.13 |
| atom | 1719 | N | THR | 221 | 14.092 | 22.404 | 34.944 | 1.00 | 36.41 |
| atom | 1720 | CA | THR | 221 | 12.869 | 23.156 | 35.221 | 1.00 | 36.06 |
| atom | 1721 | CB | THR | 221 | 13.188 | 24.585 | 35.683 | 1.00 | 34.95 |
| atom | 1722 | OG1 | THR | 221 | 13.759 | 24.530 | 36.996 | 1.00 | 34.38 |
| atom | 1723 | CG2 | THR | 221 | 11.946 | 25.463 | 35.720 | 1.00 | 33.22 |
| atom | 1724 | C | THR | 221 | 12.043 | 23.190 | 33.938 | 1.00 | 36.34 |

| atom | 1725 | O   | THR | 221 | 12.625 | 23.405 | 32.875 | 1.00 | 34.88 |
|------|------|-----|-----|-----|--------|--------|--------|------|-------|
| atom | 1726 | N   | ARG | 222 | 10.731 | 22.983 | 34.039 | 1.00 | 36.98 |
| atom | 1727 | CA  | ARG | 222 | 9.884  | 23.060 | 32.844 | 1.00 | 37.57 |
| atom | 1728 | CB  | ARG | 222 | 8.508  | 22.483 | 33.148 | 1.00 | 40.99 |
| atom | 1729 | CG  | ARG | 222 | 7.479  | 22.624 | 32.026 | 1.00 | 45.90 |
| atom | 1730 | CD  | ARG | 222 | 6.087  | 22.255 | 32.521 | 1.00 | 51.30 |
| atom | 1731 | NE  | ARG | 222 | 5.301  | 23.366 | 33.056 | 1.00 | 54.33 |
| atom | 1732 | CZ  | ARG | 222 | 4.534  | 23.272 | 34.147 | 1.00 | 54.54 |
| atom | 1733 | NH1 | ARG | 222 | 4.494  | 22.117 | 34.802 | 1.00 | 53.25 |
| atom | 1734 | NH2 | ARG | 222 | 3.835  | 24.316 | 34.575 | 1.00 | 54.17 |
| atom | 1735 | C   | ARG | 222 | 9.753  | 24.530 | 32.427 | 1.00 | 37.27 |
| atom | 1736 | O   | ARG | 222 | 9.311  | 25.326 | 33.263 | 1.00 | 36.49 |
| atom | 1737 | N   | CYS | 223 | 10.165 | 24.907 | 31.223 | 1.00 | 35.83 |
| atom | 1738 | CA  | CYS | 223 | 10.044 | 26.285 | 30.761 | 1.00 | 35.59 |
| atom | 1739 | CB  | CYS | 223 | 8.614  | 26.536 | 30.244 | 1.00 | 39.03 |
| atom | 1740 | SG  | CYS | 223 | 8.139  | 25.610 | 28.771 | 1.00 | 42.18 |
| atom | 1741 | C   | CYS | 223 | 10.322 | 27.305 | 31.856 | 1.00 | 34.38 |
| atom | 1742 | O   | CYS | 223 | 9.422  | 28.052 | 32.245 | 1.00 | 34.40 |
| atom | 1743 | N   | PHE | 224 | 11.549 | 27.379 | 32.335 | 1.00 | 33.26 |
| atom | 1744 | CA  | PHE | 224 | 11.987 | 28.195 | 33.439 | 1.00 | 31.12 |
| atom | 1745 | CB  | PHE | 224 | 13.508 | 28.080 | 33.648 | 1.00 | 28.63 |
| atom | 1746 | CG  | PHE | 224 | 14.020 | 28.724 | 34.906 | 1.00 | 27.69 |
| atom | 1747 | CD1 | PHE | 224 | 14.219 | 27.985 | 36.066 | 1.00 | 28.36 |
| atom | 1748 | CD2 | PHE | 224 | 14.310 | 30.074 | 34.943 | 1.00 | 24.89 |
| atom | 1749 | CE1 | PHE | 224 | 14.692 | 28.565 | 37.228 | 1.00 | 28.03 |
| atom | 1750 | CE2 | PHE | 224 | 14.779 | 30.664 | 36.095 | 1.00 | 26.24 |
| atom | 1751 | CZ  | PHE | 224 | 14.980 | 29.921 | 37.237 | 1.00 | 26.41 |
| atom | 1752 | C   | PHE | 224 | 11.589 | 29.654 | 33.376 | 1.00 | 31.31 |
| atom | 1753 | O   | PHE | 224 | 11.119 | 30.162 | 34.403 | 1.00 | 29.83 |
| atom | 1754 | N   | ASP | 225 | 11.801 | 30.329 | 32.250 | 1.00 | 31.32 |
| atom | 1755 | CA  | ASP | 225 | 11.481 | 31.747 | 32.148 | 1.00 | 31.56 |
| atom | 1756 | CB  | ASP | 225 | 11.838 | 32.314 | 30.784 | 1.00 | 30.13 |
| atom | 1757 | CG  | ASP | 225 | 13.331 | 32.424 | 30.537 | 1.00 | 31.05 |
| atom | 1758 | OD1 | ASP | 225 | 14.122 | 32.257 | 31.480 | 1.00 | 29.58 |
| atom | 1759 | OD2 | ASP | 225 | 13.714 | 32.677 | 29.369 | 1.00 | 32.57 |
| atom | 1760 | C   | ASP | 225 | 10.005 | 32.014 | 32.438 | 1.00 | 32.58 |

| atom | 1761 | O   | ASP | 225 | 9.650  | 32.970 | 33.147 | 1.00 | 31.78 |
|------|------|-----|-----|-----|--------|--------|--------|------|-------|
| atom | 1762 | N   | SER | 226 | 9.118  | 31.129 | 31.984 | 1.00 | 31.71 |
| atom | 1763 | CA  | SER | 226 | 7.702  | 31.360 | 32.245 | 1.00 | 33.05 |
| atom | 1764 | CB  | SER | 226 | 6.837  | 30.722 | 31.162 | 1.00 | 32.59 |
| atom | 1765 | OG  | SER | 226 | 6.479  | 29.429 | 31.569 | 1.00 | 37.12 |
| atom | 1766 | C   | SER | 226 | 7.324  | 30.979 | 33.665 | 1.00 | 33.65 |
| atom | 1767 | O   | SER | 226 | 6.243  | 31.353 | 34.148 | 1.00 | 33.81 |
| atom | 1768 | N   | THR | 227 | 8.212  | 30.351 | 34.435 | 1.00 | 33.15 |
| atom | 1769 | CA  | THR | 227 | 7.942  | 30.041 | 35.832 | 1.00 | 32.08 |
| atom | 1770 | CB  | THR | 227 | 8.662  | 28.748 | 36.256 | 1.00 | 32.39 |
| atom | 1771 | OG1 | THR | 227 | 10.056 | 29.012 | 36.439 | 1.00 | 30.64 |
| atom | 1772 | CG2 | THR | 227 | 8.404  | 27.685 | 35.193 | 1.00 | 29.17 |
| atom | 1773 | C   | THR | 227 | 8.376  | 31.179 | 36.756 | 1.00 | 32.16 |
| atom | 1774 | O   | THR | 227 | 7.981  | 31.229 | 37.922 | 1.00 | 30.79 |
| atom | 1775 | N   | VAL | 228 | 9.182  | 32.109 | 36.241 | 1.00 | 31.23 |
| atom | 1776 | CA  | VAL | 228 | 9.666  | 33.230 | 37.011 | 1.00 | 30.64 |
| atom | 1777 | CB  | VAL | 228 | 10.735 | 34.053 | 36.276 | 1.00 | 28.53 |
| atom | 1778 | CG1 | VAL | 228 | 11.236 | 35.192 | 37.165 | 1.00 | 25.31 |
| atom | 1779 | CG2 | VAL | 228 | 11.870 | 33.141 | 35.844 | 1.00 | 28.13 |
| atom | 1780 | C   | VAL | 228 | 8.495  | 34.160 | 37.348 | 1.00 | 31.86 |
| atom | 1781 | O   | VAL | 228 | 7.806  | 34.634 | 36.433 | 1.00 | 30.85 |
| atom | 1782 | N   | THR | 229 | 8.345  | 34.453 | 38.643 | 1.00 | 31.93 |
| atom | 1783 | CA  | THR | 229 | 7.226  | 35.303 | 39.030 | 1.00 | 33.44 |
| atom | 1784 | CB  | THR | 229 | 6.614  | 34.874 | 40.381 | 1.00 | 30.26 |
| atom | 1785 | OG1 | THR | 229 | 7.629  | 35.031 | 41.384 | 1.00 | 26.91 |
| atom | 1786 | CG2 | THR | 229 | 6.116  | 33.441 | 40.295 | 1.00 | 29.78 |
| atom | 1787 | C   | THR | 229 | 7.631  | 36.757 | 39.173 | 1.00 | 35.69 |
| atom | 1788 | O   | THR | 229 | 8.805  | 37.109 | 39.312 | 1.00 | 35.76 |
| atom | 1789 | N   | GLU | 230 | 6.601  | 37.590 | 39.340 | 1.00 | 36.59 |
| atom | 1790 | CA  | GLU | 230 | 6.804  | 39.011 | 39.616 | 1.00 | 38.22 |
| atom | 1791 | CB  | GLU | 230 | 5.472  | 39.763 | 39.736 | 1.00 | 43.15 |
| atom | 1792 | CG  | GLU | 230 | 4.426  | 39.216 | 38.784 | 1.00 | 50.30 |
| atom | 1793 | CD  | GLU | 230 | 3.273  | 40.123 | 38.424 | 1.00 | 53.76 |
| atom | 1794 | OE1 | GLU | 230 | 2.529  | 39.734 | 37.488 | 1.00 | 54.09 |
| atom | 1795 | OE2 | GLU | 230 | 3.123  | 41.201 | 39.056 | 1.00 | 54.15 |
| atom | 1796 | C   | GLU | 230 | 7.541  | 39.118 | 40.952 | 1.00 | 37.04 |

| atom | 1797 | O   | GLU | 230 | 8.419  | 39.954 | 41.187 | 1.00 | 37.47 |
|------|------|-----|-----|-----|--------|--------|--------|------|-------|
| atom | 1798 | N   | ASN | 231 | 7.139  | 38.241 | 41.863 | 1.00 | 35.14 |
| atom | 1799 | CA  | ASN | 231 | 7.792  | 38.143 | 43.160 | 1.00 | 35.82 |
| atom | 1800 | CB  | ASN | 231 | 7.135  | 37.067 | 44.032 | 1.00 | 37.63 |
| atom | 1801 | CG  | ASN | 231 | 8.004  | 36.655 | 45.209 | 1.00 | 42.02 |
| atom | 1802 | OD1 | ASN | 231 | 7.950  | 37.312 | 46.264 | 1.00 | 43.08 |
| atom | 1803 | ND2 | ASN | 231 | 8.847  | 35.616 | 45.100 | 1.00 | 41.24 |
| atom | 1804 | C   | ASN | 231 | 9.276  | 37.846 | 42.934 | 1.00 | 34.56 |
| atom | 1805 | O   | ASN | 231 | 10.136 | 38.510 | 43.512 | 1.00 | 34.92 |
| atom | 1806 | N   | ASP | 232 | 9.601  | 36.859 | 42.100 | 1.00 | 33.75 |
| atom | 1807 | CA  | ASP | 232 | 10.990 | 36.505 | 41.834 | 1.00 | 32.93 |
| atom | 1808 | CB  | ASP | 232 | 11.156 | 35.355 | 40.838 | 1.00 | 31.55 |
| atom | 1809 | CG  | ASP | 232 | 10.499 | 34.064 | 41.257 | 1.00 | 30.67 |
| atom | 1810 | OD1 | ASP | 232 | 10.500 | 33.838 | 42.480 | 1.00 | 31.79 |
| atom | 1811 | OD2 | ASP | 232 | 9.998  | 33.285 | 40.421 | 1.00 | 30.40 |
| atom | 1812 | C   | ASP | 232 | 11.757 | 37.717 | 41.281 | 1.00 | 31.86 |
| atom | 1813 | O   | ASP | 232 | 12.867 | 37.959 | 41.741 | 1.00 | 30.27 |
| atom | 1814 | N   | ILE | 233 | 11.136 | 38.429 | 40.349 | 1.00 | 30.49 |
| atom | 1815 | CA  | ILE | 233 | 11.741 | 39.577 | 39.702 | 1.00 | 31.10 |
| atom | 1816 | CB  | ILE | 233 | 11.020 | 39.982 | 38.414 | 1.00 | 28.69 |
| atom | 1817 | CG2 | ILE | 233 | 11.701 | 41.187 | 37.786 | 1.00 | 26.78 |
| atom | 1818 | CG1 | ILE | 233 | 11.006 | 38.795 | 37.442 | 1.00 | 28.95 |
| atom | 1819 | CD1 | ILE | 233 | 9.965  | 38.879 | 36.334 | 1.00 | 29.80 |
| atom | 1820 | C   | ILE | 233 | 11.911 | 40.772 | 40.636 | 1.00 | 31.88 |
| atom | 1821 | O   | ILE | 233 | 12.958 | 41.424 | 40.579 | 1.00 | 30.72 |
| atom | 1822 | N   | ARG | 234 | 10.964 | 40.944 | 41.558 | 1.00 | 31.85 |
| atom | 1823 | CA  | ARG | 234 | 11.089 | 41.991 | 42.564 | 1.00 | 32.92 |
| atom | 1824 | CB  | ARG | 234 | 9.740  | 42.274 | 43.233 | 1.00 | 33.58 |
| atom | 1825 | CG  | ARG | 234 | 8.698  | 42.868 | 42.278 | 1.00 | 33.48 |
| atom | 1826 | CD  | ARG | 234 | 7.527  | 43.477 | 43.065 | 1.00 | 32.42 |
| atom | 1827 | NE  | ARG | 234 | 6.594  | 44.142 | 42.154 | 1.00 | 32.66 |
| atom | 1828 | CZ  | ARG | 234 | 6.830  | 45.320 | 41.589 | 1.00 | 33.63 |
| atom | 1829 | NH1 | ARG | 234 | 7.960  | 45.972 | 41.857 | 1.00 | 33.80 |
| atom | 1830 | NH2 | ARG | 234 | 5.994  | 45.908 | 40.743 | 1.00 | 31.53 |
| atom | 1831 | C   | ARG | 234 | 12.121 | 41.535 | 43.595 | 1.00 | 34.02 |
| atom | 1832 | O   | ARG | 234 | 12.855 | 42.344 | 44.190 | 1.00 | 33.93 |

| atom | 1833 | N | VAL | 235 | 12.207 | 40.202 | 43.782 | 1.00 | 32.95 |
|------|------|------|------|------|--------|--------|--------|------|-------|
| atom | 1834 | CA | VAL | 235 | 13.224 | 39.696 | 44.706 | 1.00 | 33.27 |
| atom | 1835 | CB | VAL | 235 | 12.994 | 38.226 | 45.073 | 1.00 | 33.69 |
| atom | 1836 | CG1 | VAL | 235 | 14.233 | 37.542 | 45.623 | 1.00 | 31.65 |
| atom | 1837 | CG2 | VAL | 235 | 11.870 | 38.123 | 46.116 | 1.00 | 31.75 |
| atom | 1838 | C | VAL | 235 | 14.609 | 39.997 | 44.140 | 1.00 | 33.47 |
| atom | 1839 | O | VAL | 235 | 15.457 | 40.482 | 44.904 | 1.00 | 33.59 |
| atom | 1840 | N | GLU | 236 | 14.844 | 39.785 | 42.846 | 1.00 | 33.23 |
| atom | 1841 | CA | GLU | 236 | 16.147 | 40.042 | 42.254 | 1.00 | 34.27 |
| atom | 1842 | CB | GLU | 236 | 16.245 | 39.721 | 40.749 | 1.00 | 36.16 |
| atom | 1843 | CG | GLU | 236 | 15.399 | 38.554 | 40.315 | 1.00 | 41.35 |
| atom | 1844 | CD | GLU | 236 | 15.380 | 38.225 | 38.843 | 1.00 | 43.16 |
| atom | 1845 | OE1 | GLU | 236 | 15.724 | 39.125 | 38.040 | 1.00 | 44.54 |
| atom | 1846 | OE2 | GLU | 236 | 15.046 | 37.051 | 38.520 | 1.00 | 42.79 |
| atom | 1847 | C | GLU | 236 | 16.524 | 41.516 | 42.429 | 1.00 | 33.20 |
| atom | 1848 | O | GLU | 236 | 17.639 | 41.872 | 42.793 | 1.00 | 32.00 |
| atom | 1849 | N | GLU | 237 | 15.564 | 42.382 | 42.114 | 1.00 | 32.70 |
| atom | 1850 | CA | GLU | 237 | 15.742 | 43.822 | 42.250 | 1.00 | 32.35 |
| atom | 1851 | CB | GLU | 237 | 14.415 | 44.478 | 41.933 | 1.00 | 33.85 |
| atom | 1852 | CG | GLU | 237 | 14.526 | 45.899 | 41.476 | 1.00 | 37.22 |
| atom | 1853 | CD | GLU | 237 | 14.175 | 46.928 | 42.518 | 1.00 | 40.18 |
| atom | 1854 | OE1 | GLU | 237 | 14.050 | 48.101 | 42.108 | 1.00 | 41.00 |
| atom | 1855 | OE2 | GLU | 237 | 14.045 | 46.565 | 43.704 | 1.00 | 41.00 |
| atom | 1856 | C | GLU | 237 | 16.214 | 44.188 | 43.649 | 1.00 | 32.24 |
| atom | 1857 | O | GLU | 237 | 17.238 | 44.866 | 43.798 | 1.00 | 32.51 |
| atom | 1858 | N | SER | 238 | 15.566 | 43.649 | 44.690 | 1.00 | 31.11 |
| atom | 1859 | CA | SER | 238 | 16.030 | 43.908 | 46.050 | 1.00 | 30.88 |
| atom | 1860 | CB | SER | 238 | 15.089 | 43.406 | 47.141 | 1.00 | 29.74 |
| atom | 1861 | OG | SER | 238 | 15.187 | 42.011 | 47.345 | 1.00 | 34.88 |
| atom | 1862 | C | SER | 238 | 17.450 | 43.374 | 46.209 | 1.00 | 30.13 |
| atom | 1863 | O | SER | 238 | 18.274 | 44.024 | 46.876 | 1.00 | 30.47 |
| atom | 1864 | N | ILE | 239 | 17.788 | 42.242 | 45.590 | 1.00 | 29.38 |
| atom | 1865 | CA | ILE | 239 | 19.187 | 41.790 | 45.668 | 1.00 | 29.93 |
| atom | 1866 | CB | ILE | 239 | 19.381 | 40.378 | 45.121 | 1.00 | 29.85 |
| atom | 1867 | CG2 | ILE | 239 | 20.851 | 39.979 | 45.135 | 1.00 | 29.33 |
| atom | 1868 | CG1 | ILE | 239 | 18.549 | 39.398 | 45.958 | 1.00 | 28.55 |

| | | | | | | | | | |
|---|---|---|---|---|---|---|---|---|---|
| atom | 1869 | CD1 | ILE | 239 | 18.533 | 37.978 | 45.418 | 1.00 | 28.71 |
| atom | 1870 | C | ILE | 239 | 20.086 | 42.831 | 45.002 | 1.00 | 29.87 |
| atom | 1871 | O | ILE | 239 | 21.023 | 43.339 | 45.640 | 1.00 | 29.57 |
| atom | 1872 | N | TYR | 240 | 19.754 | 43.282 | 43.798 | 1.00 | 30.19 |
| atom | 1873 | CA | TYR | 240 | 20.512 | 44.326 | 43.117 | 1.00 | 31.40 |
| atom | 1874 | CB | TYR | 240 | 19.931 | 44.772 | 41.774 | 1.00 | 30.94 |
| atom | 1875 | CG | TYR | 240 | 19.771 | 43.671 | 40.752 | 1.00 | 32.31 |
| atom | 1876 | CD1 | TYR | 240 | 18.732 | 43.698 | 39.826 | 1.00 | 33.66 |
| atom | 1877 | CE1 | TYR | 240 | 18.581 | 42.679 | 38.890 | 1.00 | 33.95 |
| atom | 1878 | CD2 | TYR | 240 | 20.642 | 42.592 | 40.722 | 1.00 | 32.10 |
| atom | 1879 | CE2 | TYR | 240 | 20.506 | 41.581 | 39.804 | 1.00 | 32.52 |
| atom | 1880 | CZ | TYR | 240 | 19.478 | 41.631 | 38.889 | 1.00 | 33.26 |
| atom | 1881 | OH | TYR | 240 | 19.349 | 40.603 | 37.986 | 1.00 | 33.98 |
| atom | 1882 | C | TYR | 240 | 20.677 | 45.572 | 43.984 | 1.00 | 31.59 |
| atom | 1883 | O | TYR | 240 | 21.810 | 46.031 | 44.154 | 1.00 | 31.55 |
| atom | 1884 | N | GLN | 241 | 19.590 | 46.071 | 44.566 | 1.00 | 31.05 |
| atom | 1885 | CA | GLN | 241 | 19.617 | 47.250 | 45.416 | 1.00 | 31.74 |
| atom | 1886 | CB | GLN | 241 | 18.204 | 47.800 | 45.673 | 1.00 | 30.62 |
| atom | 1887 | CG | GLN | 241 | 17.373 | 48.094 | 44.428 | 1.00 | 31.27 |
| atom | 1888 | CD | GLN | 241 | 17.687 | 49.440 | 43.810 | 1.00 | 31.51 |
| atom | 1889 | OE1 | GLN | 241 | 18.495 | 50.187 | 44.368 | 1.00 | 33.92 |
| atom | 1890 | NE2 | GLN | 241 | 17.099 | 49.809 | 42.673 | 1.00 | 30.90 |
| atom | 1891 | C | GLN | 241 | 20.366 | 47.076 | 46.730 | 1.00 | 31.69 |
| atom | 1892 | O | GLN | 241 | 20.681 | 48.096 | 47.366 | 1.00 | 32.51 |
| atom | 1893 | N | CYS | 242 | 20.799 | 45.895 | 47.158 | 1.00 | 31.74 |
| atom | 1894 | CA | CYS | 242 | 21.630 | 45.684 | 48.332 | 1.00 | 30.46 |
| atom | 1895 | CB | CYS | 242 | 21.713 | 44.212 | 48.751 | 1.00 | 32.06 |
| atom | 1896 | SG | CYS | 242 | 20.263 | 43.562 | 49.650 | 1.00 | 31.70 |
| atom | 1897 | C | CYS | 242 | 23.060 | 46.160 | 48.054 | 1.00 | 30.83 |
| atom | 1898 | O | CYS | 242 | 23.850 | 46.463 | 48.951 | 1.00 | 30.90 |
| atom | 1899 | N | CYS | 243 | 23.433 | 46.203 | 46.779 | 1.00 | 29.93 |
| atom | 1900 | CA | CYS | 243 | 24.724 | 46.659 | 46.330 | 1.00 | 29.97 |
| atom | 1901 | CB | CYS | 243 | 24.892 | 46.466 | 44.819 | 1.00 | 28.11 |
| atom | 1902 | SG | CYS | 243 | 24.884 | 44.790 | 44.155 | 1.00 | 25.48 |
| atom | 1903 | C | CYS | 243 | 24.884 | 48.150 | 46.649 | 1.00 | 29.91 |
| atom | 1904 | O | CYS | 243 | 23.909 | 48.882 | 46.848 | 1.00 | 29.91 |

| atom | 1905 | N   | ASP | 244 | 26.132 | 48.584 | 46.687 | 1.00 | 29.89 |
|------|------|-----|-----|-----|--------|--------|--------|------|-------|
| atom | 1906 | CA  | ASP | 244 | 26.412 | 50.022 | 46.879 | 1.00 | 30.38 |
| atom | 1907 | CB  | ASP | 244 | 27.725 | 50.228 | 47.608 | 1.00 | 32.08 |
| atom | 1908 | CG  | ASP | 244 | 28.160 | 51.661 | 47.767 | 1.00 | 34.38 |
| atom | 1909 | OD1 | ASP | 244 | 27.452 | 52.617 | 47.387 | 1.00 | 36.57 |
| atom | 1910 | OD2 | ASP | 244 | 29.279 | 51.846 | 48.303 | 1.00 | 37.77 |
| atom | 1911 | C   | ASP | 244 | 26.425 | 50.584 | 45.458 | 1.00 | 29.68 |
| atom | 1912 | O   | ASP | 244 | 27.334 | 50.301 | 44.681 | 1.00 | 28.69 |
| atom | 1913 | N   | LEU | 245 | 25.345 | 51.262 | 45.084 | 1.00 | 30.49 |
| atom | 1914 | CA  | LEU | 245 | 25.220 | 51.755 | 43.719 | 1.00 | 31.01 |
| atom | 1915 | CB  | LEU | 245 | 23.999 | 51.127 | 43.049 | 1.00 | 28.53 |
| atom | 1916 | CG  | LEU | 245 | 23.775 | 49.624 | 43.151 | 1.00 | 27.96 |
| atom | 1917 | CD1 | LEU | 245 | 22.293 | 49.294 | 42.927 | 1.00 | 27.18 |
| atom | 1918 | CD2 | LEU | 245 | 24.661 | 48.892 | 42.149 | 1.00 | 26.51 |
| atom | 1919 | C   | LEU | 245 | 25.108 | 53.271 | 43.701 | 1.00 | 31.97 |
| atom | 1920 | O   | LEU | 245 | 24.868 | 53.858 | 44.755 | 1.00 | 33.52 |
| atom | 1921 | N   | ALA | 246 | 25.289 | 53.886 | 42.544 | 1.00 | 31.24 |
| atom | 1922 | CA  | ALA | 246 | 25.139 | 55.324 | 42.419 | 1.00 | 32.14 |
| atom | 1923 | CB  | ALA | 246 | 25.867 | 55.806 | 41.171 | 1.00 | 28.13 |
| atom | 1924 | C   | ALA | 246 | 23.651 | 55.647 | 42.338 | 1.00 | 33.72 |
| atom | 1925 | O   | ALA | 246 | 22.848 | 54.945 | 41.698 | 1.00 | 33.92 |
| atom | 1926 | N   | PRO | 247 | 23.227 | 56.755 | 42.943 | 1.00 | 34.03 |
| atom | 1927 | CD  | PRO | 247 | 24.074 | 57.648 | 43.771 | 1.00 | 33.46 |
| atom | 1928 | CA  | PRO | 247 | 21.834 | 57.188 | 42.955 | 1.00 | 34.03 |
| atom | 1929 | CB  | PRO | 247 | 21.902 | 58.639 | 43.430 | 1.00 | 32.55 |
| atom | 1930 | CG  | PRO | 247 | 23.089 | 58.651 | 44.335 | 1.00 | 33.43 |
| atom | 1931 | C   | PRO | 247 | 21.099 | 57.062 | 41.639 | 1.00 | 34.44 |
| atom | 1932 | O   | PRO | 247 | 19.908 | 56.729 | 41.614 | 1.00 | 34.98 |
| atom | 1933 | N   | GLU | 248 | 21.736 | 57.341 | 40.515 | 1.00 | 34.23 |
| atom | 1934 | CA  | GLU | 248 | 21.116 | 57.236 | 39.205 | 1.00 | 34.73 |
| atom | 1935 | CB  | GLU | 248 | 21.970 | 57.982 | 38.210 | 1.00 | 36.30 |
| atom | 1936 | CG  | GLU | 248 | 21.351 | 58.821 | 37.130 | 1.00 | 38.23 |
| atom | 1937 | CD  | GLU | 248 | 22.445 | 59.391 | 36.234 | 1.00 | 41.03 |
| atom | 1938 | OE1 | GLU | 248 | 23.573 | 59.610 | 36.751 | 1.00 | 43.01 |
| atom | 1939 | OE2 | GLU | 248 | 22.186 | 59.600 | 35.028 | 1.00 | 39.89 |
| atom | 1940 | C   | GLU | 248 | 20.953 | 55.767 | 38.808 | 1.00 | 34.35 |

| atom | 1941 | O   | GLU | 248 | 19.999 | 55.462 | 38.080 | 1.00 | 34.67 |
|------|------|-----|-----|-----|--------|--------|--------|------|-------|
| atom | 1942 | N   | ALA | 249 | 21.858 | 54.890 | 39.249 | 1.00 | 32.72 |
| atom | 1943 | CA  | ALA | 249 | 21.725 | 53.473 | 38.913 | 1.00 | 31.73 |
| atom | 1944 | CB  | ALA | 249 | 22.940 | 52.633 | 39.288 | 1.00 | 29.30 |
| atom | 1945 | C   | ALA | 249 | 20.522 | 52.876 | 39.643 | 1.00 | 30.31 |
| atom | 1946 | O   | ALA | 249 | 19.744 | 52.171 | 39.005 | 1.00 | 30.58 |
| atom | 1947 | N   | ARG | 250 | 20.365 | 53.211 | 40.917 | 1.00 | 29.18 |
| atom | 1948 | CA  | ARG | 250 | 19.256 | 52.680 | 41.711 | 1.00 | 28.53 |
| atom | 1949 | CB  | ARG | 250 | 19.203 | 53.183 | 43.149 | 1.00 | 26.75 |
| atom | 1950 | CG  | ARG | 250 | 20.409 | 52.915 | 44.029 | 1.00 | 26.39 |
| atom | 1951 | CD  | ARG | 250 | 20.163 | 53.350 | 45.483 | 1.00 | 27.42 |
| atom | 1952 | NE  | ARG | 250 | 21.282 | 52.881 | 46.282 | 1.00 | 27.05 |
| atom | 1953 | CZ  | ARG | 250 | 21.578 | 51.630 | 46.616 | 1.00 | 28.38 |
| atom | 1954 | NH1 | ARG | 250 | 20.797 | 50.603 | 46.255 | 1.00 | 27.62 |
| atom | 1955 | NH2 | ARG | 250 | 22.703 | 51.446 | 47.312 | 1.00 | 24.31 |
| atom | 1956 | C   | ARG | 250 | 17.950 | 52.988 | 40.987 | 1.00 | 27.94 |
| atom | 1957 | O   | ARG | 250 | 17.163 | 52.081 | 40.743 | 1.00 | 27.27 |
| atom | 1958 | N   | GLN | 251 | 17.781 | 54.252 | 40.617 | 1.00 | 28.58 |
| atom | 1959 | CA  | GLN | 251 | 16.601 | 54.714 | 39.896 | 1.00 | 29.25 |
| atom | 1960 | CB  | GLN | 251 | 16.664 | 56.231 | 39.684 | 1.00 | 27.02 |
| atom | 1961 | CG  | GLN | 251 | 15.497 | 56.809 | 38.890 | 1.00 | 29.30 |
| atom | 1962 | CD  | GLN | 251 | 14.307 | 57.164 | 39.765 | 1.00 | 29.04 |
| atom | 1963 | OE1 | GLN | 251 | 14.238 | 56.718 | 40.912 | 1.00 | 28.49 |
| atom | 1964 | NE2 | GLN | 251 | 13.371 | 57.956 | 39.263 | 1.00 | 27.91 |
| atom | 1965 | C   | GLN | 251 | 16.397 | 54.064 | 38.532 | 1.00 | 28.87 |
| atom | 1966 | O   | GLN | 251 | 15.265 | 53.730 | 38.167 | 1.00 | 29.21 |
| atom | 1967 | N   | ALA | 252 | 17.449 | 53.863 | 37.745 | 1.00 | 29.00 |
| atom | 1968 | CA  | ALA | 252 | 17.249 | 53.226 | 36.430 | 1.00 | 29.47 |
| atom | 1969 | CB  | ALA | 252 | 18.441 | 53.450 | 35.516 | 1.00 | 27.63 |
| atom | 1970 | C   | ALA | 252 | 16.941 | 51.741 | 36.614 | 1.00 | 29.33 |
| atom | 1971 | O   | ALA | 252 | 16.224 | 51.132 | 35.803 | 1.00 | 28.78 |
| atom | 1972 | N   | ILE | 253 | 17.486 | 51.163 | 37.688 | 1.00 | 28.79 |
| atom | 1973 | CA  | ILE | 253 | 17.257 | 49.740 | 37.968 | 1.00 | 29.82 |
| atom | 1974 | CB  | ILE | 253 | 18.278 | 49.212 | 38.983 | 1.00 | 29.96 |
| atom | 1975 | CG2 | ILE | 253 | 17.882 | 47.861 | 39.572 | 1.00 | 30.14 |
| atom | 1976 | CG1 | ILE | 253 | 19.682 | 49.151 | 38.357 | 1.00 | 27.97 |

| atom | 1977 | CD1 | ILE | 253 | 20.781 | 48.987 | 39.394 | 1.00 | 25.16 |
|------|------|-----|-----|-----|--------|--------|--------|------|-------|
| atom | 1978 | C   | ILE | 253 | 15.820 | 49.516 | 38.431 | 1.00 | 30.68 |
| atom | 1979 | O   | ILE | 253 | 15.144 | 48.602 | 37.969 | 1.00 | 29.43 |
| atom | 1980 | N   | LYS | 254 | 15.276 | 50.401 | 39.271 | 1.00 | 31.45 |
| atom | 1981 | CA  | LYS | 254 | 13.877 | 50.260 | 39.707 | 1.00 | 31.70 |
| atom | 1982 | CB  | LYS | 254 | 13.590 | 51.263 | 40.818 | 1.00 | 30.17 |
| atom | 1983 | CG  | LYS | 254 | 12.179 | 51.789 | 40.901 | 1.00 | 32.54 |
| atom | 1984 | CD  | LYS | 254 | 11.911 | 52.734 | 42.063 | 1.00 | 33.51 |
| atom | 1985 | CE  | LYS | 254 | 12.456 | 54.129 | 41.780 | 1.00 | 35.02 |
| atom | 1986 | NZ  | LYS | 254 | 11.537 | 55.177 | 42.319 | 1.00 | 38.11 |
| atom | 1987 | C   | LYS | 254 | 12.988 | 50.453 | 38.484 | 1.00 | 31.76 |
| atom | 1988 | O   | LYS | 254 | 12.042 | 49.741 | 38.158 | 1.00 | 32.10 |
| atom | 1989 | N   | SER | 255 | 13.292 | 51.524 | 37.752 | 1.00 | 31.34 |
| atom | 1990 | CA  | SER | 255 | 12.532 | 51.837 | 36.554 | 1.00 | 32.23 |
| atom | 1991 | CB  | SER | 255 | 13.111 | 53.087 | 35.898 | 1.00 | 31.85 |
| atom | 1992 | OG  | SER | 255 | 12.188 | 53.573 | 34.941 | 1.00 | 33.85 |
| atom | 1993 | C   | SER | 255 | 12.514 | 50.677 | 35.560 | 1.00 | 31.80 |
| atom | 1994 | O   | SER | 255 | 11.458 | 50.270 | 35.064 | 1.00 | 31.55 |
| atom | 1995 | N   | LEU | 256 | 13.682 | 50.101 | 35.268 | 1.00 | 31.94 |
| atom | 1996 | CA  | LEU | 256 | 13.761 | 48.981 | 34.333 | 1.00 | 30.51 |
| atom | 1997 | CB  | LEU | 256 | 15.179 | 48.567 | 33.940 | 1.00 | 30.00 |
| atom | 1998 | CG  | LEU | 256 | 15.995 | 49.520 | 33.068 | 1.00 | 30.40 |
| atom | 1999 | CD1 | LEU | 256 | 17.481 | 49.171 | 33.103 | 1.00 | 28.92 |
| atom | 2000 | CD2 | LEU | 256 | 15.495 | 49.518 | 31.624 | 1.00 | 28.39 |
| atom | 2001 | C   | LEU | 256 | 13.013 | 47.776 | 34.886 | 1.00 | 29.34 |
| atom | 2002 | O   | LEU | 256 | 12.424 | 47.067 | 34.055 | 1.00 | 28.40 |
| atom | 2003 | N   | THR | 257 | 13.021 | 47.511 | 36.195 | 1.00 | 29.37 |
| atom | 2004 | CA  | THR | 257 | 12.276 | 46.336 | 36.630 | 1.00 | 30.23 |
| atom | 2005 | CB  | THR | 257 | 12.759 | 45.589 | 37.868 | 1.00 | 33.02 |
| atom | 2006 | OG1 | THR | 257 | 11.735 | 45.568 | 38.879 | 1.00 | 36.13 |
| atom | 2007 | CG2 | THR | 257 | 14.099 | 46.016 | 38.362 | 1.00 | 27.89 |
| atom | 2008 | C   | THR | 257 | 10.766 | 46.556 | 36.662 | 1.00 | 30.84 |
| atom | 2009 | O   | THR | 257 | 10.069 | 45.601 | 36.273 | 1.00 | 30.66 |
| atom | 2010 | N   | GLU | 258 | 10.273 | 47.754 | 36.941 | 1.00 | 30.17 |
| atom | 2011 | CA  | GLU | 258 | 8.858  | 48.037 | 36.900 | 1.00 | 30.50 |
| atom | 2012 | CB  | GLU | 258 | 8.532  | 49.424 | 37.484 | 1.00 | 33.29 |

| atom | 2013 | CG | GLU | 258 | 8.508 | 49.513 | 39.001 | 1.00 | 35.92 |
|------|------|------|------|------|------|------|------|------|------|
| atom | 2014 | CD | GLU | 258 | 7.451 | 48.609 | 39.598 | 1.00 | 36.56 |
| atom | 2015 | OE1 | GLU | 258 | 6.355 | 48.501 | 39.014 | 1.00 | 39.71 |
| atom | 2016 | OE2 | GLU | 258 | 7.771 | 48.021 | 40.639 | 1.00 | 38.35 |
| atom | 2017 | C | GLU | 258 | 8.289 | 48.121 | 35.477 | 1.00 | 30.58 |
| atom | 2018 | O | GLU | 258 | 7.112 | 47.804 | 35.264 | 1.00 | 28.37 |
| atom | 2019 | N | ARG | 259 | 9.133 | 48.628 | 34.555 | 1.00 | 29.58 |
| atom | 2020 | CA | ARG | 259 | 8.625 | 48.879 | 33.213 | 1.00 | 28.63 |
| atom | 2021 | CB | ARG | 259 | 9.170 | 50.226 | 32.688 | 1.00 | 29.39 |
| atom | 2022 | CG | ARG | 259 | 8.813 | 51.405 | 33.589 | 1.00 | 28.34 |
| atom | 2023 | CD | ARG | 259 | 9.389 | 52.741 | 33.148 | 1.00 | 28.75 |
| atom | 2024 | NE | ARG | 259 | 9.217 | 53.065 | 31.749 | 1.00 | 28.65 |
| atom | 2025 | CZ | ARG | 259 | 9.751 | 54.053 | 31.061 | 1.00 | 28.28 |
| atom | 2026 | NH1 | ARG | 259 | 10.544 | 54.948 | 31.636 | 1.00 | 28.28 |
| atom | 2027 | NH2 | ARG | 259 | 9.495 | 54.181 | 29.759 | 1.00 | 28.16 |
| atom | 2028 | C | ARG | 259 | 8.925 | 47.807 | 32.193 | 1.00 | 28.58 |
| atom | 2029 | O | ARG | 259 | 8.266 | 47.811 | 31.151 | 1.00 | 27.71 |
| atom | 2030 | N | LEU | 260 | 9.918 | 46.973 | 32.460 | 1.00 | 29.20 |
| atom | 2031 | CA | LEU | 260 | 10.341 | 45.981 | 31.478 | 1.00 | 29.41 |
| atom | 2032 | CB | LEU | 260 | 11.684 | 46.447 | 30.923 | 1.00 | 29.72 |
| atom | 2033 | CG | LEU | 260 | 12.197 | 46.039 | 29.549 | 1.00 | 31.17 |
| atom | 2034 | CD1 | LEU | 260 | 13.721 | 45.947 | 29.599 | 1.00 | 29.50 |
| atom | 2035 | CD2 | LEU | 260 | 11.561 | 44.796 | 28.961 | 1.00 | 30.71 |
| atom | 2036 | C | LEU | 260 | 10.485 | 44.581 | 32.055 | 1.00 | 29.19 |
| atom | 2037 | O | LEU | 260 | 9.897 | 43.646 | 31.519 | 1.00 | 29.29 |
| atom | 2038 | N | TYR | 261 | 11.272 | 44.385 | 33.097 | 1.00 | 29.50 |
| atom | 2039 | CA | TYR | 261 | 11.538 | 43.066 | 33.645 | 1.00 | 30.04 |
| atom | 2040 | CB | TYR | 261 | 12.633 | 43.157 | 34.719 | 1.00 | 29.84 |
| atom | 2041 | CG | TYR | 261 | 13.948 | 43.713 | 34.194 | 1.00 | 28.50 |
| atom | 2042 | CD1 | TYR | 261 | 14.269 | 43.578 | 32.850 | 1.00 | 27.55 |
| atom | 2043 | CE1 | TYR | 261 | 15.449 | 44.069 | 32.345 | 1.00 | 27.36 |
| atom | 2044 | CD2 | TYR | 261 | 14.864 | 44.342 | 35.028 | 1.00 | 26.74 |
| atom | 2045 | CE2 | TYR | 261 | 16.053 | 44.812 | 34.526 | 1.00 | 26.27 |
| atom | 2046 | CZ | TYR | 261 | 16.349 | 44.670 | 33.192 | 1.00 | 26.36 |
| atom | 2047 | OH | TYR | 261 | 17.513 | 45.143 | 32.636 | 1.00 | 26.74 |
| atom | 2048 | C | TYR | 261 | 10.341 | 42.343 | 34.226 | 1.00 | 31.33 |

| atom | 2049 | O   | TYR | 261 | 10.176 | 41.155 | 33.938 | 1.00 | 31.94 |
|------|------|-----|-----|-----|--------|--------|--------|------|-------|
| atom | 2050 | N   | ILE | 262 | 9.512  | 43.031 | 35.002 | 1.00 | 31.51 |
| atom | 2051 | CA  | ILE | 262 | 8.357  | 42.417 | 35.627 | 1.00 | 32.29 |
| atom | 2052 | CB  | ILE | 262 | 7.743  | 43.326 | 36.693 | 1.00 | 37.27 |
| atom | 2053 | CG2 | ILE | 262 | 6.984  | 44.508 | 36.088 | 1.00 | 39.35 |
| atom | 2054 | CG1 | ILE | 262 | 6.813  | 42.487 | 37.579 | 1.00 | 41.12 |
| atom | 2055 | CD1 | ILE | 262 | 6.561  | 43.178 | 38.906 | 1.00 | 45.20 |
| atom | 2056 | C   | ILE | 262 | 7.341  | 41.941 | 34.609 | 1.00 | 31.85 |
| atom | 2057 | O   | ILE | 262 | 6.662  | 40.931 | 34.827 | 1.00 | 31.62 |
| atom | 2058 | N   | GLY | 263 | 7.243  | 42.593 | 33.457 | 1.00 | 31.76 |
| atom | 2059 | CA  | GLY | 263 | 6.296  | 42.129 | 32.443 | 1.00 | 31.63 |
| atom | 2060 | C   | GLY | 263 | 6.027  | 43.263 | 31.466 | 1.00 | 32.65 |
| atom | 2061 | O   | GLY | 263 | 6.658  | 44.315 | 31.602 | 1.00 | 32.33 |
| atom | 2062 | N   | GLY | 264 | 5.093  | 43.054 | 30.536 | 1.00 | 32.81 |
| atom | 2063 | CA  | GLY | 264 | 4.775  | 44.099 | 29.582 | 1.00 | 33.45 |
| atom | 2064 | C   | GLY | 264 | 4.121  | 43.577 | 28.315 | 1.00 | 34.18 |
| atom | 2065 | O   | GLY | 264 | 3.856  | 42.380 | 28.182 | 1.00 | 35.43 |
| atom | 2066 | N   | PRO | 265 | 3.774  | 44.486 | 27.412 | 1.00 | 33.62 |
| atom | 2067 | CD  | PRO | 265 | 4.032  | 45.941 | 27.541 | 1.00 | 33.87 |
| atom | 2068 | CA  | PRO | 265 | 3.143  | 44.177 | 26.145 | 1.00 | 33.40 |
| atom | 2069 | CB  | PRO | 265 | 2.707  | 45.534 | 25.580 | 1.00 | 33.01 |
| atom | 2070 | CG  | PRO | 265 | 3.642  | 46.515 | 26.202 | 1.00 | 33.60 |
| atom | 2071 | C   | PRO | 265 | 4.081  | 43.537 | 25.139 | 1.00 | 32.96 |
| atom | 2072 | O   | PRO | 265 | 5.256  | 43.886 | 25.027 | 1.00 | 33.11 |
| atom | 2073 | N   | LEU | 266 | 3.547  | 42.619 | 24.349 | 1.00 | 33.29 |
| atom | 2074 | CA  | LEU | 266 | 4.311  | 41.895 | 23.346 | 1.00 | 33.29 |
| atom | 2075 | CB  | LEU | 266 | 4.057  | 40.395 | 23.522 | 1.00 | 33.54 |
| atom | 2076 | CG  | LEU | 266 | 4.269  | 39.807 | 24.921 | 1.00 | 32.13 |
| atom | 2077 | CD1 | LEU | 266 | 3.638  | 38.430 | 24.979 | 1.00 | 31.41 |
| atom | 2078 | CD2 | LEU | 266 | 5.759  | 39.733 | 25.242 | 1.00 | 33.38 |
| atom | 2079 | C   | LEU | 266 | 3.853  | 42.264 | 21.952 | 1.00 | 34.40 |
| atom | 2080 | O   | LEU | 266 | 2.659  | 42.130 | 21.643 | 1.00 | 35.07 |
| atom | 2081 | N   | THR | 267 | 4.777  | 42.719 | 21.114 | 1.00 | 34.78 |
| atom | 2082 | CA  | THR | 267 | 4.342  | 43.062 | 19.766 | 1.00 | 36.07 |
| atom | 2083 | CB  | THR | 267 | 4.241  | 44.564 | 19.469 | 1.00 | 36.12 |
| atom | 2084 | OG1 | THR | 267 | 4.959  | 44.906 | 18.277 | 1.00 | 35.59 |

| atom | 2085 | CG2 | THR | 267 | 4.650 | 45.435 | 20.613 | 1.00 | 32.67 |
|------|------|-----|-----|-----|-------|--------|--------|------|-------|
| atom | 2086 | C | THR | 267 | 5.109 | 42.293 | 18.709 | 1.00 | 37.27 |
| atom | 2087 | O | THR | 267 | 6.330 | 42.155 | 18.713 | 1.00 | 38.24 |
| atom | 2088 | N | ASN | 268 | 4.320 | 41.740 | 17.785 | 1.00 | 38.02 |
| atom | 2089 | CA | ASN | 268 | 4.891 | 40.942 | 16.702 | 1.00 | 39.40 |
| atom | 2090 | CB | ASN | 268 | 3.790 | 40.179 | 15.999 | 1.00 | 39.87 |
| atom | 2091 | CG | ASN | 268 | 2.826 | 40.976 | 15.152 | 1.00 | 40.65 |
| atom | 2092 | OD1 | ASN | 268 | 3.106 | 42.024 | 14.576 | 1.00 | 39.40 |
| atom | 2093 | ND2 | ASN | 268 | 1.614 | 40.436 | 15.059 | 1.00 | 41.34 |
| atom | 2094 | C | ASN | 268 | 5.736 | 41.848 | 15.808 | 1.00 | 40.00 |
| atom | 2095 | O | ASN | 268 | 5.851 | 43.051 | 16.075 | 1.00 | 39.86 |
| atom | 2096 | N | SER | 269 | 6.346 | 41.304 | 14.769 | 1.00 | 40.67 |
| atom | 2097 | CA | SER | 269 | 7.219 | 42.057 | 13.889 | 1.00 | 43.88 |
| atom | 2098 | CB | SER | 269 | 8.225 | 41.111 | 13.224 | 1.00 | 44.83 |
| atom | 2099 | OG | SER | 269 | 7.556 | 40.284 | 12.279 | 1.00 | 46.18 |
| atom | 2100 | C | SER | 269 | 6.482 | 42.862 | 12.826 | 1.00 | 45.48 |
| atom | 2101 | O | SER | 269 | 7.101 | 43.545 | 12.006 | 1.00 | 45.80 |
| atom | 2102 | N | LYS | 270 | 5.164 | 42.825 | 12.832 | 1.00 | 46.62 |
| atom | 2103 | CA | LYS | 270 | 4.275 | 43.553 | 11.955 | 1.00 | 47.63 |
| atom | 2104 | CB | LYS | 270 | 3.269 | 42.568 | 11.329 | 1.00 | 48.50 |
| atom | 2105 | CG | LYS | 270 | 3.794 | 41.913 | 10.065 | 1.00 | 51.67 |
| atom | 2106 | CD | LYS | 270 | 3.375 | 40.471 | 9.896 | 1.00 | 52.63 |
| atom | 2107 | CE | LYS | 270 | 1.892 | 40.267 | 9.678 | 1.00 | 54.43 |
| atom | 2108 | NZ | LYS | 270 | 1.534 | 40.002 | 8.258 | 1.00 | 53.62 |
| atom | 2109 | C | LYS | 270 | 3.527 | 44.630 | 12.734 | 1.00 | 47.68 |
| atom | 2110 | O | LYS | 270 | 2.400 | 45.014 | 12.403 | 1.00 | 48.57 |
| atom | 2111 | N | GLY | 271 | 4.070 | 45.028 | 13.881 | 1.00 | 47.00 |
| atom | 2112 | CA | GLY | 271 | 3.510 | 46.022 | 14.765 | 1.00 | 45.87 |
| atom | 2113 | C | GLY | 271 | 2.224 | 45.678 | 15.479 | 1.00 | 46.02 |
| atom | 2114 | O | GLY | 271 | 1.668 | 46.591 | 16.117 | 1.00 | 45.92 |
| atom | 2115 | N | GLN | 272 | 1.754 | 44.433 | 15.447 | 1.00 | 45.68 |
| atom | 2116 | CA | GLN | 272 | 0.489 | 44.086 | 16.095 | 1.00 | 45.76 |
| atom | 2117 | CB | GLN | 272 | -0.246 | 42.977 | 15.340 | 1.00 | 48.38 |
| atom | 2118 | CG | GLN | 272 | -0.310 | 43.141 | 13.829 | 1.00 | 51.71 |
| atom | 2119 | CD | GLN | 272 | -0.808 | 41.907 | 13.112 | 1.00 | 54.09 |
| atom | 2120 | OE1 | GLN | 272 | -1.552 | 41.949 | 12.129 | 1.00 | 56.77 |

| atom | 2121 | NE2 | GLN | 272 | -0.414 | 40.733 | 13.583 | 1.00 | 54.72 |
|------|------|-----|-----|-----|--------|--------|--------|------|-------|
| atom | 2122 | C | GLN | 272 | 0.713 | 43.649 | 17.536 | 1.00 | 45.50 |
| atom | 2123 | O | GLN | 272 | 1.727 | 43.020 | 17.852 | 1.00 | 46.01 |
| atom | 2124 | N | ASN | 273 | -0.223 | 44.005 | 18.405 | 1.00 | 44.29 |
| atom | 2125 | CA | ASN | 273 | -0.160 | 43.660 | 19.821 | 1.00 | 42.07 |
| atom | 2126 | CB | ASN | 273 | -1.085 | 44.526 | 20.667 | 1.00 | 39.00 |
| atom | 2127 | CG | ASN | 273 | -1.181 | 44.115 | 22.115 | 1.00 | 41.00 |
| atom | 2128 | OD1 | ASN | 273 | -0.194 | 43.855 | 22.813 | 1.00 | 41.85 |
| atom | 2129 | ND2 | ASN | 273 | -2.390 | 44.007 | 22.673 | 1.00 | 41.63 |
| atom | 2130 | C | ASN | 273 | -0.551 | 42.191 | 19.946 | 1.00 | 41.69 |
| atom | 2131 | O | ASN | 273 | -1.687 | 41.893 | 19.629 | 1.00 | 41.80 |
| atom | 2132 | N | CYS | 274 | 0.332 | 41.328 | 20.394 | 1.00 | 41.16 |
| atom | 2133 | CA | CYS | 274 | 0.147 | 39.906 | 20.522 | 1.00 | 39.88 |
| atom | 2134 | CB | CYS | 274 | 1.471 | 39.169 | 20.213 | 1.00 | 41.22 |
| atom | 2135 | SG | CYS | 274 | 1.747 | 38.854 | 18.474 | 1.00 | 43.94 |
| atom | 2136 | C | CYS | 274 | -0.232 | 39.438 | 21.918 | 1.00 | 39.19 |
| atom | 2137 | O | CYS | 274 | -0.750 | 38.327 | 22.061 | 1.00 | 38.19 |
| atom | 2138 | N | GLY | 275 | 0.001 | 40.257 | 22.933 | 1.00 | 39.14 |
| atom | 2139 | CA | GLY | 275 | -0.341 | 39.838 | 24.292 | 1.00 | 38.47 |
| atom | 2140 | C | GLY | 275 | 0.493 | 40.598 | 25.319 | 1.00 | 38.10 |
| atom | 2141 | O | GLY | 275 | 1.158 | 41.572 | 25.015 | 1.00 | 36.94 |
| atom | 2142 | N | TYR | 276 | 0.433 | 40.120 | 26.547 | 1.00 | 38.05 |
| atom | 2143 | CA | TYR | 276 | 1.088 | 40.679 | 27.701 | 1.00 | 37.33 |
| atom | 2144 | CB | TYR | 276 | 0.012 | 41.280 | 28.623 | 1.00 | 38.53 |
| atom | 2145 | CG | TYR | 276 | 0.548 | 42.451 | 29.412 | 1.00 | 42.07 |
| atom | 2146 | CD1 | TYR | 276 | 0.509 | 43.741 | 28.895 | 1.00 | 43.14 |
| atom | 2147 | CE1 | TYR | 276 | 1.023 | 44.808 | 29.622 | 1.00 | 43.93 |
| atom | 2148 | CD2 | TYR | 276 | 1.129 | 42.250 | 30.665 | 1.00 | 43.11 |
| atom | 2149 | CE2 | TYR | 276 | 1.637 | 43.307 | 31.398 | 1.00 | 43.61 |
| atom | 2150 | CZ | TYR | 276 | 1.578 | 44.577 | 30.865 | 1.00 | 44.68 |
| atom | 2151 | OH | TYR | 276 | 2.082 | 45.644 | 31.570 | 1.00 | 47.15 |
| atom | 2152 | C | TYR | 276 | 1.898 | 39.613 | 28.436 | 1.00 | 36.60 |
| atom | 2153 | O | TYR | 276 | 1.436 | 38.487 | 28.646 | 1.00 | 36.76 |
| atom | 2154 | N | ARG | 277 | 3.109 | 39.961 | 28.849 | 1.00 | 34.71 |
| atom | 2155 | CA | ARG | 277 | 4.015 | 39.051 | 29.536 | 1.00 | 33.18 |
| atom | 2156 | CB | ARG | 277 | 5.447 | 39.141 | 28.989 | 1.00 | 29.40 |

| atom | 2157 | CG | ARG | 277 | 6.554 | 38.573 | 29.860 | 1.00 | 29.31 |
|------|------|-----|-----|-----|-------|--------|--------|------|-------|
| atom | 2158 | CD | ARG | 277 | 7.954 | 38.735 | 29.271 | 1.00 | 29.38 |
| atom | 2159 | NE | ARG | 277 | 8.204 | 40.155 | 28.967 | 1.00 | 27.72 |
| atom | 2160 | CZ | ARG | 277 | 8.675 | 40.999 | 29.882 | 1.00 | 24.92 |
| atom | 2161 | NH1 | ARG | 277 | 9.039 | 40.597 | 31.097 | 1.00 | 22.62 |
| atom | 2162 | NH2 | ARG | 277 | 8.834 | 42.268 | 29.549 | 1.00 | 26.44 |
| atom | 2163 | C | ARG | 277 | 4.030 | 39.365 | 31.031 | 1.00 | 33.09 |
| atom | 2164 | O | ARG | 277 | 4.083 | 40.541 | 31.380 | 1.00 | 32.34 |
| atom | 2165 | N | ARG | 278 | 3.976 | 38.342 | 31.876 | 1.00 | 32.37 |
| atom | 2166 | CA | ARG | 278 | 4.048 | 38.501 | 33.317 | 1.00 | 32.25 |
| atom | 2167 | CB | ARG | 278 | 2.733 | 38.255 | 34.068 | 1.00 | 33.09 |
| atom | 2168 | CG | ARG | 278 | 1.715 | 39.350 | 33.888 | 1.00 | 34.92 |
| atom | 2169 | CD | ARG | 278 | 0.372 | 39.132 | 34.589 | 1.00 | 36.40 |
| atom | 2170 | NE | ARG | 278 | -0.646 | 39.860 | 33.802 | 1.00 | 36.37 |
| atom | 2171 | CZ | ARG | 278 | -1.313 | 39.255 | 32.812 | 1.00 | 35.81 |
| atom | 2172 | NH1 | ARG | 278 | -1.123 | 37.971 | 32.536 | 1.00 | 32.19 |
| atom | 2173 | NH2 | ARG | 278 | -2.180 | 39.986 | 32.124 | 1.00 | 34.43 |
| atom | 2174 | C | ARG | 278 | 5.146 | 37.581 | 33.869 | 1.00 | 30.67 |
| atom | 2175 | O | ARG | 278 | 5.124 | 37.144 | 35.018 | 1.00 | 30.60 |
| atom | 2176 | N | CYS | 279 | 6.121 | 37.277 | 33.028 | 1.00 | 28.97 |
| atom | 2177 | CA | CYS | 279 | 7.233 | 36.420 | 33.482 | 1.00 | 27.80 |
| atom | 2178 | CB | CYS | 279 | 6.975 | 34.974 | 33.085 | 1.00 | 24.67 |
| atom | 2179 | SG | CYS | 279 | 6.905 | 34.774 | 31.288 | 1.00 | 24.12 |
| atom | 2180 | C | CYS | 279 | 8.540 | 36.965 | 32.915 | 1.00 | 27.03 |
| atom | 2181 | O | CYS | 279 | 8.613 | 38.113 | 32.467 | 1.00 | 25.75 |
| atom | 2182 | N | ARG | 280 | 9.586 | 36.152 | 32.890 | 1.00 | 27.11 |
| atom | 2183 | CA | ARG | 280 | 10.882 | 36.608 | 32.411 | 1.00 | 27.61 |
| atom | 2184 | CB | ARG | 280 | 11.973 | 35.642 | 32.874 | 1.00 | 27.41 |
| atom | 2185 | CG | ARG | 280 | 13.309 | 35.826 | 32.157 | 1.00 | 28.28 |
| atom | 2186 | CD | ARG | 280 | 14.430 | 35.788 | 33.204 | 1.00 | 27.54 |
| atom | 2187 | NE | ARG | 280 | 14.341 | 36.959 | 34.041 | 1.00 | 26.48 |
| atom | 2188 | CZ | ARG | 280 | 14.626 | 36.967 | 35.342 | 1.00 | 26.75 |
| atom | 2189 | NH1 | ARG | 280 | 15.010 | 35.869 | 35.942 | 1.00 | 23.17 |
| atom | 2190 | NH2 | ARG | 280 | 14.514 | 38.112 | 36.010 | 1.00 | 25.26 |
| atom | 2191 | C | ARG | 280 | 10.990 | 36.731 | 30.895 | 1.00 | 28.06 |
| atom | 2192 | O | ARG | 280 | 10.664 | 35.733 | 30.245 | 1.00 | 28.91 |

| atom | 2193 | N   | ALA | 281 | 11.497 | 37.862 | 30.412 | 1.00 | 26.87 |
| atom | 2194 | CA  | ALA | 281 | 11.723 | 38.001 | 28.979 | 1.00 | 27.69 |
| atom | 2195 | CB  | ALA | 281 | 11.878 | 39.445 | 28.520 | 1.00 | 29.87 |
| atom | 2196 | C   | ALA | 281 | 13.052 | 37.311 | 28.674 | 1.00 | 27.81 |
| atom | 2197 | O   | ALA | 281 | 14.015 | 37.544 | 29.447 | 1.00 | 28.27 |
| atom | 2198 | N   | SER | 282 | 13.127 | 36.562 | 27.581 | 1.00 | 26.40 |
| atom | 2199 | CA  | SER | 282 | 14.412 | 35.903 | 27.302 | 1.00 | 26.12 |
| atom | 2200 | CB  | SER | 282 | 14.267 | 34.916 | 26.133 | 1.00 | 24.24 |
| atom | 2201 | OG  | SER | 282 | 14.011 | 35.718 | 24.963 | 1.00 | 26.18 |
| atom | 2202 | C   | SER | 282 | 15.497 | 36.884 | 26.885 | 1.00 | 26.32 |
| atom | 2203 | O   | SER | 282 | 16.673 | 36.555 | 27.037 | 1.00 | 26.86 |
| atom | 2204 | N   | GLY | 283 | 15.158 | 38.020 | 26.269 | 1.00 | 26.21 |
| atom | 2205 | CA  | GLY | 283 | 16.121 | 38.931 | 25.717 | 1.00 | 24.50 |
| atom | 2206 | C   | GLY | 283 | 16.592 | 40.124 | 26.493 | 1.00 | 24.78 |
| atom | 2207 | O   | GLY | 283 | 17.092 | 41.075 | 25.860 | 1.00 | 24.42 |
| atom | 2208 | N   | VAL | 284 | 16.469 | 40.153 | 27.821 | 1.00 | 23.56 |
| atom | 2209 | CA  | VAL | 284 | 16.972 | 41.302 | 28.563 | 1.00 | 23.33 |
| atom | 2210 | CB  | VAL | 284 | 16.024 | 41.743 | 29.690 | 1.00 | 22.69 |
| atom | 2211 | CG1 | VAL | 284 | 14.793 | 42.445 | 29.124 | 1.00 | 22.71 |
| atom | 2212 | CG2 | VAL | 284 | 15.627 | 40.552 | 30.570 | 1.00 | 20.58 |
| atom | 2213 | C   | VAL | 284 | 18.364 | 41.045 | 29.122 | 1.00 | 24.38 |
| atom | 2214 | O   | VAL | 284 | 18.764 | 39.929 | 29.432 | 1.00 | 23.07 |
| atom | 2215 | N   | LEU | 285 | 19.104 | 42.126 | 29.437 | 1.00 | 25.00 |
| atom | 2216 | CA  | LEU | 285 | 20.432 | 41.988 | 29.991 | 1.00 | 24.61 |
| atom | 2217 | CB  | LEU | 285 | 21.046 | 43.364 | 30.352 | 1.00 | 24.12 |
| atom | 2218 | CG  | LEU | 285 | 22.516 | 43.262 | 30.826 | 1.00 | 24.45 |
| atom | 2219 | CD1 | LEU | 285 | 23.371 | 42.600 | 29.745 | 1.00 | 17.79 |
| atom | 2220 | CD2 | LEU | 285 | 23.104 | 44.611 | 31.224 | 1.00 | 22.93 |
| atom | 2221 | C   | LEU | 285 | 20.497 | 41.091 | 31.221 | 1.00 | 25.33 |
| atom | 2222 | O   | LEU | 285 | 21.516 | 40.423 | 31.420 | 1.00 | 24.18 |
| atom | 2223 | N   | THR | 286 | 19.496 | 41.148 | 32.099 | 1.00 | 25.22 |
| atom | 2224 | CA  | THR | 286 | 19.455 | 40.392 | 33.324 | 1.00 | 25.36 |
| atom | 2225 | CB  | THR | 286 | 18.600 | 41.148 | 34.381 | 1.00 | 26.54 |
| atom | 2226 | OG1 | THR | 286 | 17.319 | 41.448 | 33.805 | 1.00 | 25.36 |
| atom | 2227 | CG2 | THR | 286 | 19.284 | 42.427 | 34.818 | 1.00 | 26.79 |
| atom | 2228 | C   | THR | 286 | 18.864 | 38.996 | 33.247 | 1.00 | 25.62 |

| atom | 2229 | O | THR | 286 | 18.786 | 38.333 | 34.300 | 1.00 | 25.94 |
|------|------|-----|-----|-----|--------|--------|--------|------|-------|
| atom | 2230 | N | THR | 287 | 18.455 | 38.476 | 32.096 | 1.00 | 25.49 |
| atom | 2231 | CA | THR | 287 | 17.918 | 37.120 | 32.066 | 1.00 | 25.72 |
| atom | 2232 | CB | THR | 287 | 17.702 | 36.664 | 30.606 | 1.00 | 26.65 |
| atom | 2233 | OG1 | THR | 287 | 16.939 | 37.679 | 29.953 | 1.00 | 25.87 |
| atom | 2234 | CG2 | THR | 287 | 17.038 | 35.298 | 30.609 | 1.00 | 25.07 |
| atom | 2235 | C | THR | 287 | 18.851 | 36.098 | 32.711 | 1.00 | 26.17 |
| atom | 2236 | O | THR | 287 | 18.484 | 35.245 | 33.527 | 1.00 | 26.55 |
| atom | 2237 | N | SER | 288 | 20.109 | 36.143 | 32.284 | 1.00 | 25.33 |
| atom | 2238 | CA | SER | 288 | 21.108 | 35.184 | 32.711 | 1.00 | 24.77 |
| atom | 2239 | CB | SER | 288 | 22.316 | 35.321 | 31.782 | 1.00 | 25.26 |
| atom | 2240 | OG | SER | 288 | 23.383 | 34.559 | 32.293 | 1.00 | 30.68 |
| atom | 2241 | C | SER | 288 | 21.430 | 35.323 | 34.184 | 1.00 | 24.87 |
| atom | 2242 | O | SER | 288 | 21.342 | 34.343 | 34.920 | 1.00 | 24.52 |
| atom | 2243 | N | CYS | 289 | 21.774 | 36.524 | 34.653 | 1.00 | 24.19 |
| atom | 2244 | CA | CYS | 289 | 22.085 | 36.744 | 36.056 | 1.00 | 22.89 |
| atom | 2245 | CB | CYS | 289 | 22.600 | 38.157 | 36.270 | 1.00 | 21.13 |
| atom | 2246 | SG | CYS | 289 | 22.956 | 38.605 | 38.001 | 1.00 | 23.20 |
| atom | 2247 | C | CYS | 289 | 20.839 | 36.477 | 36.900 | 1.00 | 23.34 |
| atom | 2248 | O | CYS | 289 | 20.901 | 35.838 | 37.949 | 1.00 | 23.17 |
| atom | 2249 | N | GLY | 290 | 19.686 | 37.000 | 36.476 | 1.00 | 23.20 |
| atom | 2250 | CA | GLY | 290 | 18.438 | 36.804 | 37.206 | 1.00 | 23.05 |
| atom | 2251 | C | GLY | 290 | 18.071 | 35.335 | 37.248 | 1.00 | 24.26 |
| atom | 2252 | O | GLY | 290 | 17.768 | 34.801 | 38.325 | 1.00 | 26.03 |
| atom | 2253 | N | ASN | 291 | 18.094 | 34.616 | 36.125 | 1.00 | 24.82 |
| atom | 2254 | CA | ASN | 291 | 17.785 | 33.177 | 36.184 | 1.00 | 26.10 |
| atom | 2255 | CB | ASN | 291 | 17.742 | 32.502 | 34.811 | 1.00 | 24.06 |
| atom | 2256 | CG | ASN | 291 | 16.564 | 32.975 | 33.985 | 1.00 | 26.25 |
| atom | 2257 | OD1 | ASN | 291 | 16.595 | 32.931 | 32.748 | 1.00 | 28.06 |
| atom | 2258 | ND2 | ASN | 291 | 15.537 | 33.454 | 34.676 | 1.00 | 23.50 |
| atom | 2259 | C | ASN | 291 | 18.788 | 32.470 | 37.092 | 1.00 | 26.11 |
| atom | 2260 | O | ASN | 291 | 18.446 | 31.546 | 37.836 | 1.00 | 26.06 |
| atom | 2261 | N | THR | 292 | 20.053 | 32.879 | 37.060 | 1.00 | 26.77 |
| atom | 2262 | CA | THR | 292 | 21.067 | 32.258 | 37.901 | 1.00 | 28.20 |
| atom | 2263 | CB | THR | 292 | 22.491 | 32.695 | 37.505 | 1.00 | 28.50 |
| atom | 2264 | OG1 | THR | 292 | 22.657 | 32.218 | 36.155 | 1.00 | 31.29 |

| atom | 2265 | CG2 | THR | 292 | 23.544 | 32.068 | 38.395 | 1.00 | 26.89 |
|------|------|-----|-----|-----|--------|--------|--------|------|-------|
| atom | 2266 | C   | THR | 292 | 20.793 | 32.494 | 39.378 | 1.00 | 27.77 |
| atom | 2267 | O   | THR | 292 | 20.813 | 31.513 | 40.130 | 1.00 | 29.08 |
| atom | 2268 | N   | LEU | 293 | 20.475 | 33.704 | 39.789 | 1.00 | 27.43 |
| atom | 2269 | CA  | LEU | 293 | 20.127 | 33.956 | 41.180 | 1.00 | 27.75 |
| atom | 2270 | CB  | LEU | 293 | 19.938 | 35.465 | 41.391 | 1.00 | 28.04 |
| atom | 2271 | CG  | LEU | 293 | 21.213 | 36.289 | 41.179 | 1.00 | 26.98 |
| atom | 2272 | CD1 | LEU | 293 | 20.886 | 37.773 | 41.254 | 1.00 | 27.35 |
| atom | 2273 | CD2 | LEU | 293 | 22.225 | 35.868 | 42.236 | 1.00 | 27.96 |
| atom | 2274 | C   | LEU | 293 | 18.858 | 33.247 | 41.620 | 1.00 | 27.75 |
| atom | 2275 | O   | LEU | 293 | 18.794 | 32.681 | 42.712 | 1.00 | 27.79 |
| atom | 2276 | N   | THR | 294 | 17.841 | 33.257 | 40.772 | 1.00 | 28.44 |
| atom | 2277 | CA  | THR | 294 | 16.553 | 32.641 | 41.140 | 1.00 | 29.61 |
| atom | 2278 | CB  | THR | 294 | 15.525 | 33.085 | 40.083 | 1.00 | 30.26 |
| atom | 2279 | OG1 | THR | 294 | 15.574 | 34.524 | 40.200 | 1.00 | 31.67 |
| atom | 2280 | CG2 | THR | 294 | 14.133 | 32.542 | 40.321 | 1.00 | 28.70 |
| atom | 2281 | C   | THR | 294 | 16.635 | 31.132 | 41.271 | 1.00 | 28.50 |
| atom | 2282 | O   | THR | 294 | 16.126 | 30.522 | 42.212 | 1.00 | 27.94 |
| atom | 2283 | N   | CYS | 295 | 17.322 | 30.528 | 40.316 | 1.00 | 27.44 |
| atom | 2284 | CA  | CYS | 295 | 17.494 | 29.084 | 40.307 | 1.00 | 28.31 |
| atom | 2285 | CB  | CYS | 295 | 18.238 | 28.644 | 39.046 | 1.00 | 24.99 |
| atom | 2286 | SG  | CYS | 295 | 18.381 | 26.845 | 38.982 | 1.00 | 26.71 |
| atom | 2287 | C   | CYS | 295 | 18.248 | 28.651 | 41.565 | 1.00 | 29.14 |
| atom | 2288 | O   | CYS | 295 | 17.820 | 27.781 | 42.318 | 1.00 | 28.71 |
| atom | 2289 | N   | TYR | 296 | 19.364 | 29.334 | 41.819 | 1.00 | 29.50 |
| atom | 2290 | CA  | TYR | 296 | 20.189 | 29.101 | 42.989 | 1.00 | 30.30 |
| atom | 2291 | CB  | TYR | 296 | 21.337 | 30.127 | 42.985 | 1.00 | 28.86 |
| atom | 2292 | CG  | TYR | 296 | 22.187 | 30.070 | 44.234 | 1.00 | 27.72 |
| atom | 2293 | CD1 | TYR | 296 | 23.291 | 29.220 | 44.310 | 1.00 | 27.11 |
| atom | 2294 | CE1 | TYR | 296 | 24.060 | 29.188 | 45.446 | 1.00 | 26.95 |
| atom | 2295 | CD2 | TYR | 296 | 21.877 | 30.851 | 45.335 | 1.00 | 27.45 |
| atom | 2296 | CE2 | TYR | 296 | 22.632 | 30.805 | 46.497 | 1.00 | 27.35 |
| atom | 2297 | CZ  | TYR | 296 | 23.737 | 29.982 | 46.534 | 1.00 | 27.89 |
| atom | 2298 | OH  | TYR | 296 | 24.519 | 29.919 | 47.667 | 1.00 | 27.59 |
| atom | 2299 | C   | TYR | 296 | 19.407 | 29.232 | 44.295 | 1.00 | 30.73 |
| atom | 2300 | O   | TYR | 296 | 19.509 | 28.404 | 45.202 | 1.00 | 31.40 |

| atom | 2301 | N | LEU | 297 | 18.679 | 30.338 | 44.442 | 1.00 | 31.05 |
|------|------|-----|-----|-----|--------|--------|--------|------|-------|
| atom | 2302 | CA | LEU | 297 | 17.901 | 30.596 | 45.639 | 1.00 | 31.15 |
| atom | 2303 | CB | LEU | 297 | 17.242 | 31.976 | 45.577 | 1.00 | 30.66 |
| atom | 2304 | CG | LEU | 297 | 16.195 | 32.284 | 46.649 | 1.00 | 32.49 |
| atom | 2305 | CD1 | LEU | 297 | 16.752 | 32.354 | 48.068 | 1.00 | 30.72 |
| atom | 2306 | CD2 | LEU | 297 | 15.501 | 33.588 | 46.284 | 1.00 | 33.88 |
| atom | 2307 | C | LEU | 297 | 16.900 | 29.468 | 45.907 | 1.00 | 30.59 |
| atom | 2308 | O | LEU | 297 | 16.913 | 28.924 | 47.009 | 1.00 | 28.99 |
| atom | 2309 | N | LYS | 298 | 16.121 | 29.057 | 44.920 | 1.00 | 30.38 |
| atom | 2310 | CA | LYS | 298 | 15.134 | 28.007 | 45.066 | 1.00 | 30.73 |
| atom | 2311 | CB | LYS | 298 | 14.187 | 27.913 | 43.853 | 1.00 | 26.40 |
| atom | 2312 | CG | LYS | 298 | 13.421 | 29.212 | 43.648 | 1.00 | 27.14 |
| atom | 2313 | CD | LYS | 298 | 12.557 | 29.198 | 42.381 | 1.00 | 23.22 |
| atom | 2314 | CE | LYS | 298 | 11.618 | 30.402 | 42.435 | 1.00 | 22.06 |
| atom | 2315 | NZ | LYS | 298 | 10.863 | 30.571 | 41.164 | 1.00 | 23.44 |
| atom | 2316 | C | LYS | 298 | 15.747 | 26.629 | 45.273 | 1.00 | 31.80 |
| atom | 2317 | O | LYS | 298 | 15.278 | 25.919 | 46.166 | 1.00 | 31.91 |
| atom | 2318 | N | ALA | 299 | 16.764 | 26.292 | 44.489 | 1.00 | 31.31 |
| atom | 2319 | CA | ALA | 299 | 17.417 | 24.996 | 44.613 | 1.00 | 32.02 |
| atom | 2320 | CB | ALA | 299 | 18.404 | 24.791 | 43.477 | 1.00 | 28.54 |
| atom | 2321 | C | ALA | 299 | 18.101 | 24.838 | 45.969 | 1.00 | 32.66 |
| atom | 2322 | O | ALA | 299 | 18.107 | 23.748 | 46.563 | 1.00 | 33.07 |
| atom | 2323 | N | SER | 300 | 18.691 | 25.926 | 46.458 | 1.00 | 32.43 |
| atom | 2324 | CA | SER | 300 | 19.321 | 25.944 | 47.767 | 1.00 | 33.37 |
| atom | 2325 | CB | SER | 300 | 19.965 | 27.286 | 48.129 | 1.00 | 34.48 |
| atom | 2326 | OG | SER | 300 | 21.213 | 27.475 | 47.523 | 1.00 | 37.45 |
| atom | 2327 | C | SER | 300 | 18.278 | 25.709 | 48.866 | 1.00 | 32.74 |
| atom | 2328 | O | SER | 300 | 18.551 | 24.955 | 49.802 | 1.00 | 32.46 |
| atom | 2329 | N | ALA | 301 | 17.131 | 26.381 | 48.752 | 1.00 | 31.85 |
| atom | 2330 | CA | ALA | 301 | 16.079 | 26.214 | 49.756 | 1.00 | 32.50 |
| atom | 2331 | CB | ALA | 301 | 14.900 | 27.149 | 49.558 | 1.00 | 28.76 |
| atom | 2332 | C | ALA | 301 | 15.558 | 24.774 | 49.720 | 1.00 | 32.40 |
| atom | 2333 | O | ALA | 301 | 15.285 | 24.154 | 50.733 | 1.00 | 33.61 |
| atom | 2334 | N | ALA | 302 | 15.460 | 24.234 | 48.528 | 1.00 | 32.04 |
| atom | 2335 | CA | ALA | 302 | 15.022 | 22.904 | 48.208 | 1.00 | 31.76 |
| atom | 2336 | CB | ALA | 302 | 14.835 | 22.770 | 46.701 | 1.00 | 28.84 |

| atom | 2337 | C | ALA | 302 | 16.008 | 21.873 | 48.733 | 1.00 | 32.29 |
|------|------|------|------|------|--------|--------|--------|------|-------|
| atom | 2338 | O | ALA | 302 | 15.536 | 20.875 | 49.272 | 1.00 | 32.46 |
| atom | 2339 | N | CYS | 303 | 17.319 | 22.109 | 48.646 | 1.00 | 32.27 |
| atom | 2340 | CA | CYS | 303 | 18.282 | 21.164 | 49.188 | 1.00 | 32.46 |
| atom | 2341 | CB | CYS | 303 | 19.740 | 21.460 | 48.912 | 1.00 | 28.56 |
| atom | 2342 | SG | CYS | 303 | 20.386 | 21.344 | 47.218 | 1.00 | 28.88 |
| atom | 2343 | C | CYS | 303 | 18.047 | 21.084 | 50.705 | 1.00 | 34.28 |
| atom | 2344 | O | CYS | 303 | 18.061 | 19.976 | 51.246 | 1.00 | 34.99 |
| atom | 2345 | N | ARG | 304 | 17.769 | 22.207 | 51.369 | 1.00 | 34.95 |
| atom | 2346 | CA | ARG | 304 | 17.520 | 22.205 | 52.801 | 1.00 | 36.53 |
| atom | 2347 | CB | ARG | 304 | 17.402 | 23.603 | 53.397 | 1.00 | 38.88 |
| atom | 2348 | CG | ARG | 304 | 18.344 | 24.620 | 52.810 | 1.00 | 41.44 |
| atom | 2349 | CD | ARG | 304 | 19.554 | 25.040 | 53.582 | 1.00 | 41.06 |
| atom | 2350 | NE | ARG | 304 | 19.349 | 26.338 | 54.195 | 1.00 | 45.30 |
| atom | 2351 | CZ | ARG | 304 | 20.120 | 27.415 | 54.257 | 1.00 | 44.75 |
| atom | 2352 | NH1 | ARG | 304 | 21.316 | 27.567 | 53.713 | 1.00 | 43.50 |
| atom | 2353 | NH2 | ARG | 304 | 19.670 | 28.490 | 54.907 | 1.00 | 44.32 |
| atom | 2354 | C | ARG | 304 | 16.233 | 21.423 | 53.098 | 1.00 | 37.68 |
| atom | 2355 | O | ARG | 304 | 16.233 | 20.610 | 54.031 | 1.00 | 37.15 |
| atom | 2356 | N | ALA | 305 | 15.208 | 21.640 | 52.274 | 1.00 | 36.72 |
| atom | 2357 | CA | ALA | 305 | 13.959 | 20.919 | 52.419 | 1.00 | 37.33 |
| atom | 2358 | CB | ALA | 305 | 12.906 | 21.441 | 51.441 | 1.00 | 33.97 |
| atom | 2359 | C | ALA | 305 | 14.100 | 19.417 | 52.180 | 1.00 | 37.93 |
| atom | 2360 | O | ALA | 305 | 13.467 | 18.664 | 52.917 | 1.00 | 37.71 |
| atom | 2361 | N | ALA | 306 | 14.896 | 18.988 | 51.205 | 1.00 | 37.98 |
| atom | 2362 | CA | ALA | 306 | 15.052 | 17.584 | 50.874 | 1.00 | 38.67 |
| atom | 2363 | CB | ALA | 306 | 15.514 | 17.448 | 49.427 | 1.00 | 36.26 |
| atom | 2364 | C | ALA | 306 | 16.003 | 16.877 | 51.830 | 1.00 | 39.76 |
| atom | 2365 | O | ALA | 306 | 16.151 | 15.649 | 51.842 | 1.00 | 40.58 |
| atom | 2366 | N | LYS | 307 | 16.746 | 17.629 | 52.626 | 1.00 | 40.63 |
| atom | 2367 | CA | LYS | 307 | 17.689 | 17.077 | 53.582 | 1.00 | 41.94 |
| atom | 2368 | CB | LYS | 307 | 16.997 | 16.055 | 54.482 | 1.00 | 40.64 |
| atom | 2369 | CG | LYS | 307 | 16.487 | 16.494 | 55.819 | 1.00 | 42.35 |
| atom | 2370 | CD | LYS | 307 | 15.056 | 16.995 | 55.852 | 1.00 | 46.63 |
| atom | 2371 | CE | LYS | 307 | 14.306 | 16.521 | 57.096 | 1.00 | 45.86 |
| atom | 2372 | NZ | LYS | 307 | 15.169 | 16.642 | 58.311 | 1.00 | 48.23 |

| atom | 2373 | C   | LYS | 307 | 18.904 | 16.455 | 52.906 | 1.00 | 42.62 |
|------|------|-----|-----|-----|--------|--------|--------|------|-------|
| atom | 2374 | O   | LYS | 307 | 19.502 | 15.501 | 53.418 | 1.00 | 42.87 |
| atom | 2375 | N   | LEU | 308 | 19.285 | 16.982 | 51.743 | 1.00 | 43.44 |
| atom | 2376 | CA  | LEU | 308 | 20.479 | 16.488 | 51.052 | 1.00 | 42.60 |
| atom | 2377 | CB  | LEU | 308 | 20.587 | 17.030 | 49.632 | 1.00 | 43.54 |
| atom | 2378 | CG  | LEU | 308 | 19.390 | 16.745 | 48.721 | 1.00 | 42.91 |
| atom | 2379 | CD1 | LEU | 308 | 19.542 | 17.379 | 47.354 | 1.00 | 42.57 |
| atom | 2380 | CD2 | LEU | 308 | 19.196 | 15.244 | 48.592 | 1.00 | 44.10 |
| atom | 2381 | C   | LEU | 308 | 21.679 | 16.884 | 51.912 | 1.00 | 42.07 |
| atom | 2382 | O   | LEU | 308 | 21.675 | 17.877 | 52.645 | 1.00 | 41.60 |
| atom | 2383 | N   | GLN | 309 | 22.716 | 16.071 | 51.846 | 1.00 | 40.83 |
| atom | 2384 | CA  | GLN | 309 | 23.890 | 16.235 | 52.676 | 1.00 | 41.21 |
| atom | 2385 | CB  | GLN | 309 | 24.059 | 15.006 | 53.568 | 1.00 | 43.78 |
| atom | 2386 | CG  | GLN | 309 | 23.843 | 13.649 | 52.946 | 1.00 | 49.41 |
| atom | 2387 | CD  | GLN | 309 | 22.445 | 13.366 | 52.410 | 1.00 | 52.42 |
| atom | 2388 | OE1 | GLN | 309 | 22.248 | 13.146 | 51.199 | 1.00 | 50.59 |
| atom | 2389 | NE2 | GLN | 309 | 21.464 | 13.441 | 53.314 | 1.00 | 52.95 |
| atom | 2390 | C   | GLN | 309 | 25.170 | 16.525 | 51.906 | 1.00 | 41.00 |
| atom | 2391 | O   | GLN | 309 | 25.500 | 15.958 | 50.857 | 1.00 | 39.77 |
| atom | 2392 | N   | ASP | 310 | 25.890 | 17.514 | 52.459 | 1.00 | 40.51 |
| atom | 2393 | CA  | ASP | 310 | 27.156 | 17.937 | 51.857 | 1.00 | 41.23 |
| atom | 2394 | CB  | ASP | 310 | 28.147 | 16.784 | 51.985 | 1.00 | 44.63 |
| atom | 2395 | CG  | ASP | 310 | 29.599 | 17.169 | 51.899 | 1.00 | 48.67 |
| atom | 2396 | OD1 | ASP | 310 | 30.490 | 16.278 | 51.966 | 1.00 | 53.08 |
| atom | 2397 | OD2 | ASP | 310 | 29.863 | 18.377 | 51.765 | 1.00 | 49.31 |
| atom | 2398 | C   | ASP | 310 | 26.914 | 18.349 | 50.406 | 1.00 | 40.11 |
| atom | 2399 | O   | ASP | 310 | 27.638 | 17.954 | 49.498 | 1.00 | 40.33 |
| atom | 2400 | N   | CYS | 311 | 25.878 | 19.132 | 50.169 | 1.00 | 38.97 |
| atom | 2401 | CA  | CYS | 311 | 25.486 | 19.625 | 48.880 | 1.00 | 38.92 |
| atom | 2402 | CB  | CYS | 311 | 24.175 | 20.433 | 48.939 | 1.00 | 40.68 |
| atom | 2403 | SG  | CYS | 311 | 22.675 | 19.479 | 48.803 | 1.00 | 43.39 |
| atom | 2404 | C   | CYS | 311 | 26.470 | 20.632 | 48.278 | 1.00 | 37.89 |
| atom | 2405 | O   | CYS | 311 | 26.947 | 21.574 | 48.906 | 1.00 | 38.01 |
| atom | 2406 | N   | THR | 312 | 26.696 | 20.444 | 46.996 | 1.00 | 36.23 |
| atom | 2407 | CA  | THR | 312 | 27.512 | 21.358 | 46.203 | 1.00 | 34.41 |
| atom | 2408 | CB  | THR | 312 | 28.885 | 20.764 | 45.922 | 1.00 | 34.03 |

| atom | 2409 | OG1 | THR | 312 | 29.444 | 20.309 | 47.160 | 1.00 | 34.43 |
|------|------|-----|-----|-----|--------|--------|--------|------|-------|
| atom | 2410 | CG2 | THR | 312 | 29.779 | 21.834 | 45.320 | 1.00 | 34.60 |
| atom | 2411 | C   | THR | 312 | 26.738 | 21.635 | 44.918 | 1.00 | 33.28 |
| atom | 2412 | 0   | THR | 312 | 26.373 | 20.706 | 44.199 | 1.00 | 32.18 |
| atom | 2413 | N   | MET | 313 | 26.393 | 22.888 | 44.698 | 1.00 | 33.09 |
| atom | 2414 | CA  | MET | 313 | 25.620 | 23.231 | 43.516 | 1.00 | 33.28 |
| atom | 2415 | CB  | MET | 313 | 24.481 | 24.186 | 43.882 | 1.00 | 35.73 |
| atom | 2416 | CG  | MET | 313 | 23.586 | 23.596 | 44.971 | 1.00 | 38.44 |
| atom | 2417 | SD  | MET | 313 | 21.940 | 24.278 | 44.982 | 1.00 | 40.96 |
| atom | 2418 | CE  | MET | 313 | 22.192 | 26.003 | 44.619 | 1.00 | 38.76 |
| atom | 2419 | C   | MET | 313 | 26.457 | 23.903 | 42.443 | 1.00 | 32.45 |
| atom | 2420 | 0   | MET | 313 | 27.323 | 24.694 | 42.775 | 1.00 | 33.24 |
| atom | 2421 | N   | LEU | 314 | 26.160 | 23.604 | 41.193 | 1.00 | 31.30 |
| atom | 2422 | CA  | LEU | 314 | 26.763 | 24.272 | 40.065 | 1.00 | 30.25 |
| atom | 2423 | CB  | LEU | 314 | 27.567 | 23.377 | 39.109 | 1.00 | 29.01 |
| atom | 2424 | CG  | LEU | 314 | 28.303 | 24.207 | 38.034 | 1.00 | 26.14 |
| atom | 2425 | CD1 | LEU | 314 | 29.179 | 25.271 | 38.688 | 1.00 | 21.68 |
| atom | 2426 | CD2 | LEU | 314 | 29.111 | 23.325 | 37.105 | 1.00 | 24.63 |
| atom | 2427 | C   | LEU | 314 | 25.617 | 24.907 | 39.271 | 1.00 | 29.08 |
| atom | 2428 | 0   | LEU | 314 | 24.819 | 24.131 | 38.742 | 1.00 | 29.26 |
| atom | 2429 | N   | VAL | 315 | 25.511 | 26.224 | 39.245 | 1.00 | 29.16 |
| atom | 2430 | CA  | VAL | 315 | 24.402 | 26.874 | 38.546 | 1.00 | 29.03 |
| atom | 2431 | CB  | VAL | 315 | 23.578 | 27.763 | 39.497 | 1.00 | 27.72 |
| atom | 2432 | CG1 | VAL | 315 | 22.330 | 28.276 | 38.788 | 1.00 | 25.33 |
| atom | 2433 | CG2 | VAL | 315 | 23.219 | 27.070 | 40.809 | 1.00 | 27.07 |
| atom | 2434 | C   | VAL | 315 | 24.829 | 27.788 | 37.400 | 1.00 | 29.94 |
| atom | 2435 | 0   | VAL | 315 | 25.690 | 28.651 | 37.549 | 1.00 | 30.61 |
| atom | 2436 | N   | ASN | 316 | 24.215 | 27.624 | 36.246 | 1.00 | 29.96 |
| atom | 2437 | CA  | ASN | 316 | 24.387 | 28.422 | 35.046 | 1.00 | 31.16 |
| atom | 2438 | CB  | ASN | 316 | 25.104 | 27.686 | 33.920 | 1.00 | 32.98 |
| atom | 2439 | CG  | ASN | 316 | 26.588 | 27.417 | 34.065 | 1.00 | 35.77 |
| atom | 2440 | OD1 | ASN | 316 | 26.956 | 26.417 | 34.688 | 1.00 | 36.41 |
| atom | 2441 | ND2 | ASN | 316 | 27.454 | 28.255 | 33.497 | 1.00 | 34.65 |
| atom | 2442 | C   | ASN | 316 | 22.980 | 28.794 | 34.549 | 1.00 | 31.23 |
| atom | 2443 | 0   | ASN | 316 | 22.308 | 27.973 | 33.919 | 1.00 | 29.54 |
| atom | 2444 | N   | GLY | 317 | 22.436 | 29.975 | 34.812 | 1.00 | 31.75 |

| atom | 2445 | CA  | GLY | 317 | 21.076 | 30.292 | 34.354 | 1.00 | 32.75 |
|------|------|-----|-----|-----|--------|--------|--------|------|-------|
| atom | 2446 | C   | GLY | 317 | 20.012 | 29.356 | 34.917 | 1.00 | 33.30 |
| atom | 2447 | O   | GLY | 317 | 19.891 | 29.208 | 36.129 | 1.00 | 32.73 |
| atom | 2448 | N   | ASP | 318 | 19.219 | 28.664 | 34.125 | 1.00 | 33.63 |
| atom | 2449 | CA  | ASP | 318 | 18.220 | 27.713 | 34.553 | 1.00 | 35.29 |
| atom | 2450 | CB  | ASP | 318 | 17.121 | 27.609 | 33.494 | 1.00 | 39.36 |
| atom | 2451 | CG  | ASP | 318 | 17.662 | 27.394 | 32.101 | 1.00 | 43.35 |
| atom | 2452 | OD1 | ASP | 318 | 17.068 | 26.586 | 31.347 | 1.00 | 47.58 |
| atom | 2453 | OD2 | ASP | 318 | 18.671 | 28.032 | 31.708 | 1.00 | 45.20 |
| atom | 2454 | C   | ASP | 318 | 18.832 | 26.327 | 34.744 | 1.00 | 35.84 |
| atom | 2455 | O   | ASP | 318 | 18.164 | 25.391 | 35.176 | 1.00 | 36.69 |
| atom | 2456 | N   | ASP | 319 | 20.090 | 26.186 | 34.354 | 1.00 | 35.25 |
| atom | 2457 | CA  | ASP | 319 | 20.804 | 24.929 | 34.416 | 1.00 | 35.84 |
| atom | 2458 | CB  | ASP | 319 | 22.011 | 25.025 | 33.470 | 1.00 | 39.39 |
| atom | 2459 | CG  | ASP | 319 | 21.910 | 23.880 | 32.479 | 1.00 | 44.23 |
| atom | 2460 | OD1 | ASP | 319 | 21.236 | 24.045 | 31.440 | 1.00 | 48.80 |
| atom | 2461 | OD2 | ASP | 319 | 22.508 | 22.844 | 32.816 | 1.00 | 45.23 |
| atom | 2462 | C   | ASP | 319 | 21.288 | 24.628 | 35.829 | 1.00 | 34.61 |
| atom | 2463 | O   | ASP | 319 | 22.041 | 25.446 | 36.361 | 1.00 | 34.77 |
| atom | 2464 | N   | LEU | 320 | 20.887 | 23.491 | 36.372 | 1.00 | 32.49 |
| atom | 2465 | CA  | LEU | 320 | 21.271 | 23.154 | 37.731 | 1.00 | 32.39 |
| atom | 2466 | CB  | LEU | 320 | 20.061 | 23.318 | 38.651 | 1.00 | 30.55 |
| atom | 2467 | CG  | LEU | 320 | 20.187 | 22.939 | 40.123 | 1.00 | 30.70 |
| atom | 2468 | CD1 | LEU | 320 | 21.083 | 23.888 | 40.890 | 1.00 | 29.25 |
| atom | 2469 | CD2 | LEU | 320 | 18.766 | 22.910 | 40.688 | 1.00 | 30.47 |
| atom | 2470 | C   | LEU | 320 | 21.796 | 21.733 | 37.893 | 1.00 | 32.52 |
| atom | 2471 | O   | LEU | 320 | 21.145 | 20.756 | 37.522 | 1.00 | 31.37 |
| atom | 2472 | N   | VAL | 321 | 22.971 | 21.662 | 38.522 | 1.00 | 32.62 |
| atom | 2473 | CA  | VAL | 321 | 23.588 | 20.393 | 38.858 | 1.00 | 32.63 |
| atom | 2474 | CB  | VAL | 321 | 24.846 | 20.032 | 38.064 | 1.00 | 35.33 |
| atom | 2475 | CG1 | VAL | 321 | 25.731 | 19.046 | 38.839 | 1.00 | 34.44 |
| atom | 2476 | CG2 | VAL | 321 | 24.395 | 19.341 | 36.783 | 1.00 | 34.79 |
| atom | 2477 | C   | VAL | 321 | 23.887 | 20.397 | 40.359 | 1.00 | 32.34 |
| atom | 2478 | O   | VAL | 321 | 24.483 | 21.347 | 40.870 | 1.00 | 32.02 |
| atom | 2479 | N   | VAL | 322 | 23.442 | 19.326 | 41.012 | 1.00 | 31.17 |
| atom | 2480 | CA  | VAL | 322 | 23.632 | 19.198 | 42.447 | 1.00 | 30.05 |

| atom | 2481 | CB  | VAL | 322 | 22.323 | 19.112 | 43.251 | 1.00 28.39 |
|------|------|-----|-----|-----|--------|--------|--------|------------|
| atom | 2482 | CG1 | VAL | 322 | 22.631 | 18.851 | 44.725 | 1.00 30.18 |
| atom | 2483 | CG2 | VAL | 322 | 21.486 | 20.375 | 43.195 | 1.00 25.92 |
| atom | 2484 | C   | VAL | 322 | 24.450 | 17.942 | 42.719 | 1.00 29.97 |
| atom | 2485 | O   | VAL | 322 | 24.128 | 16.887 | 42.191 | 1.00 29.76 |
| atom | 2486 | N   | ILE | 323 | 25.515 | 18.060 | 43.502 | 1.00 30.71 |
| atom | 2487 | CA  | ILE | 323 | 26.321 | 16.878 | 43.816 | 1.00 31.46 |
| atom | 2488 | CB  | ILE | 323 | 27.721 | 16.883 | 43.212 | 1.00 29.74 |
| atom | 2489 | CG2 | ILE | 323 | 28.563 | 15.803 | 43.903 | 1.00 28.57 |
| atom | 2490 | CG1 | ILE | 323 | 27.694 | 16.709 | 41.691 | 1.00 26.76 |
| atom | 2491 | CD1 | ILE | 323 | 28.974 | 17.151 | 41.015 | 1.00 28.40 |
| atom | 2492 | C   | ILE | 323 | 26.409 | 16.806 | 45.343 | 1.00 33.23 |
| atom | 2493 | O   | ILE | 323 | 26.832 | 17.778 | 45.970 | 1.00 33.11 |
| atom | 2494 | N   | CYS | 324 | 26.033 | 15.654 | 45.885 | 1.00 33.75 |
| atom | 2495 | CA  | CYS | 324 | 26.029 | 15.494 | 47.328 | 1.00 34.16 |
| atom | 2496 | CB  | CYS | 324 | 24.599 | 15.744 | 47.817 | 1.00 32.60 |
| atom | 2497 | SG  | CYS | 324 | 23.415 | 14.482 | 47.250 | 1.00 28.37 |
| atom | 2498 | C   | CYS | 324 | 26.524 | 14.111 | 47.741 | 1.00 36.41 |
| atom | 2499 | O   | CYS | 324 | 26.940 | 13.265 | 46.946 | 1.00 35.85 |
| atom | 2500 | N   | GLU | 325 | 26.516 | 13.906 | 49.052 | 1.00 37.42 |
| atom | 2501 | CA  | GLU | 325 | 26.935 | 12.689 | 49.735 | 1.00 38.45 |
| atom | 2502 | CB  | GLU | 325 | 27.378 | 13.104 | 51.145 | 1.00 41.19 |
| atom | 2503 | CG  | GLU | 325 | 28.247 | 12.197 | 51.960 | 1.00 40.10 |
| atom | 2504 | CD  | GLU | 325 | 29.560 | 11.853 | 51.298 | 1.00 42.45 |
| atom | 2505 | OE1 | GLU | 325 | 30.584 | 12.515 | 51.560 | 1.00 45.65 |
| atom | 2506 | OE2 | GLU | 325 | 29.554 | 10.895 | 50.504 | 1.00 44.28 |
| atom | 2507 | C   | GLU | 325 | 25.743 | 11.750 | 49.817 | 1.00 38.20 |
| atom | 2508 | O   | GLU | 325 | 24.728 | 12.120 | 50.400 | 1.00 39.10 |
| atom | 2509 | N   | SER | 326 | 25.789 | 10.606 | 49.173 | 1.00 38.71 |
| atom | 2510 | CA  | SER | 326 | 24.690 | 9.667  | 49.131 | 1.00 38.62 |
| atom | 2511 | CB  | SER | 326 | 25.021 | 8.424  | 48.306 | 1.00 37.19 |
| atom | 2512 | OG  | SER | 326 | 23.860 | 7.620  | 48.170 | 1.00 37.82 |
| atom | 2513 | C   | SER | 326 | 24.292 | 9.230  | 50.541 | 1.00 39.32 |
| atom | 2514 | O   | SER | 326 | 25.128 | 9.110  | 51.426 | 1.00 38.70 |
| atom | 2515 | N   | ALA | 327 | 22.996 | 8.999  | 50.701 | 1.00 39.45 |
| atom | 2516 | CA  | ALA | 327 | 22.424 | 8.549  | 51.959 | 1.00 39.46 |

| atom | 2517 | CB | ALA | 327 | 21.263 | 9.440 | 52.344 | 1.00 38.95 |
|------|------|-----|-----|-----|--------|-------|--------|------------|
| atom | 2518 | C | ALA | 327 | 21.921 | 7.120 | 51.773 | 1.00 40.13 |
| atom | 2519 | O | ALA | 327 | 21.355 | 6.519 | 52.679 | 1.00 40.46 |
| atom | 2520 | N | GLY | 328 | 22.130 | 6.608 | 50.565 | 1.00 39.86 |
| atom | 2521 | CA | GLY | 328 | 21.687 | 5.288 | 50.158 | 1.00 39.73 |
| atom | 2522 | C | GLY | 328 | 20.754 | 5.448 | 48.950 | 1.00 40.89 |
| atom | 2523 | O | GLY | 328 | 20.109 | 6.484 | 48.778 | 1.00 40.83 |
| atom | 2524 | N | THR | 329 | 20.615 | 4.405 | 48.154 | 1.00 40.88 |
| atom | 2525 | CA | THR | 329 | 19.776 | 4.443 | 46.971 | 1.00 41.92 |
| atom | 2526 | CB | THR | 329 | 19.832 | 3.088 | 46.238 | 1.00 43.44 |
| atom | 2527 | OG1 | THR | 329 | 21.147 | 3.013 | 45.646 | 1.00 46.46 |
| atom | 2528 | CG2 | THR | 329 | 18.824 | 2.999 | 45.115 | 1.00 43.47 |
| atom | 2529 | C | THR | 329 | 18.349 | 4.857 | 47.237 | 1.00 41.88 |
| atom | 2530 | O | THR | 329 | 17.850 | 5.766 | 46.567 | 1.00 42.54 |
| atom | 2531 | N | GLN | 330 | 17.668 | 4.219 | 48.179 | 1.00 42.20 |
| atom | 2532 | CA | GLN | 330 | 16.274 | 4.530 | 48.478 | 1.00 41.29 |
| atom | 2533 | CB | GLN | 330 | 15.612 | 3.457 | 49.346 | 1.00 46.59 |
| atom | 2534 | CG | GLN | 330 | 15.603 | 2.067 | 48.754 | 1.00 50.81 |
| atom | 2535 | CD | GLN | 330 | 14.819 | 2.023 | 47.461 | 1.00 56.28 |
| atom | 2536 | OE1 | GLN | 330 | 13.984 | 2.886 | 47.166 | 1.00 58.61 |
| atom | 2537 | NE2 | GLN | 330 | 15.111 | 0.996 | 46.667 | 1.00 59.59 |
| atom | 2538 | C | GLN | 330 | 16.144 | 5.879 | 49.163 | 1.00 39.83 |
| atom | 2539 | O | GLN | 330 | 15.175 | 6.593 | 48.925 | 1.00 40.36 |
| atom | 2540 | N | GLU | 331 | 17.094 | 6.216 | 50.011 | 1.00 38.55 |
| atom | 2541 | CA | GLU | 331 | 17.136 | 7.511 | 50.681 | 1.00 38.07 |
| atom | 2542 | CB | GLU | 331 | 18.247 | 7.498 | 51.733 | 1.00 37.75 |
| atom | 2543 | CG | GLU | 331 | 18.108 | 6.519 | 52.871 | 1.00 35.20 |
| atom | 2544 | CD | GLU | 331 | 18.521 | 5.087 | 52.645 | 1.00 34.47 |
| atom | 2545 | OE1 | GLU | 331 | 18.818 | 4.660 | 51.518 | 1.00 33.00 |
| atom | 2546 | OE2 | GLU | 331 | 18.557 | 4.294 | 53.623 | 1.00 33.15 |
| atom | 2547 | C | GLU | 331 | 17.331 | 8.602 | 49.621 | 1.00 36.90 |
| atom | 2548 | O | GLU | 331 | 16.606 | 9.591 | 49.558 | 1.00 36.16 |
| atom | 2549 | N | ASP | 332 | 18.269 | 8.411 | 48.700 | 1.00 36.25 |
| atom | 2550 | CA | ASP | 332 | 18.511 | 9.328 | 47.596 | 1.00 35.68 |
| atom | 2551 | CB | ASP | 332 | 19.642 | 8.808 | 46.697 | 1.00 33.77 |
| atom | 2552 | CG | ASP | 332 | 21.012 | 8.836 | 47.343 | 1.00 34.50 |

214

| atom | 2553 | OD1 | ASP | 332 | 21.191 | 9.522 | 48.379 | 1.00 | 31.89 |
|------|------|-----|-----|-----|--------|-------|--------|------|-------|
| atom | 2554 | OD2 | ASP | 332 | 21.928 | 8.145 | 46.848 | 1.00 | 33.11 |
| atom | 2555 | C   | ASP | 332 | 17.235 | 9.526 | 46.775 | 1.00 | 35.20 |
| atom | 2556 | O   | ASP | 332 | 16.823 | 10.661 | 46.510 | 1.00 | 35.26 |
| atom | 2557 | N   | ALA | 333 | 16.588 | 8.434 | 46.378 | 1.00 | 34.63 |
| atom | 2558 | CA  | ALA | 333 | 15.367 | 8.515 | 45.582 | 1.00 | 36.03 |
| atom | 2559 | CB  | ALA | 333 | 14.936 | 7.111 | 45.181 | 1.00 | 34.69 |
| atom | 2560 | C   | ALA | 333 | 14.248 | 9.283 | 46.272 | 1.00 | 36.20 |
| atom | 2561 | O   | ALA | 333 | 13.614 | 10.155 | 45.656 | 1.00 | 36.27 |
| atom | 2562 | N   | ALA | 334 | 14.047 | 9.044 | 47.574 | 1.00 | 36.48 |
| atom | 2563 | CA  | ALA | 334 | 13.007 | 9.767 | 48.313 | 1.00 | 36.21 |
| atom | 2564 | CB  | ALA | 334 | 12.769 | 9.124 | 49.665 | 1.00 | 35.47 |
| atom | 2565 | C   | ALA | 334 | 13.344 | 11.245 | 48.506 | 1.00 | 36.11 |
| atom | 2566 | O   | ALA | 334 | 12.467 | 12.118 | 48.559 | 1.00 | 35.78 |
| atom | 2567 | N   | SER | 335 | 14.630 | 11.547 | 48.631 | 1.00 | 35.28 |
| atom | 2568 | CA  | SER | 335 | 15.117 | 12.899 | 48.814 | 1.00 | 35.61 |
| atom | 2569 | CB  | SER | 335 | 16.596 | 12.850 | 49.211 | 1.00 | 38.47 |
| atom | 2570 | OG  | SER | 335 | 16.726 | 13.107 | 50.600 | 1.00 | 42.74 |
| atom | 2571 | C   | SER | 335 | 14.937 | 13.768 | 47.572 | 1.00 | 36.05 |
| atom | 2572 | O   | SER | 335 | 14.520 | 14.926 | 47.649 | 1.00 | 34.47 |
| atom | 2573 | N   | LEU | 336 | 15.246 | 13.207 | 46.400 | 1.00 | 35.53 |
| atom | 2574 | CA  | LEU | 336 | 15.042 | 13.916 | 45.150 | 1.00 | 36.26 |
| atom | 2575 | CB  | LEU | 336 | 15.421 | 13.079 | 43.925 | 1.00 | 36.24 |
| atom | 2576 | CG  | LEU | 336 | 16.727 | 13.515 | 43.261 | 1.00 | 37.95 |
| atom | 2577 | CD1 | LEU | 336 | 17.924 | 13.243 | 44.173 | 1.00 | 39.03 |
| atom | 2578 | CD2 | LEU | 336 | 16.899 | 12.795 | 41.936 | 1.00 | 37.53 |
| atom | 2579 | C   | LEU | 336 | 13.582 | 14.343 | 45.016 | 1.00 | 35.96 |
| atom | 2580 | O   | LEU | 336 | 13.308 | 15.512 | 44.763 | 1.00 | 35.19 |
| atom | 2581 | N   | ARG | 337 | 12.655 | 13.419 | 45.244 | 1.00 | 36.14 |
| atom | 2582 | CA  | ARG | 337 | 11.231 | 13.738 | 45.195 | 1.00 | 35.70 |
| atom | 2583 | CB  | ARG | 337 | 10.353 | 12.506 | 45.416 | 1.00 | 36.11 |
| atom | 2584 | CG  | ARG | 337 | 10.335 | 11.545 | 44.232 | 1.00 | 38.83 |
| atom | 2585 | CD  | ARG | 337 | 9.411 | 10.348 | 44.496 | 1.00 | 40.22 |
| atom | 2586 | NE  | ARG | 337 | 9.657 | 9.719 | 45.778 | 1.00 | 39.19 |
| atom | 2587 | CZ  | ARG | 337 | 10.236 | 8.561 | 46.047 | 1.00 | 41.14 |
| atom | 2588 | NH1 | ARG | 337 | 10.670 | 7.723 | 45.117 | 1.00 | 38.64 |

| atom | 2589 | NH2 | ARG | 337 | 10.400 | 8.144 | 47.307 | 1.00 43.71 |
|------|------|-----|-----|-----|--------|-------|--------|------------|
| atom | 2590 | C   | ARG | 337 | 10.887 | 14.829 | 46.198 | 1.00 34.60 |
| atom | 2591 | O   | ARG | 337 | 10.107 | 15.732 | 45.877 | 1.00 35.46 |
| atom | 2592 | N   | VAL | 338 | 11.452 | 14.838 | 47.397 | 1.00 34.15 |
| atom | 2593 | CA  | VAL | 338 | 11.180 | 15.955 | 48.314 | 1.00 33.78 |
| atom | 2594 | CB  | VAL | 338 | 11.681 | 15.698 | 49.732 | 1.00 33.92 |
| atom | 2595 | CG1 | VAL | 338 | 11.365 | 16.848 | 50.678 | 1.00 31.97 |
| atom | 2596 | CG2 | VAL | 338 | 11.052 | 14.401 | 50.233 | 1.00 33.85 |
| atom | 2597 | C   | VAL | 338 | 11.791 | 17.213 | 47.698 | 1.00 33.55 |
| atom | 2598 | O   | VAL | 338 | 11.193 | 18.285 | 47.715 | 1.00 33.64 |
| atom | 2599 | N   | PHE | 339 | 12.948 | 17.082 | 47.056 | 1.00 33.81 |
| atom | 2600 | CA  | PHE | 339 | 13.608 | 18.198 | 46.402 | 1.00 34.27 |
| atom | 2601 | CB  | PHE | 339 | 14.955 | 17.728 | 45.841 | 1.00 31.31 |
| atom | 2602 | CG  | PHE | 339 | 15.639 | 18.809 | 45.047 | 1.00 29.80 |
| atom | 2603 | CD1 | PHE | 339 | 16.553 | 19.637 | 45.684 | 1.00 30.25 |
| atom | 2604 | CD2 | PHE | 339 | 15.364 | 19.023 | 43.716 | 1.00 29.29 |
| atom | 2605 | CE1 | PHE | 339 | 17.205 | 20.631 | 45.004 | 1.00 30.41 |
| atom | 2606 | CE2 | PHE | 339 | 16.012 | 20.029 | 43.018 | 1.00 30.52 |
| atom | 2607 | CZ  | PHE | 339 | 16.931 | 20.823 | 43.655 | 1.00 30.30 |
| atom | 2608 | C   | PHE | 339 | 12.740 | 18.862 | 45.335 | 1.00 34.83 |
| atom | 2609 | O   | PHE | 339 | 12.477 | 20.076 | 45.391 | 1.00 33.14 |
| atom | 2610 | N   | THR | 340 | 12.220 | 18.059 | 44.408 | 1.00 35.31 |
| atom | 2611 | CA  | THR | 340 | 11.360 | 18.570 | 43.344 | 1.00 37.57 |
| atom | 2612 | CB  | THR | 340 | 11.255 | 17.660 | 42.103 | 1.00 38.85 |
| atom | 2613 | OG1 | THR | 340 | 9.909  | 17.437 | 41.637 | 1.00 38.26 |
| atom | 2614 | CG2 | THR | 340 | 11.938 | 16.324 | 42.280 | 1.00 35.09 |
| atom | 2615 | C   | THR | 340 | 10.014 | 19.020 | 43.881 | 1.00 38.40 |
| atom | 2616 | O   | THR | 340 | 9.352  | 19.802 | 43.205 | 1.00 38.54 |
| atom | 2617 | N   | GLU | 341 | 9.593  | 18.629 | 45.077 | 1.00 39.01 |
| atom | 2618 | CA  | GLU | 341 | 8.361  | 19.125 | 45.669 | 1.00 39.93 |
| atom | 2619 | CB  | GLU | 341 | 7.922  | 18.312 | 46.896 | 1.00 42.66 |
| atom | 2620 | CG  | GLU | 341 | 7.425  | 16.933 | 46.523 | 1.00 48.20 |
| atom | 2621 | CD  | GLU | 341 | 7.187  | 15.937 | 47.634 | 1.00 51.03 |
| atom | 2622 | OE1 | GLU | 341 | 7.205  | 16.282 | 48.831 | 1.00 51.74 |
| atom | 2623 | OE2 | GLU | 341 | 6.962  | 14.740 | 47.305 | 1.00 53.62 |
| atom | 2624 | C   | GLU | 341 | 8.548  | 20.582 | 46.107 | 1.00 38.80 |

| atom | 2625 | O | GLU | 341 | 7.714 | 21.441 | 45.837 | 1.00 | 38.70 |
|------|------|------|------|------|--------|--------|--------|------|-------|
| atom | 2626 | N | ALA | 342 | 9.663 | 20.820 | 46.792 | 1.00 | 37.52 |
| atom | 2627 | CA | ALA | 342 | 10.001 | 22.175 | 47.241 | 1.00 | 36.40 |
| atom | 2628 | CB | ALA | 342 | 11.235 | 22.110 | 48.109 | 1.00 | 35.93 |
| atom | 2629 | C | ALA | 342 | 10.163 | 23.099 | 46.036 | 1.00 | 35.52 |
| atom | 2630 | O | ALA | 342 | 9.445 | 24.092 | 45.932 | 1.00 | 33.62 |
| atom | 2631 | N | MET | 343 | 10.956 | 22.712 | 45.027 | 1.00 | 34.92 |
| atom | 2632 | CA | MET | 343 | 11.100 | 23.445 | 43.783 | 1.00 | 34.48 |
| atom | 2633 | CB | MET | 343 | 11.931 | 22.681 | 42.741 | 1.00 | 31.80 |
| atom | 2634 | CG | MET | 343 | 13.398 | 22.455 | 43.088 | 1.00 | 28.42 |
| atom | 2635 | SD | MET | 343 | 14.348 | 23.997 | 43.183 | 1.00 | 26.69 |
| atom | 2636 | CE | MET | 343 | 14.467 | 24.378 | 41.437 | 1.00 | 24.16 |
| atom | 2637 | C | MET | 343 | 9.727 | 23.752 | 43.170 | 1.00 | 35.63 |
| atom | 2638 | O | MET | 343 | 9.442 | 24.854 | 42.670 | 1.00 | 34.00 |
| atom | 2639 | N | THR | 344 | 8.834 | 22.761 | 43.198 | 1.00 | 36.17 |
| atom | 2640 | CA | THR | 344 | 7.490 | 22.934 | 42.641 | 1.00 | 37.75 |
| atom | 2641 | CB | THR | 344 | 6.732 | 21.603 | 42.631 | 1.00 | 37.35 |
| atom | 2642 | OG1 | THR | 344 | 7.519 | 20.735 | 41.797 | 1.00 | 38.53 |
| atom | 2643 | CG2 | THR | 344 | 5.330 | 21.710 | 42.053 | 1.00 | 34.74 |
| atom | 2644 | C | THR | 344 | 6.700 | 24.017 | 43.356 | 1.00 | 38.54 |
| atom | 2645 | O | THR | 344 | 6.152 | 24.898 | 42.705 | 1.00 | 38.85 |
| atom | 2646 | N | ARG | 345 | 6.694 | 24.029 | 44.676 | 1.00 | 39.11 |
| atom | 2647 | CA | ARG | 345 | 6.072 | 25.039 | 45.501 | 1.00 | 39.59 |
| atom | 2648 | CB | ARG | 345 | 6.273 | 24.694 | 46.977 | 1.00 | 39.51 |
| atom | 2649 | CG | ARG | 345 | 5.159 | 23.832 | 47.550 | 1.00 | 42.47 |
| atom | 2650 | CD | ARG | 345 | 5.363 | 23.823 | 49.068 | 1.00 | 46.19 |
| atom | 2651 | NE | ARG | 345 | 6.342 | 22.780 | 49.363 | 1.00 | 50.63 |
| atom | 2652 | CZ | ARG | 345 | 7.452 | 22.881 | 50.074 | 1.00 | 50.35 |
| atom | 2653 | NH1 | ARG | 345 | 7.827 | 24.033 | 50.604 | 1.00 | 51.38 |
| atom | 2654 | NH2 | ARG | 345 | 8.186 | 21.786 | 50.220 | 1.00 | 48.48 |
| atom | 2655 | C | ARG | 345 | 6.659 | 26.423 | 45.276 | 1.00 | 40.17 |
| atom | 2656 | O | ARG | 345 | 5.948 | 27.408 | 45.413 | 1.00 | 41.43 |
| atom | 2657 | N | TYR | 346 | 7.935 | 26.527 | 44.923 | 1.00 | 40.18 |
| atom | 2658 | CA | TYR | 346 | 8.608 | 27.764 | 44.619 | 1.00 | 40.02 |
| atom | 2659 | CB | TYR | 346 | 10.124 | 27.644 | 44.814 | 1.00 | 39.12 |
| atom | 2660 | CG | TYR | 346 | 10.568 | 27.177 | 46.183 | 1.00 | 37.04 |

| atom | 2661 | CD1 | TYR | 346 | 9.831 | 27.488 | 47.321 | 1.00 | 36.26 |
|------|------|-----|-----|-----|-------|--------|--------|------|-------|
| atom | 2662 | CE1 | TYR | 346 | 10.240 | 27.060 | 48.577 | 1.00 | 35.81 |
| atom | 2663 | CD2 | TYR | 346 | 11.736 | 26.447 | 46.334 | 1.00 | 35.04 |
| atom | 2664 | CE2 | TYR | 346 | 12.162 | 26.036 | 47.580 | 1.00 | 35.36 |
| atom | 2665 | CZ | TYR | 346 | 11.409 | 26.344 | 48.696 | 1.00 | 35.53 |
| atom | 2666 | OH | TYR | 346 | 11.815 | 25.932 | 49.937 | 1.00 | 35.54 |
| atom | 2667 | C | TYR | 346 | 8.362 | 28.173 | 43.169 | 1.00 | 40.48 |
| atom | 2668 | 0 | TYR | 346 | 8.908 | 29.176 | 42.726 | 1.00 | 40.30 |
| atom | 2669 | N | SER | 347 | 7.579 | 27.403 | 42.432 | 1.00 | 41.56 |
| atom | 2670 | CA | SER | 347 | 7.271 | 27.689 | 41.051 | 1.00 | 44.25 |
| atom | 2671 | CB | SER | 347 | 6.772 | 29.135 | 40.903 | 1.00 | 45.32 |
| atom | 2672 | OG | SER | 347 | 6.488 | 29.393 | 39.541 | 1.00 | 48.51 |
| atom | 2673 | C | SER | 347 | 8.453 | 27.445 | 40.121 | 1.00 | 45.42 |
| atom | 2674 | 0 | SER | 347 | 8.879 | 28.322 | 39.357 | 1.00 | 46.85 |
| atom | 2675 | N | ALA | 348 | 9.004 | 26.239 | 40.177 | 1.00 | 45.07 |
| atom | 2676 | CA | ALA | 348 | 10.071 | 25.794 | 39.286 | 1.00 | 45.01 |
| atom | 2677 | CB | ALA | 348 | 11.469 | 26.117 | 39.758 | 1.00 | 44.64 |
| atom | 2678 | C | ALA | 348 | 9.879 | 24.277 | 39.147 | 1.00 | 44.89 |
| atom | 2679 | 0 | ALA | 348 | 10.647 | 23.487 | 39.687 | 1.00 | 43.73 |
| atom | 2680 | N | PRO | 349 | 8.775 | 23.906 | 38.510 | 1.00 | 44.97 |
| atom | 2681 | CD | PRO | 349 | 7.810 | 24.804 | 37.838 | 1.00 | 45.37 |
| atom | 2682 | CA | PRO | 349 | 8.402 | 22.504 | 38.335 | 1.00 | 45.30 |
| atom | 2683 | CB | PRO | 349 | 6.905 | 22.579 | 38.056 | 1.00 | 45.30 |
| atom | 2684 | CG | PRO | 349 | 6.685 | 23.905 | 37.417 | 1.00 | 45.43 |
| atom | 2685 | C | PRO | 349 | 9.192 | 21.814 | 37.245 | 1.00 | 44.52 |
| atom | 2686 | 0 | PRO | 349 | 9.657 | 22.464 | 36.309 | 1.00 | 45.00 |
| atom | 2687 | N | PRO | 350 | 9.414 | 20.515 | 37.367 | 1.00 | 44.56 |
| atom | 2688 | CD | PRO | 350 | 8.907 | 19.686 | 38.485 | 1.00 | 44.84 |
| atom | 2689 | CA | PRO | 350 | 10.191 | 19.753 | 36.421 | 1.00 | 44.98 |
| atom | 2690 | CB | PRO | 350 | 10.483 | 18.445 | 37.139 | 1.00 | 44.92 |
| atom | 2691 | CG | PRO | 350 | 9.669 | 18.407 | 38.365 | 1.00 | 45.21 |
| atom | 2692 | C | PRO | 350 | 9.544 | 19.493 | 35.071 | 1.00 | 45.61 |
| atom | 2693 | 0 | PRO | 350 | 8.331 | 19.592 | 34.918 | 1.00 | 46.07 |
| atom | 2694 | N | GLY | 351 | 10.401 | 19.162 | 34.113 | 1.00 | 46.35 |
| atom | 2695 | CA | GLY | 351 | 10.005 | 18.757 | 32.775 | 1.00 | 47.99 |
| atom | 2696 | C | GLY | 351 | 10.482 | 17.328 | 32.536 | 1.00 | 48.76 |

| atom | 2697 | O   | GLY | 351 | 10.650 | 16.934 | 31.387 | 1.00 | 50.63 |
|------|------|-----|-----|-----|--------|--------|--------|------|-------|
| atom | 2698 | N   | ASP | 352 | 10.772 | 16.565 | 33.549 | 1.00 | 49.47 |
| atom | 2699 | CA  | ASP | 352 | 11.240 | 15.202 | 33.634 | 1.00 | 49.94 |
| atom | 2700 | CB  | ASP | 352 | 12.352 | 14.676 | 32.764 | 1.00 | 53.09 |
| atom | 2701 | CG  | ASP | 352 | 12.273 | 14.668 | 31.267 | 1.00 | 56.60 |
| atom | 2702 | OD1 | ASP | 352 | 11.168 | 14.516 | 30.693 | 1.00 | 58.67 |
| atom | 2703 | OD2 | ASP | 352 | 13.304 | 14.892 | 30.595 | 1.00 | 57.49 |
| atom | 2704 | C   | ASP | 352 | 11.801 | 15.115 | 35.078 | 1.00 | 48.74 |
| atom | 2705 | O   | ASP | 352 | 12.708 | 15.886 | 35.392 | 1.00 | 48.43 |
| atom | 2706 | N   | PRO | 353 | 11.209 | 14.255 | 35.880 | 1.00 | 47.25 |
| atom | 2707 | CD  | PRO | 353 | 10.129 | 13.318 | 35.538 | 1.00 | 47.18 |
| atom | 2708 | CA  | PRO | 353 | 11.686 | 14.089 | 37.245 | 1.00 | 46.26 |
| atom | 2709 | CB  | PRO | 353 | 10.943 | 12.864 | 37.751 | 1.00 | 46.47 |
| atom | 2710 | CG  | PRO | 353 | 9.733  | 12.747 | 36.880 | 1.00 | 47.48 |
| atom | 2711 | C   | PRO | 353 | 13.201 | 13.898 | 37.232 | 1.00 | 44.25 |
| atom | 2712 | O   | PRO | 353 | 13.758 | 13.200 | 36.384 | 1.00 | 43.68 |
| atom | 2713 | N   | PRO | 354 | 13.889 | 14.576 | 38.137 | 1.00 | 42.38 |
| atom | 2714 | CD  | PRO | 354 | 13.280 | 15.455 | 39.167 | 1.00 | 41.40 |
| atom | 2715 | CA  | PRO | 354 | 15.327 | 14.433 | 38.270 | 1.00 | 41.55 |
| atom | 2716 | CB  | PRO | 354 | 15.678 | 15.311 | 39.458 | 1.00 | 40.93 |
| atom | 2717 | CG  | PRO | 354 | 14.425 | 15.781 | 40.068 | 1.00 | 41.08 |
| atom | 2718 | C   | PRO | 354 | 15.682 | 12.963 | 38.489 | 1.00 | 40.83 |
| atom | 2719 | O   | PRO | 354 | 14.846 | 12.186 | 38.945 | 1.00 | 39.88 |
| atom | 2720 | N   | GLN | 355 | 16.893 | 12.540 | 38.174 | 1.00 | 40.01 |
| atom | 2721 | CA  | GLN | 355 | 17.341 | 11.172 | 38.381 | 1.00 | 40.63 |
| atom | 2722 | CB  | GLN | 355 | 17.482 | 10.394 | 37.080 | 1.00 | 44.11 |
| atom | 2723 | CG  | GLN | 355 | 16.200 | 10.029 | 36.360 | 1.00 | 48.70 |
| atom | 2724 | CD  | GLN | 355 | 15.178 | 9.250  | 37.155 | 1.00 | 50.76 |
| atom | 2725 | OE1 | GLN | 355 | 13.960 | 9.418  | 37.021 | 1.00 | 51.58 |
| atom | 2726 | NE2 | GLN | 355 | 15.639 | 8.358  | 38.025 | 1.00 | 52.35 |
| atom | 2727 | C   | GLN | 355 | 18.676 | 11.159 | 39.129 | 1.00 | 39.01 |
| atom | 2728 | O   | GLN | 355 | 19.591 | 11.874 | 38.766 | 1.00 | 39.46 |
| atom | 2729 | N   | PRO | 356 | 18.766 | 10.401 | 40.212 | 1.00 | 38.08 |
| atom | 2730 | CD  | PRO | 356 | 17.686 | 9.508  | 40.706 | 1.00 | 37.88 |
| atom | 2731 | CA  | PRO | 356 | 19.989 | 10.292 | 40.981 | 1.00 | 36.95 |
| atom | 2732 | CB  | PRO | 356 | 19.553 | 9.541  | 42.233 | 1.00 | 37.62 |

| atom | 2733 | CG | PRO | 356 | 18.347 | 8.771 | 41.837 | 1.00 | 38.20 |
| atom | 2734 | C | PRO | 356 | 21.039 | 9.542 | 40.187 | 1.00 | 35.57 |
| atom | 2735 | O | PRO | 356 | 20.764 | 8.514 | 39.575 | 1.00 | 34.77 |
| atom | 2736 | N | GLU | 357 | 22.257 | 10.039 | 40.116 | 1.00 | 35.70 |
| atom | 2737 | CA | GLU | 357 | 23.340 | 9.390 | 39.376 | 1.00 | 34.95 |
| atom | 2738 | CB | GLU | 357 | 23.751 | 10.274 | 38.200 | 1.00 | 37.14 |
| atom | 2739 | CG | GLU | 357 | 22.730 | 10.421 | 37.097 | 1.00 | 39.47 |
| atom | 2740 | CD | GLU | 357 | 22.447 | 9.146 | 36.323 | 1.00 | 42.28 |
| atom | 2741 | OE1 | GLU | 357 | 21.393 | 9.132 | 35.638 | 1.00 | 43.89 |
| atom | 2742 | OE2 | GLU | 357 | 23.211 | 8.161 | 36.357 | 1.00 | 42.55 |
| atom | 2743 | C | GLU | 357 | 24.548 | 9.117 | 40.259 | 1.00 | 34.15 |
| atom | 2744 | O | GLU | 357 | 24.857 | 9.889 | 41.175 | 1.00 | 33.32 |
| atom | 2745 | N | TYR | 358 | 25.204 | 7.971 | 40.028 | 1.00 | 33.12 |
| atom | 2746 | CA | TYR | 358 | 26.368 | 7.592 | 40.828 | 1.00 | 32.89 |
| atom | 2747 | CB | TYR | 358 | 26.121 | 6.276 | 41.572 | 1.00 | 31.78 |
| atom | 2748 | CG | TYR | 358 | 24.884 | 6.409 | 42.440 | 1.00 | 29.69 |
| atom | 2749 | CD1 | TYR | 358 | 23.643 | 6.062 | 41.921 | 1.00 | 28.37 |
| atom | 2750 | CE1 | TYR | 358 | 22.497 | 6.202 | 42.680 | 1.00 | 27.66 |
| atom | 2751 | CD2 | TYR | 358 | 24.964 | 6.929 | 43.721 | 1.00 | 28.05 |
| atom | 2752 | CE2 | TYR | 358 | 23.832 | 7.070 | 44.500 | 1.00 | 27.10 |
| atom | 2753 | CZ | TYR | 358 | 22.616 | 6.700 | 43.968 | 1.00 | 27.91 |
| atom | 2754 | OH | TYR | 358 | 21.493 | 6.859 | 44.726 | 1.00 | 29.25 |
| atom | 2755 | C | TYR | 358 | 27.617 | 7.498 | 39.961 | 1.00 | 33.56 |
| atom | 2756 | O | TYR | 358 | 28.702 | 7.054 | 40.342 | 1.00 | 33.09 |
| atom | 2757 | N | ASP | 359 | 27.465 | 8.035 | 38.758 | 1.00 | 34.00 |
| atom | 2758 | CA | ASP | 359 | 28.560 | 8.056 | 37.788 | 1.00 | 35.55 |
| atom | 2759 | CB | ASP | 359 | 28.307 | 6.982 | 36.746 | 1.00 | 39.76 |
| atom | 2760 | CG | ASP | 359 | 29.297 | 6.923 | 35.608 | 1.00 | 43.48 |
| atom | 2761 | OD1 | ASP | 359 | 29.147 | 6.071 | 34.692 | 1.00 | 45.94 |
| atom | 2762 | OD2 | ASP | 359 | 30.260 | 7.722 | 35.597 | 1.00 | 42.96 |
| atom | 2763 | C | ASP | 359 | 28.604 | 9.483 | 37.266 | 1.00 | 35.24 |
| atom | 2764 | O | ASP | 359 | 27.667 | 9.918 | 36.599 | 1.00 | 35.02 |
| atom | 2765 | N | LEU | 360 | 29.684 | 10.208 | 37.572 | 1.00 | 34.88 |
| atom | 2766 | CA | LEU | 360 | 29.817 | 11.596 | 37.176 | 1.00 | 34.12 |
| atom | 2767 | CB | LEU | 360 | 31.119 | 12.265 | 37.634 | 1.00 | 31.83 |
| atom | 2768 | CG | LEU | 360 | 31.317 | 13.716 | 37.131 | 1.00 | 30.38 |

220

| | | | | | | | | | |
|---|---|---|---|---|---|---|---|---|---|
| atom | 2769 | CD1 | LEU | 360 | 30.430 | 14.704 | 37.872 | 1.00 | 27.11 |
| atom | 2770 | CD2 | LEU | 360 | 32.770 | 14.147 | 37.246 | 1.00 | 30.40 |
| atom | 2771 | C | LEU | 360 | 29.666 | 11.777 | 35.668 | 1.00 | 34.75 |
| atom | 2772 | O | LEU | 360 | 29.054 | 12.757 | 35.231 | 1.00 | 34.42 |
| atom | 2773 | N | GLU | 361 | 30.096 | 10.817 | 34.863 | 1.00 | 34.16 |
| atom | 2774 | CA | GLU | 361 | 29.977 | 10.894 | 33.420 | 1.00 | 35.31 |
| atom | 2775 | CB | GLU | 361 | 30.780 | 9.768 | 32.756 | 1.00 | 36.45 |
| atom | 2776 | CG | GLU | 361 | 30.771 | 9.786 | 31.239 | 1.00 | 37.49 |
| atom | 2777 | CD | GLU | 361 | 31.634 | 8.725 | 30.603 | 1.00 | 39.14 |
| atom | 2778 | OE1 | GLU | 361 | 31.856 | 8.786 | 29.370 | 1.00 | 39.05 |
| atom | 2779 | OE2 | GLU | 361 | 32.106 | 7.819 | 31.328 | 1.00 | 40.86 |
| atom | 2780 | C | GLU | 361 | 28.531 | 10.900 | 32.961 | 1.00 | 35.64 |
| atom | 2781 | O | GLU | 361 | 28.240 | 11.401 | 31.873 | 1.00 | 35.05 |
| atom | 2782 | N | LEU | 362 | 27.590 | 10.416 | 33.767 | 1.00 | 36.15 |
| atom | 2783 | CA | LEU | 362 | 26.180 | 10.399 | 33.424 | 1.00 | 36.31 |
| atom | 2784 | CB | LEU | 362 | 25.566 | 9.152 | 34.049 | 1.00 | 37.48 |
| atom | 2785 | CG | LEU | 362 | 26.067 | 7.810 | 33.504 | 1.00 | 38.17 |
| atom | 2786 | CD1 | LEU | 362 | 25.373 | 6.655 | 34.214 | 1.00 | 37.08 |
| atom | 2787 | CD2 | LEU | 362 | 25.857 | 7.710 | 32.001 | 1.00 | 36.11 |
| atom | 2788 | C | LEU | 362 | 25.413 | 11.637 | 33.873 | 1.00 | 37.39 |
| atom | 2789 | O | LEU | 362 | 24.188 | 11.755 | 33.757 | 1.00 | 36.29 |
| atom | 2790 | N | ILE | 363 | 26.140 | 12.615 | 34.401 | 1.00 | 38.15 |
| atom | 2791 | CA | ILE | 363 | 25.558 | 13.860 | 34.849 | 1.00 | 39.73 |
| atom | 2792 | CB | ILE | 363 | 26.116 | 14.297 | 36.206 | 1.00 | 40.87 |
| atom | 2793 | CG2 | ILE | 363 | 25.454 | 15.628 | 36.541 | 1.00 | 41.04 |
| atom | 2794 | CG1 | ILE | 363 | 25.848 | 13.247 | 37.286 | 1.00 | 40.69 |
| atom | 2795 | CD1 | ILE | 363 | 26.375 | 13.634 | 38.655 | 1.00 | 41.48 |
| atom | 2796 | C | ILE | 363 | 25.791 | 14.958 | 33.812 | 1.00 | 40.78 |
| atom | 2797 | O | ILE | 363 | 26.895 | 15.487 | 33.664 | 1.00 | 41.08 |
| atom | 2798 | N | THR | 364 | 24.741 | 15.289 | 33.084 | 1.00 | 41.16 |
| atom | 2799 | CA | THR | 364 | 24.764 | 16.332 | 32.074 | 1.00 | 42.71 |
| atom | 2800 | CB | THR | 364 | 23.769 | 15.986 | 30.948 | 1.00 | 42.29 |
| atom | 2801 | OG1 | THR | 364 | 24.228 | 14.807 | 30.280 | 1.00 | 43.03 |
| atom | 2802 | CG2 | THR | 364 | 23.642 | 17.086 | 29.918 | 1.00 | 42.15 |
| atom | 2803 | C | THR | 364 | 24.389 | 17.694 | 32.656 | 1.00 | 43.62 |
| atom | 2804 | O | THR | 364 | 23.287 | 17.881 | 33.161 | 1.00 | 44.02 |

| atom | 2805 | N | SER | 365 | 25.284 | 18.664 | 32.555 | 1.00 | 44.40 |
|------|------|-----|-----|-----|--------|--------|--------|------|-------|
| atom | 2806 | CA | SER | 365 | 25.056 | 20.024 | 33.028 | 1.00 | 44.48 |
| atom | 2807 | CB | SER | 365 | 25.925 | 20.343 | 34.230 | 1.00 | 43.36 |
| atom | 2808 | OG | SER | 365 | 26.885 | 21.343 | 34.027 | 1.00 | 42.52 |
| atom | 2809 | C | SER | 365 | 25.366 | 21.000 | 31.894 | 1.00 | 45.05 |
| atom | 2810 | O | SER | 365 | 26.450 | 20.973 | 31.310 | 1.00 | 45.65 |
| atom | 2811 | N | CYS | 366 | 24.398 | 21.821 | 31.509 | 1.00 | 45.44 |
| atom | 2812 | CA | CYS | 366 | 24.557 | 22.753 | 30.389 | 1.00 | 45.06 |
| atom | 2813 | CB | CYS | 366 | 25.728 | 23.706 | 30.609 | 1.00 | 44.43 |
| atom | 2814 | SG | CYS | 366 | 25.536 | 24.920 | 31.938 | 1.00 | 44.13 |
| atom | 2815 | C | CYS | 366 | 24.736 | 21.951 | 29.109 | 1.00 | 44.32 |
| atom | 2816 | O | CYS | 366 | 25.583 | 22.203 | 28.261 | 1.00 | 43.80 |
| atom | 2817 | N | SER | 367 | 24.010 | 20.852 | 28.957 | 1.00 | 44.70 |
| atom | 2818 | CA | SER | 367 | 24.054 | 19.911 | 27.854 | 1.00 | 44.20 |
| atom | 2819 | CB | SER | 367 | 23.535 | 20.556 | 26.562 | 1.00 | 46.68 |
| atom | 2820 | OG | SER | 367 | 24.493 | 21.447 | 26.012 | 1.00 | 48.52 |
| atom | 2821 | C | SER | 367 | 25.435 | 19.295 | 27.631 | 1.00 | 42.31 |
| atom | 2822 | O | SER | 367 | 25.704 | 18.709 | 26.581 | 1.00 | 42.62 |
| atom | 2823 | N | SER | 368 | 26.287 | 19.303 | 28.642 | 1.00 | 40.18 |
| atom | 2824 | CA | SER | 368 | 27.633 | 18.771 | 28.546 | 1.00 | 37.66 |
| atom | 2825 | CB | SER | 368 | 28.571 | 19.988 | 28.603 | 1.00 | 39.21 |
| atom | 2826 | OG | SER | 368 | 29.879 | 19.551 | 28.251 | 1.00 | 45.58 |
| atom | 2827 | C | SER | 368 | 27.947 | 17.759 | 29.635 | 1.00 | 34.95 |
| atom | 2828 | O | SER | 368 | 27.245 | 17.730 | 30.654 | 1.00 | 34.69 |
| atom | 2829 | N | ASN | 369 | 28.935 | 16.890 | 29.461 | 1.00 | 32.39 |
| atom | 2830 | CA | ASN | 369 | 29.295 | 15.969 | 30.529 | 1.00 | 31.04 |
| atom | 2831 | CB | ASN | 369 | 28.588 | 14.618 | 30.529 | 1.00 | 25.95 |
| atom | 2832 | CG | ASN | 369 | 28.969 | 13.718 | 29.370 | 1.00 | 25.90 |
| atom | 2833 | OD1 | ASN | 369 | 28.481 | 13.977 | 28.263 | 1.00 | 24.06 |
| atom | 2834 | ND2 | ASN | 369 | 29.834 | 12.727 | 29.602 | 1.00 | 22.99 |
| atom | 2835 | C | ASN | 369 | 30.807 | 15.760 | 30.570 | 1.00 | 30.92 |
| atom | 2836 | O | ASN | 369 | 31.511 | 15.859 | 29.565 | 1.00 | 31.50 |
| atom | 2837 | N | VAL | 370 | 31.280 | 15.448 | 31.769 | 1.00 | 30.92 |
| atom | 2838 | CA | VAL | 370 | 32.709 | 15.172 | 31.939 | 1.00 | 31.89 |
| atom | 2839 | CB | VAL | 370 | 33.101 | 15.353 | 33.416 | 1.00 | 32.13 |
| atom | 2840 | CG1 | VAL | 370 | 34.538 | 14.953 | 33.666 | 1.00 | 30.83 |

| | | | | | | | | | |
|---|---|---|---|---|---|---|---|---|---|
| atom | 2841 | CG2 | VAL | 370 | 32.820 | 16.794 | 33.830 | 1.00 | 30.79 |
| atom | 2842 | C | VAL | 370 | 33.002 | 13.746 | 31.505 | 1.00 | 32.09 |
| atom | 2843 | 0 | VAL | 370 | 32.215 | 12.849 | 31.804 | 1.00 | 33.79 |
| atom | 2844 | N | SER | 371 | 34.106 | 13.512 | 30.827 | 1.00 | 32.19 |
| atom | 2845 | CA | SER | 371 | 34.468 | 12.157 | 30.428 | 1.00 | 32.30 |
| atom | 2846 | CB | SER | 371 | 33.951 | 11.790 | 29.044 | 1.00 | 32.16 |
| atom | 2847 | OG | SER | 371 | 33.909 | 10.370 | 28.902 | 1.00 | 32.16 |
| atom | 2848 | C | SER | 371 | 35.985 | 12.046 | 30.554 | 1.00 | 32.58 |
| atom | 2849 | 0 | SER | 371 | 36.646 | 13.062 | 30.744 | 1.00 | 32.53 |
| atom | 2850 | N | VAL | 372 | 36.516 | 10.841 | 30.474 | 1.00 | 33.10 |
| atom | 2851 | CA | VAL | 372 | 37.922 | 10.551 | 30.661 | 1.00 | 33.98 |
| atom | 2852 | CB | VAL | 372 | 38.090 | 9.473 | 31.772 | 1.00 | 34.27 |
| atom | 2853 | CG1 | VAL | 372 | 39.542 | 9.124 | 32.057 | 1.00 | 35.63 |
| atom | 2854 | CG2 | VAL | 372 | 37.424 | 9.936 | 33.046 | 1.00 | 33.72 |
| atom | 2855 | C | VAL | 372 | 38.569 | 9.983 | 29.401 | 1.00 | 33.96 |
| atom | 2856 | 0 | VAL | 372 | 37.956 | 9.274 | 28.611 | 1.00 | 33.59 |
| atom | 2857 | N | ALA | 373 | 39.840 | 10.249 | 29.258 | 1.00 | 34.15 |
| atom | 2858 | CA | ALA | 373 | 40.697 | 9.778 | 28.190 | 1.00 | 36.83 |
| atom | 2859 | CB | ALA | 373 | 40.553 | 10.520 | 26.879 | 1.00 | 35.63 |
| atom | 2860 | C | ALA | 373 | 42.121 | 9.943 | 28.718 | 1.00 | 37.97 |
| atom | 2861 | 0 | ALA | 373 | 42.313 | 10.371 | 29.854 | 1.00 | 37.83 |
| atom | 2862 | N | HIS | 374 | 43.087 | 9.493 | 27.932 | 1.00 | 39.90 |
| atom | 2863 | CA | HIS | 374 | 44.482 | 9.598 | 28.357 | 1.00 | 42.26 |
| atom | 2864 | CB | HIS | 374 | 45.072 | 8.199 | 28.574 | 1.00 | 43.99 |
| atom | 2865 | CG | HIS | 374 | 44.278 | 7.327 | 29.499 | 1.00 | 45.89 |
| atom | 2866 | CD2 | HIS | 374 | 43.112 | 6.664 | 29.272 | 1.00 | 45.73 |
| atom | 2867 | ND1 | HIS | 374 | 44.619 | 7.070 | 30.809 | 1.00 | 45.75 |
| atom | 2868 | CE1 | HIS | 374 | 43.700 | 6.280 | 31.340 | 1.00 | 46.05 |
| atom | 2869 | NE2 | HIS | 374 | 42.773 | 6.034 | 30.436 | 1.00 | 45.88 |
| atom | 2870 | C | HIS | 374 | 45.290 | 10.325 | 27.287 | 1.00 | 42.98 |
| atom | 2871 | 0 | HIS | 374 | 44.953 | 10.190 | 26.105 | 1.00 | 42.57 |
| atom | 2872 | N | ASP | 375 | 46.351 | 11.003 | 27.701 | 1.00 | 43.81 |
| atom | 2873 | CA | ASP | 375 | 47.229 | 11.653 | 26.731 | 1.00 | 45.84 |
| atom | 2874 | CB | ASP | 375 | 47.842 | 12.946 | 27.257 | 1.00 | 45.77 |
| atom | 2875 | CG | ASP | 375 | 48.691 | 12.754 | 28.493 | 1.00 | 46.41 |
| atom | 2876 | OD1 | ASP | 375 | 49.167 | 11.623 | 28.715 | 1.00 | 47.28 |

| atom | 2877 | OD2 | ASP | 375 | 48.860 | 13.736 | 29.251 | 1.00 | 46.70 |
|------|------|-----|-----|-----|--------|--------|--------|------|-------|
| atom | 2878 | C | ASP | 375 | 48.305 | 10.658 | 26.308 | 1.00 | 47.08 |
| atom | 2879 | O | ASP | 375 | 48.291 | 9.501 | 26.716 | 1.00 | 46.83 |
| atom | 2880 | N | ALA | 376 | 49.273 | 11.093 | 25.521 | 1.00 | 49.15 |
| atom | 2881 | CA | ALA | 376 | 50.360 | 10.276 | 25.006 | 1.00 | 51.61 |
| atom | 2882 | CB | ALA | 376 | 51.251 | 11.144 | 24.120 | 1.00 | 52.07 |
| atom | 2883 | C | ALA | 376 | 51.226 | 9.638 | 26.083 | 1.00 | 52.97 |
| atom | 2884 | O | ALA | 376 | 51.710 | 8.509 | 25.962 | 1.00 | 52.80 |
| atom | 2885 | N | SER | 377 | 51.395 | 10.352 | 27.197 | 1.00 | 54.26 |
| atom | 2886 | CA | SER | 377 | 52.164 | 9.858 | 28.327 | 1.00 | 55.35 |
| atom | 2887 | CB | SER | 377 | 52.593 | 11.008 | 29.237 | 1.00 | 57.26 |
| atom | 2888 | OG | SER | 377 | 53.596 | 11.792 | 28.607 | 1.00 | 61.87 |
| atom | 2889 | C | SER | 377 | 51.395 | 8.841 | 29.154 | 1.00 | 55.47 |
| atom | 2890 | O | SER | 377 | 51.924 | 8.328 | 30.147 | 1.00 | 56.14 |
| atom | 2891 | N | GLY | 378 | 50.132 | 8.595 | 28.839 | 1.00 | 55.00 |
| atom | 2892 | CA | GLY | 378 | 49.291 | 7.672 | 29.580 | 1.00 | 54.48 |
| atom | 2893 | C | GLY | 378 | 48.493 | 8.397 | 30.656 | 1.00 | 54.15 |
| atom | 2894 | O | GLY | 378 | 47.610 | 7.825 | 31.280 | 1.00 | 54.18 |
| atom | 2895 | N | LYS | 379 | 48.791 | 9.667 | 30.874 | 1.00 | 54.05 |
| atom | 2896 | CA | LYS | 379 | 48.150 | 10.497 | 31.875 | 1.00 | 53.89 |
| atom | 2897 | CB | LYS | 379 | 48.780 | 11.888 | 31.834 | 1.00 | 57.08 |
| atom | 2898 | CG | LYS | 379 | 48.193 | 12.977 | 32.700 | 1.00 | 59.77 |
| atom | 2899 | CD | LYS | 379 | 48.876 | 13.152 | 34.040 | 1.00 | 61.29 |
| atom | 2900 | CE | LYS | 379 | 48.686 | 14.564 | 34.577 | 1.00 | 62.90 |
| atom | 2901 | NZ | LYS | 379 | 49.596 | 15.557 | 33.934 | 1.00 | 64.60 |
| atom | 2902 | C | LYS | 379 | 46.645 | 10.594 | 31.650 | 1.00 | 52.58 |
| atom | 2903 | O | LYS | 379 | 46.186 | 10.798 | 30.523 | 1.00 | 53.27 |
| atom | 2904 | N | ARG | 380 | 45.871 | 10.429 | 32.716 | 1.00 | 50.20 |
| atom | 2905 | CA | ARG | 380 | 44.424 | 10.576 | 32.598 | 1.00 | 48.14 |
| atom | 2906 | CB | ARG | 380 | 43.646 | 10.049 | 33.801 | 1.00 | 50.63 |
| atom | 2907 | CG | ARG | 380 | 43.608 | 8.524 | 33.802 | 1.00 | 55.18 |
| atom | 2908 | CD | ARG | 380 | 43.179 | 7.995 | 35.154 | 1.00 | 57.32 |
| atom | 2909 | NE | ARG | 380 | 41.738 | 8.052 | 35.338 | 1.00 | 60.86 |
| atom | 2910 | CZ | ARG | 380 | 41.148 | 8.571 | 36.410 | 1.00 | 63.42 |
| atom | 2911 | NH1 | ARG | 380 | 41.868 | 9.109 | 37.392 | 1.00 | 63.63 |
| atom | 2912 | NH2 | ARG | 380 | 39.816 | 8.557 | 36.466 | 1.00 | 64.35 |

| | | | | | | | | | |
|---|---|---|---|---|---|---|---|---|---|
| atom | 2913 | C | ARG | 380 | 44.081 | 12.054 | 32.408 | 1.00 | 44.95 |
| atom | 2914 | O | ARG | 380 | 44.714 | 12.937 | 32.972 | 1.00 | 44.32 |
| atom | 2915 | N | VAL | 381 | 43.144 | 12.318 | 31.519 | 1.00 | 42.18 |
| atom | 2916 | CA | VAL | 381 | 42.707 | 13.671 | 31.213 | 1.00 | 40.01 |
| atom | 2917 | CB | VAL | 381 | 43.306 | 14.205 | 29.908 | 1.00 | 40.44 |
| atom | 2918 | CG1 | VAL | 381 | 42.569 | 15.440 | 29.391 | 1.00 | 39.66 |
| atom | 2919 | CG2 | VAL | 381 | 44.788 | 14.558 | 30.069 | 1.00 | 40.03 |
| atom | 2920 | C | VAL | 381 | 41.177 | 13.697 | 31.180 | 1.00 | 38.59 |
| atom | 2921 | O | VAL | 381 | 40.536 | 12.953 | 30.447 | 1.00 | 36.92 |
| atom | 2922 | N | TYR | 382 | 40.614 | 14.563 | 32.017 | 1.00 | 38.03 |
| atom | 2923 | CA | TYR | 382 | 39.168 | 14.753 | 32.103 | 1.00 | 37.24 |
| atom | 2924 | CB | TYR | 382 | 38.648 | 15.092 | 33.493 | 1.00 | 37.22 |
| atom | 2925 | CG | TYR | 382 | 38.747 | 13.983 | 34.512 | 1.00 | 37.02 |
| atom | 2926 | CD1 | TYR | 382 | 39.799 | 13.959 | 35.424 | 1.00 | 37.48 |
| atom | 2927 | CE1 | TYR | 382 | 39.925 | 12.962 | 36.374 | 1.00 | 37.09 |
| atom | 2928 | CD2 | TYR | 382 | 37.814 | 12.967 | 34.571 | 1.00 | 36.70 |
| atom | 2929 | CE2 | TYR | 382 | 37.934 | 11.963 | 35.519 | 1.00 | 38.46 |
| atom | 2930 | CZ | TYR | 382 | 38.991 | 11.952 | 36.408 | 1.00 | 38.46 |
| atom | 2931 | OH | TYR | 382 | 39.071 | 10.928 | 37.331 | 1.00 | 38.36 |
| atom | 2932 | C | TYR | 382 | 38.822 | 15.877 | 31.130 | 1.00 | 37.05 |
| atom | 2933 | O | TYR | 382 | 39.654 | 16.768 | 30.964 | 1.00 | 37.38 |
| atom | 2934 | N | TYR | 383 | 37.683 | 15.795 | 30.461 | 1.00 | 36.32 |
| atom | 2935 | CA | TYR | 383 | 37.317 | 16.825 | 29.491 | 1.00 | 36.24 |
| atom | 2936 | CB | TYR | 383 | 38.090 | 16.600 | 28.167 | 1.00 | 35.32 |
| atom | 2937 | CG | TYR | 383 | 37.566 | 15.347 | 27.491 | 1.00 | 34.72 |
| atom | 2938 | CD1 | TYR | 383 | 36.637 | 15.441 | 26.466 | 1.00 | 34.29 |
| atom | 2939 | CE1 | TYR | 383 | 36.120 | 14.301 | 25.868 | 1.00 | 33.76 |
| atom | 2940 | CD2 | TYR | 383 | 37.950 | 14.080 | 27.918 | 1.00 | 34.05 |
| atom | 2941 | CE2 | TYR | 383 | 37.438 | 12.938 | 27.334 | 1.00 | 32.33 |
| atom | 2942 | CZ | TYR | 383 | 36.521 | 13.058 | 26.317 | 1.00 | 33.15 |
| atom | 2943 | OH | TYR | 383 | 35.992 | 11.932 | 25.742 | 1.00 | 32.40 |
| atom | 2944 | C | TYR | 383 | 35.807 | 16.829 | 29.292 | 1.00 | 35.79 |
| atom | 2945 | O | TYR | 383 | 35.113 | 15.894 | 29.708 | 1.00 | 36.84 |
| atom | 2946 | N | LEU | 384 | 35.279 | 17.859 | 28.657 | 1.00 | 34.97 |
| atom | 2947 | CA | LEU | 384 | 33.859 | 17.983 | 28.395 | 1.00 | 35.04 |
| atom | 2948 | CB | LEU | 384 | 33.432 | 19.460 | 28.435 | 1.00 | 39.33 |

| atom | 2949 | CG | LEU | 384 | 33.262 | 20.079 | 29.829 | 1.00 | 43.11 |
|------|------|-----|-----|-----|--------|--------|--------|------|-------|
| atom | 2950 | CD1 | LEU | 384 | 33.165 | 21.590 | 29.676 | 1.00 | 43.79 |
| atom | 2951 | CD2 | LEU | 384 | 32.024 | 19.522 | 30.515 | 1.00 | 41.32 |
| atom | 2952 | C | LEU | 384 | 33.459 | 17.483 | 27.016 | 1.00 | 33.98 |
| atom | 2953 | O | LEU | 384 | 34.107 | 17.837 | 26.047 | 1.00 | 33.66 |
| atom | 2954 | N | THR | 385 | 32.371 | 16.736 | 26.954 | 1.00 | 33.83 |
| atom | 2955 | CA | THR | 385 | 31.777 | 16.231 | 25.746 | 1.00 | 33.24 |
| atom | 2956 | CB | THR | 385 | 31.729 | 14.693 | 25.561 | 1.00 | 32.40 |
| atom | 2957 | OG1 | THR | 385 | 32.551 | 14.004 | 26.483 | 1.00 | 36.41 |
| atom | 2958 | CG2 | THR | 385 | 32.043 | 14.363 | 24.140 | 1.00 | 26.24 |
| atom | 2959 | C | THR | 385 | 30.251 | 16.509 | 25.855 | 1.00 | 33.11 |
| atom | 2960 | O | THR | 385 | 29.826 | 17.045 | 26.858 | 1.00 | 31.29 |
| atom | 2961 | N | ARG | 386 | 29.541 | 15.963 | 24.888 | 1.00 | 32.58 |
| atom | 2962 | CA | ARG | 386 | 28.114 | 15.990 | 24.784 | 1.00 | 34.19 |
| atom | 2963 | CB | ARG | 386 | 27.629 | 17.411 | 24.440 | 1.00 | 34.94 |
| atom | 2964 | CG | ARG | 386 | 28.175 | 17.935 | 23.110 | 1.00 | 34.47 |
| atom | 2965 | CD | ARG | 386 | 27.384 | 19.165 | 22.646 | 1.00 | 32.11 |
| atom | 2966 | NE | ARG | 386 | 26.025 | 18.748 | 22.321 | 1.00 | 31.36 |
| atom | 2967 | CZ | ARG | 386 | 24.921 | 19.452 | 22.468 | 1.00 | 32.38 |
| atom | 2968 | NH1 | ARG | 386 | 24.987 | 20.695 | 22.972 | 1.00 | 30.65 |
| atom | 2969 | NH2 | ARG | 386 | 23.739 | 18.929 | 22.130 | 1.00 | 29.72 |
| atom | 2970 | C | ARG | 386 | 27.697 | 15.047 | 23.654 | 1.00 | 35.06 |
| atom | 2971 | O | ARG | 386 | 28.515 | 14.669 | 22.812 | 1.00 | 35.18 |
| atom | 2972 | N | ASP | 387 | 26.440 | 14.626 | 23.641 | 1.00 | 36.34 |
| atom | 2973 | CA | ASP | 387 | 25.938 | 13.822 | 22.503 | 1.00 | 35.99 |
| atom | 2974 | CB | ASP | 387 | 24.459 | 13.578 | 22.775 | 1.00 | 35.33 |
| atom | 2975 | CG | ASP | 387 | 23.806 | 12.697 | 21.735 | 1.00 | 36.35 |
| atom | 2976 | OD1 | ASP | 387 | 23.725 | 13.107 | 20.566 | 1.00 | 36.75 |
| atom | 2977 | OD2 | ASP | 387 | 23.396 | 11.580 | 22.124 | 1.00 | 38.67 |
| atom | 2978 | C | ASP | 387 | 26.137 | 14.721 | 21.287 | 1.00 | 36.12 |
| atom | 2979 | O | ASP | 387 | 25.816 | 15.920 | 21.325 | 1.00 | 35.42 |
| atom | 2980 | N | PRO | 388 | 26.680 | 14.218 | 20.193 | 1.00 | 36.16 |
| atom | 2981 | CD | PRO | 388 | 27.160 | 12.838 | 20.001 | 1.00 | 35.95 |
| atom | 2982 | CA | PRO | 388 | 26.977 | 15.055 | 19.044 | 1.00 | 35.58 |
| atom | 2983 | CB | PRO | 388 | 28.219 | 14.394 | 18.462 | 1.00 | 35.69 |
| atom | 2984 | CG | PRO | 388 | 28.154 | 12.976 | 18.879 | 1.00 | 36.08 |

| atom | 2985 | C | PRO | 388 | 25.861 | 15.196 | 18.048 | 1.00 | 34.78 |
|------|------|-----|-----|-----|--------|--------|--------|------|-------|
| atom | 2986 | O | PRO | 388 | 26.089 | 15.792 | 16.984 | 1.00 | 36.28 |
| atom | 2987 | N | THR | 389 | 24.650 | 14.761 | 18.349 | 1.00 | 33.85 |
| atom | 2988 | CA | THR | 389 | 23.542 | 14.879 | 17.400 | 1.00 | 33.11 |
| atom | 2989 | CB | THR | 389 | 22.268 | 14.269 | 17.999 | 1.00 | 32.28 |
| atom | 2990 | OG1 | THR | 389 | 22.490 | 12.872 | 18.236 | 1.00 | 30.88 |
| atom | 2991 | CG2 | THR | 389 | 21.117 | 14.462 | 17.028 | 1.00 | 34.42 |
| atom | 2992 | C | THR | 389 | 23.295 | 16.291 | 16.902 | 1.00 | 33.31 |
| atom | 2993 | O | THR | 389 | 23.417 | 16.560 | 15.685 | 1.00 | 33.51 |
| atom | 2994 | N | THR | 390 | 22.997 | 17.229 | 17.803 | 1.00 | 32.85 |
| atom | 2995 | CA | THR | 390 | 22.764 | 18.622 | 17.404 | 1.00 | 32.06 |
| atom | 2996 | CB | THR | 390 | 22.292 | 19.497 | 18.561 | 1.00 | 31.41 |
| atom | 2997 | OG1 | THR | 390 | 21.061 | 18.938 | 19.070 | 1.00 | 32.36 |
| atom | 2998 | CG2 | THR | 390 | 22.024 | 20.929 | 18.123 | 1.00 | 28.60 |
| atom | 2999 | C | THR | 390 | 23.936 | 19.204 | 16.643 | 1.00 | 32.52 |
| atom | 3000 | O | THR | 390 | 23.801 | 19.599 | 15.483 | 1.00 | 33.52 |
| atom | 3001 | N | PRO | 391 | 25.146 | 19.199 | 17.184 | 1.00 | 32.89 |
| atom | 3002 | CD | PRO | 391 | 25.438 | 18.741 | 18.568 | 1.00 | 31.63 |
| atom | 3003 | CA | PRO | 391 | 26.326 | 19.640 | 16.464 | 1.00 | 32.78 |
| atom | 3004 | CB | PRO | 391 | 27.455 | 19.190 | 17.382 | 1.00 | 32.01 |
| atom | 3005 | CG | PRO | 391 | 26.853 | 19.204 | 18.748 | 1.00 | 31.87 |
| atom | 3006 | C | PRO | 391 | 26.454 | 19.036 | 15.069 | 1.00 | 33.95 |
| atom | 3007 | O | PRO | 391 | 26.680 | 19.768 | 14.093 | 1.00 | 34.28 |
| atom | 3008 | N | LEU | 392 | 26.330 | 17.721 | 14.869 | 1.00 | 34.68 |
| atom | 3009 | CA | LEU | 392 | 26.436 | 17.125 | 13.534 | 1.00 | 35.01 |
| atom | 3010 | CB | LEU | 392 | 26.497 | 15.594 | 13.503 | 1.00 | 33.24 |
| atom | 3011 | CG | LEU | 392 | 27.633 | 14.875 | 14.228 | 1.00 | 33.16 |
| atom | 3012 | CD1 | LEU | 392 | 27.551 | 13.358 | 14.080 | 1.00 | 33.71 |
| atom | 3013 | CD2 | LEU | 392 | 28.988 | 15.363 | 13.739 | 1.00 | 32.20 |
| atom | 3014 | C | LEU | 392 | 25.250 | 17.579 | 12.678 | 1.00 | 34.77 |
| atom | 3015 | O | LEU | 392 | 25.421 | 17.837 | 11.490 | 1.00 | 35.58 |
| atom | 3016 | N | ALA | 393 | 24.059 | 17.698 | 13.249 | 1.00 | 34.40 |
| atom | 3017 | CA | ALA | 393 | 22.886 | 18.133 | 12.497 | 1.00 | 34.19 |
| atom | 3018 | CB | ALA | 393 | 21.643 | 18.057 | 13.367 | 1.00 | 33.08 |
| atom | 3019 | C | ALA | 393 | 23.069 | 19.540 | 11.968 | 1.00 | 35.46 |
| atom | 3020 | O | ALA | 393 | 22.954 | 19.790 | 10.767 | 1.00 | 35.67 |

| | | | | | | | | | |
|---|---|---|---|---|---|---|---|---|---|
| atom | 3021 | N | ARG | 394 | 23.494 | 20.470 | 12.837 | 1.00 | 35.98 |
| atom | 3022 | CA | ARG | 394 | 23.684 | 21.857 | 12.419 | 1.00 | 36.11 |
| atom | 3023 | CB | ARG | 394 | 23.828 | 22.786 | 13.625 | 1.00 | 34.15 |
| atom | 3024 | CG | ARG | 394 | 22.559 | 22.929 | 14.434 | 1.00 | 31.37 |
| atom | 3025 | CD | ARG | 394 | 22.781 | 23.662 | 15.744 | 1.00 | 31.88 |
| atom | 3026 | NE | ARG | 394 | 21.538 | 23.644 | 16.509 | 1.00 | 32.91 |
| atom | 3027 | CZ | ARG | 394 | 21.287 | 24.206 | 17.676 | 1.00 | 32.38 |
| atom | 3028 | NH1 | ARG | 394 | 22.226 | 24.897 | 18.303 | 1.00 | 30.99 |
| atom | 3029 | NH2 | ARG | 394 | 20.069 | 24.059 | 18.192 | 1.00 | 33.05 |
| atom | 3030 | C | ARG | 394 | 24.885 | 21.952 | 11.497 | 1.00 | 37.45 |
| atom | 3031 | O | ARG | 394 | 24.982 | 22.847 | 10.655 | 1.00 | 37.12 |
| atom | 3032 | N | ALA | 395 | 25.823 | 21.016 | 11.668 | 1.00 | 37.82 |
| atom | 3033 | CA | ALA | 395 | 26.999 | 20.974 | 10.799 | 1.00 | 39.25 |
| atom | 3034 | CB | ALA | 395 | 28.047 | 20.027 | 11.355 | 1.00 | 37.77 |
| atom | 3035 | C | ALA | 395 | 26.562 | 20.591 | 9.376 | 1.00 | 39.69 |
| atom | 3036 | O | ALA | 395 | 27.052 | 21.158 | 8.396 | 1.00 | 38.32 |
| atom | 3037 | N | ALA | 396 | 25.589 | 19.684 | 9.263 | 1.00 | 40.30 |
| atom | 3038 | CA | ALA | 396 | 25.055 | 19.312 | 7.964 | 1.00 | 42.27 |
| atom | 3039 | CB | ALA | 396 | 24.204 | 18.056 | 8.032 | 1.00 | 39.84 |
| atom | 3040 | C | ALA | 396 | 24.254 | 20.473 | 7.367 | 1.00 | 43.62 |
| atom | 3041 | O | ALA | 396 | 24.425 | 20.764 | 6.177 | 1.00 | 43.22 |
| atom | 3042 | N | TRP | 397 | 23.449 | 21.152 | 8.170 | 1.00 | 45.19 |
| atom | 3043 | CA | TRP | 397 | 22.639 | 22.279 | 7.715 | 1.00 | 47.62 |
| atom | 3044 | CB | TRP | 397 | 21.745 | 22.834 | 8.814 | 1.00 | 44.59 |
| atom | 3045 | CG | TRP | 397 | 20.716 | 23.841 | 8.418 | 1.00 | 43.89 |
| atom | 3046 | CD2 | TRP | 397 | 19.324 | 23.607 | 8.155 | 1.00 | 40.94 |
| atom | 3047 | CE2 | TRP | 397 | 18.741 | 24.849 | 7.837 | 1.00 | 40.61 |
| atom | 3048 | CE3 | TRP | 397 | 18.519 | 22.469 | 8.154 | 1.00 | 39.29 |
| atom | 3049 | CD1 | TRP | 397 | 20.905 | 25.188 | 8.255 | 1.00 | 43.89 |
| atom | 3050 | NE1 | TRP | 397 | 19.724 | 25.791 | 7.893 | 1.00 | 40.91 |
| atom | 3051 | CZ2 | TRP | 397 | 17.386 | 24.983 | 7.523 | 1.00 | 40.82 |
| atom | 3052 | CZ3 | TRP | 397 | 17.178 | 22.596 | 7.860 | 1.00 | 38.93 |
| atom | 3053 | CH2 | TRP | 397 | 16.624 | 23.849 | 7.534 | 1.00 | 39.60 |
| atom | 3054 | C | TRP | 397 | 23.533 | 23.384 | 7.185 | 1.00 | 49.78 |
| atom | 3055 | O | TRP | 397 | 23.436 | 23.781 | 6.029 | 1.00 | 50.68 |
| atom | 3056 | N | GLU | 398 | 24.506 | 23.814 | 7.976 | 1.00 | 52.21 |

| atom | 3057 | CA | GLU | 398 | 25.437 | 24.866 | 7.592 | 1.00 | 54.00 |
|------|------|------|------|-----|--------|--------|--------|------|-------|
| atom | 3058 | CB | GLU | 398 | 26.269 | 25.269 | 8.823 | 1.00 | 52.21 |
| atom | 3059 | CG | GLU | 398 | 25.362 | 25.935 | 9.842 | 1.00 | 52.41 |
| atom | 3060 | CD | GLU | 398 | 25.791 | 25.772 | 11.281 | 1.00 | 52.94 |
| atom | 3061 | OE1 | GLU | 398 | 27.003 | 25.651 | 11.534 | 1.00 | 53.03 |
| atom | 3062 | OE2 | GLU | 398 | 24.875 | 25.766 | 12.135 | 1.00 | 53.80 |
| atom | 3063 | C | GLU | 398 | 26.321 | 24.518 | 6.411 | 1.00 | 55.72 |
| atom | 3064 | O | GLU | 398 | 26.841 | 25.410 | 5.734 | 1.00 | 55.38 |
| atom | 3065 | N | THR | 399 | 26.540 | 23.240 | 6.139 | 1.00 | 57.98 |
| atom | 3066 | CA | THR | 399 | 27.334 | 22.789 | 5.006 | 1.00 | 60.29 |
| atom | 3067 | CB | THR | 399 | 27.741 | 21.310 | 5.143 | 1.00 | 59.03 |
| atom | 3068 | OG1 | THR | 399 | 28.715 | 21.169 | 6.193 | 1.00 | 57.27 |
| atom | 3069 | CG2 | THR | 399 | 28.332 | 20.761 | 3.856 | 1.00 | 55.95 |
| atom | 3070 | C | THR | 399 | 26.544 | 22.989 | 3.712 | 1.00 | 62.62 |
| atom | 3071 | O | THR | 399 | 27.077 | 23.478 | 2.723 | 1.00 | 62.55 |
| atom | 3072 | N | ALA | 400 | 25.266 | 22.634 | 3.734 | 1.00 | 64.97 |
| atom | 3073 | CA | ALA | 400 | 24.382 | 22.722 | 2.590 | 1.00 | 68.25 |
| atom | 3074 | CB | ALA | 400 | 23.345 | 21.600 | 2.680 | 1.00 | 66.31 |
| atom | 3075 | C | ALA | 400 | 23.636 | 24.032 | 2.406 | 1.00 | 70.56 |
| atom | 3076 | O | ALA | 400 | 22.955 | 24.196 | 1.397 | 1.00 | 70.31 |
| atom | 3077 | N | ARG | 401 | 23.685 | 24.930 | 3.366 | 1.00 | 73.82 |
| atom | 3078 | CA | ARG | 401 | 23.064 | 26.248 | 3.259 | 1.00 | 77.65 |
| atom | 3079 | CB | ARG | 401 | 21.815 | 26.375 | 4.112 | 1.00 | 78.00 |
| atom | 3080 | CG | ARG | 401 | 20.571 | 25.952 | 3.340 | 1.00 | 79.04 |
| atom | 3081 | CD | ARG | 401 | 19.459 | 25.515 | 4.279 | 1.00 | 80.15 |
| atom | 3082 | NE | ARG | 401 | 18.245 | 25.207 | 3.522 | 1.00 | 81.25 |
| atom | 3083 | CZ | ARG | 401 | 17.486 | 26.121 | 2.927 | 1.00 | 81.41 |
| atom | 3084 | NH1 | ARG | 401 | 17.803 | 27.411 | 3.008 | 1.00 | 81.90 |
| atom | 3085 | NH2 | ARG | 401 | 16.407 | 25.744 | 2.256 | 1.00 | 80.87 |
| atom | 3086 | C | ARG | 401 | 24.137 | 27.263 | 3.624 | 1.00 | 80.29 |
| atom | 3087 | O | ARG | 401 | 25.287 | 26.830 | 3.785 | 1.00 | 80.65 |
| atom | 3088 | N | HIS | 402 | 23.854 | 28.553 | 3.685 | 1.00 | 83.11 |
| atom | 3089 | CA | HIS | 402 | 24.924 | 29.486 | 4.048 | 1.00 | 86.13 |
| atom | 3090 | CB | HIS | 402 | 25.225 | 30.523 | 2.982 | 1.00 | 91.11 |
| atom | 3091 | CG | HIS | 402 | 25.771 | 29.917 | 1.723 | 1.00 | 94.82 |
| atom | 3092 | CD2 | HIS | 402 | 27.008 | 29.537 | 1.333 | 1.00 | 96.01 |

| atom | 3093 | ND1 | HIS | 402 | 24.928 | 29.621 | 0.671 | 1.00 | 96.32 |
|------|------|-----|-----|-----|--------|--------|--------|------|-------|
| atom | 3094 | CE1 | HIS | 402 | 25.622 | 29.097 | -0.322 | 1.00 | 96.81 |
| atom | 3095 | NE2 | HIS | 402 | 26.887 | 29.034 | 0.056 | 1.00 | 97.03 |
| atom | 3096 | C | HIS | 402 | 24.531 | 30.181 | 5.339 | 1.00 | 86.71 |
| atom | 3097 | O | HIS | 402 | 23.413 | 30.689 | 5.432 | 1.00 | 87.33 |
| atom | 3098 | N | THR | 403 | 25.420 | 30.139 | 6.319 | 1.00 | 87.01 |
| atom | 3099 | CA | THR | 403 | 25.039 | 30.722 | 7.598 | 1.00 | 87.23 |
| atom | 3100 | CB | THR | 403 | 24.571 | 29.569 | 8.531 | 1.00 | 88.22 |
| atom | 3101 | OG1 | THR | 403 | 25.411 | 28.440 | 8.220 | 1.00 | 88.58 |
| atom | 3102 | CG2 | THR | 403 | 23.118 | 29.177 | 8.395 | 1.00 | 88.27 |
| atom | 3103 | C | THR | 403 | 26.185 | 31.438 | 8.257 | 1.00 | 86.71 |
| atom | 3104 | O | THR | 403 | 26.915 | 30.816 | 9.045 | 1.00 | 87.12 |
| atom | 3105 | N | PRO | 404 | 26.201 | 32.769 | 8.228 | 1.00 | 85.86 |
| atom | 3106 | CD | PRO | 404 | 25.301 | 33.637 | 7.444 | 1.00 | 85.94 |
| atom | 3107 | CA | PRO | 404 | 27.145 | 33.546 | 9.027 | 1.00 | 84.50 |
| atom | 3108 | CB | PRO | 404 | 26.916 | 34.982 | 8.649 | 1.00 | 85.41 |
| atom | 3109 | CG | PRO | 404 | 25.622 | 35.026 | 7.921 | 1.00 | 85.62 |
| atom | 3110 | C | PRO | 404 | 26.840 | 33.206 | 10.481 | 1.00 | 82.90 |
| atom | 3111 | O | PRO | 404 | 27.719 | 33.279 | 11.340 | 1.00 | 83.57 |
| atom | 3112 | N | VAL | 405 | 25.612 | 32.787 | 10.797 | 1.00 | 80.26 |
| atom | 3113 | CA | VAL | 405 | 25.258 | 32.209 | 12.077 | 1.00 | 76.56 |
| atom | 3114 | CB | VAL | 405 | 23.914 | 32.621 | 12.655 | 1.00 | 77.33 |
| atom | 3115 | CG1 | VAL | 405 | 23.803 | 32.135 | 14.095 | 1.00 | 78.55 |
| atom | 3116 | CG2 | VAL | 405 | 23.779 | 34.136 | 12.579 | 1.00 | 77.31 |
| atom | 3117 | C | VAL | 405 | 25.395 | 30.679 | 11.954 | 1.00 | 73.50 |
| atom | 3118 | O | VAL | 405 | 24.621 | 29.795 | 11.632 | 1.00 | 74.47 |
| atom | 3119 | N | ASN | 406 | 26.666 | 30.382 | 12.116 | 1.00 | 69.31 |
| atom | 3120 | CA | ASN | 406 | 27.455 | 29.183 | 12.100 | 1.00 | 63.67 |
| atom | 3121 | CB | ASN | 406 | 28.793 | 29.615 | 11.520 | 1.00 | 63.20 |
| atom | 3122 | CG | ASN | 406 | 30.127 | 29.066 | 11.886 | 1.00 | 62.71 |
| atom | 3123 | OD1 | ASN | 406 | 31.137 | 29.632 | 11.419 | 1.00 | 61.73 |
| atom | 3124 | ND2 | ASN | 406 | 30.304 | 28.012 | 12.668 | 1.00 | 61.54 |
| atom | 3125 | C | ASN | 406 | 27.667 | 28.644 | 13.510 | 1.00 | 59.88 |
| atom | 3126 | O | ASN | 406 | 27.918 | 29.420 | 14.439 | 1.00 | 59.66 |
| atom | 3127 | N | SER | 407 | 27.647 | 27.323 | 13.630 | 1.00 | 55.29 |
| atom | 3128 | CA | SER | 407 | 27.859 | 26.678 | 14.912 | 1.00 | 49.74 |

```
atom  3129  CB   SER  407      26.591  25.893  15.266  1.00 47.28
atom  3130  OG   SER  407      26.402  24.857  14.324  1.00 45.94
atom  3131  C    SER  407      29.044  25.727  14.919  1.00 46.85
atom  3132  O    SER  407      29.650  25.524  15.970  1.00 46.32
atom  3133  N    TRP  408      29.384  25.157  13.779  1.00 44.02
atom  3134  CA   TRP  408      30.441  24.176  13.645  1.00 42.14
atom  3135  CB   TRP  408      30.528  23.673  12.201  1.00 42.71
atom  3136  CG   TRP  408      31.041  24.595  11.151  1.00 41.41
atom  3137  CD2  TRP  408      32.404  24.849  10.795  1.00 39.86
atom  3138  CE2  TRP  408      32.389  25.779   9.731  1.00 40.89
atom  3139  CE3  TRP  408      33.633  24.402  11.274  1.00 39.00
atom  3140  CD1  TRP  408      30.274  25.355  10.299  1.00 41.36
atom  3141  NE1  TRP  408      31.084  26.065   9.441  1.00 39.61
atom  3142  CZ2  TRP  408      33.565  26.255   9.146  1.00 42.06
atom  3143  CZ3  TRP  408      34.794  24.875  10.699  1.00 39.45
atom  3144  CH2  TRP  408      34.760  25.796   9.637  1.00 40.98
atom  3145  C    TRP  408      31.832  24.552  14.129  1.00 40.31
atom  3146  O    TRP  408      32.501  23.703  14.742  1.00 40.63
atom  3147  N    LEU  409      32.282  25.771  13.867  1.00 38.52
atom  3148  CA   LEU  409      33.609  26.187  14.299  1.00 37.09
atom  3149  CB   LEU  409      34.026  27.419  13.511  1.00 36.98
atom  3150  CG   LEU  409      35.421  27.942  13.889  1.00 36.72
atom  3151  CD1  LEU  409      36.486  27.060  13.280  1.00 34.87
atom  3152  CD2  LEU  409      35.544  29.407  13.489  1.00 37.52
atom  3153  C    LEU  409      33.643  26.380  15.811  1.00 36.39
atom  3154  O    LEU  409      34.633  26.061  16.473  1.00 36.42
atom  3155  N    GLY  410      32.544  26.824  16.407  1.00 34.70
atom  3156  CA   GLY  410      32.430  26.928  17.850  1.00 34.06
atom  3157  C    GLY  410      32.428  25.533  18.477  1.00 32.83
atom  3158  O    GLY  410      32.998  25.389  19.553  1.00 33.25
atom  3159  N    ASN  411      31.828  24.532  17.856  1.00 31.83
atom  3160  CA   ASN  411      31.845  23.170  18.396  1.00 31.75
atom  3161  CB   ASN  411      30.826  22.247  17.742  1.00 31.51
atom  3162  CG   ASN  411      29.406  22.544  18.185  1.00 33.79
atom  3163  OD1  ASN  411      29.036  22.563  19.365  1.00 33.87
atom  3164  ND2  ASN  411      28.548  22.792  17.197  1.00 33.65
```

| atom | 3165 | C   | ASN | 411 | 33.252 | 22.579 | 18.323 | 1.00 | 30.32 |
|------|------|-----|-----|-----|--------|--------|--------|------|-------|
| atom | 3166 | O   | ASN | 411 | 33.712 | 22.003 | 19.301 | 1.00 | 28.91 |
| atom | 3167 | N   | ILE | 412 | 33.959 | 22.838 | 17.225 | 1.00 | 30.29 |
| atom | 3168 | CA  | ILE | 412 | 35.352 | 22.412 | 17.107 | 1.00 | 29.80 |
| atom | 3169 | CB  | ILE | 412 | 35.930 | 22.759 | 15.722 | 1.00 | 28.33 |
| atom | 3170 | CG2 | ILE | 412 | 37.433 | 22.535 | 15.671 | 1.00 | 26.14 |
| atom | 3171 | CG1 | ILE | 412 | 35.204 | 21.926 | 14.665 | 1.00 | 30.28 |
| atom | 3172 | CD1 | ILE | 412 | 35.617 | 22.087 | 13.221 | 1.00 | 30.99 |
| atom | 3173 | C   | ILE | 412 | 36.197 | 22.967 | 18.243 | 1.00 | 29.45 |
| atom | 3174 | O   | ILE | 412 | 36.946 | 22.242 | 18.892 | 1.00 | 29.27 |
| atom | 3175 | N   | ILE | 413 | 36.110 | 24.258 | 18.533 | 1.00 | 30.12 |
| atom | 3176 | CA  | ILE | 413 | 36.813 | 24.898 | 19.632 | 1.00 | 29.29 |
| atom | 3177 | CB  | ILE | 413 | 36.575 | 26.426 | 19.550 | 1.00 | 27.87 |
| atom | 3178 | CG2 | ILE | 413 | 37.046 | 27.109 | 20.833 | 1.00 | 22.19 |
| atom | 3179 | CG1 | ILE | 413 | 37.294 | 26.937 | 18.286 | 1.00 | 27.39 |
| atom | 3180 | CD1 | ILE | 413 | 37.149 | 28.412 | 17.985 | 1.00 | 22.53 |
| atom | 3181 | C   | ILE | 413 | 36.361 | 24.442 | 21.016 | 1.00 | 29.91 |
| atom | 3182 | O   | ILE | 413 | 37.182 | 24.135 | 21.886 | 1.00 | 29.57 |
| atom | 3183 | N   | MET | 414 | 35.059 | 24.450 | 21.277 | 1.00 | 29.74 |
| atom | 3184 | CA  | MET | 414 | 34.520 | 24.056 | 22.575 | 1.00 | 31.48 |
| atom | 3185 | CB  | MET | 414 | 33.026 | 24.327 | 22.722 | 1.00 | 31.34 |
| atom | 3186 | CG  | MET | 414 | 32.592 | 25.778 | 22.904 | 1.00 | 34.57 |
| atom | 3187 | SD  | MET | 414 | 30.793 | 25.889 | 23.157 | 1.00 | 34.92 |
| atom | 3188 | CE  | MET | 414 | 30.203 | 25.274 | 21.588 | 1.00 | 33.88 |
| atom | 3189 | C   | MET | 414 | 34.740 | 22.569 | 22.867 | 1.00 | 32.18 |
| atom | 3190 | O   | MET | 414 | 34.991 | 22.207 | 24.007 | 1.00 | 32.15 |
| atom | 3191 | N   | TYR | 415 | 34.590 | 21.713 | 21.856 | 1.00 | 31.89 |
| atom | 3192 | CA  | TYR | 415 | 34.751 | 20.274 | 22.041 | 1.00 | 31.79 |
| atom | 3193 | CB  | TYR | 415 | 33.441 | 19.561 | 21.652 | 1.00 | 31.26 |
| atom | 3194 | CG  | TYR | 415 | 32.278 | 20.001 | 22.531 | 1.00 | 30.18 |
| atom | 3195 | CD1 | TYR | 415 | 31.287 | 20.829 | 22.025 | 1.00 | 30.57 |
| atom | 3196 | CE1 | TYR | 415 | 30.240 | 21.259 | 22.828 | 1.00 | 29.66 |
| atom | 3197 | CD2 | TYR | 415 | 32.202 | 19.612 | 23.866 | 1.00 | 28.63 |
| atom | 3198 | CE2 | TYR | 415 | 31.160 | 20.016 | 24.667 | 1.00 | 29.14 |
| atom | 3199 | CZ  | TYR | 415 | 30.183 | 20.848 | 24.141 | 1.00 | 30.26 |
| atom | 3200 | OH  | TYR | 415 | 29.118 | 21.284 | 24.896 | 1.00 | 31.45 |

| atom | 3201 | C | TYR | 415 | 35.954 | 19.747 | 21.262 | 1.00 | 31.79 |
|------|------|------|------|-----|--------|--------|--------|------|-------|
| atom | 3202 | O | TYR | 415 | 35.987 | 18.621 | 20.775 | 1.00 | 29.58 |
| atom | 3203 | N | ALA | 416 | 37.039 | 20.552 | 21.247 | 1.00 | 32.47 |
| atom | 3204 | CA | ALA | 416 | 38.276 | 20.172 | 20.567 | 1.00 | 32.91 |
| atom | 3205 | CB | ALA | 416 | 39.398 | 21.191 | 20.693 | 1.00 | 32.98 |
| atom | 3206 | C | ALA | 416 | 38.858 | 18.833 | 20.999 | 1.00 | 32.92 |
| atom | 3207 | O | ALA | 416 | 39.227 | 18.051 | 20.138 | 1.00 | 32.59 |
| atom | 3208 | N | PRO | 417 | 38.924 | 18.528 | 22.290 | 1.00 | 32.74 |
| atom | 3209 | CD | PRO | 417 | 38.558 | 19.431 | 23.409 | 1.00 | 33.21 |
| atom | 3210 | CA | PRO | 417 | 39.453 | 17.274 | 22.755 | 1.00 | 31.81 |
| atom | 3211 | CB | PRO | 417 | 39.559 | 17.438 | 24.267 | 1.00 | 32.41 |
| atom | 3212 | CG | PRO | 417 | 39.279 | 18.858 | 24.600 | 1.00 | 33.14 |
| atom | 3213 | C | PRO | 417 | 38.561 | 16.088 | 22.445 | 1.00 | 31.61 |
| atom | 3214 | O | PRO | 417 | 39.022 | 14.962 | 22.659 | 1.00 | 31.28 |
| atom | 3215 | N | THR | 418 | 37.297 | 16.277 | 22.051 | 1.00 | 30.92 |
| atom | 3216 | CA | THR | 418 | 36.432 | 15.125 | 21.902 | 1.00 | 30.90 |
| atom | 3217 | CB | THR | 418 | 34.919 | 15.435 | 21.915 | 1.00 | 28.55 |
| atom | 3218 | OG1 | THR | 418 | 34.554 | 15.925 | 20.623 | 1.00 | 25.08 |
| atom | 3219 | CG2 | THR | 418 | 34.558 | 16.409 | 23.019 | 1.00 | 24.07 |
| atom | 3220 | C | THR | 418 | 36.733 | 14.276 | 20.669 | 1.00 | 31.63 |
| atom | 3221 | O | THR | 418 | 37.258 | 14.692 | 19.642 | 1.00 | 31.81 |
| atom | 3222 | N | LEU | 419 | 36.308 | 13.029 | 20.798 | 1.00 | 31.21 |
| atom | 3223 | CA | LEU | 419 | 36.435 | 12.022 | 19.760 | 1.00 | 32.67 |
| atom | 3224 | CB | LEU | 419 | 35.841 | 10.713 | 20.280 | 1.00 | 35.25 |
| atom | 3225 | CG | LEU | 419 | 35.880 | 9.530 | 19.321 | 1.00 | 37.83 |
| atom | 3226 | CD1 | LEU | 419 | 37.294 | 8.972 | 19.278 | 1.00 | 38.55 |
| atom | 3227 | CD2 | LEU | 419 | 34.865 | 8.484 | 19.767 | 1.00 | 38.91 |
| atom | 3228 | C | LEU | 419 | 35.717 | 12.443 | 18.486 | 1.00 | 32.51 |
| atom | 3229 | O | LEU | 419 | 36.309 | 12.398 | 17.406 | 1.00 | 32.38 |
| atom | 3230 | N | TRP | 420 | 34.473 | 12.874 | 18.613 | 1.00 | 32.70 |
| atom | 3231 | CA | TRP | 420 | 33.659 | 13.278 | 17.484 | 1.00 | 33.11 |
| atom | 3232 | CB | TRP | 420 | 32.192 | 13.369 | 17.881 | 1.00 | 31.10 |
| atom | 3233 | CG | TRP | 420 | 31.902 | 14.213 | 19.079 | 1.00 | 30.75 |
| atom | 3234 | CD2 | TRP | 420 | 31.504 | 15.585 | 19.128 | 1.00 | 28.76 |
| atom | 3235 | CE2 | TRP | 420 | 31.335 | 15.922 | 20.488 | 1.00 | 29.63 |
| atom | 3236 | CE3 | TRP | 420 | 31.270 | 16.565 | 18.177 | 1.00 | 30.63 |

| atom | 3237 | CD1 | TRP | 420 | 31.971 | 13.778 | 20.380 | 1.00 | 29.23 |
|------|------|-----|-----|-----|--------|--------|--------|------|-------|
| atom | 3238 | NE1 | TRP | 420 | 31.628 | 14.798 | 21.220 | 1.00 | 28.67 |
| atom | 3239 | CZ2 | TRP | 420 | 30.942 | 17.187 | 20.922 | 1.00 | 28.35 |
| atom | 3240 | CZ3 | TRP | 420 | 30.875 | 17.832 | 18.587 | 1.00 | 30.87 |
| atom | 3241 | CH2 | TRP | 420 | 30.713 | 18.125 | 19.951 | 1.00 | 30.22 |
| atom | 3242 | C   | TRP | 420 | 34.095 | 14.570 | 16.820 | 1.00 | 34.03 |
| atom | 3243 | O   | TRP | 420 | 34.089 | 14.625 | 15.579 | 1.00 | 35.28 |
| atom | 3244 | N   | ALA | 421 | 34.498 | 15.589 | 17.566 | 1.00 | 35.05 |
| atom | 3245 | CA  | ALA | 421 | 34.930 | 16.830 | 16.912 | 1.00 | 35.21 |
| atom | 3246 | CB  | ALA | 421 | 35.090 | 17.983 | 17.886 | 1.00 | 32.54 |
| atom | 3247 | C   | ALA | 421 | 36.233 | 16.608 | 16.140 | 1.00 | 35.06 |
| atom | 3248 | O   | ALA | 421 | 36.406 | 17.135 | 15.054 | 1.00 | 34.62 |
| atom | 3249 | N   | ARG | 422 | 37.178 | 15.880 | 16.703 | 1.00 | 35.88 |
| atom | 3250 | CA  | ARG | 422 | 38.463 | 15.622 | 16.082 | 1.00 | 36.45 |
| atom | 3251 | CB  | ARG | 422 | 39.448 | 14.916 | 17.030 | 1.00 | 33.76 |
| atom | 3252 | CG  | ARG | 422 | 39.794 | 15.760 | 18.248 | 1.00 | 30.93 |
| atom | 3253 | CD  | ARG | 422 | 40.566 | 14.972 | 19.280 | 1.00 | 30.09 |
| atom | 3254 | NE  | ARG | 422 | 41.882 | 14.639 | 18.789 | 1.00 | 30.91 |
| atom | 3255 | CZ  | ARG | 422 | 42.717 | 13.716 | 19.221 | 1.00 | 30.55 |
| atom | 3256 | NH1 | ARG | 422 | 42.442 | 12.902 | 20.222 | 1.00 | 29.68 |
| atom | 3257 | NH2 | ARG | 422 | 43.892 | 13.605 | 18.614 | 1.00 | 31.62 |
| atom | 3258 | C   | ARG | 422 | 38.327 | 14.771 | 14.828 | 1.00 | 37.67 |
| atom | 3259 | O   | ARG | 422 | 38.895 | 15.124 | 13.788 | 1.00 | 38.51 |
| atom | 3260 | N   | MET | 423 | 37.581 | 13.678 | 14.903 | 1.00 | 37.75 |
| atom | 3261 | CA  | MET | 423 | 37.513 | 12.798 | 13.735 | 1.00 | 38.53 |
| atom | 3262 | CB  | MET | 423 | 37.260 | 11.347 | 14.172 | 1.00 | 36.22 |
| atom | 3263 | CG  | MET | 423 | 38.430 | 10.790 | 14.975 | 1.00 | 36.01 |
| atom | 3264 | SD  | MET | 423 | 38.431 | 8.992  | 15.184 | 1.00 | 35.56 |
| atom | 3265 | CE  | MET | 423 | 37.009 | 8.720  | 16.189 | 1.00 | 38.15 |
| atom | 3266 | C   | MET | 423 | 36.535 | 13.283 | 12.688 | 1.00 | 38.86 |
| atom | 3267 | O   | MET | 423 | 36.860 | 13.258 | 11.500 | 1.00 | 39.81 |
| atom | 3268 | N   | ILE | 424 | 35.359 | 13.749 | 13.082 | 1.00 | 38.97 |
| atom | 3269 | CA  | ILE | 424 | 34.368 | 14.174 | 12.106 | 1.00 | 37.75 |
| atom | 3270 | CB  | ILE | 424 | 32.957 | 13.779 | 12.601 | 1.00 | 38.49 |
| atom | 3271 | CG2 | ILE | 424 | 31.894 | 14.200 | 11.588 | 1.00 | 35.83 |
| atom | 3272 | CG1 | ILE | 424 | 32.917 | 12.261 | 12.856 | 1.00 | 36.65 |

| atom | 3273 | CD1 | ILE | 424 | 31.754 | 11.812 | 13.706 | 1.00 34.42 |
|------|------|-----|-----|-----|--------|--------|--------|------------|
| atom | 3274 | C   | ILE | 424 | 34.402 | 15.646 | 11.758 | 1.00 37.30 |
| atom | 3275 | O   | ILE | 424 | 34.645 | 15.977 | 10.596 | 1.00 36.87 |
| atom | 3276 | N   | LEU | 425 | 34.121 | 16.515 | 12.721 | 1.00 36.79 |
| atom | 3277 | CA  | LEU | 425 | 34.040 | 17.955 | 12.450 | 1.00 36.40 |
| atom | 3278 | CB  | LEU | 425 | 33.674 | 18.670 | 13.747 | 1.00 35.13 |
| atom | 3279 | CG  | LEU | 425 | 32.249 | 19.072 | 14.071 | 1.00 34.93 |
| atom | 3280 | CD1 | LEU | 425 | 31.172 | 18.312 | 13.307 | 1.00 33.38 |
| atom | 3281 | CD2 | LEU | 425 | 31.998 | 18.932 | 15.575 | 1.00 33.84 |
| atom | 3282 | C   | LEU | 425 | 35.291 | 18.517 | 11.796 | 1.00 36.29 |
| atom | 3283 | O   | LEU | 425 | 35.236 | 19.101 | 10.702 | 1.00 35.35 |
| atom | 3284 | N   | MET | 426 | 36.456 | 18.340 | 12.414 | 1.00 36.26 |
| atom | 3285 | CA  | MET | 426 | 37.706 | 18.845 | 11.858 | 1.00 37.08 |
| atom | 3286 | CB  | MET | 426 | 38.899 | 18.531 | 12.751 | 1.00 33.13 |
| atom | 3287 | CG  | MET | 426 | 38.912 | 19.257 | 14.089 | 1.00 30.96 |
| atom | 3288 | SD  | MET | 426 | 40.348 | 18.860 | 15.103 | 1.00 29.76 |
| atom | 3289 | CE  | MET | 426 | 39.834 | 19.429 | 16.736 | 1.00 29.24 |
| atom | 3290 | C   | MET | 426 | 37.931 | 18.312 | 10.442 | 1.00 39.02 |
| atom | 3291 | O   | MET | 426 | 38.091 | 19.052 | 9.465  | 1.00 38.37 |
| atom | 3292 | N   | THR | 427 | 37.895 | 16.985 | 10.289 | 1.00 39.87 |
| atom | 3293 | CA  | THR | 427 | 38.102 | 16.354 | 8.995  | 1.00 40.76 |
| atom | 3294 | CB  | THR | 427 | 37.949 | 14.827 | 9.106  | 1.00 40.03 |
| atom | 3295 | OG1 | THR | 427 | 38.813 | 14.360 | 10.169 | 1.00 35.72 |
| atom | 3296 | CG2 | THR | 427 | 38.312 | 14.144 | 7.796  | 1.00 36.09 |
| atom | 3297 | C   | THR | 427 | 37.167 | 16.924 | 7.939  | 1.00 41.70 |
| atom | 3298 | O   | THR | 427 | 37.639 | 17.468 | 6.946  | 1.00 41.10 |
| atom | 3299 | N   | HIS | 428 | 35.864 | 16.853 | 8.151  | 1.00 42.78 |
| atom | 3300 | CA  | HIS | 428 | 34.870 | 17.365 | 7.214  | 1.00 43.45 |
| atom | 3301 | CB  | HIS | 428 | 33.467 | 17.138 | 7.784  | 1.00 43.08 |
| atom | 3302 | CG  | HIS | 428 | 32.342 | 17.689 | 6.971  | 1.00 45.40 |
| atom | 3303 | CD2 | HIS | 428 | 31.546 | 18.774 | 7.156  | 1.00 45.59 |
| atom | 3304 | ND1 | HIS | 428 | 31.895 | 17.102 | 5.808  | 1.00 46.11 |
| atom | 3305 | CE1 | HIS | 428 | 30.892 | 17.784 | 5.307  | 1.00 45.04 |
| atom | 3306 | NE2 | HIS | 428 | 30.653 | 18.803 | 6.113  | 1.00 45.57 |
| atom | 3307 | C   | HIS | 428 | 35.080 | 18.826 | 6.844  | 1.00 44.01 |
| atom | 3308 | O   | HIS | 428 | 35.277 | 19.161 | 5.672  | 1.00 43.70 |

| atom | 3309 | N | PHE | 429 | 35.085 | 19.734 | 7.823 | 1.00 | 44.14 |
|------|------|-----|-----|-----|--------|--------|--------|------|-------|
| atom | 3310 | CA | PHE | 429 | 35.250 | 21.155 | 7.531 | 1.00 | 43.88 |
| atom | 3311 | CB | PHE | 429 | 34.887 | 22.034 | 8.742 | 1.00 | 40.24 |
| atom | 3312 | CG | PHE | 429 | 33.392 | 21.939 | 8.984 | 1.00 | 37.92 |
| atom | 3313 | CD1 | PHE | 429 | 32.882 | 21.124 | 9.975 | 1.00 | 35.82 |
| atom | 3314 | CD2 | PHE | 429 | 32.520 | 22.662 | 8.192 | 1.00 | 36.00 |
| atom | 3315 | CE1 | PHE | 429 | 31.524 | 21.042 | 10.171 | 1.00 | 37.18 |
| atom | 3316 | CE2 | PHE | 429 | 31.157 | 22.588 | 8.386 | 1.00 | 36.86 |
| atom | 3317 | CZ | PHE | 429 | 30.653 | 21.771 | 9.380 | 1.00 | 37.75 |
| atom | 3318 | C | PHE | 429 | 36.606 | 21.533 | 6.966 | 1.00 | 44.53 |
| atom | 3319 | O | PHE | 429 | 36.663 | 22.328 | 6.022 | 1.00 | 42.96 |
| atom | 3320 | N | PHE | 430 | 37.703 | 20.961 | 7.473 | 1.00 | 44.83 |
| atom | 3321 | CA | PHE | 430 | 39.013 | 21.303 | 6.921 | 1.00 | 45.27 |
| atom | 3322 | CB | PHE | 430 | 40.184 | 20.739 | 7.724 | 1.00 | 42.91 |
| atom | 3323 | CG | PHE | 430 | 40.672 | 21.791 | 8.696 | 1.00 | 44.22 |
| atom | 3324 | CD1 | PHE | 430 | 40.113 | 21.932 | 9.958 | 1.00 | 42.81 |
| atom | 3325 | CD2 | PHE | 430 | 41.682 | 22.663 | 8.308 | 1.00 | 43.15 |
| atom | 3326 | CE1 | PHE | 430 | 40.572 | 22.909 | 10.816 | 1.00 | 41.81 |
| atom | 3327 | CE2 | PHE | 430 | 42.140 | 23.639 | 9.166 | 1.00 | 42.09 |
| atom | 3328 | CZ | PHE | 430 | 41.579 | 23.758 | 10.424 | 1.00 | 41.79 |
| atom | 3329 | C | PHE | 430 | 39.048 | 20.868 | 5.466 | 1.00 | 47.01 |
| atom | 3330 | O | PHE | 430 | 39.489 | 21.593 | 4.576 | 1.00 | 47.48 |
| atom | 3331 | N | SER | 431 | 38.521 | 19.680 | 5.192 | 1.00 | 47.80 |
| atom | 3332 | CA | SER | 431 | 38.404 | 19.179 | 3.838 | 1.00 | 49.28 |
| atom | 3333 | CB | SER | 431 | 37.581 | 17.898 | 3.880 | 1.00 | 48.60 |
| atom | 3334 | OG | SER | 431 | 37.328 | 17.436 | 2.575 | 1.00 | 50.52 |
| atom | 3335 | C | SER | 431 | 37.735 | 20.222 | 2.946 | 1.00 | 50.44 |
| atom | 3336 | O | SER | 431 | 38.273 | 20.642 | 1.927 | 1.00 | 49.92 |
| atom | 3337 | N | ILE | 432 | 36.550 | 20.679 | 3.344 | 1.00 | 51.96 |
| atom | 3338 | CA | ILE | 432 | 35.811 | 21.722 | 2.647 | 1.00 | 53.03 |
| atom | 3339 | CB | ILE | 432 | 34.455 | 22.006 | 3.308 | 1.00 | 54.28 |
| atom | 3340 | CG2 | ILE | 432 | 33.912 | 23.371 | 2.879 | 1.00 | 52.78 |
| atom | 3341 | CG1 | ILE | 432 | 33.401 | 20.946 | 2.984 | 1.00 | 54.86 |
| atom | 3342 | CD1 | ILE | 432 | 33.775 | 19.505 | 3.168 | 1.00 | 57.73 |
| atom | 3343 | C | ILE | 432 | 36.647 | 22.997 | 2.576 | 1.00 | 53.85 |
| atom | 3344 | O | ILE | 432 | 36.679 | 23.664 | 1.537 | 1.00 | 54.19 |

| | | | | | | | | | |
|---|---|---|---|---|---|---|---|---|---|
| atom | 3345 | N | LEU | 433 | 37.339 | 23.354 | 3.648 | 1.00 | 54.39 |
| atom | 3346 | CA | LEU | 433 | 38.207 | 24.520 | 3.654 | 1.00 | 55.73 |
| atom | 3347 | CB | LEU | 433 | 38.786 | 24.784 | 5.047 | 1.00 | 54.14 |
| atom | 3348 | CG | LEU | 433 | 37.766 | 25.206 | 6.112 | 1.00 | 53.57 |
| atom | 3349 | CD1 | LEU | 433 | 38.372 | 25.274 | 7.506 | 1.00 | 53.54 |
| atom | 3350 | CD2 | LEU | 433 | 37.178 | 26.554 | 5.728 | 1.00 | 52.32 |
| atom | 3351 | C | LEU | 433 | 39.301 | 24.378 | 2.599 | 1.00 | 56.60 |
| atom | 3352 | O | LEU | 433 | 39.573 | 25.335 | 1.865 | 1.00 | 56.68 |
| atom | 3353 | N | LEU | 434 | 39.949 | 23.230 | 2.486 | 1.00 | 57.17 |
| atom | 3354 | CA | LEU | 434 | 41.003 | 23.027 | 1.504 | 1.00 | 58.82 |
| atom | 3355 | CB | LEU | 434 | 41.648 | 21.647 | 1.643 | 1.00 | 58.32 |
| atom | 3356 | CG | LEU | 434 | 42.701 | 21.422 | 2.720 | 1.00 | 57.32 |
| atom | 3357 | CD1 | LEU | 434 | 42.866 | 19.936 | 2.987 | 1.00 | 56.86 |
| atom | 3358 | CD2 | LEU | 434 | 44.012 | 22.054 | 2.289 | 1.00 | 56.38 |
| atom | 3359 | C | LEU | 434 | 40.487 | 23.193 | 0.078 | 1.00 | 60.29 |
| atom | 3360 | O | LEU | 434 | 41.061 | 23.925 | -0.732 | 1.00 | 60.68 |
| atom | 3361 | N | ALA | 435 | 39.392 | 22.546 | -0.283 | 1.00 | 61.41 |
| atom | 3362 | CA | ALA | 435 | 38.829 | 22.582 | -1.618 | 1.00 | 63.40 |
| atom | 3363 | CB | ALA | 435 | 37.694 | 21.565 | -1.723 | 1.00 | 61.52 |
| atom | 3364 | C | ALA | 435 | 38.281 | 23.917 | -2.095 | 1.00 | 64.58 |
| atom | 3365 | O | ALA | 435 | 38.433 | 24.245 | -3.276 | 1.00 | 65.18 |
| atom | 3366 | N | GLN | 436 | 37.671 | 24.691 | -1.204 | 1.00 | 65.33 |
| atom | 3367 | CA | GLN | 436 | 37.128 | 25.995 | -1.566 | 1.00 | 66.41 |
| atom | 3368 | CB | GLN | 436 | 35.883 | 26.287 | -0.732 | 1.00 | 67.38 |
| atom | 3369 | CG | GLN | 436 | 34.683 | 25.436 | -1.099 | 1.00 | 68.91 |
| atom | 3370 | CD | GLN | 436 | 33.494 | 25.640 | -0.188 | 1.00 | 70.95 |
| atom | 3371 | OE1 | GLN | 436 | 33.546 | 26.472 | 0.719 | 1.00 | 72.09 |
| atom | 3372 | NE2 | GLN | 436 | 32.424 | 24.884 | -0.425 | 1.00 | 71.77 |
| atom | 3373 | C | GLN | 436 | 38.206 | 27.053 | -1.403 | 1.00 | 67.36 |
| atom | 3374 | O | GLN | 436 | 37.999 | 28.262 | -1.453 | 1.00 | 67.20 |
| atom | 3375 | N | GLU | 437 | 39.428 | 26.612 | -1.127 | 1.00 | 68.17 |
| atom | 3376 | CA | GLU | 437 | 40.637 | 27.367 | -0.936 | 1.00 | 69.15 |
| atom | 3377 | CB | GLU | 437 | 41.128 | 27.958 | -2.263 | 1.00 | 70.70 |
| atom | 3378 | CG | GLU | 437 | 41.976 | 27.009 | -3.087 | 1.00 | 73.56 |
| atom | 3379 | CD | GLU | 437 | 42.477 | 27.573 | -4.395 | 1.00 | 74.34 |
| atom | 3380 | OE1 | GLU | 437 | 42.210 | 28.748 | -4.728 | 1.00 | 75.78 |

| atom | 3381 | OE2 | GLU | 437 | 43.165 | 26.835 | -5.134 | 1.00 | 74.99 |
|------|------|-----|-----|-----|--------|--------|--------|------|-------|
| atom | 3382 | C | GLU | 437 | 40.474 | 28.486 | 0.081 | 1.00 | 69.14 |
| atom | 3383 | O | GLU | 437 | 41.096 | 29.545 | -0.017 | 1.00 | 69.69 |
| atom | 3384 | N | GLN | 438 | 39.650 | 28.275 | 1.098 | 1.00 | 68.54 |
| atom | 3385 | CA | GLN | 438 | 39.347 | 29.281 | 2.091 | 1.00 | 67.99 |
| atom | 3386 | CB | GLN | 438 | 37.830 | 29.400 | 2.265 | 1.00 | 68.91 |
| atom | 3387 | CG | GLN | 438 | 36.922 | 29.732 | 1.117 | 1.00 | 70.66 |
| atom | 3388 | CD | GLN | 438 | 35.453 | 29.571 | 1.474 | 1.00 | 72.42 |
| atom | 3389 | OE1 | GLN | 438 | 35.094 | 28.874 | 2.431 | 1.00 | 73.52 |
| atom | 3390 | NE2 | GLN | 438 | 34.564 | 30.207 | 0.715 | 1.00 | 72.17 |
| atom | 3391 | C | GLN | 438 | 39.958 | 29.012 | 3.461 | 1.00 | 67.01 |
| atom | 3392 | O | GLN | 438 | 39.226 | 29.142 | 4.458 | 1.00 | 66.87 |
| atom | 3393 | N | LEU | 439 | 41.243 | 28.717 | 3.597 | 1.00 | 66.14 |
| atom | 3394 | CA | LEU | 439 | 41.807 | 28.449 | 4.917 | 1.00 | 65.32 |
| atom | 3395 | CB | LEU | 439 | 43.107 | 27.662 | 4.813 | 1.00 | 62.95 |
| atom | 3396 | CG | LEU | 439 | 42.975 | 26.138 | 4.685 | 1.00 | 61.85 |
| atom | 3397 | CD1 | LEU | 439 | 44.358 | 25.546 | 4.428 | 1.00 | 60.25 |
| atom | 3398 | CD2 | LEU | 439 | 42.330 | 25.519 | 5.915 | 1.00 | 59.52 |
| atom | 3399 | C | LEU | 439 | 42.027 | 29.693 | 5.766 | 1.00 | 65.59 |
| atom | 3400 | O | LEU | 439 | 41.977 | 29.644 | 6.998 | 1.00 | 65.69 |
| atom | 3401 | N | GLU | 440 | 42.252 | 30.828 | 5.111 | 1.00 | 65.31 |
| atom | 3402 | CA | GLU | 440 | 42.469 | 32.081 | 5.819 | 1.00 | 65.20 |
| atom | 3403 | CB | GLU | 440 | 43.654 | 32.852 | 5.244 | 1.00 | 67.57 |
| atom | 3404 | CG | GLU | 440 | 44.232 | 32.364 | 3.935 | 1.00 | 69.96 |
| atom | 3405 | CD | GLU | 440 | 45.582 | 31.688 | 4.085 | 1.00 | 70.77 |
| atom | 3406 | OE1 | GLU | 440 | 46.426 | 32.260 | 4.813 | 1.00 | 71.48 |
| atom | 3407 | OE2 | GLU | 440 | 45.790 | 30.616 | 3.478 | 1.00 | 70.59 |
| atom | 3408 | C | GLU | 440 | 41.237 | 32.973 | 5.860 | 1.00 | 64.03 |
| atom | 3409 | O | GLU | 440 | 41.388 | 34.163 | 6.132 | 1.00 | 63.63 |
| atom | 3410 | N | LYS | 441 | 40.052 | 32.416 | 5.660 | 1.00 | 63.26 |
| atom | 3411 | CA | LYS | 441 | 38.830 | 33.218 | 5.674 | 1.00 | 62.52 |
| atom | 3412 | CB | LYS | 441 | 37.826 | 32.634 | 4.686 | 1.00 | 63.67 |
| atom | 3413 | CG | LYS | 441 | 36.709 | 33.547 | 4.229 | 1.00 | 65.10 |
| atom | 3414 | CD | LYS | 441 | 35.409 | 33.298 | 4.969 | 1.00 | 67.31 |
| atom | 3415 | CE | LYS | 441 | 34.225 | 33.991 | 4.303 | 1.00 | 68.16 |
| atom | 3416 | NZ | LYS | 441 | 32.956 | 33.729 | 5.046 | 1.00 | 68.70 |

| atom | 3417 | C | LYS | 441 | 38.270 | 33.335 | 7.085 | 1.00 | 61.57 |
|------|------|------|-----|-----|--------|--------|-------|------|-------|
| atom | 3418 | 0 | LYS | 441 | 37.713 | 32.400 | 7.653 | 1.00 | 61.71 |
| atom | 3419 | N | ALA | 442 | 38.451 | 34.509 | 7.682 | 1.00 | 60.16 |
| atom | 3420 | CA | ALA | 442 | 37.974 | 34.760 | 9.035 | 1.00 | 58.38 |
| atom | 3421 | CB | ALA | 442 | 38.213 | 36.210 | 9.410 | 1.00 | 56.45 |
| atom | 3422 | C | ALA | 442 | 36.490 | 34.427 | 9.137 | 1.00 | 57.62 |
| atom | 3423 | 0 | ALA | 442 | 35.686 | 34.930 | 8.346 | 1.00 | 57.44 |
| atom | 3424 | N | LEU | 443 | 36.129 | 33.601 | 10.112 | 1.00 | 56.15 |
| atom | 3425 | CA | LEU | 443 | 34.733 | 33.248 | 10.302 | 1.00 | 55.01 |
| atom | 3426 | CB | LEU | 443 | 34.484 | 31.747 | 10.221 | 1.00 | 55.68 |
| atom | 3427 | CG | LEU | 443 | 34.082 | 31.115 | 8.891 | 1.00 | 57.40 |
| atom | 3428 | CD1 | LEU | 443 | 35.253 | 30.980 | 7.936 | 1.00 | 57.65 |
| atom | 3429 | CD2 | LEU | 443 | 33.472 | 29.737 | 9.118 | 1.00 | 56.89 |
| atom | 3430 | C | LEU | 443 | 34.235 | 33.761 | 11.654 | 1.00 | 54.60 |
| atom | 3431 | 0 | LEU | 443 | 34.939 | 33.701 | 12.666 | 1.00 | 54.65 |
| atom | 3432 | N | ASP | 444 | 33.001 | 34.268 | 11.664 | 1.00 | 53.18 |
| atom | 3433 | CA | ASP | 444 | 32.432 | 34.729 | 12.927 | 1.00 | 51.85 |
| atom | 3434 | CB | ASP | 444 | 31.300 | 35.731 | 12.750 | 1.00 | 54.15 |
| atom | 3435 | CG | ASP | 444 | 31.845 | 37.076 | 12.287 | 1.00 | 56.66 |
| atom | 3436 | OD1 | ASP | 444 | 31.474 | 38.115 | 12.872 | 1.00 | 56.77 |
| atom | 3437 | OD2 | ASP | 444 | 32.664 | 37.098 | 11.339 | 1.00 | 57.61 |
| atom | 3438 | C | ASP | 444 | 32.011 | 33.495 | 13.730 | 1.00 | 50.41 |
| atom | 3439 | 0 | ASP | 444 | 31.420 | 32.529 | 13.244 | 1.00 | 49.80 |
| atom | 3440 | N | CYS | 445 | 32.385 | 33.555 | 15.007 | 1.00 | 48.10 |
| atom | 3441 | CA | CYS | 445 | 32.105 | 32.440 | 15.895 | 1.00 | 46.01 |
| atom | 3442 | CB | CYS | 445 | 33.353 | 31.553 | 15.947 | 1.00 | 45.19 |
| atom | 3443 | SG | CYS | 445 | 33.151 | 30.116 | 17.018 | 1.00 | 44.60 |
| atom | 3444 | C | CYS | 445 | 31.677 | 32.913 | 17.268 | 1.00 | 45.03 |
| atom | 3445 | 0 | CYS | 445 | 32.441 | 33.531 | 18.011 | 1.00 | 44.71 |
| atom | 3446 | N | GLN | 446 | 30.430 | 32.615 | 17.613 | 1.00 | 43.85 |
| atom | 3447 | CA | GLN | 446 | 29.886 | 33.014 | 18.899 | 1.00 | 43.53 |
| atom | 3448 | CB | GLN | 446 | 28.410 | 33.429 | 18.744 | 1.00 | 45.01 |
| atom | 3449 | CG | GLN | 446 | 27.663 | 33.612 | 20.050 | 1.00 | 44.42 |
| atom | 3450 | CD | GLN | 446 | 27.856 | 34.950 | 20.731 | 1.00 | 44.74 |
| atom | 3451 | OE1 | GLN | 446 | 27.635 | 35.107 | 21.936 | 1.00 | 43.39 |
| atom | 3452 | NE2 | GLN | 446 | 28.255 | 35.979 | 19.988 | 1.00 | 45.27 |

| atom | 3453 | C | GLN | 446 | 30.012 | 31.892 | 19.910 | 1.00 | 42.67 |
|------|------|-----|-----|-----|--------|--------|--------|------|-------|
| atom | 3454 | 0 | GLN | 446 | 29.327 | 30.859 | 19.825 | 1.00 | 43.84 |
| atom | 3455 | N | ILE | 447 | 30.898 | 32.024 | 20.886 | 1.00 | 41.53 |
| atom | 3456 | CA | ILE | 447 | 31.032 | 31.021 | 21.929 | 1.00 | 40.30 |
| atom | 3457 | CB | ILE | 447 | 32.051 | 29.891 | 21.713 | 1.00 | 40.47 |
| atom | 3458 | CG2 | ILE | 447 | 31.552 | 28.861 | 20.705 | 1.00 | 39.77 |
| atom | 3459 | CG1 | ILE | 447 | 33.440 | 30.427 | 21.389 | 1.00 | 39.57 |
| atom | 3460 | CD1 | ILE | 447 | 33.685 | 30.920 | 19.996 | 1.00 | 40.48 |
| atom | 3461 | C | ILE | 447 | 31.408 | 31.727 | 23.223 | 1.00 | 39.41 |
| atom | 3462 | 0 | ILE | 447 | 31.999 | 32.798 | 23.212 | 1.00 | 39.16 |
| atom | 3463 | N | TYR | 448 | 31.073 | 31.137 | 24.359 | 1.00 | 39.67 |
| atom | 3464 | CA | TYR | 448 | 31.466 | 31.676 | 25.653 | 1.00 | 39.46 |
| atom | 3465 | CB | TYR | 448 | 33.007 | 31.643 | 25.737 | 1.00 | 38.64 |
| atom | 3466 | CG | TYR | 448 | 33.647 | 30.295 | 25.515 | 1.00 | 38.93 |
| atom | 3467 | CD1 | TYR | 448 | 34.700 | 30.155 | 24.619 | 1.00 | 39.13 |
| atom | 3468 | CE1 | TYR | 448 | 35.313 | 28.935 | 24.400 | 1.00 | 38.68 |
| atom | 3469 | CD2 | TYR | 448 | 33.212 | 29.157 | 26.183 | 1.00 | 38.79 |
| atom | 3470 | CE2 | TYR | 448 | 33.805 | 27.926 | 25.966 | 1.00 | 38.91 |
| atom | 3471 | CZ | TYR | 448 | 34.859 | 27.822 | 25.079 | 1.00 | 39.40 |
| atom | 3472 | OH | TYR | 448 | 35.460 | 26.601 | 24.858 | 1.00 | 39.03 |
| atom | 3473 | C | TYR | 448 | 30.974 | 33.086 | 25.922 | 1.00 | 39.40 |
| atom | 3474 | 0 | TYR | 448 | 31.612 | 33.799 | 26.696 | 1.00 | 40.07 |
| atom | 3475 | N | GLY | 449 | 29.915 | 33.592 | 25.325 | 1.00 | 39.80 |
| atom | 3476 | CA | GLY | 449 | 29.375 | 34.913 | 25.522 | 1.00 | 38.64 |
| atom | 3477 | C | GLY | 449 | 29.961 | 35.996 | 24.628 | 1.00 | 38.76 |
| atom | 3478 | 0 | GLY | 449 | 29.835 | 37.194 | 24.924 | 1.00 | 38.25 |
| atom | 3479 | N | ALA | 450 | 30.620 | 35.624 | 23.533 | 1.00 | 37.18 |
| atom | 3480 | CA | ALA | 450 | 31.200 | 36.663 | 22.683 | 1.00 | 36.32 |
| atom | 3481 | CB | ALA | 450 | 32.585 | 37.062 | 23.178 | 1.00 | 33.02 |
| atom | 3482 | C | ALA | 450 | 31.256 | 36.202 | 21.240 | 1.00 | 35.61 |
| atom | 3483 | 0 | ALA | 450 | 31.083 | 35.018 | 20.954 | 1.00 | 35.49 |
| atom | 3484 | N | CYS | 451 | 31.467 | 37.162 | 20.358 | 1.00 | 35.26 |
| atom | 3485 | CA | CYS | 451 | 31.528 | 36.843 | 18.934 | 1.00 | 35.47 |
| atom | 3486 | CB | CYS | 451 | 30.456 | 37.655 | 18.206 | 1.00 | 34.25 |
| atom | 3487 | SG | CYS | 451 | 30.741 | 37.592 | 16.427 | 1.00 | 37.71 |
| atom | 3488 | C | CYS | 451 | 32.921 | 37.117 | 18.389 | 1.00 | 34.76 |

| atom | 3489 | O | CYS | 451 | 33.288 | 38.268 | 18.157 | 1.00 | 36.59 |
|------|------|-----|------|-----|--------|--------|--------|------|-------|
| atom | 3490 | N | TYR | 452 | 33.692 | 36.067 | 18.185 | 1.00 | 33.75 |
| atom | 3491 | CA | TYR | 452 | 35.056 | 36.168 | 17.720 | 1.00 | 32.90 |
| atom | 3492 | CB | TYR | 452 | 35.852 | 34.982 | 18.285 | 1.00 | 31.76 |
| atom | 3493 | CG | TYR | 452 | 35.885 | 34.950 | 19.800 | 1.00 | 28.83 |
| atom | 3494 | CD1 | TYR | 452 | 34.908 | 34.268 | 20.507 | 1.00 | 28.43 |
| atom | 3495 | CE1 | TYR | 452 | 34.930 | 34.222 | 21.886 | 1.00 | 28.50 |
| atom | 3496 | CD2 | TYR | 452 | 36.894 | 35.581 | 20.504 | 1.00 | 27.11 |
| atom | 3497 | CE2 | TYR | 452 | 36.920 | 35.535 | 21.885 | 1.00 | 27.18 |
| atom | 3498 | CZ | TYR | 452 | 35.941 | 34.867 | 22.576 | 1.00 | 27.44 |
| atom | 3499 | OH | TYR | 452 | 35.932 | 34.813 | 23.947 | 1.00 | 25.41 |
| atom | 3500 | C | TYR | 452 | 35.150 | 36.126 | 16.207 | 1.00 | 33.41 |
| atom | 3501 | O | TYR | 452 | 34.240 | 35.660 | 15.538 | 1.00 | 33.73 |
| atom | 3502 | N | SER | 453 | 36.255 | 36.607 | 15.680 | 1.00 | 34.36 |
| atom | 3503 | CA | SER | 453 | 36.549 | 36.586 | 14.251 | 1.00 | 35.61 |
| atom | 3504 | CB | SER | 453 | 36.925 | 37.986 | 13.786 | 1.00 | 35.91 |
| atom | 3505 | OG | SER | 453 | 37.250 | 38.064 | 12.411 | 1.00 | 36.63 |
| atom | 3506 | C | SER | 453 | 37.701 | 35.583 | 14.110 | 1.00 | 36.38 |
| atom | 3507 | O | SER | 453 | 38.862 | 35.929 | 14.338 | 1.00 | 35.69 |
| atom | 3508 | N | ILE | 454 | 37.348 | 34.322 | 13.887 | 1.00 | 37.63 |
| atom | 3509 | CA | ILE | 454 | 38.331 | 33.259 | 13.838 | 1.00 | 39.19 |
| atom | 3510 | CB | ILE | 454 | 37.801 | 31.916 | 14.403 | 1.00 | 37.03 |
| atom | 3511 | CG2 | ILE | 454 | 38.991 | 30.979 | 14.567 | 1.00 | 35.02 |
| atom | 3512 | CG1 | ILE | 454 | 37.017 | 32.115 | 15.690 | 1.00 | 36.38 |
| atom | 3513 | CD1 | ILE | 454 | 37.851 | 32.260 | 16.947 | 1.00 | 37.92 |
| atom | 3514 | C | ILE | 454 | 38.851 | 32.899 | 12.451 | 1.00 | 40.53 |
| atom | 3515 | O | ILE | 454 | 38.103 | 32.582 | 11.538 | 1.00 | 41.20 |
| atom | 3516 | N | GLU | 455 | 40.160 | 32.861 | 12.366 | 1.00 | 41.69 |
| atom | 3517 | CA | GLU | 455 | 40.881 | 32.416 | 11.193 | 1.00 | 44.47 |
| atom | 3518 | CB | GLU | 455 | 42.188 | 33.187 | 11.015 | 1.00 | 48.56 |
| atom | 3519 | CG | GLU | 455 | 42.150 | 34.392 | 10.086 | 1.00 | 52.12 |
| atom | 3520 | CD | GLU | 455 | 43.543 | 34.611 | 9.521 | 1.00 | 56.68 |
| atom | 3521 | OE1 | GLU | 455 | 44.388 | 35.188 | 10.244 | 1.00 | 58.40 |
| atom | 3522 | OE2 | GLU | 455 | 43.782 | 34.166 | 8.375 | 1.00 | 60.39 |
| atom | 3523 | C | GLU | 455 | 41.199 | 30.936 | 11.444 | 1.00 | 44.83 |
| atom | 3524 | O | GLU | 455 | 42.018 | 30.583 | 12.301 | 1.00 | 44.62 |

| atom | 3525 | N | PRO | 456 | | 40.581 | 30.058 | 10.673 | 1.00 | 44.97 |
|------|------|-----|-----|-----|--|--------|--------|--------|------|-------|
| atom | 3526 | CD | PRO | 456 | | 39.603 | 30.411 | 9.606 | 1.00 | 45.05 |
| atom | 3527 | CA | PRO | 456 | | 40.764 | 28.624 | 10.774 | 1.00 | 44.55 |
| atom | 3528 | CB | PRO | 456 | | 40.160 | 28.098 | 9.480 | 1.00 | 45.22 |
| atom | 3529 | CG | PRO | 456 | | 39.053 | 29.066 | 9.194 | 1.00 | 45.06 |
| atom | 3530 | C | PRO | 456 | | 42.189 | 28.185 | 10.983 | 1.00 | 44.28 |
| atom | 3531 | O | PRO | 456 | | 42.397 | 27.354 | 11.898 | 1.00 | 44.46 |
| atom | 3532 | N | LEU | 457 | | 43.188 | 28.741 | 10.304 | 1.00 | 43.11 |
| atom | 3533 | CA | LEU | 457 | | 44.572 | 28.349 | 10.543 | 1.00 | 43.63 |
| atom | 3534 | CB | LEU | 457 | | 45.551 | 29.021 | 9.585 | 1.00 | 44.67 |
| atom | 3535 | CG | LEU | 457 | | 45.479 | 28.781 | 8.078 | 1.00 | 47.11 |
| atom | 3536 | CD1 | LEU | 457 | | 46.808 | 29.205 | 7.444 | 1.00 | 46.61 |
| atom | 3537 | CD2 | LEU | 457 | | 45.163 | 27.350 | 7.676 | 1.00 | 43.88 |
| atom | 3538 | C | LEU | 457 | | 45.024 | 28.584 | 11.982 | 1.00 | 43.53 |
| atom | 3539 | O | LEU | 457 | | 46.041 | 28.003 | 12.380 | 1.00 | 43.75 |
| atom | 3540 | N | ASP | 458 | | 44.353 | 29.408 | 12.783 | 1.00 | 42.96 |
| atom | 3541 | CA | ASP | 458 | | 44.663 | 29.687 | 14.167 | 1.00 | 42.35 |
| atom | 3542 | CB | ASP | 458 | | 44.172 | 31.069 | 14.602 | 1.00 | 44.51 |
| atom | 3543 | CG | ASP | 458 | | 44.890 | 32.253 | 14.012 | 1.00 | 46.77 |
| atom | 3544 | OD1 | ASP | 458 | | 44.274 | 33.342 | 14.026 | 1.00 | 44.23 |
| atom | 3545 | OD2 | ASP | 458 | | 46.041 | 32.104 | 13.540 | 1.00 | 50.02 |
| atom | 3546 | C | ASP | 458 | | 44.050 | 28.702 | 15.170 | 1.00 | 40.56 |
| atom | 3547 | O | ASP | 458 | | 44.234 | 28.828 | 16.382 | 1.00 | 39.59 |
| atom | 3548 | N | LEU | 459 | | 43.391 | 27.656 | 14.682 | 1.00 | 39.30 |
| atom | 3549 | CA | LEU | 459 | | 42.796 | 26.659 | 15.562 | 1.00 | 38.56 |
| atom | 3550 | CB | LEU | 459 | | 41.941 | 25.671 | 14.768 | 1.00 | 37.86 |
| atom | 3551 | CG | LEU | 459 | | 40.505 | 26.131 | 14.468 | 1.00 | 36.40 |
| atom | 3552 | CD1 | LEU | 459 | | 39.827 | 25.198 | 13.490 | 1.00 | 33.38 |
| atom | 3553 | CD2 | LEU | 459 | | 39.722 | 26.195 | 15.770 | 1.00 | 37.56 |
| atom | 3554 | C | LEU | 459 | | 43.795 | 25.999 | 16.489 | 1.00 | 37.68 |
| atom | 3555 | O | LEU | 459 | | 43.568 | 25.891 | 17.699 | 1.00 | 38.33 |
| atom | 3556 | N | PRO | 460 | | 44.935 | 25.539 | 16.006 | 1.00 | 37.27 |
| atom | 3557 | CD | PRO | 460 | | 45.316 | 25.525 | 14.567 | 1.00 | 36.60 |
| atom | 3558 | CA | PRO | 460 | | 45.941 | 24.882 | 16.839 | 1.00 | 36.11 |
| atom | 3559 | CB | PRO | 460 | | 47.120 | 24.723 | 15.892 | 1.00 | 36.11 |
| atom | 3560 | CG | PRO | 460 | | 46.477 | 24.562 | 14.547 | 1.00 | 36.19 |

| atom | 3561 | C | PRO | 460 | 46.260 | 25.678 | 18.094 | 1.00 | 35.64 |
|------|------|------|------|-----|--------|--------|--------|------|-------|
| atom | 3562 | O | PRO | 460 | 46.056 | 25.226 | 19.231 | 1.00 | 34.15 |
| atom | 3563 | N | GLN | 461 | 46.664 | 26.930 | 17.921 | 1.00 | 34.71 |
| atom | 3564 | CA | GLN | 461 | 46.981 | 27.870 | 18.981 | 1.00 | 34.49 |
| atom | 3565 | CB | GLN | 461 | 47.385 | 29.234 | 18.411 | 1.00 | 37.19 |
| atom | 3566 | CG | GLN | 461 | 48.678 | 29.289 | 17.644 | 1.00 | 42.71 |
| atom | 3567 | CD | GLN | 461 | 48.711 | 28.716 | 16.252 | 1.00 | 46.47 |
| atom | 3568 | OE1 | GLN | 461 | 47.741 | 28.233 | 15.661 | 1.00 | 47.17 |
| atom | 3569 | NE2 | GLN | 461 | 49.918 | 28.771 | 15.673 | 1.00 | 50.40 |
| atom | 3570 | C | GLN | 461 | 45.805 | 28.064 | 19.931 | 1.00 | 33.26 |
| atom | 3571 | O | GLN | 461 | 45.987 | 28.078 | 21.139 | 1.00 | 31.57 |
| atom | 3572 | N | ILE | 462 | 44.600 | 28.207 | 19.375 | 1.00 | 33.31 |
| atom | 3573 | CA | ILE | 462 | 43.408 | 28.391 | 20.184 | 1.00 | 33.76 |
| atom | 3574 | CB | ILE | 462 | 42.144 | 28.634 | 19.334 | 1.00 | 32.59 |
| atom | 3575 | CG2 | ILE | 462 | 40.905 | 28.713 | 20.233 | 1.00 | 29.28 |
| atom | 3576 | CG1 | ILE | 462 | 42.289 | 29.915 | 18.508 | 1.00 | 31.43 |
| atom | 3577 | CD1 | ILE | 462 | 41.063 | 30.303 | 17.701 | 1.00 | 29.76 |
| atom | 3578 | C | ILE | 462 | 43.176 | 27.188 | 21.096 | 1.00 | 34.55 |
| atom | 3579 | O | ILE | 462 | 42.887 | 27.342 | 22.277 | 1.00 | 34.73 |
| atom | 3580 | N | ILE | 463 | 43.286 | 25.992 | 20.540 | 1.00 | 35.09 |
| atom | 3581 | CA | ILE | 463 | 43.073 | 24.740 | 21.240 | 1.00 | 35.34 |
| atom | 3582 | CB | ILE | 463 | 43.069 | 23.538 | 20.273 | 1.00 | 35.06 |
| atom | 3583 | CG2 | ILE | 463 | 42.964 | 22.227 | 21.031 | 1.00 | 33.17 |
| atom | 3584 | CG1 | ILE | 463 | 41.877 | 23.675 | 19.312 | 1.00 | 36.76 |
| atom | 3585 | CD1 | ILE | 463 | 41.867 | 22.660 | 18.197 | 1.00 | 39.18 |
| atom | 3586 | C | ILE | 463 | 44.113 | 24.526 | 22.319 | 1.00 | 36.01 |
| atom | 3587 | O | ILE | 463 | 43.777 | 24.231 | 23.467 | 1.00 | 35.95 |
| atom | 3588 | N | GLU | 464 | 45.381 | 24.702 | 21.949 | 1.00 | 37.00 |
| atom | 3589 | CA | GLU | 464 | 46.456 | 24.568 | 22.925 | 1.00 | 37.43 |
| atom | 3590 | CB | GLU | 464 | 47.806 | 24.917 | 22.317 | 1.00 | 38.60 |
| atom | 3591 | CG | GLU | 464 | 48.920 | 24.864 | 23.360 | 1.00 | 41.71 |
| atom | 3592 | CD | GLU | 464 | 50.299 | 24.996 | 22.750 | 1.00 | 44.48 |
| atom | 3593 | OE1 | GLU | 464 | 51.268 | 24.581 | 23.420 | 1.00 | 45.93 |
| atom | 3594 | OE2 | GLU | 464 | 50.393 | 25.530 | 21.625 | 1.00 | 48.22 |
| atom | 3595 | C | GLU | 464 | 46.147 | 25.458 | 24.129 | 1.00 | 37.41 |
| atom | 3596 | O | GLU | 464 | 46.145 | 24.962 | 25.256 | 1.00 | 37.72 |

| atom | 3597 | N | ARG | 465 | 45.850 | 26.742 | 23.922 | 1.00 | 36.60 |
|---|---|---|---|---|---|---|---|---|---|
| atom | 3598 | CA | ARG | 465 | 45.519 | 27.615 | 25.039 | 1.00 | 35.75 |
| atom | 3599 | CB | ARG | 465 | 45.218 | 29.042 | 24.539 | 1.00 | 35.82 |
| atom | 3600 | CG | ARG | 465 | 44.903 | 30.014 | 25.661 | 1.00 | 36.22 |
| atom | 3601 | CD | ARG | 465 | 44.797 | 31.472 | 25.199 | 1.00 | 38.45 |
| atom | 3602 | NE | ARG | 465 | 43.394 | 31.752 | 24.826 | 1.00 | 40.96 |
| atom | 3603 | CZ | ARG | 465 | 43.053 | 31.978 | 23.572 | 1.00 | 42.01 |
| atom | 3604 | NH1 | ARG | 465 | 43.993 | 31.996 | 22.637 | 1.00 | 43.70 |
| atom | 3605 | NH2 | ARG | 465 | 41.798 | 32.204 | 23.243 | 1.00 | 45.58 |
| atom | 3606 | C | ARG | 465 | 44.336 | 27.123 | 25.872 | 1.00 | 35.17 |
| atom | 3607 | O | ARG | 465 | 44.441 | 26.992 | 27.101 | 1.00 | 33.72 |
| atom | 3608 | N | LEU | 466 | 43.187 | 26.925 | 25.223 | 1.00 | 34.52 |
| atom | 3609 | CA | LEU | 466 | 41.968 | 26.550 | 25.926 | 1.00 | 35.25 |
| atom | 3610 | CB | LEU | 466 | 40.750 | 26.496 | 25.000 | 1.00 | 35.10 |
| atom | 3611 | CG | LEU | 466 | 39.795 | 27.671 | 24.914 | 1.00 | 37.89 |
| atom | 3612 | CD1 | LEU | 466 | 40.459 | 28.994 | 25.243 | 1.00 | 38.07 |
| atom | 3613 | CD2 | LEU | 466 | 39.173 | 27.701 | 23.519 | 1.00 | 37.66 |
| atom | 3614 | C | LEU | 466 | 41.993 | 25.184 | 26.603 | 1.00 | 34.72 |
| atom | 3615 | O | LEU | 466 | 41.528 | 25.044 | 27.729 | 1.00 | 34.79 |
| atom | 3616 | N | HIS | 467 | 42.463 | 24.179 | 25.874 | 1.00 | 33.92 |
| atom | 3617 | CA | HIS | 467 | 42.414 | 22.811 | 26.347 | 1.00 | 33.47 |
| atom | 3618 | CB | HIS | 467 | 41.734 | 21.976 | 25.247 | 1.00 | 29.00 |
| atom | 3619 | CG | HIS | 467 | 40.371 | 22.489 | 24.880 | 1.00 | 28.00 |
| atom | 3620 | CD2 | HIS | 467 | 39.942 | 23.186 | 23.800 | 1.00 | 26.55 |
| atom | 3621 | ND1 | HIS | 467 | 39.276 | 22.271 | 25.690 | 1.00 | 25.73 |
| atom | 3622 | CE1 | HIS | 467 | 38.219 | 22.825 | 25.137 | 1.00 | 27.97 |
| atom | 3623 | NE2 | HIS | 467 | 38.592 | 23.383 | 24.000 | 1.00 | 29.28 |
| atom | 3624 | C | HIS | 467 | 43.760 | 22.202 | 26.691 | 1.00 | 34.43 |
| atom | 3625 | O | HIS | 467 | 43.743 | 21.148 | 27.339 | 1.00 | 35.64 |
| atom | 3626 | N | GLY | 468 | 44.878 | 22.756 | 26.249 | 1.00 | 34.10 |
| atom | 3627 | CA | GLY | 468 | 46.182 | 22.173 | 26.545 | 1.00 | 34.44 |
| atom | 3628 | C | GLY | 468 | 46.705 | 21.345 | 25.387 | 1.00 | 35.99 |
| atom | 3629 | O | GLY | 468 | 45.930 | 20.992 | 24.488 | 1.00 | 36.47 |
| atom | 3630 | N | LEU | 469 | 47.978 | 20.973 | 25.424 | 1.00 | 36.54 |
| atom | 3631 | CA | LEU | 469 | 48.594 | 20.155 | 24.394 | 1.00 | 37.94 |
| atom | 3632 | CB | LEU | 469 | 50.105 | 19.992 | 24.581 | 1.00 | 40.90 |

244

| atom | 3633 | CG | LEU | 469 | 51.000 | 20.752 | 23.593 | 1.00 | 44.36 |
|------|------|----|-----|-----|--------|--------|--------|------|-------|
| atom | 3634 | CD1 | LEU | 469 | 52.446 | 20.372 | 23.899 | 1.00 | 44.83 |
| atom | 3635 | CD2 | LEU | 469 | 50.649 | 20.480 | 22.138 | 1.00 | 45.03 |
| atom | 3636 | C | LEU | 469 | 47.972 | 18.767 | 24.289 | 1.00 | 37.18 |
| atom | 3637 | 0 | LEU | 469 | 47.933 | 18.171 | 23.211 | 1.00 | 36.97 |
| atom | 3638 | N | SER | 470 | 47.435 | 18.257 | 25.379 | 1.00 | 36.51 |
| atom | 3639 | CA | SER | 470 | 46.740 | 16.998 | 25.486 | 1.00 | 36.48 |
| atom | 3640 | CB | SER | 470 | 45.960 | 16.903 | 26.827 | 1.00 | 36.67 |
| atom | 3641 | OG | SER | 470 | 46.874 | 16.732 | 27.882 | 1.00 | 39.61 |
| atom | 3642 | C | SER | 470 | 45.649 | 16.788 | 24.451 | 1.00 | 35.35 |
| atom | 3643 | 0 | SER | 470 | 45.399 | 15.659 | 24.063 | 1.00 | 35.11 |
| atom | 3644 | N | ALA | 471 | 44.966 | 17.855 | 24.064 | 1.00 | 35.05 |
| atom | 3645 | CA | ALA | 471 | 43.858 | 17.844 | 23.136 | 1.00 | 34.63 |
| atom | 3646 | CB | ALA | 471 | 43.347 | 19.273 | 22.991 | 1.00 | 34.42 |
| atom | 3647 | C | ALA | 471 | 44.199 | 17.265 | 21.770 | 1.00 | 34.75 |
| atom | 3648 | 0 | ALA | 471 | 43.316 | 16.790 | 21.053 | 1.00 | 32.98 |
| atom | 3649 | N | PHE | 472 | 45.472 | 17.321 | 21.390 | 1.00 | 35.04 |
| atom | 3650 | CA | PHE | 472 | 45.940 | 16.786 | 20.120 | 1.00 | 35.55 |
| atom | 3651 | CB | PHE | 472 | 47.092 | 17.610 | 19.551 | 1.00 | 31.23 |
| atom | 3652 | CG | PHE | 472 | 46.816 | 19.088 | 19.501 | 1.00 | 30.69 |
| atom | 3653 | CD1 | PHE | 472 | 47.598 | 19.981 | 20.207 | 1.00 | 30.16 |
| atom | 3654 | CD2 | PHE | 472 | 45.765 | 19.599 | 18.755 | 1.00 | 31.14 |
| atom | 3655 | CE1 | PHE | 472 | 47.355 | 21.344 | 20.184 | 1.00 | 28.80 |
| atom | 3656 | CE2 | PHE | 472 | 45.497 | 20.957 | 18.714 | 1.00 | 28.67 |
| atom | 3657 | CZ | PHE | 472 | 46.292 | 21.824 | 19.437 | 1.00 | 29.04 |
| atom | 3658 | C | PHE | 472 | 46.340 | 15.314 | 20.267 | 1.00 | 36.50 |
| atom | 3659 | 0 | PHE | 472 | 46.537 | 14.686 | 19.219 | 1.00 | 35.70 |
| atom | 3660 | N | SER | 473 | 46.369 | 14.770 | 21.487 | 1.00 | 36.44 |
| atom | 3661 | CA | SER | 473 | 46.748 | 13.368 | 21.622 | 1.00 | 38.23 |
| atom | 3662 | CB | SER | 473 | 48.225 | 13.303 | 22.022 | 1.00 | 40.10 |
| atom | 3663 | OG | SER | 473 | 48.393 | 13.779 | 23.348 | 1.00 | 46.11 |
| atom | 3664 | C | SER | 473 | 45.900 | 12.529 | 22.560 | 1.00 | 38.78 |
| atom | 3665 | 0 | SER | 473 | 46.360 | 11.505 | 23.084 | 1.00 | 38.86 |
| atom | 3666 | N | LEU | 474 | 44.635 | 12.899 | 22.777 | 1.00 | 38.71 |
| atom | 3667 | CA | LEU | 474 | 43.788 | 12.081 | 23.640 | 1.00 | 38.23 |
| atom | 3668 | CB | LEU | 474 | 42.444 | 12.736 | 23.955 | 1.00 | 38.44 |

| atom | 3669 | CG  | LEU | 474 | 42.432 | 13.960 | 24.870 | 1.00 | 37.75 |
|------|------|-----|-----|-----|--------|--------|--------|------|-------|
| atom | 3670 | CD1 | LEU | 474 | 41.010 | 14.217 | 25.352 | 1.00 | 36.16 |
| atom | 3671 | CD2 | LEU | 474 | 43.409 | 13.792 | 26.021 | 1.00 | 37.63 |
| atom | 3672 | C   | LEU | 474 | 43.498 | 10.745 | 22.955 | 1.00 | 37.76 |
| atom | 3673 | O   | LEU | 474 | 43.158 | 10.727 | 21.769 | 1.00 | 37.19 |
| atom | 3674 | N   | HIS | 475 | 43.568 | 9.667  | 23.727 | 1.00 | 37.51 |
| atom | 3675 | CA  | HIS | 475 | 43.260 | 8.340  | 23.194 | 1.00 | 37.77 |
| atom | 3676 | CB  | HIS | 475 | 44.501 | 7.683  | 22.580 | 1.00 | 37.30 |
| atom | 3677 | CG  | HIS | 475 | 45.594 | 7.421  | 23.568 | 1.00 | 40.33 |
| atom | 3678 | CD2 | HIS | 475 | 46.460 | 8.285  | 24.161 | 1.00 | 40.72 |
| atom | 3679 | ND1 | HIS | 475 | 45.879 | 6.174  | 24.083 | 1.00 | 40.30 |
| atom | 3680 | CE1 | HIS | 475 | 46.875 | 6.273  | 24.939 | 1.00 | 40.48 |
| atom | 3681 | NE2 | HIS | 475 | 47.249 | 7.544  | 25.001 | 1.00 | 42.00 |
| atom | 3682 | C   | HIS | 475 | 42.630 | 7.490  | 24.284 | 1.00 | 37.42 |
| atom | 3683 | O   | HIS | 475 | 42.581 | 7.918  | 25.434 | 1.00 | 38.45 |
| atom | 3684 | N   | SER | 476 | 42.142 | 6.298  | 23.967 | 1.00 | 37.76 |
| atom | 3685 | CA  | SER | 476 | 41.491 | 5.438  | 24.959 | 1.00 | 37.80 |
| atom | 3686 | CB  | SER | 476 | 42.433 | 5.097  | 26.116 | 1.00 | 40.05 |
| atom | 3687 | OG  | SER | 476 | 43.482 | 4.263  | 25.659 | 1.00 | 43.86 |
| atom | 3688 | C   | SER | 476 | 40.241 | 6.126  | 25.503 | 1.00 | 36.36 |
| atom | 3689 | O   | SER | 476 | 40.149 | 6.431  | 26.683 | 1.00 | 36.19 |
| atom | 3690 | N   | TYR | 477 | 39.312 | 6.451  | 24.605 | 1.00 | 36.30 |
| atom | 3691 | CA  | TYR | 477 | 38.109 | 7.144  | 25.090 | 1.00 | 35.98 |
| atom | 3692 | CB  | TYR | 477 | 37.364 | 7.774  | 23.938 | 1.00 | 36.13 |
| atom | 3693 | CG  | TYR | 477 | 38.026 | 9.024  | 23.395 | 1.00 | 36.38 |
| atom | 3694 | CD1 | TYR | 477 | 38.850 | 8.974  | 22.272 | 1.00 | 36.65 |
| atom | 3695 | CE1 | TYR | 477 | 39.446 | 10.116 | 21.765 | 1.00 | 35.66 |
| atom | 3696 | CD2 | TYR | 477 | 37.813 | 10.253 | 24.001 | 1.00 | 35.64 |
| atom | 3697 | CE2 | TYR | 477 | 38.398 | 11.407 | 23.508 | 1.00 | 35.23 |
| atom | 3698 | CZ  | TYR | 477 | 39.211 | 11.329 | 22.390 | 1.00 | 35.78 |
| atom | 3699 | OH  | TYR | 477 | 39.781 | 12.464 | 21.868 | 1.00 | 34.59 |
| atom | 3700 | C   | TYR | 477 | 37.292 | 6.187  | 25.943 | 1.00 | 35.06 |
| atom | 3701 | O   | TYR | 477 | 37.581 | 4.987  | 25.951 | 1.00 | 35.38 |
| atom | 3702 | N   | SER | 478 | 36.359 | 6.709  | 26.727 | 1.00 | 33.69 |
| atom | 3703 | CA  | SER | 478 | 35.592 | 5.819  | 27.592 | 1.00 | 34.09 |
| atom | 3704 | CB  | SER | 478 | 34.759 | 6.606  | 28.601 | 1.00 | 31.64 |

| atom | 3705 | OG | SER | 478 | 33.497 | 6.926 | 28.014 | 1.00 | 32.51 |
| atom | 3706 | C | SER | 478 | 34.660 | 4.977 | 26.725 | 1.00 | 34.36 |
| atom | 3707 | O | SER | 478 | 34.236 | 5.436 | 25.661 | 1.00 | 35.61 |
| atom | 3708 | N | PRO | 479 | 34.237 | 3.831 | 27.223 | 1.00 | 33.74 |
| atom | 3709 | CD | PRO | 479 | 34.701 | 3.244 | 28.497 | 1.00 | 32.80 |
| atom | 3710 | CA | PRO | 479 | 33.288 | 2.991 | 26.508 | 1.00 | 33.95 |
| atom | 3711 | CB | PRO | 479 | 33.119 | 1.768 | 27.408 | 1.00 | 34.67 |
| atom | 3712 | CG | PRO | 479 | 34.177 | 1.836 | 28.453 | 1.00 | 33.60 |
| atom | 3713 | C | PRO | 479 | 31.956 | 3.688 | 26.292 | 1.00 | 33.96 |
| atom | 3714 | O | PRO | 479 | 31.253 | 3.562 | 25.290 | 1.00 | 33.03 |
| atom | 3715 | N | GLY | 480 | 31.552 | 4.482 | 27.289 | 1.00 | 34.92 |
| atom | 3716 | CA | GLY | 480 | 30.307 | 5.242 | 27.212 | 1.00 | 35.23 |
| atom | 3717 | C | GLY | 480 | 30.400 | 6.363 | 26.186 | 1.00 | 35.24 |
| atom | 3718 | O | GLY | 480 | 29.379 | 6.709 | 25.586 | 1.00 | 35.04 |
| atom | 3719 | N | GLU | 481 | 31.585 | 6.952 | 26.001 | 1.00 | 35.30 |
| atom | 3720 | CA | GLU | 481 | 31.690 | 8.030 | 25.019 | 1.00 | 35.54 |
| atom | 3721 | CB | GLU | 481 | 32.972 | 8.831 | 25.215 | 1.00 | 35.88 |
| atom | 3722 | CG | GLU | 481 | 33.268 | 9.885 | 24.163 | 1.00 | 34.94 |
| atom | 3723 | CD | GLU | 481 | 32.232 | 11.007 | 24.180 | 1.00 | 34.98 |
| atom | 3724 | OE1 | GLU | 481 | 31.452 | 11.065 | 25.160 | 1.00 | 32.21 |
| atom | 3725 | OE2 | GLU | 481 | 32.225 | 11.777 | 23.200 | 1.00 | 32.43 |
| atom | 3726 | C | GLU | 481 | 31.614 | 7.461 | 23.605 | 1.00 | 35.55 |
| atom | 3727 | O | GLU | 481 | 30.925 | 8.051 | 22.781 | 1.00 | 34.27 |
| atom | 3728 | N | ILE | 482 | 32.317 | 6.353 | 23.374 | 1.00 | 36.28 |
| atom | 3729 | CA | ILE | 482 | 32.407 | 5.709 | 22.085 | 1.00 | 37.35 |
| atom | 3730 | CB | ILE | 482 | 33.419 | 4.549 | 21.948 | 1.00 | 37.89 |
| atom | 3731 | CG2 | ILE | 482 | 33.741 | 4.426 | 20.453 | 1.00 | 36.99 |
| atom | 3732 | CG1 | ILE | 482 | 34.658 | 4.693 | 22.794 | 1.00 | 38.17 |
| atom | 3733 | CD1 | ILE | 482 | 36.025 | 4.800 | 22.191 | 1.00 | 38.99 |
| atom | 3734 | C | ILE | 482 | 31.079 | 5.097 | 21.637 | 1.00 | 37.71 |
| atom | 3735 | O | ILE | 482 | 30.740 | 5.217 | 20.454 | 1.00 | 38.60 |
| atom | 3736 | N | ASN | 483 | 30.367 | 4.460 | 22.556 | 1.00 | 37.73 |
| atom | 3737 | CA | ASN | 483 | 29.071 | 3.897 | 22.197 | 1.00 | 39.00 |
| atom | 3738 | CB | ASN | 483 | 28.424 | 3.106 | 23.334 | 1.00 | 41.02 |
| atom | 3739 | CG | ASN | 483 | 29.210 | 1.827 | 23.571 | 1.00 | 45.51 |
| atom | 3740 | OD1 | ASN | 483 | 30.096 | 1.472 | 22.784 | 1.00 | 46.90 |

| atom | 3741 | ND2 | ASN | 483 | 28.889 | 1.132 | 24.661 | 1.00 | 46.77 |
|------|------|-----|-----|-----|--------|-------|--------|------|-------|
| atom | 3742 | C   | ASN | 483 | 28.147 | 5.043 | 21.784 | 1.00 | 39.05 |
| atom | 3743 | O   | ASN | 483 | 27.505 | 4.994 | 20.735 | 1.00 | 38.87 |
| atom | 3744 | N   | ARG | 484 | 28.155 | 6.095 | 22.613 | 1.00 | 38.40 |
| atom | 3745 | CA  | ARG | 484 | 27.349 | 7.255 | 22.278 | 1.00 | 38.84 |
| atom | 3746 | CB  | ARG | 484 | 27.479 | 8.344 | 23.343 | 1.00 | 40.82 |
| atom | 3747 | CG  | ARG | 484 | 26.799 | 9.614 | 22.844 | 1.00 | 42.50 |
| atom | 3748 | CD  | ARG | 484 | 25.959 | 10.288 | 23.876 | 1.00 | 42.60 |
| atom | 3749 | NE  | ARG | 484 | 26.583 | 11.008 | 24.952 | 1.00 | 42.33 |
| atom | 3750 | CZ  | ARG | 484 | 27.701 | 11.690 | 25.012 | 1.00 | 41.12 |
| atom | 3751 | NH1 | ARG | 484 | 28.509 | 11.781 | 23.950 | 1.00 | 43.84 |
| atom | 3752 | NH2 | ARG | 484 | 28.032 | 12.297 | 26.138 | 1.00 | 37.04 |
| atom | 3753 | C   | ARG | 484 | 27.686 | 7.803 | 20.897 | 1.00 | 38.60 |
| atom | 3754 | O   | ARG | 484 | 26.789 | 8.029 | 20.078 | 1.00 | 38.46 |
| atom | 3755 | N   | VAL | 485 | 28.969 | 7.996 | 20.612 | 1.00 | 38.59 |
| atom | 3756 | CA  | VAL | 485 | 29.357 | 8.502 | 19.283 | 1.00 | 38.77 |
| atom | 3757 | CB  | VAL | 485 | 30.857 | 8.807 | 19.242 | 1.00 | 36.16 |
| atom | 3758 | CG1 | VAL | 485 | 31.389 | 9.192 | 17.864 | 1.00 | 34.71 |
| atom | 3759 | CG2 | VAL | 485 | 31.158 | 9.959 | 20.206 | 1.00 | 31.73 |
| atom | 3760 | C   | VAL | 485 | 28.887 | 7.519 | 18.215 | 1.00 | 39.78 |
| atom | 3761 | O   | VAL | 485 | 28.219 | 7.862 | 17.233 | 1.00 | 38.77 |
| atom | 3762 | N   | ALA | 486 | 29.167 | 6.235 | 18.461 | 1.00 | 40.74 |
| atom | 3763 | CA  | ALA | 486 | 28.772 | 5.182 | 17.535 | 1.00 | 41.87 |
| atom | 3764 | CB  | ALA | 486 | 29.312 | 3.827 | 17.986 | 1.00 | 42.62 |
| atom | 3765 | C   | ALA | 486 | 27.276 | 5.134 | 17.270 | 1.00 | 41.60 |
| atom | 3766 | O   | ALA | 486 | 26.905 | 5.147 | 16.084 | 1.00 | 41.71 |
| atom | 3767 | N   | SER | 487 | 26.399 | 5.115 | 18.258 | 1.00 | 41.77 |
| atom | 3768 | CA  | SER | 487 | 24.967 | 5.107 | 17.968 | 1.00 | 43.22 |
| atom | 3769 | CB  | SER | 487 | 24.135 | 5.074 | 19.245 | 1.00 | 42.82 |
| atom | 3770 | OG  | SER | 487 | 24.545 | 3.971 | 20.033 | 1.00 | 46.12 |
| atom | 3771 | C   | SER | 487 | 24.540 | 6.329 | 17.158 | 1.00 | 43.66 |
| atom | 3772 | O   | SER | 487 | 23.755 | 6.239 | 16.215 | 1.00 | 43.73 |
| atom | 3773 | N   | CYS | 488 | 25.054 | 7.497 | 17.540 | 1.00 | 44.01 |
| atom | 3774 | CA  | CYS | 488 | 24.702 | 8.721 | 16.835 | 1.00 | 44.25 |
| atom | 3775 | CB  | CYS | 488 | 25.309 | 9.946 | 17.528 | 1.00 | 43.28 |
| atom | 3776 | SG  | CYS | 488 | 25.270 | 11.403 | 16.453 | 1.00 | 42.10 |

| atom | 3777 | C | CYS | 488 | 25.052 | 8.675 | 15.359 | 1.00 | 44.54 |
| atom | 3778 | O | CYS | 488 | 24.179 | 9.014 | 14.550 | 1.00 | 44.72 |
| atom | 3779 | N | LEU | 489 | 26.225 | 8.237 | 14.917 | 1.00 | 45.01 |
| atom | 3780 | CA | LEU | 489 | 26.509 | 8.191 | 13.476 | 1.00 | 46.07 |
| atom | 3781 | CB | LEU | 489 | 27.963 | 7.817 | 13.202 | 1.00 | 43.99 |
| atom | 3782 | CG | LEU | 489 | 29.051 | 8.405 | 14.094 | 1.00 | 43.82 |
| atom | 3783 | CD1 | LEU | 489 | 30.450 | 8.039 | 13.615 | 1.00 | 43.66 |
| atom | 3784 | CD2 | LEU | 489 | 28.942 | 9.919 | 14.209 | 1.00 | 44.41 |
| atom | 3785 | C | LEU | 489 | 25.540 | 7.276 | 12.722 | 1.00 | 46.87 |
| atom | 3786 | O | LEU | 489 | 25.108 | 7.508 | 11.584 | 1.00 | 46.37 |
| atom | 3787 | N | ARG | 490 | 25.124 | 6.175 | 13.334 | 1.00 | 47.14 |
| atom | 3788 | CA | ARG | 490 | 24.172 | 5.262 | 12.731 | 1.00 | 48.42 |
| atom | 3789 | CB | ARG | 490 | 24.184 | 3.965 | 13.499 | 1.00 | 53.51 |
| atom | 3790 | CG | ARG | 490 | 23.083 | 2.962 | 13.264 | 1.00 | 58.98 |
| atom | 3791 | CD | ARG | 490 | 23.493 | 1.566 | 13.716 | 1.00 | 63.38 |
| atom | 3792 | NE | ARG | 490 | 24.510 | 1.577 | 14.766 | 1.00 | 66.17 |
| atom | 3793 | CZ | ARG | 490 | 25.824 | 1.613 | 14.539 | 1.00 | 67.77 |
| atom | 3794 | NH1 | ARG | 490 | 26.251 | 1.655 | 13.276 | 1.00 | 69.19 |
| atom | 3795 | NH2 | ARG | 490 | 26.670 | 1.644 | 15.555 | 1.00 | 66.89 |
| atom | 3796 | C | ARG | 490 | 22.758 | 5.835 | 12.727 | 1.00 | 48.18 |
| atom | 3797 | O | ARG | 490 | 21.978 | 5.570 | 11.815 | 1.00 | 47.75 |
| atom | 3798 | N | LYS | 491 | 22.418 | 6.589 | 13.769 | 1.00 | 47.22 |
| atom | 3799 | CA | LYS | 491 | 21.102 | 7.205 | 13.839 | 1.00 | 46.99 |
| atom | 3800 | CB | LYS | 491 | 20.900 | 7.863 | 15.201 | 1.00 | 45.67 |
| atom | 3801 | CG | LYS | 491 | 19.700 | 8.772 | 15.298 | 1.00 | 45.31 |
| atom | 3802 | CD | LYS | 491 | 19.565 | 9.410 | 16.666 | 1.00 | 45.09 |
| atom | 3803 | CE | LYS | 491 | 20.473 | 10.619 | 16.855 | 1.00 | 43.15 |
| atom | 3804 | NZ | LYS | 491 | 20.243 | 11.164 | 18.222 | 1.00 | 43.58 |
| atom | 3805 | C | LYS | 491 | 20.932 | 8.205 | 12.702 | 1.00 | 47.27 |
| atom | 3806 | O | LYS | 491 | 19.893 | 8.205 | 12.042 | 1.00 | 47.59 |
| atom | 3807 | N | LEU | 492 | 21.935 | 9.037 | 12.468 | 1.00 | 47.21 |
| atom | 3808 | CA | LEU | 492 | 21.923 | 10.087 | 11.474 | 1.00 | 47.23 |
| atom | 3809 | CB | LEU | 492 | 22.829 | 11.240 | 11.946 | 1.00 | 46.23 |
| atom | 3810 | CG | LEU | 492 | 22.352 | 12.072 | 13.133 | 1.00 | 45.72 |
| atom | 3811 | CD1 | LEU | 492 | 23.450 | 13.020 | 13.593 | 1.00 | 43.60 |
| atom | 3812 | CD2 | LEU | 492 | 21.109 | 12.865 | 12.739 | 1.00 | 45.44 |

| atom | 3813 | C | LEU | 492 | 22.431 | 9.666 | 10.104 | 1.00 | 47.38 |
|------|------|-----|-----|-----|--------|--------|--------|------|-------|
| atom | 3814 | O | LEU | 492 | 22.251 | 10.381 | 9.114 | 1.00 | 46.71 |
| atom | 3815 | N | GLY | 493 | 23.056 | 8.491 | 10.036 | 1.00 | 47.59 |
| atom | 3816 | CA | GLY | 493 | 23.591 | 8.012 | 8.768 | 1.00 | 47.06 |
| atom | 3817 | C | GLY | 493 | 24.921 | 8.674 | 8.439 | 1.00 | 46.76 |
| atom | 3818 | O | GLY | 493 | 25.206 | 8.964 | 7.280 | 1.00 | 46.08 |
| atom | 3819 | N | VAL | 494 | 25.707 | 8.961 | 9.474 | 1.00 | 47.09 |
| atom | 3820 | CA | VAL | 494 | 27.032 | 9.556 | 9.293 | 1.00 | 47.18 |
| atom | 3821 | CB | VAL | 494 | 27.495 | 10.370 | 10.507 | 1.00 | 46.34 |
| atom | 3822 | CG1 | VAL | 494 | 28.997 | 10.648 | 10.470 | 1.00 | 43.87 |
| atom | 3823 | CG2 | VAL | 494 | 26.735 | 11.688 | 10.581 | 1.00 | 47.90 |
| atom | 3824 | C | VAL | 494 | 28.000 | 8.389 | 9.091 | 1.00 | 47.52 |
| atom | 3825 | O | VAL | 494 | 27.932 | 7.432 | 9.858 | 1.00 | 47.68 |
| atom | 3826 | N | PRO | 495 | 28.880 | 8.477 | 8.114 | 1.00 | 47.74 |
| atom | 3827 | CD | PRO | 495 | 28.995 | 9.604 | 7.155 | 1.00 | 47.61 |
| atom | 3828 | CA | PRO | 495 | 29.851 | 7.433 | 7.841 | 1.00 | 47.62 |
| atom | 3829 | CB | PRO | 495 | 30.759 | 8.054 | 6.785 | 1.00 | 47.97 |
| atom | 3830 | CG | PRO | 495 | 29.904 | 9.053 | 6.086 | 1.00 | 47.82 |
| atom | 3831 | C | PRO | 495 | 30.625 | 7.022 | 9.082 | 1.00 | 47.21 |
| atom | 3832 | O | PRO | 495 | 30.830 | 7.827 | 9.980 | 1.00 | 46.61 |
| atom | 3833 | N | PRO | 496 | 31.029 | 5.759 | 9.143 | 1.00 | 47.01 |
| atom | 3834 | CD | PRO | 496 | 30.776 | 4.743 | 8.089 | 1.00 | 47.33 |
| atom | 3835 | CA | PRO | 496 | 31.734 | 5.200 | 10.273 | 1.00 | 46.21 |
| atom | 3836 | CB | PRO | 496 | 31.851 | 3.711 | 9.978 | 1.00 | 46.28 |
| atom | 3837 | CG | PRO | 496 | 31.428 | 3.486 | 8.586 | 1.00 | 46.45 |
| atom | 3838 | C | PRO | 496 | 33.069 | 5.857 | 10.527 | 1.00 | 45.68 |
| atom | 3839 | O | PRO | 496 | 33.679 | 6.448 | 9.638 | 1.00 | 45.35 |
| atom | 3840 | N | LEU | 497 | 33.566 | 5.742 | 11.760 | 1.00 | 45.61 |
| atom | 3841 | CA | LEU | 497 | 34.830 | 6.381 | 12.131 | 1.00 | 46.17 |
| atom | 3842 | CB | LEU | 497 | 35.158 | 6.121 | 13.606 | 1.00 | 43.27 |
| atom | 3843 | CG | LEU | 497 | 34.225 | 6.791 | 14.622 | 1.00 | 43.92 |
| atom | 3844 | CD1 | LEU | 497 | 34.596 | 6.429 | 16.050 | 1.00 | 41.06 |
| atom | 3845 | CD2 | LEU | 497 | 34.170 | 8.310 | 14.444 | 1.00 | 42.48 |
| atom | 3846 | C | LEU | 497 | 35.972 | 6.025 | 11.200 | 1.00 | 46.46 |
| atom | 3847 | O | LEU | 497 | 36.692 | 6.901 | 10.708 | 1.00 | 45.97 |
| atom | 3848 | N | ARG | 498 | 36.124 | 4.755 | 10.843 | 1.00 | 47.30 |

| atom | 3849 | CA | ARG | 498 | 37.144 | 4.272 | 9.929 | 1.00 48.11 |
|------|------|-----|-----|-----|--------|-------|-------|------------|
| atom | 3850 | CB | ARG | 498 | 36.992 | 2.785 | 9.584 | 1.00 51.88 |
| atom | 3851 | CG | ARG | 498 | 35.717 | 2.430 | 8.839 | 1.00 57.02 |
| atom | 3852 | CD | ARG | 498 | 35.984 | 1.523 | 7.642 | 1.00 61.13 |
| atom | 3853 | NE | ARG | 498 | 34.954 | 1.650 | 6.611 | 1.00 64.83 |
| atom | 3854 | CZ | ARG | 498 | 35.181 | 1.655 | 5.300 | 1.00 66.68 |
| atom | 3855 | NH1 | ARG | 498 | 36.430 | 1.545 | 4.847 | 1.00 67.69 |
| atom | 3856 | NH2 | ARG | 498 | 34.177 | 1.779 | 4.438 | 1.00 66.19 |
| atom | 3857 | C | ARG | 498 | 37.183 | 5.102 | 8.652 | 1.00 47.74 |
| atom | 3858 | O | ARG | 498 | 38.256 | 5.436 | 8.156 | 1.00 46.89 |
| atom | 3859 | N | VAL | 499 | 36.030 | 5.476 | 8.109 | 1.00 48.10 |
| atom | 3860 | CA | VAL | 499 | 35.943 | 6.324 | 6.935 | 1.00 48.56 |
| atom | 3861 | CB | VAL | 499 | 34.507 | 6.462 | 6.409 | 1.00 47.28 |
| atom | 3862 | CG1 | VAL | 499 | 34.405 | 7.452 | 5.256 | 1.00 46.95 |
| atom | 3863 | CG2 | VAL | 499 | 33.988 | 5.108 | 5.948 | 1.00 48.25 |
| atom | 3864 | C | VAL | 499 | 36.501 | 7.712 | 7.248 | 1.00 49.53 |
| atom | 3865 | O | VAL | 499 | 37.173 | 8.297 | 6.401 | 1.00 49.62 |
| atom | 3866 | N | TRP | 500 | 36.247 | 8.228 | 8.444 | 1.00 49.80 |
| atom | 3867 | CA | TRP | 500 | 36.757 | 9.534 | 8.836 | 1.00 50.62 |
| atom | 3868 | CB | TRP | 500 | 36.070 | 10.018 | 10.119 | 1.00 46.52 |
| atom | 3869 | CG | TRP | 500 | 34.621 | 10.275 | 9.788 | 1.00 43.97 |
| atom | 3870 | CD2 | TRP | 500 | 34.146 | 11.353 | 8.973 | 1.00 42.23 |
| atom | 3871 | CE2 | TRP | 500 | 32.750 | 11.220 | 8.895 | 1.00 41.89 |
| atom | 3872 | CE3 | TRP | 500 | 34.767 | 12.408 | 8.300 | 1.00 40.14 |
| atom | 3873 | CD1 | TRP | 500 | 33.530 | 9.551 | 10.154 | 1.00 42.81 |
| atom | 3874 | NE1 | TRP | 500 | 32.402 | 10.116 | 9.628 | 1.00 41.50 |
| atom | 3875 | CZ2 | TRP | 500 | 31.956 | 12.118 | 8.176 | 1.00 40.95 |
| atom | 3876 | CZ3 | TRP | 500 | 33.983 | 13.285 | 7.588 | 1.00 39.46 |
| atom | 3877 | CH2 | TRP | 500 | 32.593 | 13.139 | 7.545 | 1.00 40.26 |
| atom | 3878 | C | TRP | 500 | 38.269 | 9.505 | 8.995 | 1.00 52.27 |
| atom | 3879 | O | TRP | 500 | 38.946 | 10.476 | 8.651 | 1.00 52.08 |
| atom | 3880 | N | ARG | 501 | 38.791 | 8.383 | 9.481 | 1.00 53.66 |
| atom | 3881 | CA | ARG | 501 | 40.240 | 8.261 | 9.630 | 1.00 56.13 |
| atom | 3882 | CB | ARG | 501 | 40.612 | 7.000 | 10.394 | 1.00 58.21 |
| atom | 3883 | CG | ARG | 501 | 42.084 | 6.671 | 10.455 | 1.00 61.79 |
| atom | 3884 | CD | ARG | 501 | 42.450 | 5.602 | 11.473 | 1.00 65.38 |

| atom | 3885 | NE | ARG | 501 | 43.021 | 6.144 | 12.693 | 1.00 | 68.34 |
|------|------|-----|-----|-----|--------|--------|--------|------|-------|
| atom | 3886 | CZ | ARG | 501 | 42.416 | 6.417 | 13.836 | 1.00 | 70.57 |
| atom | 3887 | NH1 | ARG | 501 | 41.125 | 6.192 | 14.025 | 1.00 | 70.97 |
| atom | 3888 | NH2 | ARG | 501 | 43.117 | 6.920 | 14.850 | 1.00 | 73.20 |
| atom | 3889 | C | ARG | 501 | 40.836 | 8.332 | 8.229 | 1.00 | 57.06 |
| atom | 3890 | O | ARG | 501 | 41.706 | 9.160 | 7.959 | 1.00 | 57.05 |
| atom | 3891 | N | HIS | 502 | 40.322 | 7.513 | 7.316 | 1.00 | 57.67 |
| atom | 3892 | CA | HIS | 502 | 40.816 | 7.509 | 5.943 | 1.00 | 58.89 |
| atom | 3893 | CB | HIS | 502 | 40.032 | 6.512 | 5.088 | 1.00 | 63.64 |
| atom | 3894 | CG | HIS | 502 | 40.363 | 6.606 | 3.628 | 1.00 | 68.47 |
| atom | 3895 | CD2 | HIS | 502 | 39.762 | 7.306 | 2.630 | 1.00 | 70.37 |
| atom | 3896 | ND1 | HIS | 502 | 41.428 | 5.947 | 3.054 | 1.00 | 70.00 |
| atom | 3897 | CE1 | HIS | 502 | 41.470 | 6.229 | 1.765 | 1.00 | 70.99 |
| atom | 3898 | NE2 | HIS | 502 | 40.474 | 7.054 | 1.482 | 1.00 | 71.58 |
| atom | 3899 | C | HIS | 502 | 40.787 | 8.921 | 5.367 | 1.00 | 58.08 |
| atom | 3900 | O | HIS | 502 | 41.803 | 9.460 | 4.938 | 1.00 | 56.95 |
| atom | 3901 | N | ARG | 503 | 39.622 | 9.565 | 5.385 | 1.00 | 57.67 |
| atom | 3902 | CA | ARG | 503 | 39.459 | 10.931 | 4.914 | 1.00 | 56.62 |
| atom | 3903 | CB | ARG | 503 | 38.009 | 11.378 | 5.117 | 1.00 | 56.48 |
| atom | 3904 | CG | ARG | 503 | 37.007 | 10.605 | 4.275 | 1.00 | 55.58 |
| atom | 3905 | CD | ARG | 503 | 35.597 | 10.718 | 4.821 | 1.00 | 55.36 |
| atom | 3906 | NE | ARG | 503 | 34.585 | 10.491 | 3.800 | 1.00 | 54.08 |
| atom | 3907 | CZ | ARG | 503 | 33.308 | 10.839 | 3.881 | 1.00 | 52.72 |
| atom | 3908 | NH1 | ARG | 503 | 32.800 | 11.448 | 4.942 | 1.00 | 49.23 |
| atom | 3909 | NH2 | ARG | 503 | 32.512 | 10.563 | 2.852 | 1.00 | 52.91 |
| atom | 3910 | C | ARG | 503 | 40.436 | 11.880 | 5.600 | 1.00 | 56.16 |
| atom | 3911 | O | ARG | 503 | 41.060 | 12.705 | 4.935 | 1.00 | 55.66 |
| atom | 3912 | N | ALA | 504 | 40.646 | 11.777 | 6.906 | 1.00 | 55.30 |
| atom | 3913 | CA | ALA | 504 | 41.583 | 12.615 | 7.629 | 1.00 | 55.07 |
| atom | 3914 | CB | ALA | 504 | 41.447 | 12.413 | 9.129 | 1.00 | 53.69 |
| atom | 3915 | C | ALA | 504 | 43.030 | 12.413 | 7.213 | 1.00 | 54.86 |
| atom | 3916 | O | ALA | 504 | 43.788 | 13.389 | 7.177 | 1.00 | 54.46 |
| atom | 3917 | N | ARG | 505 | 43.461 | 11.210 | 6.857 | 1.00 | 55.53 |
| atom | 3918 | CA | ARG | 505 | 44.845 | 10.991 | 6.411 | 1.00 | 55.59 |
| atom | 3919 | CB | ARG | 505 | 45.140 | 9.501 | 6.258 | 1.00 | 59.45 |
| atom | 3920 | CG | ARG | 505 | 45.789 | 8.844 | 7.463 | 1.00 | 64.44 |

| atom | 3921 | CD | ARG | 505 | 45.470 | 7.366 | 7.599 | 1.00 | 69.50 |
|------|------|-----|-----|-----|--------|--------|--------|------|-------|
| atom | 3922 | NE | ARG | 505 | 44.829 | 6.824 | 6.412 | 1.00 | 74.47 |
| atom | 3923 | CZ | ARG | 505 | 45.045 | 5.670 | 5.799 | 1.00 | 76.94 |
| atom | 3924 | NH1 | ARG | 505 | 45.960 | 4.813 | 6.243 | 1.00 | 77.57 |
| atom | 3925 | NH2 | ARG | 505 | 44.347 | 5.361 | 4.706 | 1.00 | 77.43 |
| atom | 3926 | C | ARG | 505 | 45.154 | 11.775 | 5.135 | 1.00 | 54.32 |
| atom | 3927 | O | ARG | 505 | 46.243 | 12.330 | 4.966 | 1.00 | 53.78 |
| atom | 3928 | N | SER | 506 | 44.185 | 11.892 | 4.239 | 1.00 | 52.72 |
| atom | 3929 | CA | SER | 506 | 44.317 | 12.647 | 3.005 | 1.00 | 51.71 |
| atom | 3930 | CB | SER | 506 | 43.260 | 12.149 | 2.024 | 1.00 | 50.67 |
| atom | 3931 | OG | SER | 506 | 43.249 | 12.927 | 0.853 | 1.00 | 52.16 |
| atom | 3932 | C | SER | 506 | 44.252 | 14.145 | 3.248 | 1.00 | 50.97 |
| atom | 3933 | O | SER | 506 | 45.169 | 14.860 | 2.829 | 1.00 | 51.59 |
| atom | 3934 | N | VAL | 507 | 43.286 | 14.664 | 3.994 | 1.00 | 49.34 |
| atom | 3935 | CA | VAL | 507 | 43.197 | 16.061 | 4.379 | 1.00 | 47.12 |
| atom | 3936 | CB | VAL | 507 | 42.101 | 16.260 | 5.449 | 1.00 | 44.99 |
| atom | 3937 | CG1 | VAL | 507 | 42.175 | 17.622 | 6.114 | 1.00 | 43.28 |
| atom | 3938 | CG2 | VAL | 507 | 40.716 | 16.067 | 4.852 | 1.00 | 44.53 |
| atom | 3939 | C | VAL | 507 | 44.520 | 16.553 | 4.972 | 1.00 | 46.44 |
| atom | 3940 | O | VAL | 507 | 45.001 | 17.653 | 4.720 | 1.00 | 45.27 |
| atom | 3941 | N | ARG | 508 | 45.104 | 15.733 | 5.839 | 1.00 | 46.29 |
| atom | 3942 | CA | ARG | 508 | 46.356 | 16.042 | 6.509 | 1.00 | 46.63 |
| atom | 3943 | CB | ARG | 508 | 46.661 | 14.958 | 7.550 | 1.00 | 43.64 |
| atom | 3944 | CG | ARG | 508 | 47.975 | 15.196 | 8.275 | 1.00 | 42.76 |
| atom | 3945 | CD | ARG | 508 | 48.134 | 14.200 | 9.417 | 1.00 | 45.00 |
| atom | 3946 | NE | ARG | 508 | 49.463 | 13.657 | 9.418 | 1.00 | 48.70 |
| atom | 3947 | CZ | ARG | 508 | 50.558 | 13.872 | 10.093 | 1.00 | 51.92 |
| atom | 3948 | NH1 | ARG | 508 | 50.587 | 14.752 | 11.090 | 1.00 | 54.71 |
| atom | 3949 | NH2 | ARG | 508 | 51.661 | 13.180 | 9.803 | 1.00 | 53.07 |
| atom | 3950 | C | ARG | 508 | 47.516 | 16.223 | 5.538 | 1.00 | 46.99 |
| atom | 3951 | O | ARG | 508 | 48.231 | 17.220 | 5.561 | 1.00 | 46.16 |
| atom | 3952 | N | ALA | 509 | 47.700 | 15.254 | 4.649 | 1.00 | 48.12 |
| atom | 3953 | CA | ALA | 509 | 48.721 | 15.312 | 3.612 | 1.00 | 49.64 |
| atom | 3954 | CB | ALA | 509 | 48.592 | 14.137 | 2.643 | 1.00 | 47.77 |
| atom | 3955 | C | ALA | 509 | 48.617 | 16.638 | 2.856 | 1.00 | 50.08 |
| atom | 3956 | O | ALA | 509 | 49.618 | 17.344 | 2.716 | 1.00 | 50.22 |

| atom | 3957 | N   | ARG | 510 | 47.427 | 16.984 | 2.380  | 1.00 | 50.74 |
|------|------|-----|-----|-----|--------|--------|--------|------|-------|
| atom | 3958 | CA  | ARG | 510 | 47.231 | 18.256 | 1.697  | 1.00 | 53.26 |
| atom | 3959 | CB  | ARG | 510 | 45.753 | 18.485 | 1.424  | 1.00 | 55.46 |
| atom | 3960 | CG  | ARG | 510 | 45.003 | 17.367 | 0.725  | 1.00 | 58.22 |
| atom | 3961 | CD  | ARG | 510 | 44.875 | 17.643 | -0.766 | 1.00 | 59.92 |
| atom | 3962 | NE  | ARG | 510 | 44.393 | 18.999 | -1.005 | 1.00 | 62.36 |
| atom | 3963 | CZ  | ARG | 510 | 44.039 | 19.515 | -2.177 | 1.00 | 63.88 |
| atom | 3964 | NH1 | ARG | 510 | 44.099 | 18.782 | -3.288 | 1.00 | 65.18 |
| atom | 3965 | NH2 | ARG | 510 | 43.619 | 20.775 | -2.257 | 1.00 | 62.61 |
| atom | 3966 | C   | ARG | 510 | 47.757 | 19.384 | 2.583  | 1.00 | 54.41 |
| atom | 3967 | O   | ARG | 510 | 48.672 | 20.119 | 2.231  | 1.00 | 54.05 |
| atom | 3968 | N   | LEU | 511 | 47.189 | 19.482 | 3.787  | 1.00 | 55.60 |
| atom | 3969 | CA  | LEU | 511 | 47.557 | 20.485 | 4.769  | 1.00 | 56.78 |
| atom | 3970 | CB  | LEU | 511 | 46.945 | 20.190 | 6.143  | 1.00 | 57.30 |
| atom | 3971 | CG  | LEU | 511 | 45.459 | 20.494 | 6.331  | 1.00 | 56.59 |
| atom | 3972 | CD1 | LEU | 511 | 44.974 | 20.025 | 7.691  | 1.00 | 55.28 |
| atom | 3973 | CD2 | LEU | 511 | 45.200 | 21.984 | 6.134  | 1.00 | 56.40 |
| atom | 3974 | C   | LEU | 511 | 49.068 | 20.581 | 4.920  | 1.00 | 57.48 |
| atom | 3975 | O   | LEU | 511 | 49.639 | 21.665 | 4.790  | 1.00 | 57.14 |
| atom | 3976 | N   | LEU | 512 | 49.708 | 19.443 | 5.159  | 1.00 | 57.98 |
| atom | 3977 | CA  | LEU | 512 | 51.162 | 19.414 | 5.320  | 1.00 | 59.96 |
| atom | 3978 | CB  | LEU | 512 | 51.590 | 18.003 | 5.710  | 1.00 | 58.43 |
| atom | 3979 | CG  | LEU | 512 | 52.446 | 17.788 | 6.952  | 1.00 | 57.85 |
| atom | 3980 | CD1 | LEU | 512 | 52.026 | 18.673 | 8.115  | 1.00 | 57.57 |
| atom | 3981 | CD2 | LEU | 512 | 52.367 | 16.327 | 7.378  | 1.00 | 55.60 |
| atom | 3982 | C   | LEU | 512 | 51.874 | 19.888 | 4.062  | 1.00 | 61.49 |
| atom | 3983 | O   | LEU | 512 | 52.890 | 20.580 | 4.131  | 1.00 | 61.05 |
| atom | 3984 | N   | SER | 513 | 51.328 | 19.601 | 2.886  | 1.00 | 62.99 |
| atom | 3985 | CA  | SER | 513 | 51.847 | 19.980 | 1.592  | 1.00 | 65.12 |
| atom | 3986 | CB  | SER | 513 | 51.074 | 19.221 | 0.496  | 1.00 | 63.64 |
| atom | 3987 | OG  | SER | 513 | 50.604 | 20.093 | -0.513 | 1.00 | 62.93 |
| atom | 3988 | C   | SER | 513 | 51.802 | 21.467 | 1.270  | 1.00 | 66.83 |
| atom | 3989 | O   | SER | 513 | 52.365 | 21.894 | 0.249  | 1.00 | 67.21 |
| atom | 3990 | N   | GLN | 514 | 51.139 | 22.289 | 2.075  | 1.00 | 67.94 |
| atom | 3991 | CA  | GLN | 514 | 51.027 | 23.695 | 1.801  | 1.00 | 68.91 |
| atom | 3992 | CB  | GLN | 514 | 49.635 | 24.216 | 2.195  | 1.00 | 69.72 |

| atom | 3993 | CG  | GLN | 514 | 48.698 | 24.283 | 1.010  | 1.00 | 71.63 |
|------|------|-----|-----|-----|--------|--------|--------|------|-------|
| atom | 3994 | CD  | GLN | 514 | 47.839 | 23.037 | 0.964  | 1.00 | 74.02 |
| atom | 3995 | OE1 | GLN | 514 | 47.169 | 22.742 | 1.959  | 1.00 | 75.01 |
| atom | 3996 | NE2 | GLN | 514 | 47.858 | 22.324 | -0.156 | 1.00 | 74.45 |
| atom | 3997 | C   | GLN | 514 | 51.944 | 24.621 | 2.578  | 1.00 | 69.18 |
| atom | 3998 | O   | GLN | 514 | 52.280 | 25.666 | 2.022  | 1.00 | 69.39 |
| atom | 3999 | N   | GLY | 515 | 52.143 | 24.322 | 3.868  | 1.00 | 69.84 |
| atom | 4000 | CA  | GLY | 515 | 52.945 | 25.289 | 4.548  | 1.00 | 69.92 |
| atom | 4001 | C   | GLY | 515 | 53.413 | 25.360 | 5.944  | 1.00 | 69.37 |
| atom | 4002 | O   | GLY | 515 | 53.972 | 24.468 | 6.556  | 1.00 | 70.22 |
| atom | 4003 | N   | GLY | 516 | 53.307 | 26.608 | 6.408  | 1.00 | 69.09 |
| atom | 4004 | CA  | GLY | 516 | 53.733 | 26.988 | 7.751  | 1.00 | 67.75 |
| atom | 4005 | C   | GLY | 516 | 52.512 | 26.820 | 8.659  | 1.00 | 66.48 |
| atom | 4006 | O   | GLY | 516 | 52.546 | 25.978 | 9.554  | 1.00 | 66.81 |
| atom | 4007 | N   | ARG | 517 | 51.452 | 27.575 | 8.374  | 1.00 | 64.73 |
| atom | 4008 | CA  | ARG | 517 | 50.259 | 27.489 | 9.210  | 1.00 | 62.15 |
| atom | 4009 | CB  | ARG | 517 | 49.533 | 28.832 | 9.294  | 1.00 | 64.74 |
| atom | 4010 | CG  | ARG | 517 | 49.849 | 29.612 | 10.555 | 1.00 | 66.51 |
| atom | 4011 | CD  | ARG | 517 | 49.537 | 31.076 | 10.532 | 1.00 | 68.06 |
| atom | 4012 | NE  | ARG | 517 | 48.220 | 31.532 | 10.908 | 1.00 | 69.96 |
| atom | 4013 | CZ  | ARG | 517 | 47.268 | 32.018 | 10.118 | 1.00 | 70.48 |
| atom | 4014 | NH1 | ARG | 517 | 47.472 | 32.134 | 8.811  | 1.00 | 69.84 |
| atom | 4015 | NH2 | ARG | 517 | 46.101 | 32.402 | 10.634 | 1.00 | 69.85 |
| atom | 4016 | C   | ARG | 517 | 49.341 | 26.355 | 8.790  | 1.00 | 59.90 |
| atom | 4017 | O   | ARG | 517 | 48.717 | 25.699 | 9.631  | 1.00 | 59.47 |
| atom | 4018 | N   | ALA | 518 | 49.276 | 26.072 | 7.493  | 1.00 | 57.47 |
| atom | 4019 | CA  | ALA | 518 | 48.464 | 24.974 | 6.987  | 1.00 | 54.35 |
| atom | 4020 | CB  | ALA | 518 | 48.339 | 25.030 | 5.479  | 1.00 | 54.33 |
| atom | 4021 | C   | ALA | 518 | 49.051 | 23.643 | 7.453  | 1.00 | 52.60 |
| atom | 4022 | O   | ALA | 518 | 48.320 | 22.737 | 7.855  | 1.00 | 50.87 |
| atom | 4023 | N   | ALA | 519 | 50.380 | 23.512 | 7.454  | 1.00 | 50.92 |
| atom | 4024 | CA  | ALA | 519 | 51.027 | 22.298 | 7.917  | 1.00 | 49.71 |
| atom | 4025 | CB  | ALA | 519 | 52.488 | 22.194 | 7.525  | 1.00 | 49.15 |
| atom | 4026 | C   | ALA | 519 | 50.959 | 22.161 | 9.439  | 1.00 | 48.69 |
| atom | 4027 | O   | ALA | 519 | 51.110 | 21.055 | 9.957  | 1.00 | 48.66 |
| atom | 4028 | N   | THR | 520 | 50.800 | 23.275 | 10.140 | 1.00 | 47.46 |

| | | | | | | | | | |
|---|---|---|---|---|---|---|---|---|---|
| atom | 4029 | CA | THR | 520 | 50.666 | 23.235 | 11.594 | 1.00 | 45.98 |
| atom | 4030 | CB | THR | 520 | 50.940 | 24.616 | 12.196 | 1.00 | 45.32 |
| atom | 4031 | OG1 | THR | 520 | 52.353 | 24.847 | 11.987 | 1.00 | 45.27 |
| atom | 4032 | CG2 | THR | 520 | 50.595 | 24.686 | 13.670 | 1.00 | 42.69 |
| atom | 4033 | C | THR | 520 | 49.303 | 22.664 | 11.955 | 1.00 | 44.34 |
| atom | 4034 | O | THR | 520 | 49.208 | 21.805 | 12.836 | 1.00 | 44.40 |
| atom | 4035 | N | CYS | 521 | 48.268 | 23.014 | 11.197 | 1.00 | 43.05 |
| atom | 4036 | CA | CYS | 521 | 46.957 | 22.414 | 11.453 | 1.00 | 42.38 |
| atom | 4037 | CB | CYS | 521 | 45.808 | 23.013 | 10.666 | 1.00 | 37.78 |
| atom | 4038 | SG | CYS | 521 | 45.567 | 24.777 | 10.937 | 1.00 | 39.22 |
| atom | 4039 | C | CYS | 521 | 47.040 | 20.917 | 11.167 | 1.00 | 41.86 |
| atom | 4040 | O | CYS | 521 | 46.608 | 20.099 | 11.970 | 1.00 | 41.96 |
| atom | 4041 | N | GLY | 522 | 47.679 | 20.565 | 10.060 | 1.00 | 42.17 |
| atom | 4042 | CA | GLY | 522 | 47.864 | 19.193 | 9.633 | 1.00 | 41.79 |
| atom | 4043 | C | GLY | 522 | 48.535 | 18.332 | 10.693 | 1.00 | 41.24 |
| atom | 4044 | O | GLY | 522 | 48.110 | 17.228 | 11.021 | 1.00 | 40.62 |
| atom | 4045 | N | LYS | 523 | 49.590 | 18.844 | 11.277 | 1.00 | 40.85 |
| atom | 4046 | CA | LYS | 523 | 50.426 | 18.183 | 12.264 | 1.00 | 41.09 |
| atom | 4047 | CB | LYS | 523 | 51.690 | 19.051 | 12.353 | 1.00 | 39.12 |
| atom | 4048 | CG | LYS | 523 | 52.768 | 18.644 | 13.325 | 1.00 | 41.02 |
| atom | 4049 | CD | LYS | 523 | 54.053 | 19.416 | 12.995 | 1.00 | 41.25 |
| atom | 4050 | CE | LYS | 523 | 54.806 | 19.777 | 14.259 | 1.00 | 43.14 |
| atom | 4051 | NZ | LYS | 523 | 55.361 | 18.565 | 14.921 | 1.00 | 45.08 |
| atom | 4052 | C | LYS | 523 | 49.794 | 18.005 | 13.632 | 1.00 | 41.61 |
| atom | 4053 | O | LYS | 523 | 49.921 | 16.961 | 14.284 | 1.00 | 41.09 |
| atom | 4054 | N | TYR | 524 | 49.131 | 19.045 | 14.138 | 1.00 | 41.86 |
| atom | 4055 | CA | TYR | 524 | 48.505 | 19.045 | 15.442 | 1.00 | 41.36 |
| atom | 4056 | CB | TYR | 524 | 48.529 | 20.474 | 16.021 | 1.00 | 41.97 |
| atom | 4057 | CG | TYR | 524 | 49.930 | 20.802 | 16.515 | 1.00 | 43.28 |
| atom | 4058 | CD1 | TYR | 524 | 50.923 | 21.196 | 15.630 | 1.00 | 42.95 |
| atom | 4059 | CE1 | TYR | 524 | 52.197 | 21.502 | 16.075 | 1.00 | 42.79 |
| atom | 4060 | CD2 | TYR | 524 | 50.250 | 20.717 | 17.864 | 1.00 | 43.24 |
| atom | 4061 | CE2 | TYR | 524 | 51.523 | 21.011 | 18.318 | 1.00 | 42.65 |
| atom | 4062 | CZ | TYR | 524 | 52.490 | 21.404 | 17.417 | 1.00 | 42.78 |
| atom | 4063 | OH | TYR | 524 | 53.769 | 21.709 | 17.826 | 1.00 | 42.29 |
| atom | 4064 | C | TYR | 524 | 47.090 | 18.496 | 15.465 | 1.00 | 41.48 |

| atom | 4065 | O | TYR | 524 | 46.805 | 17.585 | 16.254 | 1.00 | 40.75 |
|------|------|-----|-----|-----|--------|--------|--------|------|-------|
| atom | 4066 | N | LEU | 525 | 46.183 | 19.023 | 14.644 | 1.00 | 40.81 |
| atom | 4067 | CA | LEU | 525 | 44.798 | 18.608 | 14.601 | 1.00 | 39.80 |
| atom | 4068 | CB | LEU | 525 | 43.968 | 19.538 | 13.679 | 1.00 | 36.75 |
| atom | 4069 | CG | LEU | 525 | 44.152 | 21.034 | 13.958 | 1.00 | 37.95 |
| atom | 4070 | CD1 | LEU | 525 | 43.372 | 21.908 | 12.985 | 1.00 | 36.60 |
| atom | 4071 | CD2 | LEU | 525 | 43.722 | 21.377 | 15.395 | 1.00 | 37.64 |
| atom | 4072 | C | LEU | 525 | 44.624 | 17.183 | 14.109 | 1.00 | 40.41 |
| atom | 4073 | O | LEU | 525 | 43.610 | 16.560 | 14.419 | 1.00 | 40.21 |
| atom | 4074 | N | PHE | 526 | 45.537 | 16.689 | 13.263 | 1.00 | 40.85 |
| atom | 4075 | CA | PHE | 526 | 45.374 | 15.370 | 12.677 | 1.00 | 40.64 |
| atom | 4076 | CB | PHE | 526 | 45.162 | 15.482 | 11.151 | 1.00 | 37.74 |
| atom | 4077 | CG | PHE | 526 | 43.886 | 16.177 | 10.756 | 1.00 | 34.10 |
| atom | 4078 | CD1 | PHE | 526 | 43.900 | 17.513 | 10.416 | 1.00 | 31.45 |
| atom | 4079 | CD2 | PHE | 526 | 42.670 | 15.512 | 10.752 | 1.00 | 32.89 |
| atom | 4080 | CE1 | PHE | 526 | 42.735 | 18.175 | 10.065 | 1.00 | 28.36 |
| atom | 4081 | CE2 | PHE | 526 | 41.501 | 16.165 | 10.404 | 1.00 | 30.56 |
| atom | 4082 | CZ | PHE | 526 | 41.545 | 17.495 | 10.060 | 1.00 | 28.98 |
| atom | 4083 | C | PHE | 526 | 46.463 | 14.367 | 12.979 | 1.00 | 41.11 |
| atom | 4084 | O | PHE | 526 | 46.574 | 13.386 | 12.233 | 1.00 | 41.69 |
| atom | 4085 | N | ASN | 527 | 47.181 | 14.482 | 14.091 | 1.00 | 41.21 |
| atom | 4086 | CA | ASN | 527 | 48.169 | 13.478 | 14.466 | 1.00 | 40.98 |
| atom | 4087 | CB | ASN | 527 | 49.008 | 13.944 | 15.652 | 1.00 | 41.27 |
| atom | 4088 | CG | ASN | 527 | 50.450 | 13.478 | 15.559 | 1.00 | 42.21 |
| atom | 4089 | OD1 | ASN | 527 | 51.043 | 13.224 | 16.602 | 1.00 | 41.73 |
| atom | 4090 | ND2 | ASN | 527 | 51.042 | 13.388 | 14.373 | 1.00 | 42.15 |
| atom | 4091 | C | ASN | 527 | 47.529 | 12.137 | 14.813 | 1.00 | 41.43 |
| atom | 4092 | O | ASN | 527 | 48.160 | 11.098 | 14.612 | 1.00 | 41.00 |
| atom | 4093 | N | TRP | 528 | 46.283 | 12.130 | 15.293 | 1.00 | 41.21 |
| atom | 4094 | CA | TRP | 528 | 45.557 | 10.907 | 15.586 | 1.00 | 42.13 |
| atom | 4095 | CB | TRP | 528 | 44.180 | 11.192 | 16.230 | 1.00 | 37.93 |
| atom | 4096 | CG | TRP | 528 | 43.317 | 12.076 | 15.380 | 1.00 | 34.34 |
| atom | 4097 | CD2 | TRP | 528 | 42.426 | 11.694 | 14.326 | 1.00 | 32.91 |
| atom | 4098 | CE2 | TRP | 528 | 41.864 | 12.867 | 13.798 | 1.00 | 32.39 |
| atom | 4099 | CE3 | TRP | 528 | 42.030 | 10.462 | 13.794 | 1.00 | 34.49 |
| atom | 4100 | CD1 | TRP | 528 | 43.274 | 13.435 | 15.432 | 1.00 | 34.38 |

| atom | 4101 | NE1 | TRP | 528 | 42.389 | 13.918 | 14.488 | 1.00 | 33.58 |
|------|------|-----|-----|-----|--------|--------|--------|------|-------|
| atom | 4102 | CZ2 | TRP | 528 | 40.937 | 12.861 | 12.760 | 1.00 | 34.21 |
| atom | 4103 | CZ3 | TRP | 528 | 41.108 | 10.440 | 12.759 | 1.00 | 35.84 |
| atom | 4104 | CH2 | TRP | 528 | 40.571 | 11.638 | 12.253 | 1.00 | 36.49 |
| atom | 4105 | C   | TRP | 528 | 45.336 | 10.064 | 14.332 | 1.00 | 43.57 |
| atom | 4106 | O   | TRP | 528 | 45.218 | 8.852  | 14.416 | 1.00 | 43.38 |
| atom | 4107 | N   | ALA | 529 | 45.198 | 10.676 | 13.165 | 1.00 | 46.15 |
| atom | 4108 | CA  | ALA | 529 | 44.955 | 9.982  | 11.923 | 1.00 | 49.40 |
| atom | 4109 | CB  | ALA | 529 | 44.407 | 10.949 | 10.879 | 1.00 | 48.31 |
| atom | 4110 | C   | ALA | 529 | 46.170 | 9.250  | 11.372 | 1.00 | 51.74 |
| atom | 4111 | O   | ALA | 529 | 45.951 | 8.255  | 10.665 | 1.00 | 51.94 |
| atom | 4112 | N   | VAL | 530 | 47.392 | 9.684  | 11.657 | 1.00 | 54.38 |
| atom | 4113 | CA  | VAL | 530 | 48.559 | 9.017  | 11.115 | 1.00 | 57.58 |
| atom | 4114 | CB  | VAL | 530 | 49.811 | 9.901  | 10.913 | 1.00 | 56.64 |
| atom | 4115 | CG1 | VAL | 530 | 49.705 | 10.591 | 9.564  | 1.00 | 55.90 |
| atom | 4116 | CG2 | VAL | 530 | 50.062 | 10.871 | 12.049 | 1.00 | 55.80 |
| atom | 4117 | C   | VAL | 530 | 49.062 | 7.817  | 11.909 | 1.00 | 60.30 |
| atom | 4118 | O   | VAL | 530 | 49.098 | 7.798  | 13.130 | 1.00 | 60.61 |
| atom | 4119 | N   | LYS | 531 | 49.562 | 6.847  | 11.144 | 1.00 | 63.26 |
| atom | 4120 | CA  | LYS | 531 | 50.161 | 5.639  | 11.685 | 1.00 | 66.27 |
| atom | 4121 | CB  | LYS | 531 | 50.666 | 4.759  | 10.541 | 1.00 | 69.64 |
| atom | 4122 | CG  | LYS | 531 | 49.885 | 3.501  | 10.220 | 1.00 | 72.08 |
| atom | 4123 | CD  | LYS | 531 | 50.835 | 2.453  | 9.638  | 1.00 | 74.25 |
| atom | 4124 | CE  | LYS | 531 | 50.173 | 1.092  | 9.518  | 1.00 | 75.05 |
| atom | 4125 | NZ  | LYS | 531 | 51.138 | -0.023 | 9.730  | 1.00 | 75.24 |
| atom | 4126 | C   | LYS | 531 | 51.339 | 5.945  | 12.607 | 1.00 | 67.35 |
| atom | 4127 | O   | LYS | 531 | 51.403 | 5.445  | 13.727 | 1.00 | 67.82 |
| atom | 4128 | N   | THR | 532 | 52.297 | 6.734  | 12.140 | 1.00 | 68.66 |
| atom | 4129 | CA  | THR | 532 | 53.485 | 7.051  | 12.924 | 1.00 | 70.54 |
| atom | 4130 | CB  | THR | 532 | 54.760 | 6.991  | 12.055 | 1.00 | 71.43 |
| atom | 4131 | OG1 | THR | 532 | 54.746 | 5.781  | 11.285 | 1.00 | 70.87 |
| atom | 4132 | CG2 | THR | 532 | 56.012 | 7.006  | 12.923 | 1.00 | 71.75 |
| atom | 4133 | C   | THR | 532 | 53.376 | 8.424  | 13.574 | 1.00 | 71.04 |
| atom | 4134 | O   | THR | 532 | 53.632 | 9.443  | 12.939 | 1.00 | 70.97 |
| atom | 4135 | N   | LYS | 533 | 53.030 | 8.409  | 14.858 | 1.00 | 71.72 |
| atom | 4136 | CA  | LYS | 533 | 52.820 | 9.650  | 15.569 | 1.00 | 71.93 |

| atom | 4137 | CB | LYS | 533 | 52.196 | 9.400 | 16.954 | 1.00 | 74.51 |
| atom | 4138 | CG | LYS | 533 | 50.763 | 8.932 | 16.736 | 1.00 | 76.61 |
| atom | 4139 | CD | LYS | 533 | 49.912 | 8.974 | 17.989 | 1.00 | 77.96 |
| atom | 4140 | CE | LYS | 533 | 48.519 | 8.433 | 17.670 | 1.00 | 78.89 |
| atom | 4141 | NZ | LYS | 533 | 47.701 | 8.233 | 18.898 | 1.00 | 79.35 |
| atom | 4142 | C | LYS | 533 | 54.011 | 10.583 | 15.649 | 1.00 | 71.49 |
| atom | 4143 | O | LYS | 533 | 55.127 | 10.271 | 16.022 | 1.00 | 71.85 |
| atom | 4144 | N | LEU | 534 | 53.640 | 11.801 | 15.232 | 1.00 | 70.94 |
| atom | 4145 | CA | LEU | 534 | 54.544 | 12.931 | 15.317 | 1.00 | 70.05 |
| atom | 4146 | CB | LEU | 534 | 54.174 | 14.176 | 14.536 | 1.00 | 69.66 |
| atom | 4147 | CG | LEU | 534 | 54.187 | 14.156 | 13.011 | 1.00 | 69.70 |
| atom | 4148 | CD1 | LEU | 534 | 54.161 | 15.585 | 12.469 | 1.00 | 69.87 |
| atom | 4149 | CD2 | LEU | 534 | 55.417 | 13.445 | 12.459 | 1.00 | 69.68 |
| atom | 4150 | C | LEU | 534 | 54.545 | 13.314 | 16.811 | 1.00 | 69.63 |
| atom | 4151 | O | LEU | 534 | 53.594 | 13.115 | 17.566 | 1.00 | 69.18 |
| atom | 4152 | N | LYS | 535 | 55.691 | 13.865 | 17.165 | 1.00 | 69.01 |
| atom | 4153 | CA | LYS | 535 | 55.851 | 14.329 | 18.552 | 1.00 | 68.42 |
| atom | 4154 | CB | LYS | 535 | 57.289 | 14.124 | 18.967 | 1.00 | 71.55 |
| atom | 4155 | CG | LYS | 535 | 57.611 | 14.418 | 20.418 | 1.00 | 73.51 |
| atom | 4156 | CD | LYS | 535 | 57.973 | 15.884 | 20.590 | 1.00 | 75.34 |
| atom | 4157 | CE | LYS | 535 | 59.363 | 16.215 | 20.066 | 1.00 | 75.70 |
| atom | 4158 | NZ | LYS | 535 | 60.415 | 15.943 | 21.085 | 1.00 | 75.79 |
| atom | 4159 | C | LYS | 535 | 55.355 | 15.769 | 18.566 | 1.00 | 66.92 |
| atom | 4160 | O | LYS | 535 | 55.752 | 16.564 | 17.708 | 1.00 | 67.34 |
| atom | 4161 | N | LEU | 536 | 54.402 | 16.053 | 19.449 | 1.00 | 64.88 |
| atom | 4162 | CA | LEU | 536 | 53.817 | 17.385 | 19.503 | 1.00 | 62.01 |
| atom | 4163 | CB | LEU | 536 | 52.290 | 17.282 | 19.536 | 1.00 | 58.68 |
| atom | 4164 | CG | LEU | 536 | 51.673 | 16.465 | 18.400 | 1.00 | 56.57 |
| atom | 4165 | CD1 | LEU | 536 | 50.164 | 16.431 | 18.529 | 1.00 | 55.26 |
| atom | 4166 | CD2 | LEU | 536 | 52.077 | 17.029 | 17.043 | 1.00 | 56.85 |
| atom | 4167 | C | LEU | 536 | 54.310 | 18.186 | 20.694 | 1.00 | 61.38 |
| atom | 4168 | O | LEU | 536 | 54.070 | 17.818 | 21.843 | 1.00 | 60.98 |
| atom | 4169 | N | THR | 537 | 54.994 | 19.282 | 20.380 | 1.00 | 60.65 |
| atom | 4170 | CA | THR | 537 | 55.542 | 20.195 | 21.378 | 1.00 | 60.08 |
| atom | 4171 | CB | THR | 537 | 57.042 | 20.462 | 21.152 | 1.00 | 59.39 |
| atom | 4172 | OG1 | THR | 537 | 57.223 | 21.100 | 19.879 | 1.00 | 59.90 |

| atom | 4173 | CG2 | THR | 537 | 57.841 | 19.175 | 21.198 | 1.00 | 57.38 |
|------|------|-----|-----|-----|--------|--------|--------|------|-------|
| atom | 4174 | C   | THR | 537 | 54.793 | 21.520 | 21.323 | 1.00 | 60.03 |
| atom | 4175 | O   | THR | 537 | 54.024 | 21.766 | 20.396 | 1.00 | 59.74 |
| atom | 4176 | N   | PRO | 538 | 54.994 | 22.378 | 22.309 | 1.00 | 60.50 |
| atom | 4177 | CD  | PRO | 538 | 55.854 | 22.151 | 23.491 | 1.00 | 60.05 |
| atom | 4178 | CA  | PRO | 538 | 54.296 | 23.648 | 22.394 | 1.00 | 62.34 |
| atom | 4179 | CB  | PRO | 538 | 54.863 | 24.299 | 23.650 | 1.00 | 60.91 |
| atom | 4180 | CG  | PRO | 538 | 55.312 | 23.149 | 24.478 | 1.00 | 60.04 |
| atom | 4181 | C   | PRO | 538 | 54.432 | 24.536 | 21.173 | 1.00 | 64.63 |
| atom | 4182 | O   | PRO | 538 | 55.537 | 24.869 | 20.741 | 1.00 | 65.28 |
| atom | 4183 | N   | ILE | 539 | 53.298 | 24.952 | 20.611 | 1.00 | 67.68 |
| atom | 4184 | CA  | ILE | 539 | 53.290 | 25.810 | 19.432 | 1.00 | 71.31 |
| atom | 4185 | CB  | ILE | 539 | 51.881 | 25.985 | 18.841 | 1.00 | 69.01 |
| atom | 4186 | CG2 | ILE | 539 | 51.804 | 27.065 | 17.775 | 1.00 | 66.70 |
| atom | 4187 | CG1 | ILE | 539 | 51.380 | 24.656 | 18.259 | 1.00 | 67.98 |
| atom | 4188 | CD1 | ILE | 539 | 50.167 | 24.128 | 18.987 | 1.00 | 67.78 |
| atom | 4189 | C   | ILE | 539 | 53.908 | 27.172 | 19.733 | 1.00 | 74.86 |
| atom | 4190 | O   | ILE | 539 | 53.411 | 27.937 | 20.557 | 1.00 | 74.77 |
| atom | 4191 | N   | PRO | 540 | 54.985 | 27.494 | 19.022 | 1.00 | 78.13 |
| atom | 4192 | CD  | PRO | 540 | 55.612 | 26.602 | 18.007 | 1.00 | 78.72 |
| atom | 4193 | CA  | PRO | 540 | 55.688 | 28.755 | 19.166 | 1.00 | 80.96 |
| atom | 4194 | CB  | PRO | 540 | 56.725 | 28.726 | 18.049 | 1.00 | 80.38 |
| atom | 4195 | CG  | PRO | 540 | 56.898 | 27.298 | 17.676 | 1.00 | 79.74 |
| atom | 4196 | C   | PRO | 540 | 54.824 | 30.003 | 19.060 | 1.00 | 83.92 |
| atom | 4197 | O   | PRO | 540 | 53.877 | 30.076 | 18.270 | 1.00 | 84.20 |
| atom | 4198 | N   | ALA | 541 | 55.166 | 31.032 | 19.835 | 1.00 | 86.83 |
| atom | 4199 | CA  | ALA | 541 | 54.427 | 32.288 | 19.864 | 1.00 | 90.36 |
| atom | 4200 | CB  | ALA | 541 | 54.914 | 33.125 | 21.044 | 1.00 | 90.83 |
| atom | 4201 | C   | ALA | 541 | 54.529 | 33.093 | 18.574 | 1.00 | 92.46 |
| atom | 4202 | O   | ALA | 541 | 55.108 | 32.644 | 17.586 | 1.00 | 92.76 |
| atom | 4203 | N   | ALA | 542 | 53.955 | 34.294 | 18.573 | 1.00 | 94.67 |
| atom | 4204 | CA  | ALA | 542 | 53.965 | 35.176 | 17.412 | 1.00 | 97.14 |
| atom | 4205 | CB  | ALA | 542 | 52.662 | 35.960 | 17.315 | 1.00 | 96.80 |
| atom | 4206 | C   | ALA | 542 | 55.146 | 36.145 | 17.431 | 1.00 | 98.65 |
| atom | 4207 | O   | ALA | 542 | 55.520 | 36.675 | 18.477 | 1.00 | 99.00 |
| atom | 4208 | N   | SER | 543 | 55.728 | 36.382 | 16.260 | 1.00 | 100.02 |

| atom | 4209 | CA | SER | 543 | 56.878 | 37.267 | 16.130 | 1.00101.75 |
|------|------|-----|-----|-----|--------|--------|--------|------------|
| atom | 4210 | CB | SER | 543 | 58.103 | 36.477 | 15.662 | 1.00101.78 |
| atom | 4211 | OG | SER | 543 | 57.861 | 35.747 | 14.475 | 1.00101.37 |
| atom | 4212 | C | SER | 543 | 56.592 | 38.432 | 15.192 | 1.00102.85 |
| atom | 4213 | O | SER | 543 | 55.457 | 38.653 | 14.775 | 1.00102.81 |
| atom | 4214 | N | GLN | 544 | 57.630 | 39.186 | 14.869 | 1.00103.89 |
| atom | 4215 | CA | GLN | 544 | 57.610 | 40.337 | 13.995 | 1.00104.95 |
| atom | 4216 | CB | GLN | 544 | 56.528 | 40.294 | 12.911 | 1.00106.48 |
| atom | 4217 | CG | GLN | 544 | 56.950 | 40.994 | 11.635 | 1.00107.82 |
| atom | 4218 | CD | GLN | 544 | 55.886 | 41.816 | 10.948 | 1.00108.80 |
| atom | 4219 | OE1 | GLN | 544 | 55.451 | 41.491 | 9.841 | 1.00109.07 |
| atom | 4220 | NE2 | GLN | 544 | 55.456 | 42.912 | 11.571 | 1.00109.05 |
| atom | 4221 | C | GLN | 544 | 57.458 | 41.636 | 14.791 | 1.00105.21 |
| atom | 4222 | O | GLN | 544 | 57.581 | 42.726 | 14.192 | 1.00105.61 |
| atom | 4223 | O | HOH | 601 | 39.416 | 32.373 | 21.959 | 1.00 38.26 |
| atom | 4224 | O | HOH | 602 | 34.853 | 12.289 | 23.051 | 1.00 25.35 |
| atom | 4225 | O | HOH | 603 | 7.176 | 41.823 | 27.240 | 1.00 34.90 |
| atom | 4226 | O | HOH | 604 | 27.015 | 49.222 | 35.013 | 1.00 21.37 |
| atom | 4227 | O | HOH | 605 | 11.909 | 45.427 | 20.466 | 1.00 23.37 |
| atom | 4228 | O | HOH | 606 | 45.945 | 15.106 | 16.832 | 1.00 25.63 |
| atom | 4229 | O | HOH | 607 | 22.293 | 44.940 | 22.409 | 1.00 26.54 |
| atom | 4230 | O | HOH | 608 | 24.789 | 15.423 | 25.741 | 1.00 31.72 |
| atom | 4231 | O | HOH | 609 | 36.607 | 20.122 | 27.816 | 1.00 32.48 |
| atom | 4232 | O | HOH | 610 | 15.150 | 17.094 | 36.202 | 1.00 32.73 |
| atom | 4233 | O | HOH | 611 | 33.052 | 48.857 | 20.494 | 1.00 62.14 |
| atom | 4234 | O | HOH | 612 | 16.443 | 23.960 | 36.558 | 1.00 26.79 |
| atom | 4235 | O | HOH | 613 | 12.419 | 39.814 | 32.488 | 1.00 25.49 |
| atom | 4236 | O | HOH | 614 | 12.846 | 33.895 | 43.699 | 1.00 34.61 |
| atom | 4237 | O | HOH | 615 | 8.062 | 45.477 | 29.238 | 1.00 35.36 |
| atom | 4238 | O | HOH | 616 | 13.451 | 46.746 | 18.419 | 1.00 28.42 |
| atom | 4239 | O | HOH | 617 | 22.219 | 45.078 | 27.235 | 1.00 32.79 |
| atom | 4240 | O | HOH | 618 | 14.351 | 35.851 | 42.540 | 1.00 31.94 |
| atom | 4241 | O | HOH | 619 | 35.894 | 9.447 | 27.106 | 1.00 29.78 |
| atom | 4242 | O | HOH | 620 | 6.654 | 38.401 | 15.563 | 1.00 33.57 |
| atom | 4243 | O | HOH | 621 | 30.549 | 10.500 | 27.836 | 1.00 30.60 |
| atom | 4244 | O | HOH | 622 | 42.028 | 18.441 | 19.060 | 1.00 32.56 |

| atom | 4245 | O | HOH | 623 | 19.629 | 1.886 | 53.249 | 1.00 | 40.72 |
|------|------|---|-----|-----|--------|-------|--------|------|-------|
| atom | 4246 | O | HOH | 624 | 18.601 | 43.513 | 26.480 | 1.00 | 37.90 |
| atom | 4247 | O | HOH | 625 | 28.748 | 26.644 | 18.484 | 1.00 | 41.26 |
| atom | 4248 | O | HOH | 626 | 26.560 | 11.619 | 29.877 | 1.00 | 38.48 |
| atom | 4249 | O | HOH | 627 | 29.465 | 15.171 | 34.150 | 1.00 | 36.73 |
| atom | 4250 | O | HOH | 628 | 43.035 | 16.934 | 17.134 | 1.00 | 36.73 |
| atom | 4251 | O | HOH | 629 | 23.822 | 46.573 | 24.097 | 1.00 | 33.20 |
| atom | 4252 | O | HOH | 630 | 25.271 | 50.335 | 38.538 | 1.00 | 25.12 |
| atom | 4253 | O | HOH | 631 | 4.320 | 37.148 | 42.301 | 1.00 | 32.46 |
| atom | 4254 | O | HOH | 632 | 40.613 | 17.436 | 39.080 | 1.00 | 49.80 |
| atom | 4255 | O | HOH | 633 | 12.003 | 29.726 | 38.297 | 1.00 | 29.29 |
| atom | 4256 | O | HOH | 634 | 35.382 | 50.151 | 34.603 | 1.00 | 34.72 |
| atom | 4257 | O | HOH | 635 | 13.096 | 55.001 | 28.185 | 1.00 | 36.33 |
| atom | 4258 | O | HOH | 636 | 20.632 | 26.171 | 14.508 | 1.00 | 33.70 |
| atom | 4259 | O | HOH | 637 | 28.974 | 41.981 | 32.988 | 1.00 | 43.64 |
| atom | 4260 | O | HOH | 638 | 7.781 | 57.786 | 29.001 | 1.00 | 38.97 |
| atom | 4261 | O | HOH | 639 | 38.145 | 24.924 | 39.145 | 1.00 | 29.73 |
| atom | 4262 | O | HOH | 640 | 27.943 | 22.537 | 14.423 | 1.00 | 37.91 |
| atom | 4263 | O | HOH | 641 | 28.202 | 32.555 | 23.637 | 1.00 | 51.86 |
| atom | 4264 | O | HOH | 642 | 39.590 | 20.940 | 28.396 | 1.00 | 38.94 |
| atom | 4265 | O | HOH | 643 | 14.505 | 36.769 | 53.588 | 1.00 | 37.92 |
| atom | 4266 | O | HOH | 644 | 16.399 | 35.986 | 23.736 | 1.00 | 35.19 |
| atom | 4267 | O | HOH | 645 | 7.012 | 57.783 | 36.498 | 1.00 | 32.48 |
| atom | 4269 | O | HOH | 647 | 27.052 | 22.485 | 23.212 | 1.00 | 33.74 |
| atom | 4270 | O | HOH | 648 | 20.118 | 11.628 | 49.419 | 1.00 | 33.05 |
| atom | 4271 | O | HOH | 649 | 14.595 | 42.910 | 38.429 | 1.00 | 28.04 |
| atom | 4272 | O | HOH | 650 | 48.673 | 28.709 | 22.473 | 1.00 | 36.18 |
| atom | 4273 | O | HOH | 651 | 16.154 | 46.223 | 18.351 | 1.00 | 28.46 |
| atom | 4274 | O | HOH | 652 | 4.856 | 50.821 | 25.300 | 1.00 | 40.58 |
| atom | 4275 | O | HOH | 653 | 15.174 | 52.133 | 22.544 | 1.00 | 33.68 |
| atom | 4276 | O | HOH | 654 | 27.053 | 58.765 | 29.773 | 1.00 | 28.44 |
| atom | 4277 | O | HOH | 655 | 22.999 | 55.126 | 46.074 | 1.00 | 32.29 |
| atom | 4278 | O | HOH | 656 | 18.007 | 14.225 | 36.118 | 1.00 | 41.28 |
| atom | 4279 | O | HOH | 657 | 42.757 | 18.415 | 26.706 | 1.00 | 40.21 |
| atom | 4280 | O | HOH | 658 | 10.348 | 47.542 | 21.144 | 1.00 | 40.90 |
| atom | 4281 | O | HOH | 659 | 24.714 | 53.454 | 48.065 | 1.00 | 35.45 |

```
atom  4282  0  HOH  660   26.171  52.196  40.388  1.00 36.38
atom  4283  0  HOH  661   22.662  38.585  33.007  1.00 30.29
atom  4284  0  HOH  662   28.462  38.673  21.230  1.00 40.56
atom  4285  0  HOH  663   27.665  61.143  24.885  1.00 34.88
atom  4286  0  HOH  664    5.749  46.729  33.345  1.00 39.87
atom  4287  0  HOH  665   32.097   4.335  13.546  1.00 44.93
atom  4288  0  HOH  666   15.509  57.627  43.098  1.00 38.96
atom  4289  0  HOH  667   28.878  55.157  39.117  1.00 39.74
atom  4290  0  HOH  668    2.344  34.193  39.491  1.00 41.74
atom  4291  0  HOH  669   18.705  17.526  18.312  1.00 48.89
atom  4292  0  HOH  670   18.545  40.630  23.557  1.00 40.79
atom  4293  0  HOH  671   18.093  56.934  43.757  1.00 43.01
atom  4294  0  HOH  672   33.865  21.464  45.335  1.00 45.20
atom  4295  0  HOH  673   18.738  11.296  52.312  1.00 40.67
atom  4296  0  HOH  674   49.842  21.756  27.208  1.00 45.26
atom  4297  0  HOH  675   14.077  61.768  26.894  1.00 44.21
atom  4298  0  HOH  676   29.352  37.793  38.488  1.00 47.48
atom  4299  0  HOH  677   14.688  40.086  34.203  1.00 32.06
atom  4300  0  HOH  678   -0.130  42.110  35.386  1.00 50.62
atom  4301  0  HOH  679   23.917  59.062  40.381  1.00 52.82
atom  4302  0  HOH  680   21.039  27.441  18.989  1.00 53.38
atom  4303  0  HOH  681    4.465  42.536  40.933  1.00 53.59
atom  4304  0  HOH  682   28.864  53.264  42.979  1.00 49.83
atom  4305  0  HOH  683   12.790  53.372  23.492  1.00 41.76
atom  4306  0  HOH  684   20.419  19.429  22.191  1.00 56.08
atom  4307  0  HOH  685   32.405   6.817  33.858  1.00 43.52
atom  4308  0  HOH  686   41.032  18.710  28.730  1.00 35.22
atom  4309  0  HOH  687   33.372  56.650  29.484  1.00 38.25
atom  4310  0  HOH  688   12.177  53.403  26.018  1.00 47.47
atom  4311  0  HOH  689   13.067  24.959  19.948  1.00 39.99
atom  4312  0  HOH  690   26.208  56.586  32.718  1.00 35.69
atom  4313  0  HOH  691   10.850  35.203  26.471  1.00 51.20
atom  4314  0  HOH  692   -1.246  47.904  29.933  1.00 74.47
atom  4315  0  HOH  693   15.332  41.394  36.356  1.00 33.45
atom  4316  0  HOH  694   31.936   5.151  29.986  1.00 44.42
atom  4317  0  HOH  695   20.757  13.531  36.698  1.00 37.98
```

| atom | 4318 | 0 | HOH | 696 | 41.789 | 31.683 | 2.247 | 1.00 40.60 |
|------|------|---|-----|-----|--------|--------|--------|-----------|
| atom | 4319 | 0 | HOH | 697 | 13.768 | 27.205 | 18.627 | 1.00 51.03 |
| atom | 4320 | 0 | HOH | 698 | 14.296 | 13.956 | 52.787 | 1.00 49.10 |
| atom | 4321 | 0 | HOH | 699 | 1.909 | 45.188 | 21.728 | 1.00 39.05 |
| atom | 4322 | 0 | HOH | 700 | 3.892 | 35.085 | 43.819 | 1.00 46.10 |
| atom | 4323 | 0 | HOH | 701 | 28.415 | 57.528 | 34.019 | 1.00 37.60 |
| atom | 4324 | 0 | HOH | 702 | 17.015 | 45.788 | 49.000 | 1.00 52.80 |
| atom | 4325 | 0 | HOH | 703 | 13.341 | 58.649 | 23.958 | 1.00 39.43 |
| atom | 4326 | 0 | HOH | 704 | 1.465 | 28.797 | 15.139 | 1.00 46.86 |
| atom | 4327 | 0 | HOH | 705 | 28.207 | 9.256 | 27.941 | 1.00 44.81 |
| atom | 4328 | 0 | HOH | 706 | 12.303 | 56.985 | 21.644 | 1.00 59.92 |
| atom | 4329 | 0 | HOH | 707 | 45.674 | 18.021 | 30.152 | 1.00 44.70 |
| atom | 4330 | 0 | HOH | 708 | 16.390 | 10.721 | 5.568 | 1.00 47.62 |
| atom | 4331 | 0 | HOH | 709 | 33.735 | 40.733 | 36.056 | 1.00 45.60 |
| atom | 4332 | 0 | HOH | 710 | 22.082 | 49.360 | 49.539 | 1.00 40.39 |
| atom | 4333 | 0 | HOH | 711 | 12.143 | 56.551 | 25.310 | 1.00 52.77 |
| atom | 4334 | 0 | HOH | 712 | 23.693 | 10.126 | 29.221 | 1.00 39.54 |
| atom | 4335 | 0 | HOH | 713 | 15.947 | 9.210 | 7.818 | 1.00 52.62 |
| atom | 4336 | 0 | HOH | 714 | 18.643 | 63.643 | 26.154 | 1.00 45.78 |
| atom | 4337 | 0 | HOH | 715 | 20.433 | 63.664 | 28.381 | 1.00 43.88 |
| atom | 4338 | 0 | HOH | 716 | 4.576 | 31.597 | 52.144 | 1.00 43.55 |
| atom | 4339 | 0 | HOH | 717 | 27.625 | 58.111 | 38.906 | 1.00 48.38 |
| atom | 4340 | 0 | HOH | 718 | 34.785 | 24.278 | 25.937 | 1.00 36.67 |
| atom | 4341 | 0 | HOH | 719 | 38.676 | 32.144 | 25.131 | 1.00 48.13 |
| atom | 4342 | 0 | HOH | 720 | 11.971 | 44.859 | 44.730 | 1.00 39.23 |
| atom | 4343 | 0 | HOH | 721 | 27.713 | 4.896 | 10.913 | 1.00 37.81 |
| atom | 4344 | 0 | HOH | 722 | 26.253 | 2.516 | 9.114 | 1.00 41.98 |
| atom | 4345 | 0 | HOH | 723 | 25.335 | 25.234 | 18.476 | 1.00 46.74 |
| atom | 4346 | 0 | HOH | 724 | 28.698 | 6.858 | 44.415 | 1.00 56.04 |
| atom | 4347 | 0 | HOH | 725 | 25.724 | 22.733 | 17.953 | 1.00 45.92 |
| atom | 4348 | 0 | HOH | 726 | 30.617 | 28.694 | 15.393 | 1.00 56.96 |
| atom | 4349 | 0 | HOH | 727 | 24.267 | 5.963 | 38.117 | 1.00 49.89 |
| atom | 4350 | 0 | HOH | 728 | 36.317 | 56.063 | 21.506 | 1.00 55.28 |
| atom | 4351 | 0 | HOH | 729 | 24.123 | 28.153 | 13.537 | 1.00 53.34 |
| atom | 4352 | 0 | HOH | 730 | 24.186 | 59.247 | 30.993 | 1.00 49.92 |
| atom | 4353 | 0 | HOH | 731 | 7.413 | 53.048 | 27.667 | 1.00 37.69 |

| | | | | | | | | | |
|---|---|---|---|---|---|---|---|---|---|
| atom | 4354 | O | HOH | 732 | 33.542 | 1.246 | 14.005 | 1.00 | 44.57 |
| atom | 4355 | O | HOH | 733 | 27.985 | 60.350 | 27.701 | 1.00 | 47.94 |
| atom | 4356 | O | HOH | 734 | -1.449 | 42.665 | 33.002 | 1.00 | 45.86 |
| atom | 4357 | O | HOH | 735 | 49.651 | 16.083 | 23.075 | 1.00 | 44.34 |
| atom | 4358 | O | HOH | 736 | 28.788 | 6.954 | 29.339 | 1.00 | 59.77 |
| atom | 4359 | O | HOH | 737 | 11.485 | 48.716 | 23.565 | 1.00 | 24.30 |
| atom | 4360 | O | HOH | 738 | 43.978 | 7.666 | 17.742 | 1.00 | 52.44 |
| atom | 4361 | O | HOH | 739 | -5.600 | 36.652 | 31.754 | 1.00 | 45.41 |
| atom | 4362 | O | HOH | 740 | -1.755 | 36.423 | 20.500 | 1.00 | 48.38 |
| atom | 4363 | O | HOH | 741 | 1.470 | 26.349 | 16.442 | 1.00 | 62.39 |
| atom | 4364 | O | HOH | 742 | 25.968 | 15.337 | 28.212 | 1.00 | 44.37 |
| atom | 4365 | O | HOH | 743 | 35.235 | 37.148 | 11.152 | 1.00 | 40.43 |
| atom | 4366 | O | HOH | 744 | 3.609 | 36.727 | 39.779 | 1.00 | 38.39 |
| atom | 4367 | O | HOH | 745 | 7.630 | 24.434 | 11.385 | 1.00 | 39.65 |
| atom | 4369 | O | HOH | 746 | 35.611 | 26.631 | 42.519 | 1.00 | 46.91 |
| atom | 4370 | O | HOH | 747 | 21.977 | 25.528 | 12.231 | 1.00 | 48.62 |
| atom | 4371 | O | HOH | 748 | 7.061 | 26.561 | 50.562 | 1.00 | 43.78 |
| atom | 4372 | O | HOH | 749 | -2.200 | 42.958 | 26.253 | 1.00 | 44.29 |
| atom | 4373 | O | HOH | 750 | 14.510 | 23.745 | 31.041 | 1.00 | 52.28 |
| atom | 4374 | O | HOH | 751 | 33.359 | 49.132 | 31.548 | 1.00 | 44.21 |
| atom | 4375 | O | HOH | 752 | 23.513 | 56.496 | 31.204 | 1.00 | 50.34 |

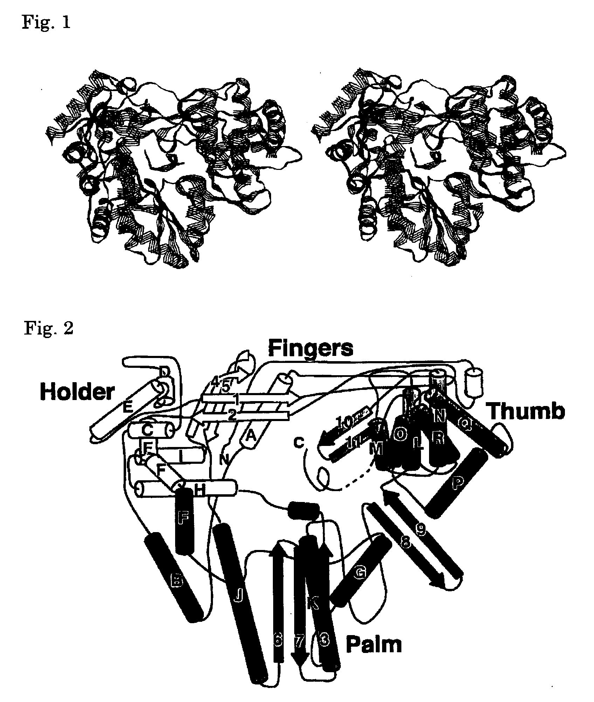

The structure of the HCV polymerase was visualized using the program software RasMol (Free Soft, Roger Sayle, Glaxo Research & Development, Greenford, Middlesex, UK). Figure 1 shows the three-dimensional structure obtained by visualizing the structural coordinate of $NS5B_{570}$ in Table 2.

Crystal structure analysis revealed that the crystal of HCV polymerase $NS5B_{570}$ belongs to the space group of $P4_32_12$ of a =b = 63.7 Å, c = 262.9 Å, and the HCV polymerase is 67 X 63 X 68 Å spherical protein comprising the cone shape in the structure.

The three-dimensional structure of the HCV polymerase $NS5B_{570}$ characteristically has a glove-like structure shown in Figure 1, comprising Fingers, Palm, Thumb, and Holder domains. Figure 2 schematically shows the structure of the HCV polymerase.

The Fingers domain comprises four β sheets and one α helix, similar to the structure of the HIV reverse transcriptase, although there is no similarity in the amino acid sequence to this enzyme. There are two long loops (one loop extending from the N-terminus to α A helix, and the other loop between β 1 and β 2), and a net is formed from the lower part of the cone shape to the upper end of the Thumb domain. The lower part of the net is open, and presumably is the entrance for a substrate ribonucleoside triphosphate (rNTP).

It is known that the structure of the poliovirus polymerase (Structure 5, 1109-1122, 1997) comprises Fingers, Palm, and Thumb domains. The structure of the Fingers domain is disordered except for the net end containing a short helix in which the Fingers domain extends to the Thumb domain. The region corresponding to the connecting region between the Holder and Palm domains in the HCV polymerase was identified as the Fingers domain, however, most of the rest of the structure in the Fingers domain has not been revealed yet.

The Holder domain consists of two helixes, αH and αI, located like supporting this region, a part of each αC, αD, αE, and αF, and a long loop like inserted to the Fingers and Palm domains between αD and αE. This domain forms a valley which is one wall of the cone shape between the Palm domain and this domain, and the U-shaped valley between the Fingers domain and the domain. In two valleys, basic amino acid residue align, which are positively charged. The positively charged surface conveniently binds to a negatively charged template RNA, thus the U-shaped valley is supposed to be an entrance for a template RNA.

The Palm domain comprises the structure similar to HIV reverse transcriptase, *E*. *coli*, or Taq DNA-dependent DNA polymerase and T7 DNA-dependent polymerase.

The Thumb domain consists of seven helixes, two distorted β sheets. The core structure of this domain comprises the structure similar to the HIV reverse transcriptase. The β sheet extending from the apex of the Thumb domain consists of nonhydrophilic residues, except for the hydrophilic junction, and hangs down to the center of the cone shape, like pushing the C-terminal nonhydrophilic region. This long β sheet is not observed in the other polymerases.

The N-terminus of the HCV polymerase forms mimic β sheet at the center of the Fingers domain with β5. This means that N-terminus-truncated variants lose the replicase activity.

EXAMPLE 4

Determination of the active site and the RNA binding cleft in the HCV polymerase

The active site and the RNA binding cleft in the HCV polymerase were determined based on the three-dimensional structures of the obtained HCV polymerases ($NS5B_{570}$, $NS5B_{544}$, $NS5B_{536}$, and $NS5B_{531}$), the conformational variation among each HCV polymerase, and the comparison of the three-dimensional structures with other proteins comprising the similar functions.

The Palm domain of the HCV polymerase was revealed to have the structure similar to HIV reverse transcriptase, *E*. *coli*, or Taq DNA-dependent DNA polymerase, and T7 DNA-dependent polymerase. Comparison of the conserved amino acids sequences between the active site of the structurally known Palm domains of the other polymerase and the Palm domain of the HCV polymerase deduced that the active site is the space formed by Asp 220, 318, and 319, Lys 141, Arg 158, and/or the hydrophilic shallow cavity formed by Ser 282, Thr 287, and Asn 291.

Asp 225 corresponds to Tyr 115 of the HIV reverse tranasctriptase and this difference of the amino acids presumably determines whether the substrate is rNTP or dNTP. Arg 158 and Lys 141 of the Fingers domain, the conserved residues between the HCV polymerase and the HIV reverse transcriptase, would have an important role in the binding of rNTP.

The Thumb domain of the HCV polymerase can structurally move against the Palm and Fingers domains and this movement changes the inner space of the Palm domain. This movement can be compared to the open and closed states of a glove. This space was confirmed to be formed by the regions of amino acids 197 to 223, 310 to 325, and 348 to 366, and is designated "the inner space of the Palm domain".

A compound existing in this space presumably inhibits the spatial formation and inactivates the polymerase activity. It is rationally assumed that "the inner space of the Palm domain" can be a target for HCV polymerase inhibitors. Even if distortion is generated to some extent, this space is a part of the RNA binding site, and this region may thus shift in about 1 to about 20, preferably about 1 to about 10, and more preferably about 1 to about 5 amino acids.

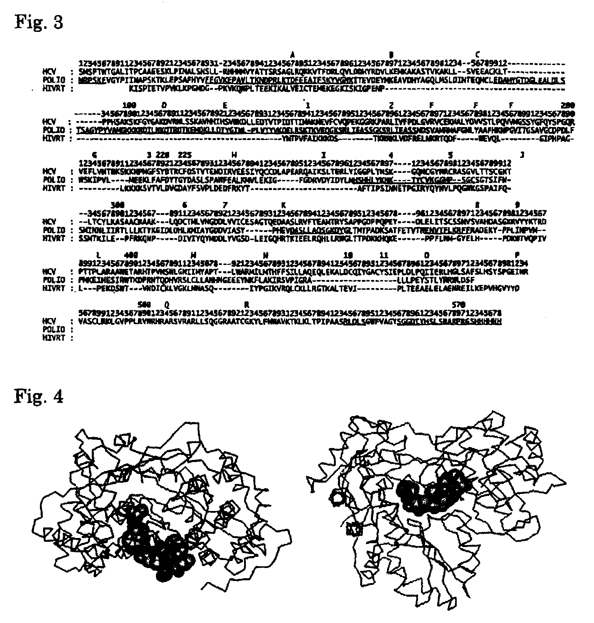

Figure 3 compares the amino acid sequences of the HCV polymerase, poliovirus polymerase, and HIV reverse transcriptase. HCV, POLIO, and HIVRT in the figure indicate HCV polymerase, poliovirus polymerase, and HIV reverse transcriptase, respectively. The underlined sequences indicate the parts where the structures have not been clarified by the above structure analysis. It is difficult to deduce the active site and RNA binding cleft only from the two-dimensional structures, as obvious in these amino acid sequences.

The three-dimensional structure analysis of $NS5B_{570}$ showed that the C-terminal structure at positions 545 to 570 can be incorporated into a part of the RNA binding cleft of the HCV polymerase itself. Figure 4 schematically shows the structure of $NS5B_{570}$, emphasizing its C-terminus. The RNA binding cleft in which the C-terminal structure is incorporated is narrow in comparison with that the C-terminus-truncated variants, $NS5B_{544}$ $NS5B_{536}$, and $NS5B_{531}$, and the glove is slightly closed. This supports that the RNA binding cleft can be a target for the inhibition of the polymerase activity.

Lys 90, 98, 106, and 175, and Arg 168 in the Holder domain, and Arg 465 in the Thumb domain are located within 5 Å from the phosphodiester backbone of the RNA double strand model in the binding model made on the computer, consisting of the tentative short double strand model formed by a template and a primer. These amino acids would have an important role on the binding of the RNA strand. Therefore, these regions can also be a target for the inhibition of the polymerase activity.

EXAMPLE 5

Evaluation of the HCV polymerase activity

Synthesis of a template RNA

The DNA fragment (148 bp) containing polyU and 3' X sequence was completely synthesized using synthetic primers designed based on the sequence of the 3' untranslational region of the HCV genome, and cloned into plasmid pBluescript SK II (+) (Stratagene). The cDNA encoding the full length NS5B prepared in the same manner as in Example 1 was digested with restriction enzyme *KpnI* to obtain the cDNA fragment covers the region from the restriction enzyme cleavage site to the stop codon. This cDNA fragment was ligated to the upstream of 3' untranslational region DNA of pBluescript SK II (+). The DNA insert of about 450 bp in total was used as a template for preparing the template RNA. The plasmid was cleaved just after 3' X sequence, circulated, treated with phenol/chloroform, and purified by ethanol precipitation to recover the DNA.

Using the purified DNA as a template, utilizing the promoter in pBluescript SK II (+), RNA was synthesized by the run-off method using MEGAscript RNA synthesis kit (Ambion), and T7 RNA polymerase (at 37°C for 3 hours). DNaseI was added thereto and further incubated for 1 hour, and the template DNA was decomposed and removed to obtain the crude RNA product. The crude product was treated with phenol/chloroform, and purified by ethanol precipitation to obtain the desired template RNA. After confirming the quality of the RNA by agarose gel electrophoresis, the RNA was stored at -80°C. The RNA is a template suitable for the highly sensitive measurement of the polymerase activity.

Measurement of the HCV polymerase activity

The reaction mixture with the composition mentioned below (30 μl) was reacted at 25°C for 90 min.

Reaction mixture: the HCV polymerase obtained in Example 1 (1 μg/ml), the template RNA obtained in Example 5 (10 μg /ml), ATP (50 μM), GTP (50 μM), CTP (50 μM), UTP (2 μM), [5, 6-$^3$H] UTP (46 Ci/mmol (Amersham), 1.5 μCi), 20 mM Tris-HCl (pH 7.5), EDTA (1 mM), $MgCl_2$ (5 mM), NaCl (50 mM), DTT (1 mM), BSA (0.01%).

The reaction was terminated by adding 10% trichloroacetic acid at 4°C and 1% sodium diphosphate solution (150 μl) to the reaction mixture, and kept in ice for 15 min to precipitate the RNA. The RNA was trapped on a glass filter (Whatman, GF/C) by suction filtration. The filter was washed with a solution containing 1% trichloroacetic acid and 0.1% sodium diphosphate, then with 90% ethanol, and dried. A liquid scintillation cocktail (Packard) was added to the RNA synthesized by the enzyme reaction, and the radioactivity of the RNA was measured by a liquid scintillation counter.

The activity of the HCV polymerase was calculated from the value of radioactivity in the enzyme reaction of each NS5B using the radioactivity in the enzyme reaction of $NS5B_{591}$ as a standard. Table 4 shows the result.

Table 4

| HCV polymerase activity | | | | | | |
|---|---|---|---|---|---|---|
| | HCV polymerase | | | | | |
| | $NS5B_{591}$ | $NS5B_{570}$ | $NS5B_{552}$ | $NS5B_{544}$ | $NS5B_{536}$ | $NS5B_{531}$ |
| relative activity (%) | 100 | 3 | 147 | 2014 | 2107 | 2149 |

EXAMPLE 6

HCV polymerase useful for measuring the HCV polymerase activity

The above results confirmed that $NS5B_{544}$, $NS5B_{536}$, and $NS5B_{531}$, show the HCV polymerase activity higher than the wild-type HCV polymerase $NS5B_{591}$, and $NS5B_{570}$; surprisingly 20-fold or more higher than $NS5B_{591}$.

Crystal structural analysis confirmed that evaluation of the HCV polymerase activity for the various mutans showed that $NS5B_{552}$ did not have high activity, and amino acid residues 517 to 526 of NS5B is the helix structure contained in the secondary structure which has an important role for maintaining higher structure (Example 3). From this result, the HCV polymerase comprising the amino acid sequence of $NS5B_{526}$, $NS5B_{527}$, $NS5B_{528}$, • • • to $NS5B_{551}$ are assumed to comprise high HCV polymerase activity.

These NS5B<sub>544</sub>, NS5B<sub>536</sub> and NS5B<sub>531</sub> comprising the high HCV polymerase activity are extremely useful for evaluating the inhibition level of the inhibitor candidate compounds in the evaluation of inhibitory activity *in vitro*, and HCV polymerase inhibitors can be efficiently designed or identified.

In addition, the inhibitors can be efficiently designed or identified by the above virtual screening based on structural coordinates of the HCV polymerase, for example, the structural coordinate of NS5B$_{544}$ shown in Table 3.

EXAMPLE 7

Identification of inhibitors for the HCV polymerase

The three-dimensional structural analysis of NS5B$_{570}$ obtained in Example 3 revealed that the polypeptide region at positions 545 to 570, the C-terminal structure of the HCV polymerase NS5B$_{570}$, is incorporated into a part of the RNA binding cleft of the HCV polymerase itself. Moreover, the comparison with the three-dimensional structure of NS5B$_{544}$ or other variants, confirmed that the RNA binding cleft in which the C-terminal structure is incorporated becomes slightly narrower than that in the C-terminus-truncated variants, NS5B$_{544}$, NS5B$_{536}$, and NS5B$_{531}$. This is compared to the slightly closed state of the glove. This suggests that the RNA binding deft can be a target for the for polymerase inhibitors.

On the other hand, the measurement of the HCV polymerase activity in Example 5 revealed that the HCV polymerase activities of NS5B$_{544}$, NS5B$_{536}$, and NS5B$_{531}$ were higher than those of NS5B$_{591}$, NS5B$_{570}$, and NS5B$_{552}$.

These facts suggest that the polypeptide regions at positions 545 to 570 and a compound with the similar structure to the region can be an HCV polymerase inhibitor.

It is easily assumed that the polypeptide fragment at positions 545 to 570 can be an HCV polymerase inhibitor.

The difference of the HCV polymerase activities was observed between NS5B$_{552}$ and NS5B$_{544}$. This indicates that the polypeptide fragment at positions 545 to 552 and its partial fragment or a compound containing these fragments are particularly effective as HCV polymerase inhibitors.

The computational analysis of the three-dimensional structure using the program software QUANTA (MSI) confirmed that the region at positions 545 to 552 maintains hydrophobic interaction with the region comprising the hydrophobic surface existing in "boundary site between Thumb and Palm domains." Furthermore, the computational analysis confirmed that in the polypeptide fragment at positions 545 to 552, Leu 547, Trp 550, and Phe 551, especially Trp 550 and Phe 551, strongly interact with said hydrophobic surface.

Specifically "boundary site between Thumb and Palm domains" means the site which is formed by Ser 196, Pro 197, Ile 413, Met 414, Ile 447, Tyr 448, Tyr 452, Ile 454, Ile 462, and Leu 466 in the amino acid sequence of the HCV polymerase. This experimental result suggests "boundary domain between Thumb and Palm domains" or a domain containing a part of it can be a target for the HCV polymerase inhibitors. Particularly, it was confirmed that the polypeptide region at positions 545 to 552 and its partial fragment or a compound containing these fragments are effective as HCV polymerase inhibitors.

Next, a synthetic peptide was prepared by a conventional method and its HCV polymerase-inhibitory activity was assessed. The synthetic peptide consists of the polypeptide region at positions 546 to 551 of HCV polymerase NS5B to both ends of which a Lys residue is attached and represented by the formula Lys-Asp-Leu-Ser-Gly-Trp-Phe-Lys. The synthetic peptide and NS5B$_{544}$ were pre-incubated at 25 °C for 30 minutes, and the polymerase activity was measured in the same manner as in Example 5. The synthetic peptide inhibited the polymerase activity 40 to 50% at a final concentration of 30 μM.

**Claims**

1. A polypeptide derived from HCV polymerase NS5B having an HCV polymerase activity and consisting of an amino acid sequence X-Y, wherein X is a consecutive amino acid sequence which is a portion of the NS5B, an N-terminal amino acid of X is the amino acid residue 1 (Ser) of the NS5B, and a C-terminal amino acid residue of X is any one of amino acid residues 531 (Lys) to 570 (Arg) of the NS5B; and wherein Y is a carboxyl group or an amino acid sequence which is not derived from NS5B; and one or more amino acids in the amino acid sequence of X may be modified, and methionine residues in the amino acid sequence of X may be replaced by selenomethionoine residues.

2. The polypeptide of claim 1, wherein the C-terminal amino acid residue of X is any one of amino acid residues 536 (Leu) to 552 (Val) of the NS5B.

3. The polypeptide of claim 2, wherein the C-terminal amino acid residue of X is any one of amino acid residues 536 (Leu) to 544 (Gln) of the NS5B.

4. The polypeptide of claim 2, wherein the C-terminal amino acid residue of X is any one of amino acid residues 531 (Lys) to 544 (Gln) in the NS5B.

5. The polypeptides of any one of claims 1 to 4, wherein methionine residues in the amino acid sequence of X are replaced by selenomethionine residues.

6. The polypeptides of any one of claims 1 to 5, wherein Y is an amino acid sequence not derived from NS5B, and said amino acid sequence is suitable for a column purification.

7. The polypeptides of any one of claims 1 to 6, wherein the NS5B comprises an amino acid sequence of SEQ ID NO: 1.

8. The polypeptide of claim 1, wherein said polypeptide is identified by an three-dimensional structural coordinates shown in Table 2 or 3.

9. A crystal comprising the polypeptide of any one of claims 1 to 8.

10. A DNA encoding the polypeptide of anyone of claims 1 to 8.

11. A method for determining a three-dimensional structural coordinates of a cocomplex or a variant of HCV polymerase NS5B by the molecular replacement method using a three-dimensional structure coordinate of said NS5B.

12. A method for designing or identifying HCV polymerase inhibitors, which comprises determining the complementarity of a test compound with an active site and/or RNA binding cleft of a polypeptide using the three-dimensional structural coordinate of said polypeptide or its part and the three dimensional structural coordinate of the test compound, wherein said polypeptide is derived from the HCV polymerase NS5B having an HCV polymerase activity and consisting of an amino acid sequence X-Y, wherein X is a consecutive amino acid sequence which is a portion of the NS5B, an N-terminal amino acid of X is the amino acid residue 1 (Ser) of the NS5B, a C-terminal amino acid residue of X is any one of amino acid residues 531 (Lys) to 570 (Arg) of the NS5B: and wherein Y is a carboxyl group or another amino acid sequence which is not derived from NS5B; and one or more amino acids in X may be modified, and methionine residues in the amino acid sequence of X may be replaced by selenomethionine residues.

13. A method for designing or identifying HCV polymerase inhibitors, which comprises the steps of:

(a) determining the complementarity of a test compound with an active site and/or RNA binding cleft of the a polypeptide using a three-dimensional structural coordinate of said polypeptide or its part and a three-dimensional structural coordinate of said test compound, wherein said polypeptide is derived from the HCV polymerase NS5B having an HCV polymerase activity and consisting of an amino acid sequence X-Y, wherein X is a consecutive amino acid sequence which is a portion of the NS5B, an N-terminal amino acid of X is the amino acid residue 1 (Ser) of the NS5B, a C-terminal amino acid residue of X is any one of amino acid residues 531 (Lys) to 570 (Arg) of the NS5B; and wherein Y is a carboxyl group of another amino acid sequence which is not derived from NS5B; and one or more amino acids in X may be modified, and methionine residues in the amino acid sequence of X may be replaced by selenomethionin residues;
(b) determining HCV polymerase-inhibitory activity of said test compound; and
(c) designing or determining HCV polymerase inhibitors using the complementarity data of said test compound determined in the above (a), and the inhibitory activity data obtained in the above (b).

14. The method of any one of claims 11 to 13, wherein the three-dimensional structural coordinate of the polypeptide is any one of the three-dimensional structural coordinates shown in Table 2 or 3.

15. A method for identifying HCV polymerase inhibitors, which comprises the steps of:

(a) obtaining a polypeptide, which is derived from the HCV polymerase NS5B has an HCV polymerase activity, and consisting of the amino add sequence X'-Y, wherein X' is a consecutive amino acid, sequence which is a portion of the NS5B, an N-terminal amino acid of X' is the amino acid residue 1 (Ser) of the NS5B, a C-terminal amino acid residue of X' is any one of amino acid residues 531 (Lys) to 544 (Gln) of the NS5B; and wherein Y is a carboxyl group or another amino acid sequence which is not derived from NS5B; and one or more amino acids in X' may be modified, and methionine residues in the amino acid sequence of X' may be replaced by selenomethionin residues;
(b) determining the HCV polymerase activity of said polypeptide by reacting said polypeptide obtained in the above (a) with a template RNA and substrates in the presence of a test compound;
(c) determining the HCV polymerase activity of said polypeptide by reacting polypeptide obtained in the above (a) with a template RNA and substrates in the absence of said test compound; and
(d) comparing the HCV polymerase activity of the above (b) with the HCV polymerase activity of the above (c).

16. An HCV polymerase inhibitor, identified by the method in any one of claims 12 to 15.

17. An HCV polymerase inhibitor that inhibits the HCV polymerase activity of HCV polymerase NS5B by acting the boundary between the Thumb and Palm domains of NS5B.

18. The HCV polymerase inhibitor of claim 17, wherein said inhibitor is a polypeptide represented by the formula (I) or a pharmaceutically acceptable salf thereof:

$$Z^1\text{-}Z^2\text{-}Z^3\text{-Leu-}Z^4\text{-}Z^5\text{-Trp-Phe-}Z^6 \qquad\qquad (I)$$

wherein $Z^1$ and $Z^6$ each represent a hydrophilic group or an amino acid residue; $Z^2$ and $Z^3$ each represent a single bond or an amino acid residue; and $Z^4$ and $Z^5$ each represent an amino acid residue.

Fig. 1

Fig. 2

## Fig. 3

## Fig. 4